(19) Europäisches Patentamt
European Patent Office
Office européen des brevets

(11) EP 1 764 360 A1

(12) **EUROPEAN PATENT APPLICATION**
published in accordance with Art. 158(3) EPC

(43) Date of publication:
**21.03.2007 Bulletin 2007/12**

(21) Application number: **05758059.9**

(22) Date of filing: **01.07.2005**

(51) Int Cl.:
***C07D 211/62*** *(2006.01)*

(86) International application number:
**PCT/JP2005/012635**

(87) International publication number:
**WO 2006/004200 (12.01.2006 Gazette 2006/02)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **02.07.2004 JP 2004196723**

(71) Applicant: **Sankyo Company, Limited**
**Tokyo 103-8426 (JP)**

(72) Inventors:
- **KURATA, Hitoshi c/o Sankyo Company Limited Shinagawa-ku, Tokyo 140-8710 (JP)**
- **UTO, Yoshikazu c/o Sankyo Company Limited Shinagawa-ku, Tokyo 140-8710 (JP)**
- **FUJIBAYASHI, Yuko c/o Sankyo Company Limited Shinagawa-ku, Tokyo 140-8710 (JP)**
- **KOHAMA, Takafumi c/o Sankyo Company Limited Shinagawa-ku, Tokyo 140-8710 (JP)**
- **TANIMOTO, Tatsuo c/o Sankyo Company Limited Shinagawa-ku, Tokyo 140-8710 (JP)**
- **KARASAWA, Hiroshi c/o Sankyo Company Limited Shinagawa-ku, Tokyo 140-8710 (JP)**

(74) Representative: **Gibson, Christian John Robert**
**Marks & Clerk**
**90 Long Acre**
**London WC2E 9RA (GB)**

(54) **UREA DERIVATIVE**

(57) The present invention relates to a urea derivative or a pharmacologically acceptable salt thereof having an excellent DGAT inhibitory effect. A urea derivative having the formula:

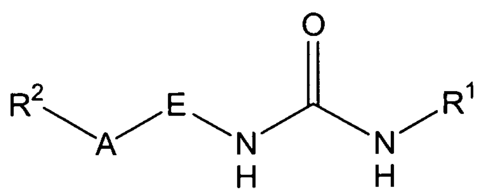

(I)

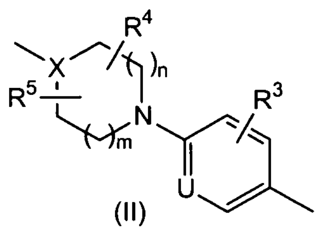

(II)

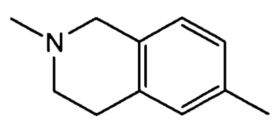

(III)

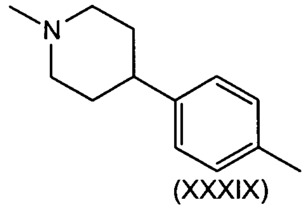

(XXXIX)

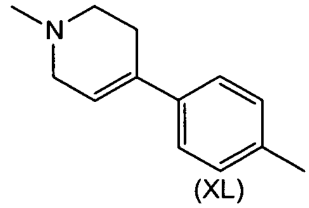

(XL)

[wherein $R^1$ is a $C_6$-$C_{10}$ aryl group which may be independently mono- to pentasubstituted by a group selected from Substituent Group a or others; $R^2$ is a $C_6$-$C_{10}$ aryl group which may be independently mono- to pentasubstituted by a group selected from Substituent Group a or others; E is a group having the formula (II) or the formula (III) (wherein $R^3$

EP 1 764 360 A1

is a hydrogen atom or others; $R^4$ and $R^5$, which are the same or different, are a hydrogen atom or others; X and U, which are the same or different, are a group represented by the formula CH or others; m and n, which are the same or different, are 1 or another number) or others; and A is a group represented by the formula -NH-C(=O)- or others], or a pharmacologically acceptable salt thereof.

## Description

### Technical Field

The present invention relates to a urea derivative having a specific chemical structure or a pharmacologically acceptable salt thereof, which has an excellent acyl-coenzyme A: diacylglycerol acyltransferase (Acyl-CoA: diacylglycerol acyltransferase, hereinafter also DGAT) inhibitory effect.

### Background Art

Adiposity is a condition of having significantly greater body weight than an average body weight as a result of accumulation of neutral fat (triacylglycerol, or triglyceride, hereinafter TG) in fat cells due to continuous excess energy intake compared to energy consumption (Eiji Itagaki, "STEP series, Metabolism, Endocrinology", KAIBASHOBO, LTD. 1st ed., 1998, p.105). Being adipose leads to life-style related diseases such as hyperlipidemia, hypertriglyceridemia, diabetes, hypertension and arteriosclerosis, cerebrovascular disorders, coronary artery diseases, respiratory abnormality, lower back pain, knee osteoarthritis, gout or cholecystitis. Adiposity with a complication of these diseases or adiposity which may lead to such a complication later is defined as obesity and treated as a disease.

Further, it has been recently revealed that adiposity is one of the major causes of a life-style related disease called metabolic syndrome (Zimmet, P. et al., Nature, 2001, vol.414, p.782-787). It has been reported that fatty acid and factors such as TNF-$\alpha$ are released from accumulated visceral fat in obese individuals, and cause insulin resistance in skeletal muscle, the liver and fat tissue, and facilitate synthesis of neutral fat in the liver, resulting in hyperlipidemia. Moreover, increased insulin concentration in the blood caused by insulin resistance leads to impaired glucose tolerance and diabetes, and systemic vascular resistance increases due to increased reabsorption of Na ions in the kidney and activation of sympathetic nerves, causing hypertension. It is considered that hyperlipidemia, diabetes and hypertension caused by adiposity trigger angiopathy such as cerebrovascular disorders or coronary artery diseases caused by arteriosclerosis, leading to severe, life-threatening clinical conditions.

Central anorectics such as mazindol (Engstrom, R. G. et al., (1975) Arch. Intern. Pharmacodyn., 1975, vol. 214, p.308-321) and sibutramine (Bray, G. A. et al., Obes. Res., 1996, vol.4, p.263-270) and pancreatic lipase inhibitors such as orlistat are known as anti-obesity drugs presently used. However, central anorectics have side effects such as dry mouth, constipation, gastric discomfort, and in some cases auditory hallucination and visual hallucination. Orlistat (Davidson, M. H. et al., The Journal of the American Medical Association, 1999, vo1.281, p.235-242) may have side effects in the gastrointestinal tract, such as diarrhea, incontinence, steatorrhea and flatus. Accordingly, development of effective drugs with fewer side effects is desired.

Animals and plants store lipids as insoluble TG and produce energy by decomposing TG according to need. TG taken from food is decomposed into free fatty acid and monoacylglycerol in the lumen of the small intestine by the action of bile acid and pancreatic lipase. Micelles composed of free fatty acid, monoacylglycerol and bile acid are absorbed into epithelial cells of the small intestine, and TG is newly synthesized in the endoplasmic reticulum by the action of acyl-coenzyme A synthetase (hereinafter ACS), acyl-coenzyme A: monoacylglycerol acyltransferase and DGAT. TG is combined with phospholipid, cholesterol and apolipoprotein and secreted into the lymph vessels in the stomach and intestine in the form of chylomicron. TG is then secreted into the blood through the lymphatic duct, transferred to peripheries and used. On the other hand, TG is also synthesized in fat tissue from glycerol 3-phosphate and free fatty acid by the action of ACS, glycerol 3-phosphate acyltransferase, lysophosphatidic acid acyltransferase and DGAT (Coleman, R., Bell, R., J. Biol. Chem., 1976, vol. 251, p.4537-4543). TG taken excessively is thus accumulated in fat tissue, and consequently adiposity is caused.

DGAT is an enzyme present in the endoplasmic reticulum of cells and catalyzes the most important reaction in the final step in the pathway of TG synthesis, i.e., the reaction of transferring acyl groups of acyl-coenzyme A to the 3 position of 1,2-diacylglycerol (Coleman, R., Methods in Enzymology, 1992, vol. 209, p.98-104; Lehner, R., Kuksis, A., Prog. Lipid Res., 1996, vol. 35, p.169-201; R. Bell., Ann. Rev. Biochem., 1980, vol.49, p.459-487). It is reported that DGAT has two isozymes DGAT1 (Cases, S. et al., Proc. Natl. Acad. Sci. USA., 1998, vol.95, p.13018-13023) and DGAT2 (Cases, S. et al., J. Biol. Chem., 2001, vol.276, p.38870-38876). It is believed that since DGAT1 is highly expressed in the small intestine and fat tissue and DGAT2 is highly expressed in the liver and fat tissue, DGAT1 is involved mostly in absorption of fat from the small intestine and accumulation of fat in fat tissue, and DGAT2 is involved mostly in the synthesis of TG or secretion of VLDL (very low density lipoproteins) in the liver and accumulation of fat in fat tissue. Although the difference in the role of DGAT1 and DGAT2 has not been fully elucidated yet, it is suggested that DGAT is relevant to adiposity, lipid metabolism and glucose metabolism (Coleman, R.A., Lee, D.P., Progress in Lipid Research, 2004, vol.43, p.134-176). DGAT is a key enzyme for synthesizing TG in epithelial cells in the gastrointestinal tract and fat tissue. Drugs which inhibit DGAT inhibit TG synthesis and thus inhibit absorption of fat in the gastrointestinal tract and accumulation of fat in fat tissue. Accordingly, such drugs are expected to be useful as a therapeutic or prophylactic

agent for adiposity, obesity, hyperlipidemia, hypertriglyceridemia, lipidosis, insulin resistance syndrome, diabetes, or hyperlipidemia, hypertriglyceridemia, lipidosis, insulin resistance syndrome, diabetes, hypertension, arteriosclerosis or cerebrovascular disorders caused by adiposity or coronary artery diseases (Smith, S.J. et al., Nat. Genet., 2000, vol. 25, p.87-90; Chen, H. C., J. Clin. Invest., 2002, vol.109, p.1049-1055; Buhman, K.K., J. Biol. Chem., 2002, vol.277, p. 25474-25479; Gaziano, J., et al., Circulation, 1997, vol.96, p.2520-2525).

Although several compounds with a DGAT inhibitory effect have been known so far, all these compounds have a different structure from the compound of the present invention (for example, see Japanese Patent Application Publication No. 2002-306199, Tomoda, H. et al., J. Antibiot. (Tokyo), 1995, vol.48, p.937-941, Yang, D.J. et al., J. Antibiot. (Tokyo), 1996, vol.49, p.223-229, Tomoda, H. et al., J. Antibiot. (Tokyo), 1999, vol. 52, p.689-694, or Tabata, N. et al., Phytochemistry, 1997, vol.46, p.683-687).

While compounds having a structure similar to that of the compound of the present invention are known, the fact that such compounds have a DGAT inhibitory effect is not known (see, for example, International Publication No. WO 03/045926).

Moreover, compounds which have an effect to inhibit absorption of fat from the small intestine by inhibiting mainly DGAT1 of DGAT have not been known before.

Disclosure of the Invention

The present inventors have conducted intensive studies on compounds with a DGAT inhibitory effect and as a result have found that a urea compound having a specific chemical structure has an excellent DGAT inhibitory effect and particularly high inhibitory effect against DGAT1. The present inventors have also found that the urea compound is useful as an active ingredient of a medicine for prevention and/or treatment of a disease selected from the group consisting of adiposity, obesity, hyperlipidemia, hypertriglyceridemia, lipidosis, insulin resistance syndrome, impaired glucose tolerance, diabetes, diabetic complications (including diabetic peripheral neuropathy, diabetic nephropathy, diabetic retinopathy and diabetic macroangiopathy), cataract, gestational diabetes mellitus, polycystic ovary syndrome, arteriosclerosis (including arteriosclerosis caused by the diseases described above and below), atherosclerosis and diabetic atherosclerosis, or as an active ingredient of a medicine for treatment and/or prevention of a disease described below caused by adiposity selected from the group consisting of hyperlipidemia, hypertriglyceridemia, lipidosis, insulin resistance syndrome, impaired glucose tolerance, diabetes, diabetic complications (including diabetic peripheral neuropathy, diabetic nephropathy, diabetic retinopathy and diabetic macroangiopathy), cataract, gestational diabetes mellitus, polycystic ovary syndrome, arteriosclerosis (including arteriosclerosis caused by the diseases described above and below), atherosclerosis, diabetic atherosclerosis, hypertension, cerebrovascular disorders, coronary artery diseases, fatty liver, respiratory abnormality, lower back pain, knee osteoarthritis, gout and cholecystitis. The present invention has been completed based on the above findings.

The present invention relates to:

(1) a urea derivative having the general formula (I):

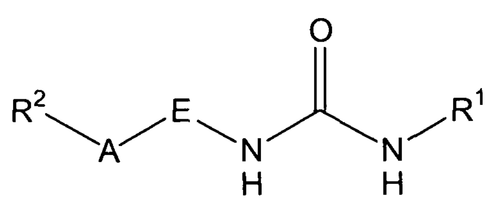

(I)

[wherein $R^1$ represents a $C_1$-$C_{10}$ alkyl group, a $C_3$-$C_6$ cycloalkyl group, a $C_6$-$C_{10}$ aryl group which may be independently mono- to pentasubstituted by a group selected from Substituent Group a or a heterocyclic group which may be independently mono- to trisubstituted by a group selected from Substituent Group a,

$R^2$ represents a hydrogen atom, a $C_1$-$C_6$ alkyl group, a $C_6$-$C_{10}$ aryl group which may be independently mono- to pentasubstituted by a group selected from Substituent Group a, a heterocyclic group which may be independently mono- to trisubstituted by a group selected from Substituent Group a, a $C_7$-$C_{16}$ aralkyl group which may be independently mono- to trisubstituted by a group selected from Substituent Group a, $C_3$-$C_6$ cycloalkyl group which may be monosubstituted by a $C_1$-$C_6$ alkyl group, a $C_7$-$C_{10}$ bicycloalkyl group or a tetralyl group,

E is a group having the formula (II), the formula (III), the formula (XXXIX) or the formula (XL):

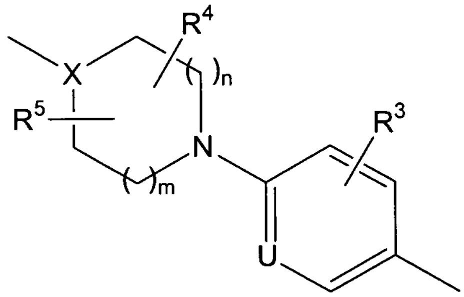

(II)

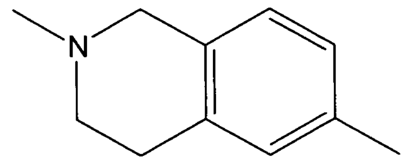

(III)

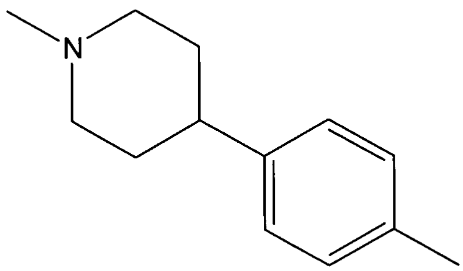

(XXXIX)

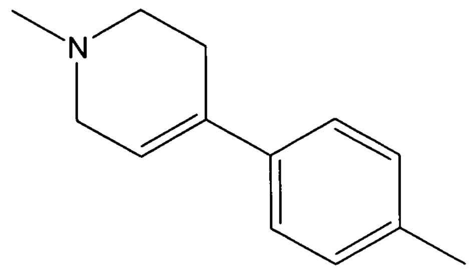

(XL)

(wherein $R^3$ represents a hydrogen atom, a $C_1$-$C_6$ alkyl group, a halogen atom or a cyano group, $R^4$ represents a hydrogen atom or a $C_1$-$C_6$ alkyl group, $R^5$ represents a hydrogen atom or a $C_1$-$C_6$ alkyl group, X and U, which are the same or different, represent a group represented by the formula CH or a nitrogen atom, m and n, which are the same or different, represent an integer of 1 or 2; provided that the X side of the group having the formula (II) and the nitrogen atom side of the groups having the formula (III), the formula (XXXIX) and the formula (XL) are each bonded to A in the compound represented by the general formula (I), and the carbon atom side of the aromatic ring of the groups having the formula (II), the formula (III), the formula (XXXIX) and the formula (XL) is bonded to the nitrogen atom in the compound represented by the general formula (I); and $R^4$ and $R^5$ may be bonded to different carbon atoms or the same carbon atom),
A represents a single bond, a group represented by the formula -O-C(=O)-, a group represented by the formula -O-C(=S)-, a group represented by the formula-NH-C(=O)-, a group represented by the formula -NH-C(=S)-, a carbonyl group, a thiocarbonyl group or a group represented by the formula -CH(OH)-C(=O)-(provided that the oxygen atom side, the nitrogen atom side and the side of the group represented by the formula CH(OH) are each bonded to $R^2$ in the compound represented by the general formula (I), and the carbonyl group side and the thiocarbonyl group side are each bonded to E in the compound represented by the general formula (I)),
excluding the case where $R^2$ represents a hydrogen atom and A represents a single bond,
Substituent Group a means the group consisting of a halogen atom, a $C_1$-$C_{10}$ alkyl group, a $C_1$-$C_6$ halogenated alkyl group, a $C_1$-$C_6$ hydroxyalkyl group, a $C_1$-$C_6$ alkyl group monosubstituted by -P(=O)(O-$C_1$-$C_6$ alkyl)$_2$, a $C_3$-$C_6$ cycloalkyl group, a $C_1$-$C_{10}$ alkoxy group, a $C_1$-$C_6$ halogenated alkoxy group, a $C_1$-$C_6$ alkoxy group mono- or disubstituted by a hydroxyl group, a $C_1$-$C_6$ alkoxy group monosubstituted by a $C_3$-$C_6$ cycloalkyl group, a $C_2$-$C_6$ alkenyloxy group, a $C_2$-$C_6$ alkynyloxy group, a ($C_1$-$C_6$ alkoxy)-($C_1$-$C_6$ alkyl) group, a $C_1$-$C_6$ alkylthio group, a carboxyl group, a $C_2$-$C_7$ alkoxycarbonyl group, an amino group, a mono-$C_2$-$C_7$ alkylcarbonylamino group, a mono-$C_1$-$C_6$ alkylsulfonylamino group, a mono-$C_1$-$C_6$ alkylamino group, a di-($C_1$-$C_6$ alkyl)amino group, a N-($C_1$-$C_6$ alkyl)-N-($C_1$-$C_6$ hydroxyalkyl)amino group, a di-($C_1$-$C_6$ hydroxyalkyl)amino group, a cyano group, a nitro group, a nitrogen-containing heterocyclic group which may be monosubstituted by a hydroxyl group, a $C_6$-$C_{10}$ aryl group which may be independently mono- to trisubstituted by a group selected from Substituent Group b, a $C_6$-$C_{10}$ aryloxy group which may be independently mono- to trisubstituted by a group selected from Substituent Group b, a hydroxyl group, a $C_1$-$C_6$ alkyl group disubstituted by hydroxyl groups, a $C_1$-$C_6$ alkyl group monosubstituted by -P(=O)(OH)(O-$C_1$-$C_6$ alkyl), a $C_1$-$C_6$ alkyl group monosubstituted by a carboxyl group, a $C_1$-$C_6$ alkyl group monosubstituted by a $C_2$-$C_7$ alkoxycarbonyl group and 2,2-dimethyl-1,3-dioxa-4-cyclopentyl group, and
Substituent Group b means the group consisting of a halogen atom, a $C_1$-$C_6$ alkyl group and a $C_1$-$C_6$ halogenated alkyl group], or a pharmacologically acceptable salt thereof.

The present invention preferably includes:

(2) The urea derivative or the pharmacologically acceptable salt thereof according to (1), wherein $R^1$ represents a phenyl group independently mono- to trisubstituted by a group selected from the group consisting of a halogen atom, a $C_1$-$C_6$ alkyl group, a $C_1$-$C_6$ halogenated alkyl group, a $C_1$-$C_6$ alkoxy group, a carboxyl group, an amino group, a mono-$C_2$-$C_7$ alkylcarbonylamino group, a di-($C_1$-$C_6$ alkyl)amino group, a cyano group and a nitro group or a thiazolyl group which may be independently mono- or disubstituted by a $C_1$-$C_6$ alkyl group;

(3) The urea derivative or the pharmacologically acceptable salt thereof according to (1), wherein $R^1$ is a phenyl group independently mono- to trisubstituted by a group selected from the group consisting of a halogen atom, a $C_1$-$C_6$ alkyl group and a $C_1$-$C_6$ alkoxy group or a thiazolyl group independently mono- or disubstituted by a $C_1$-$C_6$ alkyl group;

(4) The urea derivative or the pharmacologically acceptable salt thereof according to (1), wherein $R^1$ is a phenyl group substituted by a group selected from the group consisting of a fluorine atom, a chlorine atom, a methyl group, a methoxy group and an ethoxy group at the 2- or 3-position; a phenyl group independently disubstituted by a group selected from the group consisting of a fluorine atom, a chlorine atom, a methyl group, a methoxy group and an ethoxy group at the 2, 3 or 5-position; or a 5-methyl-2-thiazolyl group;

(5) The urea derivative or the pharmacologically acceptable salt thereof according to (1), wherein $R^1$ is a 2-fluorophenyl group, a 2-ethoxyphenyl group, a 3-ethoxyphenyl group, a 5-fluoro-2-methoxyphenyl group, a 2-ethoxy-5-fluorophenyl group, a 5-chloro-2-methoxyphenyl group, a 5-chloro-2-ethoxyphenyl group, a 2-methoxy-5-methylphenyl group or a 3,5-dimethoxymethylphenyl group;

(6) The urea derivative or the pharmacologically acceptable salt thereof according to (1), wherein $R^1$ is a $C_1$-$C_6$ alkyl group; or a phenyl group which may be independently mono- to trisubstituted by a group selected from the group consisting of a halogen atom, a $C_1$-$C_6$ alkyl group, a $C_1$-$C_2$ halogenated alkyl group, a $C_1$-$C_3$ hydroxyalkyl group, a $C_1$-$C_6$ alkoxy group, a $C_1$-$C_2$ halogenated alkoxy group, a carboxyl group, an amino group, a mono-$C_2$-$C_3$ alkylcarbonylamino group, a di-$C_1$-$C_2$ alkylamino group, a cyano group, a nitro group and a phenyloxy group;

(7) The urea derivative or the pharmacologically acceptable salt thereof according to (1), wherein $R^1$ is a $C_1$-$C_6$ alkyl group; or a phenyl group which may be independently mono- to trisubstituted by a group selected from the group consisting of a fluorine atom, a chlorine atom, a bromine atom, a methyl group, an ethyl group, a propyl group, an isopropyl group, a butyl group, an isobutyl group, a s-butyl group, a t-butyl group, a trifluoromethyl group, a methoxy group, an ethoxy group, a propoxy group, an isopropoxy group, a butoxy group, a carboxyl group, an amino group, a methylcarbonylamino group, a dimethylamino group, a cyano group and a nitro group;

(8) The urea derivative or the pharmacologically acceptable salt thereof according to (1), wherein $R^1$ is a hexyl group; a phenyl group substituted by a fluorine atom, a chlorine atom, a methyl group, an ethyl group, a trifluoromethyl group, a methoxy group or an ethoxy group at the 2- and 5-positions; a phenyl group substituted by a butoxy group or a dimethylamino group at the 4-position; or a phenyl group substituted by a fluorine atom, a chlorine atom, a methyl group, an ethyl group, a methoxy group or an ethoxy group at the 2-position;

(9) The urea derivative or the pharmacologically acceptable salt thereof according to (1), wherein $R^1$ is a 5-fluoro-2-methoxyphenyl group, a 2-ethoxy-5-fluorophenyl group, a 5-chloro-2-methoxyphenyl group, a 5-chloro-2-ethoxyphenyl group, a 2-methoxy-5-methylphenyl group, a 2-ethoxy-5-methylphenyl group, a 4-butoxyphenyl group, a 4-dimethylaminophenyl group, a 2-fluorophenyl group or a 2-ethoxyphenyl group;

(10) The urea derivative or the pharmacologically acceptable salt thereof according to any one of (1) to (9), wherein $R^2$ is a phenyl or pyridyl group which may be independently mono- to trisubstituted by a group selected from the group consisting of a halogen atom, a $C_1$-$C_6$ alkyl group, a $C_1$-$C_6$ halogenated alkyl group, a $C_1$-$C_6$ hydroxyalkyl group, a $C_1$-$C_6$ alkoxy group, a $C_1$-$C_6$ alkoxy group mono- or disubstituted by a hydroxyl group, a carboxyl group, an amino group, a mono-$C_2$-$C_7$ alkylcarbonylamino group, a cyano group, a nitro group, a hydroxyl group, a $C_1$-$C_6$ alkyl group monosubstituted by a carboxyl group and a $C_1$-$C_6$ alkyl group monosubstituted by a $C_2$-$C_7$ alkoxycarbonyl group;

(11) The urea derivative or the pharmacologically acceptable salt thereof according to any one of (1) to (9), wherein $R^2$ is a phenyl or pyridyl group which may be independently mono- to trisubstituted by a group selected from the group consisting of a halogen atom, a $C_1$-$C_6$ alkyl group, a $C_1$-$C_6$ halogenated alkyl group, a $C_1$-$C_6$ alkoxy group mono- or disubstituted by a hydroxyl group and a cyano group;

(12) The urea derivative or the pharmacologically acceptable salt thereof according to any one of (1)to (9), wherein $R^2$ is a phenyl group substituted by a group selected from the group consisting of a fluorine atom, a chlorine atom, a methyl group and a trifluoromethyl group at the 2-position and substituted by a group selected from the group consisting of a 2-hydroxyethoxy group and a 2,3-dihydroxypropoxy group at the 4- or 5-position; a 3-methyl-2-pyridyl group; a 3-cyano-2-pyridyl group; or a 3,5-difluoro-2-pyridyl group;

(13) The urea derivative or the pharmacologically acceptable salt thereof according to any one of (1) to (9), wherein $R^2$ is a 2-chloro-4-(2-hydroxyethoxy)phenyl group, a 2-chloro-5-(2-hydroxyethoxy)phenyl group, a 4-(2-hydroxyethoxy)-2-trifluoromethylphenyl group, a 2-chloro-4-(2,3-dihydroxypropoxy)phenyl group, a 4-(2,3-dihydroxypropoxy)-2-trifluoromethylphenyl group, a 2-chloro-5-(2,3-dihydroxypropoxy)phenyl group or a 3-methyl-2-pyridyl

group;

(14) The urea derivative or the pharmacologically acceptable salt thereof according to any one of (1) to (9), wherein $R^2$ is a $C_1$-$C_4$ alkyl group; or a phenyl or benzyl group which may be independently mono- to trisubstituted by a group selected from the group consisting of a halogen atom, a $C_1$-$C_6$ alkyl group, a $C_1$-$C_2$ halogenated alkyl group, a $C_1$-$C_3$ hydroxyalkyl group, a $C_1$-$C_6$ alkoxy group, a $C_1$-$C_2$ halogenated alkoxy group, a carboxyl group, an amino group, a mono-$C_2$-$C_3$ alkylcarbonylamino group, a di-$C_1$-$C_2$ alkylamino group, a cyano group, a nitro group and a phenyloxy group;

(15) The urea derivative or the pharmacologically acceptable salt thereof according to any one of (1) to (9), wherein $R^2$ is a $C_1$-$C_4$ alkyl group; or a phenyl group which may be independently mono- to trisubstituted by a group selected from the group consisting of a fluorine atom, a chlorine atom, a bromine atom, a methyl group, an ethyl group, a propyl group, an isopropyl group, a butyl group, an isobutyl group, a s-butyl group, a t-butyl group, a trifluoromethyl group, a hydroxymethyl group, a methoxy group, an ethoxy group, a propoxy group, an isopropoxy group, a butoxy group, a carboxyl group, an amino group, an acetamido group, a dimethylamino group, a cyano group and a nitro group;

(16) The urea derivative or the pharmacologically acceptable salt thereof according to any one of (1) to (9), wherein $R^2$ is a t-butyl group; a phenyl group substituted by a fluorine atom, a chlorine atom, a bromine atom, a methyl group, a trifluoromethyl group, a hydroxymethyl group, a carboxyl group, an amino group or a nitro group at the 2- and 6-positions; a phenyl group substituted by a fluorine atom, a chlorine atom, a bromine atom, a methyl group, an ethyl group, a propyl group, an isopropyl group, a butyl group, a trifluoromethyl group, a methoxy group, a carboxyl group, an amino group, an acetamido group or a cyano group at the 2- and 4-positions; or a phenyl group substituted by a chlorine atom, a bromine atom, a methyl group or a trifluoromethyl group at the 2-position;

(17) The urea derivative or the pharmacologically acceptable salt thereof according to any one of (1) to (9), wherein $R^2$ is a 2,6-difluorophenyl group, a 2-fluoro-6-trifluoromethylphenyl group, a 2,6-dichlorophenyl group, a 2-chloro-6-methylphenyl group, a 2,6-dimethylphenyl group, a 2-hydroxymethyl-6-methylphenyl group, a 2-methoxy-6-methylphenyl group, a 2-carboxy-6-methylphenyl group, a 4-fluoro-2-trifluoromethylphenyl group, a 2-chloro-4-methylphenyl group, 4-carboxy-2-chlorophenyl group, a 4-amino-2-chlorophenyl group, a 4-acetamido-2-chlorophenyl group, a 4-methoxy-2-methylphenyl group, a 4-carboxy-2-methylphenyl group, a 4-amino-2-trifluoromethylphenyl group, a 4-acetamido-2-trifluoromethylphenyl group, a 2-methylphenyl group or a 2-trifluoromethylphenyl group;

(18) The urea derivative or the pharmacologically acceptable salt thereof according to any one of (1) to (17), wherein E is a group having the above formula (II);

(19) The urea derivative or the pharmacologically acceptable salt thereof according to (18), wherein $R^3$ is a hydrogen atom, a fluorine atom, a chlorine atom or a methyl group;

(20) The urea derivative or the pharmacologically acceptable salt thereof according to (18), wherein $R^3$ is a hydrogen atom, a fluorine atom or a chlorine atom;

(21) The urea derivative or the pharmacologically acceptable salt thereof according to (18), wherein $R^3$ is a hydrogen atom;

(22) The urea derivative or the pharmacologically acceptable salt thereof according to any one of (18) to (21), wherein $R^4$ is a hydrogen atom or a methyl group;

(23) The urea derivative or the pharmacologically acceptable salt thereof according to any one of (18) to (21), wherein $R^4$ is a hydrogen atom;

(24) The urea derivative or the pharmacologically acceptable salt thereof according to any one of (18) to (23), wherein $R^5$ is a hydrogen atom or a methyl group;

(25) The urea derivative or the pharmacologically acceptable salt thereof according to any one of (18) to (23), wherein $R^5$ is a hydrogen atom;

(26) The urea derivative or the pharmacologically acceptable salt thereof according to any one of (18) to (25), wherein X is a nitrogen atom;

(27) The urea derivative or the pharmacologically acceptable salt thereof according to any one of (18) to (26), wherein U is a group represented by the formula CH;

(28) The urea derivative or the pharmacologically acceptable salt thereof according to any one of (18) to (27), wherein m and n are 1;

(29) The urea derivative or the pharmacologically acceptable salt thereof according to any one of (1) to (17), wherein E is a group having the above formula (XL);

(30) The urea derivative or the pharmacologically acceptable salt thereof according to any one of (1) to (29), wherein A is a single bond, a group represented by the formula -O-C(=O)-, a group represented by the formula -NH-C(=O)- or a carbonyl group;

(31) The urea derivative or the pharmacologically acceptable salt thereof according to any one of (1) to (29), wherein A is a group represented by the formula -O-C(=O)- or a group represented by the formula -NH-C(=O)-;

(32) The urea derivative or the pharmacologically acceptable salt thereof according to any one of (1) to (29), wherein

A is a group represented by the formula -NH-C(=O)-;

(33) The urea derivative or the pharmacologically acceptable salt thereof according to any one of (1) to (29), wherein A is a group represented by the formula -CH(OH)-C(=O)-;

(34) The urea derivative or the pharmacologically acceptable salt thereof according to (1), wherein $R^1$ is a $C_1$-$C_{10}$ alkyl group, a $C_3$-$C_6$ cycloalkyl group, a $C_6$-$C_{10}$ aryl group which may be independently mono- to pentasubstituted by a group selected from Substituent Group a or a heterocyclic group which may be independently mono- to trisubstituted by a group selected from Substituent Group a, $R^2$ is a hydrogen atom, a $C_1$-$C_6$ alkyl group, a $C_6$-$C_{10}$ aryl group which may be independently mono- to pentasubstituted by a group selected from Substituent Group a, a heterocyclic group which may be independently mono- to trisubstituted by a group selected from Substituent Group a, a $C_7$-$C_{16}$ aralkyl group which may be independently mono- to trisubstituted by a group selected from Substituent Group a, $C_3$-$C_6$ cycloalkyl group which may be monosubstituted by a $C_1$-$C_6$ alkyl group, a $C_7$-$C_{10}$ bicycloalkyl group or a tetralyl group, E is a group having the formula (II) or the formula (III), $R^3$ is a hydrogen atom, a $C_1$-$C_6$ alkyl group or a halogen atom, $R^4$ is a hydrogen atom, a $C_1$-$C_6$ alkyl group, a $C_1$-$C_6$ hydroxyalkyl group, a carboxyl group or an oxo group, $R^5$ is a hydrogen atom, a $C_1$-$C_6$ alkyl group or an oxo group, X and U, which are the same or different, are a group represented by the formula CH or a nitrogen atom, m and n, which are the same or different, are an integer of 1 to 3 (provided that the X side of the group having the formula (II) and the nitrogen atom side of the group having the formula (III) are each bonded to A in the compound represented by the general formula (I), and the carbon atom side of the aromatic ring of the group having the formula (II) or the formula (III) is bonded to the nitrogen atom in the compound represented by the general formula (I); $R^4$ and $R^5$ may be bonded to different carbon atoms or the same carbon atom), A is a single bond, a group represented by the formula -O-C(=O)-, a group represented by the formula -O-C(=S)-, a group represented by the formula -NH-C(=O)-, a group represented by the formula -NH-C(=S)-, a carbonyl group or a thiocarbonyl group (provided that the oxygen atom side and the nitrogen atom side are each bonded to $R^2$ in the compound represented by the general formula (I), and the carbonyl group side and the thiocarbonyl group side are each bonded to E in the compound represented by the general formula (I)), excluding the case where $R^2$ is a hydrogen atom and A is a single bond, Substituent Group a is the group consisting of a halogen atom, a $C_1$-$C_{10}$ alkyl group, a $C_1$-$C_6$ halogenated alkyl group, a $C_1$-$C_6$ hydroxyalkyl group, a $C_1$-$C_6$ alkyl group monosubstituted by -P(=O)(O-$C_1$-$C_6$ alkyl)$_2$, a $C_3$-$C_6$ cycloalkyl group, a $C_1$-$C_{10}$ alkoxy group, a $C_1$-$C_6$ halogenated alkoxy group, a $C_1$-$C_6$ alkoxy group mono- or disubstituted by a hydroxyl group, a $C_1$-$C_6$ alkoxy group monosubstituted by a $C_3$-$C_6$ cycloalkyl group, a $C_2$-$C_6$ alkenyloxy group, a $C_2$-$C_6$ alkynyloxy group, a ($C_1$-$C_6$ alkoxy)-($C_1$-$C_6$ alkyl) group, a $C_1$-$C_6$ alkylthio group, a carboxyl group, a $C_2$-$C_7$ alkoxycarbonyl group, an amino group, a mono-$C_2$-$C_7$ alkylcarbonylamino group, a mono-$C_1$-$C_6$ alkylsulfonylamino group, a mono-$C_1$-$C_6$ alkylamino group, a di-($C_1$-$C_6$ alkyl)amino group, a N-($C_1$-$C_6$ alkyl)-N-($C_1$-$C_6$ hydroxyalkyl)amino group, a di-($C_1$-$C_6$ hydroxyalkyl)amino group, a cyano group, a nitro group, a nitrogen-containing heterocyclic group which may be monosubstituted by a hydroxyl group, a $C_6$-$C_{10}$ aryl group which may be independently mono- to trisubstituted by a group selected from Substituent Group b and a $C_6$-$C_{10}$ aryloxy group which may be independently mono- to trisubstituted by a group selected from Substituent Group b, and Substituent Group b is the group consisting of a halogen atom, a $C_1$-$C_6$ alkyl group and a $C_1$-$C_6$ halogenated alkyl group;

(35) The urea derivative or the pharmacologically acceptable salt thereof according to (1), wherein $R^1$ is a phenyl group independently mono- to trisubstituted by a group selected from the group consisting of a halogen atom, a $C_1$-$C_6$ alkyl group, a $C_1$-$C_6$ halogenated alkyl group, a $C_1$-$C_6$ alkoxy group, a carboxyl group, an amino group, a mono-$C_2$-$C_7$ alkylcarbonylamino group, a di-($C_1$-$C_6$ alkyl)amino group, a cyano group and a nitro group or a thiazolyl group which may be independently mono- or disubstituted by a $C_1$-$C_6$ alkyl group, $R^2$ is a phenyl or pyridyl group which may be independently mono- to trisubstituted by a group selected from the group consisting of a halogen atom, a $C_1$-$C_6$ alkyl group, a $C_1$-$C_6$ halogenated alkyl group, a $C_1$-$C_6$ hydroxyalkyl group, a $C_1$-$C_6$ alkoxy group, a $C_1$-$C_6$ alkoxy group mono- or disubstituted by a hydroxyl group, a carboxyl group, an amino group, a mono-$C_2$-$C_7$ alkylcarbonylamino group, a cyano group, a nitro group, a hydroxyl group, a $C_1$-$C_6$ alkyl group monosubstituted by a carboxyl group, a $C_1$-$C_6$ alkyl group monosubstituted by a $C_2$-$C_7$ alkoxycarbonyl group, E is a group having the formula (IV)

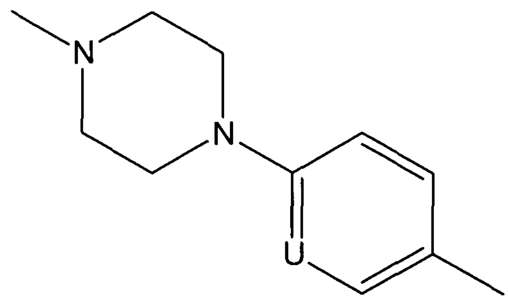

(IV)

or the formula (XL), U is a group represented by the formula CH or a nitrogen atom, and A is a group represented by the formula -O-C(=O)-, a group represented by the formula -NH-C(=O)- or a group represented by the formula -CH(OH)-C(=O)-;

(36) The urea derivative or the pharmacologically acceptable salt thereof according to (1), wherein $R^1$ is a phenyl group independently mono- to trisubstituted by a group selected from the group consisting of a halogen atom, a $C_1$-$C_6$ alkyl group and $C_1$-$C_6$ alkoxy group or a thiazolyl group independently mono- or disubstituted by a $C_1$-$C_6$ alkyl group, $R^2$ is a phenyl or pyridyl group which may be independently mono- to trisubstituted by a group selected from the group consisting of a halogen atom, a $C_1$-$C_6$ alkyl group, a $C_1$-$C_6$ halogenated alkyl group, a $C_1$-$C_6$ alkoxy group mono- or disubstituted by a hydroxyl group and a cyano group, E is a group having the formula (IV), U is a group represented by the formula CH or a nitrogen atom, and A is a group represented by the formula -NH-C(=O)- or a group represented by the formula -CH(OH)-C(=O)-;

(37) The urea derivative or the pharmacologically acceptable salt thereof according to (1), wherein $R^1$ is a phenyl group substituted by a group selected from the group consisting of a fluorine atom, a chlorine atom, a methyl group, a methoxy group and an ethoxy group at the 2- or 3-position; a phenyl group independently disubstituted by a group selected from the group consisting of a fluorine atom, a chlorine atom, a methyl group, a methoxy group and an ethoxy group at the 2, 3 or 5-position; or a 5-methyl-2-thiazolyl group, $R^2$ is a phenyl group substituted by a group selected from the group consisting of a fluorine atom, a chlorine atom, a methyl group and a trifluoromethyl group at the 2-position and substituted by a group selected from the group consisting of a 2-hydroxyethoxy group and a 2,3-dihydroxypropoxy group at the 4- or 5-position; a 3-methyl-2-pyridyl group; a 3-cyano-2-pyridyl group; or a 3,5-difluoro-2-pyridyl group, E is a group having the formula (IV), U is a group represented by the formula CH or a nitrogen atom, and A is a group represented by the formula -NH-C(=O)-;

(38) The urea derivative or the pharmacologically acceptable salt thereof according to (1), wherein $R^1$ is a 2-fluorophenyl group, a 2-ethoxyphenyl group, a 3-ethoxyphenyl group, a 5-fluoro-2-methoxyphenyl group, a 2-ethoxy-5-fluorophenyl group, a 5-chloro-2-methoxyphenyl group, a 5-chloro-2-ethoxyphenyl group, a 2-methoxy-5-methylphenyl group or a 3,5-dimethoxymethylphenyl group, $R^2$ is a 2-chloro-4-(2-hydroxyethoxy)phenyl group, a 2-chloro-5-(2-hydroxyethoxy)phenyl group, a 4-(2-hydroxyethoxy)-2-trifluoromethylphenyl group, a 2-chloro-4-(2,3-dihydroxypropoxy)phenyl group, a 4-(2,3-dihydroxypropoxy)-2-trifluoromethylphenyl group, a 2-chloro-5-(2,3-dihydroxypropoxy)phenyl group or a 3-methyl-2-pyridyl group, E is a group having the formula (IV), U is a group represented by the formula CH or a nitrogen atom and A is a group represented by the formula -NH-C(=O)-;

(39) The urea derivative or the pharmacologically acceptable salt thereof according to (1), wherein $R^1$ is a $C_1$-$C_6$ alkyl group; or a phenyl group which may be independently mono- to trisubstituted by a group selected from the group consisting of a halogen atom, a $C_1$-$C_6$ alkyl group, a $C_1$-$C_2$ halogenated alkyl group, a $C_1$-$C_3$ hydroxyalkyl group, a $C_1$-$C_6$ alkoxy group, a $C_1$-$C_2$ halogenated alkoxy group, a carboxyl group, an amino group, a mono-$C_2$-$C_3$ alkylcarbonylamino group, a di-$C_1$-$C_2$ alkylamino group, a cyano group, a nitro group and a phenyloxy group, $R^2$ is a $C_1$-$C_4$ alkyl group; or a phenyl or benzyl group which may be independently mono-to trisubstituted by a group selected from the group consisting of a halogen atom, a $C_1$-$C_6$ alkyl group, a $C_1$-$C_2$ halogenated alkyl group, a $C_1$-$C_3$ hydroxyalkyl group, a $C_1$-$C_6$ alkoxy group, a $C_1$-$C_2$ halogenated alkoxy group, a carboxyl group, an amino group, a mono-$C_2$-$C_3$ alkylcarbonylamino group, a di-$C_1$-$C_2$ alkylamino group, a cyano group, a nitro group and a phenyloxy group, E is a group having the formula (IV), U is a group represented by the formula CH or a nitrogen atom, and A is a single bond, a group represented by the formula -O-C(=O)-, a group represented by the formula -NH-C(=O)- or a carbonyl group;

(40) The urea derivative or the pharmacologically acceptable salt thereof according to (1), wherein $R^1$ is a $C_1$-$C_6$ alkyl group; or a phenyl group which may be independently mono- to trisubstituted by a group selected from the group consisting of a fluorine atom, a chlorine atom, a bromine atom, a methyl group, an ethyl group, a propyl group,

an isopropyl group, a butyl group, an isobutyl group, a s-butyl group, a t-butyl group, a trifluoromethyl group, a methoxy group, an ethoxy group, a propoxy group, an isopropoxy group, a butoxy group, a carboxyl group, an amino group, a methylcarbonylamino group, a dimethylamino group, a cyano group and a nitro group, $R^2$ is a $C_1$-$C_4$ alkyl group; or a phenyl group which may be independently mono- to trisubstituted by a group selected from the group consisting of a fluorine atom, a chlorine atom, a bromine atom, a methyl group, an ethyl group, a propyl group, an isopropyl group, a butyl group, an isobutyl group, a s-butyl group, a t-butyl group, a trifluoromethyl group, a hydroxymethyl group, a methoxy group, an ethoxy group, a propoxy group, an isopropoxy group, a butoxy group, a carboxyl group, an amino group, an acetamido group, a dimethylamino group, a cyano group and a nitro group, E is a group having the above formula (IV), U is a group represented by the formula CH or a nitrogen atom, and A is a group represented by the formula -NH-C(=O)-;

(41) The urea derivative or the pharmacologically acceptable salt thereof according to (1), wherein $R^1$ is a hexyl group; a phenyl group substituted by a fluorine atom, a chlorine atom, a methyl group, an ethyl group, a trifluoromethyl group, a methoxy group or an ethoxy group at the 2- and 5-positions; a phenyl group substituted by a butoxy group or a dimethylamino group at the 4-position; or a phenyl group substituted by a fluorine atom, a chlorine atom, a methyl group, an ethyl group, a methoxy group or an ethoxy group at the 2-position, $R^2$ is a t-butyl group; a phenyl group substituted by a fluorine atom, a chlorine atom, a bromine atom, a methyl group, a trifluoromethyl group, a hydroxymethyl group, a carboxyl group, an amino group or a nitro group at the 2- and 6-positions; a phenyl group substituted by a fluorine atom, a chlorine atom, a bromine atom, a methyl group, an ethyl group, a propyl group, an isopropyl group, a butyl group, a trifluoromethyl group, a methoxy group, a carboxyl group, an amino group, an acetamido group or a cyano group at the 2- and 4-positions; or a phenyl group substituted by a chlorine atom, a bromine atom, a methyl group or a trifluoromethyl group at the 2-position, E is a group having the above formula (IV), U is a group represented by the formula CH or a nitrogen atom, and A is a group represented by the formula -NH-C(=O)-;

(42) The urea derivative or the pharmacologically acceptable salt thereof according to (1), wherein $R^1$ is a 5-fluoro-2-methoxyphenyl group, a 2-ethoxy-5-fluorophenyl group, a 5-chloro-2-methoxyphenyl group, a 5-chloro-2-ethoxyphenyl group, a 2-methoxy-5-methylphenyl group, a 2-ethoxy-5-methylphenyl group, a 4-butoxyphenyl group, a 4-dimethylaminophenyl group, a 2-fluorophenyl group or a 2-ethoxyphenyl group, $R^2$ is a 2,6-difluorophenyl group, a 2-fluoro-6-trifluoromethylphenyl group, a 2,6-dichlorophenyl group, a 2-chloro-6-methylphenyl group, a 2,6-dimethylphenyl group, a 2-hydroxymethyl-6-methylphenyl group, a 2-methoxy-6-methylphenyl group, a 2-carboxy-6-methylphenyl group, a 4-fluoro-2-trifluoromethylphenyl group, a 2-chloro-4-methylphenyl group, 4-carboxy-2-chlorophenyl group, a 4-amino-2-chlorophenyl group, a 4-acetamido-2-chlorophenyl group, a 4-methoxy-2-methylphenyl group, a 4-carboxy-2-methylphenyl group, a 4-amino-2-trifluoromethylphenyl group, a 4-acetamido-2-trifluoromethylphenyl group, a 2-methylphenyl group or a 2-trifluoromethylphenyl group, E is a group having the above formula (IV), U is a group represented by the formula CH or a nitrogen atom, and A is a group represented by the formula -NH-C(=O)-;

(43) The urea derivative or the pharmacologically acceptable salt thereof according to (1), which is represented by
4-{4-[3-(2-ethoxy-phenyl)-ureido]-phenyl}-piperazine-1-carboxylic acid (3-methyl-pyridin-2-yl)-amide,
4-{4-[3-(2-methoxy-5-methyl-phenyl)-ureido]-phenyl}-piperazine-1-carboxylic acid (3-methyl-pyridin-2-yl)-amide,
4-{4-[3-(3,5-dimethoxy-phenyl)-ureido]-phenyl}-piperazine-1-carboxylic acid (3-methyl-pyridin-2-yl)-amide,
4- { S-[3-(S-fluoro-2-methoxy-phenyl)-ureido]-pyridin-2-yl } -piperazine-1-carboxylic acid (3-methyl-pyridin-2-yl)-amide,
4- {5-[3-(2-ethoxy-5-fluoro-phenyl)-ureido]-pyridin-2-yl} -piperazine-1-carboxylic acid (3-methyl-pyridin-2-yl)-amide,
4- { 5-[3-(5-chloro-2-methoxy-phenyl)-ureido]-pyridin-2-yl} -piperazine-1-carboxylic acid (3-methyl-pyridin-2-yl)-amide,
4-{5-[3-(5-chloro-2-ethoxy-phenyl)-ureido]-pyridin-2-yl}-piperazine-1-carboxylic acid (3-methyl-pyridin-2-yl)-amide,
4-{4-[3-(2-methoxy-5-methyl-phenyl)-ureido]-phenyl}-piperazine-1-carboxylic acid [2-chloro-4-(2-hydroxy-ethoxy)-phenyl]-amide,
4- {4-[3-(3,5-dimethoxy-phenyl)-ureido]-phenyl}-piperazine-l-carboxylic acid [2-chloro-4-(2-hydroxy-ethoxy)-phenyl]-amide,
4- {4-[3-(2-methoxy-5-methyl-phenyl)-ureido]-phenyl} -piperazine-1-carboxylic acid [2-chloro-4-(2,3-dihydroxy-propoxy)-phenyl]-amide,
4-{4-[3-(2-methoxy-5-methyl-phenyl)-ureido]-phenyl}-piperazine-1-carboxylic acid [4-(2-hydroxy-ethoxy)-2-trifluoromethyl-phenyl]-amide,
4-{4-[3-(5-methyl-thiazol-2-yl)-ureido]-phenyl}-piperazine-1-carboxylic acid [4-(2-hydroxy-ethoxy)-2-trifluoromethyl-phenyl]-amide,
4-{4-[3-(2-methoxy-5-methyl-phenyl)-ureido]-phenyl}-piperazine-1-carboxylic acid [4-(2,3-dihydroxy-propoxy)-2-trifluoromethyl-phenyl]-amide,
4-{4-[3-(2-methoxy-5-methyl-phenyl)-ureido]-phenyl}-piperazine-1-carboxylic acid [2-chloro-5-(2-hydroxy-ethoxy)-phenyl]-amide,
4-{4-[3-(2-methoxy-5-methyl-phenyl)-ureido]-phenyl}-piperazine-1-carboxylic acid [4-(2-hydroxy-ethoxy)-2-methyl-phenyl]-amide,
4-{4-[3-(2-methoxy-5-methyl-phenyl)-ureido]-phenyl}-piperazine-1-carboxylic acid [2-fluoro-4-(2-hydroxy-ethoxy)-phenyl]-amide,
4-{4-[3-(2-methoxy-5-methyl-phenyl)-ureido]-phenyl}-piperazine-1-carboxylic acid (3,5-difluoro-pyridin-2-yl)-amide,
4-{4-[3-(3-ethoxy-phenyl)-ureido]-phenyl}-piperazine-1-carboxylic acid [4-(2-hydroxy-ethoxy)-2-trifluoromethyl-phenyl]-amide,
4-{4-[3-(3-ethoxy-phenyl)-ureido]-phenyl}-piperazine-1-carboxylic acid (3-methyl-pyridin-2-yl)-amide,
4-{4-[3-(2-ethoxy-phenyl)-ureido]-phenyl}-piperazine-1-carboxylic acid (3,5-difluoro-pyridin-2-yl)-amide,
4-{4-[3-(2-methoxy-3-methyl-phenyl)-ureido]-phenyl}-piperazine-1-carboxylic acid (3-methyl-pyridin-2-yl)-amide,
4-{2-chloro-4-[3-(2-fluoro-phenyl)-ureido]-phenyl}-piperazine-1-carboxylic acid [4-(2-hydroxy-ethoxy)-2-trifluoromethyl-phenyl]-amide,
4- {4-[3-(2-methoxy-phenyl)-ureido]-phenyl} -3,6-dihydro-2H-pyridine-1-carboxylic acid (3-methyl-pyridin-2-yl)-amide,
4- {4-[3-(2-methoxy-5-methyl-phenyl)-ureido]-phenyl} -3,6-dihydro-2H-pyridine-1-carboxylic acid (3-methyl-pyridin-2-yl)-amide,
4-{4-[3-(3,5-dimethoxy-phenyl)-ureido]-phenyl}-3,6-dihydro-2H-pyridine-1-carboxylic acid (3-methyl-pyridin-2-yl)-amide, or
4- {4-[3-(2-methoxy-5-methyl-phenyl)-ureido]-phenyl} -3,6-dihydro-2H-pyridine-1-carboxylic acid (3,5-difluoro-pyridin-2-yl)-amide;
(44) The urea derivative or the pharmacologically acceptable salt thereof according to (1), which is represented by
4-{4-[3-(2-ethoxy-phenyl)-ureido]-phenyl}-piperazine-1-carboxylic acid (3-methyl-pyridin-2-yl)-amide,
4-{4-[3-(2-methoxy-5-methyl-phenyl)-ureido]-phenyl}-piperazine-1-carboxylic acid (3-methyl-pyridin-2-yl)-amide,
4-{4-[3-(3,5-dimethoxy-phenyl)-ureido]-phenyl}-piperazine-1-carboxylic acid (3-methyl-pyridin-2-yl)-amide,
4-{5-[3-(2-ethoxy-5-fluoro-phenyl)-ureido]-pyridin-2-yl}-piperazine-1-carboxylic acid (3-methyl-pyridin-2-yl)-amide,
4-{5-[3-(5-chloro-2-methoxy-phenyl)-ureido]-pyridin-2-yl}-piperazine-1-carboxylic acid (3-methyl-pyridin-2-yl)-amide,
4-{4-[3-(2-methoxy-5-methyl-phenyl)-ureido]-phenyl}-piperazine-1-carboxylic acid [2-chloro-4-(2-hydroxy-ethoxy)-phenyl]-amide,
4- {4-[3-(2-methoxy-5-methyl-phenyl)-ureido]-phenyl} -piperazine- l -carboxylic acid [2-chloro-4-(2,3-dihydroxy-propoxy)-phenyl]-amide,
4-{4-[3-(2-methoxy-5-methyl-phenyl)-ureido]-phenyl}-piperazine-1-carboxylic acid [4-(2-hydroxy-ethoxy)-2-trifluoromethyl-phenyl]-amide, 4-{4-[3-(2-methoxy-5-methyl-phenyl)-ureido]-phenyl}-piperazine-1-carboxylic acid [2-chloro-5-(2-hydroxy-ethoxy)-phenyl]-amide,
4-{4-[3-(3-ethoxy-phenyl)-ureido]-phenyl}-piperazine-1-carboxylic acid (3-methyl-pyridin-2-yl)-amide,
4-{2-chloro-4-[3-(2-fluoro-phenyl)-ureido]-phenyl}-piperazine-1-carboxylic acid [4-(2-hydroxy-ethoxy)-2-trifluoromethyl-phenyl]-amide, or
4-{4-[3-(2-methoxy-5-methyl-phenyl)-ureido]-phenyl}-3,6-dihydro-2H-pyridine-1-carboxylic acid (3-methyl-pyridin-2-yl)-amide;
(45) The urea derivative or the pharmacologically acceptable salt thereof according to (1), which is represented by
4-{4-[3-(5-chloro-2-methoxy-phenyl)-ureido]-phenyl}-piperazine-1-carboxylic acid (2,6-dichloro-phenyl)-amide,
4-{4-[3-(5-chloro-2-ethoxy-phenyl)-ureido]-phenyl}-piperazine-1-carboxylic acid (2,6-dichloro-phenyl)-amide,
4-{4-[3-(2-ethoxy-5-methyl-phenyl)-ureido]-phenyl}-piperazine-1-carboxylic acid (2,6-dichloro-phenyl)-amide,
4-{4-[3-(5-chloro-2-ethoxy-phenyl)-ureido]-phenyl}-piperazine-1-carboxylic acid (2-chloro-6-methyl-phenyl)-amide,
4-{4-[3-(2-methoxy-5-methyl-phenyl)-ureido]-phenyl}-piperazine-1-carboxylic acid (2-hydroxymethyl-6-methyl-phenyl)-amide,
4-{5-[3-(2-methoxy-5-methyl-phenyl)-ureido]-pyridin-2-yl}-piperazine-1-carboxylic acid (2-hydroxymethyl-6-methyl-phenyl)-amide,
4-{4-[3-(2-ethoxy-5-methyl-phenyl)-ureido]-phenyl}-piperazine-1-carboxylic acid (2-methoxy-6-methyl-phenyl)-amide,
4-{5-[3-(2-ethoxy-5-methyl-phenyl)-ureido]-pyridin-2-yl}-piperazine-1-carboxylic acid (2-methoxy-6-methyl-phenyl)-amide, 4-{5-[3-(2-ethoxy-phenyl)-ureido]-pyridin-2-yl}-piperazine-1-carboxylic acid (2-methoxy-6-methyl-phenyl)-amide,
3-chloro-4-[(4-{4-[3-(2-methoxy-5-methyl-phenyl)-ureido]-phenyl}-piperazine-1-carbonyl)-amino]-benzoic acid,
3-chloro-4-[(4-{5-[3-(2-methoxy-5-methyl-phenyl)-ureido]-pyridin-2-yl}-piperazine-1-carbonyl)-amino]-benzoic acid,
4-{4-[3-(5-fluoro-2-methoxy-phenyl)-ureido]-phenyl}-piperazine-1-carboxylic acid (4-methoxy-2-methyl-phenyl)-amide,
4-{4-[3-(2-ethoxy-5-fluoro-phenyl)-ureido]-phenyl}-piperazine-1-carboxylic acid (4-methoxy-2-methyl-phenyl)-amide,
4-{4-[3-(5-chloro-2-ethoxy-phenyl)-ureido]-phenyl}-piperazine-1-carboxylic acid (4-methoxy-2-methyl-phenyl)-amide,
4-{4-[3-(2-ethoxy-phenyl)-ureido]-phenyl}-piperazine-l-carboxylic acid (4-methoxy-2-methyl-phenyl)-amide,
4-[(4-{4-[3-(2-methoxy-5-methyl-phenyl)-ureido]-phenyl}-piperazine-1-carbonyl)-amino]-3-methyl-benzoic acid,
4-[(4-{5-[3-(2-methoxy-5-methyl-phenyl)-ureido]-pyridin-2-yl}-piperazine-1-carbonyl)-amino]-3-methyl-benzoic acid,
4-{4-[3-(2-fluoro-phenyl)-ureido]-phenyl}-piperazine-1-carboxylic acid (2-chloro-6-methyl-phenyl)-amide,
4-{4-[3-(4-dimethylamino-phenyl)-ureido]-phenyl}-piperazine-1-carboxylic acid (2-chloro-6-methyl-phenyl)-amide,
4-{4-[3-(2-methoxy-5-methyl-phenyl)-ureido]-phenyl}-piperazine-1-carboxylic acid (4-amino-2-trifluoromethyl-phenyl)-amide, or
4- {4-[3-(2-methoxy-5-methyl-phenyl)-ureido]-phenyl}-piperazine-1-carboxylic acid (4-amino-2-chloro-phenyl)-amide;
(46) The urea derivative or the pharmacologically acceptable salt thereof according to (1), which is represented by
4-{4-[3-(5-chloro-2-methoxy-phenyl)-ureido]-phenyl}-piperazine-1-carboxylic acid (2,6-dichloro-phenyl)-amide,
4-{4-[3-(5-chloro-2-ethoxy-phenyl)-ureido]-phenyl}-piperazine-1-carboxylic acid (2,6-dichloro-phenyl)-amide,
4-{4-[3-(5-chloro-2-ethoxy-phenyl)-ureido]-phenyl}-piperazine-1-carboxylic acid (2-chloro-6-methyl-phenyl)-amide,
4-{4-[3-(2-ethoxy-5-methyl-phenyl)-ureido]-phenyl}-piperazine-1-carboxylic acid (2-methoxy-6-methyl-phenyl)-amide,
3-chloro-4-[(4-{4-[3-(2-methoxy-5-methyl-phenyl)-ureido]-phenyl}-piperazine-1-carbonyl)-amino]-benzoic acid,
4-{4-[3-(5-fluoro-2-methoxy-phenyl)-ureido]-phenyl}-piperazine-1-carboxylic acid (4-methoxy-2-methyl-phenyl)-amide,
4-[(4- {4-[3-(2-methoxy-5-methyl-phenyl)-ureido]-phenyl} -piperazine-1-carbonyl)-amino]-3-methyl-benzoic acid,
4-{4-[3-(2-fluoro-phenyl)-ureido]-phenyl}-piperazine-1-carboxylic acid (2-chloro-6-methyl-phenyl)-amide,
4-{4-[3-(2-methoxy-5-methyl-phenyl)-ureido]-phenyl}-piperazine-1-carboxylic acid (4-amino-2-trifluoromethyl-phenyl)-amide, or
4-{4-[3-(2-methoxy-5-methyl-phenyl)-ureido]-phenyl}-piperazine-1-carboxylic acid (4-amino-2-chloro-phenyl)-amide;
(47) An acyl-coenzyme A: diacylglycerol acyltransferase inhibitor comprising the urea derivative or the pharmacologically acceptable salt thereof according to any one of (1) to (46) as an active ingredient;
(48) A pharmaceutical composition comprising the urea derivative or the pharmacologically acceptable salt thereof according to any one of (1) to (46) as an active ingredient;
(49) The pharmaceutical composition according to (48), which has an acyl-coenzyme A: diacylglycerol acyltransferase inhibitory effect;
(50) The pharmaceutical composition according to (49), which is for treatment and/or prevention of adiposity, obesity, hyperlipidemia, hypertriglyceridemia, lipidosis, insulin resistance syndrome, impaired glucose tolerance, diabetes, diabetic complications (including diabetic peripheral neuropathy, diabetic nephropathy, diabetic retinopathy and diabetic macroangiopathy), cataract, gestational diabetes mellitus, polycystic ovary syndrome, arteriosclerosis (including arteriosclerosis caused by the diseases described above and below), atherosclerosis or diabetic atherosclerosis;
(51) The pharmaceutical composition according to (49), which is for treatment and/or prevention of the following diseases caused by adiposity: hyperlipidemia, hypertriglyceridemia, lipidosis, insulin resistance syndrome, impaired glucose tolerance, diabetes, diabetic complications (including diabetic peripheral neuropathy, diabetic nephropathy, diabetic retinopathy and diabetic macroangiopathy), cataract, gestational diabetes mellitus, polycystic ovary syndrome, arteriosclerosis (including arteriosclerosis caused by the diseases described above and below), atherosclerosis, diabetic atherosclerosis, hypertension, cerebrovascular disorders, coronary artery diseases, fatty liver, respiratory abnormality, lower back pain, knee osteoarthritis, gout or cholecystitis;
(52) The pharmaceutical composition according to (49), which is for treatment and/or prevention of adiposity, obesity or hyperlipidemia, hypertriglyceridemia, diabetes, hypertension or arteriosclerosis caused by adiposity;
(53) The pharmaceutical composition according to (49), which is for treatment and/or prevention of adiposity or obesity;
(54) The pharmaceutical composition according to (49), which is for inhibiting absorption of fat from the small intestine;
(55) Use of the urea derivative or the pharmacologically acceptable salt thereof according to any one of (1) to (46) for producing a pharmaceutical composition;
(56) The use according to (55), wherein the pharmaceutical composition inhibits acyl-coenzyme A: diacylglycerol acyltransferase;

(57) The use according to (55), wherein the pharmaceutical composition is a composition for treatment and/or prevention of adiposity, obesity, hyperlipidemia, hypertriglyceridemia, lipidosis, insulin resistance syndrome, impaired glucose tolerance, diabetes, diabetic complications (including diabetic peripheral neuropathy, diabetic nephropathy, diabetic retinopathy and diabetic macroangiopathy), cataract, gestational diabetes mellitus, polycystic ovary syndrome, arteriosclerosis (including arteriosclerosis caused by the diseases described above and below), atherosclerosis or diabetic atherosclerosis;

(58) The use according to (55), wherein the pharmaceutical composition is a composition for treatment and/or prevention of the following diseases caused by adiposity: hyperlipidemia, hypertriglyceridemia, lipidosis, insulin resistance syndrome, impaired glucose tolerance, diabetes, diabetic complications (including diabetic peripheral neuropathy, diabetic nephropathy, diabetic retinopathy and diabetic macroangiopathy), cataract, gestational diabetes mellitus, polycystic ovary syndrome, arteriosclerosis (including arteriosclerosis caused by the diseases described above and below), atherosclerosis, diabetic atherosclerosis, hypertension, cerebrovascular disorders, coronary artery diseases, fatty liver, respiratory abnormality, lower back pain, knee osteoarthritis, gout or cholecystitis;

(59) The use according to (55), wherein the pharmaceutical composition is a composition for treatment and/or prevention of adiposity, obesity or hyperlipidemia, hypertriglyceridemia, diabetes, hypertension or arteriosclerosis caused by adiposity;

(60) The use according to (55), wherein the pharmaceutical composition is a composition for treatment and/or prevention of adiposity or obesity;

(61) The use according to (55), wherein the pharmaceutical composition is a composition for inhibiting absorption of fat from the small intestine;

(62) A method for inhibiting acyl-coenzyme A, which comprises administering a pharmacologically effective amount of the urea derivative or the pharmacologically acceptable salt thereof according to any one of (1) to (46) to a warm-blooded animal;

(63) A method for treating and/or preventing a disease, which comprises administering a pharmacologically effective amount of the urea derivative or the pharmacologically acceptable salt thereof according to any one of (1) to (46) to a warm-blooded animal;

(64) The method according to (63), wherein the disease includes adiposity, obesity, hyperlipidemia, hypertriglyceridemia, lipidosis, insulin resistance syndrome, impaired glucose tolerance, diabetes, diabetic complications (including diabetic peripheral neuropathy, diabetic nephropathy, diabetic retinopathy and diabetic macroangiopathy), cataract, gestational diabetes mellitus, polycystic ovary syndrome, arteriosclerosis (including arteriosclerosis caused by the diseases described above and below), atherosclerosis or diabetic atherosclerosis;

(65) The method according to (63), wherein the disease includes the following diseases caused by adiposity: hyperlipidemia, hypertriglyceridemia, lipidosis, insulin resistance syndrome, impaired glucose tolerance, diabetes, diabetic complications (including diabetic peripheral neuropathy, diabetic nephropathy, diabetic retinopathy and diabetic macroangiopathy), cataract, gestational diabetes mellitus, polycystic ovary syndrome, arteriosclerosis (including arteriosclerosis caused by the diseases described above and below), atherosclerosis, diabetic atherosclerosis, hypertension, cerebrovascular disorders, coronary artery diseases, fatty liver, respiratory abnormality, lower back pain, knee osteoarthritis, gout or cholecystitis;

(66) The method according to (63), wherein the disease includes adiposity, obesity or hyperlipidemia, hypertriglyceridemia, diabetes, hypertension or arteriosclerosis caused by adiposity;

(67) The method according to (63), wherein the disease includes adiposity or obesity;

(68) A method for inhibiting absorption of fat from the small intestine, which comprises administering a pharmacologically effective amount of the urea derivative or the pharmacologically acceptable salt thereof according to any one of (1) to (46) to a warm-blooded animal; and

(69) The method according to any one of (62) to (68), wherein the warm-blooded animal is a human being.

In the present invention, the "$C_1$-$C_{10}$ alkyl group" includes a linear or branched alkyl group having 1 to 10 carbon atom(s) such as, for example, a methyl, ethyl, propyl, isopropyl, butyl, isobutyl, s-butyl, t-butyl, pentyl, isopentyl, 2-methylbutyl, neopentyl, 1-ethylpropyl, hexyl, isohexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 1-ethylbutyl, 2-ethylbutyl, heptyl, octyl, nonyl or decyl group, preferably, a linear or branched alkyl group having 1 to 6 carbon atom(s) ($C_1$-$C_6$ alkyl group). In $R^1$, it is more preferably a hexyl group and in Substituent Group a, it is more preferably a linear or branched alkyl group having 1 to 4 carbon atom(s) ($C_1$-$C_4$ alkyl group), further more preferably a methyl group or an ethyl group ($C_1$-$C_2$ alkyl group), particularly preferably a methyl group.

In the present invention, the "$C_3$-$C_6$ cycloalkyl group" includes a cyclopropyl, cyclobutyl, cyclopentyl or cyclohexyl group, preferably a cyclohexyl group.

In the present invention, the "$C_1$-$C_6$ alkyl group" includes a linear or branched alkyl group having 1 to 6 carbon atom(s) such as, for example, a methyl, ethyl, propyl, isopropyl, butyl, isobutyl, s-butyl, t-butyl, pentyl, isopentyl, 2-methylbutyl, neopentyl, 1-ethylpropyl, hexyl, isohexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 1-ethylbutyl or 2-ethylbutyl group, preferably a linear or branched alkyl group having 1 to 4 carbon atom(s) ($C_1$-$C_4$ alkyl group). In $R^2$, it is more preferably a t-butyl group and in $R^3$, $R^4$, $R^5$ or Substituent Group b, it is more preferably a linear or branched alkyl group having 1 to 4 carbon atom(s) ($C_1$-$C_4$ alkyl group), further more preferably a methyl group or an ethyl group ($C_1$-$C_2$ alkyl group), particularly preferably a methyl group.

In the present invention, the "$C_3$-$C_6$ cycloalkyl group which may be monosubstituted by a $C_1$-$C_6$ alkyl group" means the above "$C_3$-$C_6$ cycloalkyl group" which may be substituted by one "$C_1$-$C_6$ alkyl group" described above and includes, for example, a cyclopropyl, cyclobutyl, cyclopentyl, cyclohexyl, 2-methylcyclopropyl, 2-methylcyclobutyl, 2-methylcyclopentyl, 2-methylcyclohexyl, 4-methylcyclohexyl or 2-ethylcyclohexyl group, preferably 2-methylcyclohexyl group.

In the present invention, the "$C_7$-$C_{10}$ bicycloalkyl group" includes an alkyl group having 7 to 10 carbon atoms and composed only of two rings which share two or more atoms such as, for example, a bicyclo[2.2.1]heptyl, bicyclo [2.2.2]octyl, bicyclo[3.2.1]octyl or bicyclo[3.3.2]decyl group, preferably a 2-bicyclo[2.2.1]heptyl group.

In the present invention, the "tetralyl group" includes a 5,6,7,8-tetrahydronaphthyl group, preferably a 5-tetralyl group.

In the present invention, the "halogen atom" includes a fluorine atom, a chlorine atom, a bromine atom or an iodine atom, preferably a fluorine atom, a chlorine atom or a bromine atom, more preferably a fluorine atom or a chlorine atom.

In the present invention, the "$C_1$-$C_6$ halogenated alkyl group" means a group in which the same or different 1 to 5 "halogen atom(s)" described above are bonded to the above "$C_1$-$C_6$ alkyl group" and includes, for example, a trifluoromethyl, trichloromethyl, difluoromethyl, dichloromethyl, dibromomethyl, fluoromethyl, 2,2,2-trifluoroethyl, 2,2,2-trichloroethyl, 2-bromoethyl, 2-chloroethyl, 2-fluoroethyl, 2-iodoethyl, pentafluoroethyl, 3-chloropropyl, 4-fluorobutyl, 6-iodohexyl or 2,2-dibromoethyl group, preferably a group in which the same or different 1 to 5 "halogen atom(s)" described above are bonded to the above "$C_1$-$C_4$ alkyl group" ($C_1$-$C_4$ halogenated alkyl group), more preferably a group in which the same or different 1 to 5 "halogen atom(s)" described above are bonded to the above "$C_1$-$C_2$ alkyl group" ($C_1$-$C_2$ halogenated alkyl group), further more preferably a trifluoromethyl group.

In the present invention, the "$C_1$-$C_6$ hydroxyalkyl group" means a group in which a hydroxyl group is bonded to the above "$C_1$-$C_6$ alkyl group" and includes, for example, a hydroxymethyl, hydroxyethyl or hydroxypropyl group, preferably a group in which a hydroxyl group is bonded to a linear or branched alkyl group having 1 to 3 carbon atom (s) ($C_1$-$C_3$ hydroxyalkyl group), more preferably a hydroxymethyl, 2-hydroxyethyl or 3-hydroxypropyl group, further more preferably a 2-hydroxylethyl group.

In the present invention, "-P(=O)(O-$C_1$-$C_6$ alkyl)$_2$" means a group in which two oxygen atoms each bonded to one "$C_1$-$C_6$ alkyl group" described above are bonded to a phosphoryl group and is preferably phosphonic acid diethyl ester or phosphonic acid dimethyl ester, more preferably phosphonic acid diethyl ester.

In the present invention, the "$C_1$-$C_6$ alkyl group monosubstituted by - P(=O)(O-$C_1$-$C_6$ alkyl)$_2$" means a group in which one "-P(=O)(O-$C_1$-$C_6$ alkyl)$_2$" described above is bonded to the above "$C_1$-$C_6$ alkyl group" and is preferably phosphonic acid diethyl ester methyl or phosphonic acid diethyl ester ethyl, more preferably phosphonic acid diethyl ester methyl.

In the present invention, the "$C_1$-$C_{10}$ alkoxy group" means a group in which the above "$C_1$-$C_{10}$ alkyl group" is bonded to an oxygen atom and includes a linear or branched alkoxy group having 1 to 10 carbon atom(s) such as, for example, a methoxy, ethoxy, propoxy, isopropoxy, butoxy, isobutoxy, s-butoxy, t-butoxy, pentoxy, isopentoxy, 2-methylbutoxy, 1-ethylpropoxy, 2-ethylpropoxy, neopentoxy, hexyloxy, 4-methylpentoxy, 3-methylpentoxy, 2-methylpentoxy, 3,3-dimethylbutoxy, 2,2-dimethylbutoxy, 1,1-dimethylbutoxy, 1,2-dimethylbutoxy, 1,3-dimethylbutoxy, 2,3-dimethylbutoxy, heptyloxy, octyloxy, nonyloxy or decyloxy group, preferably a linear or branched alkoxy group having 1 to 6 carbon atom(s) ($C_1$-$C_6$ alkoxy group), more preferably a linear or branched alkoxy group having 1 to 4 carbon atom(s) ($C_1$-$C_4$ alkoxy group), further more preferably a methoxy group or an ethoxy group ($C_1$-$C_2$ alkoxy group), particularly preferably a methoxy group.

In the present invention, the "$C_1$-$C_6$ halogenated alkoxy group" means a group in which the same or different 1 to 5 "halogen atom(s)" described above are bonded to the above "$C_1$-$C_6$ alkoxy group" and includes, for example, a trifluoromethoxy, trichloromethoxy, difluoromethoxy, dichloromethoxy, dibromomethoxy, fluoromethoxy, 2,2,2-trifluoroethoxy, 2,2,2-trichloroethoxy, 2-bromoethoxy, 2-chloroethoxy, 2-fluoroethoxy, 2-iodoethoxy, pentafluoroethoxy, 3-chloropropoxy, 4-fluorobutoxy, 6-iodoheptyloxy or 2,2-dibromoethoxy group, preferably a group in which the same or different 1 to 5 "halogen atom(s)" described above are bonded to the above "$C_1$-$C_4$ alkoxy group" ($C_1$-$C_4$ halogenated alkoxy group), more preferably a group in which the same or different 1 to 5 the same or different "halogen atom(s)" described above are bonded to the above "$C_1$-$C_2$ alkoxy group" ($C_1$-$C_2$ halogenated alkoxy group), further more preferably a trifluoromethoxy group.

In the present invention, the "$C_1$-$C_6$ alkoxy group mono- or disubstituted by a hydroxyl group" means a group

in which 1 or 2 hydroxyl group(s) are bonded to the above "$C_1$-$C_6$ alkoxy group" and includes, for example, a hydroxyethoxy, hydroxypropoxy, dihydroxypropoxy, hydroxybutoxy or dihydroxybutoxy group, preferably a group in which 1 or 2 hydroxyl group(s) are bonded to a linear or branched alkoxy group having 2 to 4 carbon atoms ($C_2$-$C_4$ hydroxyalkoxy group mono- or disubstituted by a hydroxyl group), more preferably a 2-hydroxyethoxy or 2,3-dihydroxypropoxy group.

In the present invention, the "$C_1$-$C_6$ alkoxy group monosubstituted by a $C_3$-$C_6$ cycloalkyl group" means a group in which one "$C_3$-$C_6$ cycloalkyl group" described above is bonded to the above "$C_1$-$C_6$ alkoxy group" and includes, for example, a cyclopropylmethyloxy, cyclobutylmethyloxy, cyclopentylmethyloxy, cyclohexylmethyloxy, 2-cyclopropylethoxy, 2-cyclobutylethoxy, 1-cyclopropylethoxy, 1-cyclobutylethoxy, 3-cyclopropylpropoxy or 3-cyclobutylpropoxy group, preferably a group in which one "$C_3$-$C_6$ cycloalkyl group" described above is bonded to the above "$C_1$-$C_4$ alkoxy group", more preferably a group in which one "$C_3$-$C_6$ cycloalkyl group" described above is bonded to the above "$C_1$-$C_2$ alkoxy group", further more preferably a cyclopropylmethyloxy group.

In the present invention, the "$C_2$-$C_6$ alkenyloxy group" means, of the above "$C_1$-$C_6$ alkoxy groups", those having 2 to 6 carbon atoms and one double bond and includes, for example, an 1-ethenyloxy, 2-propenyloxy, 1-propenyloxy, 3-butenyloxy, 2-butenyloxy, 1-butenyloxy or 5-hexenyloxy group, preferably a $C_2$-$C_4$ alkenyloxy group, more preferably a 2-propenyloxy group.

In the present invention, the "$C_2$-$C_6$ alkynyloxy group" means, of the above "$C_1$-$C_6$ alkoxy groups", those having 2 to 6 carbon atoms and one triple bond and includes, for example, an 1-ethynyloxy, 2-propynyloxy, 1-propynyloxy, 3-butynyloxy, 2-butynyloxy, 1-butynyloxy or 5-hexynyloxy group, preferably a $C_2$-$C_4$ alkynyloxy group, more preferably a 2-propynyloxy group.

In the present invention, the "($C_1$-$C_6$ alkoxy)-($C_1$-$C_6$ alkyl) group" means a group in which one "$C_1$-$C_6$ alkoxy group" described above is bonded to the above "$C_1$-$C_6$ alkyl group" and includes, for example, a methoxymethyl, ethoxymethyl, propoxymethyl, isopropoxymethyl, butoxymethyl, isobutoxymethyl, s-butoxymethyl, t-butoxymethyl, 2-methoxyethyl, 2-ethoxyethyl, 2-propoxyethyl, 2-isopropoxyethyl, 2-butoxyethyl, 2-isobutoxyethyl, 2-(s-butoxy)ethyl, 2-(t-butoxy)ethyl, 1-methoxyethyl, 1-ethoxyethyl, 1-propoxyethyl, 1-isopropoxyethyl, 1-butoxyethyl, 1-isobutoxyethyl, 1-(s-butoxy)ethyl, 1-(t-butoxy)ethyl or 3-isopropoxypropyl group, preferably a group in which one "$C_1$-$C_4$ alkoxy group" described above is bonded to the above "$C_1$-$C_4$ alkyl group" (($C_1$-$C_4$ alkoxy)-($C_1$-$C_4$ alkyl) group), more preferably a group in which one "$C_1$-$C_2$ alkoxy group" described above is bonded to the above "$C_1$-$C_2$ alkyl group" (($C_1$-$C_2$ alkoxy)-($C_1$-$C_2$ alkyl) group), further more preferably a methoxymethyl group.

In the present invention, the "$C_1$-$C_6$ alkylthio group" means a group in which one "$C_1$-$C_6$ alkyl group" described above is bonded to a sulfur atom and includes a linear or branched alkylthio group having 1 to 6 carbon atom(s) such as, for example, a methylthio, ethylthio, propylthio, isopropylthio, butylthio, isobutylthio, s-butylthio, t-butylthio, pentylthio, isopentylthio, 2-methylbutylthio, neopentylthio, 1-ethylpropylthio, hexylthio, isohexylthio, 4-methylpentylthio, 3-methylpentylthio, 2-methylpentylthio, 1-methylpentylthio, 3,3-dimethylbutylthio, 2,2-dimethylbutylthio, 1,1-dimethylbutylthio, 1,2-dimethylbutylthio, 1,3-dimethylbutylthio, 2,3-dimethylbutylthio, 1-ethylbutylthio or 2-ethylbutylthio group, preferably a linear or branched alkylthio group having 1 to 4 carbon atom(s) ($C_1$-$C_4$ alkylthio group), more preferably a methylthio group or an ethylthio group ($C_1$-$C_2$ alkylthio group), further more preferably a methylthio group.

In the present invention, the "$C_2$-$C_7$ alkoxycarbonyl group" means a group in which the above "$C_1$-$C_6$ alkoxy group" is bonded to a carbonyl group and includes, for example, a methoxycarbonyl, ethoxycarbonyl, propoxycarbonyl, isopropoxycarbonyl, butoxycarbonyl, isobutoxycarbonyl, s-butoxycarbonyl, t-butoxycarbonyl, pentoxycarbonyl, isopentoxycarbonyl, 2-methylbutoxycarbonyl, neopentoxycarbonyl, hexyloxycarbonyl, 4-methylpentoxycarbonyl, 3-methylpentoxycarbonyl, 2-methylpentoxycarbonyl, 3,3-dimethylbutoxycarbonyl, 2,2-dimethylbutoxycarbonyl, 1,1-dimethylbutoxycarbonyl, 1,2-dimethylbutoxycarbonyl, 1,3-dimethylbutoxycarbonyl or 2,3-dimethylbutoxy carbonyl group, preferably a group in which the above "$C_1$-$C_4$ alkoxy group" is bonded to a carbonyl group ($C_2$-$C_5$ alkoxycarbonyl group), more preferably a methoxycarbonyl or ethoxycarbonyl group ($C_2$-$C_3$ alkoxycarbonyl group), further more preferably a methoxycarbonyl group.

In the present invention, the "mono-$C_2$-$C_7$ alkylcarbonylamino group" means a group in which a carbonyl group bonded to one "$C_1$-$C_6$ alkyl group" described above is bonded to an amino group and includes, for example, an acetamido, ethylcarbonylamino, propylcarbonylamino, isopropylcarbonylamino, butylcarbonylamino, isobutylcarbonylamino, s-butylcarbonylamino, t-butylcarbonylamino, pentylcarbonylamino, isopentylcarbonylamino, 2-methylbutylcarbonylamino, neopentylcarbonylamino, 1-ethylpropylcarbonylamino, hexylcarbonylamino, isohexylcarbonylamino, 4-methylpentylcarbonylamino, 3-methylpentylcarbonylamino, 2-methylpentylcarbonylamino, 1-methylpentylcarbonylamino, 3,3-dimethylbutylcarbonylamino, 2,2-dimethylbutylcarbonylamino, 1,1-dimethylbutylcarbonylamino, 1,2-dimethylbutylcarbonylamino, 1,3-dimethylbutylcarbonylamino, 2,3-dimethylbutylcarbonylamino or 2-ethylbutylcarbonylamino group, preferably a group in which a carbonyl group bonded to one "$C_1$-$C_4$ alkyl group" described above is bonded to an amino group (mono-$C_2$-$C_5$ alkylcarbonylamino group), more preferably an acetamido or ethylcarbonylamino group (mono-$C_2$-$C_3$ alkylcarbonylamino group), further more preferably an acetamido group.

In the present invention, the "mono-$C_1$-$C_6$ alkylsulfonylamino group" means a group in which a sulfonyl group bonded to one "$C_1$-$C_6$ alkyl group" described above is bonded to an amino group and includes, for example, a methylsulfonylamino, ethylsulfonylamino, propylsulfonylamino, isopropylsulfonylamino, butylsulfonylamino, isobutylsulfonylamino, s-butylsulfonylamino, t-butylsulfonylamino, pentylsulfonylamino, isopentylsulfonylamino, 2-methylbutylsulfonylamino, neopentylsulfonylamino, 1-ethylpropylsulfonylamino, hexylsulfonylamino, isohexylsulfonylamino, 4-methylpentylsulfonylamino, 3-methylpentylsulfonylamino, 2-methylpentylsulfonylamino, 1-methylpentylsulfonylamino, 3,3-dimethylbutylsulfonylamino, 2,2-dimethylbutylsulfonylamino, 1,1-dimethylbutylsulfonylamino, 1,2-dimethylbutylsulfonylamino, 1,3-dimethylbutylsulfonylamino, 2,3-dimethylbutylsulfonylamino or 2-ethylbutylsulfonylamino group, preferably a group in which a sulfonyl group bonded to one "$C_1$-$C_4$ alkyl group" described above is bonded to an amino group (mono-$C_1$-$C_4$ alkylsulfonylamino group), more preferably a methylsulfonylamino or ethylsulfonylamino group (mono-$C_1$-$C_2$ alkylsulfonylamino group), further more preferably a methylsulfonylamino group.

In the present invention, the "mono-$C_1$-$C_6$ alkylamino group" means a group in which one "$C_1$-$C_6$ alkyl group" described above is bonded to an amino group and includes, for example, a methylamino, ethylamino, propylamino, isopropylamino, butylamino, isobutylamino, s-butylamino, t-butylamino, pentylamino, isopentylamino, 2-methylbutylamino, neopentylamino, 1-ethylpropylamino, hexylamino, isohexylamino, 4-methylpentylamino, 3-methylpentylamino, 2-methylpentylamino, 1-methylpentylamino, 3,3-dimethylbutylamino, 2,2-dimethylbutylamino, 1,1-dimethylbutylamino, 1,2-dimethylbutylamino, 1,3-dimethylbutylamino, 2,3-dimethylbutylamino or 2-ethylbutylamino group, preferably a group in which one "$C_1$-$C_4$ alkyl group" described above is bonded to an amino group (mono-$C_1$-$C_4$ alkylamino group), more preferably a methylamino group or an ethylamino group (mono-$C_1$-$C_2$ alkylamino group), further more preferably a methylamino group.

In the present invention, the "di-($C_1$-$C_6$ alkyl)amino group" means a group in which the same or different two "$C_1$-$C_6$ alkyl groups" described above are bonded to an amino group and includes, for example, a dimethylamino, diethylamino, dipropylamino, diisopropylamino, dibutylamino, diisobutylamino, dipentylamino, diisopentylamino, dineopentylamino, dihexylamino, diisohexylamino, N-ethyl-N-methylamino, N-methyl-N-propylamino, N-isopropyl-N-methylamino, N-butyl-N-methylamino, N-isobutyl-N-methylamino, N-methyl-N-pentylamino, N-isopentyl-N-methylamino, N-ethyl-N-propylamino, N-ethyl-N-isopropylamino, N-butyl-N-ethylamino, N-ethyl-N-isobutylamino, N-ethyl-N-pentylamino or N-ethyl-N-isopentylamino group, preferably a group in which the same or different two "$C_1$-$C_4$ alkyl groups" described above are bonded to an amino group (di-$C_1$-$C_4$ alkylamino group), more preferably a dimethylamino group, a diethylamino group or a N-ethyl-N-methylamino group (di-$C_1$-$C_2$ alkylamino group), further more preferably a dimethylamino group.

In the present invention, "N-($C_1$-$C_6$ alkyl)-N-($C_1$-$C_6$ hydroxyalkyl)amino group" means a group in which one "$C_1$-$C_6$ alkyl group" described above and one "$C_1$-$C_6$ hydroxyalkyl group" described above are bonded to an amino group and includes, for example, a N-(2-hydroxyethyl)-N-methylamino, N-(3-hydroxypropyl)-N-methylamino, N-ethyl-N-(2-hydroxyethyl)amino, N-ethyl-N-(3-hydroxypropyl)amino, N-(2-hydroxyethyl)-N-propylamino or N-(3-hydroxypropyl)-N-propylamino group, preferably a group in which one "$C_1$-$C_4$ alkyl group" described above and one "$C_1$-$C_3$ hydroxyalkyl group" described above are bonded to an amino group (N-($C_1$-$C_4$ alkyl)-N-($C_1$-$C_3$ hydroxyalkyl)amino group), more preferably a N-(2-hydroxyethyl)-N-methylamino or N-(3-hydroxypropyl)-N-methylamino group.

In the present invention, the "di-($C_1$-$C_6$ hydroxyalkyl)amino group" means a group in which the same or different two "$C_1$-$C_6$ hydroxyalkyl groups" described above are bonded to an amino group and includes, for example, a di-(2-hydroxyethyl)amino, di-(3-hydroxypropyl)amino, di-(2-hydroxypropyl)amino, di-(4-hydroxybutyl)amino, di-(5-hydroxypentyl)amino, di-(6-hydroxyhexyl)amino, N-(2-hydroxyethyl)-N-(3-hydroxypropyl)amino, N-(2-hydroxyethyl)-N-(2-hydroxypropyl)amino or N-(4-hydroxybutyl)-N-(2-hydroxyethyl)amino group, preferably a group in which the same or different two "$C_1$-$C_3$ hydroxyalkyl groups" described above are bonded to an amino group (di-$C_1$-$C_3$ hydroxyalkylamino group), more preferably a di-(2-hydroxyethyl)amino group.

In the present invention, the "nitrogen-containing heterocyclic group" means a 4 to 7-membered heterocyclic group containing 1 or 2 nitrogen atom(s) and includes a "partially or completely reduced saturated heterocyclic group" such as, for example, an azetidinyl, pyrrolidinyl, piperidinyl, pyrazolidinyl or piperazinyl group, preferably a pyrrolidinyl group.

In the present invention, the "nitrogen-containing heterocyclic group which may be monosubstituted by a hydroxyl group" means the aforementioned "nitrogen-containing heterocyclic group" which may be monosubstituted by a hydroxyl group and includes, for example, a 3-hydroxy-1-azetidinyl, 3-hydroxy-1-pyrrolidinyl, 4-hydroxy-1-piperidinyl, 4-hydroxy-1-pyrazolidinyl or 3-hydroxy-1-piperazinyl group, preferably a 3-hydroxy-1-pyrrolidinyl group.

In the present invention, the "$C_6$-$C_{10}$ aryl group" means an aromatic hydrocarbon group having 6 to 10 carbon atoms, such as, for example, a phenyl, indenyl or naphthyl group, preferably a phenyl or naphthyl group, more preferably a phenyl group.

In the present invention, the "$C_6$-$C_{10}$ aryl group which may be independently mono- to trisubstituted by a group selected from Substituent Group b" means the aforementioned "$C_6$-$C_{10}$ aryl group" which may be independently mono- to trisubstituted by a group selected from Substituent Group b, preferably a phenyl group which may be monosubstituted by a fluorine atom, a chlorine atom, a bromine atom, a methyl group, an ethyl group or a trifluoromethyl group at the 2, 3 or 4 position.

In the present invention, the "$C_6$-$C_{10}$ aryloxy group" means a group in which the above "$C_6$-$C_{10}$ aryl group" is

bonded to an oxygen atom and includes an aryloxy group having 6 to 10 carbon atoms such as, for example, a phenyloxy, indenyloxy or naphthyloxy group, preferably a phenyloxy or naphthyloxy group, more preferably a phenyloxy group.

In the present invention, the "$C_6$-$C_{10}$ aryloxy group which may be independently mono- to trisubstituted by a group selected from Substituent Group b" means the aforementioned "$C_6$-$C_{10}$ aryloxy group" which may be independently mono- to trisubstituted by a group selected from Substituent Group b, preferably a phenyloxy group which may be monosubstituted by a fluorine atom, a chlorine atom, a bromine atom, a methyl group, an ethyl group or a trifluoromethyl group at the 2, 3 or 4 position.

In the present invention, the "$C_1$-$C_6$ alkyl group disubstituted by a hydroxyl group" means a group in which two hydroxyl groups are bonded to the aforementioned "$C_1$-$C_6$ alkyl group" and includes, for example, a dihydroxyethyl, dihydroxypropyl or dihydroxybutyl group, preferably a group in which two hydroxyl groups are bonded to a linear or branched alkyl group having 2 to 4 carbon atoms ($C_2$-$C_4$ hydroxyalkyl group disubstituted by a hydroxyl group), more preferably a 2,3-dihydroxypropyl group.

In the present invention, the "-P(=O)(OH)(O-$C_1$-$C_6$ alkyl)" means a group in which an oxygen atom bonded to one "$C_1$-$C_6$ alkyl group" described above and a hydroxyl group are bonded to a phosphoryl group and is preferably phosphonic acid monoethyl ester or phosphonic acid monomethyl ester, more preferably phosphonic acid monoethyl ester.

In the present invention, the "$C_1$-$C_6$ alkyl group monosubstituted by - P(=O)(OH)(O-$C_1$-$C_6$ alkyl)" means a group in which one "-P(=O)(OH)(O-$C_1$-$C_6$ alkyl)" described above is bonded to the above "$C_1$-$C_6$ alkyl group" and is preferably phosphonic acid monoethyl ester methyl or phosphonic acid monoethyl ester ethyl, more preferably phosphonic acid monoethyl ester methyl.

In the present invention, the "$C_1$-$C_6$ alkyl group monosubstituted by a carboxyl group" means a group in which a carboxyl group is bonded to the above "$C_1$-$C_6$ alkyl group" and is preferably hydroxycarbonylmethyl or hydroxycarbonylethyl, more preferably hydroxycarbonylmethyl.

In the present invention, the "$C_1$-$C_6$ alkyl group monosubstituted by a $C_2$-$C_7$ alkoxycarbonyl group" means a group in which one "$C_2$-$C_7$ alkoxycarbonyl group" described above is bonded to the above "$C_1$-$C_6$ alkyl group" and is preferably methoxycarbonylmethyl or methoxycarbonylethyl, more preferably methoxycarbonylmethyl.

In the present invention, the "heterocyclic group" means a 4 to 7-membered heterocyclic group containing 1 to 3 sulfur, oxygen and/or nitrogen atom(s) and includes an "aromatic heterocyclic group" such as, for example, oxazolyl, thiazolyl, pyridyl or pyrimidinyl. The above heterocyclic group may form a condensed ring with another cyclic group such as a benzene ring ("condensed bicyclic heteroaryl group") and includes, for example, a benzothiazolyl, benzoxazolyl, dihydrobenzofuryl, quinolyl or quinazolyl group. It is preferably an aromatic heterocyclic group or a condensed bicyclic heteroaryl group, more preferably a pyridyl, thiazolyl, benzoxazolyl or dihydrobenzofuryl group. In $R^1$, it is further more preferably a 2-thiazolyl group, in $R^2$, it is further more preferably a 2-pyridyl group.

In the present invention, the "$C_7$-$C_{16}$ aralkyl group" means a group in which one "$C_6$-$C_{10}$ aryl group" described above is bonded to the above "$C_1$-$C_6$ alkyl group"and includes, for example, a benzyl, α-naphthylmethyl, β-naphthylmethyl, indenylmethyl, 1-phenethyl, 2-phenethyl, 1-naphthylethyl, 2-naphthylethyl, 1-phenylpropyl, 2-phenylpropyl, 3-phenylpropyl, 1-naphthylpropyl, 2-naphthylpropyl, 3-naphthylpropyl, 1-phenylbutyl, 2-phenylbutyl, 3-phenylbutyl, 4-phenylbutyl, 1-naphthylbutyl, 2-naphthylbutyl, 3-naphthylbutyl, 4-naphthylbutyl, 1-phenylpentyl, 2-phenylpentyl, 3-phenylpentyl, 4-phenylpentyl, 5-phenylpentyl, 1-naphthylpentyl, 2-naphthylpentyl, 3-naphthylpentyl, 4-naphthylpentyl, 5-naphthylpentyl, 1-phenylhexyl, 2-phenylhexyl, 3-phenylhexyl, 4-phenylhexyl, 5-phenylhexyl, 6-phenylhexyl, 1-naphthylhexyl, 2-naphthylhexyl, 3-naphthylhexyl, 4-naphthylhexyl, 5-naphthylhexyl or 6-naphthylhexyl group, preferably a group in which one "$C_6$-$C_{10}$ aryl group" described above is bonded the above "$C_1$-$C_4$ alkyl group", more preferably a benzyl group.

In the present invention, the "$C_6$-$C_{10}$ aryl group which may be independently mono- to pentasubstituted by a group selected from Substituent Group a" means the aforementioned "$C_6$-$C_{10}$ aryl group" which may be independently mono- to pentasubstituted by a group selected from Substituent Group a. In $R^1$, it is preferably a phenyl group independently mono- to trisubstituted by a group selected from the group consisting of a halogen atom, a $C_1$-$C_6$ alkyl group, a $C_1$-$C_6$ halogenated alkyl group, a $C_1$-$C_6$ alkoxy group, a carboxyl group, an amino group, a mono-$C_2$-$C_7$ alkylcarbonylamino group, a di-($C_1$-$C_6$ alkyl)amino group, a cyano group and a nitro group, more preferably a phenyl group independently mono- to trisubstituted by a group selected from the group consisting of a halogen atom, a $C_1$-$C_6$ alkyl group and a $C_1$-$C_6$ alkoxy group, further more preferably a phenyl group substituted by a group selected from the group consisting of a fluorine atom, a chlorine atom, a methyl group, a methoxy group and an ethoxy group at the 2- or 3-position; or a phenyl group independently disubstituted by a group selected from the group consisting of a fluorine atom, a chlorine atom, a methyl group, a methoxy group and an ethoxy group at the 2, 3 or 5-position, particularly preferably a 2-fluorophenyl group, a 2-ethoxyphenyl group, a 3-ethoxyphenyl group, a 5-fluoro-2-methoxyphenyl group, a 2-ethoxy-5-fluorophenyl group, a 5-chloro-2-methoxyphenyl group, a 5-chloro-2-ethoxyphenyl group, a 2-methoxy-5-methylphenyl group or a 3,5-dimethoxymethylphenyl group. In $R^2$, it is preferably a phenyl group which may be independently mono- to trisubstituted by a group selected from the group consisting of a halogen atom, a $C_1$-$C_6$ alkyl group, a $C_1$-$C_6$ halogenated

alkyl group, a $C_1$-$C_6$ hydroxyalkyl group, a $C_1$-$C_6$ alkoxy group, a $C_1$-$C_6$ alkoxy group mono- or disubstituted by a hydroxyl group, a carboxyl group, an amino group, a mono-$C_2$-$C_7$ alkylcarbonylamino group, a cyano group, a nitro group, a hydroxyl group, a $C_1$-$C_6$ alkyl group monosubstituted by a carboxyl group and a

$C_1$-$C_6$ alkyl group monosubstituted by a $C_2$-$C_7$ alkoxycarbonyl group, more preferably a phenyl group which may be independently mono- to trisubstituted by a group selected from the group consisting of a halogen atom, a $C_1$-$C_6$ alkyl group, a $C_1$-$C_6$ halogenated alkyl group, a $C_1$-$C_6$ alkoxy group mono- or disubstituted by a hydroxyl group and a cyano group, further more preferably a phenyl group substituted by a group selected from the group consisting of a fluorine atom, a chlorine atom, a methyl group and a trifluoromethyl group at the 2-position and substituted by a group selected from the group consisting of a 2-hydroxyethoxy group and a 2,3-dihydroxypropoxy group at the 4- or 5-position, particularly preferably a 2-chloro-4-(2-hydroxyethoxy)phenyl group, a 2-chloro-5-(2-hydroxyethoxy)phenyl group, a 4-(2-hydroxyethoxy)-2-trifluoromethylphenyl group, a 2-chloro-4-(2,3-dihydroxypropoxy)phenyl group, a 4-(2,3-dihydroxy-propoxy)-2-trifluoromethylphenyl group or a 2-chloro-5-(2,3-dihydroxypropoxy)phenyl group.

In the present invention, the "heterocyclic group which may be independently mono- to trisubstituted by a group selected from Substituent Group a" means the aforementioned "heterocyclic group" which may be independently mono-to trisubstituted by a group selected from Substituent Group a. In $R^1$, it is preferably a 2-thiazolyl group which may be independently mono- or disubstituted by a group selected from the group consisting of a methyl group, an ethyl group, a t-butyl group and a cyclopropyl group; a 2-pyridyl, 3-pyridyl or 4-pyridyl group which is monosubstituted by a methoxy group; 1,4-benzodioxan-5-yl; 2,3-dihydrobenzofuran-5-yl; or a 2,3-dihydrobenzofuran-7-yl group, more preferably a 2-thiazolyl group which may be independently mono- or disubstituted by a group selected from the group consisting of a methyl group and a t-butyl group, further more preferably a 5-methyl-2-thiazolyl group. In $R^2$, it is preferably a 2-pyridyl, 3-pyridyl or 4-pyridyl group which is independently mono- or disubstituted by a group selected from the group consisting of a fluorine atom, a chlorine atom, a methyl group, a trifluoromethyl group, a hydroxymethyl group, a carboxyl group and a cyano group, more preferably a 2-pyridyl group monosubstituted by a group selected from the group consisting of a fluorine atom, a chlorine atom, a methyl group, a trifluoromethyl group, a hydroxymethyl group and a cyano group, or a 2-pyridyl group independently disubstituted by a group selected from the group consisting of a fluorine atom and a chlorine atom, further more preferably a 3-methyl-2-pyridyl group, a 3-cyano-2-pyridyl group or a 3,5-difluoro-2-pyridyl group, particularly preferably a 3-methyl-2-pyridyl group.

In the present invention, the "$C_7$-$C_{16}$ aralkyl group which may be independently mono- to trisubstituted by a group selected from Substituent Group a" means the aforementioned "$C_7$-$C_{16}$ aralkyl" group which may be independently mono- to trisubstituted by a group selected from Substituent Group a and is preferably a benzyl group which may be independently mono- to trisubstituted by a group selected from the group consisting of a halogen atom, a $C_1$-$C_6$ alkyl group, a $C_1$-$C_2$ halogenated alkyl group, a $C_1$-$C_3$ hydroxyalkyl group, a $C_1$-$C_6$ alkoxy group, a $C_1$-$C_2$ halogenated alkoxy group, a carboxyl group, an amino group, a mono-$C_2$-$C_3$ alkylcarbonylamino group, a di-$C_1$-$C_2$ alkylamino group, a cyano group, a nitro group and a phenyloxy group, more preferably a benzyl group substituted by a fluorine atom, a chlorine atom, a bromine atom, a methyl group, a trifluoromethyl group, a hydroxymethyl group, a carboxyl group, an amino group or a nitro group at the 2- and 6-positions; or a benzyl group substituted by a fluorine atom, a chlorine atom, a bromine atom, a methyl group or a trifluoromethyl group at the 2-position, further more preferably a 2-fluorophenylmethyl group or a 2,6-difluorophenylmethyl group.

In the present invention, the phrase "A represents a single bond" means, for example, that the "group $R^2$-A-E-" is a "group $R^2$-E-".

In the present invention, $R^1$ is preferably a phenyl group independently mono-to trisubstituted by a group selected from the group consisting of a halogen atom, a $C_1$-$C_6$ alkyl group, a $C_1$-$C_6$ halogenated alkyl group, a $C_1$-$C_6$ alkoxy group, a carboxyl group, an amino group, a mono-$C_2$-$C_7$ alkylcarbonylamino group, a di-($C_1$ - $C_6$ alkyl)amino group, a cyano group and a nitro group, or a thiazolyl group which may be independently mono- or disubstituted by a $C_1$-$C_6$ alkyl group, more preferably a phenyl group independently mono- to trisubstituted by a group selected from the group consisting of a halogen atom, a $C_1$-$C_6$ alkyl group and a $C_1$-$C_6$ alkoxy group or a thiazolyl group independently mono- or disubstituted by a $C_1$-$C_6$ alkyl group, further more preferably a phenyl group substituted by a group selected from the group consisting of a fluorine atom, a chlorine atom, a methyl group, a methoxy group and an ethoxy group at the 2- or 3-position; a phenyl group independently disubstituted by a group selected from the group consisting of a fluorine atom, a chlorine atom, a methyl group, a methoxy group and an ethoxy group at the 2, 3 or 5-position; or a 5-methyl-2-thiazolyl group, particularly preferably a 2-fluorophenyl group, a 2-ethoxyphenyl group, a 3-ethoxyphenyl group, a 5-fluoro-2-methoxyphenyl group, a 2-ethoxy-5-fluorophenyl group, a 5-chloro-2-methoxyphenyl group, a 5-chloro-2-ethoxyphenyl group, a 2-methoxy-5-methylphenyl group or a 3,5-dimethoxymethylphenyl group.

In the present invention, $R^2$ is preferably a phenyl or pyridyl group which may be independently mono- to trisubstituted by a group selected from the group consisting of a halogen atom, a $C_1$-$C_6$ alkyl group, a $C_1$-$C_6$ halogenated alkyl group, a $C_1$-$C_6$ hydroxyalkyl group, a $C_1$-$C_6$ alkoxy group, a $C_1$-$C_6$ alkoxy group mono- or disubstituted by a hydroxyl group, a carboxyl group, an amino group, a mono-$C_2$-$C_7$ alkylcarbonylamino group, a cyano group, a nitro group, a hydroxyl group, a $C_1$-$C_6$ alkyl group monosubstituted by a carboxyl group and a $C_1$-$C_6$ alkyl group monosubstituted by

a $C_2$-$C_7$ alkoxycarbonyl group, more preferably a phenyl or pyridyl group which may be independently mono- to trisubstituted by a group selected from the group consisting of a halogen atom, a $C_1$-$C_6$ alkyl group, a $C_1$-$C_6$ halogenated alkyl group, a $C_1$-$C_6$ alkoxy group mono- or disubstituted by a hydroxyl group and a cyano group, further more preferably a phenyl group substituted by a group selected from the group consisting of a fluorine atom, a chlorine atom, a methyl group and a trifluoromethyl group at the 2-position and substituted by a group selected from the group consisting of a 2-hydroxyethoxy group and a 2,3-dihydroxypropoxy group at the 4- or 5-position; a 3-methyl-2-pyridyl group; a 3-cyano-2-pyridyl group; or a 3,5-difluoro-2-pyridyl group, particularly preferably a 2-chloro-4-(2-hydroxyethoxy)phenyl group, a 2-chloro-5-(2-hydroxyethoxy)phenyl group, a 4-(2-hydroxyethoxy)-2-trifluoromethylphenyl group, a 2-chloro-4-(2,3-dihydroxypropoxy)phenyl group, a 4-(2,3-dihydroxypropoxy)-2-trifluoromethylphenyl group, a 2-chloro-5-(2,3-dihydroxypropoxy)phenyl group or a 3-methyl-2-pyridyl group.

In the present invention, E is preferably a group having the above formula (II) or a group having the formula (XL), more preferably a group having the above formula (II).

In the present invention, $R^3$ is preferably a hydrogen atom, a fluorine atom, a chlorine atom or a methyl group, more preferably a hydrogen atom, a fluorine atom or a chlorine atom, and further more preferably a hydrogen atom.

In the present invention, $R^4$ is preferably a hydrogen atom or a methyl group, more preferably a hydrogen atom.

In the present invention, $R^5$ is preferably a hydrogen atom or a methyl group, more preferably a hydrogen atom.

In the present invention, X is preferably a nitrogen atom.

In the present invention, U is preferably a group represented by the formula CH.

In the present invention, m is preferably 1.

In the present invention, n is preferably 1.

In the present invention, A is preferably a single bond, a group represented by the formula -O-C(=O)-, a group represented by the formula -NH-C(=O)-, a carbonyl group or a group represented by the formula -CH(OH)-C(=O)-, more preferably a group represented by the formula -O-C(=O)-, a group represented by formula -NH-C(=O)- or a group represented by the formula -CH(OH)-C(=O)-, further more preferably a group represented by the formula -NH-C(=O)- or a group represented by the formula -CH(OH)-C(=O)-, particularly preferably a group represented by the formula -NH-C(=O)-.

The "pharmacologically acceptable salt thereof" means a salt formed by allowing the urea derivative having the general formula (I) of the present invention to react with acid when the urea derivative contains a basic group such as an amino group, or with base when the urea derivative contains an acidic group such as a carboxyl group.

The salts derived from a basic group include, for example, inorganic salts such as hydrohalide including hydrofluoride, hydrochloride, hydrobromide and hydroiodide, nitrate, perchlorate, sulfate and phosphate; organic salts such as $C_1$-$C_6$ alkyl sulfonate including methanesulfonate, trifluoromethanesulfonate and ethanesulfonate, aryl sulfonate including benzenesulfonate and p-toluenesulfonate, acetate, malate, fumarate, succinate, citrate, ascorbate, tartrate, oxalate and maleate; and amino acid salts such as glycine salt, lysine salt, arginine salt, ornithine salt, glutamate and aspartate.

The salts derived from an acidic group include, for example, alkali metal salts such as sodium salt, potassium salt and lithium salt, alkaline earth metal salts such as calcium salt and magnesium salt, metal salts such as aluminium salt and iron salt; amine salts including inorganic salts such as ammonium salt and organic salts such as t-octylamine salt, dibenzylamine salt, morpholine salt, glucosamine salt, phenylglycine alkyl ester salt, ethylenediamine salt, N-methylglucamine salt, guanidine salt, diethylamine salt, triethylamine salt, dicyclohexylamine salt, N,N'-dibenzylethylenediamine salt, chloroprocaine salt, procaine salt, diethanolamine salt, N-benzylphenethylamine salt, piperazine salt, tetramethylammonium salt or tris(hydroxymethyl)aminomethane salt; and amino acid salts such as glycine salt, lysine salt, arginine salt, ornithine salt, glutamic acid and aspartate.

The urea derivative having the general formula (I) or a pharmacologically acceptable salt thereof according to the present invention may absorb water, or water is attached thereto to form a hydrate when left in the air or recrystallized. The salt of the present invention also encompasses such hydrates.

The urea derivative having the general formula (I) or a pharmacologically acceptable salt thereof according to the present invention may absorb another certain solvent to form a solvate. The salt of the present invention also encompasses such solvates.

Specific examples of compounds having the general formula (I) of the present invention are, for example, the compounds shown in the following Table 1 to Table 5, but the present invention is not limited to these groups.

Abbreviations in the following Table 1 to Table 5 stand for the following.

-: single bond
Me: Methyl
t-Bu: t-Butyl n-Hex: n-Hexyl
CycPent: Cyclopentyl
CycHex: Cyclohexyl
2-Me-CycHex: 2-Methylcyclohexyl

Ph: Phenyl
Bn: Benzyl
2-F-Ph: 2-Fluorophenyl
2-Cl-Ph: 2-Chlorophenyl
3-Cl-Ph: 3-Chlorophenyl
2-Br-Ph: 2-Bromophenyl
2-Et-Ph: 2-Ethylphenyl
2-n-Pr-Ph: 2-(n-Propyl)phenyl
4-i-Pr-Ph: 4-(i-Propyl)phenyl
4-n-Bu-Ph: 4-(n-Butyl)phenyl
4-t-Bu-Ph: 4-(tert-Butyl)phenyl
2-s-Bu-Ph: 2-(sec-Butyl)phenyl
2-$CF_3$-Ph: 2-Trifluoromethylphenyl
2-OMe-Ph: 2-Methoxyphenyl
2-OEt-Ph: 2-Ethoxyphenyl
4-O-n-Bu-Ph: 4-(n-Butoxy)phenyl
4-O-n-Hept-Ph: 4-(n-Heptoxy)phenyl
2-$OCF_2H$-Ph: 2-Difluoromethoxyphenyl
4-$NMe_2$-Ph: 4-Dimethylaminophenyl
2-CN-Ph: 2-Cyanophenyl
2-$NO_2$-Ph: 2-Nitrophenyl
2-OPh-Ph: 2-Phenoxyphenyl
4-Phos-Ph: 4-Phosphonic acid diethyl ester methylphenyl
2-F-$C_6H_4$-$CH_2$: 2-Fluorophenylmethyl
2,6-di-F-$C_6H_3$-$CH_2$: 2,6-Difluorophenylmethyl
2-F-5-Me-Ph: 2-Fluoro-5-methylphenyl
2-F-5-$CF_3$-Ph: 2-Fluoro-5-trifluoromethylphenyl
2-F-6-$CF_3$-Ph: 2-Fluoro-6-trifluoromethylphenyl
5-F-2-OMe-Ph: 5-Fluoro-2-methoxyphenyl
2-OEt-5-F-Ph: 2-Ethoxy-5-fluorophenyl
2,6-di-Cl-Ph: 2,6-Dichlorophenyl
4-Br-2-Cl-Ph: 4-Bromo-2-chlorophenyl
2-Cl-4-Me-Ph: 2-Chloro-4-methylphenyl
2-Cl-5-Me-Ph: 2-Chloro-5-methylphenyl
2-Cl-5-$CF_3$-Ph: 2-Chloro-5-trifluoromethylphenyl
5-Cl-2-OMe-Ph: 5-Chloro-2-methoxyphenyl
5-Cl-2-OEt-Ph: 5-Chloro-2-ethoxyphenyl
2-Cl-4-$OCH_2CH_2OH$-Ph: 2-Chloro-4-(2-hydroxyethoxy)phenyl
2-Cl-5-$OCH_2CH_2OH$-Ph: 2-Chloro-5-(2-hydroxyethoxy)phenyl
4-$NH_2$-2-Cl-Ph: 4-Amino-2-chlorophenyl
5-Cl-2-$NHSO_2Me$-Ph: 5-Chloro-2-methylsulfonylaminophenyl
5-Cl-2-$NO_2$-Ph: 5-Chloro-2-nitrophenyl
4-COOH-2-Cl-Ph : 4-Carboxy-2-chlorophenyl
4-NHCOMe-2-Cl-Ph : 4-Acetamido-2-chlorophenyl
5-Cl-2-OPh-Ph: 5-Chloro-2-phenoxyphenyl
4-Br-2-Me-Ph: 4-Bromo-2-methylphenyl
2-Br-6-Me-Ph: 2-Bromo-6-methylphenyl
4-Br-2-CN-Ph: 4-Bromo-2-cyanophenyl
2,3-di-Me-Ph: 2,3-Dimethylphenyl
2-Et-6-Me-Ph: 2-Ethyl-6-methylphenyl
4-n-Bu-2-Me-Ph: 4-(n-Butyl)-2-methylphenyl
3,5-di-OMe-Ph: 3,5-Dimethoxyphenyl
4-i-Pr-2-$CF_3$-Ph: 4-(i-Propyl)-2-trifluoromethylphenyl
2-$NH_2$-6-Me-Ph: 2-Amino-6-methylphenyl
4-$NH_2$-2-$CF_3$-Ph: 2-Amino-2-trifluoromethylphenyl
2-OMe-5-Me-Ph: 2-Methoxy-5-methylphenyl
4-OMe-2-$CF_3$-Ph: 4-Methoxy-2-trifluoromethylphenyl
2-$CH_2OH$-6-Me-Ph: 2-Hydroxymethyl-6-methylphenyl

4-$OCH_2CH_2OH$-2-$CF_3$-Ph: 4-(2-Hydroxyethoxy)-2-trifluoromethylphenyl
4-COOH-2-Me-Ph: 4-Carboxy-2-methylphenyl
2- Me-6- $NO_2$- Ph: 2-Methyl-6-nitrophenyl
4-NHCOMe-2-$CF_3$-Ph: 4-Acetamido-2-trifluoromethylphenyl
2-Bic[2.2.1]hept: 2-Bicyclo[2.2.1]heptyl
4-Cl-2,6-di-Me-Ph: 4-Chloro-2,6-dimethylphenyl
2,4,6-tri-Me-Ph: 2,4,6-Trimethylphenyl
2-Pyri: 2-Pyrimidinyl
3-Me-2-Py: 3-Methyl-2-pyridyl
3,5-di-C1-2-Py: 3,5-Dichloro-2-pyridyl
5-Me-2-thiazo: 5-Methyl-2-thiazolyl
4-CycPr-2-thiazo: 4-Cyclopropyl-2-thiazolyl
5-tetra: 5-Tetralyl
Ph(A): 2-Chloro-4-(2,3-dihydroxypropoxy)phenyl
Ph(B): 4-(2,3-Dihydroxypropoxy)-2-trifluoromethylphenyl
Ph(C): 2-Chloro-5-(2,3-dihydroxypropoxy)phenyl
Ph(D): 4-(3-hydroxy-1-pyrrolidinyl)-2-methylphenyl
Ph(E): 4-(2,3-Dihydroxypropoxy)-2-fluorophenyl
Ph(F): 4-(2,3-Dihydroxypropoxy)-2-methylphenyl
Ph(G): 4-[4-(S)-2,2-dimethyl-1,3-dioxa-4-cyclopentyl]-2-methylphenyl
Ph(H): 4-[1-(S)-1,2-dihydroxy-ethyl]-2-methylphenyl
Het(A): 1,4-Benzodioxan-5-yl
Het(B): 2,3-Dihydrobenzofuran-5-yl
Het(C): 2,3-Dihydrobenzofuran-7-yl.

(Table 1)

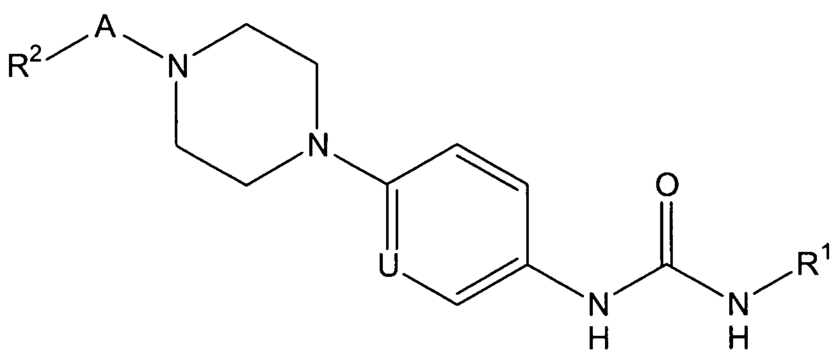

(V)

| Compound No. | $R^2$ | A | U | $R^1$ |
|---|---|---|---|---|
| 1-1 | 2,6-di-F-Ph | -NH-C(=O)- | CH | 5-F-2-OMe-Ph |
| 1-2 | 2,6-di-F-Ph | -NH-C(=O)- | CH | 2-Oet-5-F-Ph |
| 1-3 | 2,6-di-F-Ph | -NH-C(=O)- | CH | 5-Cl-2-OMe-Ph |
| 1-4 | 2,6-di-F-Ph | -NH-C(=O)- | CH | 5-Cl-2-OEt-Ph |
| 1-5 | 2,6-di-F-Ph | -NH-C(=O)- | CH | 2-OMe-5-Me-Ph |
| 1-6 | 2,6-di-F-Ph | -NH-C(=O)- | CH | 2-Oet-5-Me-Ph |
| 1-7 | 2,6-di-F-Ph | -NH-C(=O)- | CH | 2-Oet-Ph |
| 1-8 | 2,6-di-F-Ph | -NH-C(=O)- | N | 5-F-2-OMe-Ph |
| 1-9 | 2,6-di-F-Ph | -NH-C(=O)- | N | 2-Oet-5-F-Ph |
| 1-10 | 2,6-di-F-Ph | -NH-C(=O)- | N | 5-Cl-2-OMe-Ph |
| 1-11 | 2,6-di-F-Ph | -NH-C(=O)- | N | 5-Cl-2-OEt-Ph |
| 1-12 | 2,6-di-F-Ph | -NH-C(=O)- | N | 2-OMe-5-Me-Ph |
| 1-13 | 2,6-di-F-Ph | -NH-C(=O)- | N | 2-Oet-5-Me-Ph |
| 1-14 | 2,6-di-F-Ph | -NH-C(=O)- | N | 2-Oet-Ph |
| 1-15 | 2-F-6-$CF_3$-Ph | -NH-C(=O)- | CH | 5-F-2-OMe-Ph |

(continued)

| Compound No. | $R^2$ | A | U | $R^1$ |
|---|---|---|---|---|
| 1-16 | 2-F-6-$CF_3$-Ph | -NH-C(=O)- | CH | 2-Oet-5-F-Ph |
| 1-17 | 2-F-6-$CF_3$-Ph | -NH-C(=O)- | CH | 5-Cl-2-OMe-Ph |
| 1-18 | 2-F-6-$CF_3$-Ph | -NH-C(=O)- | CH | 5-Cl-2-OEt-Ph |
| 1-19 | 2-F-6-$CF_3$-Ph | -NH-C(=O)- | CH | 2-OMe-5-Me-Ph |
| 1-20 | 2-F-6-$CF_3$-Ph | -NH-C(=O)- | CH | 2-Oet-5-Me-Ph |
| 1-21 | 2-F-6-$CF_3$-Ph | -NH-C(=O)- | CH | 2-Oet-Ph |
| 1-22 | 2-F-6-$CF_3$-Ph | -NH-C(=O)- | N | 5-F-2-OMe-Ph |
| 1-23 | 2-F-6-$CF_3$-Ph | -NH-C(=O)- | N | 2-Oet-5-F-Ph |
| 1-24 | 2-F-6-$CF_3$-Ph | -NH-C(=O)- | N | 5-Cl-2-OMe-Ph |
| 1-25 | 2-F-6-$CF_3$-Ph | -NH-C(=O)- | N | 5-Cl-2-OEt-Ph |
| 1-26 | 2-F-6-$CF_3$-Ph | -NH-C(=O)- | N | 2-OMe-5-Me-Ph |
| 1-27 | 2-F-6-$CF_3$-Ph | -NH-C(=O)- | N | 2-Oet-5-Me-Ph |
| 1-28 | 2-F-6-$CF_3$-Ph | -NH-C(=O)- | N | 2-Oet-Ph |
| 1-29 | 2,6-di-Cl-Ph | -NH-C(=O)- | CH | 5-F-2-OMe-Ph |
| 1-30 | 2,6-di-Cl-Ph | -NH-C(=O)- | CH | 2-Oet-5-F-Ph |
| 1-31 | 2,6-di-Cl-Ph | -NH-C(=O)- | CH | 5-Cl-2-OMe-Ph |
| 1-32 | 2,6-di-Cl-Ph | -NH-C(=O)- | CH | 5-Cl-2-OEt-Ph |
| 1-33 | 2,6-di-Cl-Ph | -NH-C(=O)- | CH | 2-OMe-5-Me-Ph |
| 1-34 | 2,6-di-Cl-Ph | -NH-C(=O)- | CH | 2-Oet-5-Me-Ph |
| 1-35 | 2,6-di-Cl-Ph | -NH-C(=O)- | CH | 2-Oet-Ph |
| 1-36 | 2,6-di-Cl-Ph | -NH-C(=O)- | N | 5-F-2-OMe-Ph |
| 1-37 | 2,6-di-Cl-Ph | -NH-C(=O)- | N | 2-Oet-5-F-Ph |
| 1-38 | 2,6-di-Cl-Ph | -NH-C(=O)- | N | 5-Cl-2-OMe-Ph |
| 1-39 | 2,6-di-Cl-Ph | -NH-C(=O)- | N | 5-Cl-2-OEt-Ph |
| 1-40 | 2,6-di-Cl-Ph | -NH-C(=O)- | N | 2-OMe-5-Me-Ph |
| 1-41 | 2,6-di-Cl-Ph | -NH-C(=O)- | N | 2-OEt-5-Me-Ph |
| 1-42 | 2,6-di-Cl-Ph | -NH-C(=O)- | N | 2-OEt-Ph |
| 1-43 | 2-Cl-6-Me-Ph | -NH-C(=O)- | CH | 5-F-2-OMe-Ph |
| 1-44 | 2-Cl-6-Me-Ph | -NH-C(=O)- | CH | 2-OEt-5-F-Ph |
| 1-45 | 2-Cl-6-Me-Ph | -NH-C(=O)- | CH | 5-Cl-2-OMe-Ph |
| 1-46 | 2-Cl-6-Me-Ph | -NH-C(=O)- | CH | 5-Cl-2-OEt-Ph |
| 1-47 | 2-Cl-6-Me-Ph | -NH-C(=O)- | CH | 2-OMe-5-Me-Ph |
| 1-48 | 2-Cl-6-Me-Ph | -NH-C(=O)- | CH | 2-OEt-5-Me-Ph |
| 1-49 | 2-Cl-6-Me-Ph | -NH-C(=O)- | CH | 2-OEt-Ph |
| 1-50 | 2-Cl-6-Me-Ph | -NH-C(=O)- | N | 5-F-2-OMe-Ph |
| 1-51 | 2-Cl-6-Me-Ph | -NH-C(=O)- | N | 2-OEt-5-F-Ph |
| 1-52 | 2-Cl-6-Me-Ph | -NH-C(=O)- | N | 5-Cl-2-OMe-Ph |
| 1-53 | 2-Cl-6-Me-Ph | -NH-C(=O)- | N | 5-Cl-2-OEt-Ph |
| 1-54 | 2-Cl-6-Me-Ph | -NH-C(=O)- | N | 2-OMe-5-Me-Ph |
| 1-55 | 2-Cl-6-Me-Ph | -NH-C(=O)- | N | 2-OEt-5-Me-Ph |
| 1-56 | 2-Cl-6-Me-Ph | -NH-C(=O)- | N | 2-OEt-Ph |
| 1-57 | 2,6-di-Me-Ph | -NH-C(=O)- | CH | 5-F-2-OMe-Ph |
| 1-58 | 2,6-di-Me-Ph | -NH-C(=O)- | CH | 2-OEt-5-F-Ph |
| 1-59 | 2,6-di-Me-Ph | -NH-C(=O)- | CH | 5-Cl-2-OMe-Ph |
| 1-60 | 2,6-di-Me-Ph | -NH-C(=O)- | CH | 5-Cl-2-OEt-Ph |
| 1-61 | 2,6-di-Me-Ph | -NH-C(=O)- | CH | 2-OMe-5-Me-Ph |
| 1-62 | 2,6-di-Me-Ph | -NH-C(=O)- | CH | 2-OEt-5-Me-Ph |
| 1-63 | 2,6-di-Me-Ph | -NH-C(=O)- | CH | 2-OEt-Ph |
| 1-64 | 2,6-di-Me-Ph | -NH-C(=O)- | N | 5-F-2-OMe-Ph |
| 1-65 | 2,6-di-Me-Ph | -NH-C(=O)- | N | 2-OEt-5-F-Ph |

(continued)

| Compound No. | $R^2$ | A | U | $R^1$ |
|---|---|---|---|---|
| 1-66 | 2,6-di-Me-Ph | -NH-C(=O)- | N | 5-Cl-2-OMe-Ph |
| 1-67 | 2,6-di-Me-Ph | -NH-C(=O)- | N | 5-Cl-2-OEt-Ph |
| 1-68 | 2,6-di-Me-Ph | -NH-C(=O)- | N | 2-OMe-5-Me-Ph |
| 1-69 | 2,6-di-Me-Ph | -NH-C(=O)- | N | 2-OEt-5-Me-Ph |
| 1-70 | 2,6-di-Me-Ph | -NH-C(=O)- | N | 2-OEt-Ph |
| 1-71 | 2-$CH_2$OH-6-Me-Ph | -NH-C(=O)- | CH | 5-F-2-OMe-Ph |
| 1-72 | 2-$CH_2$OH-6-Me-Ph | -NH-C(=O)- | CH | 2-OEt-5-F-Ph |
| 1-73 | 2-$CH_2$OH-6-Me-Ph | -NH-C(=O)- | CH | 5-Cl-2-OMe-Ph |
| 1-74 | 2-$CH_2$OH-6-Me-Ph | -NH-C(=O)- | CH | 5-Cl-2-OEt-Ph |
| 1-75 | 2-$CH_2$OH-6-Me-Ph | -NH-C(=O)- | CH | 2-OMe-5-Me-Ph |
| 1-76 | 2-$CH_2$OH-6-Me-Ph | -NH-C(=O)- | CH | 2-OEt-5-Me-Ph |
| 1-77 | 2-$CH_2$OH-6-Me-Ph | -NH-C(=O)- | CH | 2-OEt-Ph |
| 1-78 | 2-$CH_2$OH-6-Me-Ph | -NH-C(=O)- | N | 5-F-2-OMe-Ph |
| 1-79 | 2-$CH_2$OH-6-Me-Ph | -NH-C(=O)- | N | 2-OEt-5-F-Ph |
| 1-80 | 2-$CH_2$OH-6-Me-Ph | -NH-C(=O)- | N | 5-Cl-2-OMe-Ph |
| 1-81 | 2-$CH_2$OH-6-Me-Ph | -NH-C(=O)- | N | 5-Cl-2-OEt-Ph |
| 1-82 | 2-$CH_2$OH-6-Me-Ph | -NH-C(=O)- | N | 2-OMe-5-Me-Ph |
| 1-83 | 2-$CH_2$OH-6-Me-Ph | -NH-C(=O)- | N | 2-OEt-5-Me-Ph |
| 1-84 | 2-$CH_2$OH-6-Me-Ph | -NH-C(=O)- | N | 2-OEt-Ph |
| 1-85 | 2-OMe-6-Me-Ph | -NH-C(=O)- | CH | 5-F-2-OMe-Ph |
| 1-86 | 2-OMe-6-Me-Ph | -NH-C(=O)- | CH | 2-OEt-5-F-Ph |
| 1-87 | 2-OMe-6-Me-Ph | -NH-C(=O)- | CH | 5-Cl-2-OMe-Ph |
| 1-88 | 2-OMe-6-Me-Ph | -NH-C(=O)- | CH | 5-Cl-2-OEt-Ph |
| 1-89 | 2-OMe-6-Me-Ph | -NH-C(=O)- | CH | 2-OMe-5-Me-Ph |
| 1-90 | 2-OMe-6-Me-Ph | -NH-C(=O)- | CH | 2-OEt-5-Me-Ph |
| 1-91 | 2-OMe-6-Me-Ph | -NH-C(=O)- | CH | 2-OEt-Ph |
| 1-92 | 2-OMe-6-Me-Ph | -NH-C(=O)- | N | 5-F-2-OMe-Ph |
| 1-93 | 2-OMe-6-Me-Ph | -NH-C(=O)- | N | 2-OEt-5-F-Ph |
| 1-94 | 2-OMe-6-Me-Ph | -NH-C(=O)- | N | 5-Cl-2-OMe-Pb |
| 1-95 | 2-OMe-6-Me-Ph | -NH-C(=O)- | N | 5-Cl-2-OEt-Ph |
| 1-96 | 2-OMe-6-Me-Ph | -NH-C(=O)- | N | 2-OMe-5-Me-Ph |
| 1-97 | 2-OMe-6-Me-Ph | -NH-C(=O)- | N | 2-Oet-5-Me-Ph |
| 1-98 | 2-OMe-6-Me-Ph | -NH-C(=O)- | N | 2-Oet-Ph |
| 1-99 | 4-F-2-$CF_3$-Ph | -NH-C(=O)- | CH | 5-F-2-OMe-Ph |
| 1-100 | 4-F-2-$CF_3$-Ph | -NH-C(=O)- | CH | 2-Oet-5-F-Ph |
| 1-101 | 4-F-2-$CF_3$-Ph | -NH-C(=O)- | CH | 5-Cl-2-OMe-Ph |
| 1-102 | 4-F-2-$CF_3$-Ph | -NH-C(=O)- | CH | 5-Cl-2-OEt-Ph |
| 1-103 | 4-F-2-$CF_3$-Ph | -NH-C(=O)- | CH | 2-OMe-5-Me-Ph |
| 1-104 | 4-F-2-$CF_3$-Ph | -NH-C(=O)- | CH | 2-Oet-5-Me-Ph |
| 1-105 | 4-F-2-$CF_3$-Ph | -NH-C(=O)- | CH | 2-Oet-Ph |
| 1-106 | 4-F-2-$CF_3$-Ph | -NH-C(=O)- | N | 5-F-2-OMe-Ph |
| 1-107 | 4-F-2-$CF_3$-Ph | -NH-C(=O)- | N | 2-Oet-5-F-Ph |
| 1-108 | 4-F-2-$CF_3$-Ph | -NH-C(=O)- | N | 5-Cl-2-OMe-Ph |
| 1-109 | 4-F-2-$CF_3$-Ph | -NH-C(=O)- | N | 5-Cl-2-OEt-Ph |
| 1-110 | 4-F-2-$CF_3$-Ph | -NH-C(=O)- | N | 2-OMe-5-Me-Ph |
| 1-111 | 4-F-2-$CF_3$-Ph | -NH-C(=O)- | N | 2-Oet-5-Me-Ph |
| 1-112 | 4-F-2-$CF_3$-Ph | -NH-C(=O)- | N | 2-Oet-Ph |
| 1-113 | 2-Cl-4-Me-Ph | -NH-C(=O)- | CH | 5-F-2-OMe-Ph |
| 1-114 | 2-Cl-4-Me-Ph | -NH-C(=O)- | CH | 2-Oet-5-F-Ph |
| 1-115 | 2-Cl-4-Me-Ph | -NH-C(=O)- | CH | 5-Cl-2-OMe-Ph |

(continued)

| Compound No. | $R^2$ | A | U | $R^1$ |
|---|---|---|---|---|
| 1-116 | 2-Cl-4-Me-Ph | -NH-C(=O)- | CH | 5-Cl-2-OEt-Ph |
| 1-117 | 2-Cl-4-Me-Ph | -NH-C(=O)- | CH | 2-OMe-5-Me-Ph |
| 1-118 | 2-Cl-4-Me-Ph | -NH-C(=O)- | CH | 2-Oet-5-Me-Ph |
| 1-119 | 2-Cl-4-Me-Ph | -NH-C(=O)- | CH | 2-Oet-Ph |
| 1-120 | 2-Cl-4-Me-Ph | -NH-C(=O)- | N | 5-F-2-OMe-Ph |
| 1-121 | 2-Cl-4-Me-Ph | -NH-C(=O)- | N | 2-Oet-5-F-Ph |
| 1-122 | 2-Cl-4-Me-Ph | -NH-C(=O)- | N | 5-Cl-2-OMe-Ph |
| 1-123 | 2-Cl-4-Me-Ph | -NH-C(=O)- | N | 5-Cl-2-OEt-Ph |
| 1-124 | 2-Cl-4-Me-Ph | -NH-C(=O)- | N | 2-OMe-5-Me-Ph |
| 1-125 | 2-Cl-4-Me-Ph | -NH-C(=O)- | N | 2-Oet-5-Me-Ph |
| 1-126 | 2-Cl-4-Me-Ph | -NH-C(=O)- | N | 2-Oet-Ph |
| 1-127 | 4-COOH-2-Cl-Ph | -NH-C(=O)- | CH | 5-F-2-OMe-Ph |
| 1-128 | 4-COOH-2-Cl-Ph | -NH-C(=O)- | CH | 2-Oet-5-F-Ph |
| 1-129 | 4-COOH-2-Cl-Ph | -NH-C(=O)- | CH | 5-Cl-2-OMe-Ph |
| 1-130 | 4-COOH-2-Cl-Ph | -NH-C(=O)- | CH | 5-Cl-2-OEt-Ph |
| 1-131 | 4-COOH-2-Cl-Ph | -NH-C(=O)- | CH | 2-OMe-5-Me-Ph |
| 1-132 | 4-COOH-2-Cl-Ph | -NH-C(=O)- | CH | 2-Oet-5-Me-Ph |
| 1-133 | 4-COOH-2-Cl-Ph | -NH-C(=O)- | CH | 2-Oet-Ph |
| 1-134 | 4-COOH-2-Cl-Ph | -NH-C(=O)- | N | 5-F-2-OMe-Ph |
| 1-135 | 4-COOH-2-Cl-Ph | -NH-C(=O)- | N | 2-Oet-5-F-Ph |
| 1-136 | 4-COOH-2-Cl-Ph | -NH-C(=O)- | N | 5-Cl-2-OMe-Ph |
| 1-137 | 4-COOH-2-Cl-Ph | -NH-C(=O)- | N | 5-Cl-2-OEt-Ph |
| 1-138 | 4-COOH-2-Cl-Ph | -NH-C(=O)- | N | 2-OMe-5-Me-Ph |
| 1-139 | 4-COOH-2-Cl-Ph | -NH-C(=O)- | N | 2-Oet-5-Me-Ph |
| 1-140 | 4-COOH-2-Cl-Ph | -NH-C(=O)- | N | 2-Oet-Ph |
| 1-141 | 4-OMe-2-Me-Ph | -NH-C(=O)- | CH | 5-F-2-OMe-Ph |
| 1-142 | 4-OMe-2-Me-Ph | -NH-C(=O)- | CH | 2-Oet-5-F-Ph |
| 1-143 | 4-OMe-2-Me-Ph | -NH-C(=O)- | CH | 5-Cl-2-OMe-Ph |
| 1-144 | 4-OMe-2-Me-Ph | -NH-C(=O)- | CH | 5-Cl-2-OEt-Ph |
| 1-145 | 4-OMe-2-Me-Ph | -NH-C(=O)- | CH | 2-OMe-5-Me-Ph |
| 1-146 | 4-OMe-2-Me-Ph | -NH-C(=O)- | CH | 2-Oet-5-Me-Ph |
| 1-147 | 4-OMe-2-Me-Ph | -NH-C(=O)- | CH | 2-Oet-Ph |
| 1-148 | 4-OMe-2-Me-Ph | -NH-C(=O)- | N | 5-F-2-OMe-Ph |
| 1-149 | 4-OMe-2-Me-Ph | -NH-C(=O)- | N | 2-Oet-5-F-Ph |
| 1-150 | 4-OMe-2-Me-Ph | -NH-C(=O)- | N | 5-Cl-2-OMe-Ph |
| 1-151 | 4-OMe-2-Me-Ph | -NH-C(=O)- | N | 5-Cl-2-OEt-Ph |
| 1-152 | 4-OMe-2-Me-Ph | -NH-C(=O)- | N | 2-OMe-5-Me-Ph |
| 1-153 | 4-OMe-2-Me-Ph | -NH-C(=O)- | N | 2-Oet-5-Me-Ph |
| 1-154 | 4-OMe-2-Me-Ph | -NH-C(=O)- | N | 2-Oet-Ph |
| 1-155 | 4-COOH-2-Me-Ph | -NH-C(=O)- | CH | 5-F-2-OMe-Ph |
| 1-156 | 4-COOH-2-Me-Ph | -NH-C(=O)- | CH | 2-Oet-5-F-Ph |
| 1-157 | 4-COOH-2-Me-Ph | -NH-C(=O)- | CH | 5-Cl-2-OMe-Ph |
| 1-158 | 4-COOH-2-Me-Ph | -NH-C(=O)- | CH | 5-Cl-2-OEt-Ph |
| 1-159 | 4-COOH-2-Me-Ph | -NH-C(=O)- | CH | 2-OMe-5-Me-Ph |
| 1-160 | 4-COOH-2-Me-Ph | -NH-C(=O)- | CH | 2-Oet-5-Me-Ph |
| 1-161 | 4-COOH-2-Me-Ph | -NH-C(=O)- | CH | 2-OEt-Ph |
| 1-162 | 4-COOH-2-Me-Ph | -NH-C(=O)- | N | 5-F-2-OMe-Ph |
| 1-163 | 4-COOH-2-Me-Ph | -NH-C(=O)- | N | 2-OEt-5-F-Ph |
| 1-164 | 4-COOH-2-Me-Ph | -NH-C(=O)- | N | 5-Cl-2-OMe-Ph |
| 1-165 | 4-COOH-2-Me-Ph | -NH-C(=O)- | N | 5-Cl-2-OEt-Ph |

(continued)

| Compound No. | $R^2$ | A | U | $R^1$ |
|---|---|---|---|---|
| 1-166 | 4-COOH-2-Me-Ph | -NH-C(=O)- | N | 2-OMe-5-Me-Ph |
| 1-167 | 4-COOH-2-Me-Ph | -NH-C(=O)- | N | 2-OEt-5-Me-Ph |
| 1-168 | 4-COOH-2-Me-Ph | -NH-C(=O)- | N | 2-OEt-Ph |
| 1-169 | 4-NHCOMe-2-$CF_3$-Ph | -NH-C(=O)- | CH | 5-F-2-OMe-Ph |
| 1-170 | 4-NHCOMe-2-$CF_3$-Ph | -NH-C(=O) | CH | 2-OEt-5-F-Ph |
| 1-171 | 4-NHCOMe-2-$CF_3$-Ph | -NH-C(=O)- | CH | 5-Cl-2-OMe-Ph |
| 1-172 | 4-NHCOMe-2-$CF_3$-Ph | -NH-C(=O)- | CH | 5-Cl-2-OEt-Ph |
| 1-173 | 4-NHCOMe-2-$CF_3$-Ph | -NH-C(=O)- | CH | 2-OMe-5-Me-Ph |
| 1-174 | 4-NHCOMe-2-$CF_3$-Ph | -NH-C(=O)- | CH | 2-OEt-5-Me-Ph |
| 1-175 | 4-NHCOMe-2-$CF_3$-Ph | -NH-C(=O)- | CH | 2-OEt-Ph |
| 1-176 | 4-NHCOMe-2-$CF_3$-Ph | -NH-C(=O)- | N | 5-F-2-OMe-Ph |
| 1-177 | 4-NHCOMe-2-$CF_3$-Ph | -NH-C(=O)- | N | 2-OEt-5-F-Ph |
| 1-178 | 4-NHCOMe-2-$CF_3$-Ph | -NH-C(=O)- | N | 5-Cl-2-OMe-Ph |
| 1-179 | 4-NHCOMe-2-$CF_3$-Ph | -NH-C(=O)- | N | 5-Cl-2-OEt-Ph |
| 1-180 | 4-NHCOMe-2-$CF_3$-Ph | -NH-C(=O)- | N | 2-OMe-5-Me-Ph |
| 1-181 | 4-NHCOMe-2-$CF_3$-Ph | -NH-C(=O)- | N | 2-OEt-5-Me-Ph |
| 1-182 | 4-NHCOMe-2-$CF_3$-Ph | -NH-C(=O)- | N | 2-OEt-Ph |
| 1-183 | 2-Me-Ph | -NH-C(=O)- | CH | 5-F-2-OMe-Ph |
| 1-184 | 2-Me-Ph | -NH-C(=O)- | CH | 2-OEt-5-F-Ph |
| 1-185 | 2-Me-Ph | -NH-C(=O)- | CH | 5-Cl-2-OMe-Ph |
| 1-186 | 2-Me-Ph | -NH-C(=O)- | CH | 5-Cl-2-OEt-Ph |
| 1-187 | 2-Me-Ph | -NH-C(=O)- | CH | 2-OMe-5-Me-Ph |
| 1-188 | 2-Me-Ph | -NH-C(=O)- | CH | 2-OEt-5-Me-Ph |
| 1-189 | 2-Me-Ph | -NH-C(=O)- | CH | 2-OEt-Ph |
| 1-190 | 2-Me-Ph | -NH-C(=O)- | N | 5-F-2-OMe-Ph |
| 1-191 | 2-Me-Ph | -NH-C(=O)- | N | 2-OEt-5-F-Ph |
| 1-192 | 2-Me-Ph | -NH-C(=O)- | N | 5-Cl-2-OMe-Ph |
| 1-193 | 2-Me-Ph | -NH-C(=O)- | N | 5-Cl-2-OEt-Ph |
| 1-194 | 2-Me-Ph | -NH-C(=O)- | N | 2-OMe-5-Me-Ph |
| 1-195 | 2-Me-Ph | -NH-C(=O)- | N | 2-OEt-5-Me-Ph |
| 1-196 | 2-Me-Ph | -NH-C(=O)- | N | 2-OEt-Ph |
| 1-197 | 2-$CF_3$-Ph | -NH-C(=O)- | CH | 5-F-2-OMe-Ph |
| 1-198 | 2-$CF_3$-Ph | -NH-C(=O)- | CH | 2-OEt-5-F-Ph |
| 1-199 | 2-$CF_3$-Ph | -NH-C(=O)- | CH | 5-Cl-2-OMe-Ph |
| 1-200 | 2-$CF_3$-Ph | -NH-C(=O)- | CH | 5-Cl-2-OEt-Ph |
| 1-201 | 2-$CF_3$-Ph | -NH-C(=O)- | CH | 2-OMe-5-Me-Ph |
| 1-202 | 2-$CF_3$-Ph | -NH-C(=O)- | CH | 2-OEt-5-Me-Ph |
| 1-203 | 2-$CF_3$-Ph | -NH-C(=O)- | CH | 2-OEt-Ph |
| 1-204 | 2-$CF_3$-Ph | -NH-C(=O)- | N | 5-F-2-OMe-Ph |
| 1-205 | 2-$CF_3$-Ph | -NH-C(=O)- | N | 2-OEt-5-F-Ph |
| 1-206 | 2-$CF_3$-Ph | -NH-C(=O)- | N | 5-Cl-2-OMe-Ph |
| 1-207 | 2-$CF_3$-Ph | -NH-C(=O)- | N | 5-Cl-2-OEt-Ph |
| 1-208 | 2-$CF_3$-Ph | -NH-C(=O)- | N | 2-OMe-5-Me-Ph |
| 1-209 | 2-$CF_3$-Ph | -NH-C(=O)- | N | 2-OEt-5-Me-Ph |
| 1-210 | 2-$CF_3$-Ph | -NH-C(=O)- | N | 2-OEt-Ph |
| 1-211 | t-Bu | -O-C(=O)- | CH | 5-F-2-OMe-Ph |
| 1-212 | t-Bu | -O-C(=O)- | CH | 2-OEt-5-F-Ph |
| 1-213 | t-Bu | -O-C(=O)- | CH | 5-Cl-2-OMe-Ph |
| 1-214 | t-Bu | -O-C(=O)- | CH | 5-Cl-2-OEt-Ph |
| 1-215 | t-Bu | -O-C(=O)- | CH | 2-OMe-5-Me-Ph |

(continued)

| Compound No. | $R^2$ | A | U | $R^1$ |
|---|---|---|---|---|
| 1-216 | t-Bu | -O-C(=O)- | CH | 2-OEt-5-Me-Ph |
| 1-217 | t-Bu | -O-C(=O)- | CH | 2-OEt-Ph |
| 1-218 | t-Bu | -O-C(=O)- | N | 5-F-2-OMe-Ph |
| 1-219 | t-Bu | -O-C(=O)- | N | 2-OEt-5-F-Ph |
| 1-220 | t-Bu | -O-C(=O)- | N | 5-Cl-2-OMe-Ph |
| 1-221 | t-Bu | -O-C(=O)- | N | 5-Cl-2-OEt-Ph |
| 1-222 | t-Bu | -O-C(=O)- | N | 2-OMe-5-Me-Ph |
| 1-223 | t-Bu | -O-C(=O)- | N | 2-OEt-5-Me-Ph |
| 1-224 | t-Bu | -O-C(=O)- | N | 2-OEt-Ph |
| 1-225 | t-Bu | -O-C(=O)- | CH | 3-OPh-Ph |
| 1-226 | t-Bu | -O-C(=O)- | CH | 4-On-Hept-Ph |
| 1-227 | t-Bu | -O-C(=O)- | CH | 2-$NO_2$-Ph |
| 1-228 | t-Bu | -O-C(=O)- | CH | 2-OPh-Ph |
| 1-229 | t-Bu | -O-C(=O)- | CH | 5-Cl-2-OPh-Ph |
| 1-230 | t-Bu | -O-C(=O)- | CH | 2-$OCF_2H$-Ph |
| 1-231 | t-Bu | -O-C(=O)- | CH | 5-Cl-2-$NO_2$-Ph |
| 1-232 | t-Bu | -O-C(=O)- | CH | 2-$NH_2$-5-Cl-Ph |
| 1-233 | t-Bu | -O-C(=O)- | CH | 5-Cl-2-$NHSO_2Me$-Ph |
| 1-234 | t-Bu | -O-C(=O)- | CH | 2-OMe-Ph |
| 1-235 | t-Bu | -O-C(=O)- | CH | 2-F-Ph |
| 1-236 | t-Bu | -O-C(=O)- | CH | 2-Cl-Ph |
| 1-237 | t-Bu | -O-C(=O)- | CH | 2-Me-Ph |
| 1-238 | t-Bu | -O-C(=O)- | CH | 2-Et-Ph |
| 1-239 | t-Bu | -O-C(=O)- | CH | 2-n-Pr-Ph |
| 1-240 | t-Bu | -O-C(=O)- | CH | 2,5-di-Me-Ph |
| 1-241 | t-Bu | -O-C(=O)- | CH | 4-$NMe_2$-Ph |
| 1-242 | t-Bu | -O-C(=O)- | CH | 2-s-Bu-Ph |
| 1-243 | t-Bu | -O-C(=O)- | CH | 4-i-Pr-Ph |
| 1-244 | t-Bu | -O-C(=O)- | CH | 4-OEt-Ph |
| 1-245 | t-Bu | -O-C(=O)- | CH | 4-Et-Ph |
| 1-246 | t-Bu | -O-C(=O)- | CH | 4-s-Bu-Ph |
| 1-247 | t-Bu | -O-C(=O)- | CH | 4-On-Bu-Ph |
| 1-248 | t-Bu | -O-C(=O)- | CH | 2-F-5-$CF_3$-Ph |
| 1-249 | t-Bu | -O-C(=O)- | CH | 2-Cl-5-$CF_3$-Ph |
| 1-250 | t-Bu | -O-C(=O)- | CH | 2-Cl-5-Me-Ph |
| 1-251 | t-Bu | -O-C(=O)- | CH | 3-Et-Ph |
| 1-252 | t-Bu | -O-C(=O)- | CH | 3-Me-Ph |
| 1-253 | t-Bu | -O-C(=O)- | CH | 3-Cl-Ph |
| 1-254 | t-Bu | -O-C(=O)- | CH | 4-t-Bu-Ph |
| 1-255 | t-Bu | -O-C(=O)- | CH | 4-n-Bu-Ph |
| 1-256 | t-Bu | -O-C(=O)- | CH | 5-Cl-2-Me-Ph |
| 1-257 | t-Bu | -O-C(=O)- | CH | 5-F-2-Me-Ph |
| 1-258 | t-Bu | -O-C(=O)- | CH | 2-F-5-Me-Ph |
| 1-259 | Ph | -O-C(=O)- | CH | 2-OMe-5-Me-Ph |
| 1-260 | CycHex | -C(=O)- | CH | 2-OMe-5-Me-Ph |
| 1-261 | Ph | -NH-C(=O)- | CH | 5-F-2-OMe-Ph |
| 1-262 | Ph | -NH-C(=O)- | CH | 2-OMe-5-Me-Ph |
| 1-263 | Ph | -NH-C(=O)- | CH | 2-OEt-Ph |
| 1-264 | Ph | -NH-C(=O)- | N | 5-F-2-OMe-Ph |
| 1-265 | Ph | -NH-C(=O)- | N | 2-OMe-5-Me-Ph |

(continued)

| Compound No. | $R^2$ | A | U | $R^1$ |
|---|---|---|---|---|
| 1-266 | Ph | -NH-C(=O)- | N | 2-OEt-Ph |
| 1-267 | Bn | -NH-C(=O)- | CH | 5-F-2-OMe-Ph |
| 1-268 | Bn | -NH-C(=O)- | CH | 2-OMe-5-Me-Ph |
| 1-269 | Bn | -NH-C(=O)- | CH | 2-OEt-Ph |
| 1-270 | Bn | -NH-C(=O)- | N | 5-F-2-OMe-Ph |
| 1-271 | Bn | -NH-C(=O)- | N | 2-OMe-5-Me-Ph |
| 1-272 | Bn | -NH-C(=O)- | N | 2-OEt-Ph |
| 1-273 | H | -NH-C(=O)- | CH | 2-OMe-5-Me-Ph |
| 1-274 | 2-F-Ph | -NH-C(=O)- | CH | 5-F-2-OMe-Ph |
| 1-275 | 2-F-Ph | -NH-C(=O)- | CH | 2-OMe-5-Me-Ph |
| 1-276 | 2-F-Ph | -NH-C(=O)- | CH | 2-OEt-Ph |
| 1-277 | 2-F-Ph | -NH-C(=O)- | N | 5-F-2-OMe-Ph |
| 1-278 | 2-F-Ph | -NH-C(=O)- | N | 2-OMe-5-Me-Ph |
| 1-279 | 2-F-Ph | -NH-C(=O)- | N | 2-OEt-Ph |
| 1-280 | 2-Cl-Ph | -NH-C(=O)- | CH | 5-F-2-OMe-Ph |
| 1-281 | 2-Cl-Ph | -NH-C(=O)- | CH | 2-OMe-5-Me-Ph |
| 1-282 | 2-Cl-Ph | -NH-C(=O)- | CH | 2-OEt-Ph |
| 1-283 | 2-Cl-Ph | -NH-C(=O)- | N | 5-F-2-OMe-Ph |
| 1-284 | 2-Cl-Ph | -NH-C(=O)- | N | 2-OMe-5-Me-Ph |
| 1-285 | 2-Cl-Ph | -NH-C(=O)- | N | 2-OEt-Ph |
| 1-286 | 2-Br-Ph | -NH-C(=O)- | CH | 5-F-2-OMe-Ph |
| 1-287 | 2-Br-Ph | -NH-C(=O)- | CH | 2-OMe-5-Me-Ph |
| 1-288 | 2-Br-Ph | -NH-C(=O)- | CH | 2-OEt-Ph |
| 1-289 | 2-Br-Ph | -NH-C(=O)- | N | 5-F-2-OMe-Ph |
| 1-290 | 2-Br-Ph | -NH-C(=O)- | N | 2-OMe-5-Me-Ph |
| 1-291 | 2-Br-Ph | -NH-C(=O)- | N | 2-OEt-Ph |
| 1-292 | 2-Et-Ph | -NH-C(=O)- | CH | 5-F-2-OMe-Ph |
| 1-293 | 2-Et-Ph | -NH-C(=O)- | CH | 2-OMe-5-Me-Ph |
| 1-294 | 2-Et-Ph | -NH-C(=O)- | CH | 2-OEt-Ph |
| 1-295 | 2-Et-Ph | -NH-C(=O)- | N | 5-F-2-OMe-Ph |
| 1-296 | 2-Et-Ph | -NH-C(=O)- | N | 2-OMe-5-Me-Ph |
| 1-297 | 2-Et-Ph | -NH-C(=O)- | N | 2-OEt-Ph |
| 1-298 | 2-Me-6-$NO_2$-Ph | -NH-C(=O)- | CH | 5-F-2-OMe-Ph |
| 1-299 | 2-Me-6-$NO_2$-Ph | -NH-C(=O)- | CH | 2-OMe-5-Me-Ph |
| 1-300 | 2-Me-6-$NO_2$-Ph | -NH-C(=O)- | CH | 2-OEt-Ph |
| 1-301 | 2-Me-6-$NO_2$-Ph | -NH-C(=O)- | N | 5-F-2-OMe-Ph |
| 1-302 | 2-Me-6-$NO_2$-Ph | -NH-C(=O)- | N | 2-OMe-5-Me-Ph |
| 1-303 | 2-Me-6-$NO_2$-Ph | -NH-C(=O)- | N | 2-OEt-Ph |
| 1-304 | 2-CN-Ph | -NH-C(=O)- | CH | 5-F-2-OMe-Ph |
| 1-305 | 2-CN-Ph | -NH-C(=O)- | CH | 2-OMe-5-Me-Ph |
| 1-306 | 2-CN-Ph | -NH-C(=O)- | CH | 2-OEt-Ph |
| 1-307 | 2-CN-Ph | -NH-C(=O)- | N | 5-F-2-OMe-Ph |
| 1-308 | 2-CN-Ph | -NH-C(=O)- | N | 2-OMe-5-Me-Ph |
| 1-309 | 2-CN-Ph | -NH-C(=O)- | N | 2-OEt-Ph |
| 1-310 | 2-Et-6-Me-Ph | -NH-C(=O)- | CH | 5-F-2-OMe-Ph |
| 1-311 | 2-Et-6-Me-Ph | -NH-C(=O)- | CH | 2-OMe-5-Me-Ph |
| 1-312 | 2-Et-6-Me-Ph | -NH-C(=O)- | CH | 2-OEt-Ph |
| 1-313 | 2-Et-6-Me-Ph | -NH-C(=O)- | N | 5-F-2-OMe-Ph |
| 1-314 | 2-Et-6-Me-Ph | -NH-C(=O)- | N | 2-OMe-5-Me-Ph |
| 1-315 | 2-Et-6-Me-Ph | -NH-C(=O)- | N | 2-OEt-Ph |

(continued)

| Compound No. | $R^2$ | A | U | $R^1$ |
|---|---|---|---|---|
| 1-316 | 2-Cl-6-$CF_3$-Ph | -NH-C(=O)- | CH | 5-F-2-OMe-Ph |
| 1-317 | 2-Cl-6-$CF_3$-Ph | -NH-C(=O)- | CH | 2-OMe-5-Me-Ph |
| 1-318 | 2-Cl-6-$CF_3$-Ph | -NH-C(=O)- | CH | 2-OEt-Ph |
| 1-319 | 2-Cl-6-$CF_3$-Ph | -NH-C(=O)- | N | 5-F-2-OMe-Ph |
| 1-320 | 2-Cl-6-$CF_3$-Ph | -NH-C(=O)- | N | 2-OMe-5-Me-Ph |
| 1-321 | 2-Cl-6-$CF_3$-Ph | -NH-C(=O)- | N | 2-OEt-Ph |
| 1-322 | 2-F-Ph | -NH-C(=O)- | CH | 5-Cl-2-OMe-Ph |
| 1-323 | 4-Br-2-Me-Ph | -NH-C(=O)- | CH | 5-F-2-OMe-Ph |
| 1-324 | 4-Br-2-Me-Ph | -NH-C(=O)- | CH | 2-OMe-5-Me-Ph |
| 1-325 | 4-Br-2-Me-Ph | -NH-C(=O)- | CH | 2-OEt-Ph |
| 1-326 | 4-Br-2-Me-Ph | -NH-C(=O)- | N | 5-F-2-OMe-Ph |
| 1-327 | 4-Br-2-Me-Ph | -NH-C(=O)- | N | 2-OMe-5-Me-Ph |
| 1-328 | 4-Br-2-Me-Ph | -NH-C(=O)- | N | 2-OEt-Ph |
| 1-329 | 2,4-di-Me-Ph | -NH-C(=O)- | CH | 5-F-2-OMe-Ph |
| 1-330 | 2,4-di-Me-Ph | -NH-C(=O)- | CH | 2-OMe-5-Me-Ph |
| 1-331 | 2,4-di-Me-Ph | -NH-C(=O)- | CH | 2-OEt-Ph |
| 1-332 | 2,4-di-Me-Ph | -NH-C(=O)- | N | 5-F-2-OMe-Ph |
| 1-333 | 2,4-di-Me-Ph | -NH-C(=O)- | N | 2-OMe-5-Me-Ph |
| 1-334 | 2,4-di-Me-Ph | -NH-C(=O)- | N | 2-OEt-Ph |
| 1-335 | 2,4-di-F-Ph | -NH-C(=O)- | CH | 5-F-2-OMe-Ph |
| 1-336 | 2,4-di-F-Ph | -NH-C(=O)- | CH | 2-OMe-5-Me-Ph |
| 1-337 | 2,4-di-F-Ph | -NH-C(=O)- | CH | 2-OEt-Ph |
| 1-338 | 2,4-di-F-Ph | -NH-C(=O)- | N | 5-F-2-OMe-Ph |
| 1-339 | 2,4-di-F-Ph | -NH-C(=O)- | N | 2-OMe-5-Me-Ph |
| 1-340 | 2,4-di-F-Ph | -NH-C(=O)- | N | 2-OEt-Ph |
| 1-341 | 4-Cl-2-Me-Ph | -NH-C(=O)- | CH | 5-F-2-OMe-Ph |
| 1-342 | 4-Cl-2-Me-Ph | -NH-C(=O)- | CH | 2-OMe-5-Me-Ph |
| 1-343 | 4-Cl-2-Me-Ph | -NH-C(=O)- | CH | 2-OEt-Ph |
| 1-344 | 4-Cl-2-Me-Ph | -NH-C(=O)- | N | 5-F-2-OMe-Ph |
| 1-345 | 4-Cl-2-Me-Ph | -NH-C(=O)- | N | 2-OMe-5-Me-Ph |
| 1-346 | 4-Cl-2-Me-Ph | -NH-C(=O)- | N | 2-OEt-Ph |
| 1-347 | 4-n-Bu-2-Me-Ph | -NH-C(=O)- | CH | 5-F-2-OMe-Ph |
| 1-348 | 4-n-Bu-2-Me-Ph | -NH-C(=O)- | CH | 2-OMe-5-Me-Ph |
| 1-349 | 4-n-Bu-2-Me-Ph | -NH-C(=O)- | CH | 2-OEt-Ph |
| 1-350 | 4-n-Bu-2-Me-Ph | -NH-C(=O)- | N | 5-F-2-OMe-Ph |
| 1-351 | 4-n-Bu-2-Me-Ph | -NH-C(=O)- | N | 2-OMe-5-Me-Ph |
| 1-352 | 4-n-Bu-2-Me-Ph | -NH-C(=O)- | N | 2-OEt-Ph |
| 1-353 | 2-Cl-Ph | -NH-C(=O)- | CH | 5-Cl-2-OMe-Ph |
| 1-354 | 4-Cl-2-$CF_3$-Ph | -NH-C(=O)- | CH | 5-F-2-OMe-Ph |
| 1-355 | 4-Cl-2-$CF_3$-Ph | -NH-C(=O)- | CH | 2-OMe-5-Me-Ph |
| 1-356 | 4-Cl-2-$CF_3$-Ph | -NH-C(=O)- | CH | 2-OEt-Ph |
| 1-357 | 4-Cl-2-$CF_3$-Ph | -NH-C(=O)- | N | 5-F-2-OMe-Ph |
| 1-358 | 4-Cl-2-$CF_3$-Ph | -NH-C(=O)- | N | 2-OMe-5-Me-Ph |
| 1-359 | 4-Cl-2-$CF_3$-Ph | -NH-C(=O)- | N | 2-OEt-Ph |
| 1-360 | 2,4,6-tri-Me-Ph | -NH-C(=O)- | CH | 5-F-2-OMe-Ph |
| 1-361 | 2,4,6-tri-Me-Ph | -NH-C(=O)- | CH | 2-OMe-5-Me-Ph |
| 1-362 | 2,4,6-tri-Me-Ph | -NH-C(=O)- | CH | 2-OEt-Ph |
| 1-363 | 2,4,6-tri-Me-Ph | -NH-C(=O)- | N | 5-F-2-OMe-Ph |
| 1-364 | 2,4,6-tri-Me-Ph | -NH-C(=O)- | N | 2-OMe-5-Me-Ph |
| 1-365 | 2,4,6-tri-Me-Ph | -NH-C(=O)- | N | 2-OEt-Ph |

(continued)

| Compound No. | $R^2$ | A | U | $R^1$ |
|---|---|---|---|---|
| 1-366 | 2-F-$C_6H_4$-$CH_2$ | -NH-C(=O)- | CH | 5-F-2-OMe-Ph |
| 1-367 | 2-F-$C_6H_4$-$CH_2$ | -NH-C(=O)- | CH | 2-OMe-5-Me-Ph |
| 1-368 | 2-F-$C_6H_4$-$CH_2$ | -NH-C(=O)- | CH | 2-OEt-Ph |
| 1-369 | 2-F-$C_6H_4$-$CH_2$ | -NH-C(=O)- | N | 5-F-2-OMe-Ph |
| 1-370 | 2-F-$C_6H_4$-$CH_2$ | -NH-C(=O)- | N | 2-OMe-5-Me-Ph |
| 1-371 | 2-F-$C_6H_4$-$CH_2$ | -NH-C(=O)- | N | 2-OEt-Ph |
| 1-372 | 2,3-di-Me-Ph | -NH-C(=O)- | CH | 5-F-2-OMe-Ph |
| 1-373 | 2,3-di-Me-Ph | -NH-C(=O)- | CH | 2-OMe-5-Me-Ph |
| 1-374 | 2,3-di-Me-Ph | -NH-C(=O)- | CH | 2-OEt-Ph |
| 1-375 | 2,3-di-Me-Ph | -NH-C(=O)- | N | 5-F-2-OMe-Ph |
| 1-376 | 2,3-di-Me-Ph | -NH-C(=O)- | N | 2-OMe-5-Me-Ph |
| 1-377 | 2,3-di-Me-Ph | -NH-C(=O)- | N | 2-OEt-Ph |
| 1-378 | 2-Cl-6-Me-Ph | -NH-C(=O)- | CH | 2-OMe-Ph |
| 1-379 | 2-Cl-6-Me-Ph | -NH-C(=O)- | CH | 2-F-Ph |
| 1-380 | 2-Cl-6-Me-Ph | -NH-C(=O)- | CH | 2-Cl-Ph |
| 1-381 | 2-Cl-6-Me-Ph | -NH-C(=O)- | CH | 2-Me-Ph |
| 1-382 | 2-Cl-6-Me-Ph | -NH-C(=O)- | CH | 2-Et-Ph |
| 1-383 | 4-Br-2-Cl-Ph | -NH-C(=O)- | CH | 5-F-2-OMe-Ph |
| 1-384 | 4-Br-2-Cl-Ph | -NH-C(=O)- | CH | 2-OMe-5-Me-Ph |
| 1-385 | 4-Br-2-Cl-Ph | -NH-C(=O)- | CH | 2-OEt-Ph |
| 1-386 | 4-Br-2-Cl-Ph | -NH-C(=O)- | N | 5-F-2-OMe-Ph |
| 1-387 | 4-Br-2-Cl-Ph | -NH-C(=O)- | N | 2-OMe-5-Me-Ph |
| 1-388 | 4-Br-2-Cl-Ph | -NH-C(=O)- | N | 2-OEt-Ph |
| 1-389 | 2-Cl-6-Me-Ph | -NH-C(=O)- | CH | 2-n-Pr-Ph |
| 1-390 | 2-Cl-6-Me-Ph | -NH-C(=O)- | CH | 2,5-di-Me-Ph |
| 1-391 | 3-Cl-2-Me-Ph | -NH-C(=O)- | CH | 5-F-2-OMe-Ph |
| 1-392 | 3-Cl-2-Me-Ph | -NH-C(=O)- | CH | 2-OMe-5-Me-Ph |
| 1-393 | 3-Cl-2-Me-Ph | -NH-C(=O)- | CH | 2-OEt-Ph |
| 1-394 | 3-Cl-2-Me-Ph | -NH-C(=O)- | N | 5-F-2-OMe-Ph |
| 1-395 | 3-Cl-2-Me-Ph | -NH-C(=O)- | N | 2-OMe-5-Me-Ph |
| 1-396 | 3-Cl-2-Me-Ph | -NH-C(=O)- | N | 2-OEt-Ph |
| 1-397 | 2-Cl-6-Me-Ph | -NH-C(=O)- | CH | 4-$NMe_2$-Ph |
| 1-398 | 2-Cl-6-Me-Ph | -NH-C(=O)- | CH | 2-s-Bu-Ph |
| 1-399 | 2-Cl-6-Me-Ph | -NH-C(=O)- | CH | 4-i-Pr-Ph |
| 1-400 | 2-Cl-6-Me-Ph | -NH-C(=O)- | CH | 4-OEt-Ph |
| 1-401 | 2-Cl-6-Me-Ph | -NH-C(=O)- | CH | 4-Et-Ph |
| 1-402 | 2-Cl-6-Me-Ph | -NH-C(=O)- | CH | 4-s-Bu-Ph |
| 1-403 | 2-Cl-6-Me-Ph | -NH-C(=O)- | CH | 4-On-Bu-Ph |
| 1-404 | 2-Cl-6-Me-Ph | -NH-C(=O)- | CH | 2-F-5-$CF_3$-Ph |
| 1-405 | 2-Cl-6-Me-Ph | -NH-C(=O)- | CH | 2-Cl-5-$CF_3$-Ph |
| 1-406 | 2-Cl-6-Me-Ph | -NH-C(=O)- | CH | 2-Cl-5-Me-Ph |
| 1-407 | 4-$NH_2$-2-$CF_3$-Ph | -NH-C(=O)- | CH | 5-F-2-OMe-Ph |
| 1-408 | 4-$NH_2$-2-$CF_3$-Ph | -NH-C(=O)- | CH | 2-OMe-5-Me-Ph |
| 1-409 | 4-$NH_2$-2-$CF_3$-Ph | -NH-C(=O)- | CH | 2-OEt-Ph |
| 1-410 | 4-$NH_2$-2-$CF_3$-Ph | -NH-C(=O)- | N | 5-F-2-OMe-Ph |
| 1-411 | 4-$NH_2$-2-$CF_3$-Ph | -NH-C(=O)- | N | 2-OMe-5-Me-Ph |
| 1-412 | 4-$NH_2$-2-$CF_3$-Ph | -NH-C(=O)- | N | 2-OEt-Ph |
| 1-413 | 2-$NH_2$-6-Me-Ph | -NH-C(=O)- | CH | 5-F-2-OMe-Ph |
| 1-414 | 2-$NH_2$-6-Me-Ph | -NH-C(=O)- | CH | 2-OMe-5-Me-Ph |
| 1-415 | 2-$NH_2$-6-Me-Ph | -NH-C(=O)- | CH | 2-OEt-Ph |

(continued)

| Compound No. | $R^2$ | A | U | $R^1$ |
|---|---|---|---|---|
| 1-416 | 2-$NH_2$-6-Me-Ph | -NH-C(=O)- | N | 5-F-2-OMe-Ph |
| 1-417 | 2-$NH_2$-6-Me-Ph | -NH-C(=O)- | N | 2-OMe-5-Me-Ph |
| 1-418 | 2-$NH_2$-6-Me-Ph | -NH-C(=O)- | N | 2-OEt-Ph |
| 1-419 | 4-$NH_2$-2-Cl-Ph | -NH-C(=O)- | CH | 5-F-2-OMe-Ph |
| 1-420 | 4-$NH_2$-2-Cl-Ph | -NH-C(=O)- | CH | 2-OMe-5-Me-Ph |
| 1-421 | 4-$NH_2$-2-Cl-Ph | -NH-C(=O)- | CH | 2-OEt-Ph |
| 1-422 | 4-$NH_2$-2-Cl-Ph | -NH-C(=O)- | N | 5-F-2-OMe-Ph |
| 1-423 | 4-$NH_2$-2-Cl-Ph | -NH-C(=O)- | N | 2-OMe-5-Me-Ph |
| 1-424 | 4-$NH_2$-2-Cl-Ph | -NH-C(=O)- | N | 2-OEt-Ph |
| 1-425 | 4-NHCOMe-2-Cl-Ph | -NH-C(=O)- | CH | 5-F-2-OMe-Ph |
| 1-426 | 4-NHCOMe-2-Cl-Ph | -NH-C(=O)- | CH | 2-OMe-5-Me-Ph |
| 1-427 | 4-NHCOMe-2-Cl-Ph | -NH-C(=O)- | CH | 2-OEt-Ph |
| 1-428 | 4-NHCOMe-2-Cl-Ph | -NH-C(=O)- | N | 5-F-2-OMe-Ph |
| 1-429 | 4-NHCOMe-2-Cl-Ph | -NH-C(=O)- | N | 2-OMe-5-Me-Ph |
| 1-430 | 4-NHCOMe-2-Cl-Ph | -NH-C(=O)- | N | 2-OEt-Ph |
| 1-431 | 4-Br-2-CN-Ph | -NH-C(=O)- | CH | 5-F-2-OMe-Ph |
| 1-432 | 4-Br-2-CN-Ph | -NH-C(=O)- | CH | 2-OMe-5-Me-Ph |
| 1-433 | 4-Br-2-CN-Ph | -NH-C(=O)- | CH | 2-OEt-Ph |
| 1-434 | 4-Br-2-CN-Ph | -NH-C(=O)- | N | 5-F-2-OMe-Ph |
| 1-435 | 4-Br-2-CN-Ph | -NH-C(=O)- | N | 2-OMe-5-Me-Ph |
| 1-436 | 4-Br-2-CN-Ph | -NH-C(=O)- | N | 2-OEt-Ph |
| 1-437 | 4-F-2-Me-Ph | -NH-C(=O)- | CH | 5-F-2-OMe-Ph |
| 1-438 | 4-F-2-Me-Ph | -NH-C(=O)- | CH | 2-OMe-5-Me-Ph |
| 1-439 | 4-F-2-Me-Ph | -NH-C(=O)- | CH | 2-OEt-Ph |
| 1-440 | 4-F-2-Me-Ph | -NH-C(=O)- | N | 5-F-2-OMe-Ph |
| 1-441 | 4-F-2-Me-Ph | -NH-C(=O)- | N | 2-OMe-5-Me-Ph |
| 1-442 | 4-F-2-Me-Ph | -NH-C(=O)- | N | 2-OEt-Ph |
| 1-443 | 4-NHCOEt-2-$CF_3$-Ph | -NH-C(=O)- | CH | 5-F-2-OMe-Ph |
| 1-444 | 4-NHCOEt-2-$CF_3$-Ph | -NH-C(=O)- | CH | 2-OMe-5-Me-Ph |
| 1-445 | 4-NHCOEt-2-$CF_3$-Ph | -NH-C(=O)- | CH | 2-OEt-Ph |
| 1-446 | 4-NHCOEt-2-$CF_3$-Ph | -NH-C(=O)- | N | 5-F-2-OMe-Ph |
| 1-447 | 4-NHCOEt-2-$CF_3$-Ph | -NH-C(=O)- | N | 2-OMe-5-Me-Ph |
| 1-448 | 4-NHCOEt-2-$CF_3$-Ph | -NH-C(=O)- | N | 2-OEt-Ph |
| 1-449 | 4-Cl-2,6-di-Me-Ph | -NH-C(=O)- | CH | 5-F-2-OMe-Ph |
| 1-450 | 4-Cl-2,6-di-Me-Ph | -NH-C(=O)- | CH | 2-OMe-5-Me-Ph |
| 1-451 | 4-Cl-2,6-di-Me-Ph | -NH-C(=O)- | CH | 2-OEt-Ph |
| 1-452 | 4-Cl-2,6-di-Me-Ph | -NH-C(=O)- | N | 5-F-2-OMe-Ph |
| 1-453 | 4-Cl-2,6-di-Me-Ph | -NH-C(=O)- | N | 2-OMe-5-Me-Ph |
| 1-454 | 4-Cl-2,6-di-Me-Ph | -NH-C(=O)- | N | 2-OEt-Ph |
| 1-455 | 4-OMe-2-$CF_3$-Ph | -NH-C(=O)- | CH | 5-F-2-OMe-Ph |
| 1-456 | 4-OMe-2-$CF_3$-Ph | -NH-C(=O)- | CH | 2-OMe-5-Me-Ph |
| 1-457 | 4-OMe-2-$CF_3$-Ph | -NH-C(=O)- | CH | 2-OEt-Ph |
| 1-458 | 4-OMe-2-$CF_3$-Ph | -NH-C(=O)- | N | 5-F-2-OMe-Ph |
| 1-459 | 4-OMe-2-$CF_3$-Ph | -NH-C(=O)- | N | 2-OMe-5-Me-Ph |
| 1-460 | 4-OMe-2-$CF_3$-Ph | -NH-C(=O)- | N | 2-OEt-Ph |
| 1-461 | 5-tetra | -NH-C(=O)- | CH | 5-F-2-OMe-Ph |
| 1-462 | 5-tetra | -NH-C(=O)- | CH | 2-OMe-5-Me-Ph |
| 1-463 | 5-tetra | -NH-C(=O)- | CH | 2-OEt-Ph |
| 1-464 | 5-tetra | -NH-C(=O)- | N | 5-F-2-OMe-Ph |
| 1-465 | 5-tetra | -NH-C(=O)- | N | 2-OMe-5-Me-Ph |

(continued)

| Compound No. | $R^2$ | A | U | $R^1$ |
|---|---|---|---|---|
| 1-466 | 5-tetra | -NH-C(=O)- | N | 2-OEt-Ph |
| 1-467 | 2-Br-6-Me-Ph | -NH-C(=O)- | CH | 5-F-2-OMe-Ph |
| 1-468 | 2-Br-6-Me-Ph | -NH-C(=O)- | CH | 2-OMe-5-Me-Ph |
| 1-469 | 2-Br-6-Me-Ph | -NH-C(=O)- | CH | 2-OEt-Ph |
| 1-470 | 2-Br-6-Me-Ph | -NH-C(=O)- | N | 5-F-2-OMe-Ph |
| 1-471 | 2-Br-6-Me-Ph | -NH-C(=O)- | N | 2-OMe-5-Me-Ph |
| 1-472 | 2-Br-6-Me-Ph | -NH-C(=O)- | N | 2-OEt-Ph |
| 1-473 | 4-i-Pr-2-$CF_3$-Ph | -NH-C(=O)- | CH | 5-F-2-OMe-Ph |
| 1-474 | 4-i-Pr-2-$CF_3$-Ph | -NH-C(=O)- | CH | 2-OMe-5-Me-Ph |
| 1-475 | 4-i-Pr-2-$CF_3$-Ph | -NH-C(=O)- | CH | 2-OEt-Ph |
| 1-476 | 4-i-Pr-2-$CF_3$-Ph | -NH-C(=O)- | N | 5-F-2-OMe-Ph |
| 1-477 | 4-i-Pr-2-$CF_3$-Ph | -NH-C(=O)- | N | 2-OMe-5-Me-Ph |
| 1-478 | 4-i-Pr-2-$CF_3$-Ph | -NH-C(=O)- | N | 2-OEt-Ph |
| 1-479 | 2-COOH-6-Me-Ph | -NH-C(=O)- | CH | 5-F-2-OMe-Ph |
| 1-480 | 2-COOH-6-Me-Ph | -NH-C(=O)- | CH | 2-OMe-5-Me-Ph |
| 1-481 | 2-COOH-6-Me-Ph | -NH-C(=O)- | CH | 2-OEt-Ph |
| 1-482 | 2-COOH-6-Me-Ph | -NH-C(=O)- | N | 5-F-2-OMe-Ph |
| 1-483 | 2-COOH-6-Me-Ph | -NH-C(=O)- | N | 2-OMe-5-Me-Ph |
| 1-484 | 2-COOH-6-Me-Ph | -NH-C(=O)- | N | 2-OEt-Ph |
| 1-485 | 2-Bic[2.2.1]hept | -C(=O)- | CH | 2-OMe-5-Me-Ph |
| 1-486 | 2-Me-CycHex | -C(=O)- | CH | 2-OMe-5-Me-Ph |
| 1-487 | Bn | - | CH | 2-OMe-5-Me-Ph |
| 1-488 | 2,6-di-F-$C_6H_3$-$CH_2$ | -C(=O)- | CH | 2-OMe-5-Me-Ph |
| 1-489 | 2-Pyri | - | CH | 2-OMe-5-Me-Ph |
| 1-490 | 2-Cl-6-Me-Ph | -O-C(=O)- | CH | 2-OMe-5-Me-Ph |
| 1-491 | 2-Cl-6-Me-Ph | -NH-C(=O)- | CH | 4-Phos-Ph |
| 1-492 | 2,6-di-F-Ph | -NH-C(=O)- | CH | n-Hex |
| 1-493 | 2-F-6-$CF_3$-Ph | -NH-C(=O)- | CH | n-Hex |
| 1-494 | 2,6-di-Cl-Ph | -NH-C(=O)- | CH | n-Hex |
| 1-495 | 2-Cl-6-Me-Ph | -NH-C(=O)- | CH | n-Hex |
| 1-496 | 2,6-di-Me-Ph | -NH-C(=O)- | CH | n-Hex |
| 1-497 | 2-$CH_2$OH-6-Me-Ph | -NH-C(=O)- | CH | n-Hex |
| 1-498 | 2-OMe-6-Me-Ph | -NH-C(=O)- | CH | n-Hex |
| 1-499 | 4-F-2-$CF_3$-Ph | -NH-C(=O)- | CH | n-Hex |
| 1-500 | 2-Cl-4-Me-Ph | -NH-C(=O)- | CH | n-Hex |
| 1-501 | 4-COOH-2-Cl-Ph | -NH-C(=O)- | CH | n-Hex |
| 1-502 | 4-OMe-2-Me-Ph | -NH-C(=O)- | CH | n-Hex |
| 1-503 | 4-COOH-2-Me-Ph | -NH-C(=O)- | CH | n-Hex |
| 1-504 | 4-NHCOMe-2-$CF_3$-Ph | -NH-C(=O)- | CH | n-Hex |
| 1-505 | 2-Me-Ph | -NH-C(=O)- | CH | n-Hex |
| 1-506 | 2-$CF_3$-Ph | -NH-C(=O)- | CH | n-Hex |
| 1-507 | t-Bu | -O-C(=O)- | CH | n-Hex |
| 1-508 | 2,6-di-F-Ph | -NH-C(=O)- | CH | CycHex |
| 1-509 | 2-F-6-$CF_3$-Ph | -NH-C(=O)- | CH | CycHex |
| 1-510 | 2,6-di-Cl-Ph | -NH-C(=O)- | CH | CycHex |
| 1-511 | 2-Cl-6-Me-Ph | -NH-C(=O)- | CH | CycHex |
| 1-512 | 2,6-di-Me-Ph | -NH-C(=O)- | CH | CycHex |
| 1-513 | 2-$CH_2$OH-6-Me-Ph | -NH-C(=O)- | CH | CycHex |
| 1-514 | 2-OMe-6-Me-Ph | -NH-C(=O)- | CH | CycHex |
| 1-515 | 4-F-2-$CF_3$-Ph | -NH-C(=O)- | CH | CycHex |

(continued)

| Compound No. | $R^2$ | A | U | $R^1$ |
|---|---|---|---|---|
| 1-516 | 2-Cl-4-Me-Ph | -NH-C(=O)- | CH | CycHex |
| 1-517 | 4-COOH-2-Cl-Ph | -NH-C(=O)- | CH | CycHex |
| 1-518 | 4-OMe-2-Me-Ph | -NH-C(=O)- | CH | CycHex |
| 1-519 | 4-COOH-2-Me-Ph | -NH-C(=O)- | CH | CycHex |
| 1-520 | 4-NHCOMe-2-$CF_3$-Ph | -NH-C(=O)- | CH | CycHex |
| 1-521 | 2-Me-Ph | -NH-C(=O)- | CH | CycHex |
| 1-522 | 2-$CF_3$-Ph | -NH-C(=O)- | CH | CycHex |
| 1-523 | t-Bu | -O-C(=O)- | CH | CycHex |
| 1-524 | 3-Me-2-Py | -NH-C(=O)- | CH | 2-F-Ph |
| 1-525 | 3-Me-2-Py | -NH-C(=O)- | CH | 2-OMe-Ph |
| 1-526 | 3-Me-2-Py | -NH-C(=O)- | CH | 2-OEt-Ph |
| 1-527 | 3-Me-2-Py | -NH-C(=O)- | CH | 5-F-2-OMe-Ph |
| 1-528 | 3-Me-2-Py | -NH-C(=O)- | CH | 2-OEt-5-F-Ph |
| 1-529 | 3-Me-2-Py | -NH-C(=O)- | CH | 5-Cl-2-OMe-Ph |
| 1-530 | 3-Me-2-Py | -NH-C(=O)- | CH | 5-Cl-2-OEt-Ph |
| 1-531 | 3-Me-2-Py | -NH-C(=O)- | CH | 2-OMe-5-Me-Ph |
| 1-532 | 3-Me-2-Py | -NH-C(=O)- | CH | 2-OEt-5-Me-Ph |
| 1-533 | 3-Me-2-Py | -NH-C(=O)- | CH | 3,5-di-OMe-Ph |
| 1-534 | 3-Me-2-Py | -NH-C(=O)- | CH | 5-Me-2-thiazo |
| 1-535 | 3-Me-2-Py | -NH-C(=O)- | CH | 4-t-Bu-2-thiazo |
| 1-536 | 3-Me-2-Py | -NH-C(=O)- | N | 2-F-Ph |
| 1-537 | 3-Me-2-Py | -NH-C(=O)- | N | 2-OMe-Ph |
| 1-538 | 3-Me-2-Py | -NH-C(=O)- | N | 2-OEt-Ph |
| 1-539 | 3-Me-2-Py | -NH-C(=O)- | N | 5-F-2-OMe-Ph |
| 1-540 | 3-Me-2-Py | -NH-C(=O)- | N | 2-OEt-5-F-Ph |
| 1-541 | 3-Me-2-Py | -NH-C(=O)- | N | 5-Cl-2-OMe-Ph |
| 1-542 | 3-Me-2-Py | -NH-C(=O)- | N | 5-Cl-2-OEt-Ph |
| 1-543 | 3-Me-2-Py | -NH-C(=O)- | N | 2-OMe-5-Me-Ph |
| 1-544 | 3-Me-2-Py | -NH-C(=O)- | N | 2-OEt-5-Me-Ph |
| 1-545 | 3-Me-2-Py | -NH-C(=O)- | N | 3,5-di-OMe-Ph |
| 1-546 | 3-Me-2-Py | -NH-C(=O)- | N | 5-Me-2-thiazo |
| 1-547 | 3-Me-2-Py | -NH-C(=O)- | N | 4-t-Bu-2-thiazo |
| 1-548 | 2-Cl-4-$OCH_2CH_2OH$-Ph | -NH-C(=O)- | CH | 2-F-Ph |
| 1-549 | 2-Cl-4-$OCH_2CH_2OH$-Ph | -NH-C(=O)- | CH | 2-OMe-Ph |
| 1-550 | 2-Cl-4-$OCH_2CH_2OH$-Ph | -NH-C(=O)- | CH | 2-OEt-Ph |
| 1-551 | 2-Cl-4-$OCH_2CH_2OH$-Ph | -NH-C(=O)- | CH | 5-F-2-OMe-Ph |
| 1-552 | 2-Cl-4-$OCH_2CH_2OH$-Ph | -NH-C(=O)- | CH | 2-OEt-5-F-Ph |
| 1-553 | 2-Cl-4-$OCH_2CH_2OH$-Ph | -NH-C(=O)- | CH | 5-Cl-2-OMe-Ph |
| 1-554 | 2-Cl-4-$OCH_2CH_2OH$-Ph | -NH-C(=O)- | CH | 5-Cl-2-OEt-Ph |
| 1-555 | 2-Cl-4-$OCH_2CH_2OH$-Ph | -NH-C(=O)- | CH | 2-OMe-5-Me-Ph |
| 1-556 | 2-Cl-4-$OCH_2CH_2OH$-Ph | -NH-C(=O)- | CH | 2-OEt-5-Me-Ph |
| 1-557 | 2-Cl-4-$OCH_2CH_2OH$-Ph | -NH-C(=O)- | CH | 3,5-di-OMe-Ph |
| 1-558 | 2-Cl-4-$OCH_2CH_2OH$-Ph | -NH-C(=O)- | CH | 5-Me-2-thiazo |
| 1-559 | 2-Cl-4-$OCH_2CH_2OH$-Ph | -NH-C(=O)- | CH | 4-t-Bu-2-thiazo |
| 1-560 | 2-Cl-4-$OCH_2CH_2OH$-Ph | -NH-C(=O)- | N | 2-F-Ph |
| 1-561 | 2-Cl-4-$OCH_2CH_2OH$-Ph | -NH-C(=O)- | N | 2-OMe-Ph |
| 1-562 | 2-Cl-4-$OCH_2CH_2OH$-Ph | -NH-C(=O)- | N | 2-OEt-Ph |
| 1-563 | 2-Cl-4-$OCH_2CH_2OH$-Ph | -NH-C(=O)- | N | 5-F-2-OMe-Ph |
| 1-564 | 2-Cl-4-$OCH_2CH_2OH$-Ph | -NH-C(=O)- | N | 2-OEt-5-F-Ph |
| 1-565 | 2-Cl-4-$OCH_2CH_2OH$-Ph | -NH-C(=O)- | N | 5-Cl-2-OMe-Ph |

(continued)

| Compound No. | $R^2$ | A | U | $R^1$ |
|---|---|---|---|---|
| 1-566 | 2-Cl-4-$OCH_2CH_2OH$-Ph | -NH-C(=O)- | N | 5-Cl-2-OEt-Ph |
| 1-567 | 2-Cl-4-$OCH_2CH_2OH$-Ph | -NH-C(=O)- | N | 2-OMe-5-Me-Ph |
| 1-568 | 2-Cl-4-$OCH_2CH_2OH$-Ph | -NH-C(=O)- | N | 2-OEt-5-Me-Ph |
| 1-569 | 2-Cl-4-$OCH_2CH_2OH$-Ph | -NH-C(=O)- | N | 3,5-di-OMe-Ph |
| 1-570 | 2-Cl-4-$OCH_2CH_2OH$-Ph | -NH-C(=O)- | N | 5-Me-2-thiazo |
| 1-571 | 2-Cl-4-$OCH_2CH_2OH$-Ph | -NH-C(=O)- | N | 4-t-Bu-2-thiazo |
| 1-572 | Ph(A) | -NH-C(=O)- | CH | 2-F-Ph |
| 1-573 | Ph(A) | -NH-C(=O)- | CH | 2-OMe-Ph |
| 1-574 | Ph(A) | -NH-C(=O)- | CH | 2-OEt-Ph |
| 1-575 | Ph(A) | -NH-C(=O)- | CH | 5-F-2-OMe-Ph |
| 1-576 | Ph(A) | -NH-C(=O)- | CH | 2-OEt-5-F-Ph |
| 1-577 | Ph(A) | -NH-C(=O)- | CH | 5-Cl-2-OMe-Ph |
| 1-578 | Ph(A) | -NH-C(=O)- | CH | 5-Cl-2-OEt-Ph |
| 1-579 | Ph(A) | -NH-C(=O)- | CH | 2-OMe-5-Me-Ph |
| 1-580 | Ph(A) | -NH-C(=O)- | CH | 2-OEt-5-Me-Ph |
| 1-581 | Ph(A) | -NH-C(=O)- | CH | 3,5-di-OMe-Ph |
| 1-582 | Ph(A) | -NH-C(=O)- | CH | 5-Me-2-thiazo |
| 1-583 | Ph(A) | -NH-C(=O)- | CH | 4-t-Bu-2-thiazo |
| 1-584 | Ph(A) | -NH-C(=O)- | N | 2-F-Ph |
| 1-585 | Ph(A) | -NH-C(=O)- | N | 2-OMe-Ph |
| 1-586 | Ph(A) | -NH-C(=O)- | N | 2-OEt-Ph |
| 1-587 | Ph(A) | -NH-C(=O)- | N | 5-F-2-OMe-Ph |
| 1-588 | Ph(A) | -NH-C(=O)- | N | 2-OEt-5-F-Ph |
| 1-589 | Ph(A) | -NH-C(=O)- | N | 5-Cl-2-OMe-Ph |
| 1-590 | Ph(A) | -NH-C(=O)- | N | 5-Cl-2-OEt-Ph |
| 1-591 | Ph(A) | -NH-C(=O)- | N | 2-OMe-5-Me-Ph |
| 1-592 | Ph(A) | -NH-C(=O)- | N | 2-OEt-5-Me-Ph |
| 1-593 | Ph(A) | -NH-C(=O)- | N | 3,5-di-OMe-Ph |
| 1-594 | Ph(A) | -NH-C(=O)- | N | 5-Me-2-thiazo |
| 1-595 | Ph(A) | -NH-C(=O)- | N | 4-t-Bu-2-thiazo |
| 1-596 | 4-$OCH_2CH_2OH$-2-$CF_3$-Ph | -NH-C(=O)- | CH | 2-F-Ph |
| 1-597 | 4-$OCH_2CH_2OH$-2-$CF_3$-Ph | -NH-C(=O)- | CH | 2-OMe-Ph |
| 1-598 | 4-$OCH_2CH_2OH$-2-$CF_3$-Ph | -NH-C(=O)- | CH | 2-OEt-Ph |
| 1-599 | 4-$OCH_2CH_2OH$-2-$CF_3$-Ph | -NH-C(=O)- | CH | 5-F-2-OMe-Ph |
| 1-600 | 4-$OCH_2CH_2OH$-2-$CF_3$-Ph | -NH-C(=O)- | CH | 2-OEt-5-F-Ph |
| 1-601 | 4-$OCH_2CH_2OH$-2-$CF_3$-Ph | -NH-C(=O)- | CH | 5-Cl-2-OMe-Ph |
| 1-602 | 4-$OCH_2CH_2OH$-2-$CF_3$-Ph | -NH-C(=O)- | CH | 5-Cl-2-OEt-Ph |
| 1-603 | 4-$OCH_2CH_2OH$-2-$CF_3$-Ph | -NH-C(=O)- | CH | 2-OMe-5-Me-Ph |
| 1-604 | 4-$OCH_2CH_2OH$-2-$CF_3$-Ph | -NH-C(=O)- | CH | 2-OEt-5-Me-Ph |
| 1-605 | 4-$OCH_2CH_2OH$-2-$CF_3$-Ph | -NH-C(=O)- | CH | 3,5-di-OMe-Ph |
| 1-606 | 4-$OCH_2CH_2OH$-2-$CF_3$-Ph | -NH-C(=O)- | CH | 5-Me-2-thiazo |
| 1-607 | 4-$OCH_2CH_2OH$-2-$CF_3$-Ph | -NH-C(=O)- | CH | 4-t-Bu-2-thiazo |
| 1-608 | 4-$OCH_2CH_2OH$-2-$CF_3$-Ph | -NH-C(=O)- | N | 2-F-Ph |
| 1-609 | 4-$OCH_2CH_2OH$-2-$CF_3$-Ph | -NH-C(=O)- | N | 2-OMe-Ph |
| 1-610 | 4-$OCH_2CH_2OH$-2-$CF_3$-Ph | -NH-C(=O)- | N | 2-OEt-Ph |
| 1-611 | 4-$OCH_2CH_2OH$-2-$CF_3$-Ph | -NH-C(=O)- | N | 5-F-2-OMe-Ph |
| 1-612 | 4-$OCH_2CH_2OH$-2-$CF_3$-Ph | -NH-C(=O)- | N | 2-OEt-5-F-Ph |
| 1-613 | 4-$OCH_2CH_2OH$-2-$CF_3$-Ph | -NH-C(=O)- | N | 5-Cl-2-OMe-Ph |
| 1-614 | 4-$OCH_2CH_2OH$-2-$CF_3$-Ph | -NH-C(=O)- | N | 5-Cl-2-OEt-Ph |
| 1-615 | 4-$OCH_2CH_2OH$-2-$CF_3$-Ph | -NH-C(=O)- | N | 2-OMe-5-Me-Ph |

(continued)

| Compound No. | $R^2$ | A | U | $R^1$ |
|---|---|---|---|---|
| 1-616 | 4-$OCH_2CH_2OH$-2-$CF_3$-Ph | -NH-C(=O)- | N | 2-OEt-5-Me-Ph |
| 1-617 | 4-$OCH_2CH_2OH$-2-$CF_3$-Ph | -NH-C(=O)- | N | 3,5-di-OMe-Ph |
| 1-618 | 4-$OCH_2CH_2OH$-2-$CF_3$-Ph | -NH-C(=O)- | N | 5-Me-2-thiazo |
| 1-619 | 4-$OCH_2CH_2OH$-2-$CF_3$-Ph | -NH-C(=O)- | N | 4-t-Bu-2-thiazo |
| 1-620 | Ph(B) | -NH-C(=O)- | CH | 2-F-Ph |
| 1-621 | Ph(B) | -NH-C(=O)- | CH | 2-OMe-Ph |
| 1-622 | Ph(B) | -NH-C(=O)- | CH | 2-OEt-Ph |
| 1-623 | Ph(B) | -NH-C(=O)- | CH | 5-F-2-OMe-Ph |
| 1-624 | Ph(B) | -NH-C(=O)- | CH | 2-OEt-5-F-Ph |
| 1-625 | Ph(B) | -NH-C(=O)- | CH | 5-Cl-2-OMe-Ph |
| 1-626 | Ph(B) | -NH-C(=O)- | CH | 5-Cl-2-OEt-Ph |
| 1-627 | Ph(B) | -NH-C(=O)- | CH | 2-OMe-5-Me-Ph |
| 1-628 | Ph(B) | -NH-C(=O)- | CH | 2-OEt-5-Me-Ph |
| 1-629 | Ph(B) | -NH-C(=O)- | CH | 3,5-di-OMe-Ph |
| 1-630 | Ph(B) | -NH-C(=O)- | CH | 5-Me-2-thiazo |
| 1-631 | Ph(B) | -NH-C(=O)- | CH | 4-t-Bu-2-thiazo |
| 1-632 | Ph(B) | -NH-C(=O)- | N | 2-F-Ph |
| 1-633 | Ph(B) | -NH-C(=O)- | N | 2-OMe-Ph |
| 1-634 | Ph(B) | -NH-C(=O)- | N | 2-OEt-Ph |
| 1-635 | Ph(B) | -NH-C(=O)- | N | 5-F-2-OMe-Ph |
| 1-636 | Ph(B) | -NH-C(=O)- | N | 2-OEt-5-F-Ph |
| 1-637 | Ph(B) | -NH-C(=O)- | N | 5-Cl-2-OMe-Ph |
| 1-638 | Ph(B) | -NH-C(=O)- | N | 5-Cl-2-OEt-Ph |
| 1-639 | Ph(B) | -NH-C(=O)- | N | 2-OMe-5-Me-Ph |
| 1-640 | Ph(B) | -NH-C(=O)- | N | 2-OEt-5-Me-Ph |
| 1-641 | Ph(B) | -NH-C(=O)- | N | 3,5-di-OMe-Ph |
| 1-642 | Ph(B) | -NH-C(=O)- | N | 5-Me-2-thiazo |
| 1-643 | Ph(B) | -NH-C(=O)- | N | 4-t-Bu-2-thiazo |
| 1-644 | 2-Cl-5-$OCH_2CH_2OH$-Ph | -NH-C(=O)- | CH | 2-F-Ph |
| 1-645 | 2-Cl-5-$OCH_2CH_2OH$-Ph | -NH-C(=O)- | CH | 2-OMe-Ph |
| 1-646 | 2-Cl-5-$OCH_2CH_2OH$-Ph | -NH-C(=O)- | CH | 2-OEt-Ph |
| 1-647 | 2-Cl-5-$OCH_2CH_2OH$-Ph | -NH-C(=O)- | CH | 5-F-2-OMe-Ph |
| 1-648 | 2-Cl-5-$OCH_2CH_2OH$-Ph | -NH-C(=O)- | CH | 2-OEt-5-F-Ph |
| 1-649 | 2-Cl-5-$OCH_2CH_2OH$-Ph | -NH-C(=O)- | CH | 5-Cl-2-OMe-Ph |
| 1-650 | 2-Cl-5-$OCH_2CH_2OH$-Ph | -NH-C(=O)- | CH | 5-Cl-2-OEt-Ph |
| 1-651 | 2-Cl-5-$OCH_2CH_2OH$-Ph | -NH-C(=O)- | CH | 2-OMe-5-Me-Ph |
| 1-652 | 2-Cl-5-$OCH_2CH_2OH$-Ph | -NH-C(=O)- | CH | 2-OEt-5-Me-Ph |
| 1-653 | 2-Cl-5-$OCH_2CH_2OH$-Ph | -NH-C(=O)- | CH | 3,5-di-OMe-Ph |
| 1-654 | 2-Cl-5-$OCH_2CH_2OH$-Ph | -NH-C(=O)- | CH | 5-Me-2-thiazo |
| 1-655 | 2-Cl-5-$OCH_2CH_2OH$-Ph | -NH-C(=O)- | CH | 4-t-Bu-2-thiazo |
| 1-656 | 2-Cl-5-$OCH_2CH_2OH$-Ph | -NH-C(=O)- | N | 2-F-Ph |
| 1-657 | 2-Cl-5-$OCH_2CH_2OH$-Ph | -NH-C(=O)- | N | 2-OMe-Ph |
| 1-658 | 2-Cl-5-$OCH_2CH_2OH$-Ph | -NH-C(=O)- | N | 2-OEt-Ph |
| 1-659 | 2-Cl-5-$OCH_2CH_2OH$-Ph | -NH-C(=O)- | N | 5-F-2-OMe-Ph |
| 1-660 | 2-Cl-5-$OCH_2CH_2OH$-Ph | -NH-C(=O)- | N | 2-OEt-5-F-Ph |
| 1-661 | 2-Cl-5-$OCH_2CH_2OH$-Ph | -NH-C(=O)- | N | 5-Cl-2-OMe-Ph |
| 1-662 | 2-Cl-5-$OCH_2CH_2OH$-Ph | -NH-C(=O)- | N | 5-Cl-2-OEt-Ph |
| 1-663 | 2-Cl-5-$OCH_2CH_2OH$-Ph | -NH-C(=O)- | N | 2-OMe-5-Me-Ph |
| 1-664 | 2-Cl-5-$OCH_2CH_2OH$-Ph | -NH-C(=O)- | N | 2-OEt-5-Me-Ph |
| 1-665 | 2-Cl-5-$OCH_2CH_2OH$-Ph | -NH-C(=O)- | N | 3,5-di-OMe-Ph |

(continued)

| Compound No. | $R^2$ | A | U | $R^1$ |
|---|---|---|---|---|
| 1-666 | 2-Cl-5-$OCH_2CH_2OH$-Ph | -NH-C(=O)- | N | 5-Me-2-thiazo |
| 1-667 | 2-Cl-5-$OCH_2CH_2OH$-Ph | -NH-C(=O)- | N | 4-t-Bu-2-thiazo |
| 1-668 | Ph(C) | -NH-C(=O)- | CH | 2-F-Ph |
| 1-669 | Ph(C) | -NH-C(=O)- | CH | 2-OMe-Ph |
| 1-670 | Ph(C) | -NH-C(=O)- | CH | 2-OEt-Ph |
| 1-671 | Ph(C) | -NH-C(=O)- | CH | 5-F-2-OMe-Ph |
| 1-672 | Ph(C) | -NH-C(=O)- | CH | 2-OEt-5-F-Ph |
| 1-673 | Ph(C) | -NH-C(=O)- | CH | 5-Cl-2-OMe-Ph |
| 1-674 | Ph(C) | -NH-C(=O)- | CH | 5-Cl-2-OEt-Ph |
| 1-675 | Ph(C) | -NH-C(=O)- | CH | 2-OMe-5-Me-Ph |
| 1-676 | Ph(C) | -NH-C(=O)- | CH | 2-OEt-5-Me-Ph |
| 1-677 | Ph(C) | -NH-C(=O)- | CH | 3,5-di-OMe-Ph |
| 1-678 | Ph(C) | -NH-C(=O)- | CH | 5-Me-2-thiazo |
| 1-679 | Ph(C) | -NH-C(=O)- | CH | 4-t-Bu-2-thiazo |
| 1-680 | Ph(C) | -NH-C(=O)- | N | 2-F-Ph |
| 1-681 | Ph(C) | -NH-C(=O)- | N | 2-OMe-Ph |
| 1-682 | Ph(C) | -NH-C(=O)- | N | 2-OEt-Ph |
| 1-683 | Ph(C) | -NH-C(=O)- | N | 5-F-2-OMe-Ph |
| 1-684 | Ph(C) | -NH-C(=O)- | N | 2-OEt-5-F-Ph |
| 1-685 | Ph(C) | -NH-C(=O)- | N | 5-Cl-2-OMe-Ph |
| 1-686 | Ph(C) | -NH-C(=O)- | N | 5-Cl-2-OEt-Ph |
| 1-687 | Ph(C) | -NH-C(=O)- | N | 2-OMe-5-Me-Ph |
| 1-688 | Ph(C) | -NH-C(=O)- | N | 2-OEt-5-Me-Ph |
| 1-689 | Ph(C) | -NH-C(=O)- | N | 3,5-di-OMe-Ph |
| 1-690 | Ph(C) | -NH-C(=O)- | N | 5-Me-2-thiazo |
| 1-691 | Ph(C) | -NH-C(=O)- | N | 4-t-Bu-2-thiazo |
| 1-692 | 4-$OCH_2CH_2OH$-2-Me-Ph | -NH-C(=O)- | CH | 2-OMe-5-Me-Ph |
| 1-693 | 4-$OCH_2CH_2OH$-2-Me-Ph | -NH-C(=O)- | N | 2-OMe-5-Me-Ph |
| 1-694 | 3-Me-4-Py | -NH-C(=O)- | CH | 2-F-Ph |
| 1-695 | 3-Me-4-Py | -NH-C(=O)- | CH | 2-OMe-Ph |
| 1-696 | 3-Me-4-Py | -NH-C(=O)- | CH | 2-OEt-Ph |
| 1-697 | 3-Me-4-Py | -NH-C(=O)- | CH | 5-F-2-OMe-Ph |
| 1-698 | 3-Me-4-Py | -NH-C(=O)- | CH | 2-OEt-5-F-Ph |
| 1-699 | 3-Me-4-Py | -NH-C(=O)- | CH | 5-Cl-2-OMe-Ph |
| 1-700 | 3-Me-4-Py | -NH-C(=O)- | CH | 5-Cl-2-OEt-Ph |
| 1-701 | 3-Me-4-Py | -NH-C(=O)- | CH | 2-OMe-5-Me-Ph |
| 1-702 | 3-Me-4-Py | -NH-C(=O)- | CH | 2-OEt-5-Me-Ph |
| 1-703 | 3-Me-4-Py | -NH-C(=O)- | CH | 3,5-di-OMe-Ph |
| 1-704 | 3-Me-4-Py | -NH-C(=O)- | CH | 5-Me-2-thiazo |
| 1-705 | 3-Me-4-Py | -NH-C(=O)- | CH | 4-t-Bu-2-thiazo |
| 1-706 | 3-CN-2-Py | -NH-C(=O)- | CH | 2-F-Ph |
| 1-707 | 3-CN-2-Py | -NH-C(=O)- | CH | 2-OMe-Ph |
| 1-708 | 3-CN-2-Py | -NH-C(=O)- | CH | 2-OEt-Ph |
| 1-709 | 3-CN-2-Py | -NH-C(=O)- | CH | 5-F-2-OMe-Ph |
| 1-710 | 3-CN-2-Py | -NH-C(=O)- | CH | 2-OEt-5-F-Ph |
| 1-711 | 3-CN-2-Py | -NH-C(=O)- | CH | 5-Cl-2-OMe-Ph |
| 1-712 | 3-CN-2-Py | -NH-C(=O)- | CH | 5-Cl-2-OEt-Ph |
| 1-713 | 3-CN-2-Py | -NH-C(=O)- | CH | 2-OMe-5-Me-Ph |
| 1-714 | 3-CN-2-Py | -NH-C(=O)- | CH | 2-OEt-5-Me-Ph |
| 1-715 | 3-CN-2-Py | -NH-C(=O)- | CH | 3,5-di-OMe-Ph |

(continued)

| Compound No. | $R^2$ | A | U | $R^1$ |
|---|---|---|---|---|
| 1-716 | 3-CN-2-Py | -NH-C(=O)- | CH | 5-Me-2-thiazo |
| 1-717 | 3-CN-2-Py | -NH-C(=O)- | CH | 4-t-Bu-2-thiazo |
| 1-718 | 3,5-di-Cl-2-Py | -NH-C(=O)- | CH | 2-F-Ph |
| 1-719 | 3,5-di-Cl-2-Py | -NH-C(=O)- | CH | 2-OMe-Ph |
| 1-720 | 3,5-di-Cl-2-Py | -NH-C(=O)- | CH | 2-OEt-Ph |
| 1-721 | 3,5-di-Cl-2-Py | -NH-C(=O)- | CH | 5-F-2-OMe-Ph |
| 1-722 | 3,5-di-Cl-2-Py | -NH-C(=O)- | CH | 2-OEt-5-F-Ph |
| 1-723 | 3,5-di-Cl-2-Py | -NH-C(=O)- | CH | 5-Cl-2-OMe-Ph |
| 1-724 | 3,5-di-Cl-2-Py | -NH-C(=O)- | CH | 5-Cl-2-OEt-Ph |
| 1-725 | 3,5-di-Cl-2-Py | -NH-C(=O)- | CH | 2-OMe-5-Me-Ph |
| 1-726 | 3,5-di-Cl-2-Py | -NH-C(=O)- | CH | 2-OEt-5-Me-Ph |
| 1-727 | 3,5-di-Cl-2-Py | -NH-C(=O)- | CH | 3,5-di-OMe-Ph |
| 1-728 | 3,5-di-Cl-2-Py | -NH-C(=O)- | CH | 5-Me-2-thiazo |
| 1-729 | 3,5-di-Cl-2-Py | -NH-C(=O)- | CH | 4-t-Bu-2-thiazo |
| 1-730 | 3,5-di-Cl-2-Py | -NH-C(=O)- | N | 2-F-Ph |
| 1-731 | 3,5-di-Cl-2-Py | -NH-C(=O)- | N | 2-OMe-Ph |
| 1-732 | 3,5-di-Cl-2-Py | -NH-C(=O)- | N | 2-OEt-Ph |
| 1-733 | 3,5-di-Cl-2-Py | -NH-C(=O)- | N | 5-F-2-OMe-Ph |
| 1-734 | 3,5-di-Cl-2-Py | -NH-C(=O)- | N | 2-OEt-5-F-Ph |
| 1-735 | 3,5-di-Cl-2-Py | -NH-C(=O)- | N | 5-Cl-2-OMe-Ph |
| 1-736 | 3,5-di-Cl-2-Py | -NH-C(=O)- | N | 5-Cl-2-OEt-Ph |
| 1-737 | 3,5-di-Cl-2-Py | -NH-C(=O)- | N | 2-OMe-5-Me-Ph |
| 1-738 | 3,5-di-Cl-2-Py | -NH-C(=O)- | N | 2-OEt-5-Me-Ph |
| 1-739 | 3,5-di-Cl-2-Py | -NH-C(=O)- | N | 3,5-di-OMe-Ph |
| 1-740 | 3,5-di-Cl-2-Py | -NH-C(=O)- | N | 5-Me-2-thiazo |
| 1-741 | 3,5-di-Cl-2-Py | -NH-C(=O)- | N | 4-t-Bu-2-thiazo |
| 1-742 | 3-CN-2-Py | -NH-C(=O)- | N | 2-OEt-5-F-Ph |
| 1-743 | 2-Cl-4-$NHSO_2$Me-Ph | -NH-C(=O)- | CH | 2-OMe-5-Me-Ph |
| 1-744 | 2-Cl-4-$NHSO_2$Me-Ph | -NH-C(=O)- | N | 2-OMe-5-Me-Ph |
| 1-745 | 4-$NMe_2$-2-$CF_3$-Ph | -NH-C(=O)- | CH | 2-OMe-5-Me-Ph |
| 1-746 | 4-$NMe_2$-2-$CF_3$-Ph | -NH-C(=O)- | N | 2-OMe-5-Me-Ph |
| 1-747 | Ph(D) | -NH-C(=O)- | CH | 2-OMe-5-Me-Ph |
| 1-748 | Ph(D) | -NH-C(=O)- | N | 2-OMe-5-Me-Ph |
| 1-749 | Ph(E) | -NH-C(=O)- | CH | 2-OMe-5-Me-Ph |
| 1-750 | Ph(E) | -NH-C(=O)- | N | 2-OMe-5-Me-Ph |
| 1-751 | 2-Cl-4-$CH_2CO_2$Me-Ph | -NH-C(=O)- | CH | 2-OMe-5-Me-Ph |
| 1-752 | 2-Cl-4-$CH_2CO_2$Me-Ph | -NH-C(=O)- | N | 2-OMe-5-Me-Ph |
| 1-753 | 2-Cl-4-$CH_2CO_2$H-Ph | -NH-C(=O)- | CH | 2-OMe-5-Me-Ph |
| 1-754 | 2-Cl-4-$CH_2CO_2$H-Ph | -NH-C(=O)- | N | 2-OMe-5-Me-Ph |
| 1-755 | Ph(F) | -NH-C(=O)- | CH | 2-OMe-5-Me-Ph |
| 1-756 | Ph(F) | -NH-C(=O)- | N | 2-OMe-5-Me-Ph |
| 1-757 | 4-NMe($CH_2CH_2$OH)-2-Me-Ph | -NH-C(=O)- | CH | 2-OMe-5-Me-Ph |
| 1-758 | 4-NMe($CH_2CH_2$OH)-2-Me-Ph | -NH-C(=O)- | N | 2-OMe-5-Me-Ph |
| 1-759 | 4-$CH_2$OH-2-Me-Ph | -NH-C(=O)- | CH | 2-OMe-5-Me-Ph |
| 1-760 | 4-$CH_2$OH-2-Me-Ph | -NH-C(=O)- | N | 2-OMe-5-Me-Ph |
| 1-761 | 2-F-4-$OCH_2CH_2$OH-Ph | -NH-C(=O)- | CH | 2-OMe-5-Me-Ph |
| 1-762 | 2-F-4-$OCH_2CH_2$OH-Ph | -NH-C(=O)- | N | 2-OMe-5-Me-Ph |
| 1-763 | 2-Cl-4-$CH_2$OH-Ph | -NH-C(=O)- | CH | 2-OMe-5-Me-Ph |

(continued)

| Compound No. | $R^2$ | A | U | $R^1$ |
|---|---|---|---|---|
| 1-764 | 2-Cl-4-$CH_2OH$-Ph | -NH-C(=O)- | N | 2-OMe-5-Me-Ph |
| 1-765 | 4-$CH_2CO_2Me$-2-Me-Ph | -NH-C(=O)- | CH | 2-OMe-5-Me-Ph |
| 1-766 | 4-$CH_2CO_2Me$-2-Me-Ph | -NH-C(=O)- | N | 2-OMe-5-Me-Ph |
| 1-767 | 4-$CH_2CO_2H$-2-Me-Ph | -NH-C(=O)- | CH | 2-OMe-5-Me-Ph |
| 1-768 | 4-$CH_2CO_2H$-2-Me-Ph | -NH-C(=O)- | N | 2-OMe-5-Me-Ph |
| 1-769 | 4-$CH_2PO(OEt)_2$-2-Me-Ph | -NH-C(=O)- | CH | 2-OMe-5-Me-Ph |
| 1-770 | 4-$CH_2PO(OEt)_2$-2-Me-Ph | -NH-C(=O)- | N | 2-OMe-5-Me-Ph |
| 1-771 | 5-$OCH_2CH_2OH$-2-Me-Ph | -NH-C(=O)- | CH | 2-OMe-5-Me-Ph |
| 1-772 | 5-$OCH_2CH_2OH$-2-Me-Ph | -NH-C(=O)- | N | 2-OMe-5-Me-Ph |
| 1-773 | 4-$CH_2CH_2OH$-2-Me-Ph | -NH-C(=O)- | CH | 2-OMe-5-Me-Ph |
| 1-774 | 4-$CH_2CH_2OH$-2-Me-Ph | -NH-C(=O)- | N | 2-OMe-5-Me-Ph |
| 1-775 | Ph(G) | -NH-C(=O)- | CH | 2-OMe-5-Me-Ph |
| 1-776 | Ph(G) | -NH-C(=O)- | N | 2-OMe-5-Me-Ph |
| 1-777 | 2-Cl-6-$CH_2OH$-Ph | -NH-C(=O)- | CH | 2-OMe-5-Me-Ph |
| 1-778 | 2-Cl-6-$CH_2OH$-Ph | -NH-C(=O)- | N | 2-OMe-5-Me-Ph |
| 1-779 | 6-$CH_2OH$-2-CF3-Ph | -NH-C(=O)- | CH | 2-OMe-5-Me-Ph |
| 1-780 | 6-$CH_2OH$-2-CF3-Ph | -NH-C(=O)- | N | 2-OMe-5-Me-Ph |
| 1-781 | Ph(H) | -NH-C(=O)- | CH | 2-OMe-5-Me-Ph |
| 1-782 | Ph(H) | -NH-C(=O)- | N | 2-OMe-5-Me-Ph |
| 1-783 | 4-Me-3-Py | -NH-C(=O)- | CH | 2-OMe-5-Me-Ph |
| 1-784 | 4-Me-3-Py | -NH-C(=O)- | N | 2-OMe-5-Me-Ph |
| 1-785 | 4-$CH_2PO(OH)(OEt)$-2-Me-Ph | -NH-C(=O)- | CH | 2-OMe-5-Me-Ph |
| 1-786 | 4-$CH_2PO(OH)(OEt)$-2-Me-Ph | -NH-C(=O)- | N | 2-OMe-5-Me-Ph |
| 1-787 | 3,5-di-F-2-Py | -NH-C(=O)- | CH | 2-OMe-5-Me-Ph |
| 1-788 | 3,5-di-F-2-Py | -NH-C(=O)- | N | 2-OMe-5-Me-Ph |
| 1-789 | 2-Cl-6-Me-Ph | -NH-C(=O)- | CH | CycPent |
| 1-790 | 4-$OCH_2CH_2OH$-2-$CF_3$-Ph | -NH-C(=O)- | CH | CycPent |
| 1-791 | 4-$CO_2H$-2-Cl-Ph | -NH-C(=O)- | CH | 2-F-Ph |
| 1-792 | 4-$CO_2H$-2-Cl-Ph | -NH-C(=O)- | N | 2-F-Ph |
| 1-793 | 6-$CH_2OH$-2-Me-Ph | -NH-C(=O)- | CH | 2-F-Ph |
| 1-794 | 6-$CH_2OH$-2-Me-Ph | -NH-C(=O)- | N | 2-F-Ph |
| 1-795 | 2-F-6-$CF_3$-Ph | -NH-C(=O)- | N | 2-F-Ph |
| 1-796 | 2,6-di-Cl-Ph | -NH-C(=O)- | N | 2-F-Ph |
| 1-797 | 4-$CH_2OH$-2-Me-Ph | -NH-C(=O)- | CH | 2-F-Ph |
| 1-798 | 4-$CH_2OH$-2-Me-Ph | -NH-C(=O)- | N | 2-F-Ph |
| 1-799 | 2,6-di-F-Ph | -NH-C(=O)- | N | 2-F-Ph |
| 1-800 | 2-Cl-Ph | -CH(OH)-C(=O)- | N | 2-F-Ph |
| 1-801 | t-Bu | -O-C(=O)- | N | 2-F-Ph |
| 1-802 | 2-Cl-4-$CH_2CH_2OH$-Me-Ph | -O-C(=O)- | CH | 2-OMe-5-Me-Ph |
| 1-803 | t-Bu | -O-C(=O)- | CH | 5-Et-2-thiazo |
| 1-804 | t-Bu | -O-C(=O)- | CH | Het(A) |
| 1-805 | 2-Cl-Ph | -CH(OH)-C(=O)- | CH | 2-OMe-5-Me-Ph |
| 1-806 | 2-Cl-Ph | -CH(OH)-C(=O)- | N | 2-OMe-5-Me-Ph |
| 1-807 | 2,4-di-F-Ph | -CH(OH)-C(=O)- | CH | 2-OMe-5-Me-Ph |
| 1-808 | 2,4-di-F-Ph | -CH(OH)-C(=O)- | N | 2-OMe-5-Me-Ph |
| 1-809 | 2-Cl-4-OH-Ph | -CH(OH)-C(=O)- | CH | 2-F-Ph |
| 1-810 | 2-Cl-4-OH-Ph | -CH(OH)-C(=O)- | N | 2-F-Ph |
| 1-811 | t-Bu | -O-C(=O)- | CH | 4-t-Bu-2-thiazo |

(continued)

| Compound No. | $R^2$ | A | U | $R^1$ |
|---|---|---|---|---|
| 1-812 | t-Bu | -O-C(=O)- | CH | 4,5-di-Me-2-thiazo |
| 1-813 | t-Bu | -O-C(=O)- | CH | 4-Me-2-thiazo |
| 1-814 | 2-Cl-4-$OCH_2CH_2OH$-Ph | -CH(OH)-C(=O)- | CH | 2-OMe-5-Me-Ph |
| 1-815 | 2-Cl-4-$OCH_2CH_2OH$-Ph | -CH(OH)-C(=O)- | N | 2-OMe-5-Me-Ph |
| 1-816 | 3-Me-2-Py | -CH(OH)-C(=O)- | CH | 2-OMe-5-Me-Ph |
| 1-817 | 3-Me-2-Py | -CH(OH)-C(=O)- | N | 2-OMe-5-Me-Ph |
| 1-818 | Ph(A) | -CH(OH)-C(=O)- | CH | 2-OMe-5-Me-Ph |
| 1-819 | Ph(A) | -CH(OH)-C(=O)- | N | 2-OMe-5-Me-Ph |
| 1-820 | 4-$OCH_2CH_2OH$-2-$CF_3$-Ph | -CH(OH)-C(=O)- | CH | 2-OMe-5-Me-Ph |
| 1-821 | 4-$OCH_2CH_2OH$-2-$CF_3$-Ph | -CH(OH)-C(=O)- | N | 2-OMe-5-Me-Ph |
| 1-822 | Ph(B) | -CH(OH)-C(=O)- | CH | 2-OMe-5-Me-Ph |
| 1-823 | Ph(B) | -CH(OH)-C(=O)- | N | 2-OMe-5-Me-Ph |
| 1-824 | 2-Cl-5-$OCH_2CH_2OH$-Ph | -CH(OH)-C(=O)- | CH | 2-OMe-5-Me-Ph |
| 1-825 | 2-Cl-5-$OCH_2CH_2OH$-Ph | -CH(OH)-C(=O)- | N | 2-OMe-5-Me-Ph |
| 1-826 | Ph(C) | -CH(OH)-C(=O)- | CH | 2-OMe-5-Me-Ph |
| 1-827 | Ph(C) | -CH(OH)-C(=O)- | N | 2-OMe-5-Me-Ph |
| 1-828 | 2-Cl-4-$OCH_2CH_2OH$-Ph | -CH(OH)-C(=O)- | CH | 2-OMe-Ph |
| 1-829 | 2-Cl-4-$OCH_2CH_2OH$-Ph | -CH(OH)-C(=O)- | N | 2-OMe-Ph |
| 1-830 | 3-Me-2-Py | -CH(OH)-C(=O)- | CH | 2-OMe-Ph |
| 1-831 | 3-Me-2-Py | -CH(OH)-C(=O)- | N | 2-OMe-Ph |
| 1-832 | Ph(A) | -CH(OH)-C(=O)- | CH | 2-OMe-Ph |
| 1-833 | Ph(A) | -CH(OH)-C(=O)- | N | 2-OMe-Ph |
| 1-834 | 4-$OCH_2CH_2OH$-2-$CF_3$-Ph | -CH(OH)-C(=O)- | CH | 2-OMe-Ph |
| 1-835 | 4-$OCH_2CH_2OH$-2-$CF_3$-Ph | -CH(OH)-C(=O)- | N | 2-OMe-Ph |
| 1-836 | Ph(B) | -CH(OH)-C(=O)- | CH | 2-OMe-Ph |
| 1-837 | Ph(B) | -CH(OH)-C(=O)- | N | 2-OMe-Ph |
| 1-838 | 2-Cl-5-$OCH_2CH_2OH$-Ph | -CH(OH)-C(=O)- | CH | 2-OMe-Ph |
| 1-839 | 2-Cl-5-$OCH_2CH_2OH$-Ph | -CH(OH)-C(=O)- | N | 2-OMe-Ph |
| 1-840 | Ph(C) | -CH(OH)-C(=O)- | CH | 2-OMe-Ph |
| 1-841 | Ph(C) | -CH(OH)-C(=O)- | N | 2-OMe-Ph |
| 1-842 | 4-Me-3-Py | -NH-C(=O)- | CH | 5-F-2-OMe-Ph |
| 1-843 | 4-Me-3-Py | -NH-C(=O)- | N | 5-F-2-OMe-Ph |
| 1-844 | 4-$OCH_2CH_2OH$-2-$CF_3$-Ph | -NH-C(=O)- | CH | 3-OMe-Ph |
| 1-845 | 4-$OCH_2CH_2OH$-2-$CF_3$-Ph | -NH-C(=O)- | N | 3-OMe-Ph |
| 1-846 | 4-$OCH_2CH_2OH$-2-$CF_3$-Ph | -NH-C(=O)- | CH | 3-OEt-Ph |
| 1-847 | 4-$OCH_2CH_2OH$-2-$CF_3$-Ph | -NH-C(=O)- | N | 3-OEt-Ph |
| 1-848 | 4-$OCH_2CH_2OH$-2-$CF_3$-Ph | -NH-C(=O)- | CH | 4,5-di-Me-2-thiazo |
| 1-849 | 4-$OCH_2CH_2OH$-2-$CF_3$-Ph | -NH-C(=O)- | N | 4,5-di-Me-2-thiazo |
| 1-850 | 4-$OCH_2CH_2OH$-2-$CF_3$-Ph | -NH-C(=O)- | CH | 4-Me-2-thiazo |
| 1-851 | 4-$OCH_2CH_2OH$-2-$CF_3$-Ph | -NH-C(=O)- | N | 4-Me-2-thiazo |
| 1-852 | 2-Cl-4-$OCH_2CH_2OH$-Ph | -NH-C(=O)- | CH | 3-OMe-Ph |
| 1-853 | 2-Cl-4-$OCH_2CH_2OH$-Ph | -NH-C(=O)- | N | 3-OMe-Ph |
| 1-854 | 3-Me-2-Py | -NH-C(=O)- | CH | 3-OMe-Ph |
| 1-855 | 3-Me-2-Py | -NH-C(=O)- | N | 3-OMe-Ph |
| 1-856 | 3-Me-2-Py | -NH-C(=O)- | CH | 3-OEt-Ph |
| 1-857 | 3-Me-2-Py | -NH-C(=O)- | N | 3-OEt-Ph |
| 1-858 | 2-Cl-5-$OCH_2CH_2OH$-Ph | -NH-C(=O)- | CH | 3-OEt-Ph |
| 1-859 | 2-Cl-5-$OCH_2CH_2OH$-Ph | -NH-C(=O)- | N | 3-OEt-Ph |
| 1-860 | 2-Cl-4-$OCH_2CH_2OH$-Ph | -NH-C(=O)- | CH | 3-OMe-2-Py |
| 1-861 | 2-Cl-4-$OCH_2CH_2OH$-Ph | -NH-C(=O)- | N | 3-OMe-2-Py |

(continued)

| Compound No. | $R^2$ | A | U | $R^1$ |
|---|---|---|---|---|
| 1-862 | 2-Cl-5-$OCH_2CH_2OH$-Ph | -NH-C(=O)- | CH | 4,5-di-Me-2-thiazo |
| 1-863 | 2-Cl-5-$OCH_2CH_2OH$-Ph | -NH-C(=O)- | N | 4,5-di-Me-2-thiazo |
| 1-864 | 2-Cl-4-$OCH_2CH_2OH$-Ph | -NH-C(=O)- | CH | 4,5-di-Me-2-thiazo |
| 1-865 | 2-Cl-4-$OCH_2CH_2OH$-Ph | -NH-C(=O)- | N | 4,5-di-Me-2-thiazo |
| 1-866 | 2-Cl-4-$OCH_2CH_2OH$-Ph | -NH-C(=O)- | CH | 3-OEt-Ph |
| 1-867 | 2-Cl-4-$OCH_2CH_2OH$-Ph | -NH-C(=O)- | N | 3-OEt-Ph |
| 1-868 | 4-$OCH_2CH_2OH$-2-$CF_3$-Ph | -NH-C(=O)- | CH | 2-$OCF_3$-Ph |
| 1-869 | 3-CN-2-Py | -NH-C(=O)- | CH | 2-$OCF_3$-Ph |
| 1-870 | 4-$OCH_2CH_2OH$-2-$CF_3$-Ph | -NH-C(=O)- | CH | 2-SMe-Ph |
| 1-871 | 3-Me-2-Py | -NH-C(=O)- | CH | 2-$OCF_2H$-Ph |
| 1-872 | 3-CN-2-Py | -NH-C(=O)- | CH | 2-$OCF_2H$-Ph |
| 1-873 | 4-$OCH_2CH_2OH$-2-$CF_3$-Ph | -NH-C(=O)- | CH | 2-$OCF_2H$-Ph |
| 1-874 | 4-$OCH_2CH_2OH$-2-$CF_3$-Ph | -NH-C(=O)- | CH | Het(A) |
| 1-875 | 3-CN-2-Py | -NH-C(=O)- | CH | Het(A) |
| 1-876 | 4-$OCH_2CH_2OH$-2-$CF_3$-Ph | -NH-C(=O)- | CH | Het(B) |
| 1-877 | 3-$CF_3$-2-Py | -NH-C(=O)- | N | 2-OEt-5-F-Ph |
| 1-878 | 3-Me-2-Py | -NH-C(=O)- | CH | 2-OMe-3-Py |
| 1-879 | 3-CN-2-Py | -NH-C(=O)- | CH | 3-OEt-Ph |
| 1-880 | 3-CN-2-Py | -NH-C(=O)- | CH | 2,3-di-OMe-Ph |
| 1-881 | 4-$OCH_2CH_2OH$-2-$CF_3$-Ph | -NH-C(=O)- | CH | 2,3-di-OMe-Ph |
| 1-882 | 3-Me-2-Py | -NH-C(=O)- | CH | 2,3-di-OMe-Ph |
| 1-883 | 3,5-di-F-2-Py | -NH-C(=O)- | CH | 2,3-di-OMe-Ph |
| 1-884 | 3,5-di-F-2-Py | -NH-C(=O)- | CH | 2-OEt-Ph |
| 1-885 | 3,5-di-F-2-Py | -NH-C(=O)- | CH | 3-OEt-Ph |
| 1-886 | 3,5-di-F-2-Py | -NH-C(=O)- | CH | 3,5-di-OMe-Ph |
| 1-887 | 3,5-di-F-2-Py | -NH-C(=O)- | CH | 2-OEt-5-F-Ph |
| 1-888 | 3,5-di-F-2-Py | -NH-C(=O)- | N | 2-OEt-5-F-Ph |
| 1-889 | 3,5-di-F-2-Py | -NH-C(=O)- | CH | 2-$OCF_3$-Ph |
| 1-890 | 3,5-di-F-2-Py | -NH-C(=O)- | CH | 2-$OCF_2H$-Ph |
| 1-891 | 3,5-di-F-2-Py | -NH-C(=O)- | CH | 2-SMe-Ph |
| 1-892 | 3,5-di-F-2-Py | -NH-C(=O)- | CH | Het(A) |
| 1-893 | 3-Me-2-Py | -NH-C(=O)- | CH | 2-$OCF_3$-Ph |
| 1-894 | 3-Me-2-Py | -NH-C(=O)- | CH | 2-$OCF_2H$-Ph |
| 1-895 | 3-Me-2-Py | -NH-C(=O)- | CH | 2-SMe-Ph |
| 1-896 | 3-Me-2-Py | -NH-C(=O)- | CH | Het(A) |
| 1-897 | 3-Me-2-Py | -NH-C(=O)- | CH | 2-OMe-3-Me-Ph |
| 1-898 | 3-Cl-2-Py | -NH-C(=O)- | CH | 2-OMe-5-Me-Ph |
| 1-899 | 3-F-2-Py | -NH-C(=O)- | CH | 2-OMe-5-Me-Ph |
| 1-900 | 4-$OCH_2CH_2OH$-2-$CF_3$-Ph | -NH-C(=O)- | CH | 4-Et-2-thiazo |
| 1-901 | 4-$OCH_2CH_2OH$-2-$CF_3$-Ph | -NH-C(=O)- | N | 4-Et-2-thiazo |
| 1-902 | 3-Me-2-Py | -NH-C(=O)- | CH | 2-OMe-4-Me-Ph |
| 1-903 | 3-Me-2-Py | -NH-C(=O)- | N | 2-OMe-4-Me-Ph |
| 1-904 | 3-Me-2-Py | -NH-C(=O)- | CH | Het(C) |
| 1-905 | 3-Me-2-Py | -NH-C(=O)- | N | Het(C) |
| 1-906 | 4-$OCH_2CH_2OH$-2-$CF_3$-Ph | -NH-C(=O)- | CH | 4-CycPr-2-thiazo |
| 1-907 | 4-$OCH_2CH_2OH$-2-$CF_3$-Ph | -NH-C(=O)- | N | 4-CycPr-2-thiazo |
| 1-908 | 3-Cl-2-Py | -NH-C(=O)- | CH | 2-F-Ph |
| 1-909 | 3-Cl-2-Py | -NH-C(=O)- | CH | 2-OMe-Ph |
| 1-910 | 3-Cl-2-Py | -NH-C(=O)- | CH | 3-OEt-Ph |
| 1-911 | 3-Cl-2-Py | -NH-C(=O)- | CH | 3,5-di-OMe-Ph |

(continued)

| Compound No. | $R^2$ | A | U | $R^1$ |
|---|---|---|---|---|
| 1-912 | 3-Cl-2-Py | -NH-C(=O)- | CH | 2-OMe-3-Me-Ph |
| 1-913 | 3-Cl-2-Py | -NH-C(=O)- | CH | 2-OEt-Ph |
| 1-914 | 3-Cl-2-Py | -NH-C(=O)- | N | 2-F-Ph |
| 1-915 | 3-Cl-2-Py | -NH-C(=O)- | N | 2-OMe-Ph |
| 1-916 | 3-Cl-2-Py | -NH-C(=O)- | N | 3-OEt-Ph |
| 1-917 | 3-Cl-2-Py | -NH-C(=O)- | N | 3,5-di-OMe-Ph |
| 1-918 | 3-Cl-2-Py | -NH-C(=O)- | N | 2-OMe-3-Me-Ph |
| 1-919 | 3-Cl-2-Py | -NH-C(=O)- | N | 2-OEt-Ph |
| 1-920 | 2-Cl-6-Me-Ph | -NH-C(=O)- | N | 2-F-Ph |
| 1-921 | 3,5-di-F-2-Py | -NH-C(=O)- | CH | 2-OMe-3-Me-Ph |
| 1-922 | 3,5-di-F-2-Py | -NH-C(=O)- | N | 2-OMe-3-Me-Ph |
| 1-923 | 3-CN-2-Py | -NH-C(=O)- | CH | 2-OMe-3-Me-Ph |
| 1-924 | 3-CN-2-Py | -NH-C(=O)- | N | 2-OMe-3-Me-Ph |
| 1-925 | 4-$OCH_2CH_2OH$-2-$CF_3$-Ph | -NH-C(=O)- | CH | 2-OMe-3-Py |
| 1-926 | 4-$OCH_2CH_2OH$-2-$CF_3$-Ph | -NH-C(=O)- | N | 2-OMe-3-Py |
| 1-927 | 3-$CO_2H$-2-Py | -NH-C(=O)- | CH | 2-OMe-5-Me-Ph |
| 1-928 | 3-Cl-2-Py | -NH-C(=O)- | CH | Het(C) |
| 1-929 | 3-CN-2-Py | -NH-C(=O)- | CH | Het(C) |
| 1-930 | 4-$OCH_2CH_2OH$-2-$CF_3$-Ph | -NH-C(=O)- | CH | Het(C) |
| 1-931 | 3,5-di-F-2-Py | -NH-C(=O)- | CH | Het(C) |
| 1-932 | 3-Me-2-Py | -NH-C(=O)- | CH | 2-F-Ph |
| 1-933 | 3-Me-2-Py | -NH-C(=O)- | N | 2-F-Ph |
| 1-934 | 3-$CH_2OH$-2-Py | -NH-C(=O)- | CH | 2-OMe-5-Me-Ph |
| 1-935 | 3-$CH_2OH$-2-Py | -NH-C(=O)- | N | 2-OMe-5-Me-Ph |
| 1-936 | 3-CN-2-Py | -NH-C(=O)- | CH | 2-OMe-4-Me-Ph |
| 1-937 | 3-CN-2-Py | -NH-C(=O)- | N | 2-OMe-4-Me-Ph |
| 1-938 | 3,5-di-F-2-Py | -NH-C(=O)- | CH | 2-OMe-4-Me-Ph |
| 1-939 | 3,5-di-F-2-Py | -NH-C(=O)- | N | 2-OMe-4-Me-Ph |
| 1-940 | 4-$OCH_2CH_2OH$-2-$CF_3$-Ph | -NH-C(=O)- | CH | 3-OMe-4-Py |
| 1-941 | 3-Me-2-Py | -NH-C(=O)- | CH | 3-OMe-4-Py |
| 1-942 | 3-$CH_2OH$-2-Py | -NH-C(=O)- | CH | 2-F-Ph |
| 1-943 | 3-$CH_2OH$-2-Py | -NH-C(=O)- | CH | 2-OMe-Ph |
| 1-944 | 3-$CH_2OH$-2-Py | -NH-C(=O)- | CH | 2-OEt-Ph |
| 1-945 | 3-$CH_2OH$-2-Py | -NH-C(=O)- | CH | 3-OEt-Ph |
| 1-946 | 3-$CH_2OH$-2-Py | -NH-C(=O)- | CH | 3,5-di-OMe-Ph |

(Table 2)

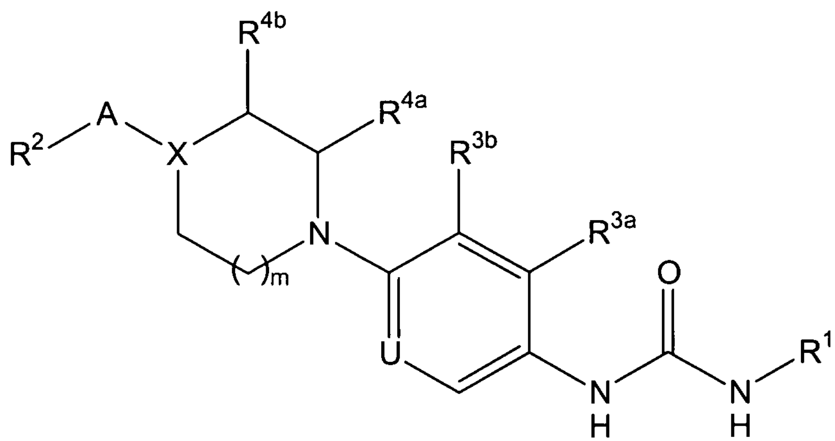

(VI)

| Compound No. | $R^2$ | A | X | $R^{4b}$ | $R^{4a}$ | m | U | $R^{3b}$ | $R^{3a}$ | $R^1$ |
|---|---|---|---|---|---|---|---|---|---|---|
| 2-1 | 2-Cl-6-Me-Ph | -NH- C (=O)- | N | H | H | 1 | CH | H | Me | 2-OMe-5-Me-Ph |
| 2-2 | 2-Cl-6-Me-Ph | -NH- C (=O)- | N | H | H | 1 | N | H | Me | 2-OMe-5-Me-Ph |
| 2-3 | 2-Cl-6-Me-Ph | -NH- C (=O)- | N | H | H | 1 | CH | Me | H | 2-OMe-5-Me-Ph |
| 2-4 | 2-Cl-6-Me-Ph | -NH- C (=O)- | N | H | H | 1 | N | Me | H | 2-OMe-5-Me-Ph |
| 2-5 | 2-Cl-6-Me-Ph | -NH- C (=O)- | N | H | H | 1 | CH | H | F | 2-OMe-5-Me-Ph |
| 2-6 | 2-Cl-6-Me-Ph | -NH- C (=O)- | N | H | H | 1 | N | H | F | 2-OMe-5-Me-Ph |
| 2-7 | 2-Cl-6-Me-Ph | -NH- C (=O)- | N | H | H | 1 | CH | F | H | 2-OMe-5-Me-Ph |
| 2-8 | 2-Cl-6-Me-Ph | -NH- C (=O)- | N | H | H | 1 | N | F | H | 2-OMe-5-Me-Ph |
| 2-9 | 2-Cl-6-Me-Ph | -NH- C (=O)- | N | H | H | 2 | CH | H | H | 2-OMe-5-Me-Ph |
| 2-10 | 2-Cl-6-Me-Ph | -NH- C (=O)- | N | H | H | 2 | N | H | H | 2-OMe-5-Me-Ph |
| 2-11 | 2-Cl-6-Me-Ph | -NH- C (=O)- | N | H | Me | 1 | CH | H | H | 2-OMe-5-Me-Ph |
| 2-12 | 2-Cl-6-Me-Ph | -NH- C (=O)- | N | H | Me | 1 | N | H | H | 2-OMe-5-Me-Ph |
| 2-13 | 2-Cl-6-Me-Ph | -NH- C (=O)- | N | Me | H | 1 | CH | H | H | 2-OMe-5-Me-Ph |
| 2-14 | 2-Cl-6-Me-Ph | -NH- C (=O)- | N | Me | H | 1 | N | H | H | 2-OMe-5-Me-Ph |
| 2-15 | 2-Cl-6-Me-Ph | -NH- C (=O)- | N | H | Et | 1 | CH | H | H | 2-OMe-5-Me-Ph |
| 2-16 | 2-Cl-6-Me-Ph | -NH- C (=O)- | N | H | Et | 1 | N | H | H | 2-OMe-5-Me-Ph |

(continued)

| Compound No. | $R^2$ | A | X | $R^{4b}$ | $R^{4a}$ | m | U | $R^{3b}$ | $R^{3a}$ | $R^1$ |
|---|---|---|---|---|---|---|---|---|---|---|
| 2-17 | 2-Cl-6-Me-Ph | -NH- C (=O)- | N | Et | H | 1 | CH | H | H | 2-OMe-5-Me-Ph |
| 2-18 | 2-Cl-6-Me-Ph | -NH- C (=O)- | N | Et | H | 1 | N | H | H | 2-OMe-5-Me-Ph |
| 2-19 | 2-Cl-6-Me-Ph | -NH- C (=O)- | CH | H | H | 1 | CH | H | H | 2-OMe-5-Me-Ph |
| 2-20 | 2-Cl-6-Me-Ph | -NH- C (=O)- | CH | H | H | 1 | N | H | H | 2-OMe-5-Me-Ph |
| 2-21 | 2,6-di-Me-Ph | -NH- C (=O)- | N | H | H | 1 | CH | H | Me | 2-OMe-5-Me-Ph |
| 2-22 | 2,6-di-Me-Ph | -NH- C (=O)- | N | H | H | 1 | N | H | Me | 2-OMe-5-Me-Ph |
| 2-23 | 2,6-di-Me-Ph | -NH- C (=O)- | N | H | H | 1 | CH | Me | H | 2-OMe-5-Me-Ph |
| 2-24 | 2,6-di-Me-Ph | -NH- C (=O)- | N | H | H | 1 | N | Me | H | 2-OMe-5-Me-Ph |
| 2-25 | 2,6-di-Me-Ph | -NH- C (=O)- | N | H | H | 1 | CH | H | F | 2-OMe-5-Me-Ph |
| 2-26 | 2,6-di-Me-Ph | -NH- C (=O)- | N | H | H | 1 | N | H | F | 2-OMe-5-Me-Ph |
| 2-27 | 2,6-di-Me-Ph | -NH- C (=O)- | N | H | H | 1 | CH | F | H | 2-OMe-5-Me-Ph |
| 2-28 | 2,6-di-Me-Ph | -NH- C (=O)- | N | H | H | 1 | N | F | H | 2-OMe-5-Me-Ph |
| 2-29 | 2,6-di-Me-Ph | -NH- C (=O)- | N | H | H | 2 | CH | H | H | 2-OMe-5-Me-Ph |
| 2-30 | 2,6-di-Me-Ph | -NH- C (=O)- | N | H | H | 2 | N | H | H | 2-OMe-5-Me-Ph |
| 2-31 | 2,6-di-Me-Ph | -NH- C (=O)- | N | H | Me | 1 | CH | H | H | 2-OMe-5-Me-Ph |
| 2-32 | 2,6-di-Me-Ph | -NH- C (=O)- | N | H | Me | 1 | N | H | H | 2-OMe-5-Me-Ph |
| 2-33 | 2,6-di-Me-Ph | -NH- C (=O)- | N | Me | H | 1 | CH | H | H | 2-OMe-5-Me-Ph |
| 2-34 | 2,6-di-Me-Ph | -NH- C (=O)- | N | Me | H | 1 | N | H | H | 2-OMe-5-Me-Ph |
| 2-35 | 2,6-di-Me-Ph | -NH- C (=O)- | N | H | Et | 1 | CH | H | H | 2-OMe-5-Me-Ph |
| 2-36 | 2,6-di-Me-Ph | -NH- C (=O)- | N | H | Et | 1 | N | H | H | 2-OMe-5-Me-Ph |
| 2-37 | 2,6-di-Me-Ph | -NH- C (=O)- | N | Et | H | 1 | CH | H | H | 2-OMe-5-Me-Ph |
| 2-38 | 2,6-di-Me-Ph | -NH- C (=O)- | N | Et | H | 1 | N | H | H | 2-OMe-5-Me-Ph |
| 2-39 | 2,6-di-Me-Ph | -NH- C (=O)- | CH | H | H | 1 | CH | H | H | 2-OMe-5-Me-Ph |
| 2-40 | 2,6-di-Me-Ph | -NH- C (=O)- | CH | H | H | 1 | N | H | H | 2-OMe-5-Me-Ph |
| 2-41 | t-Bu | -O- C (=O)- | N | H | H | 1 | CH | H | Me | 2-OMe-5-Me-Ph |
| 2-42 | t-Bu | -O- C (=O)- | N | H | H | 1 | N | H | Me | 2-OMe-5-Me-Ph |
| 2-43 | t-Bu | -O- C (=O)- | N | H | H | 1 | CH | Me | H | 2-OMe-5-Me-Ph |
| 2-44 | t-Bu | -O- C (=O)- | N | H | H | 1 | N | Me | H | 2-OMe-5-Me-Ph |
| 2-45 | t-Bu | -O- C (=O)- | N | H | H | 1 | CH | H | F | 2-OMe-5-Me-Ph |
| 2-46 | t-Bu | -O- C (=O)- | N | H | H | 1 | N | H | F | 2-OMe-5-Me-Ph |
| 2-47 | t-Bu | -O- C (=O)- | N | H | H | 1 | CH | F | H | 2-OMe-5-Me-Ph |
| 2-48 | t-Bu | -O- C (=O)- | N | H | H | 1 | N | F | H | 2-OMe-5-Me-Ph |
| 2-49 | t-Bu | -O- C (=O)- | N | H | H | 2 | CH | H | H | 2-OMe-5-Me-Ph |

(continued)

| Compound No. | $R^2$ | A | X | $R^{4b}$ | $R^{4a}$ | m | U | $R^{3b}$ | $R^{3a}$ | $R^1$ |
|---|---|---|---|---|---|---|---|---|---|---|
| 2-50 | t-Bu | -O- C (=O)- | N | H | H | 2 | N | H | H | 2-OMe-5-Me-Ph |
| 2-51 | t-Bu | -O- C (=O)- | N | H | Me | 1 | CH | H | H | 2-OMe-5-Me-Ph |
| 2-52 | t-Bu | -O- C (=O)- | N | H | Me | 1 | N | H | H | 2-OMe-5-Me-Ph |
| 2-53 | t-Bu | -O- C (=O)- | N | Me | H | 1 | CH | H | H | 2-OMe-5-Me-Ph |
| 2-54 | t-Bu | -O- C (=O)- | N | Me | H | 1 | N | H | H | 2-OMe-5-Me-Ph |
| 2-55 | t-Bu | -O- C (=O)- | N | H | Et | 1 | CH | H | H | 2-OMe-5-Me-Ph |
| 2-56 | t-Bu | -O- C (=O)- | N | H | Et | 1 | N | H | H | 2-OMe-5-Me-Ph |
| 2-57 | t-Bu | -O- C (=O)- | N | Et | H | 1 | CH | H | H | 2-OMe-5-Me-Ph |
| 2-58 | t-Bu | -O- C (=O)- | N | Et | H | 1 | N | H | H | 2-OMe-5-Me-Ph |
| 2-59 | t-Bu | -O- C (=O)- | CH | H | H | 1 | CH | H | H | 2-OMe-5-Me-Ph |
| 2-60 | t-Bu | -O- C (=O)- | CH | H | H | 1 | N | H | H | 2-OMe-5-Me-Ph |
| 2-61 | Ph | -C (=O)- | N | H | H | 1 | CH | H | Me | 2-OMe-5-Me-Ph |
| 2-62 | Ph | -C (=O)- | N | H | H | 1 | N | H | Me | 2-OMe-5-Me-Ph |
| 2-63 | Ph | -C (=O)- | N | H | H | 1 | CH | Me | H | 2-OMe-5-Me-Ph |
| 2-64 | Ph | -C (=O)- | N | H | H | 1 | N | Me | H | 2-OMe-5-Me-Ph |
| 2-65 | Ph | -C (=O)- | N | H | H | 1 | CH | H | F | 2-OMe-5-Me-Ph |
| 2-66 | Ph | -C (=O)- | N | H | H | 1 | N | H | F | 2-OMe-5-Me-Ph |
| 2-67 | Ph | -C (=O)- | N | H | H | 1 | CH | F | H | 2-OMe-5-Me-Ph |
| 2-68 | Ph | -C (=O)- | N | H | H | 1 | N | F | H | 2-OMe-5-Me-Ph |
| 2-69 | Ph | -C (=O)- | N | H | H | 2 | CH | H | H | 2-OMe-5-Me-Ph |
| 2-70 | Ph | -C (=O)- | N | H | H | 2 | N | H | H | 2-OMe-5-Me-Ph |
| 2-71 | Ph | -C (=O)- | N | H | Me | 1 | CH | H | H | 2-OMe-5-Me-Ph |
| 2-72 | Ph | -C (=O)- | N | H | Me | 1 | N | H | H | 2-OMe-5-Me-Ph |
| 2-73 | Ph | -C (=O)- | N | Me | H | 1 | CH | H | H | 2-OMe-5-Me-Ph |
| 2-74 | Ph | -C (=O)- | N | Me | H | 1 | N | H | H | 2-OMe-5-Me-Ph |
| 2-75 | Ph | -C (=O)- | N | H | Et | 1 | CH | H | H | 2-OMe-5-Me-Ph |
| 2-76 | Ph | -C (=O)- | N | H | Et | 1 | N | H | H | 2-OMe-5-Me-Ph |
| 2-77 | Ph | -C (=O)- | N | Et | H | 1 | CH | H | H | 2-OMe-5-Me-Ph |
| 2-78 | Ph | -C (=O)- | N | Et | H | 1 | N | H | H | 2-OMe-5-Me-Ph |
| 2-79 | Ph | -C (=O)- | CH | H | H | 1 | CH | H | H | 2-OMe-5-Me-Ph |
| 2-80 | Ph | -C (=O)- | CH | H | H | 1 | N | H | H | 2-OMe-5-Me-Ph |
| 2-81 | 3-Me-2-Py | -NH- C (=O)- | N | H | H | 1 | CH | H | Me | 2-OMe-5-Me-Ph |
| 2-82 | 3-Me-2-Py | -NH- C (=O)- | N | H | H | 1 | N | H | Me | 2-OMe-5-Me-Ph |

(continued)

| Compound No. | $R^2$ | A | X | $R^{4b}$ | $R^{4a}$ | m | U | $R^{3b}$ | $R^{3a}$ | $R^1$ |
|---|---|---|---|---|---|---|---|---|---|---|
| 2-83 | 3-Me-2-Py | -NH- C (=O)- | N | H | H | 1 | CH | Me | H | 2-OMe-5-Me-Ph |
| 2-84 | 3-Me-2-Py | -NH- C (=O)- | N | H | H | 1 | N | Me | H | 2-OMe-5-Me-Ph |
| 2-85 | 3-Me-2-Py | -NH- C (=O)- | N | H | H | 1 | CH | F | H | 2-OMe-5-Me-Ph |
| 2-86 | 3-Me-2-Py | -NH- C (=O)- | N | H | H | 1 | N | F | H | 2-OMe-5-Me-Ph |
| 2-87 | 3-Me-2-Py | -NH- C (=O)- | N | H | H | 2 | CH | H | H | 2-OMe-5-Me-Ph |
| 2-88 | 3-Me-2-Py | -NH- C (=O)- | N | H | H | 1 | CH | CN | H | 2-OMe-5-Me-Ph |
| 2-89 | 3-Me-2-Py | -NH- C (=O)- | N | Me | H | 1 | CH | H | H | 2-OMe-5-Me-Ph |
| 2-90 | 3-Me-2-Py | -NH- C (=O)- | N | Me | H | 1 | N | H | H | 2-OMe-5-Me-Ph |
| 2-91 | 2-Cl-4-$OCH_2CH_2OH$-Ph | -NH- C (=O)- | N | H | H | 1 | CH | H | Me | 2-OMe-5-Me-Ph |
| 2-92 | 2-Cl-4-$OCH_2CH_2OH$-Ph | -NH- C (=O)- | N | H | H | 1 | N | H | Me | 2-OMe-5-Me-Ph |
| 2-93 | 2-Cl-4-$OCH_2CH_2OH$-Ph | -NH- C (=O)- | N | H | H | 1 | CH | Me | H | 2-OMe-5-Me-Ph |
| 2-94 | 2-Cl-4-$OCH_2CH_2OH$-Ph | -NH- C (=O)- | N | H | H | 1 | N | Me | H | 2-OMe-5-Me-Ph |
| 2-95 | 2-Cl-4-$OCH_2CH_2OH$-Ph | -NH- C (=O)- | N | H | H | 1 | CH | F | H | 2-OMe-5-Me-Ph |
| 2-96 | 2-Cl-4-$OCH_2CH_2OH$-Ph | -NH- C (=O)- | N | H | H | 1 | N | F | H | 2-OMe-5-Me-Ph |
| 2-97 | 2-Cl-4-$OCH_2CH_2OH$-Ph | -NH- C (=O)- | N | H | H | 2 | CH | H | H | 2-OMe-5-Me-Ph |
| 2-98 | 2-Cl-4-$OCH_2CH_2OH$-Ph | -NH- C (=O)- | N | H | H | 1 | CH | CN | H | 2-OMe-5-Me-Ph |
| 2-99 | 2-Cl-4-$OCH_2CH_2OH$-Ph | -NH- C (=O)- | N | Me | H | 1 | CH | H | H | 2-OMe-5-Me-Ph |
| 2-100 | 2-Cl-4-$OCH_2CH_2OH$-Ph | -NH- C (=O)- | N | Me | H | 1 | N | H | H | 2-OMe-5-Me-Ph |
| 2-101 | 4-$OCH_2CH_2OH$-2-$CF_3$-Ph | -NH- C (=O)- | N | H | H | 1 | CH | H | Me | 2-OMe-5-Me-Ph |
| 2-102 | 4-$OCH_2CH_2OH$-2-$CF_3$-Ph | -NH- C (=O)- | N | H | H | 1 | N | H | Me | 2-OMe-5-Me-Ph |
| 2-103 | 4-$OCH_2CH_2OH$-2-$CF_3$-Ph | -NH- C (=O)- | N | H | H | 1 | CH | Me | H | 2-OMe-5-Me-Ph |
| 2-104 | 4-$OCH_2CH_2OH$-2-$CF_3$-Ph | -NH- C (=O)- | N | H | H | 1 | N | Me | H | 2-OMe-5-Me-Ph |
| 2-105 | 4-$OCH_2CH_2OH$-2-$CF_3$-Ph | -NH- C (=O)- | N | H | H | 1 | CH | F | H | 2-OMe-5-Me-Ph |
| 2-106 | 4-$OCH_2CH_2OH$-2-$CF_3$-Ph | -NH- C (=O)- | N | H | H | 1 | N | F | H | 2-OMe-5-Me-Ph |
| 2-107 | 4-$OCH_2CH_2OH$-2-$CF_3$-Ph | -NH- C (=O)- | N | H | H | 2 | CH | H | H | 2-OMe-5-Me-Ph |
| 2-108 | 4-$OCH_2CH_2OH$-2-$CF_3$-Ph | -NH- C (=O)- | N | H | H | 1 | CH | CN | H | 2-OMe-5-Me-Ph |
| 2-109 | 4-$OCH_2CH_2OH$-2-$CF_3$-Ph | -NH- C (=O)- | N | Me | H | 1 | CH | H | H | 2-OMe-5-Me-Ph |
| 2-110 | 4-$OCH_2CH_2OH$-2-$CF_3$-Ph | -NH- C (=O)- | N | Me | H | 1 | N | H | H | 2-OMe-5-Me-Ph |
| 2-111 | 2-Cl-5-$OCH_2CH_2OH$-Ph | -NH- C (=O)- | N | H | H | 1 | CH | H | Me | 2-OMe-5-Me-Ph |
| 2-112 | 2-Cl-5-$OCH_2CH_2OH$-Ph | -NH- C (=O)- | N | H | H | 1 | N | H | Me | 2-OMe-5-Me-Ph |
| 2-113 | 2-Cl-5-$OCH_2CH_2OH$-Ph | -NH- C (=O)- | N | H | H | 1 | CH | Me | H | 2-OMe-5-Me-Ph |
| 2-114 | 2-Cl-5-$OCH_2CH_2OH$-Ph | -NH- C (=O)- | N | H | H | 1 | N | Me | H | 2-OMe-5-Me-Ph |
| 2-115 | 2-Cl-5-$OCH_2CH_2OH$-Ph | -NH- C (=O)- | N | H | H | 1 | CH | F | H | 2-OMe-5-Me-Ph |

(continued)

| Compound No. | $R^2$ | A | X | $R^{4b}$ | $R^{4a}$ | m | U | $R^{3b}$ | $R^{3a}$ | $R^1$ |
|---|---|---|---|---|---|---|---|---|---|---|
| 2-116 | 2-Cl-5-$OCH_2CH_2OH$-Ph | -NH- C (=O)- | N | H | H | 1 | N | F | H | 2-OMe-5-Me-Ph |
| 2-117 | 2-Cl-5-$OCH_2CH_2OH$-Ph | -NH- C (=O)- | N | H | H | 2 | CH | H | H | 2-OMe-5-Me-Ph |
| 2-118 | 2-Cl-5-$OCH_2CH_2OH$-Ph | -NH- C (=O)- | N | H | H | 1 | CH | CN | H | 2-OMe-5-Me-Ph |
| 2-119 | 2-Cl-5-$OCH_2CH_2OH$-Ph | -NH- C (=O)- | N | Me | H | 1 | CH | H | H | 2-OMe-5-Me-Ph |
| 2-120 | 2-Cl-5-$OCH_2CH_2OH$-Ph | -NH- C (=O)- | N | Me | H | 1 | N | H | H | 2-OMe-5-Me-Ph |
| 2-121 | 4-$OCH_2CH_2OH$-2-$CF_3$-Ph | -NH- C (=O)- | N | H | H | 1 | CH | Cl | H | 2-F-Ph |
| 2-122 | 4-$OCH_2CH_2OH$-2-$CF_3$-Ph | -NH- C (=O)- | N | H | H | 1 | N | Cl | H | 2-F-Ph |
| 2-123 | 4-$OCH_2CH_2OH$-2-$CF_3$-Ph | -NH- C (=O)- | N | H | H | 1 | CH | F | H | 2-F-Ph |
| 2-124 | 4-$OCH_2CH_2OH$-2-$CF_3$-Ph | -NH- C (=O)- | N | H | H | 1 | N | F | H | 2-F-Ph |
| 2-125 | 4-$OCH_2CH_2OH$-2-$CF_3$-Ph | -NH- C (=O)- | N | Me | H | 1 | CH | H | H | 2-F-Ph |
| 2-126 | 4-$OCH_2CH_2OH$-2-$CF_3$-Ph | -NH- C (=O)- | N | Me | H | 1 | N | H | H | 2-F-Ph |
| 2-127 | 3-Me-2-Py | -NH- C (=O)- | N | H | H | 1 | CH | Cl | H | 2-OMe-Ph |
| 2-128 | 3-Me-2-Py | -NH- C (=O)- | N | H | H | 1 | N | Cl | H | 2-OMe-Ph |
| 2-129 | 3-Me-2-Py | -NH- C (=O)- | N | H | H | 1 | CH | F | H | 2-OMe-Ph |
| 2-130 | 3-Me-2-Py | -NH- C (=O)- | N | H | H | 1 | N | F | H | 2-OMe-Ph |
| 2-131 | 3-Me-2-Py | -NH- C (=O)- | N | Me | H | 1 | CH | H | H | 2-OMe-Ph |
| 2-132 | 3-Me-2-Py | -NH- C (=O)- | N | Me | H | 1 | N | H | H | 2-OMe-Ph |
| 2-133 | 2-Cl-4-$OCH_2CH_2OH$-Ph | -NH- C (=O)- | N | H | H | 1 | CH | Cl | H | 2-OMe-Ph |
| 2-134 | 2-Cl-4-$OCH_2CH_2OH$-Ph | -NH- C (=O)- | N | H | H | 1 | N | Cl | H | 2-OMe-Ph |
| 2-135 | 2-Cl-4-$OCH_2CH_2OH$-Ph | -NH- C (=O)- | N | H | H | 1 | CH | F | H | 2-OMe-Ph |
| 2-136 | 2-Cl-4-$OCH_2CH_2OH$-Ph | -NH- C (=O)- | N | H | H | 1 | N | F | H | 2-OMe-Ph |
| 2-137 | 2-Cl-4-$OCH_2CH_2OH$-Ph | -NH- C (=O)- | N | Me | H | 1 | CH | H | H | 2-OMe-Ph |
| 2-138 | 2-Cl-4-$OCH_2CH_2OH$-Ph | -NH- C (=O)- | N | Me | H | 1 | N | H | H | 2-OMe-Ph |
| 2-139 | 2-Cl-5-$OCH_2CH_2OH$-Ph | -NH- C (=O)- | N | H | H | 1 | CH | Cl | H | 2-OMe-Ph |
| 2-140 | 2-Cl-5-$OCH_2CH_2OH$-Ph | -NH- C (=O)- | N | H | H | 1 | N | Cl | H | 2-OMe-Ph |
| 2-141 | 2-Cl-5-$OCH_2CH_2OH$-Ph | -NH- C (=O)- | N | H | H | 1 | CH | F | H | 2-OMe-Ph |
| 2-142 | 2-Cl-5-$OCH_2CH_2OH$-Ph | -NH- C (=O)- | N | H | H | 1 | N | F | H | 2-OMe-Ph |
| 2-143 | 2-Cl-5-$OCH_2CH_2OH$-Ph | -NH- C (=O)- | N | Me | H | 1 | CH | H | H | 2-OMe-Ph |
| 2-144 | 2-Cl-5-$OCH_2CH_2OH$-Ph | -NH- C (=O)- | N | Me | H | 1 | N | H | H | 2-OMe-Ph |
| 2-145 | 3-Me-2-Py | -NH- C (=O)- | N | H | H | 1 | CH | Cl | H | 2-F-Ph |
| 2-146 | 3-Me-2-Py | -NH- C (=O)- | CH | H | H | 1 | N | H | H | 2-OMe-5-Me-Ph |
| 2-147 | 3-Me-2-Py | -NH- C (=O)- | CH | H | H | 1 | CH | H | H | 2-OMe-5-Me-Ph |
| 2-148 | 3,5-di-F-2-Py | -NH- C (=O)- | N | H | H | 1 | CH | F | H | 2-OMe-Ph |

(continued)

| Compound No. | $R^2$ | A | X | $R^{4b}$ | $R^{4a}$ | m | U | $R^{3b}$ | $R^{3a}$ | $R^1$ |
|---|---|---|---|---|---|---|---|---|---|---|
| 2-149 | 3,5-di-F-2-Py | -NH- C (=O)- | N | H | H | 1 | N | F | H | 2-OMe-Ph |
| 2-150 | 3-CN-2-Py | -NH- C (=O)- | N | H | H | 1 | CH | F | H | 2-OMe-Ph |
| 2-151 | 3-CN-2-Py | -NH- C (=O)- | N | H | H | 1 | N | F | H | 2-OMe-Ph |

(Table 3)

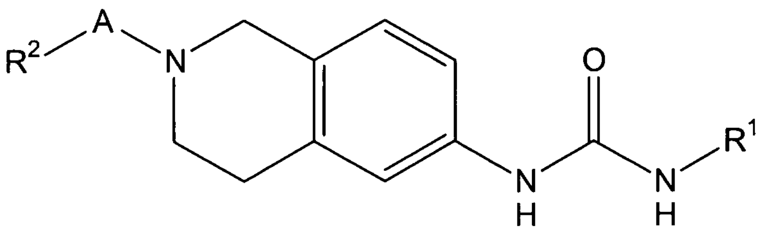

(VII)

| Compound No. | $R^2$ | A | $R^1$ |
|---|---|---|---|
| 3-1 | 2,6-di-F-Ph | -NH- C (=O)- | 5-F-2-OMe-Ph |
| 3-2 | 2,6-di-F-Ph | -NH- C (=O)- | 2-OMe-5-Me-Ph |
| 3-3 | 2,6-di-F-Ph | -NH- C (=O)- | 2-OEt-Ph |
| 3-4 | 2,6-di-F-Ph | -O- C (=O)- | 5-F-2-OMe-Ph |
| 3-5 | 2,6-di-F-Ph | -O- C (=O)- | 2-OMe-5-Me-Ph |
| 3-6 | 2,6-di-F-Ph | -O- C (=O)- | 2-OEt-Ph |
| 3-7 | 2,6-di-Cl-Ph | -NH- C (=O)- | 5-F-2-OMe-Ph |
| 3-8 | 2,6-di-Cl-Ph | -NH- C (=O)- | 2-OMe-5-Me-Ph |
| 3-9 | 2,6-di-Cl-Ph | -NH- C (=O)- | 2-OEt-Ph |
| 3-10 | 2,6-di-Cl-Ph | -O- C (=O)- | 5-F-2-OMe-Ph |
| 3-11 | 2,6-di-Cl-Ph | -O- C (=O)- | 2-OMe-5-Me-Ph |
| 3-12 | 2,6-di-Cl-Ph | -O- C (=O)- | 2-OEt-Ph |
| 3-13 | 2-Cl-6-Me-Ph | -NH- C (=O)- | 5-F-2-OMe-Ph |
| 3-14 | 2-Cl-6-Me-Ph | -NH- C (=O)- | 2-OMe-5-Me-Ph |
| 3-15 | 2-Cl-6-Me-Ph | -NH- C (=O)- | 2-OEt-Ph |
| 3-16 | 2-Cl-6-Me-Ph | -O- C (=O)- | 5-F-2-OMe-Ph |
| 3-17 | 2-Cl-6-Me-Ph | -O- C (=O)- | 2-OMe-5-Me-Ph |
| 3-18 | 2-Cl-6-Me-Ph | -O- C (=O)- | 2-OEt-Ph |
| 3-19 | 2,6-di-Me-Ph | -NH- C (=O)- | 5-F-2-OMe-Ph |
| 3-20 | 2,6-di-Me-Ph | -NH- C (=O)- | 2-OMe-5-Me-Ph |
| 3-21 | 2,6-di-Me-Ph | -NH- C (=O)- | 2-OEt-Ph |
| 3-22 | 2,6-di-Me-Ph | -O- C (=O)- | 5-F-2-OMe-Ph |
| 3-23 | 2,6-di-Me-Ph | -O- C (=O)- | 2-OMe-5-Me-Ph |
| 3-24 | 2,6-di-Me-Ph | -O- C (=O)- | 2-OEt-Ph |
| 3-25 | 2-OMe-6-Me-Ph | -NH- C (=O)- | 5-F-2-OMe-Ph |
| 3-26 | 2-OMe-6-Me-Ph | -NH- C (=O)- | 2-OMe-5-Me-Ph |
| 3-27 | 2-OMe-6-Me-Ph | -NH- C (=O)- | 2-OEt-Ph |
| 3-28 | 2-OMe-6-Me-Ph | -O- C (=O)- | 5-F-2-OMe-Ph |
| 3-29 | 2-OMe-6-Me-Ph | -O- C (=O)- | 2-OMe-5-Me-Ph |
| 3-30 | 2-OMe-6-Me-Ph | -O- C (=O)- | 2-OEt-Ph |
| 3-31 | 2-Cl-4-Me-Ph | -NH- C (=O)- | 5-F-2-OMe-Ph |
| 3-32 | 2-Cl-4-Me-Ph | -NH- C (=O)- | 2-OMe-5-Me-Ph |
| 3-33 | 2-Cl-4-Me-Ph | -NH- C (=O)- | 2-OEt-Ph |
| 3-34 | 2-Cl-4-Me-Ph | -O- C (=O)- | 5-F-2-OMe-Ph |
| 3-35 | 2-Cl-4-Me-Ph | -O- C (=O)- | 2-OMe-5-Me-Ph |
| 3-36 | 2-Cl-4-Me-Ph | -O- C (=O)- | 2-OEt-Ph |
| 3-37 | 4-OMe-2-Me-Ph | -NH- C (=O)- | 5-F-2-OMe-Ph |
| 3-38 | 4-OMe-2-Me-Ph | -NH- C (=O)- | 2-OMe-5-Me-Ph |

(continued)

| Compound No. | $R^2$ | A | $R^1$ |
|---|---|---|---|
| 3-39 | 4-OMe-2-Me-Ph | -NH- C (=O)- | 2-OEt-Ph |
| 3-40 | 4-OMe-2-Me-Ph | -O- C (=O)- | 5-F-2-OMe-Ph |
| 3-41 | 4-OMe-2-Me-Ph | -O- C (=O)- | 2-OMe-5-Me-Ph |
| 3-42 | 4-OMe-2-Me-Ph | -O- C (=O)- | 2-OEt-Ph |
| 3-43 | 2-Me-Ph | -NH- C (=O)- | 5-F-2-OMe-Ph |
| 3-44 | 2-Me-Ph | -NH- C (=O)- | 2-OMe-5-Me-Ph |
| 3-45 | 2-Me-Ph | -NH- C (=O)- | 2-OEt-Ph |
| 3-46 | 2-Me-Ph | -O- C (=O)- | 5-F-2-OMe-Ph |
| 3-47 | 2-Me-Ph | -O- C (=O)- | 2-OMe-5-Me-Ph |
| 3-48 | 2-Me-Ph | -O- C (=O)- | 2-OEt-Ph |
| 3-49 | 2,6-di-F-Ph | -NH- C (=O)- | 2-F-Ph |
| 3-50 | 2,6-di-F-Ph | -NH- C (=O)- | 2-OMe-Ph |
| 3-51 | 3-Me-2-Py | -NH- C (=O)- | 2-F-Ph |
| 3-52 | 3-Me-2-Py | -NH- C (=O)- | 2-OMe-Ph |
| 3-53 | 3-Me-2-Py | -NH- C (=O)- | 3-OEt-Ph |
| 3-54 | 3-Me-2-Py | -NH- C (=O)- | 2-OMe-5-Me-Ph |
| 3-55 | 3-Me-2-Py | -NH- C (=O)- | 3,5-di-OMe-Ph |
| 3-56 | 3-Me-2-Py | -O- C (=O)- | 2-F-Ph |
| 3-57 | 3-Me-2-Py | -O- C (=O)- | 2-OMe-Ph |
| 3-58 | 3-Me-2-Py | -O- C (=O)- | 3-OEt-Ph |
| 3-59 | 3-Me-2-Py | -O- C (=O)- | 2-OMe-5-Me-Ph |
| 3-60 | 3-Me-2-Py | -O- C (=O)- | 3,5-di-OMe-Ph |
| 3-61 | 2-Me-CycHex | -NH- C (=O)- | 2-F-Ph |
| 3-62 | 2-Me-CycHex | -NH- C (=O)- | 2-OMe-Ph |
| 3-63 | 2-Me-CycHex | -NH- C (=O)- | 3-OEt-Ph |
| 3-64 | 2-Me-CycHex | -NH- C (=O)- | 2-OMe-5-Me-Ph |
| 3-65 | 2-Me-CycHex | -NH- C (=O)- | 3,5-di-OMe-Ph |
| 3-66 | 2-Me-CycHex | -O- C (=O)- | 2-F-Ph |
| 3-67 | 2-Me-CycHex | -O- C (=O)- | 2-OMe-Ph |
| 3-68 | 2-Me-CycHex | -O- C (=O)- | 3-OEt-Ph |
| 3-69 | 2-Me-CycHex | -O- C (=O)- | 2-OMe-5-Me-Ph |
| 3-70 | 2-Me-CycHex | -O- C (=O)- | 3,5-di-OMe-Ph |
| 3-71 | 2-Cl-4-$OCH_2CH_2OH$-Ph | -NH- C (=O)- | 2-F-Ph |
| 3-72 | 2-Cl-4-$OCH_2CH_2OH$-Ph | -NH- C (=O)- | 2-OMe-Ph |
| 3-73 | 2-Cl-4-$OCH_2CH_2OH$-Ph | -NH- C (=O)- | 3-OEt-Ph |
| 3-74 | 2-Cl-4-$OCH_2CH_2OH$-Ph | -NH- C (=O)- | 2-OMe-5-Me-Ph |
| 3-75 | 2-Cl-4-$OCH_2CH_2OH$-Ph | -NH- C (=O)- | 3,5-di-OMe-Ph |
| 3-76 | 2-Cl-4-$OCH_2CH_2OH$-Ph | -O- C (=O)- | 2-F-Ph |
| 3-77 | 2-Cl-4-$OCH_2CH_2OH$-Ph | -O- C (=O)- | 2-OMe-Ph |
| 3-78 | 2-Cl-4-$OCH_2CH_2OH$-Ph | -O- C (=O)- | 3-OEt-Ph |
| 3-79 | 2-Cl-4-$OCH_2CH_2OH$-Ph | -O- C (=O)- | 2-OMe-5-Me-Ph |
| 3-80 | 2-Cl-4-$OCH_2CH_2OH$-Ph | -O- C (=O)- | 3,5-di-OMe-Ph |
| 3-81 | Ph(A) | -NH- C (=O)- | 2-F-Ph |
| 3-82 | Ph(A) | -NH- C (=O)- | 2-OMe-Ph |
| 3-83 | Ph(A) | -NH- C (=O)- | 3-OEt-Ph |
| 3-84 | Ph(A) | -NH- C (=O)- | 2-OMe-5-Me-Ph |
| 3-85 | Ph(A) | -NH- C (=O)- | 3,5-di-OMe-Ph |
| 3-86 | Ph(A) | -O- C (=O)- | 2-F-Ph |
| 3-87 | Ph(A) | -O- C (=O)- | 2-OMe-Ph |
| 3-88 | Ph(A) | -O- C (=O)- | 3-OEt-Ph |

(continued)

| Compound No. | $R^2$ | A | $R^1$ |
|---|---|---|---|
| 3-89 | Ph(A) | -O- C (=O)- | 2-OMe-5-Me-Ph |
| 3-90 | Ph(A) | -O- C (=O)- | 3,5-di-OMe-Ph |
| 3-91 | 4-$OCH_2CH_2OH$-2-$CF_3$-Ph | -NH- C (=O)- | 2-F-Ph |
| 3-92 | 4-$OCH_2CH_2OH$-2-$CF_3$-Ph | -NH- C (=O)- | 2-OMe-Ph |
| 3-93 | 4-$OCH_2CH_2OH$-2-$CF_3$-Ph | -NH- C (=O)- | 3-OEt-Ph |
| 3-94 | 4-$OCH_2CH_2OH$-2-$CF_3$-Ph | -NH- C (=O)- | 2-OMe-5-Me-Ph |
| 3-95 | 4-$OCH_2CH_2OH$-2-$CF_3$-Ph | -NH- C (=O)- | 3,5-di-OMe-Ph |
| 3-96 | 4-$OCH_2CH_2OH$-2-$CF_3$-Ph | -O- C (=O)- | 2-F-Ph |
| 3-97 | 4-$OCH_2CH_2OH$-2-$CF_3$-Ph | -O- C (=O)- | 2-OMe-Ph |
| 3-98 | 4-$OCH_2CH_2OH$-2-$CF_3$-Ph | -O- C (=O)- | 3-OEt-Ph |
| 3-99 | 4-$OCH_2CH_2OH$-2-$CF_3$-Ph | -O- C (=O)- | 2-OMe-5-Me-Ph |
| 3-100 | 4-$OCH_2CH_2OH$-2-$CF_3$-Ph | -O- C (=O)- | 3,5-di-OMe-Ph |
| 3-101 | Ph(B) | -NH- C (=O)- | 2-F-Ph |
| 3-102 | Ph(B) | -NH- C (=O)- | 2-OMe-Ph |
| 3-103 | Ph(B) | -NH- C (=O)- | 3-OEt-Ph |
| 3-104 | Ph(B) | -NH- C (=O)- | 2-OMe-5-Me-Ph |
| 3-105 | Ph(B) | -NH- C (=O)- | 3,5-di-OMe-Ph |
| 3-106 | Ph(B) | -O- C (=O)- | 2-F-Ph |
| 3-107 | Ph(B) | -O- C (=O)- | 2-OMe-Ph |
| 3-108 | Ph(B) | -O- C (=O)- | 3-OEt-Ph |
| 3-109 | Ph(B) | -O- C (=O)- | 2-OMe-5-Me-Ph |
| 3-110 | Ph(B) | -O- C (=O)- | 3,5-di-OMe-Ph |
| 3-111 | 2-Cl-5-$OCH_2CH_2OH$-Ph | -NH- C (=O)- | 2-F-Ph |
| 3-112 | 2-Cl-5-$OCH_2CH_2OH$-Ph | -NH- C (=O)- | 2-OMe-Ph |
| 3-113 | 2-Cl-5-$OCH_2CH_2OH$-Ph | -NH- C (=O)- | 3-OEt-Ph |
| 3-114 | 2-Cl-5-$OCH_2CH_2OH$-Ph | -NH- C (=O)- | 2-OMe-5-Me-Ph |
| 3-115 | 2-Cl-5-$OCH_2CH_2OH$-Ph | -NH- C (=O)- | 3,5-di-OMe-Ph |
| 3-116 | 2-Cl-5-$OCH_2CH_2OH$-Ph | -O- C (=O)- | 2-F-Ph |
| 3-117 | 2-Cl-5-$OCH_2CH_2OH$-Ph | -O- C (=O)- | 2-OMe-Ph |
| 3-118 | 2-Cl-5-$OCH_2CH_2OH$-Ph | -O- C (=O)- | 3-OEt-Ph |
| 3-119 | 2-Cl-5-$OCH_2CH_2OH$-Ph | -O- C (=O)- | 2-OMe-5-Me-Ph |
| 3-120 | 2-Cl-5-$OCH_2CH_2OH$-Ph | -O- C (=O)- | 3,5-di-OMe-Ph |
| 3-121 | Ph(C) | -NH- C (=O)- | 2-F-Ph |
| 3-122 | Ph(C) | -NH- C (=O)- | 2-OMe-Ph |
| 3-123 | Ph(C) | -NH- C (=O)- | 3-OEt-Ph |
| 3-124 | Ph(C) | -NH- C (=O)- | 2-OMe-5-Me-Ph |
| 3-125 | Ph(C) | -NH- C (=O)- | 3,5-di-OMe-Ph |
| 3-126 | Ph(C) | -O- C (=O)- | 2-F-Ph |
| 3-127 | Ph(C) | -O- C (=O)- | 2-OMe-Ph |
| 3-128 | Ph(C) | -O- C (=O)- | 3-OEt-Ph |
| 3-129 | Ph(C) | -O- C (=O)- | 2-OMe-5-Me-Ph |
| 3-130 | Ph(C) | -O- C (=O)- | 3,5-di-OMe-Ph |
| 3-131 | 2-Cl-4-$CO_2Me$-Ph | -NH- C (=O)- | 2-F-Ph |
| 3-132 | 2-Cl-4-$CO_2Me$-Ph | -NH- C (=O)- | 2-OMe-Ph |
| 3-133 | 2-Cl-4-$CO_2Me$-Ph | -NH- C (=O)- | 3-OEt-Ph |
| 3-134 | 2-Cl-4-$CO_2Me$-Ph | -NH- C (=O)- | 2-OMe-5-Me-Ph |
| 3-135 | 2-Cl-4-$CO_2Me$-Ph | -NH- C (=O)- | 3,5-di-OMe-Ph |
| 3-136 | 2-Cl-4-$CO_2Me$-Ph | -O- C (=O)- | 2-F-Ph |
| 3-137 | 2-Cl-4-$CO_2Me$-Ph | -O- C (=O)- | 2-OMe-Ph |
| 3-138 | 2-Cl-4-$CO_zMe$-Ph | -O- C (=O)- | 3-OEt-Ph |

(continued)

| Compound No. | $R^2$ | A | $R^1$ |
|---|---|---|---|
| 3-139 | 2-Cl-4-$CO_2$Me-Ph | -O- C (=O)- | 2-OMe-5-Me-Ph |
| 3-140 | 2-Cl-4-$CO_2$Me-Ph | -O- C (=O)- | 3,5-di-OMe-Ph |
| 3-141 | 4-$CO_2$H-2-Cl-Ph | -NH- C (=O)- | 2-F-Ph |
| 3-142 | 4-$CO_2$H-2-Cl-Ph | -NH- C (=O)- | 2-OMe-Ph |
| 3-143 | 4-$CO_2$H-2-Cl-Ph | -NH- C (=O)- | 3-OEt-Ph |
| 3-144 | 4-$CO_2$H-2-Cl-Ph | -NH- C (=O)- | 2-OMe-5-Me-Ph |
| 3-145 | 4-$CO_2$H-2-Cl-Ph | -NH- C (=O)- | 3,5-di-OMe-Ph |
| 3-146 | 4-$CO_2$H-2-Cl-Ph | -O- C (=O)- | 2-F-Ph |
| 3-147 | 4-$CO_2$H-2-Cl-Ph | -O- C (=O)- | 2-OMe-Ph |
| 3-148 | 4-$CO_2$H-2-Cl-Ph | -O- C (=O)- | 3-OEt-Ph |
| 3-149 | 4-$CO_2$H-2-Cl-Ph | -O- C (=O)- | 2-OMe-5-Me-Ph |
| 3-150 | 4-$CO_2$H-2-Cl-Ph | -O- C (=O)- | 3,5-di-OMe-Ph |
| 3-151 | 4-$CH_2CO_2$H-2-Cl-Ph | -NH- C (=O)- | 2-F-Ph |
| 3-152 | 4-$CH_2CO_2$H-2-Cl-Ph | -NH- C (=O)- | 2-OMe-Ph |
| 3-153 | 4-$CH_2CO_2$H-2-Cl-Ph | -NH- C (=O)- | 3-OEt-Ph |
| 3-154 | 4-$CH_2CO_2$H-2-Cl-Ph | -NH- C (=O)- | 2-OMe-5-Me-Ph |
| 3-155 | 4-$CH_2CO_2$H-2-Cl-Ph | -NH- C (=O)- | 3,5-di-OMe-Ph |
| 3-156 | 4-$CH_2CO_2$H-2-Cl-Ph | -O- C (=O)- | 2-F-Ph |
| 3-157 | 4-$CH_2CO_2$H-2-Cl-Ph | -O- C (=O)- | 2-OMe-Ph |
| 3-158 | 4-$CH_2CO_2$H-2-Cl-Ph | -O- C (=O)- | 3-OEt-Ph |
| 3-159 | 4-$CH_2CO_2$H-2-Cl-Ph | -O- C (=O)- | 2-OMe-5-Me-Ph |
| 3-160 | 4-$CH_2CO_2$H-2-Cl-Ph | -O- C (=O)- | 3,5-di-OMe-Ph |

(Table 4)

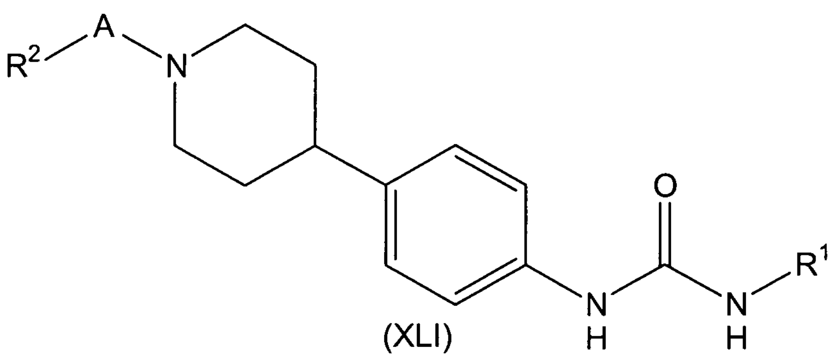

(XLI)

| Compound No. | $R^2$ | A | $R^1$ |
|---|---|---|---|
| 4-1 | 3-Me-2-Py | -NH- C (=O)- | 2-F-Ph |
| 4-2 | 3-Me-2-Py | -NH- C (=O)- | 2-OMe-Ph |
| 4-3 | 3-Me-2-Py | -NH- C (=O)- | 3-OEt-Ph |
| 4-4 | 3-Me-2-Py | -NH- C (=O)- | 2-OMe-5-Me-Ph |
| 4-5 | 3-Me-2-Py | -NH- C (=O)- | 3,5-di-OMe-Ph |
| 4-6 | 3-Me-2-Py | -CH (OH)-C (=O)- | 2-F-Ph |
| 4-7 | 3-Me-2-Py | -CH (OH)-C (=O)- | 2-OMe-Ph |
| 4-8 | 3-Me-2-Py | -CH (OH)-C (=O)- | 3-OEt-Ph |
| 4-9 | 3-Me-2-Py | -CH (OH)-C (=O)- | 2-OMe-5-Me-Ph |
| 4-10 | 3-Me-2-Py | -CH (OH)-C (=O)- | 3,5-di-OMe-Ph |

(continued)

| Compound No. | $R^2$ | A | $R^1$ |
|---|---|---|---|
| 4-11 | 2-Cl-6-Me-Ph | -NH- C (=O)- | 2-F-Ph |
| 4-12 | 2-Cl-6-Me-Ph | -NH- C (=O)- | 2-OMe-Ph |
| 4-13 | 2-Cl-6-Me-Ph | -NH- C (=O)- | 3-OEt-Ph |
| 4-14 | 2-Cl-6-Me-Ph | -NH- C (=O)- | 2-OMe-5-Me-Ph |
| 4-15 | 2-Cl-6-Me-Ph | -NH- C (=O)- | 3,5-di-OMe-Ph |
| 4-16 | 2-Cl-6-Me-Ph | -CH (OH)-C (=O)- | 2-F-Ph |
| 4-17 | 2-Cl-6-Me-Ph | -CH (OH)-C (=O)- | 2-OMe-Ph |
| 4-18 | 2-Cl-6-Me-Ph | -CH (OH)-C (=O)- | 3-OEt-Ph |
| 4-19 | 2-Cl-6-Me-Ph | -CH (OH)-C (=O)- | 2-OMe-5-Me-Ph |
| 4-20 | 2-Cl-6-Me-Ph | -CH (OH)-C (=O)- | 3,5-di-OMe-Ph |
| 4-21 | 2-Cl-4-$OCH_2CH_2OH$-Ph | -NH- C (=O)- | 2-F-Ph |
| 4-22 | 2-Cl-4-$OCH_2CH_2OH$-Ph | -NH- C (=O)- | 2-OMe-Ph |
| 4-23 | 2-Cl-4-$OCH_2CH_2OH$-Ph | -NH- C (=O)- | 3-OEt-Ph |
| 4-24 | 2-Cl-4-$OCH_2CH_2OH$-Ph | -NH- C (=O)- | 2-OMe-5-Me-Ph |
| 4-25 | 2-Cl-4-$OCH_2CH_2OH$-Ph | -NH- C (=O)- | 3,5-di-OMe-Ph |
| 4-26 | 2-Cl-4-$OCH_2CH_2OH$-Ph | -CH (OH)-C (=O)- | 2-F-Ph |
| 4-27 | 2-Cl-4-$OCH_2CH_2OH$-Ph | -CH (OH)-C (=O)- | 2-OMe-Ph |
| 4-28 | 2-Cl-4-$OCH_2CH_2OH$-Ph | -CH (OH)-C (=O)- | 3-OEt-Ph |
| 4-29 | 2-Cl-4-$OCH_2CH_2OH$-Ph | -CH (OH)-C (=O)- | 2-OMe-5-Me-Ph |
| 4-30 | 2-Cl-4-$OCH_2CH_2OH$-Ph | -CH (OH)-C (=O)- | 3,5-di-OMe-Ph |
| 4-31 | Ph(A) | -NH- C (=O)- | 2-F-Ph |
| 4-32 | Ph(A) | -NH- C (=O)- | 2-OMe-Ph |
| 4-33 | Ph(A) | -NH- C (=O)- | 3-OEt-Ph |
| 4-34 | Ph(A) | -NH- C (=O)- | 2-OMe-5-Me-Ph |
| 4-35 | Ph(A) | -NH- C (=O)- | 3,5-di-OMe-Ph |
| 4-36 | Ph(A) | -CH (OH)-C (=O)- | 2-F-Ph |
| 4-37 | Ph(A) | -CH (OH)-C (=O)- | 2-OMe-Ph |
| 4-38 | Ph(A) | -CH (OH)-C (=O)- | 3-OEt-Ph |
| 4-39 | Ph(A) | -CH (OH)-C (=O)- | 2-OMe-5-Me-Ph |
| 4-40 | Ph(A) | -CH (OH)-C (=O)- | 3,5-di-OMe-Ph |
| 4-41 | 4-$OCH_2CH_2OH$-2-$CF_3$-Ph | -NH- C (=O)- | 2-F-Ph |
| 4-42 | 4-$OCH_2CH_2OH$-2-$CF_3$-Ph | -NH- C (=O)- | 2-OMe-Ph |
| 4-43 | 4-$OCH_2CH_2OH$-2-$CF_3$-Ph | -NH- C (=O)- | 3-OEt-Ph |
| 4-44 | 4-$OCH_2CH_2OH$-2-$CF_3$-Ph | -NH- C (=O)- | 2-OMe-5-Me-Ph |
| 4-45 | 4-$OCH_2CH_2OH$-2-$CF_3$-Ph | -NH- C (=O)- | 3,5-di-OMe-Ph |
| 4-46 | 4-$OCH_2CH_2OH$-2-$CF_3$-Ph | -CH (OH)-C (=O)- | 2-F-Ph |
| 4-47 | 4-$OCH_2CH_2OH$-2-$CF_3$-Ph | -CH (OH)-C (=O)- | 2-OMe-Ph |
| 4-48 | 4-$OCH_2CH_2OH$-2-$CF_3$-Ph | -CH (OH)-C (=O)- | 3-OEt-Ph |
| 4-49 | 4-$OCH_2CH_2OH$-2-$CF_3$-Ph | -CH (OH)-C (=O)- | 2-OMe-5-Me-Ph |
| 4-50 | 4-$OCH_2CH_2OH$-2-$CF_3$-Ph | -CH (OH)-C (=O)- | 3,5-di-OMe-Ph |
| 4-51 | Ph(B) | -NH- C (=O)- | 2-F-Ph |
| 4-52 | Ph(B) | -NH- C (=O)- | 2-OMe-Ph |
| 4-53 | Ph(B) | -NH- C (=O)- | 3-OEt-Ph |
| 4-54 | Ph(B) | -NH- C (=O)- | 2-OMe-5-Me-Ph |
| 4-55 | Ph(B) | -NH- C (=O)- | 3,5-di-OMe-Ph |
| 4-56 | Ph(B) | -CH (OH)-C (=O)- | 2-F-Ph |
| 4-57 | Ph(B) | -CH (OH)-C (=O)- | 2-OMe-Ph |
| 4-58 | Ph(B) | -CH (OH)-C (=O)- | 3-OEt-Ph |
| 4-59 | Ph(B) | -CH (OH)-C (=O)- | 2-OMe-5-Me-Ph |
| 4-60 | Ph(B) | -CH (OH)-C (=O)- | 3,5-di-OMe-Ph |

(continued)

| Compound No. | $R^2$ | A | $R^1$ |
|---|---|---|---|
| 4-61 | 2-Cl-5-$OCH_2CH_2OH$-Ph | -NH- C (=O)- | 2-F-Ph |
| 4-62 | 2-Cl-5-$OCH_2CH_2OH$-Ph | -NH- C (=O)- | 2-OMe-Ph |
| 4-63 | 2-Cl-5-$OCH_2CH_2OH$-Ph | -NH- C (=O)- | 3-OEt-Ph |
| 4-64 | 2-Cl-5-$OCH_2CH_2OH$-Ph | -NH- C (=O)- | 2-OMe-5-Me-Ph |
| 4-65 | 2-Cl-5-$OCH_2CH_2OH$-Ph | -NH- C (=O)- | 3,5-di-OMe-Ph |
| 4-66 | 2-Cl-5-$OCH_2CH_2OH$-Ph | -CH (OH)-C (=O)- | 2-F-Ph |
| 4-67 | 2-Cl-5-$OCH_2CH_2OH$-Ph | -CH (OH)-C (=O)- | 2-OMe-Ph |
| 4-68 | 2-Cl-5-$OCH_2CH_2OH$-Ph | -CH (OH)-C (=O)- | 3-OEt-Ph |
| 4-69 | 2-Cl-5-$OCH_2CH_2OH$-Ph | -CH (OH)-C (=O)- | 2-OMe-5-Me-Ph |
| 4-70 | 2-Cl-5-$OCH_2CH_2OH$-Ph | -CH (OH)-C (=O)- | 3,5-di-OMe-Ph |
| 4-71 | Ph(C) | -NH- C (=O)- | 2-F-Ph |
| 4-72 | Ph(C) | -NH- C (=O)- | 2-OMe-Ph |
| 4-73 | Ph(C) | -NH- C (=O)- | 3-OEt-Ph |
| 4-74 | Ph(C) | -NH- C (=O)- | 2-OMe-5-Me-Ph |
| 4-75 | Ph(C) | -NH- C (=O)- | 3,5-di-OMe-Ph |
| 4-76 | Ph(C) | -CH (OH)-C (=O)- | 2-F-Ph |
| 4-77 | Ph(C) | -CH (OH)-C (=O)- | 2-OMe-Ph |
| 4-78 | Ph(C) | -CH (OH)-C (=O)- | 3-OEt-Ph |
| 4-79 | Ph(C) | -CH (OH)-C (=O)- | 2-OMe-5-Me-Ph |
| 4-80 | Ph(C) | -CH (OH)-C (=O)- | 3,5-di-OMe-Ph |
| 4-81 1 | 3,5-di-F-2-Py | -NH- C (=O)- | 2-F-Ph |
| 4-82 | 3,5-di-F-2-Py | -NH- C (=O)- | 2-OMe-Ph |
| 4-83 | 3,5-di-F-2-Py | -NH- C (=O)- | 3-OEt-Ph |
| 4-84 | 3,5-di-F-2-Py | -NH- C (=O)- | 2-OMe-5-Me-Ph |
| 4-85 5 | 3,5-di-F-2-Py | -NH- C (=O)- | 3,5-di-OMe-Ph |
| 4-86 6 | 3,5-di-F-2-Py | -CH (OH)-C (=O)- | 2-F-Ph |
| 4-87 | 3,5-di-F-2-Py | -CH (OH)-C (=O)- | 2-OMe-Ph |
| 4-88 8 | 3,5-di-F-2-Py | -CH (OH)-C (=O)- | 3-OEt-Ph |
| 4-89 | 3,5-di-F-2-Py | -CH (OH)-C (=O)- | 2-OMe-5-Me-Ph |
| 4-90 | 3,5-di-F-2-Py | -CH (OH)-C (=O)- | 3,5-di-OMe-Ph |
| 4-91 | 3-CN-2-Py | -NH- C (=O)- | 2-F-Ph |
| 4-92 | 3-CN-2-Py | -NH- C (=O)- | 2-OMe-Ph |
| 4-93 | 3-CN-2-Py | -NH- C (=O)- | 3-OEt-Ph |
| 4-94 | 3-CN-2-Py | -NH- C (=O)- | 2-OMe-5-Me-Ph |
| 4-95 | 3-CN-2-Py | -NH- C (=O)- | 3,5-di-OMe-Ph |
| 4-96 | 3-CN-2-Py | -CH (OH)-C (=O)- | 2-F-Ph |
| 4-97 | 3-CN-2-Py | -CH (OH)-C (=O)- | 2-OMe-Ph |
| 4-98 | 3-CN-2-Py | -CH (OH)-C (=O)- | 3-OEt-Ph |
| 4-99 | 3-CN-2-Py | -CH (OH)-C (=O)- | 2-OMe-5-Me-Ph |
| 4-100 | 3-CN-2-Py | -CH (OH)-C (=O)- | 3,5-di-OMe-Ph |
| 4-101 | 3-Cl-2-Py | -NH- C (=O)- | 2-F-Ph |
| 4-102 | 3-Cl-2-Py | -NH- C (=O)- | 2-OMe-Ph |
| 4-103 | 3-Cl-2-Py | -NH- C (=O)- | 3-OEt-Ph |
| 4-104 | 3-Cl-2-Py | -NH- C (=O)- | 2-OMe-5-Me-Ph |
| 4-105 | 3-Cl-2-Py | -NH- C (=O)- | 3,5-di-OMe-Ph |
| 4-106 | 3-Cl-2-Py | -NH- C (=O)- | 2-OMe-3-Me-Ph |
| 4-107 | 3-Cl-2-Py | -NH- C (=O)- | 2-OEt-Ph |
| 4-108 | 3-$CH_2OH$-2-Py | -NH- C (=O)- | 2-OMe-Ph |
| 4-109 | 3-$CH_2OH$-2-Py | -NH- C (=O)- | 3-OEt-Ph |
| 4-110 | 3-$CH_2OH$-2-Py | -NH- C (=O)- | 2-OMe-5-Me-Ph |

(continued)

| Compound No. | $R^2$ | A | $R^1$ |
|---|---|---|---|
| 4-111 | 3-F-2-Py | -NH- C (=O)- | 2-OMe-Ph |
| 4-112 | 3-F-2-Py | -NH- C (=O)- | 3-OEt-Ph |
| 4-113 | 3-F-2-Py | -NH- C (=O)- | 2-OMe-5-Me-Ph |
| 4-114 | 3-Me-2-Py | -NH- C (=O)- | 5-Me-2-thiazo |
| 4-115 | 3-Me-2-Py | -NH- C (=O)- | 4-tBu-2-thiazo |

(Table 5)

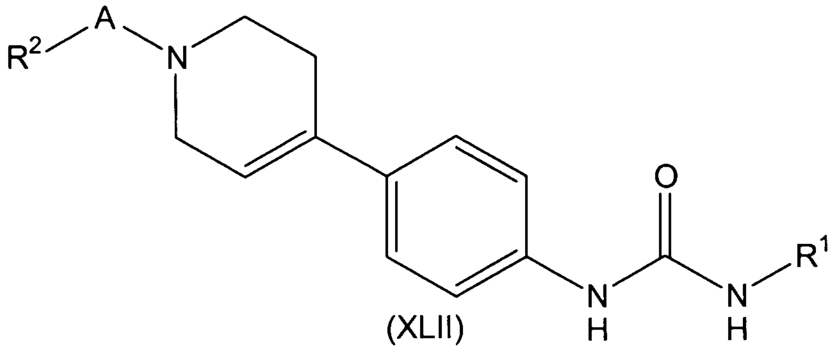

(XLII)

| Compound No. | $R^2$ | A | $R^1$ |
|---|---|---|---|
| 5-1 | 3-Me-2-Py | -NH- C (=O)- | 2-F-Ph |
| 5-2 | 3-Me-2-Py | -NH- C (=O)- | 2-OMe-Ph |
| 5-3 | 3-Me-2-Py | -NH- C (=O)- | 3-OEt-Ph |
| 5-4 | 3-Me-2-Py | -NH- C (=O)- | 2-OMe-5-Me-Ph |
| 5-5 | 3-Me-2-Py | -NH- C (=O)- | 3,5-di-OMe-Ph |
| 5-6 | 3-Me-2-Py | -CH (OH)-C (=O)- | 2-F-Ph |
| 5-7 | 3-Me-2-Py | -CH (OH)-C (=O)- | 2-OMe-Ph |
| 5-8 | 3-Me-2-Py | -CH (OH)-C (=O)- | 3-OEt-Ph |
| 5-9 | 3-Me-2-Py | -CH (OH)-C (=O)- | 2-OMe-5-Me-Ph |
| 5-10 | 3-Me-2-Py | -CH (OH)-C (=O)- | 3,5-di-OMe-Ph |
| 5-11 | 2-Cl-6-Me-Ph | -NH- C (=O)- | 2-F-Ph |
| 5-12 | 2-Cl-6-Me-Ph | -NH- C (=O)- | 2-OMe-Ph |
| 5-13 | 2-Cl-6-Me-Ph | -NH- C (=O)- | 3-OEt-Ph |
| 5-14 | 2-Cl-6-Me-Ph | -NH- C (=O)- | 2-OMe-5-Me-Ph |
| 5-15 | 2-Cl-6-Me-Ph | -NH- C (=O)- | 3,5-di-OMe-Ph |
| 5-16 | 2-Cl-6-Me-Ph | -CH (OH)-C (=O)- | 2-F-Ph |
| 5-17 | 2-Cl-6-Me-Ph | -CH (OH)-C (=O)- | 2-OMe-Ph |
| 5-18 | 2-Cl-6-Me-Ph | -CH (OH)-C (=O)- | 3-OEt-Ph |
| 5-19 | 2-Cl-6-Me-Ph | -CH (OH)-C (=O)- | 2-OMe-5-Me-Ph |
| 5-20 | 2-Cl-6-Me-Ph | -CH (OH)-C (=O)- | 3,5-di-OMe-Ph |
| 5-21 | 2-Cl-4-$OCH_2CH_2OH$-Ph | -NH- C (=O)- | 2-F-Ph |
| 5-22 | 2-Cl-4-$OCH_2CH_2OH$-Ph | -NH- C (=O)- | 2-OMe-Ph |
| 5-23 | 2-Cl-4-$OCH_2CH_2OH$-Ph | -NH- C (=O)- | 3-OEt-Ph |
| 5-24 | 2-Cl-4-$OCH_2CH_2OH$-Ph | -NH- C (=O)- | 2-OMe-5-Me-Ph |
| 5-25 | 2-Cl-4-$OCH_2CH_2OH$-Ph | -NH- C (=O)- | 3,5-di-OMe-Ph |
| 5-26 | 2-Cl-4-$OCH_2CH_2OH$-Ph | -CH (OH)-C (=O)- | 2-F-Ph |
| 5-27 | 2-Cl-4-$OCH_2CH_2OH$-Ph | -CH (OH)-C (=O)- | 2-OMe-Ph |
| 5-28 | 2-Cl-4-$OCH_2CH_2OH$-Ph | -CH (OH)-C (=O)- | 3-OEt-Ph |

(continued)

| Compound No. | $R^2$ | A | $R^1$ |
|---|---|---|---|
| 5-29 | 2-Cl-4-$OCH_2CH_2OH$-Ph | -CH (OH)-C (=O)- | 2-OMe-5-Me-Ph |
| 5-30 | 2-Cl-4-$OCH_2CH_2OH$-Ph | -CH (OH)-C (=O)- | 3,5-di-OMe-Ph |
| 5-31 | Ph(A) | -NH- C (=O)- | 2-F-Ph |
| 5-32 | Ph(A) | -NH- C (=O)- | 2-OMe-Ph |
| 5-33 | Ph(A) | -NH- C (=O)- | 3-OEt-Ph |
| 5-34 | Ph(A) | -NH- C (=O)- | 2-OMe-5-Me-Ph |
| 5-35 | Ph(A) | -NH- C (=O)- | 3,5-di-OMe-Ph |
| 5-36 | Ph(A) | -CH (OH)-C (=O)- | 2-F-Ph |
| 5-37 | Ph(A) | -CH (OH)-C (=O)- | 2-OMe-Ph |
| 5-38 | Ph(A) | -CH (OH)-C (=O)- | 3-OEt-Ph |
| 5-39 | Ph(A) | -CH (OH)-C (=O)- | 2-OMe-5-Me-Ph |
| 5-40 | Ph(A) | -CH (OH)-C (=O)- | 3,5-di-OMe-Ph |
| 5-41 | 4-$OCH_2CH_2OH$-2-$CF_3$-Ph | -NH- C (=O)- | 2-F-Ph |
| 5-42 | 4-$OCH_2CH_2OH$-2-$CF_3$-Ph | -NH- C (=O)- | 2-OMe-Ph |
| 5-43 | 4-$OCH_2CH_2OH$-2-$CF_3$-Ph | -NH- C (=O)- | 3-OEt-Ph |
| 5-44 | 4-$OCH_2CH_2OH$-2-$CF_3$-Ph | -NH- C (=O)- | 2-OMe-5-Me-Ph |
| 5-45 | 4-$OCH_2CH_2OH$-2-$CF_3$-Ph | -NH- C (=O)- | 3,5-di-OMe-Ph |
| 5-46 | 4-$OCH_2CH_2OH$-2-$CF_3$-Ph | -CH (OH)-C (=O)- | 2-F-Ph |
| 5-47 | 4-$OCH_2CH_2OH$-2-$CF_3$-Ph | -CH (OH)-C (=O)- | 2-OMe-Ph |
| 5-48 | 4-$OCH_2CH_2OH$-2-$CF_3$-Ph | -CH (OH)-C (=O)- | 3-OEt-Ph |
| 5-49 | 4-$OCH_2CH_2OH$-2-$CF_3$-Ph | -CH (OH)-C (=O)- | 2-OMe-5-Me-Ph |
| 5-50 | 4-$OCH_2CH_2OH$-2-$CF_3$-Ph | -CH (OH)-C (=O)- | 3,5-di-OMe-Ph |
| 5-51 | Ph(B) | -NH- C (=O)- | 2-F-Ph |
| 5-52 | Ph(B) | -NH- C (=O)- | 2-OMe-Ph |
| 5-53 | Ph(B) | -NH- C (=O)- | 3-OEt-Ph |
| 5-54 | Ph(B) | -NH- C (=O)- | 2-OMe-5-Me-Ph |
| 5-55 | Ph(B) | -NH- C (=O)- | 3,5-di-OMe-Ph |
| 5-56 | Ph(B) | -CH (OH)-C (=O)- | 2-F-Ph |
| 5-57 | Ph(B) | -CH (OH)-C (=O)- | 2-OMe-Ph |
| 5-58 | Ph(B) | -CH (OH)-C (=O)- | 3-OEt-Ph |
| 5-59 | Ph(B) | -CH (OH)-C (=O)- | 2-OMe-5-Me-Ph |
| 5-60 | Ph(B) | -CH (OH)-C (=O)- | 3,5-di-OMe-Ph |
| 5-61 | 2-Cl-5-$OCH_2CH_2OH$-Ph | -NH- C (=O)- | 2-F-Ph |
| 5-62 | 2-Cl-5-$OCH_2CH_2OH$-Ph | -NH- C (=O)- | 2-OMe-Ph |
| 5-63 | 2-Cl-5-$OCH_2CH_2OH$-Ph | -NH- C (=O)- | 3-OEt-Ph |
| 5-64 | 2-Cl-5-$OCH_2CH_2OH$-Ph | -NH- C (=O)- | 2-OMe-5-Me-Ph |
| 5-65 | 2-Cl-5-$OCH_2CH_2OH$-Ph | -NH- C (=O)- | 3,5-di-OMe-Ph |
| 5-66 | 2-Cl-5-$OCH_2CH_2OH$-Ph | -CH (OH)-C (=O)- | 2-F-Ph |
| 5-67 | 2-Cl-5-$OCH_2CH_2OH$-Ph | -CH (OH)-C (=O)- | 2-OMe-Ph |
| 5-68 | 2-Cl-5-$OCH_2CH_2OH$-Ph | -CH (OH)-C (=O)- | 3-OEt-Ph |
| 5-69 | 2-Cl-5-$OCH_2CH_2OH$-Ph | -CH (OH)-C (=O)- | 2-OMe-5-Me-Ph |
| 5-70 | 2-Cl-5-$OCH_2CH_2OH$-Ph | -CH (OH)-C (=O)- | 3,5-di-OMe-Ph |
| 5-71 | Ph(C) | -NH- C (=O)- | 2-F-Ph |
| 5-72 | Ph(C) | -NH- C (=O)- | 2-OMe-Ph |
| 5-73 | Ph(C) | -NH- C (=O)- | 3-OEt-Ph |
| 5-74 | Ph(C) | -NH- C (=O)- | 2-OMe-5-Me-Ph |
| 5-75 | Ph(C) | -NH- C (=O)- | 3,5-di-OMe-Ph |
| 5-76 | Ph(C) | -CH (OH)-C (=O)- | 2-F-Ph |
| 5-77 | Ph(C) | -CH (OH)-C (=O)- | 2-OMe-Ph |
| 5-78 | Ph(C) | -CH (OH)-C (=O)- | 3-OEt-Ph |

(continued)

| Compound No. | $R^2$ | A | $R^1$ |
|---|---|---|---|
| 5-79 | Ph(C) | -CH (OH)-C (=O)- | 2-OMe-5-Me-Ph |
| 5-80 | Ph(C) | -CH (OH)-C (=O)- | 3,5-di-OMe-Ph |
| 5-81 | 3,5-di-F-2-Py | -NH- C (=O)- | 2-F-Ph |
| 5-82 | 3,5-di-F-2-Py | -NH- C (=O)- | 2-OMe-Ph |
| 5-83 | 3,5-di-F-2-Py | -NH- C (=O)- | 3-OEt-Ph |
| 5-84 | 3,5-di-F-2-Py | -NH- C (=O)- | 2-OMe-5-Me-Ph |
| 5-85 | 3,5-di-F-2-Py | -NH- C (=O)- | 3,5-di-OMe-Ph |
| 5-86 | 3,5-di-F-2-Py | -CH (OH)-C (=O)- | 2-F-Ph |
| 5-87 | 3,5-di-F-2-Py | -CH (OH)-C (=O)- | 2-OMe-Ph |
| 5-88 | 3,5-di-F-2-Py | -CH (OH)-C (=O)- | 3-OEt-Ph |
| 5-89 | 3,5-di-F-2-Py | -CH (OH)-C (=O)- | 2-OMe-5-Me-Ph |
| 5-90 | 3,5-di-F-2-Py | -CH (OH)-C (=O)- | 3,5-di-OMe-Ph |
| 5-91 | 3-CN-2-Py | -NH- C (=O)- | 2-F-Ph |
| 5-92 | 3-CN-2-Py | -NH- C (=O)- | 2-OMe-Ph |
| 5-93 | 3-CN-2-Py | -NH- C (=O)- | 3-OEt-Ph |
| 5-94 | 3-CN-2-Py | -NH- C (=O)- | 2-OMe-5-Me-Ph |
| 5-95 | 3-CN-2-Py | -NH- C (=O)- | 3,5-di-OMe-Ph |
| 5-96 | 3-CN-2-Py | -CH (OH)-C (=O)- | 2-F-Ph |
| 5-97 | 3-CN-2-Py | -CH (OH)-C (=O)- | 2-OMe-Ph |
| 5-98 | 3-CN-2-Py | -CH (OH)-C (=O)- | 3-OEt-Ph |
| 5-99 | 3-CN-2-Py | -CH (OH)-C (=O)- | 2-OMe-5-Me-Ph |
| 5-100 | 3-CN-2-Py | -CH (OH)-C (=O)- | 3,5-di-OMe-Ph |
| 5-101 | 3-Cl-2-Py | -NH- C (=O)- | 2-F-Ph |
| 5-102 | 3-Cl-2-Py | -NH- C (=O)- | 2-OMe-Ph |
| 5-103 | 3-Cl-2-Py | -NH- C (=O)- | 3-OEt-Ph |
| 5-104 | 3-Cl-2-Py | -NH- C (=O)- | 2-OMe-5-Me-Ph |
| 5-105 | 3-Cl-2-Py | -NH- C (=O)- | 3,5-di-OMe-Ph |
| 5-106 | 3-Cl-2-Py | -NH- C (=O)- | 2-OMe-3-Me-Ph |
| 5-107 | 3-Cl-2-Py | -NH- C (=O)- | 2-OEt-Ph |
| 5-108 | 3-$CH_2$OH-2-Py | -NH- C (=O)- | 2-OMe-Ph |
| 5-109 | 3-$CH_2$OH-2-Py | -NH- C (=O)- | 3-OEt-Ph |
| 5-110 | 3-$CH_2$OH-2-Py | -NH- C (=O)- | 2-OMe-5-Me-Ph |
| 5-111 | 3-F-2-Py | -NH- C (=O)- | 2-OMe-Ph |
| 5-112 | 3-F-2-Py | -NH- C (=O)- | 3-OEt-Ph |
| 5-113 | 3-F-2-Py | -NH- C (=O)- | 2-OMe-5-Me-Ph |
| 5-114 | 3-Me-2-Py | -NH- C (=O)- | 5-Me-2-thiazo |
| 5-115 | 3-Me-2-Py | -NH- C (=O)- | 4-tBu-2-thiazo |

In Table 1 to Table 5, preferable compounds are compound Nos. 1-524, 1-525, 1-526, 1-527, 1-528, 1-529, 1-530, 1-531, 1-532, 1-533, 1-536, 1-539, 1-540, 1-541, 1-542, 1-543, 1-544, 1-548, 1-549, 1-555, 1-557, 1-567, 1-572, 1-579, 1-596, 1-597, 1-599, 1-603, 1-605, 1-606, 1-610, 1-627, 1-635, 1-645, 1-651, 1-653, 1-657, 1-663, 1-692, 1-755, 1-761, 1-787, 1-844, 1-846, 1-852, 1-854, 1-856, 1-858, 1-859, 1-866, 1-868, 1-870, 1-871, 1-873, 1-874, 1-876, 1-881, 1-882, 1-884, 1-885, 1-886, 1-888, 1-889, 1-890, 1-891, 1-892, 1-893, 1-895, 1-896, 1-897, 1-898, 1-899, 1-900, 1-902, 2-83, 2-85, 2-95, 2-99, 2-103, 2-107, 2-121, 2-128, 2-145, 3-72, 3-74, 3-75, 3-84, 3-85, 3-94, 4-1, 4-2, 4-4, 4-5, 4-22, 4-24, 4-32, 4-34, 4-35, 4-81, 4-82, 4-83, 4-84, 4-91, 4-92, 4-94, 4-95, 5-1, 5-2, 5-4, 5-5, 5-22, 5-25, 5-32, 5-34, 5-35, 5-41, 5-44, 5-54, 5-64, or 5-84,
more preferable compounds are compound Nos. 1-524, 1-526, 1-527, 1-529, 1-531, 1-533, 1-536, 1-539, 1-540, 1-541, 1-542, 1-543, 1-548, 1-549, 1-555, 1-557, 1-567, 1-572, 1-579, 1-596, 1-597, 1-599, 1-603, 1-605, 1-606, 1-627, 1-651, 1-653, 1-663, 1-692, 1-755, 1-761, 1-787, 1-844, 1-846, 1-852, 1-856, 1-858, 1-866, 1-882, 1-884, 1-888, 1-895, 1-897, 1-898, 1-899, 1-900, 1-902, 2-95, 2-121, 2-128, 2-145, 3-74, 3-94, 4-24, 4-32, 4-34, 4-81, 4-82, 4-83, 4-84, 4-91, 4-92, 4-95, 5-1, 5-2, 5-4, 5-5, 5-22, 5-25, 5-32, 5-34, 5-35, 5-41, 5-44, 5-54, or 5-84,

further more preferable compounds are compound Nos. 1-524, 1-526, 1-527, 1-529, 1-531, 1-533, 1-536, 1-539, 1-540, 1-541, 1-542, 1-543, 1-549, 1-555, 1-557, 1-572, 1-579, 1-597, 1-603, 1-606, 1-627, 1-651, 1-663, 1-692, 1-761, 1-787, 1-844, 1-846, 1-856, 1-858, 1-866, 1-884, 1-888, 1-897, 2-121, 2-145, 3-94, 5-1, 5-2, 5-4, 5-5, 5-22, 5-25, 5-32, 5-34, 5-35, 5-41, 5-44, or 5-84,

particularly preferable compounds are

4-{4-[3-(2-ethoxy-phenyl)-ureido]-phenyl}-piperazine-1-carboxylic acid (3-methyl-pyridin-2-yl)-amide (compound No. 1-526),

4-{4-[3-(2-methoxy-5-methyl-phenyl)-ureido]-phenyl}-piperazine-1-carboxylic acid (3-methyl-pyridin-2-yl)-amide (compound No. 1-531),

4-{4-[3-(3,5-dimethoxy-phenyl)-ureido]-phenyl}-piperazine-1-carboxylic acid (3-methyl-pyridin-2-yl)-amide (compound No. 1-533),

4- {5-[3-(5-fluoro-2-methoxy-phenyl)-ureido]-pyridin-2-yl}-piperazine-1-carboxylic acid (3-methyl-pyridin-2-yl)-amide (compound No. 1-539),

4-{5-[3-(2-ethoxy-5-fluoro-phenyl)-ureido]-pyridin-2-yl}-piperazine-1-carboxylic acid (3-methyl-pyridin-2-yl)-amide (compound No. 1-540),

4-{5-[3-(5-chloro-2-methoxy-phenyl)-ureido]-pyridin-2-yl}-piperazine-1-carboxylic acid (3-methyl-pyridin-2-yl)-amide (compound No. 1-541),

4- {5-[3-(5-chloro-2-ethoxy-phenyl)-ureido]-pyridin-2-yl} -piperazine-1-carboxylic acid (3-methyl-pyridin-2-yl)-amide (compound No. 1-542),

4-{4-[3-(2-methoxy-5-methyl-phenyl)-ureido]-phenyl}-piperazine-1-carboxylic acid [2-chloro-4-(2-hydroxy-ethoxy)-phenyl]-amide (compound No. 1-555),

4-{4-[3-(3,5-dimethoxy-phenyl)-ureido]-phenyl}-piperazine-1-carboxylic acid [2-chloro-4-(2-hydroxy-ethoxy)-phenyl]-amide (compound No. 1-557),

4-{4-[3-(2-methoxy-5-methyl-phenyl)-ureido]-phenyl}-piperazine-1-carboxylic acid [2-chloro-4-(2,3-dihydroxy-propoxy)-phenyl]-amide (compound No. 1-579),

4-{4-[3-(2-methoxy-5-methyl-phenyl)-ureido]-phenyl}-piperazine-1-carboxylic acid [4-(2-hydroxy-ethoxy)-2-trifluoromethyl-phenyl]-amide (compound No. 1-603),

4-{4-[3-(5-methyl-thiazol-2-yl)-ureido]-phenyl}-piperazine-1-carboxylic acid [4-(2-hydroxy-ethoxy)-2-trifluoromethyl-phenyl]-amide (compound No. 1-606),

4- {4-[3-(2-methoxy-5-methyl-phenyl)-ureido]-phenyl} -piperazine- I -carboxylic acid [4-(2,3-dihydroxy-propoxy)-2-trifluoromethyl-phenyl]-amide (compound No. 1-627),

4-{4-[3-(2-methoxy-5-methyl-phenyl)-ureido]-phenyl}-piperazine-1-carboxylic acid [2-chloro-5-(2-hydroxy-ethoxy)-phenyl]-amide (compound No. 1-651),

4-{4-[3-(2-methoxy-5-methyl-phenyl)-ureido]-phenyl}-piperazine-1-carboxylic acid [4-(2-hydroxy-ethoxy)-2-methyl-phenyl]-amide (compound No. 1-692),

4-{4-[3-(2-methoxy-5-methyl-phenyl)-ureido]-phenyl}-piperazine-1-carboxylic acid [2-fluoro-4-(2-hydroxy-ethoxy)-phenyl]-amide (compound No. 1-761),

4-{4-[3-(2-methoxy-5-methyl-phenyl)-ureido]-phenyl}-piperazine-1-carboxylic acid (3,5-difluoro-pyridin-2-yl)-amide (compound No. 1-787),

4-{4-[3-(3-ethoxy-phenyl)-ureido]-phenyl}-piperazine-1-carboxylic acid [4-(2-hydroxy-ethoxy)-2-trifluoromethyl-phenyl]-amide (compound No. 1-846),

4-{4-[3-(3-ethoxy-phenyl)-ureido]-phenyl}-piperazine-1-carboxylic acid (3-methyl-pyridin-2-yl)-amide (compound No. 1-856),

4-{4-[3-(2-ethoxy-phenyl)-ureido]-phenyl}-piperazine-1-carboxylic acid (3,5-difluoro-pyridin-2-yl)-amide (compound No. 1-884),

4-{4-[3-(2-methoxy-3-methyl-phenyl)-ureido]-phenyl}-piperazine-1-carboxylic acid (3-methyl-pyridin-2-yl)-amide (compound No. 1-897),

4-{2-chloro-4-[3-(2-fluoro-phenyl)-ureido]-phenyl}-piperazine-1-carboxylic acid [4-(2-hydroxy-ethoxy)-2-trifluoromethyl-phenyl]-amide (compound No. 2-121),

4- {4-[3-(2-methoxy-phenyl)-ureido]-phenyl} -3,6-dihydro-2H-pyridine-1-carboxylic acid (3-methyl-pyridin-2-yl)-amide (compound No. 5-2),

4- {4-[3-(2-methoxy-5-methyl-phenyl)-ureido]-phenyl} -3,6-dihydro-2H-pyridine-1-carboxylic acid (3-methyl-pyridin-2-yl)-amide (compound No. 5-4),

4-{4-[3-(3,5-dimethoxy-phenyl)-ureido]-phenyl}-3,6-dihydro-2H-pyridine-1-carboxylic acid (3-methyl-pyridin-2-yl)-amide (compound No. 5-5), or

4- {4-[3-(2-methoxy-5-methyl-phenyl)-ureido]-phenyl]-3,6-dihydro-2H-pyridine-1-carboxylic acid (3,5-difluoro-pyridin-2-yl)-amide (compound No. 5-84),

most preferable compounds are

4-{4-[3-(2-ethoxy-phenyl)-ureido]-phenyl}-piperazine-1-carboxylic acid (3-methyl-pyridin-2-yl)-amide (compound No. 1-526),
4-{4-[3-(2-methoxy-5-methyl-phenyl)-ureido]-phenyl}-piperazine-1-carboxylic acid (3-methyl-pyridin-2-yl)-amide (compound No. 1-531),
4-{4-[3-(3,5-dimethoxy-phenyl)-ureido]-phenyl}-piperazine-1-carboxylic acid (3-methyl-pyridin-2-yl)-amide (compound No. 1-533),
4- {5-[3-(2-ethoxy-5-fluoro-phenyl)-ureido]-pyridin-2-yl} -piperazine-1-carboxylic acid (3-methyl-pyridin-2-yl)-amide (compound No. 1-540),
4-{5-[3-(5-chloro-2-methoxy-phenyl)-ureido]-pyridin-2-yl}-piperazine-1-carboxylic acid (3-methyl-pyridin-2-yl)-amide (compound No. 1-541),
4-{4-[3-(2-methoxy-5-methyl-phenyl)-ureido]-phenyl}-piperazine-1-carboxylic acid [2-chloro-4-(2-hydroxy-ethoxy)-phenyl]-amide (compound No. 1-555),
4-{4-[3-(2-methoxy-5-methyl-phenyl)-ureido]-phenyl}-piperazine-1-carboxylic acid [2-chloro-4-(2,3-dihydroxy-propoxy)-phenyl]-amide (compound No. 1-579),
4-{4-[3-(2-methoxy-5-methyl-phenyl)-ureido]-phenyl}-piperazine-1-carboxylic acid [4-(2-hydroxy-ethoxy)-2-trifluoromethyl-phenyl]-amide (compound No. 1-603),
4-{4-[3-(2-methoxy-S-methyl-phenyl)-ureido]-phenyl}-piperazine-1-carboxylic acid [2-chloro-S-(2-hydroxy-ethoxy)-phenyl]-amide (compound No. 1-651),
4-{4-[3-(3-ethoxy-phenyl)-ureido]-phenyl}-piperazine-1-carboxylic acid (3-methyl-pyridin-2-yl)-amide (compound No. 1-856),
4-{2-chloro-4-[3-(2-fluoro-phenyl)-ureido]-phenyl}-piperazine-1-carboxylic acid [4-(2-hydroxy-ethoxy)-2-trifluoromethyl-phenyl]-amide (compound No. 2-121), or
4-{4-[3-(2-methoxy-5-methyl-phenyl)-ureido]-phenyl}-3,6-dihydro-2H-pyridine-1-carboxylic acid (3-methyl-pyridin-2-yl)-amide (compound No. 5-4).

The compound having the general formula (I) of the present invention can be produced by the process described below.

Process AA is a process for producing a compound having the general formula (IAA).

Process AA

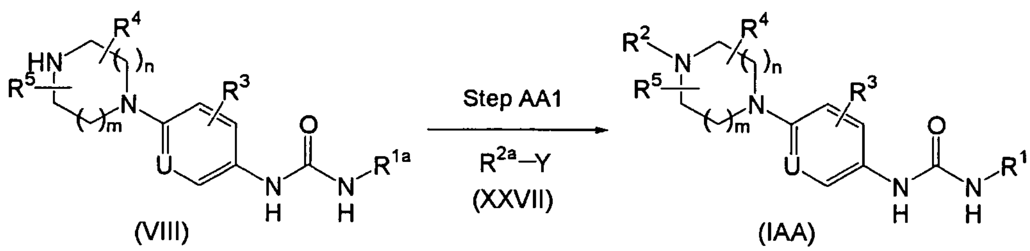


(VIII) (IAA)

In the present invention, $R^1$, $R^2$, $R^3$, $R^4$, $R^5$, U, m and n are the same as described above. $R^{1a}$ and $R^{2a}$ are the same group as $R^1$ and $R^2$ except that the amino group, the hydroxyl group and/or the carboxyl group contained in $R^1$ and $R^2$ as a substituent are/is an amino group, a hydroxyl group and/or a carboxyl group which may be protected. Y represents a halogen atom (e.g., a fluorine atom, a chlorine atom, a bromine atom or an iodine atom, preferably a fluorine atom or a chlorine atom), an aryl sulfonate group (e.g., a phenyl sulfonate group which may be independently mono- or disubstituted by a group selected from a $C_1$-$C_6$ alkyl group and a halogen atom, preferably a 4-methylphenyl sulfonate group) or a $C_1$-$C_6$ alkyl sulfonate group (a group in which a $C_1$-$C_6$ alkyl group is bonded to a sulfonate group, preferably a methyl sulfonate group).

Step AA1

This is a step of producing a compound having the general formula (IAA).

The step is performed by allowing a compound having the general formula (VIII) to react with a compound having the general formula (XXVII) publicly known or easily obtained using a publicly known compound as a starting material as in a known method in an inert solvent in the presence or absence (preferably presence) of a base, and then removing the protecting group of the amino group, the hydroxyl group and/or the carboxyl group in $R^{1a}$ and $R^{2a}$ as desired.

The inert solvent used in this step is not particularly limited as long as the solvent dissolves a certain amount of starting materials without inhibiting the reaction. Examples thereof include hydrocarbons such as petroleum ether;

amides such as formamide, N,N-dimethylformamide, N,N-dimethylacetamide, N-methyl-2-pyrrolidone, N-methylpyrrolidinone and hexamethylphosphoric triamide; ethers such as diethyl ether, diisopropyl ether, tetrahydrofuran, dioxane, dimethoxyethane and diethylene glycol dimethyl ether; sulfoxides such as dimethyl sulfoxide and sulfolane; nitriles such as acetonitrile and isobutyronitrile; esters such as ethyl formate, ethyl acetate, propyl acetate, butyl acetate and diethyl carbonate; ketones such as acetone, methyl ethyl ketone, 4-methyl-2-pentanone, methyl isobutyl ketone, isophorone and cyclohexanone; nitro compounds such as nitroethane and nitrobenzene; halogenated hydrocarbons such as dichloromethane, 1,2-dichloroethane, dichlorobenzene, chloroform and carbon tetrachloride; aromatic hydrocarbons such as benzene, toluene and xylene; or a mixed solvent thereof, preferably amides, more preferably N,N-dimethylacetamide.

Examples of bases used in this step include inorganic bases like alkali metal carbonates such as sodium carbonate, potassium carbonate and lithium carbonate; alkali metal hydrogen carbonates such as sodium hydrogen carbonate, potassium hydrogen carbonate and lithium hydrogen carbonate; alkali metal hydroxides such as sodium hydroxide, potassium hydroxide, barium hydroxide and lithium hydroxide; alkali metal fluorides such as sodium fluoride and potassium fluoride; alkali metal trialkyl siloxides such as sodium trimethylsiloxide, potassium trimethylsiloxide and lithium trimethylsiloxide; or organic bases such as N-methylmorpholine, triethylamine, tripropylamine, tributylamine, diisopropylethylamine, dicyclohexylamine, N-methylpiperidine, pyridine, 4-pyrrolidinopyridine, picoline, 4-(N,N-dimethylamino)pyridine, 2,6-di(t-butyl)-4-methylpyridine, quinoline, N,N-dimethylaniline, N,N-diethylaniline, 1,5-diazabicyclo[4.3.0]non-5-ene (DBN), 1,4-diazabicyclo[2.2.2]octane (DABCO) and 1,8-diazabicyclo[5.4.0]undec-7-ene (DBU), preferably alkali metal carbonates, more preferably potassium carbonate.

The reaction temperature in this step varies depending on the raw material compounds and the inert solvent to be used, and is generally 20˚C to 100˚C, preferably 60˚C to 80˚C.

The reaction time in this step varies depending on the raw material compounds, the inert solvent to be used and the reaction temperature, and is generally 0.5 hour to 24 hours, preferably 4 hours to 8 hours.

Process AB is a process for producing a compound having the general formula (IAB).

Process AB

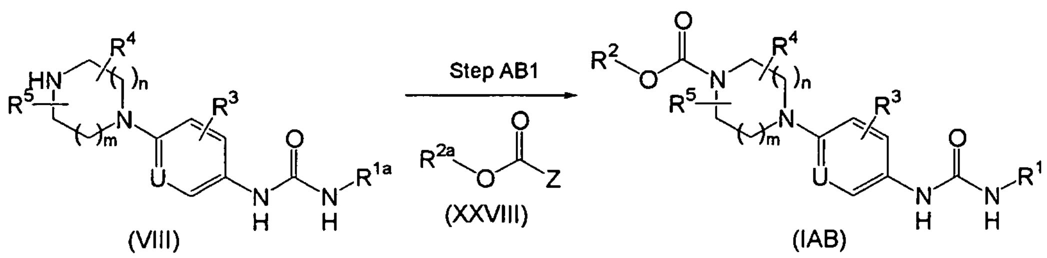


(VIII) (XXVIII) (IAB)

In the present invention, $R^1$, $R^2$, $R^3$, $R^4$, $R^5$, $R^{1a}$, $R^{1a}$, U, m and n are the same as described above, and Z represents a halogen atom (e.g., a fluorine atom, a chlorine atom, a bromine atom or an iodine atom, preferably a chlorine atom).

Step AB1

This is a step of producing a compound having the general formula (IAB).

The step is performed by allowing a compound having the general formula (VIII) to react with a compound having the general formula (XXVIII) publicly known or easily obtained using a publicly known compound as a starting material as in a known method in an inert solvent in the presence or absence (preferably presence) of a base, and then removing the protecting group of the amino group, the hydroxyl group and/or the carboxyl group in $R^{1a}$ and $R^{2a}$ as desired.

The inert solvent used in this step is not particularly limited as long as the solvent dissolves a certain amount of starting materials without inhibiting the reaction. Examples thereof include hydrocarbons such as petroleum ether; amides such as formamide, N,N-dimethylformamide, N,N-dimethylacetamide, N-methyl-2-pyrrolidone, N-methylpyrrolidinone and hexamethylphosphoric triamide; ethers such as diethyl ether, diisopropyl ether, tetrahydrofuran, dioxane, dimethoxyethane and diethylene glycol dimethyl ether; sulfoxides such as dimethyl sulfoxide and sulfolane; nitriles such as acetonitrile and isobutyronitrile; esters such as ethyl formate, ethyl acetate, propyl acetate, butyl acetate and diethyl carbonate; ketones such as acetone, methyl ethyl ketone, 4-methyl-2-pentanone, methyl isobutyl ketone, isophorone and cyclohexanone; nitro compounds such as nitroethane and nitrobenzene; halogenated hydrocarbons such as dichloromethane, 1,2-dichloroethane, dichlorobenzene, chloroform and carbon tetrachloride; aromatic hydrocarbons such as benzene, toluene and xylene; or a mixed solvent thereof, preferably ethers, more preferably tetrahydrofuran.

Examples of bases used in this step include inorganic bases like alkali metal carbonates such as sodium

carbonate, potassium carbonate and lithium carbonate; alkali metal hydrogen carbonates such as sodium hydrogen carbonate, potassium hydrogen carbonate and lithium hydrogen carbonate; alkali metal hydroxides such as sodium hydroxide, potassium hydroxide, barium hydroxide and lithium hydroxide; alkali metal fluorides such as sodium fluoride and potassium fluoride; alkali metal trialkyl siloxides such as sodium trimethylsiloxide, potassium trimethylsiloxide and lithium trimethylsiloxide; or organic bases such as N-methylmorpholine, triethylamine, tripropylamine, tributylamine, diisopropylethylamine, dicyclohexylamine, N-methylpiperidine, pyridine, 4-pyrrolidinopyridine, picoline, 4-(N,N-dimethylamino)pyridine, 2,6-di(t-butyl)-4-methylpyridine, quinoline, N,N-dimethylaniline, N,N-diethylaniline, 1,5-diazabicyclo[4.3.0]non-5-ene (DBN), 1,4-diazabicyclo[2.2.2]octane (DABCO) and 1,8-diazabicyclo[5.4.0]undec-7-ene (DBU), preferably alkali metal carbonates, more preferably potassium carbonate.

The reaction temperature in this step varies depending on the raw material compounds and the inert solvent to be used, and is generally -20˚C to 80˚C, preferably 0˚C to 25˚C.

The reaction time in this step varies depending on the raw material compounds, the inert solvent to be used and the reaction temperature, and is generally 0.5 hour to 24 hours, preferably 2 hours to 4 hours.

Process ACa is a process for producing a compound having the general formula (IAC).

Process ACa

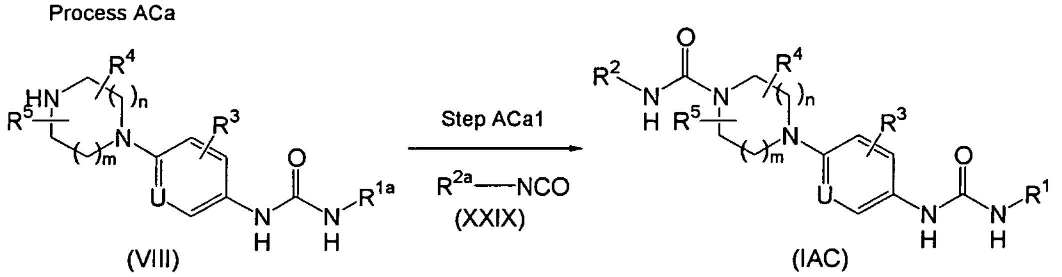


(VIII) (IAC)

In the present invention, $R^1$, $R^2$, $R^3$, $R^4$, $R^5$, $R^{1a}$, $R^{2a}$, U, m and n are the same as described above.

Step ACa1

This is a step of producing a compound having the general formula (IAC).

The step is performed by allowing a compound having the general formula (VIII) to react with a compound having the general formula (XXIX) publicly known or easily obtained using a publicly known compound as a starting material as in a known method in an inert solvent, and then removing the protecting group of the amino group, the hydroxyl group and/or the carboxyl group in $R^{1a}$ and $R^{2a}$ as desired.

The inert solvent used in this step is not particularly limited as long as the solvent dissolves a certain amount of starting materials without inhibiting the reaction. Examples thereof include hydrocarbons such as petroleum ether; amides such as formamide, N,N-dimethylformamide, N,N-dimethylacetamide, N-methyl-2-pyrrolidone, N-methylpyrrolidinone and hexamethylphosphoric triamide; ethers such as diethyl ether, diisopropyl ether, tetrahydrofuran, dioxane, dimethoxyethane and diethylene glycol dimethyl ether; sulfoxides such as dimethyl sulfoxide and sulfolane; nitriles such as acetonitrile and isobutyronitrile; esters such as ethyl formate, ethyl acetate, propyl acetate, butyl acetate and diethyl carbonate; ketones such as acetone, methyl ethyl ketone, 4-methyl-2-pentanone, methyl isobutyl ketone, isophorone and cyclohexanone; nitro compounds such as nitroethane and nitrobenzene; halogenated hydrocarbons such as dichloromethane, 1,2-dichloroethane, dichlorobenzene, chloroform and carbon tetrachloride; aromatic hydrocarbons such as benzene, toluene and xylene; or a mixed solvent thereof, preferably ethers, more preferably tetrahydrofuran.

The reaction temperature in this step varies depending on the raw material compounds and the inert solvent to be used, and is generally -20˚C to 80˚C, preferably 20˚C to 30˚C.

The reaction time in this step varies depending on the raw material compounds, the inert solvent to be used and the reaction temperature, and is generally 0.5 hour to 24 hours, preferably 2 hours to 4 hours.

Process ACb is a process for producing a compound having the general formula (IAC).

Process ACb

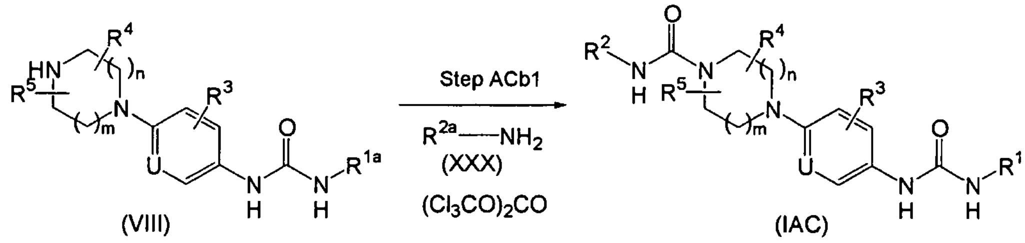


In the present invention, $R^1$, $R^2$, $R^3$, $R^4$, $R^5$, $R^{1a}$, $R^{2a}$, U, m and n are the same as described above.

Step ACb1

This is a step of producing a compound having the general formula (IAC).

The step is performed by allowing a compound having the general formula (VIII) to react with a compound having the general formula (XXX) publicly known or easily obtained using a publicly known compound as a starting material as in a known method and triphosgene in an inert solvent in the presence of a base, and then removing the protecting group of the amino group, the hydroxyl group and/or the carboxyl group in $R^{1a}$ and $R^{2a}$ as desired.

The inert solvent used in this step is not particularly limited as long as the solvent dissolves starting materials without inhibiting the reaction, and examples thereof include hydrocarbons such as petroleum ether; amides such as formamide, N,N-dimethylformamide, N,N-dimethylacetamide, N-methyl-2-pyrrolidone, N-methylpyrrolidinone and hexamethylphosphoric triamide; ethers such as diethyl ether, diisopropyl ether, tetrahydrofuran, dioxane, dimethoxyethane and diethylene glycol dimethyl ether; sulfoxides such as dimethyl sulfoxide and sulfolane; nitriles such as acetonitrile and isobutyronitrile; esters such as ethyl formate, ethyl acetate, propyl acetate, butyl acetate and diethyl carbonate; ketones such as acetone, methyl ethyl ketone, 4-methyl-2-pentanone, methyl isobutyl ketone, isophorone and cyclohexanone; nitro compounds such as nitroethane and nitrobenzene; halogenated hydrocarbons such as dichloromethane, 1,2-dichloroethane, dichlorobenzene, chloroform and carbon tetrachloride; aromatic hydrocarbons such as benzene, toluene and xylene; or a mixed solvent thereof, preferably halogenated hydrocarbons, more preferably dichloromethane.

Examples of bases used in this step include alkali metal carbonates such as sodium carbonate, potassium carbonate and lithium carbonate; alkali metal hydrogen carbonates such as sodium hydrogen carbonate, potassium hydrogen carbonate and lithium hydrogen carbonate; or organic bases such as N-methylmorpholine, triethylamine, tripropylamine, tributylamine, diisopropylethylamine, dicyclohexylamine, N-methylpiperidine, pyridine, 4-pyrrolidinopyridine, picoline, 4-(N,N-dimethylamino)pyridine, 2,6-di(t-butyl)-4-methylpyridine, quinoline, N,N-dimethylaniline, N,N-diethylaniline, 1,5-diazabicyclo[4.3.0]non-S-ene (DBN), 1,4-diazabicyclo[2.2.2]octane (DABCO) and 1,8-diazabicyclo [5.4.0]undec-7-ene (DBU), preferably organic bases, more preferably diisopropylethylamine.

The reaction temperature in this step varies depending on the raw material compounds and the inert solvent to be used, and is generally -20˚C to 60˚C, preferably 20˚C to 30˚C.

The reaction time in this step varies depending on the raw material compounds, the inert solvent to be used and the reaction temperature, and is generally 0.5 hour to 24 hours, preferably 2 hours to 4 hours.

Process ACc is a process for producing a compound having the general formula (IAC).

Process ACc

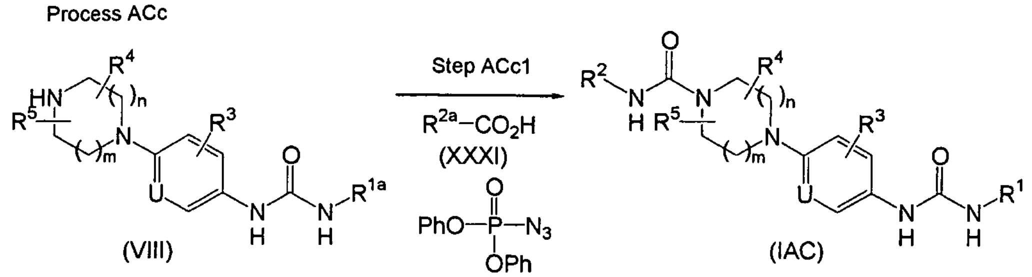


In the present invention, $R^1$, $R^2$, $R^3$, $R^4$, $R^5$, $R^{1a}$, $R^{2a}$, U, m and n are the same as described above.

Step ACc1

This is a step of producing a compound having the general formula (IAC).

The step is performed by allowing a compound having the general formula (VIII) to react with a compound having the general formula (XXXI) publicly known or easily obtained using a publicly known compound as a starting material as in a known method and diphenylphosphoric acid azide (DPPA) in an inert solvent in the presence of a base, and then removing the protecting group of the amino group, the hydroxyl group and/or the carboxyl group in $R^{1a}$ and $R^{2a}$ as desired.

The inert solvent used in this step is not particularly limited as long as the solvent dissolves a certain amount of starting materials without inhibiting the reaction. Examples thereof include hydrocarbons such as petroleum ether; amides such as formamide, N,N-dimethylformamide, N,N-dimethylacetamide, N-methyl-2-pyrrolidone, N-methylpyrrolidinone and hexamethylphosphoric triamide; ethers such as diethyl ether, diisopropyl ether, tetrahydrofuran, dioxane, dimethoxyethane and diethylene glycol dimethyl ether; sulfoxides such as dimethyl sulfoxide and sulfolane; nitriles such as acetonitrile and isobutyronitrile; esters such as ethyl formate, ethyl acetate, propyl acetate, butyl acetate and diethyl carbonate; ketones such as acetone, methyl ethyl ketone, 4-methyl-2-pentanone, methyl isobutyl ketone, isophorone and cyclohexanone; nitro compounds such as nitroethane and nitrobenzene; halogenated hydrocarbons such as dichloromethane, 1,2-dichloroethane, dichlorobenzene, chloroform and carbon tetrachloride; aromatic hydrocarbons such as benzene, toluene and xylene; or a mixed solvent thereof, preferably ethers, more preferably tetrahydrofuran.

Examples of bases used in this step include inorganic bases like alkali metal carbonates such as sodium carbonate, potassium carbonate and lithium carbonate; alkali metal hydrogen carbonates such as sodium hydrogen carbonate, potassium hydrogen carbonate and lithium hydrogen carbonate; alkali metal hydroxides such as sodium hydroxide, potassium hydroxide, barium hydroxide and lithium hydroxide; alkali metal fluorides such as sodium fluoride and potassium fluoride; alkali metal trialkyl siloxides such as sodium trimethylsiloxide, potassium trimethylsiloxide and lithium trimethylsiloxide; or organic bases such as N-methylmorpholine, triethylamine, tripropylamine, tributylamine, diisopropylethylamine, dicyclohexylamine, N-methylpiperidine, pyridine, 4-pyrrolidinopyridine, picoline, 4-(N,N-dimethylamino) pyridine, 2,6-di(t-butyl)-4-methylpyridine, quinoline, N,N-dimethylaniline, N,N-diethylaniline, 1,5-diazabicyclo [4.3.0]non-5-ene (DBN), 1,4-diazabicyclo[2.2.2]octane (DABCO), 1,8-diazabicyclo[5.4.0]undec-7-ene (DBU), preferably organic bases, more preferably triethylamine.

The reaction temperature in this step varies depending on the raw material compounds and the inert solvent to be used, and is generally 20°C to 100°C, preferably 60°C to 80°C.

The reaction time in this step varies depending on the raw material compounds, the inert solvent to be used and the reaction temperature, and is generally 2 hours to 24 hours, preferably 4 hours to 6 hours.

Process ACd is a process for producing a compound having the general formula (IAC).

Process ACd

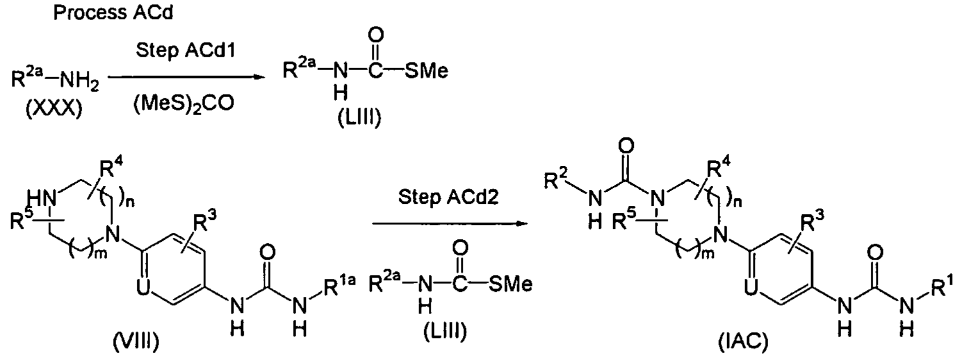


In the present invention, $R^1$, $R^2$, $R^3$, $R^4$, $R^5$, $R^{1a}$, $R^{2a}$, U, m and n are the same as described above.

Step ACd1

This is a step of producing a compound having the general formula (LIII).

The step is performed in accordance with the method described in J. Org. Chem. 61, 4175 (1996) or J. Org. Chem. 69, 1866 (2004), comprising allowing a compound having the general formula (XXX) publicly known or easily obtained using a publicly known compound as a starting material as in a known method to react with S,S-dimethyl dithiocarbonate (DMDTC) in an inert solvent in the presence of a base.

The inert solvent used in this step is not particularly limited as long as the solvent dissolves a certain amount of starting materials without inhibiting the reaction. Examples thereof include hydrocarbons such as pentane, hexane, octane, petroleum ether and ligroin; amides such as formamide, N,N-dimethylformamide, N,N-dimethylacetamide, N-methyl-2-pyrrolidone, N-methylpyrrolidinone and hexamethylphosphoric triamide; ethers such as diethyl ether, diisopropyl ether, tetrahydrofuran, dioxane, dimethoxyethane and diethylene glycol dimethyl ether; sulfoxides such as dimethyl sulfoxide and sulfolane; nitriles such as acetonitrile and isobutyronitrile; nitro compounds such as nitroethane and nitrobenzene; halogenated hydrocarbons such as dichloromethane, 1,2-dichloroethane, dichlorobenzene, chloroform and carbon tetrachloride; aromatic hydrocarbons such as benzene, toluene and xylene; or a mixed solvent thereof, preferably ethers, more preferably tetrahydrofuran.

Examples of bases used in this step include alkali metal hydrides such as lithium hydride, sodium hydride and potassium hydride; alkali metal alkoxides such as sodium methoxide, sodium ethoxide, sodium t-butoxide, potassium methoxide, potassium ethoxide, potassium t-butoxide and lithium methoxide; alkali metal trialkyl siloxides such as sodium trimethylsiloxide, potassium trimethylsiloxide and lithium trimethylsiloxide; alkali metal mercaptans such as sodium methylmercaptan and sodium ethylmercaptan; or organometallic bases such as butyl lithium, lithium diisopropylamide, lithium bis(trimethylsilyl)amide and sodium bis(trimethylsilyl)amide, preferably organometallic bases, more preferably lithium diisopropylamide or lithium bis(trimethylsilyl)amide.

The reaction temperature in this step varies depending on the raw material compounds and the inert solvent to be used, and is generally -100˚C to 40˚C, preferably -78˚C to 25˚C.

The reaction time in this step varies depending on the raw material compounds, the inert solvent to be used and the reaction temperature, and is generally 1 hour to 96 hours, preferably 6 hours to 24 hours.

Step ACd2

This is a step of producing a compound having the general formula (IAC).

The step is performed in accordance with the method described in J. Org. Chem. 69, 1866 (2004), comprising allowing a compound having the general formula (VIII) to react with a compound having the general formula (LIII) in an inert solvent, and then by removing the protecting group of the amino group, the hydroxyl group and/or the carboxyl group in $R^{1a}$ and $R^{2a}$ as desired.

The inert solvent used in this step is not particularly limited as long as the solvent dissolves a certain amount of starting materials without inhibiting the reaction. Examples thereof include hydrocarbons such as pentane, hexane, octane, petroleum ether and ligroin; amides such as formamide, N,N-dimethylformamide, N,N-dimethylacetamide, N-methyl-2-pyrrolidone, N-methylpyrrolidinone and hexamethylphosphoric triamide; ethers such as diethyl ether, diisopropyl ether, tetrahydrofuran, dioxane, dimethoxyethane and diethylene glycol dimethyl ether; sulfoxides such as dimethyl sulfoxide and sulfolane; nitriles such as acetonitrile and isobutyronitrile; esters such as ethyl formate, ethyl acetate, propyl acetate, butyl acetate and diethyl carbonate; ketones such as acetone, methyl ethyl ketone, 4-methyl-2-pentanone, methyl isobutyl ketone, isophorone and cyclohexanone; nitro compounds such as nitroethane and nitrobenzene; halogenated hydrocarbons such as dichloromethane, 1,2-dichloroethane, dichlorobenzene, chloroform and carbon tetrachloride; aromatic hydrocarbons such as benzene, toluene and xylene; or a mixed solvent thereof, preferably nitriles or ethers, more preferably acetonitrile or tetrahydrofuran.

The reaction temperature in this step varies depending on the raw material compounds and the inert solvent to be used, and is generally 0˚C to 150˚C, preferably 25˚C to 110˚C.

The reaction time in this step varies depending on the raw material compounds, the inert solvent to be used and the reaction temperature, and is generally 0.5 hour to 96 hours, preferably 1 hour to 24 hours.

Process ACe is a process for producing a compound having the general formula (IAC).

Process ACe

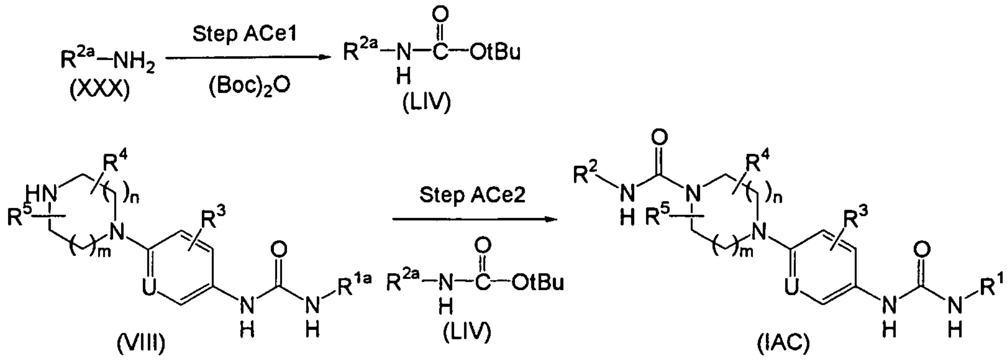

(XXX) (LIV) (VIII) (LIV) (IAC)

In the present invention, $R^1$, $R^2$, $R^3$, $R^4$, $R^5$, $R^{1a}$, $R^{2a}$, U, m and n are the same as described above.

Step ACe1

This is a step of producing a compound having the general formula (LIV).

The step is performed in accordance with the method described in SYNTHESIS 877(1996) or Tetrahedron Lett. 35, 9003 (1994), comprising allowing a compound having the general formula (XXX) publicly known or easily obtained using a publicly known compound as a starting material as in a known method to react with di-tert-butyl dicarbonate in an inert solvent in the presence or absence of a base.

The inert solvent used in this step is not particularly limited as long as the solvent dissolves a certain amount of starting materials without inhibiting the reaction. Examples thereof include hydrocarbons such as pentane, hexane, octane, petroleum ether and ligroin; amides such as formamide, N,N-dimethylformamide, N,N-dimethylacetamide, N-methyl-2-pyrrolidone, N-methylpyrrolidinone and hexamethylphosphoric triamide; ethers such as diethyl ether, diisopropyl ether, tetrahydrofuran, dioxane, dimethoxyethane and diethylene glycol dimethyl ether; sulfoxides such as dimethyl sulfoxide and sulfolane; nitriles such as acetonitrile and isobutyronitrile; esters such as ethyl formate, ethyl acetate, propyl acetate, butyl acetate and diethyl carbonate; ketones such as acetone, methyl ethyl ketone, 4-methyl-2-pentanone, methyl isobutyl ketone, isophorone and cyclohexanone; nitro compounds such as nitroethane and nitrobenzene; halogenated hydrocarbons such as dichloromethane, 1,2-dichloroethane, dichlorobenzene, chloroform and carbon tetrachloride; aromatic hydrocarbons such as benzene, toluene and xylene; or a mixed solvent thereof. In the absence of a base, the solvent is preferably hydrocarbons, esters or a mixed solvent thereof, more preferably hexane, ethyl acetate or a mixed solvent thereof, further more preferably a mixed solvent of hexane and ethyl acetate. In the presence of a base, the solvent is preferably ethers, more preferably tetrahydrofuran.

Examples of bases used in this step include alkali metal hydrides such as lithium hydride, sodium hydride and potassium hydride; alkali metal alkoxides such as sodium methoxide, sodium ethoxide, sodium t-butoxide, potassium methoxide, potassium ethoxide, potassium t-butoxide and lithium methoxide; alkali metal trialkyl siloxides such as sodium trimethylsiloxide, potassium trimethylsiloxide and lithium trimethylsiloxide; alkali metal mercaptans such as sodium methylmercaptan and sodium ethylmercaptan; or organometallic bases such as butyl lithium, lithium diisopropylamide, lithium bis(trimethylsilyl)amide and sodium bis(trimethylsilyl)amide, preferably organometallic bases, more preferably sodium bis(trimethylsilyl)amide.

The reaction temperature in this step varies depending on the raw material compounds and the inert solvent to be used, and is generally 0°C to 100°C, preferably 20°C to 80°C.

The reaction time in this step varies depending on the raw material compounds, the inert solvent to be used and the reaction temperature, and is generally 0.5 hour to 96 hours, preferably 1 hour to 24 hours.

Step ACe2

This is a step of producing a compound having the general formula (IAC).

The step is performed by allowing a compound having the general formula (VIII) to react with a compound having the general formula (LIV) in an inert solvent and then removing the protecting group of the amino group, the hydroxyl group and/or the carboxyl group in $R^{1a}$ and $R^{2a}$ as desired.

The inert solvent used in this step is not particularly limited as long as the solvent dissolves a certain amount of starting materials without inhibiting the reaction. Examples thereof include hydrocarbons such as pentane, hexane,

octane, petroleum ether and ligroin; amides such as formamide, N,N-dimethylformamide, N,N-dimethylacetamide, N-methyl-2-pyrrolidone, N-methylpyrrolidinone and hexamethylphosphoric triamide; ethers such as diethyl ether, diisopropyl ether, tetrahydrofuran, dioxane, dimethoxyethane and diethylene glycol dimethyl ether; sulfoxides such as dimethyl sulfoxide and sulfolane; nitriles such as acetonitrile and isobutyronitrile; esters such as ethyl formate, ethyl acetate, propyl acetate, butyl acetate and diethyl carbonate; ketones such as acetone, methyl ethyl ketone, 4-methyl-2-pentanone, methyl isobutyl ketone, isophorone and cyclohexanone; nitro compounds such as nitromethane, nitroethane and nitrobenzene; halogenated hydrocarbons such as dichloromethane, 1,2-dichloroethane, dichlorobenzene, chloroform and carbon tetrachloride; aromatic hydrocarbons such as benzene, toluene and xylene; or a mixed solvent thereof, preferably ethers, nitriles or nitro compounds, more preferably tetrahydrofuran, acetonitrile or nitromethane.

The reaction temperature in this step varies depending on the raw material compounds and the inert solvent to be used, and is generally 0°C to 150°C, preferably 60°C to 110°C.

The reaction time in this step varies depending on the raw material compounds, the inert solvent to be used and the reaction temperature, and is generally 0.5 hour to 96 hours, preferably 1 hour to 24 hours.

Process AD is a process for producing a compound having the general formula (IAD).

Process AD

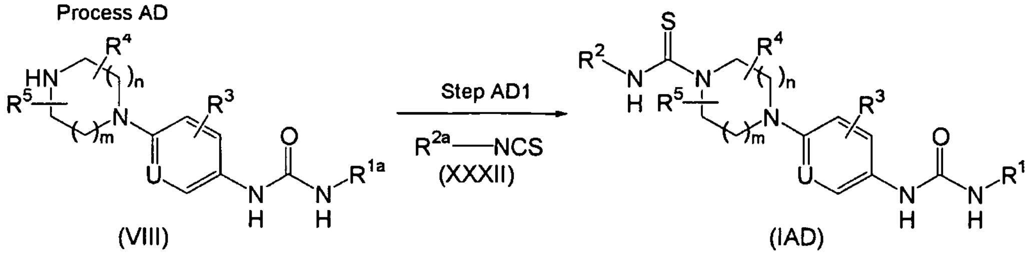


In the present invention, $R^1$, $R^2$, $R^3$, $R^4$, $R^5$, $R^{1a}$, $R^{2a}$, U, m and n are the same as described above.

Step AD1

This is a step of producing a compound having the general formula (IAD).

The step is performed by allowing a compound having the general formula (VIII) to react with a compound having the general formula (XXXII) publicly known or easily obtained using a publicly known compound as a starting material as in a known method in an inert solvent, and then removing the protecting group of the amino group, the hydroxyl group and/or the carboxyl group in $R^{1a}$ and $R^{2a}$ as desired.

The inert solvent used in this step is not particularly limited as long as the solvent dissolves a certain amount of starting materials without inhibiting the reaction. Examples thereof include hydrocarbons such as petroleum ether; amides such as formamide, N,N-dimethylformamide, N,N-dimethylacetamide, N-methyl-2-pyrrolidone, N-methylpyrrolidinone and hexamethylphosphoric triamide; ethers such as diethyl ether, diisopropyl ether, tetrahydrofuran, dioxane, dimethoxyethane and diethylene glycol dimethyl ether; sulfoxides such as dimethyl sulfoxide and sulfolane; nitriles such as acetonitrile and isobutyronitrile; esters such as ethyl formate, ethyl acetate, propyl acetate, butyl acetate and diethyl carbonate; ketones such as acetone, methyl ethyl ketone, 4-methyl-2-pentanone, methyl isobutyl ketone, isophorone and cyclohexanone; nitro compounds such as nitroethane and nitrobenzene; halogenated hydrocarbons such as dichloromethane, 1,2-dichloroethane, dichlorobenzene, chloroform and carbon tetrachloride; aromatic hydrocarbons such as benzene, toluene and xylene; or a mixed solvent thereof, preferably ethers, more preferably tetrahydrofuran.

The reaction temperature in this step varies depending on the raw material compounds and the inert solvent to be used, and is generally -20°C to 80°C, preferably 20°C to 30°C.

The reaction time in this step varies depending on the raw material compounds, the inert solvent to be used and the reaction temperature, and is generally 0.5 hour to 24 hours, preferably 2 hours to 4 hours.

Process AE is a process for producing a compound having the general formula (IAE).

Process AE

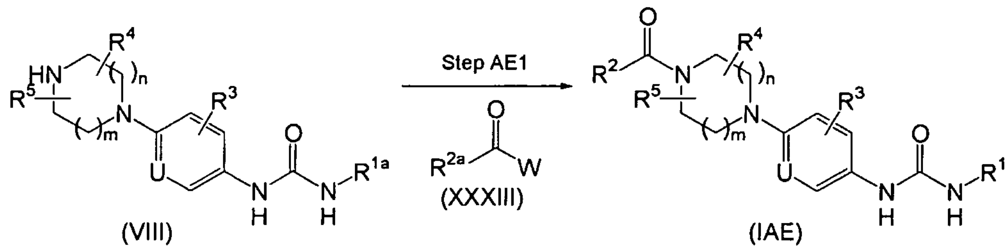


(VIII) (XXXIII) (IAE)

In the present invention, $R^1$, $R^2$, $R^3$, $R^4$, $R^5$, $R^{1a}$, $R^{2a}$, U, m and n are the same as described above, and W represents a halogen atom (e.g., a fluorine atom, a chlorine atom, a bromine atom or an iodine atom, preferably a chlorine atom) or a hydroxyl group.

Step AE1

This is a step of producing a compound having the general formula (IAE).

(i) When W represents a halogen atom

The step is performed by allowing a compound having the general formula (VIII) to react with a compound having the general formula (XXXIII) publicly known or easily obtained using a publicly known compound as a starting material as in a known method in an inert solvent in the presence or absence (preferably presence) of a base, and then removing the protecting group of the amino group, the hydroxyl group and/or the carboxyl group in $R^{1a}$ and $R^{2a}$ as desired.
The inert solvent used in this step is not particularly limited as long as the solvent dissolves a certain amount of starting materials without inhibiting the reaction. Examples thereof include hydrocarbons such as petroleum ether; amides such as formamide, N,N-dimethylformamide, N,N-dimethylacetamide, N-methyl-2-pyrrolidone, N-methylpyrrolidinone and hexamethylphosphoric triamide; ethers such as diethyl ether, diisopropyl ether, tetrahydrofuran, dioxane, dimethoxyethane and diethylene glycol dimethyl ether; sulfoxides such as dimethyl sulfoxide and sulfolane; nitriles such as acetonitrile and isobutyronitrile; esters such as ethyl formate, ethyl acetate, propyl acetate, butyl acetate and diethyl carbonate; ketones such as acetone, methyl ethyl ketone, 4-methyl-2-pentanone, methyl isobutyl ketone, isophorone and cyclohexanone; nitro compounds such as nitroethane and nitrobenzene; halogenated hydrocarbons such as dichloromethane, 1,2-dichloroethane, dichlorobenzene, chloroform and carbon tetrachloride; aromatic hydrocarbons such as benzene, toluene and xylene; or a mixed solvent thereof, preferably amides, more preferably N,N-dimethylacetamide.
Examples of bases used in this step include inorganic bases like alkali metal carbonates such as sodium carbonate, potassium carbonate and lithium carbonate; alkali metal hydrogen carbonates such as sodium hydrogen carbonate, potassium hydrogen carbonate and lithium hydrogen carbonate; alkali metal hydroxides such as sodium hydroxide, potassium hydroxide, barium hydroxide and lithium hydroxide; alkali metal fluorides such as sodium fluoride and potassium fluoride; alkali metal trialkyl siloxides such as sodium trimethylsiloxide, potassium trimethylsiloxide and lithium trimethylsiloxide; or organic bases such as N-methylmorpholine, triethylamine, tripropylamine, tributylamine, diisopropylethylamine, dicyclohexylamine, N-methylpiperidine, pyridine, 4-pyrrolidinopyridine, picoline, 4-(N,N-dimethylamino)pyridine, 2,6-di(t-butyl)-4-methylpyridine, quinoline, N,N-dimethylaniline, N,N-diethylaniline, 1,5-diazabicyclo[4.3.0]non-5-ene (DBN), 1,4-diazabicyclo[2.2.2]octane (DABCO) and 1,8-diazabicyclo[5.4.0]undec-7-ene (DBU), preferably organic bases, more preferably triethylamine.
The reaction temperature in this step varies depending on the raw material compounds and the inert solvent to be used, and is generally -20˚C to 80˚C, preferably 20˚C to 30˚C.
The reaction time in this step varies depending on the raw material compounds, the inert solvent to be used and the reaction temperature, and is generally 0.5 hour to 24 hours, preferably 2 hours to 4 hours.

(ii) When W represents a hydroxyl group

The step is performed by allowing a compound having the general formula (VIII) to react with a compound having the general formula (XXXIII) publicly known or easily obtained using a publicly known compound as a starting material as in a known method and a condensing agent in an inert solvent in the presence of a base, and then removing the protecting group of the amino group, the hydroxyl group and/or the carboxyl group in $R^{1a}$ and $R^{2a}$ as desired.
The inert solvent used in this step is not particularly limited as long as the solvent dissolves a certain amount

of starting materials without inhibiting the reaction. Examples thereof include hydrocarbons such as petroleum ether; amides such as formamide, N,N-dimethylformamide, N,N-dimethylacetamide, N-methyl-2-pyrrolidone, N-methylpyrrolidinone and hexamethylphosphoric triamide; ethers such as diethyl ether, diisopropyl ether, tetrahydrofuran, dioxane, dimethoxyethane and diethylene glycol dimethyl ether; alcohols such as methanol, ethanol, n-propanol, i-propanol, n-butanol, t-butanol, isoamyl alcohol, diethylene glycol, glycerol, octanol, cyclohexanol and methyl cellosolve; sulfoxides such as dimethyl sulfoxide and sulfolane; nitriles such as acetonitrile and isobutyronitrile; esters such as ethyl formate, ethyl acetate, propyl acetate, butyl acetate and diethyl carbonate; ketones such as acetone, methyl ethyl ketone, 4-methyl-2-pentanone, methyl isobutyl ketone, isophorone and cyclohexanone; nitro compounds such as nitroethane and nitrobenzene; halogenated hydrocarbons such as dichloromethane, 1,2-dichloroethane, dichlorobenzene, chloroform and carbon tetrachloride; aromatic hydrocarbons such as benzene, toluene and xylene; or a mixed solvent thereof, preferably amides, more preferably N,N-dimethylacetamide.

Examples of bases used in this step include inorganic bases like alkali metal carbonates such as sodium carbonate, potassium carbonate and lithium carbonate; alkali metal hydrogen carbonates such as sodium hydrogen carbonate, potassium hydrogen carbonate and lithium hydrogen carbonate; alkali metal hydroxides such as sodium hydroxide, potassium hydroxide, barium hydroxide and lithium hydroxide; alkali metal fluorides such as sodium fluoride and potassium fluoride; alkali metal trialkyl siloxides such as sodium trimethylsiloxide, potassium trimethylsiloxide and lithium trimethylsiloxide; or organic bases such as N-methylmorpholine, triethylamine, tripropylamine, tributylamine, diisopropylethylamine, dicyclohexylamine, N-methylpiperidine, pyridine, 4-pyrrolidinopyridine, picoline, 4-(N,N-dimethylamino)pyridine, 2,6-di(t-butyl)-4-methylpyridine, quinoline, N,N-dimethylaniline, N,N-diethylaniline, 1,5-diazabicyclo [4.3.0]non-5-ene (DBN), 1,4-diazabicyclo[2.2.2]octane (DABCO) and 1,8-diazabicyclo[5.4.0]undec-7-ene (DBU), preferably organic bases, more preferably triethylamine.

Examples of condensing agents used in this step include 1-propanephosphonic acid cyclic anhydride (T3P), 1-(3-dimethylaminopropyl)-3-ethylcarbodiimide hydrochloride (EDCI), isobutyl chloroformate (IBCF), 1,1'-carbonylbis-1H-imidazol (CDI), diethyl cyanophosphonate (DEPC), diphenylphosphoric acid azide (DPPA) and benzotriazol-1-yloxytris(dimethylamino)phosphonium hexafluorophosphate (BOP reagent), preferably T3P or BOP reagent.

The reaction temperature in this step varies depending on the raw material compounds and the inert solvent to be used, and is generally -20˚C to 100˚C, preferably 20˚C to 80˚C.

The reaction time in this step varies depending on the raw material compounds, the inert solvent to be used and the reaction temperature, and is generally 2 hours to 72 hours, preferably 6 hours to 24 hours.

After completion of the reaction in each step of the above processes AA, AB, ACa, ACb, ACc, ACd, ACe, AD and AE, the respective target compounds are obtained from the reaction mixture in accordance with a conventional method. For example, the target compound can be obtained by neutralizing the reaction mixture suitably, removing insoluble matter if any by filtration, adding thereto water and an immiscible organic solvent such as ethyl acetate, separating the organic layer containing the target compound, and after washing with water or other solutions, drying over anhydrous magnesium sulfate, anhydrous sodium sulfate or anhydrous sodium hydrogen carbonate, filtering and distilling off the solvent. The resulting target compound can be separated and purified by using methods conventionally used for separation and purification of organic compounds, such as recrystallization and reprecipitation in combination, and eluting with an appropriate eluent using chromatography, if necessary. Target compounds insoluble in the solvent can be purified by washing the resulting solid crude product with the solvent.

Process B is a process for producing a compound having the general formula (IAA).

Process B

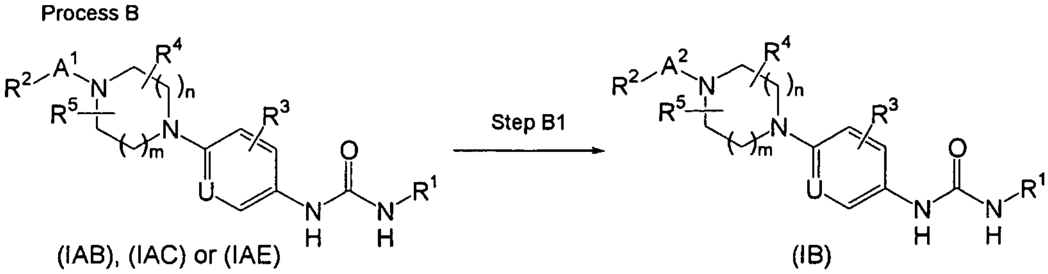


(IAB), (IAC) or (IAE)

(IB)

In the present invention, $R^1$, $R^2$, $R^3$, $R^4$, $R^5$, U, m and n are the same as described above, $A^1$ represents a group represented by the formula -O-C(=O)-, a group represented by the formula -NH-C(=O)- or a carbonyl group, and $A^2$ represents a group represented by the formula -O-C(=S)-, a group represented by the formula -NH-C(=S)- or a thiocarbonyl group.

Step B1

This is a step of producing a compound having the general formula (IB).

The step is performed by allowing a compound having the general formula (IAB), (IAC) or (IAE) to react with a sulfurizing agent in an inert solvent.

The inert solvent used in this step is not particularly limited as long as the solvent dissolves a certain amount of starting materials without inhibiting the reaction. Examples thereof include hydrocarbons such as petroleum ether; amides such as hexamethylphosphoric triamide; ethers such as diethyl ether, diisopropyl ether, tetrahydrofuran, dioxane, dimethoxyethane and diethylene glycol dimethyl ether; sulfoxides such as dimethyl sulfoxide and sulfolane; nitriles such as acetonitrile and isobutyronitrile; nitro compounds such as nitroethane and nitrobenzene; halogenated hydrocarbons such as dichloromethane, 1,2-dichloroethane, dichlorobenzene, chloroform and carbon tetrachloride; aromatic hydrocarbons such as benzene, toluene and xylene; or a mixed solvent thereof, preferably ethers, more preferably dimethoxyethane.

Examples of sulfurizing agents used in this step include Lawesson's reagent and phosphorus pentasulfide, preferably Lawesson's reagent.

The reaction temperature in this step varies depending on the raw material compounds and the inert solvent to be used, and is generally 0˚C to 80˚C, preferably 20˚C to 30˚C.

The reaction time in this step varies depending on the raw material compounds, the inert solvent to be used and the reaction temperature, and is generally 0.5 hour to 24 hours, preferably 2 hours to 6 hours.

After completion of the reaction in the step of the above process B, the target compound is obtained from the reaction mixture in accordance with a conventional method. For example, the target compound can be obtained by neutralizing the reaction mixture suitably, removing insoluble matter if any by filtration, adding thereto water and an immiscible organic solvent such as ethyl acetate, separating the organic layer containing the target compound, and after washing with water or other solutions, drying over anhydrous magnesium sulfate, anhydrous sodium sulfate or anhydrous sodium hydrogen carbonate, filtering and distilling off the solvent. The resulting target compound can be separated and purified by using methods conventionally used for separation and purification of organic compounds, such as recrystallization and reprecipitation in combination, and eluting with an appropriate eluent using chromatography, if necessary. Target compounds insoluble in the solvent can be purified by washing the resulting solid crude product with the solvent.

Process C is a process for producing a compound having the general formula (VIII), which is a raw material compound in the above processes AA, AB, ACa, ACb, ACc, the step ACd2 in the above process ACd, the step ACe2 in the above process ACe, and the above processes AD and AE.

Process C

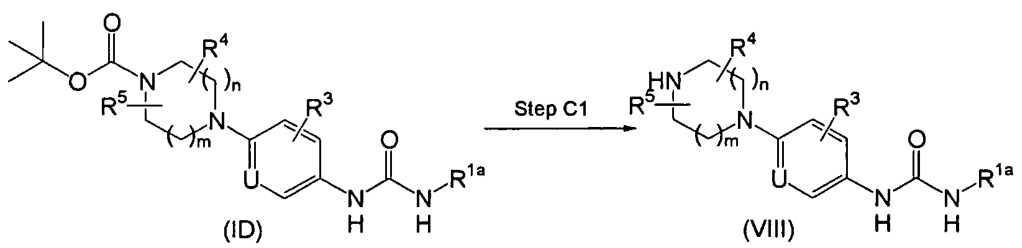


In the present invention, $R^3$, $R^4$, $R^5$, $R^{1a}$, U, m and n are the same as described above.

Step C1

This is a step of producing a compound having the general formula (VIII). The step is performed by allowing a compound having the general formula (ID) to react with acid in an inert solvent in the presence or absence of anisole.

The inert solvent used in this step is not particularly limited as long as the solvent dissolves a certain amount of starting materials without inhibiting the reaction. Examples thereof include hydrocarbons such as pentane, hexane, octane, petroleum ether and ligroin; amides such as formamide, N,N-dimethylformamide, N,N-dimethylacetamide, N-methyl-2-pyrrolidone, N-methylpyrrolidinone and hexamethylphosphoric triamide; ethers such as diethyl ether, diisopropyl ether, tetrahydrofuran, dioxane, dimethoxyethane and diethylene glycol dimethyl ether; alcohols such as methanol, ethanol, n-propanol, i-propanol, n-butanol, t-butanol, isoamyl alcohol, diethylene glycol, glycerol, octanol, cyclohexanol and methyl cellosolve; sulfoxides such as dimethyl sulfoxide and sulfolane; nitriles such as acetonitrile and isobutyronitrile;

esters such as ethyl formate, ethyl acetate, propyl acetate, butyl acetate and diethyl carbonate; ketones such as acetone, methyl ethyl ketone, 4-methyl-2-pentanone, methyl isobutyl ketone, isophorone and cyclohexanone; nitro compounds such as nitroethane and nitrobenzene; halogenated hydrocarbons such as dichloromethane, 1,2-dichloroethane, dichlorobenzene, chloroform and carbon tetrachloride; aromatic hydrocarbons such as benzene, toluene and xylene; or a mixed solvent thereof, preferably halogenated hydrocarbons, more preferably dichloromethane.

Examples of acid used in this step include hydrogen halides such as hydrogen chloride gas or hydrogen bromide gas; mineral acids such as sulfuric acid, hydrobromic acid or hydrochloric acid; organic sulfonic acids such as methanesulfonic acid, p-toluenesulfonic acid, camphorsulfonic acid or trifluoromethanesulfonic acid; carboxylic acids such as acetic acid, formic acid or trifluoroacetic acid; Lewis acids such as zinc chloride, tin tetrachloride, boron trifluoride or boron tribromide; or acidic ion exchange resin, preferably carboxylic acids, more preferably trifluoroacetic acid.

The reaction temperature in this step varies depending on the raw material compounds and the inert solvent to be used, and is generally 0˚C to 100˚C, preferably 20˚C to 30˚C.

The reaction time in this step varies depending on the raw material compounds, the inert solvent to be used and the reaction temperature, and is generally 0.5 hour to 24 hours, preferably 2 hours to 4 hours.

After completion of the reaction in the step of the above process C, the target compound is obtained from the reaction mixture in accordance with a conventional method. For example, the target compound can be obtained by neutralizing the reaction mixture suitably, removing insoluble matter if any by filtration, adding thereto water and an immiscible organic solvent such as ethyl acetate, separating the organic layer containing the target compound, and after washing with water or other solutions, drying over anhydrous magnesium sulfate, anhydrous sodium sulfate or anhydrous sodium hydrogen carbonate, filtering and distilling off the solvent. The resulting target compound can be separated and purified by using methods conventionally used for separation and purification of organic compounds, such as recrystallization and reprecipitation in combination, and eluting with an appropriate eluent using chromatography, if necessary. Target compounds insoluble in the solvent can be purified by washing the resulting solid crude product with the solvent.

Process DA is a process for producing a compound having the general formula (ID), which is a raw material compound in the above above process C.

Process DA

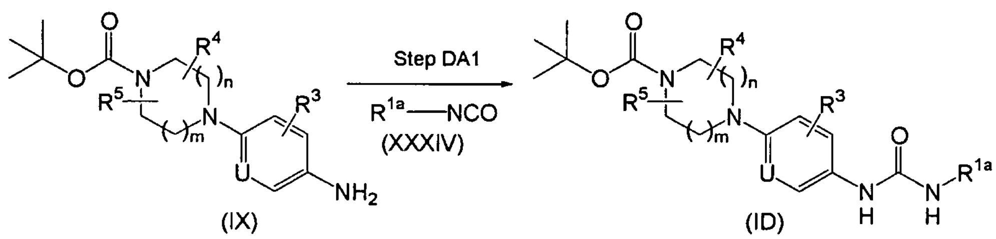


(IX) (ID)

In the present invention, $R^3$, $R^4$, $R^5$, $R^{1a}$, U, m and n are the same as described above.

Step DA1

This is a step of producing a compound having the general formula (ID).

The step is performed by allowing a compound having the general formula (IX) to react with a compound having the general formula (XXXIV) publicly known or easily obtained using a publicly known compound as a starting material as in a known method in an inert solvent in the same manner as in the step ACal in the above process ACa.

Process DB is a process for producing a compound having the general formula (ID), which is a raw material compound in the above process C.

Process DB

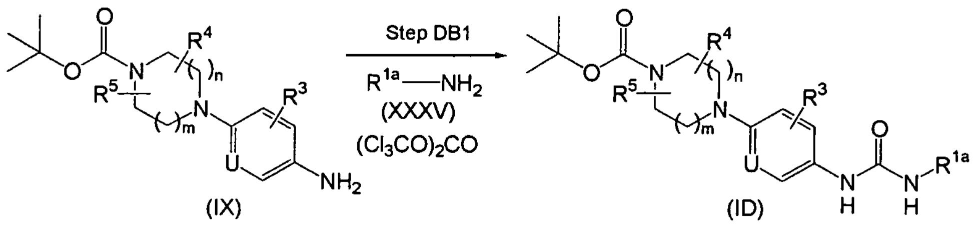


In the present invention, $R^3$, $R^4$, $R^5$, $R^{1a}$, U, m and n are the same as described above.

Step DB 1

This is a step of producing a compound having the general formula (ID).

The step is performed by allowing a compound having the general formula (IX) to react with a compound having the general formula (XXXV) publicly known or easily obtained using a publicly known compound as a starting material as in a known method in an inert solvent in the same manner as in the step ACb1 in the above process ACb.

Process DC is a process for producing a compound having the general formula (ID), which is a raw material compound in the above process C.

Process DC

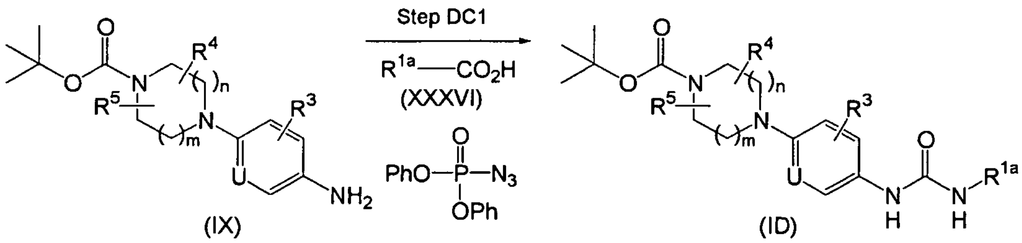


In the present invention, $R^3$, $R^4$, $R^5$, $R^{1a}$, U, m and n are the same as described above.

Step DC 1

This is a step of producing a compound having the general formula (ID). The step is performed by allowing a compound having the general formula (IX) to react with a compound having the general formula (XXXVI) publicly known or easily obtained using a publicly known compound as a starting material as in a known method in an inert solvent in the same manner as in the step ACc1 in the above process ACc.

Process DD is a process for producing a compound having the general formula (ID), which is a raw material compound in the above process C.

Process DD

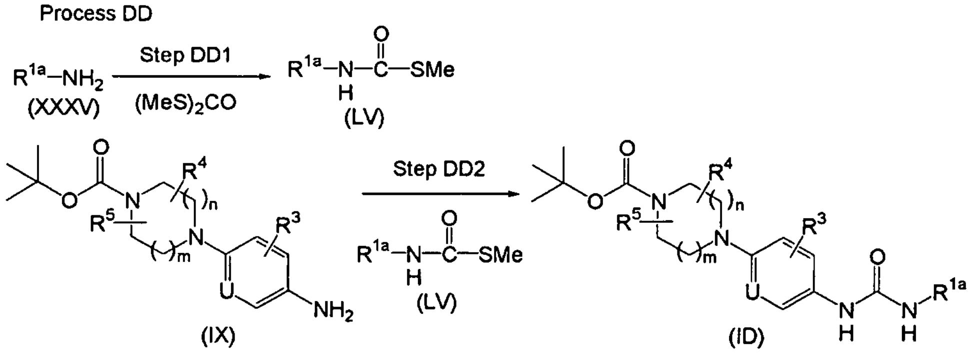


In the present invention, $R^3$, $R^4$, $R^5$, $R^{1a}$, U, m and n are the same as described above.

Step DD1

This is a step of producing a compound having the general formula (LV).

The step is performed by allowing a compound having the general formula (XXXV) publicly known or easily obtained using a publicly known compound as a starting material as in a known method to react with S,S-dimethyl dithiocarbonate (DMDTC) in an inert solvent in the presence of a base in the same manner as in the step ACd1 in the above process ACd.

Step DD2

This is a step of producing a compound having the general formula (ID).

The step is performed by allowing a compound having the general formula (IX) to react with a compound having the general formula (LV) in an inert solvent in the same manner as in the step ACd2 in the above process ACd.

Process DE is a process for producing a compound having the general formula (ID), which is a raw material compound in the above process C.

Process DE

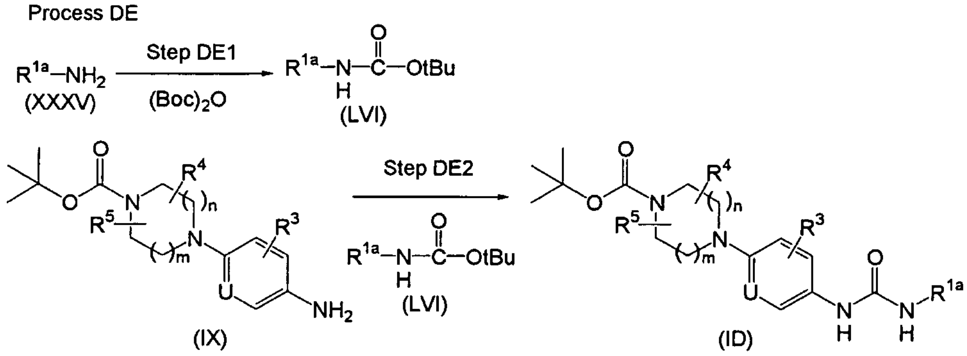


In the present invention, $R^3$, $R^4$, $R^5$, $R^{1a}$, U, m and n are the same as described above.

Step DE1

This is a step of producing a compound having the general formula (LVI).

The step is performed by allowing a compound having the general formula (XXXV) publicly known or easily obtained using a publicly known compound as a starting material as in a known method to react with di-tert-butyl dicarbonate in an inert solvent in the same manner as in the step ACe1 in the above process ACe.

Step DE2

This is a step of producing a compound having the general formula (ID).

The step is performed by allowing a compound having the general formula (IX) to react with a compound having the general formula (LVI) in an inert solvent in the same manner as in the step ACe2 in the above process ACe.

Process DF is a process for producing a compound having the general formula (ID), which is a raw material compound in the above process C.

Process DF

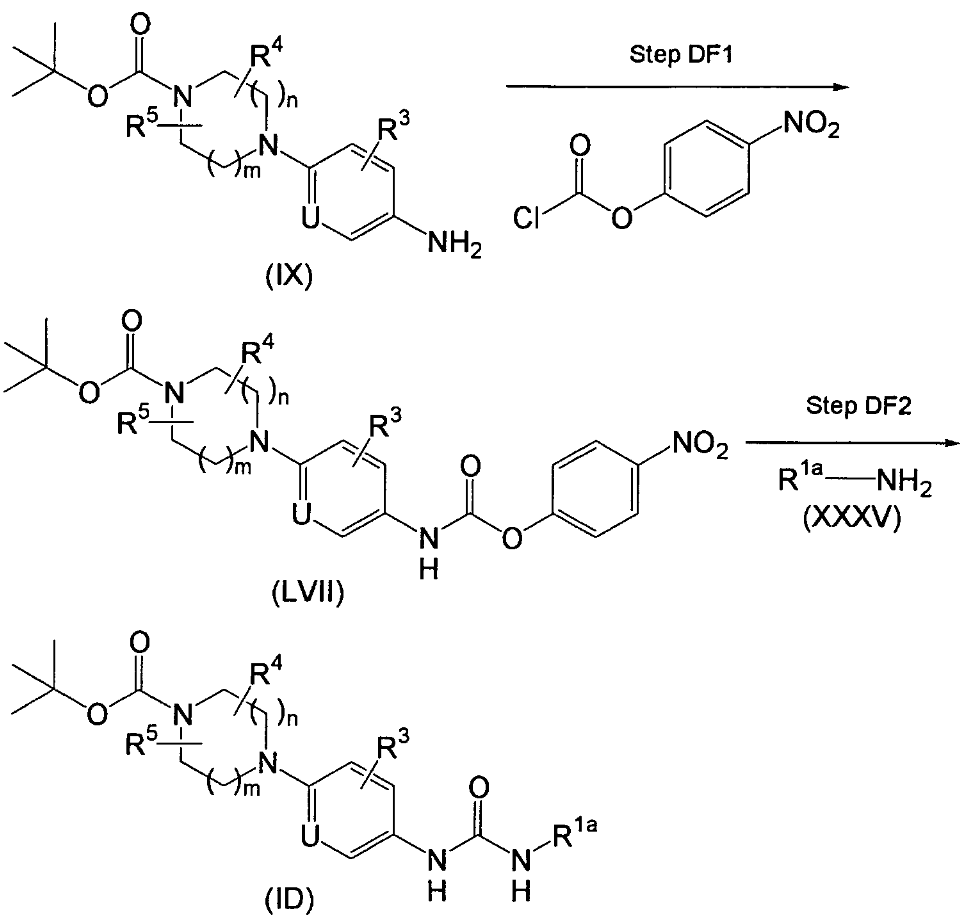


(IX)

(LVII)

(XXXV)

(ID)

In the present invention, $R^3$, $R^4$, $R^5$, $R^{1a}$, U, m and n are the same as described above.

Step DF1

This is a step of producing a compound having the general formula (LVII).

The step is performed by allowing a compound having the general formula (IX) to react with p-nitrophenyl chloroformate in an inert solvent in the presence of a base.

The inert solvent used in this step is not particularly limited as long as the solvent dissolves a certain amount of starting materials without inhibiting the reaction. Examples thereof include hydrocarbons such as pentane, hexane, octane, petroleum ether and ligroin; amides such as formamide, N,N-dimethylformamide, N,N-dimethylacetamide, N-methyl-2-pyrrolidone, N-methylpyrrolidinone and hexamethylphosphoric triamide; ethers such as diethyl ether, diisopropyl ether, tetrahydrofuran, dioxane, dimethoxyethane and diethylene glycol dimethyl ether; sulfoxides such as dimethyl sulfoxide and sulfolane; nitriles such as acetonitrile and isobutyronitrile; esters such as ethyl formate, ethyl acetate, propyl acetate, butyl acetate and diethyl carbonate; ketones such as acetone, methyl ethyl ketone, 4-methyl-2-pentanone, methyl isobutyl ketone, isophorone and cyclohexanone; nitro compounds such as nitroethane and nitrobenzene; halogenated hydrocarbons such as dichloromethane, 1,2-dichloroethane, dichlorobenzene, chloroform and carbon tetrachloride; aromatic hydrocarbons such as benzene, toluene and xylene; or a mixed solvent thereof, preferably ethers, more preferably tetrahydrofuran.

Examples of bases used in this step include alkali metal carbonates such as sodium carbonate, potassium carbonate and lithium carbonate; alkali metal hydrogen carbonates such as sodium hydrogen carbonate, potassium hydrogen carbonate and lithium hydrogen carbonate; alkali metal hydrides such as lithium hydride, sodium hydride and potassium hydride; alkali metal hydroxides such as sodium hydroxide, potassium hydroxide, barium hydroxide and lithium hydroxide; alkali metal alkoxides such as sodium methoxide, sodium ethoxide, sodium t-butoxide, potassium methoxide, potassium ethoxide, potassium t-butoxide and lithium methoxide; alkali metal trialkyl siloxides such as sodium trimethylsiloxide, potassium trimethylsiloxide and lithium trimethylsiloxide; alkali metal mercaptans such as sodium methylmercaptan and sodium ethylmercaptan; or organic bases such as N-methylmorpholine, triethylamine, tripropylamine, tributylamine, diisopropylethylamine, dicyclohexylamine, N-methylpiperidine, pyridine, 4-pyrrolidinopyridine, picoline, 4-(N,N-dimethylamino)pyridine, 2,6-di(t-butyl)-4-methylpyridine, quinoline, N,N-dimethylaniline, N,N-diethylaniline, 1,5-diazabicyclo[4.3.0]non-5-ene (DBN), 1,4-diazabicyclo[2.2.2]octane (DABCO) and 1,8-diazabicyclo[5.4.0]undec-7-ene (DBU); or organometallic bases such as lithium diisopropylamide and lithium bis(trimethylsilyl)amide, preferably organic bases, more preferably pyridine.

The reaction temperature in this step varies depending on the raw material compounds and the inert solvent to be used, and is generally -20˚C to 100˚C, preferably 0˚C to 40˚C.

The reaction time in this step varies depending on the raw material compounds, the inert solvent to be used and the reaction temperature, and is generally 0.5 hour to 96 hours, preferably 1 hour to 24 hours.

Step DF2

This is a step of producing a compound having the general formula (ID).

The step is performed by allowing a compound having the general formula (LVII) to react with a compound having the general formula (XXXV) publicly known or easily obtained using a publicly known compound as a starting material as in a known method in an inert solvent in the presence or absence (preferably absence) of a base.

The inert solvent used in this step is not particularly limited as long as the solvent dissolves a certain amount of starting materials without inhibiting the reaction. Examples thereof include hydrocarbons such as pentane, hexane, octane, petroleum ether and ligroin; amides such as formamide, N,N-dimethylformamide, N,N-dimethylacetamide, N-methyl-2-pyrrolidone, N-methylpyrrolidinone and hexamethylphosphoric triamide; ethers such as diethyl ether, diisopropyl ether, tetrahydrofuran, dioxane, dimethoxyethane and diethylene glycol dimethyl ether; sulfoxides such as dimethyl sulfoxide and sulfolane; nitriles such as acetonitrile and isobutyronitrile; esters such as ethyl formate, ethyl acetate, propyl acetate, butyl acetate and diethyl carbonate; ketones such as acetone, methyl ethyl ketone, 4-methyl-2-pentanone, methyl isobutyl ketone, isophorone and cyclohexanone; nitro compounds such as nitroethane and nitrobenzene; halogenated hydrocarbons such as dichloromethane, 1,2-dichloroethane, dichlorobenzene, chloroform and carbon tetrachloride; aromatic hydrocarbons such as benzene, toluene and xylene; or a mixed solvent thereof, preferably nitriles, more preferably acetonitrile.

Examples of bases used in this step include alkali metal carbonates such as sodium carbonate, potassium carbonate and lithium carbonate; alkali metal hydrogen carbonates such as sodium hydrogen carbonate, potassium hydrogen carbonate and lithium hydrogen carbonate; alkali metal hydrides such as lithium hydride, sodium hydride and potassium hydride; alkali metal hydroxides such as sodium hydroxide, potassium hydroxide, barium hydroxide and lithium hydroxide; alkali metal alkoxides such as sodium methoxide, sodium ethoxide, sodium t-butoxide, potassium methoxide, potassium ethoxide, potassium t-butoxide and lithium methoxide; alkali metal trialkyl siloxides such as sodium trimethylsiloxide, potassium trimethylsiloxide and lithium trimethylsiloxide; alkali metal mercaptans such as sodium methylmercaptan and sodium ethylmercaptan; or organic bases such as N-methylmorpholine, triethylamine, tripropylamine, tributylamine, diisopropylethylamine, dicyclohexylamine, N-methylpiperidine, pyridine, 4-pyrrolidinopyridine, picoline, 4-(N,N-dimethylamino)pyridine, 2,6-di(t-butyl)-4-methylpyridine, quinoline, N,N-dimethylaniline, N,N-diethylaniline, 1,5-diazabicyclo[4.3.0]non-5-ene (DBN), 1,4-diazabicyclo[2.2.2]octane (DABCO) and 1,8-diazabicyclo[5.4.0]undec-7-ene (DBU); or organometallic bases such as lithium diisopropylamide and lithium bis(trimethylsilyl)amide, preferably organic bases, more preferably triethylamine.

The reaction temperature in this step varies depending on the raw material compounds and the inert solvent to be used, and is generally 0˚C to 120˚C, preferably 25˚C to 80˚C.

The reaction time in this step varies depending on the raw material compounds, the inert solvent to be used and the reaction temperature, and is generally 0.5 hour to 96 hours, preferably 1 hour to 24 hours.

After completion of the reaction in each step of the above processes DA, DB, DC, DD, DE and DF, the respective target compounds are obtained from the reaction mixture in accordance with a conventional method. For example, the target compound can be obtained by neutralizing the reaction mixture suitably, removing insoluble matter if any by

filtration, adding thereto water and an immiscible organic solvent such as ethyl acetate, separating the organic layer containing the target compound, and after washing with water or other solutions, drying over anhydrous magnesium sulfate, anhydrous sodium sulfate or anhydrous sodium hydrogen carbonate, filtering and distilling off the solvent. The resulting target compound can be separated and purified by using methods conventionally used for separation and purification of organic compounds, such as recrystallization and reprecipitation in combination, and eluting with an appropriate eluent using chromatography, if necessary. Target compounds insoluble in the solvent can be purified by washing the resulting solid crude product with the solvent.

Process E is a process for producing a compound having the general formula (IX), which is a raw material compound in the above processes DA, DB, DC, the step DD2 in the above process DD, the step DE2 in the above process DE and the above process DF (e.g., Tetrahedron Lett. 41, 385 (2000)).

Process E

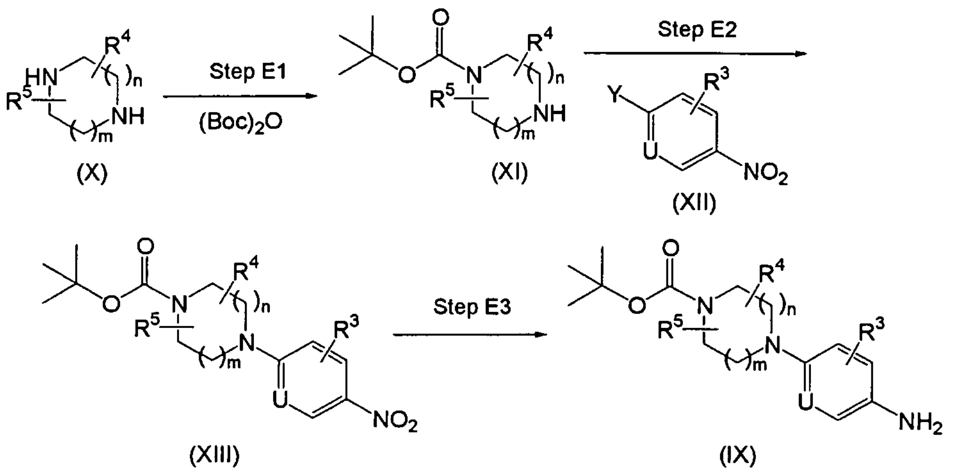


In the present invention, $R^3$, $R^4$, $R^5$, U, Y, m and n are the same as described above.

Step E1

This is a step of producing a compound having the general formula (XI).

The step is performed by allowing a compound having the general formula (X) publicly known or easily obtained using a publicly known compound as a starting material as in a known method to react with di-tert-butyl dicarbonate in an inert solvent.

The inert solvent used in this step is not particularly limited as long as the solvent dissolves a certain amount of starting materials without inhibiting the reaction. Examples thereof include hydrocarbons such as pentane, hexane, octane, petroleum ether and ligroin; amides such as formamide, N,N-dimethylformamide, N,N-dimethylacetamide, N-methyl-2-pyrrolidone, N-methylpyrrolidinone and hexamethylphosphoric triamide; ethers such as diethyl ether, diisopropyl ether, tetrahydrofuran, dioxane, dimethoxyethane and diethylene glycol dimethyl ether; alcohols such as methanol, ethanol, n-propanol, i-propanol, n-butanol, t-butanol, isoamyl alcohol, diethylene glycol, glycerol, octanol, cyclohexanol and methyl cellosolve; sulfoxides such as dimethyl sulfoxide and sulfolane; nitriles such as acetonitrile and isobutyronitrile; esters such as ethyl formate, ethyl acetate, propyl acetate, butyl acetate and diethyl carbonate; ketones such as acetone, methyl ethyl ketone, 4-methyl-2-pentanone, methyl isobutyl ketone, isophorone and cyclohexanone; nitro compounds such as nitroethane and nitrobenzene; halogenated hydrocarbons such as dichloromethane, 1,2-dichloroethane, dichlorobenzene, chloroform and carbon tetrachloride; aromatic hydrocarbons such as benzene, toluene and xylene; or a mixed solvent thereof, preferably ethers, more preferably tetrahydrofuran.

The reaction temperature in this step varies depending on the raw material compounds and the inert solvent to be used, and is generally 0°C to 100°C, preferably 20°C to 30°C.

The reaction time in this step varies depending on the raw material compounds, the inert solvent to be used and the reaction temperature, and is generally 0.5 hour to 72 hours, preferably 2 hours to 8 hours.

Step E2

This is a step of producing a compound having the general formula (XIII).

The step is performed by allowing a compound having the general formula (XI) to react with a compound having the general formula (XII) publicly known or easily obtained using a publicly known compound as a starting material as in a known method in an inert solvent in the presence or absence (preferably presence) of a base in the same manner as in the step AA1 in the above process AA.

Step E3

This is a step of producing a compound having the general formula (IX).

The step is performed in accordance with the method described in J. Org. Chem. 26, 2223 (1961) or J. Med. Chem. 46, 1690 (2003), comprising reducing a compound having the general formula (XIII) in the presence of a palladium catalyst in an inert solvent under a hydrogen atmosphere.

After completion of the reaction in each step of the above process E, the respective target compounds are obtained from the reaction mixture in accordance with a conventional method. For example, the target compound can be obtained by neutralizing the reaction mixture suitably, removing insoluble matter if any by filtration, adding thereto water and an immiscible organic solvent such as ethyl acetate, separating the organic layer containing the target compound, and after washing with water or other solutions, drying over anhydrous magnesium sulfate, anhydrous sodium sulfate or anhydrous sodium hydrogen carbonate, filtering and distilling off the solvent. The resulting target compound can be separated and purified by using methods conventionally used for separation and purification of organic compounds, such as recrystallization and reprecipitation in combination, and eluting with an appropriate eluent using chromatography, if necessary. Target compounds insoluble in the solvent can be purified by washing the resulting solid crude product with the solvent.

Process F is a process for producing a compound having the general formula (IX), which is a raw material compound in the above processes DA, DB, DC, the step DD2 in the above process DD, the step DE2 in the above process DE and the above process DF (e.g., Tetrahedron Lett. 41, 385 (2000)).

Process F

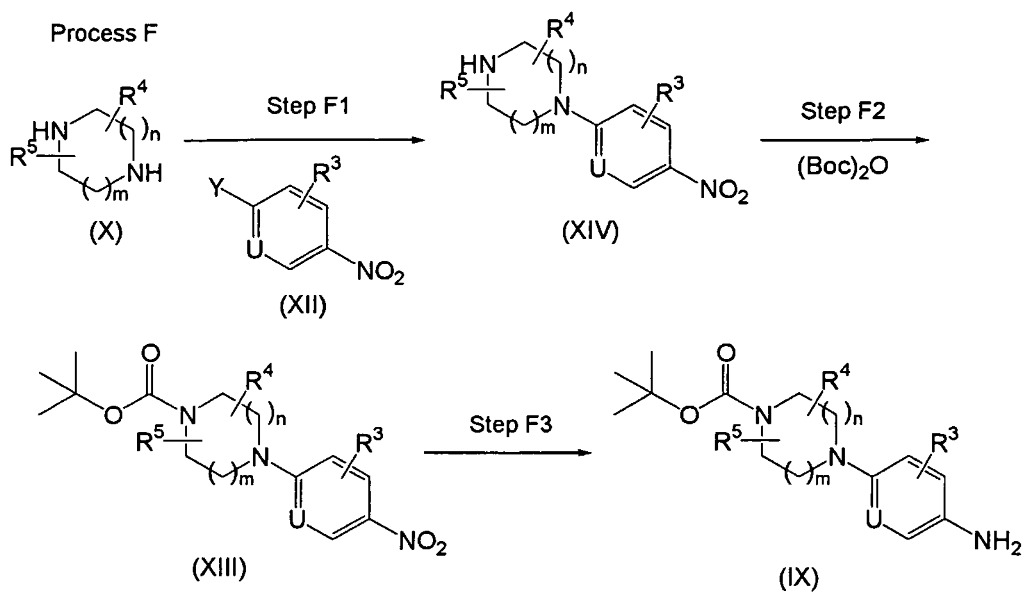


In the present invention, $R^3$, $R^4$, $R^5$, U, Y, m and n are the same as described above.

Step F1

This is a step of producing a compound having the general formula (XIV). The step is performed by allowing a compound having the general formula (X) publicly known or easily obtained using a publicly known compound as a starting material as in a known method to react with a compound having the general formula (XII) publicly known or easily obtained using a publicly known compound as a starting material as in a known method in an inert solvent in the

presence or absence (preferably presence) of a base in the same manner as in the step AA1 in the above process AA.

Step F2

This is a step of producing a compound having the general formula (XIII). The step is performed by allowing a compound having the general formula (XIV) to react with di-tert-butyl dicarbonate in an inert solvent in the same manner as in the step E1 in the above process E.

Step F3

This is a step of producing a compound having the general formula (IX).

The step is performed in accordance with the method described in J. Org. Chem. 26, 2223 (1961) or J. Med. Chem. 46, 1690 (2003), comprising reducing a compound having the general formula (XIII) in the presence of a palladium catalyst in an inert solvent under a hydrogen atmosphere.

After completion of the reaction in each step of the above process F, the respective target compounds are obtained from the reaction mixture in accordance with a conventional method. For example, the target compound can be obtained by neutralizing the reaction mixture suitably, removing insoluble matter if any by filtration, adding thereto water and an immiscible organic solvent such as ethyl acetate, separating the organic layer containing the target compound, and after washing with water or other solutions, drying over anhydrous magnesium sulfate, anhydrous sodium sulfate or anhydrous sodium hydrogen carbonate, filtering and distilling off the solvent. The resulting target compound can be separated and purified by using methods conventionally used for separation and purification of organic compounds, such as recrystallization and reprecipitation in combination, and eluting with an appropriate eluent using chromatography, if necessary. Target compounds insoluble in the solvent can be purified by washing the resulting solid crude product with the solvent.

Process GA is a process for producing a compound having the general formula (IGA).

Process GA

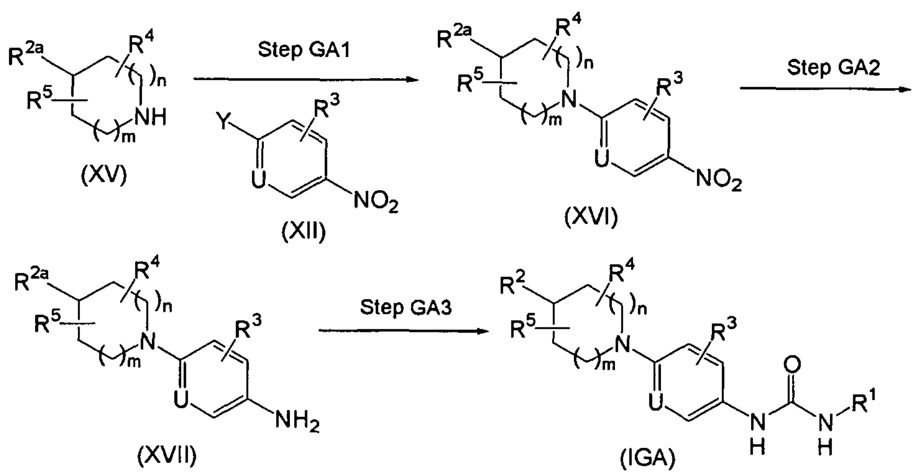


In the present invention, $R^1$, $R^2$, $R^3$, $R^4$, $R^5$, $R^{2a}$, U, Y, m and n are the same as described above.

Step GA1

This is a step of producing a compound having the general formula (XVI). The step is performed by allowing a compound having the general formula (XV) publicly known or easily obtained using a publicly known compound as a starting material as in a known method to react with a compound having the general formula (XII) publicly known or easily obtained using a publicly known compound as a starting material as in a known method in an inert solvent in the presence or absence (preferably presence) of a base in the same manner as in the step AA1 in the above process AA.

Step GA2

This is a step of producing a compound having the general formula (XVII).

The step is performed in accordance with the method described in J. Org. Chem. 26, 2223 (1961) or J. Med. Chem. 46, 1690 (2003), comprising reducing a compound having the general formula (XVI) in the presence of a palladium catalyst in an inert solvent under a hydrogen atmosphere.

Step GA3

This is a step of producing a compound having the general formula (IGA). The step is performed by allowing a compound having the general formula (XVII) to react in the same manner as in the step DA1 in the above process DA, the step DB 1 in the above process DB, the step DC1 in the above process DC, the step DD2 in the above process DD, the step DE2 in the above process DE or the step DF1 and the step DF2 in the above process DF, and then removing the protecting group of the amino group, the hydroxyl group and/or the carboxyl group in $R^{1a}$ and $R^{2a}$ as required.
Process GB is a process for producing a compound having the general formula (IGB).

Process GB

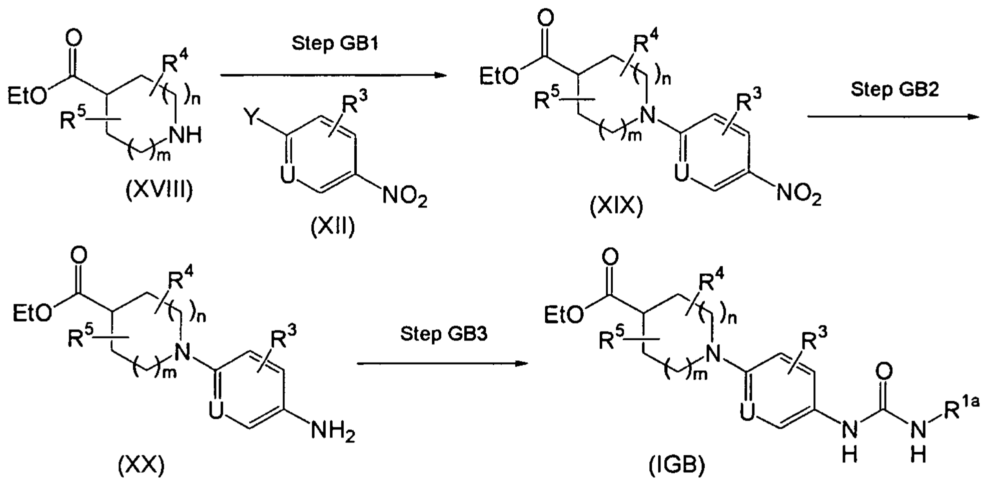


In the present invention, $R^3$, $R^4$, $R^5$, $R^{1a}$, U, Y, m and n are the same as described above.

Step GB1

This is a step of producing a compound having the general formula (XIX). The step is performed by allowing a compound having the general formula (XVIII) publicly known or easily obtained using a publicly known compound as a starting material as in a known method to react with a compound having the general formula (XII) publicly known or easily obtained using a publicly known compound as a starting material as in a known method in an inert solvent in the presence or absence (preferably presence) of a base in the same manner as in the step AA1 in the above process AA.

Step GB2

This is a step of producing a compound having the general formula (XX).
The step is performed in accordance with the method described in J. Org. Chem. 26, 2223 (1961) or J. Med. Chem. 46, 1690 (2003), comprising reducing a compound having the general formula (XIX) in the presence of a palladium catalyst in an inert solvent under a hydrogen atmosphere.

Step GB3

This is a step of producing a compound having the general formula (IGB).
The step is performed by allowing a compound having the general formula (XX) to react in the same manner as in the step DA1 in the above process DA, the step DB 1 in the above process DB, the step DC1 in the above process DC, the step DD2 in the above process DD, the step DE2 in the above process DE or the step DF1 and the step DF2 in the above process DF.
Process GC is a process for producing a compound having the general formula (IGC).

Process GC

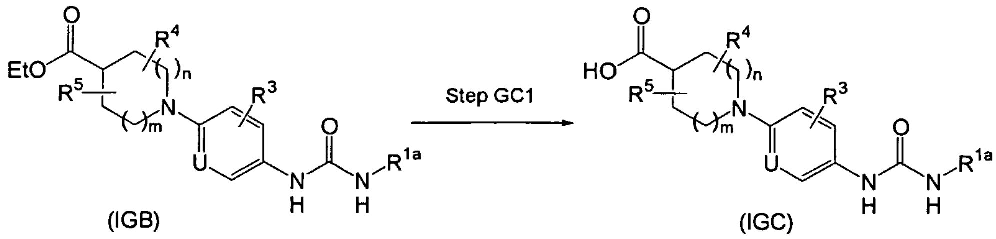


(IGB) (IGC)

In the present invention, $R^3$, $R^4$, $R^5$, $R^{1a}$, U, m and n are the same as described above.

Step GC1

This is a step of producing a compound having the general formula (IGC).

The step is performed by allowing a compound having the general formula (IGB) to react with a base in an inert solvent in the presence of a base.

The inert solvent used in this step is not particularly limited as long as the solvent dissolves a certain amount of starting materials without inhibiting the reaction. Examples thereof include hydrocarbons such as pentane, hexane, octane, petroleum ether and ligroin; amides such as formamide, N,N-dimethylformamide, N,N-dimethylacetamide, N-methyl-2-pyrrolidone, N-methylpyrrolidinone and hexamethylphosphoric triamide; ethers such as diethyl ether, diisopropyl ether, tetrahydrofuran, dioxane, dimethoxyethane and diethylene glycol dimethyl ether; alcohols such as methanol, ethanol, n-propanol, i-propanol, n-butanol, t-butanol, isoamyl alcohol, diethylene glycol, glycerol, octanol, cyclohexanol and methyl cellosolve; sulfoxides such as dimethyl sulfoxide and sulfolane; nitriles such as acetonitrile and isobutyronitrile; ketones such as acetone, methyl ethyl ketone, 4-methyl-2-pentanone, methyl isobutyl ketone, isophorone and cyclohexanone; nitro compounds such as nitroethane and nitrobenzene; halogenated hydrocarbons such as dichloromethane, 1,2-dichloroethane, dichlorobenzene, chloroform and carbon tetrachloride; aromatic hydrocarbons such as benzene, toluene and xylene; or a mixed solvent thereof, preferably ethers, more preferably tetrahydrofuran. The solvent may be a mixed solvent with water (mixing ratio: 1:100 to 100:1, preferably 1:1 to 4:1) where necessary.

Examples of bases used in this step include sodium carbonate, alkali metal hydroxides such as sodium hydroxide, potassium hydroxide, barium hydroxide and lithium hydroxide; or alkali metal trialkyl siloxides such as sodium trimethylsiloxide, potassium trimethylsiloxide and lithium trimethylsiloxide, preferably alkali metal hydroxides, more preferably sodium hydroxide.

The reaction temperature in this step varies depending on the raw material compounds and the inert solvent to be used, and is generally 0˚C to 100˚C, preferably 20˚C to 80˚C.

The reaction time in this step varies depending on the raw material compounds, the inert solvent to be used and the reaction temperature, and is generally 2 hours to 72 hours, preferably 6 hours to 24 hours.

Process GD is a process for producing a compound having the general formula (IGD).

Process GD

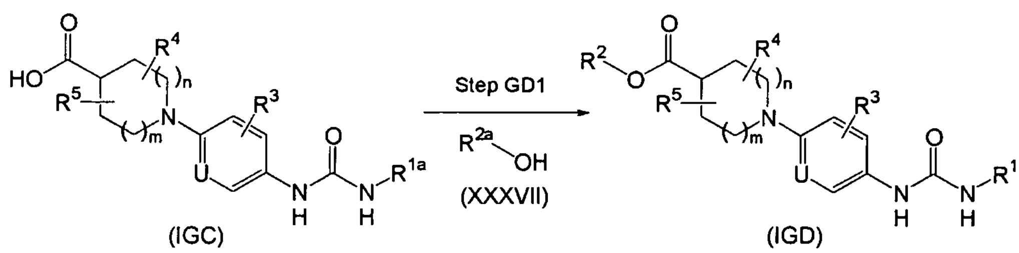


(IGC) (IGD)

In the present invention, $R^1$, $R^2$, $R^3$, $R^4$, $R^5$, $R^{1a}$, $R^{1a}$, U, m and n are the same as described above.

Step GD1

This is a step of producing a compound having the general formula (IGD).

The step is performed by allowing a compound having the general formula (IGC) to react with a compound having the general formula (XXXVII) publicly known or easily obtained using a publicly known compound as a starting material as in a known method and a condensing agent in an inert solvent in the presence of a base, and then removing the protecting group of the amino group, the hydroxyl group and/or the carboxyl group in $R^{1a}$ and $R^{2a}$ as required.

The inert solvent used in this step is not particularly limited as long as the solvent dissolves a certain amount of starting materials without inhibiting the reaction. Examples thereof include hydrocarbons such as petroleum ether; amides such as formamide, N,N-dimethylformamide, N,N-dimethylacetamide, N-methyl-2-pyrrolidone, N-methylpyrrolidinone and hexamethylphosphoric triamide; ethers such as diethyl ether, diisopropyl ether, tetrahydrofuran, dioxane, dimethoxyethane and diethylene glycol dimethyl ether; alcohols such as methanol, ethanol, n-propanol, i-propanol, n-butanol, t-butanol, isoamyl alcohol, diethylene glycol, glycerol, octanol, cyclohexanol and methyl cellosolve; sulfoxides such as dimethyl sulfoxide and sulfolane; nitriles such as acetonitrile and isobutyronitrile; esters such as ethyl formate, ethyl acetate, propyl acetate, butyl acetate and diethyl carbonate; ketones such as acetone, methyl ethyl ketone, 4-methyl-2-pentanone, methyl isobutyl ketone, isophorone and cyclohexanone; nitro compounds such as nitroethane and nitrobenzene; halogenated hydrocarbons such as dichloromethane, 1,2-dichloroethane, dichlorobenzene, chloroform and carbon tetrachloride; aromatic hydrocarbons such as benzene, toluene and xylene; or a mixed solvent thereof, preferably amides, more preferably N,N-dimethylacetamide.

Examples of bases used in this step include inorganic bases like alkali metal carbonates such as sodium carbonate, potassium carbonate and lithium carbonate; alkali metal hydrogen carbonates such as sodium hydrogen carbonate, potassium hydrogen carbonate and lithium hydrogen carbonate; alkali metal hydroxides such as sodium hydroxide, potassium hydroxide, barium hydroxide and lithium hydroxide; alkali metal fluorides such as sodium fluoride and potassium fluoride; alkali metal trialkyl siloxides such as sodium trimethylsiloxide, potassium trimethylsiloxide and lithium trimethylsiloxide; or organic bases such as N-methylmorpholine, triethylamine, tripropylamine, tributylamine, diisopropylethylamine, dicyclohexylamine, N-methylpiperidine, pyridine, 4-pyrrolidinopyridine, picoline, 4-(N,N-dimethylamino)pyridine, 2,6-di(t-butyl)-4-methylpyridine, quinoline, N,N-dimethylaniline, N,N-diethylaniline, 1,5-diazabicyclo [4.3.0]non-5-ene (DBN), 1,4-diazabicyclo[2.2.2]octane (DABCO) and 1,8-diazabicyclo[5.4.0]undec-7-ene (DBU), preferably organic bases, more preferably triethylamine.

Examples of condensing agents used in this step include 1-propanephosphonic acid cyclic anhydride (T3P), 1-(3-dimethylaminopropyl)-3-ethylcarbodiimide hydrochloride (EDCI), isobutyl chloroformate (IBCF), 1,1'-carbonylbis-1H-imidazol (CDI), diethyl cyanophosphonate (DEPC), diphenylphosphoric acid azide (DPPA) and benzotriazol-1-yloxytris(dimethylamino)phosphonium hexafluorophosphate (BOP reagent), preferably T3P or BOP reagent.

The reaction temperature in this step varies depending on the raw material compounds and the inert solvent to be used, and is generally -20˚C to 100˚C, preferably 20˚C to 80˚C.

The reaction time in this step varies depending on the raw material compounds, the inert solvent to be used and the reaction temperature, and is generally 2 hours to 72 hours, preferably 6 hours to 24 hours.

Process GE is a process for producing a compound having the general formula (IGE).

Process GE

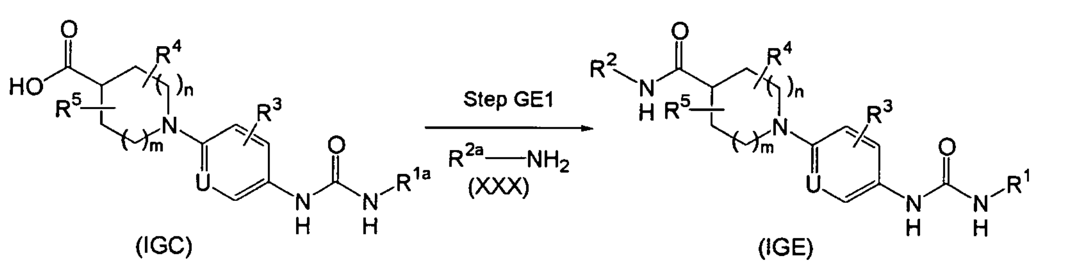


In the present invention, $R^1$, $R^2$, $R^3$, $R^4$, $R^5$, $R^{1a}$, $R^{2a}$, U, m and n are the same as described above.

Step GE 1

This is a step of producing a compound having the general formula (IGE).

The step is performed by allowing a compound having the general formula (IGC) to react with a compound having the general formula (XXX) publicly known or easily obtained using a publicly known compound as a starting material as in a known method and a condensing agent in an inert solvent in the presence of a base in the same manner as in the step GD1 in the above process GD, and then removing the protecting group of the amino group, the hydroxyl

group and/or the carboxyl group in $R^{1a}$ and $R^{2a}$ as required.

Process GF is a process for producing a compound having the general formula (IGF).

Process GF

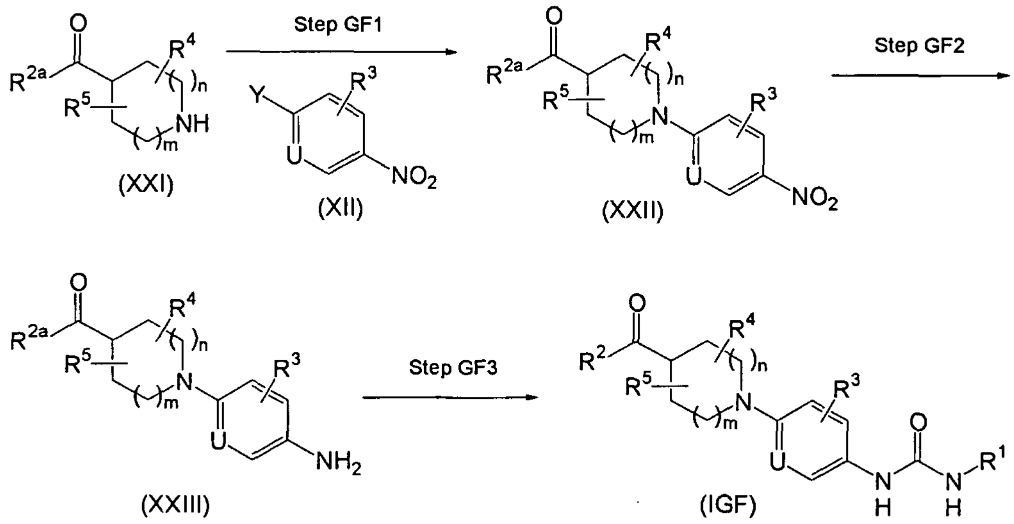

(XXI) (XII) (XXII) (XXIII) (IGF)

In the present invention, $R^1$, $R^2$, $R^3$, $R^4$, $R^5$, $R^{2a}$, U, Y, m and n are the same as described above.

Step GF1

This is a step of producing a compound having the general formula (XXII).

The step is performed by allowing a compound having the general formula (XXI) publicly known or easily obtained using a publicly known compound as a starting material as in a known method to react with a compound having the general formula (XII) publicly known or easily obtained using a publicly known compound as a starting material as in a known method in an inert solvent in the presence or absence (preferably presence) of a base in the same manner as in the step AA1 in the above process AA.

Step GF2

This is a step of producing a compound having the general formula (XXIII).

The step is performed in accordance with the method described in J. Org. Chem. 26, 2223 (1961) or J. Med. Chem. 46, 1690 (2003), comprising reducing a compound having the general formula (XXII) in the presence of a palladium catalyst in an inert solvent under a hydrogen atmosphere.

Step GF3

This is a step of producing a compound having the general formula (IGF).

The step is performed by allowing a compound having the general formula (XXIII) to react in the same manner as in the step DA1 in the above process DA, the step DB1 in the above process DB, the step DC1 in the above process DC, the step DD2 in the above process DD, the step DE2 in the above process DE or the step DF1 and the step DF2 in the above process DF, and then removing the protecting group of the amino group, the hydroxyl group and/or the carboxyl group in $R^{1a}$ and $R^{2a}$ as required.

Process GG is a process for producing a compound having the general formula (IGG).

Process GG

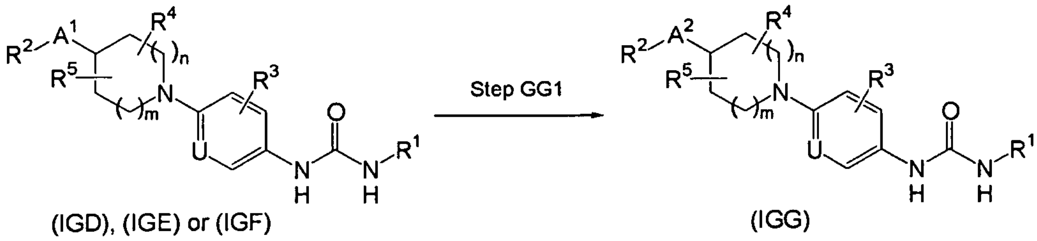


(IGD), (IGE) or (IGF) (IGG)

In the present invention, $R^1$, $R^2$, $R^3$, $R^4$, $R^5$, U, $A^1$, $A^2$, m and n are the same as described above.

Step GG1

This is a step of producing a compound having the general formula (IGG).

The step is performed by allowing a compound having the general formula (IGD), (IGE) or (IGF) to react with a sulfurizing agent in an inert solvent in the same manner as in the Step B 1 in the above process B.

After completion of the reaction in each step of the above processes GA, GB, GC, GD, GE, GF and GG, the respective target compounds are obtained from the reaction mixture in accordance with a conventional method. For example, the target compound can be obtained by neutralizing the reaction mixture suitably, removing insoluble matters by filtration if any, adding thereto a water and an immiscible organic solvent such as ethyl acetate, separating the organic layer containing the target compound, and after washing with water or other solutions, drying over anhydrous magnesium sulfate, anhydrous sodium sulfate or anhydrous sodium hydrogen carbonate, filtering and distilling off the solvent. The resulting target compound can be separated and purified by using methods conventionally used for separation and purification of organic compounds, such as recrystallization and reprecipitation in combination, and eluting with an appropriate eluent using chromatography, if necessary. Target compounds insoluble in the solvent can be purified by washing the resulting solid crude product with the solvent.

Process HA is a process for producing a compound having the general formula (IHA).

Process HA

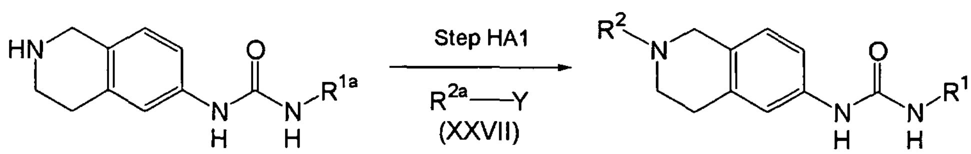


(XXIV) (IHA)

In the present invention, $R^1$, $R^2$, $R^{1a}$, $R^{2a}$ and Y are the same as described above.

Step HA1

This is a step of producing a compound having the general formula (IHA).

The step is performed by allowing a compound having the general formula (XXIV) to react with a compound having the general formula (XXVII) publicly known or easily obtained using a publicly known compound as a starting material as in a known method in an inert solvent in the presence or absence (preferably presence) of a base in the same manner as in the step AA1 in the above process AA, and then removing the protecting group of the amino group, the hydroxyl group and/or the carboxyl group in $R^{1a}$ and $R^{2a}$ as required.

Process HB is a process for producing a compound having the general formula (IHB).

Process HB

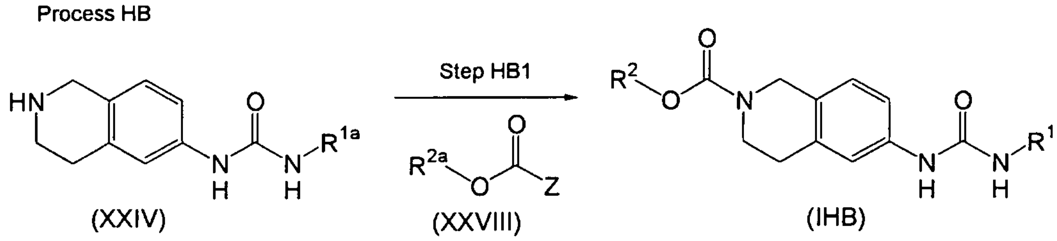


In the present invention, $R^1$, $R^2$, $R^{1a}$, $R^{2a}$ and Z are the same as described above.

Step HB 1

This is a step of producing a compound having the general formula (IHB).

The step is performed by allowing a compound having the general formula (XXIV) to react with a compound having the general formula (XXVIII) publicly known or easily obtained using a publicly known compound as a starting material as in a known method in an inert solvent in the presence or absence (preferably presence) of a base in the same manner as in the step AB1 in the above process AB, and then removing the protecting group of the amino group, the hydroxyl group and/or the carboxyl group in $R^{1a}$ and $R^{2a}$ as required.

Process HC is a process for producing a compound having the general formula (IHC).

Process HC

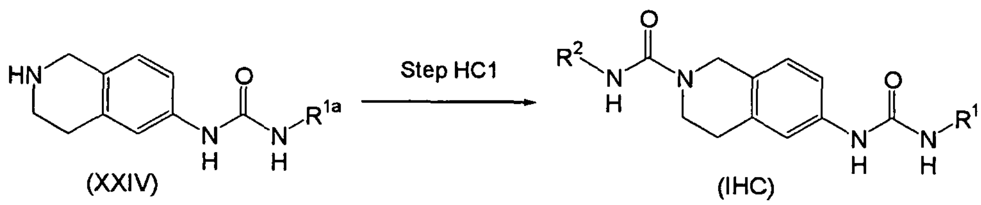


In the present invention, $R^1$, $R^2$ and $R^{1a}$ are the same as described above.

Step HC1

This is a step of producing a compound having the general formula (IHC).

The step is performed by allowing a compound having the general formula (XXIV) to react in the same manner as in the step ACa1 in the above process ACa, the step ACb1 in the above process ACb, the step ACc1 in the above process ACc, the step ACd2 in the above process ACd or the step ACe2 in the above process ACe, and then removing the protecting group of the amino group, the hydroxyl group and/or the carboxyl group in $R^{1a}$ and $R^{2a}$ as required.

Process HD is a process for producing a compound having the general formula (IHD).

Process HD

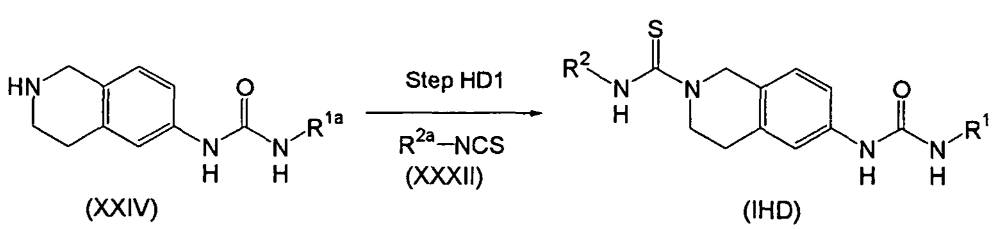


In the present invention, $R^1$, $R^2$, $R^{1a}$ and $R^{2a}$ are the same as described above.

Step HD1

This is a step of producing a compound having the general formula (IHD).

The step is performed by allowing a compound having the general formula (XXIV) to react with a compound having the general formula (XXXII) publicly known or easily obtained using a publicly known compound as a starting material as in a known method in an inert solvent in the same manner as in the step AD 1 in the above process AD, and then removing the protecting group of the amino group, the hydroxyl group and/or the carboxyl group in $R^{1a}$ and $R^{2a}$ as required.

Process HE is a process for producing a compound having the general formula (IHE).

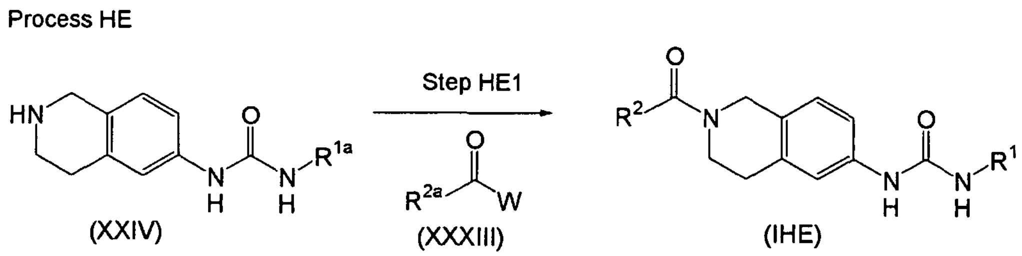


In the present invention, $R^1$, $R^2$, $R^{1a}$, $R^{2a}$ and W are the same as described above.

Step HE1

This is a step of producing a compound having the general formula (IHE).

(i) When W represents a halogen atom

The step is performed by allowing a compound having the general formula (XXIV) to react with a compound having the general formula (XXXIII) publicly known or easily obtained using a publicly known compound as a starting material as in a known method in an inert solvent in the presence or absence (preferably presence) of a base in the same manner as in (i) in the step AE1 in the above process AE, and then removing the protecting group of the amino group, the hydroxyl group and/or the carboxyl group in $R^{1a}$ and $R^{2a}$ as required.

(ii) When W represents a hydroxyl group

The step is performed by allowing a compound having the general formula (XXIV) to react with a compound having the general formula (XXXIII) publicly known or easily obtained using a publicly known compound as a starting material as in a known method and a condensing agent in an inert solvent in the presence of a base in the same manner as in (ii) in the step AE1 in the above process AE, and then removing the protecting group of the amino group, the hydroxyl group and/or the carboxyl group in $R^{1a}$ and $R^{2a}$ as required.

Process HF is a process for producing a compound having the general formula (IHF).

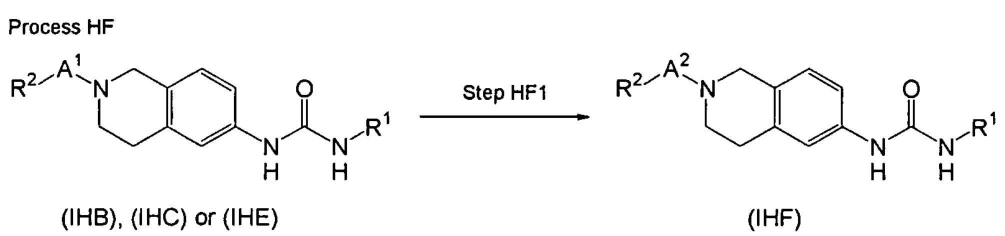


In the present invention, $R^1$, $R^2$, $A^1$ and $A^2$ are the same as described above.

Step HF1

This is a step of producing a compound having the general formula (IHF).

The step is performed by allowing a compound having the general formula (IHB), (IHC) or (IHE) to react with a sulfurizing agent in an inert solvent in the same manner as in the step B 1 in the above process B.

After completion of the reaction in each step of the above processes HA, HB, HC, HD, HE and HF, the respective target compounds are obtained from the reaction mixture in accordance with a conventional method. For example, the target compound can be obtained by neutralizing the reaction mixture suitably, removing insoluble matter if any by filtration, adding thereto water and an immiscible organic solvent such as ethyl acetate, separating the organic layer containing the target compound, and after washing with water or other solutions, drying over anhydrous magnesium sulfate, anhydrous sodium sulfate or anhydrous sodium hydrogen carbonate, filtering and distilling off the solvent. The resulting target compound can be separated and purified by using methods conventionally used for separation and purification of organic compounds, such as recrystallization and reprecipitation in combination, and eluting with an appropriate eluent using chromatography, if necessary. Target compounds insoluble in the solvent can be purified by washing the resulting solid crude product with the solvent.

Process I is a process for producing a compound having the general formula (XXIV), which is a raw material compound in the above processes HA, HB, HC, HD and HE.

Process I

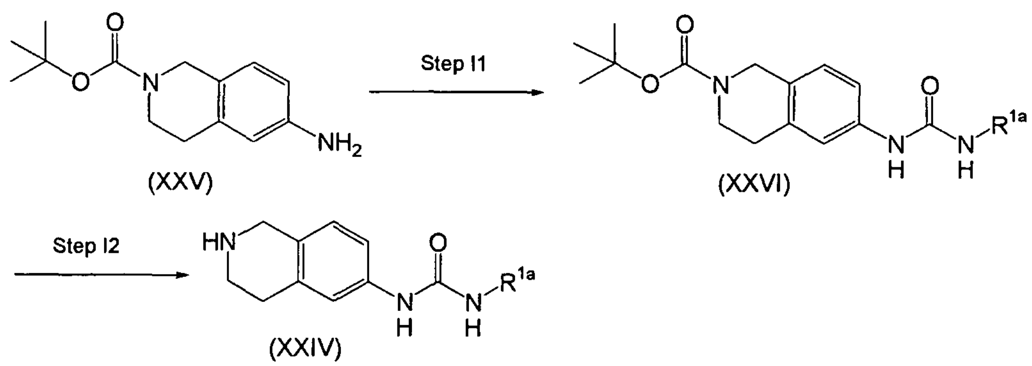


(XXV) (XXVI) (XXIV)

In the present invention, $R^{1a}$ is the same as described above.

Step I1

This is a step of producing a compound having the general formula (XXVI).

The step is performed by allowing a compound having the general formula (XXV) to react in the same manner as in the step DA1 in the above process DA, the step DB1 in the above process DB, the step DC1 in the above process DC, the step DD2 step in the above process DD, the step DE2 in the above process DE or the step DF1 and the step DF2 in the above process DF.

Step 12

This is a step of producing a compound having the general formula (XXIV).

The step is performed by allowing a compound having the general formula (XXVI) to react with acid in an inert solvent in the presence or absence of anisole in the same manner as in the step C1 in the above process C.

After completion of the reaction in each step of the above process I, the respective target compounds are obtained from the reaction mixture in accordance with a conventional method. For example, the target compound can be obtained by neutralizing the reaction mixture suitably, removing insoluble matter if any by filtration, adding thereto water and an immiscible organic solvent such as ethyl acetate, separating the organic layer containing the target compound, and after washing with water or other solutions, drying over anhydrous magnesium sulfate, anhydrous sodium sulfate or anhydrous sodium hydrogen carbonate, filtering and distilling off the solvent. The resulting target compound can be separated and purified by using methods conventionally used for separation and purification of organic compounds, such as recrystallization and reprecipitation in combination, and eluting with an appropriate eluent using chromatography, if necessary. Target compounds insoluble in the solvent can be purified by washing the resulting solid crude product with the solvent.

Process J is a process for producing a compound having the general formula (XXXVIII) wherein Z is a chlorine atom in the compound having the general formula (XXVIII), which is a raw material compound in the above process AB and the above process HB.

Process J

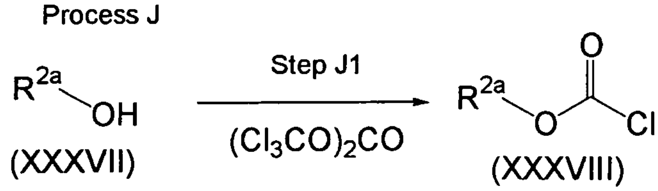


In the present invention, $R^{2a}$ is the same as described above.

Step J1

This is a step of producing a compound having the general formula (XXXVIII).
The step is performed by allowing a compound having the general formula (XXXVII) publicly known or easily obtained using a publicly known compound as a starting material as in a known method to react with triphosgene in an inert solvent.
The inert solvent used in this step is not particularly limited as long as the solvent dissolves a certain amount of starting materials without inhibiting the reaction. Examples thereof include hydrocarbons such as pentane, hexane, octane, petroleum ether and ligroin; amides such as formamide, N,N-dimethylformamide, N,N-dimethylacetamide, N-methyl-2-pyrrolidone, N-methylpyrrolidinone and hexamethylphosphoric triamide; ethers such as diethyl ether, diisopropyl ether, tetrahydrofuran, dioxane, dimethoxyethane and diethylene glycol dimethyl ether; sulfoxides such as dimethyl sulfoxide and sulfolane; nitriles such as acetonitrile and isobutyronitrile; esters such as ethyl formate, ethyl acetate, propyl acetate, butyl acetate and diethyl carbonate; ketones such as acetone, methyl ethyl ketone, 4-methyl-2-pentanone, methyl isobutyl ketone, isophorone and cyclohexanone; nitro compounds such as nitroethane and nitrobenzene; halogenated hydrocarbons such as dichloromethane, 1,2-dichloroethane, dichlorobenzene, chloroform and carbon tetrachloride; aromatic hydrocarbons such as benzene, toluene and xylene; or a mixed solvent thereof, preferably halogenated hydrocarbons, more preferably dichloromethane.
The reaction temperature in this step varies depending on the raw material compounds and the inert solvent to be used, and is generally -20˚C to 60˚C, preferably 0˚C to 30˚C.
The reaction time in this step varies depending on the raw material compounds, the inert solvent to be used and the reaction temperature, and is generally 0.25 hour to 24 hours, preferably 0.5 hour to 2 hours.
After completion of the reaction in the step of the above process J, the target compound is obtained from the reaction mixture in accordance with a conventional method. For example, the target compound can be obtained by neutralizing the reaction mixture suitably, removing insoluble matter if any by filtration, adding thereto water and an immiscible organic solvent such as ethyl acetate, separating the organic layer containing the target compound, and after washing with water or other solutions, drying over anhydrous magnesium sulfate, anhydrous sodium sulfate or anhydrous sodium hydrogen carbonate, filtering and distilling off the solvent. The resulting target compound can be separated and purified by using methods conventionally used for separation and purification of organic compounds, such as recrystallization and reprecipitation in combination, and eluting with an appropriate eluent using chromatography, if necessary. Target compounds insoluble in the solvent can be purified by washing the resulting solid crude product with the solvent.
Process KA is a process for producing a compound having the general formula (IKA).

Process KA

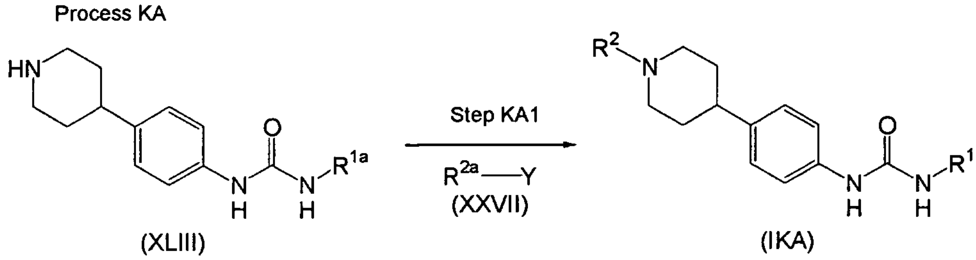

In the present invention, $R^1$, $R^2$, $R^{1a}$, $R^{2a}$ and Y are the same as described above.

Step KA1

This is a step of producing a compound having the general formula (IKA).

The step is performed by allowing a compound having the general formula (XLIII) to react with a compound having the general formula (XXVII) publicly known or easily obtained using a publicly known compound as a starting material as in a known method in an inert solvent in the presence or absence (preferably presence) of a base in the same manner as in the step AA1 in the above process AA, and then removing the protecting group of the amino group, the hydroxyl group and/or the carboxyl group in $R^{1a}$ and $R^{2a}$ as required.

Process KB is a process for producing a compound having the general formula (IKB).

Process KB

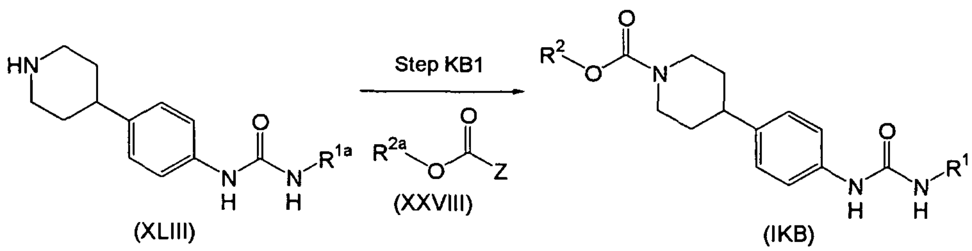


In the present invention, $R^1$, $R^2$, $R^{1a}$, $R^{2a}$ and Z are the same as described above.

Step KB 1

This is a step of producing a compound having the general formula (IKB).

The step is performed by allowing a compound having the general formula (XLIII) to react with a compound having the general formula (XXVIII) publicly known or easily obtained using a publicly known compound as a starting material as in a known method in an inert solvent in the presence or absence (preferably presence) of a base in the same manner as in the step AB 1 in the above process AB, and then removing the protecting group of the amino group, the hydroxyl group and/or the carboxyl group in $R^{1a}$ and $R^{2a}$ as required.

Process KC is a process for producing a compound having the general formula (IKC).

Process KC

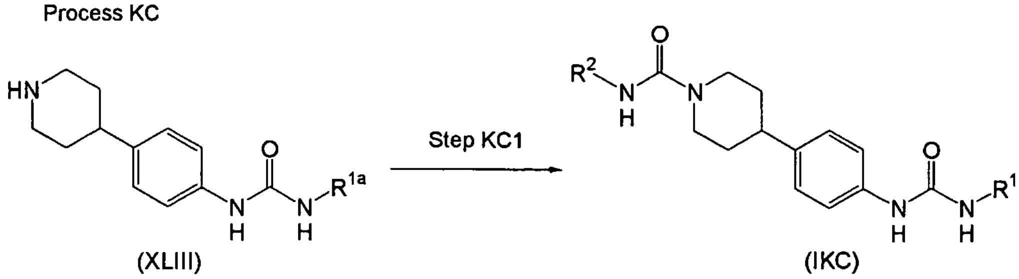


In the present invention, $R^1$, $R^2$ and $R^{1a}$ are the same as described above.

Step KC 1

This is a step of producing a compound having the general formula (IKC).

The step is performed by allowing a compound having the general formula (XLIII) to react in the same manner as in the step ACa1 in the above process ACa, the step ACb1 in the above process ACb, the step ACc1 in the above process ACc, the step ACd2 in the above process ACd or the step ACe2 in the above process ACe, and then removing the protecting group of the amino group, the hydroxyl group and/or the carboxyl group in $R^{1a}$ and $R^{2a}$ as required.

Process KD is a process for producing a compound having the general formula (IKD).

Process KD

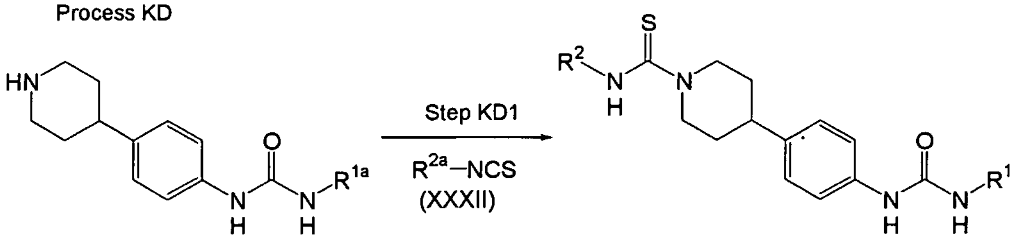


(XLIII) (IKD)

In the present invention, $R^1$, $R^2$, $R^{1a}$ and $R^{2a}$ are the same as described above.

Step KD 1

This is a step of producing a compound having the general formula (IKD).

The step is performed by allowing a compound having the general formula (XLIII) to react with a compound having the general formula (XXXII) publicly known or easily obtained using a publicly known compound as a starting material as in a known method in an inert solvent in the same manner as in the step AD 1 in the above process AD, and then removing the protecting group of the amino group, the hydroxyl group and/or the carboxyl group in $R^{1a}$ and $R^{2a}$ as required.

Process KE is a process for producing a compound having the general formula (IKE).

Process KE

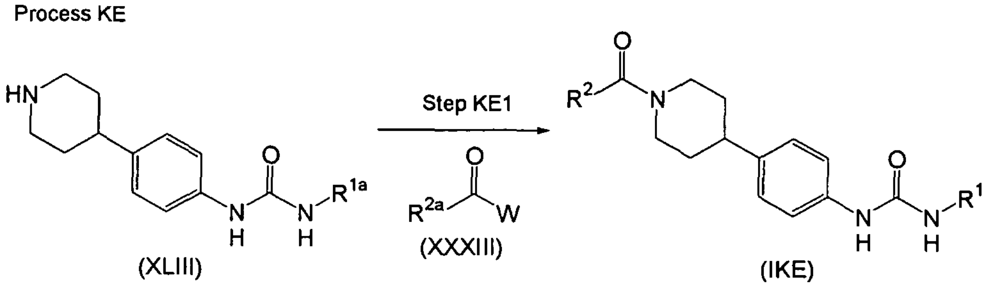


(XLIII) (XXXIII) (IKE)

In the present invention, $R^1$, $R^2$, $R^{1a}$, $R^{2a}$ and W are the same as described above.

Step KE1

This is a step of producing a compound having the general formula (IKE).

(i) When W represents a halogen atom

The step is performed by allowing a compound having the general formula (XLIII) to react with a compound having the general formula (XXXIII) publicly known or easily obtained using a publicly known compound as a starting material as in a known method in an inert solvent in the presence or absence (preferably presence) of a base in the same manner as in (i) in the step AE1 in the above process AE, and then removing the protecting group of the amino group, the hydroxyl group and/or the carboxyl group in $R^{1a}$ and $R^{2a}$ as required.

(ii) When W represents a hydroxyl group

The step is performed by allowing a compound having the general formula (XLIII) to react with a compound having the general formula (XXXIII) publicly known or easily obtained using a publicly known compound as a starting material as in a known method and a condensing agent in an inert solvent in the presence of a base in the same manner as in (ii) in the step AE1 in the above process AE, and then removing the protecting group of the amino group, the hydroxyl group and/or the carboxyl group in $R^{1a}$ and $R^{2a}$ as required.

Process KF is a process for producing a compound having the general formula (IKF).

Process KF

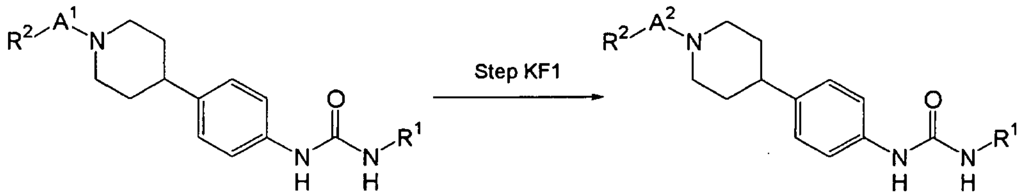


(IKB), (IKC) or (IKE) (IKF)

In the present invention, $R^1$, $R^2$, $A^1$ and $A^2$ are the same as described above.

Step KF1

This is a step of producing a compound having the general formula (IKF).

The step is performed by allowing a compound having the general formula (IKB), (IKC) or (IKE) to react with a sulfurizing agent in an inert solvent in the same manner as in the step B 1 in the above process B.

After completion of the reaction in each step of the above processes KA, KB, KC, KD, KE and KF, the respective target compounds are obtained from the reaction mixture in accordance with a conventional method. For example, the target compound can be obtained by neutralizing the reaction mixture suitably, removing insoluble matter if any by filtration, adding thereto water and an immiscible organic solvent such as ethyl acetate, separating the organic layer containing the target compound, and after washing with water or other solutions, drying over anhydrous magnesium sulfate, anhydrous sodium sulfate or anhydrous sodium hydrogen carbonate, filtering and distilling off the solvent. The resulting target compound can be separated and purified by using methods conventionally used for separation and purification of organic compounds, such as recrystallization and reprecipitation in combination, and eluting with an appropriate eluent using chromatography, if necessary. Target compounds insoluble in the solvent can be purified by washing the resulting solid crude product with the solvent.

Process L is a process for producing a compound having the general formula (XLIII), which is a raw material compound in the above processes KA, KB, KC, KD and KE.

Process L

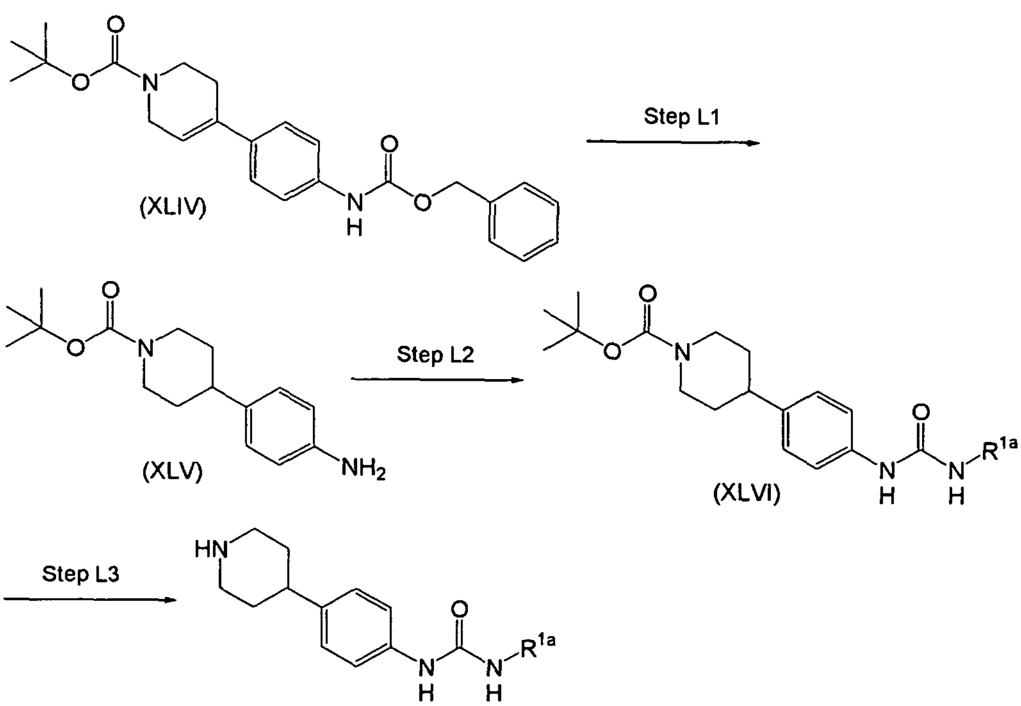


(XLIV)

(XLV)

(XLVI)

(XLIII)

In the present invention, $R^{1a}$ is the same as described above.

Step L1

This is a step of producing a compound having the general formula (XLV).

The step is performed by allowing a compound having the general formula (XLIV) to react in the presence of a palladium catalyst in an inert solvent under a hydrogen atmosphere.

The inert solvent used in this step is not particularly limited as long as the solvent dissolves a certain amount of starting materials without inhibiting the reaction. Examples thereof include amides such as formamide, N,N-dimethylformamide, N,N-dimethylacetamide, N-methyl-2-pyrrolidone, N-methylpyrrolidinone and hexamethylphosphoric triamide; ethers such as diethyl ether, diisopropyl ether, tetrahydrofuran, dioxane, dimethoxyethane and diethylene glycol dimethyl ether; alcohols such as methanol, ethanol, n-propanol, i-propanol, n-butanol, t-butanol, isoamyl alcohol, diethylene glycol, glycerol, octanol, cyclohexanol and methyl cellosolve; sulfoxides such as dimethyl sulfoxide and sulfolane; nitriles such as acetonitrile; esters such as ethyl formate, ethyl acetate, propyl acetate, butyl acetate and diethyl carbonate; aromatic hydrocarbons such as benzene, toluene and xylene; or a mixed solvent thereof, preferably alcohols, more preferably methanol.

Examples of palladium catalysts used in this step include divalent palladium catalysts or zero-valent palladium catalysts, preferably palladium-activated carbon, palladium (II) acetate, palladium (II) trifluoroacetate, palladium black, palladium (II) bromide, palladium (II) chloride, palladium (II) iodide, palladium (II) cyanide, palladium (II) nitrate, palladium (II) oxide, palladium (II) sulfate, dichlorobis(acetonitrile) palladium (II), dichlorobis(benzonitrile) palladium (II), dichloro (1,5-cyclooctadiene) palladium (II), acetylacetone palladium (II), palladium (II) sulfide, tris(dibenzylideneacetone)dipalladium (0), tetrakis(triphenylphosphine)palladium (0) tetrafluoroborate or allylpalladium chloride dimer, more preferably palladium-activated carbon.

The reaction temperature in this step varies depending on the raw material compounds and the inert solvent to be used, and is generally 0°C to 100°C, preferably 25°C to 65°C.

The reaction time in this step varies depending on the raw material compounds, the inert solvent to be used and the reaction temperature, and is generally 0.5 hour to 96 hours, preferably 2 hours to 24 hours.

Step L2

This is a step of producing a compound having the general formula (XLVI).

The step is performed by allowing a compound having the general formula (XLV) to react in the same manner as in the step DA1 in the above process DA, the step DB1 in the above process DB, the step DC1 in the above process DC, the step DD2 in the above process DD, the step DE2 in the above process DE or the step DF1 and the step DF2 in the above process DF.

Step L3

This is a step of producing a compound having the general formula (XLIII).

The step is performed by allowing a compound having the general formula (XLVI) to react with acid in an inert solvent in the presence or absence of anisole in the same manner as in the step C1 in the above process C.

After completion of the reaction in each step of the above process L, the respective target compounds are obtained from the reaction mixture in accordance with a conventional method. For example, the target compound can be obtained by neutralizing the reaction mixture suitably, removing insoluble matter if any by filtration, adding thereto water and an immiscible organic solvent such as ethyl acetate, separating the organic layer containing the target compound, and after washing with water or other solutions, drying over anhydrous magnesium sulfate, anhydrous sodium sulfate or anhydrous sodium hydrogen carbonate, filtering and distilling off the solvent. The resulting target compound can be separated and purified by using methods conventionally used for separation and purification of organic compounds, such as recrystallization and reprecipitation in combination, and eluting with an appropriate eluent using chromatography, if necessary. Target compounds insoluble in the solvent can be purified by washing the resulting solid crude product with the solvent.

Process M is a process for producing a compound (XLIV), which is a raw material compound in the above process L.

Process M

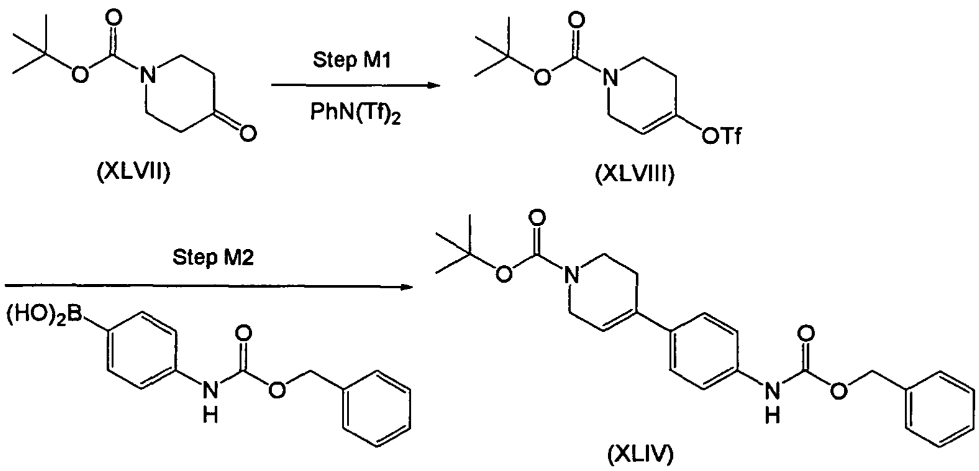


Step M1

This is a step of producing a compound (XLVIII).

The step is performed in accordance with the method described in J. Med. Chem. 43, 2703 (2000) or Org. Lett. 3, 2317 (2001), comprising allowing a publicly known compound (XLVII) to react with N-phenyl-bistrifluoromethane sulfonimide in an inert solvent in the presence of a base.

The inert solvent used in this step is not particularly limited as long as the solvent dissolves a certain amount of starting materials without inhibiting the reaction. Examples thereof include hydrocarbons such as pentane, hexane, octane, petroleum ether and ligroin; ethers such as diethyl ether, diisopropyl ether, tetrahydrofuran, dioxane, dimethoxyethane and diethylene glycol dimethyl ether; nitriles such as acetonitrile and isobutyronitrile; halogenated hydrocarbons such as dichloromethane, 1,2-dichloroethane, dichlorobenzene, chloroform and carbon tetrachloride; aromatic hydrocarbons such as benzene, toluene and xylene; or a mixed solvent thereof, preferably ethers, more preferably tetrahydrofuran.

Examples of bases used in this step include alkali metal hydrides such as lithium hydride, sodium hydride and potassium hydride; alkali metal alkoxides such as sodium methoxide, sodium ethoxide, sodium t-butoxide, potassium methoxide, potassium ethoxide, potassium t-butoxide and lithium methoxide; alkali metal trialkyl siloxides such as sodium trimethylsiloxide, potassium trimethylsiloxide and lithium trimethylsiloxide; alkali metal mercaptans such as sodium methylmercaptan and sodium ethylmercaptan; or organometallic bases such as butyl lithium, lithium diisopropylamide and lithium bis(trimethylsilyl)amide, preferably organometallic bases, more preferably lithium diisopropylamide.

The reaction temperature in this step varies depending on the raw material compounds and the inert solvent to be used, and is generally -100˚C to 50˚C, preferably -78˚C to 25˚C.

The reaction time in this step varies depending on the raw material compounds, the inert solvent to be used and the reaction temperature, and is generally 0.5 hour to 96 hours, preferably 1 hour to 24 hours.

Step M2

This is a step of producing a compound (XLIV).

The step is performed in accordance with the method described in Org. Lett. 3, 2317 (2001), comprising allowing a compound (XLVIII) to react with (4-benzyloxycarbonylaminophenyl) boric acid in an inert solvent in the presence of a palladium catalyst and an inorganic base.

The inert solvent used in this step is not particularly limited as long as the solvent dissolves a certain amount of starting materials without inhibiting the reaction. Examples thereof include hydrocarbons such as pentane, hexane, octane, petroleum ether and ligroin; amides such as formamide, N,N-dimethylformamide, N,N-dimethylacetamide, N-methyl-2-pyrrolidone, N-methylpyrrolidinone and hexamethylphosphoric triamide; ethers such as diethyl ether, diisopropyl ether, tetrahydrofuran, dioxane, dimethoxyethane and diethylene glycol dimethyl ether; sulfoxides such as dimethyl sulfoxide and sulfolane; nitriles such as acetonitrile and isobutyronitrile; esters such as ethyl formate, ethyl acetate, propyl acetate, butyl acetate and diethyl carbonate; ketones such as acetone, methyl ethyl ketone, 4-methyl-2-pentanone, methyl isobutyl ketone, isophorone and cyclohexanone; halogenated hydrocarbons such as dichloromethane, 1,2-dichloroethane, dichlorobenzene, chloroform and carbon tetrachloride; aromatic hydrocarbons such as benzene, toluene and

xylene; or a mixed solvent thereof, preferably ethers, more preferably 1,2-dimethoxyethane. The solvent may be a mixed solvent with water (mixing ratio: 1:100 to 100:1, preferably 1:10 to 1:2, more preferably 1:5) where necessary. The solvent is further preferably a mixed solvent of 1,2-dimethoxyethane and water.

Examples of palladium catalysts used in this step include divalent palladium catalysts or zero-valent palladium catalysts, preferably, palladium-activated carbon, palladium (II) acetate, palladium (II) trifluoroacetate, palladium black, palladium (II) bromide, palladium (II) chloride, palladium (II) iodide, palladium (II) cyanide, palladium (II) nitrate, palladium (II) oxide, palladium (II) sulfate, dichlorobis(acetonitrile) palladium (II), dichlorobis(benzonitrile) palladium (II), dichloro (1,5-cyclooctadiene) palladium (II), acetylacetone palladium (II), palladium (II) sulfide, dichloro[1,1'-bis(diphenylphosphino)ferrocene]palladium (II), tris(dibenzylideneacetone)dipalladium (0), tetrakis(triphenylphosphine)palladium (0), tetrafluoroborate or allylpalladium chloride dimer, more preferably dichloro[1,1'-bis(diphenylphosphino)ferrocene]palladium (II) or tetrakis(triphenylphosphine)palladium (0), further preferably dichloro[1,1'-bis(diphenylphosphino)ferrocene] palladium (II) dichloromethane complex or tetrakis(triphenylphosphine)palladium (0), particularly preferably tetrakis (triphenylphosphine)palladium (0).

Examples of bases used in this step include inorganic bases like alkali metal carbonates such as sodium carbonate, potassium carbonate and lithium carbonate; alkali metal hydrogen carbonates such as sodium hydrogen carbonate, potassium hydrogen carbonate and lithium hydrogen carbonate; alkali metal hydrides such as lithium hydride, sodium hydride and potassium hydride; alkali metal hydroxides such as sodium hydroxide, potassium hydroxide, barium hydroxide and lithium hydroxide; alkali metal fluorides such as sodium fluoride and potassium fluoride; alkali metal alkoxides such as sodium methoxide, sodium ethoxide, sodium t-butoxide, potassium methoxide, potassium ethoxide, potassium t-butoxide and lithium methoxide; alkali metal trialkyl siloxides such as sodium trimethylsiloxide, potassium trimethylsiloxide and lithium trimethylsiloxide; alkali metal mercaptans such as sodium methylmercaptan and sodium ethylmercaptan; or organometallic bases such as butyl lithium, lithium diisopropylamide and lithium bis(trimethylsilyl) amide, preferably alkali metal carbonates, more preferably sodium carbonate. Lithium chloride may be used together where necessary.

The reaction temperature in this step varies depending on the raw material compounds and the inert solvent to be used, and is generally 20°C to 150°C, preferably 60°C to 100°C.

The reaction time in this step varies depending on the raw material compounds, the inert solvent to be used and the reaction temperature, and is generally 0.5 hour to 96 hours, preferably 1 hour to 24 hours.

Process NA is a process for producing a compound having the general formula (INA).

Process NA

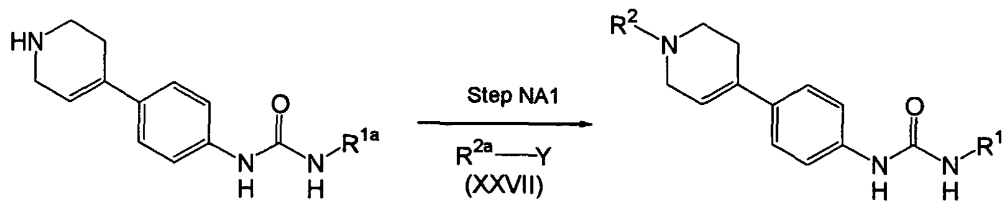


(XLIX) (INA)

In the present invention, $R^1$, $R^2$, $R^{1a}$, $R^{2a}$ and Y are the same as described above.

Step NA1

This is a step of producing a compound having the general formula (INA).

The step is performed by allowing a compound having the general formula (XLIX) to react with a compound having the general formula (XXVII) publicly known or easily obtained using a publicly known compound as a starting material as in a known method in an inert solvent in the presence or absence (preferably presence) of a base in the same manner as in the step AA1 in the above process AA, and then removing the protecting group of the amino group, the hydroxyl group and/or the carboxyl group in $R^{1a}$ and $R^{2a}$ as required.

Process NB is a process for producing a compound having the general formula (INB).

Process NB

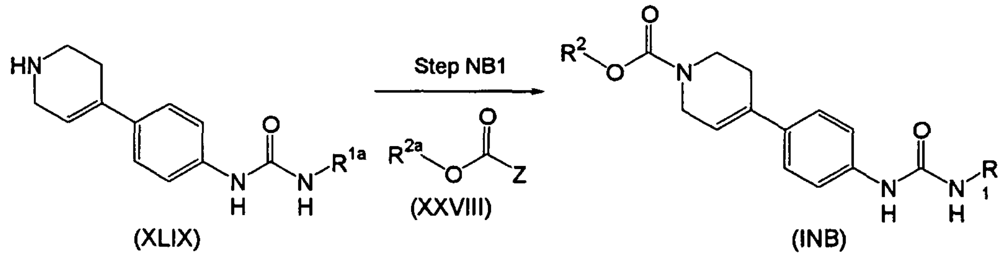


(XLIX) (XXVIII) (INB)

In the present invention, $R^1$, $R^2$, $R^{1a}$, $R^{2a}$ and Z are the same as described above.

Step NB1

This is a step of producing a compound having the general formula (INB).

The step is performed by allowing a compound having the general formula (XLIX) to react with a compound having the general formula (XXVIII) publicly known or easily obtained using a publicly known compound as a starting material as in a known method in an inert solvent in the presence or absence (preferably presence) of a base in the same manner as in the step AB1 in the above process AB, and then removing the protecting group of the amino group, the hydroxyl group and/or the carboxyl group in $R^{1a}$ and $R^{2a}$ as required.

Process NC is a process for producing a compound having the general formula (INC).

Process NC

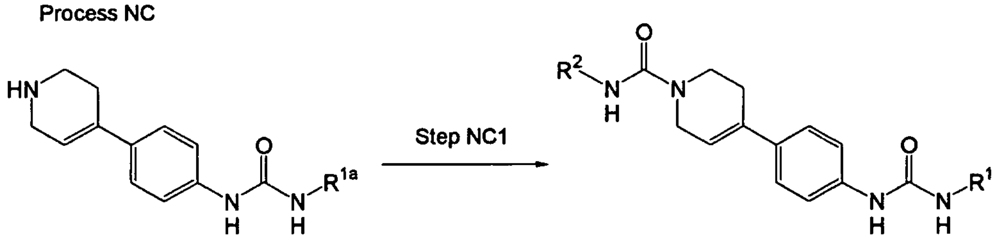


(XLIX) (INC)

In the present invention, $R^1$, $R^2$ and $R^{1a}$ are the same as described above.

Step NC1

This is a step of producing a compound having the general formula (INC).

The step is performed by allowing a compound having the general formula (XLIX) to react in the same manner as in the step ACa1 in the above process ACa, the step ACb1 in the above process ACb, the step ACc1 in the above process ACc, the step ACd2 in the above process ACd or the step ACe2 in the above process ACe, and then removing the protecting group of the amino group, the hydroxyl group and/or the carboxyl group in $R^{1a}$ and $R^{2a}$ as required.

Process ND is a process for producing a compound having the general formula (IND).

Process ND

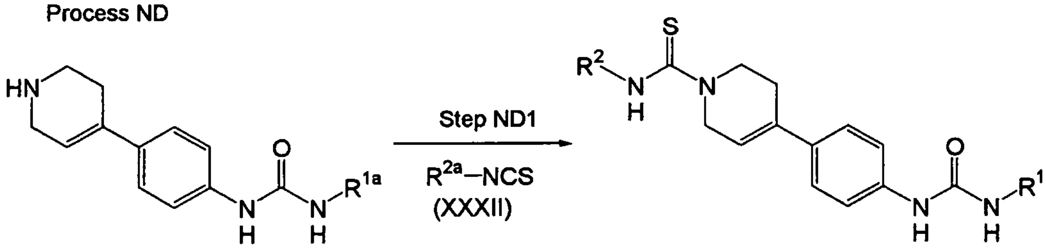


(XLIX) (XXXII) (IND)

In the present invention, $R^1$, $R^2$, $R^{1a}$ and $R^{2a}$ are the same as described above.

Step ND1

This is a step of producing a compound having the general formula (IND).

The step is performed by allowing a compound having the general formula (XLIX) to react with a compound having the general formula (XXXII) publicly known or easily obtained using a publicly known compound as a starting material as in a known method in an inert solvent in the same manner as in the step AD 1 in the above process AD, and then removing the protecting group of the amino group, the hydroxyl group and/or the carboxyl group in $R^{1a}$ and $R^{2a}$ as required.

Process NE is a process for producing a compound having the general formula (INE).

Process NE

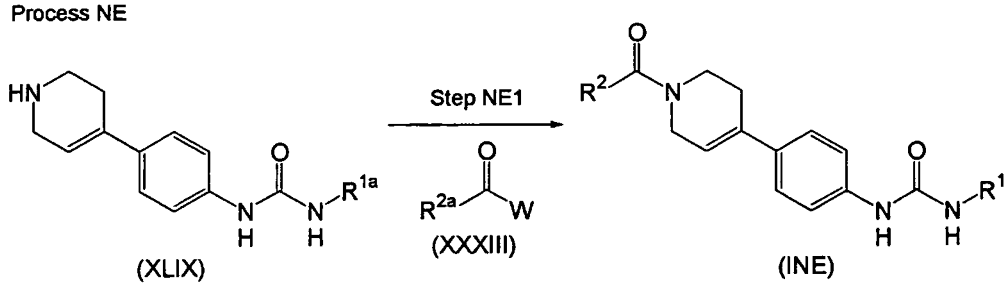


(XLIX) (XXXIII) (INE)

In the present invention, $R^1$, $R^2$, $R^{1a}$, $R^{2a}$ and W are the same as described above.

Step NE1

This is a step of producing a compound having the general formula (INE).

(i) When W represents a halogen atom

The step is performed by allowing a compound having the general formula (XLIX) to react with a compound having the general formula (XXXIII) publicly known or easily obtained using a publicly known compound as a starting material as in a known method in an inert solvent in the presence or absence (preferably presence) of a base in the same manner as in (i) in the step AE1 in the above process AE, and then removing the protecting group of the amino group, the hydroxyl group and/or the carboxyl group in $R^{1a}$ and $R^{2a}$ as required.

(ii) When W represents a hydroxyl group

The step is performed by allowing a compound having the general formula (XLIX) to react with a compound having the general formula (XXXIII) publicly known or easily obtained using a publicly known compound as a starting material as in a known method and a condensing agent in an inert solvent in the presence of a base in the same manner as in (ii) in the step AE1 in the above process AE, and then removing the protecting group of the amino group, the hydroxyl group and/or the carboxyl group in $R^{1a}$ and $R^{2a}$ as required.

Process NF is a process for producing a compound having the general formula (INF).

Process NF

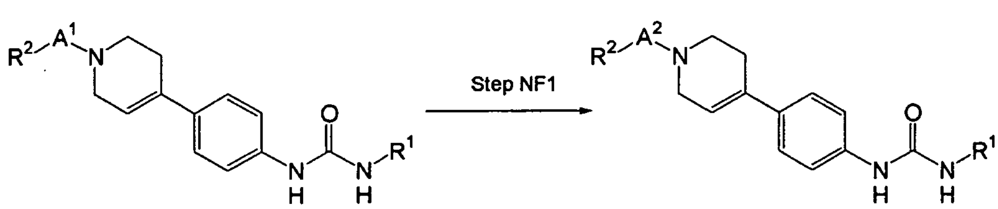


(INB), (INC) or (INE) (INF)

In the present invention, $R^1$, $R^2$, $A^1$ and $A^2$ are the same as described above.

Step NF1

This is a step of producing a compound having the general formula (INF).

The step is performed by allowing a compound having the general formula (INB), (INC) or (INE) to react with a sulfurizing agent in an inert solvent in the same manner as in the step B1 in the above process B.

After completion of the reaction in each step of the above process NA, NB, NC, ND, NE and NF, the respective target compounds are obtained from the reaction mixture in accordance with a conventional method. For example, the target compound can be obtained by neutralizing the reaction mixture suitably, removing insoluble matter if any by filtration, adding thereto water and an immiscible organic solvent such as ethyl acetate, separating the organic layer containing the target compound, and after washing with water or other solutions, drying over anhydrous magnesium sulfate, anhydrous sodium sulfate or anhydrous sodium hydrogen carbonate, filtering and distilling off the solvent. The resulting target compound can be separated and purified by using methods conventionally used for separation and purification of organic compounds, such as recrystallization and reprecipitation in combination, and eluting with an appropriate eluent using chromatography, if necessary. Target compounds insoluble in the solvent can be purified by washing the resulting solid crude product with the solvent.

Process O is a process for producing a compound having the general formula (XLIX), which is a raw material compound in the above processes NA, NB, NC, ND and NE.

Process O

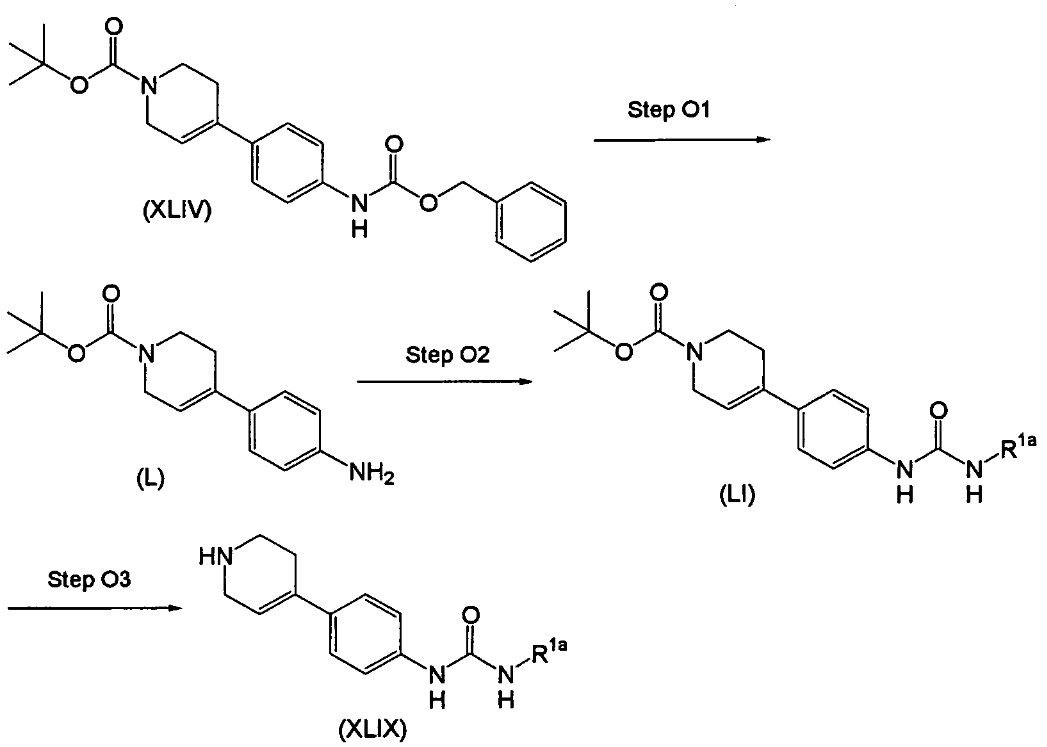


(XLIV) (L) (LI) (XLIX)

In the present invention, $R^{1a}$ is the same as described above.

Step O1

This is a step of producing a compound (L).

The step is performed by allowing the compound (XLIV) obtained in the step M2 in the above process M to react with basic aqueous solution.

Examples of basic aqueous solutions used in this step include aqueous solutions of alkali metal hydroxides such as an aqueous sodium hydroxide solution, an aqueous potassium hydroxide solution, an aqueous barium hydroxide solution and an aqueous lithium hydroxide solution, preferably aqueous solutions of alkali metal hydroxides, more

preferably an aqueous sodium hydroxide solution or an aqueous potassium hydroxide solution.

The reaction temperature in this step varies depending on the raw material compounds and the basic aqueous solution to be used, and is generally 20°C to 120°C, preferably 80°C to 110°C.

The reaction time in this step varies depending on the raw material compounds, the basic aqueous solution to be used and the reaction temperature, and is generally 0.5 hour to 96 hours, preferably 1 hour to 24 hours.

Step 02

This is a step of producing a compound having the general formula (LI).

The step is performed by allowing the compound (L) to react in the same manner as in the step DA1 in the above process DA, the step DB1 in the above process DB, the step DC1 in the above process DC, the step DD2 in the above process DD, the step DE2 in the above process DE or the step DF1 and the step DF2 in the above process DF.

Step 03

This is a step of producing a compound having the general formula (XLIX).

The step is performed by allowing a compound having the general formula (LI) to react with acid in an inert solvent in the presence or absence of anisole in the same manner as in the step C 1 in the above process C.

After completion of the reaction in each step of the above process O, the respective target compounds are obtained from the reaction mixture in accordance with a conventional method. For example, the target compound can be obtained by neutralizing the reaction mixture suitably, removing insoluble matter if any by filtration, adding thereto water and an immiscible organic solvent such as ethyl acetate, separating the organic layer containing the target compound, and after washing with water or other solutions, drying over anhydrous magnesium sulfate, anhydrous sodium sulfate or anhydrous sodium hydrogen carbonate, filtering and distilling off the solvent. The resulting target compound can be separated and purified by using methods conventionally used for separation and purification of organic compounds, such as recrystallization and reprecipitation in combination, and eluting with an appropriate eluent using chromatography, if necessary. Target compounds insoluble in the solvent can be purified by washing the resulting solid crude product with the solvent.

Process AF is a process for producing a compound having the general formula (IAF).

Process AF

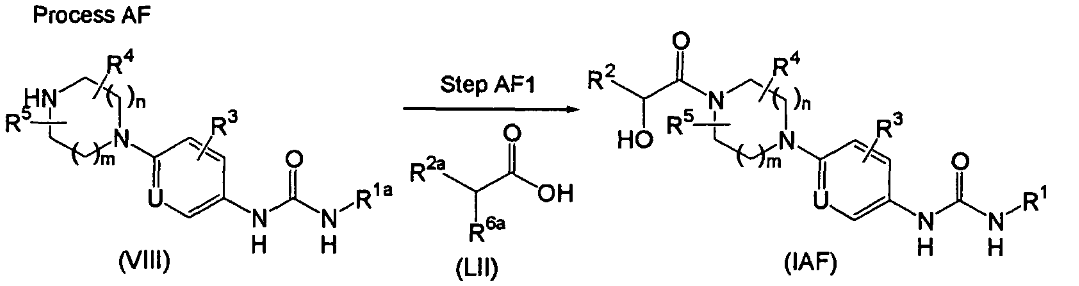


(VIII) (LII) (IAF)

In the present invention, $R^1$, $R^2$, $R^3$, $R^4$, $R^5$, $R^{1a}$, $R^{2a}$, U, m and n are the same as described above and $R^{6a}$ represents the aforementioned hydroxyl group which may be protected.

Step AF1

This is a step of producing a compound having the general formula (IAF).

The step is performed by allowing the compound having the general formula (VIII) obtained in the step C1 in the above process C to react with a compound having the general formula (LII) publicly known or easily obtained using a publicly known compound as a starting material as in a known method and a condensing agent in an inert solvent in the presence of a base in the same manner as in (ii) in the step AE1 in the above process AE, and then removing the protecting group of the amino group, the hydroxyl group and/or the carboxyl group in $R^{1a}$ and $R^{2a}$ and the protecting group of the hydroxyl group in $R^{6a}$ as required.

Process HG is a process for producing a compound having the general formula (IHG).

Process HG

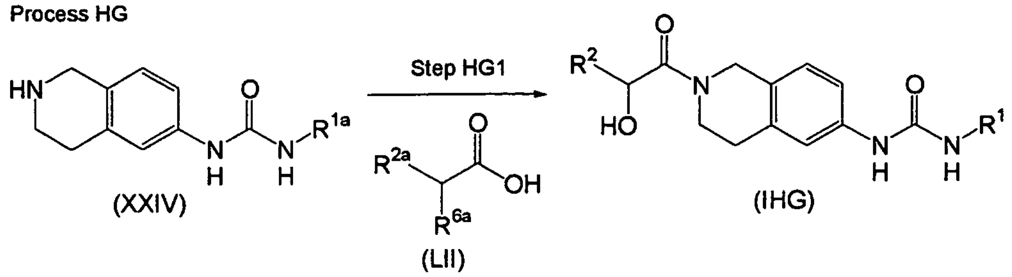


(XXIV) (LII) (IHG)

In the present invention, $R^1$, $R^2$, $R^{1a}$, $R^{2a}$ and $R^{6a}$ are the same as described above.

Step HG1

This is a step of producing a compound having the general formula (IHG).

The step is performed by allowing the compound having the general formula (XXIV) obtained in the step 12 in the above process I to react with a compound having the general formula (LII) publicly known or easily obtained using a publicly known compound as a starting material as in a known method and a condensing agent in an inert solvent in the presence of a base in the same manner as in (ii) in the step AE1 in the above process AE, and then removing the protecting group of the amino group, the hydroxyl group and/or the carboxyl group in $R^{1a}$ and $R^{2a}$ and the protecting group of the hydroxyl group in $R^{6a}$ as required.

Process KG is a process for producing a compound having the general formula (IKG).

Process KG

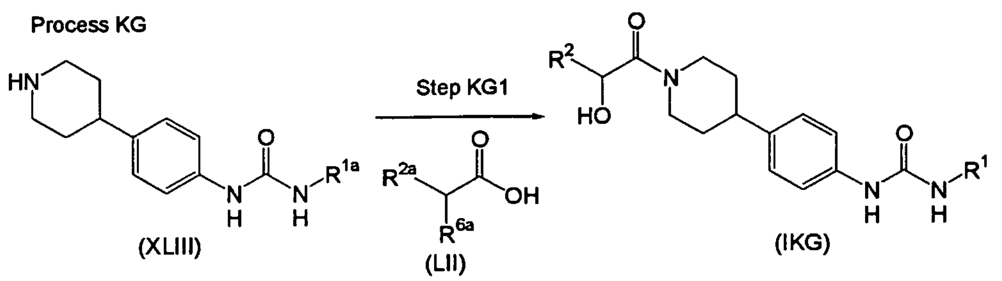


(XLIII) (LII) (IKG)

In the present invention, $R^1$, $R^2$, $R^{1a}$, $R^{2a}$ and $R^{6a}$ are the same as described above.

Step KG1

This is a step of producing a compound having the general formula (IKG).

The step is performed by allowing the compound having the general formula (XLIII) obtained in the step L3 in the above process L to react with a compound having the general formula (LII) publicly known or easily obtained using a publicly known compound as a starting material as in a known method and a condensing agent in an inert solvent in the presence of a base in the same manner as in (ii) in the step AE1 in the above process AE, and then removing the protecting group of the amino group, the hydroxyl group and/or the carboxyl group in $R^{1a}$ and $R^{2a}$ and the protecting group of the hydroxyl group in $R^{6a}$ as required.

Process NG is a process for producing a compound having the general formula (ING).

Process NG

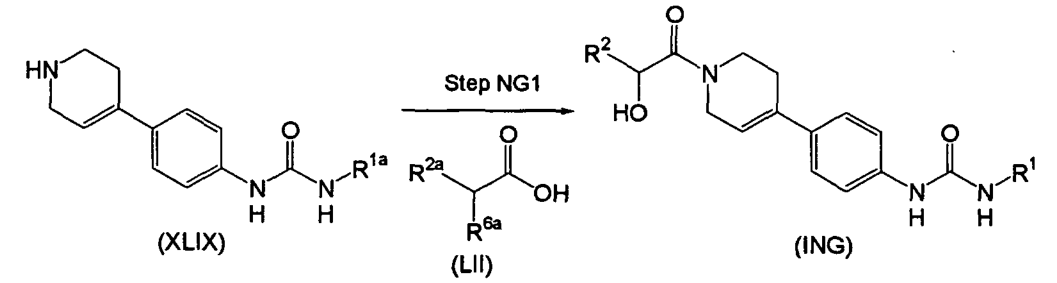


(XLIX) (LII) (ING)

In the present invention, $R^1$, $R^2$, $R^{1a}$, $R^{2a}$ and $R^{6a}$ are the same as described above.

Step NG1

This is a step of producing a compound having the general formula (ING).

The step is performed by allowing the compound having the general formula (XLIX) obtained in the step 03 in the above process O to react with a compound having the general formula (LII) publicly known or easily obtained using a publicly known compound as a starting material as in a known method and a condensing agent in an inert solvent in the presence of a base in the same manner as in (ii) in the step AE1 in the above process AE, and then removing the protecting group of the amino group, the hydroxyl group and/or the carboxyl group in $R^{1a}$ and $R^{2a}$ and the protecting group of the hydroxyl group in $R^{6a}$ as required.

After completion of the reaction in each step of the above processes AF, HG, KG and NG, the respective target compounds are obtained from the reaction mixture in accordance with a conventional method. For example, the target compound can be obtained by neutralizing the reaction mixture suitably, removing insoluble matter if any by filtration, adding thereto water and an immiscible organic solvent such as ethyl acetate, separating the organic layer containing the target compound, and after washing with water or other solutions, drying over anhydrous magnesium sulfate, anhydrous sodium sulfate or anhydrous sodium hydrogen carbonate, filtering and distilling off the solvent. The resulting target compound can be separated and purified by using methods conventionally used for separation and purification of organic compounds, such as recrystallization and reprecipitation in combination, and eluting with an appropriate eluent using chromatography, if necessary. Target compounds insoluble in the solvent can be purified by washing the resulting solid crude product with the solvent.

Raw material compounds (X), (XII), (XV), (XVIII), (XXI), (XXV), (XXVII), (XXVIII), (XXIX), (XXX), (XXXI), (XXXII), (XXXIII), (XXXIV), (XXXV), (XXXVI), (XXXVII), (XLVII) and (LII) are publicly known compounds or easily prepared using a publicly known compound as a starting material by a known method or a method similar thereto. For example, (XXX) is prepared by the method described in J. Org. Chem. 26, 2223 (1961), J. Med. Chem. 42, 5277 (1999) or J. Med. Chem. 46, 1690 (2003), and (XXXVI) is prepared by the method described in Pharmazie. 47, 295 (1992).

In the above description, the protecting group of the "amino group which may be protected", the "hydroxyl group which may be protected" and the "carboxyl group which may be protected" in the definition of $R^{1a}$ and $R^{2a}$ and the "hydroxyl group which may be protected" in the definition of $R^{6a}$ mean a protecting group which can be cleaved by a chemical method such as hydrogenolysis, hydrolysis, electrolysis or photolysis. This refers to protecting groups generally used in synthetic organic chemistry (see, for example, T. W. Greene et al., Protective Groups in Organic Synthesis, 3rd Edition, John Wiley & Sons, Inc. (1999)).

In the above description, the "protecting group" of the "hydroxyl group which may be protected" in the definition of $R^{1a}$, $R^{2a}$ and $R^{6a}$ is not particularly limited as long as it is a protecting group of a hydroxyl group generally used in the field of organic synthetic chemistry. Examples thereof include "general protecting groups associated with an ester based on a hydroxyl group", and are preferably "alkylcarbonyl groups which may be substituted" including alkanoyl groups such as formyl, acetyl, propionyl, butyryl, isobutyryl, pentanoyl, pivaloyl, valeryl, isovaleryl, octanoyl, nonanoyl, decanoyl, 3-methylnonanoyl, 8-methylnonanoyl, 3-ethyloctanoyl, 3,7-dimethyloctanoyl, undecanoyl, dodecanoyl, tridecanoyl, tetradecanoyl, pentadecanoyl, hexadecanoyl, 1-methylpentadecanoyl, 14-methylpentadecanoyl, 13,13-dimethyltetradecanoyl, heptadecanoyl, 15-methylhexadecanoyl, octadecanoyl, 1-methylheptadecanoyl, nonadecanoyl, eicosanoyl and heneicosanoyl, halogenated alkylcarbonyl groups such as chloroacetyl, dichloroacetyl, trichloroacetyl and trifluoroacetyl, alkoxyalkylcarbonyl groups such as methoxyacetyl and unsaturated alkylcarbonyl groups such as acryloyl, propioloyl, methacryloyl, crotonoyl, isocrotonoyl and (E)-2-methyl-2-butenoyl; "arylacyl groups which may be substituted" including arylcarbonyl groups such as benzoyl, α-naphthoyl and β-naphthoyl, halogenated arylcarbonyl groups such as 2-bromobenzoyl and 4-chlorobenzoyl, $C_1$-$C_6$ alkylated arylcarbonyl groups such as 2,4,6-trimethylbenzoyl and 4-toloyl, $C_1$-$C_6$ alkoxylated arylcarbonyl groups such as 4-anisoyl, nitrated arylcarbonyl groups such as 4-nitrobenzoyl and 2-

nitrobenzoyl, $C_2$-$C_7$ alkoxycarbonylated arylcarbonyl groups such as 2-(methoxycarbonyl)benzoyl and arylated arylcarbonyl groups such as 4-phenylbenzoyl; "alkoxycarbonyl groups" including the aforementioned "$C_2$-$C_7$ alkoxycarbonyl groups" and $C_2$-$C_7$ alkoxycarbonyl groups substituted by halogen or a tri-($C_1$-$C_6$ alkyl)silyl group such as 2,2,2-trichloroethoxycarbonyl and 2-trimethylsilylethoxycarbonyl; "tetrahydropyranyl or tetrahydrothiopyranyl groups" such as tetrahydropyran-2-yl, 3-bromotetrahydropyran-2-yl, 4-methoxytetrahydropyran-4-yl, tetrahydrothiopyran-2-yl and 4-methoxytetrahydrothiopyran-4-yl; "tetrahydrofuranyl or tetrahydrothiofuranyl groups" such as tetrahydrofuran-2-yl and tetrahydrothiofuran-2-yl; "silyl groups" including tri-($C_1$-$C_6$ alkyl)silyl groups such as trimethylsilyl, triethylsilyl, isopropyldimethylsilyl, t-butyldimethylsilyl, methyldiisopropylsilyl, methyldi-t-butylsilyl and triisopropylsilyl, and ($C_1$-$C_6$ alkyl)diarylsilyl or di-($C_1$-$C_6$ alkyl)arylsilyl groups such as diphenylmethylsilyl, diphenylbutylsilyl, diphenylisopropylsilyl and phenyldiisopropylsilyl; "alkoxymethyl groups" including $C_1$-$C_6$ alkoxymethyl groups such as methoxymethyl, 1,1-dimethyl-1-methoxymethyl, ethoxymethyl, propoxymethyl, isopropoxymethyl, butoxymethyl and t-butoxymethyl, $C_1$-$C_6$ alkoxy $C_1$-$C_6$ alkoxymethyl groups such as 2-methoxyethoxymethyl and $C_1$-$C_6$ halogenated alkoxymethyl such as 2,2,2-trichloroethoxymethyl and bis(2-chloroethoxy) methyl; "substituted ethyl groups" including $C_1$-$C_6$ alkoxyethyl groups such as 1-ethoxyethyl and 1-(isopropoxy)ethyl and halogenated ethyl groups such as 2,2,2-trichloroethyl; "aralkyl groups" including $C_1$-$C_6$ alkyl groups substituted by 1 to 3 aryl groups such as benzyl, α-naphthylmethyl, β-naphthylmethyl, diphenylmethyl, triphenylmethyl, α-naphthyldiphenylmethyl and 9-anthrylmethyl, and $C_1$-$C_6$ alkyl groups substituted by 1 to 3 aryl groups, in which the aryl ring is substituted by a $C_1$-$C_6$ alkyl, $C_1$-$C_6$ alkoxy, nitro, halogen or cyano group, such as 4-methylbenzyl, 2,4,6-trimethylbenzyl, 3,4,5-trimethylbenzyl, 4-methoxybenzyl, 4-methoxyphenyldiphenylmethyl, 2-nitrobenzyl, 4-nitrobenzyl, 4-chlorobenzyl, 4-bromobenzyl and 4-cyanobenzyl; "alkenyloxycarbonyl groups" such as vinyloxycarbonyl and allyloxycarbonyl; and "aralkyloxycarbonyl groups" in which the aryl ring may be substituted by 1 or 2 $C_1$-$C_6$ alkoxy or nitro groups, such as benzyloxycarbonyl, 4-methoxybenzyloxycarbonyl, 3,4-dimethoxybenzyloxycarbonyl, 2-nitrobenzyloxycarbonyl and 4-nitrobenzyloxycarbonyl, preferably alkylcarbonyl groups which may be substituted, silyl groups or aralkyl groups.

In the above description, the "protecting group" of the "carboxyl group which may be protected" in the definition of $R^{1a}$ and $R^{2a}$ is not particularly limited as long as it is a protecting group of a carboxyl group generally used in the field of organic synthetic chemistry. Examples thereof include "general protecting groups associated with an ester based on a carboxyl group", and are preferably the aforementioned "$C_1$-$C_6$ alkyl groups"; "$C_2$-$C_6$ alkenyl groups" such as ethenyl, 1-propenyl and 2-propenyl; "$C_2$-$C_6$ alkynyl groups" such as ethynyl, 1-propynyl and 2-propynyl; the aforementioned "$C_1$-$C_6$ halogenated alkyl groups"; hydroxy "$C_1$-$C_6$ alkyl groups" such as 2-hydroxyethyl, 2,3-dihydroxypropyl, 3-hydroxypropyl, 3,4-dihydroxybutyl and 4-hydroxybutyl; "$C_2$-$C_7$ alkylcarbonyl"-"$C_1$-$C_6$ alkyl groups" such as acetylmethyl; the aforementioned "aralkyl groups"; or the aforementioned "silyl groups", preferably $C_1$-$C_6$ alkyl groups or aralkyl groups.

In the above description, the "protecting group" of the "amino group which may be protected" in the definition of $R^{1a}$ and $R^{2a}$ is not particularly limited as long as it is a protecting group of an amino group generally used in the field of organic synthetic chemistry. Examples thereof include the same "alkylcarbonyl groups"; "arylcarbonyl groups"; "alkoxycarbonyl groups"; "alkenyloxycarbonyl groups"; "aralkyloxycarbonyl groups"; "silyl groups"; or "aralkyl groups" in the aforementioned "general protecting group associated with an ester based on a hydroxyl group", or "substituted methylene groups which form a Schiff base" such as N,N-dimethylaminomethylene, benzylidene, 4-methoxybenzylidene, 4-nitrobenzylidene, salicylidene, 5-chlorosalicylidene, diphenylmethylene and (5-chloro-2-hydroxyphenyl)phenylmethylene, preferably alkylcarbonyl groups, arylcarbonyl groups or alkoxycarbonyl group, most preferably alkoxycarbonyl groups.

The steps requiring protection and deprotection are performed in accordance with a known method (e.g., the method described in "Protective Groups in Organic Synthesis" (written by Theodora W. Greene and Peter G. M. Wuts, 1999, A Wiley-Interscience Publication)).

When the urea derivative having the above general formula (I) or a pharmacologically acceptable salt thereof according to the present invention is used as a medicine, the medicine is administered as is or after mixing with an appropriate pharmacologically acceptable excipient or diluent orally in the form of, for example, tablets, capsules, granules, powder or syrup, or parenterally by injection or in the form of suppository.

These formulations are prepared by a known method using additives such as an excipient (e.g., organic excipients including sugar derivatives such as lactose, sucrose, glucose, mannitol and sorbitol; starch derivatives such as corn starch, potato starch, α-starch and dextrin; cellulose derivatives such as crystalline cellulose; gum arabic; dextran; and pullulan; inorganic excipients including silicate derivatives such as light anhydrous silicic acid, synthetic aluminum silicate, calcium silicate and magnesium aluminometasilicate; phosphates such as calcium hydrogen phosphate; carbonates such as calcium carbonate; and sulfates such as calcium sulfate), a lubricant (e.g., stearic acid and metal stearates such as calcium stearate and magnesium stearate; talc; colloidal silica; waxes such as beegum and spermaceti; boric acid; adipic acid; sulfates such as sodium sulfate; glycol; fumaric acid; sodium benzoate; DL leucine; fatty acid sodium salt; lauryl sulfates such as sodium lauryl sulfate and magnesium lauryl sulfate; silicic acids such as silicic acid anhydride and silicic acid hydrate; and the above starch derivatives), a binder (e.g., hydroxypropyl cellulose, hydroxypropyl methylcellulose, polyvinylpyrrolidone, macrogol and compounds listed above as excipients), a disintegrant (e.g., cellulose derivatives such as low substitution degree hydroxypropyl cellulose, carboxymethylcellulose, calcium carboxymethylcellulose and internally cross-linked sodium carboxymethylcellulose; and chemically modified starch/cellulose such as carboxymethyl starch, sodium carboxymethyl starch and cross-linked polyvinylpyrrolidone), a stabilizer (parahydroxybenzoate such as methylparaben and propylparaben; alcohols such as chlorobutanol, benzyl alcohol and phenylethyl alcohol; benzalkonium chloride; phenols such as phenol and cresol; thimerosal; dehydroacetic acid; and sorbic acid), a corrigent (e.g., sweeteners, acidulants and flavourings generally used), or a diluent.

The dose varies depending on symptoms, age and other factors. Desirably, when administered orally, the compound is administered to an adult human in an amount of 0.0015 mg/kg body weight (preferably 0.008 mg/kg body weight) as a lower limit and 70 mg/kg body weight (preferably 7 mg/kg body weight) as an upper limit, and when administered intravenously, the compound is administered to an adult human in an amount of 0.00015 mg/kg body weight (preferably 0.0008 mg/kg body weight) as a lower limit and 8.5 mg/kg body weight (preferably 5 mg/kg body weight) as an upper limit per dose per day 1 to 6 times per day depending on symptoms.

Best Mode for Carrying Out the Invention

Hereinbelow, the present invention is described in detail by way of Examples, Test Examples and Preparation Examples but the present invention is not limited to these.

The elution in column chromatography in Examples was performed under observation by TLC (Thin Layer Chromatography). In TLC observation, silica gel $60F_{254}$ manufactured by Merck was adopted as a TLC plate, a solvent used for eluent in column chromatography as a developing solvent and a UV detector as a detecting method. As for silica gel for columns, silica gel SK-85 (230-400 mesh) also manufactured by Merck was used. Abbreviations used in Examples have the following meanings.
mg: milligram, g: gram, mL: millilitre, MHz: megahertz.

(Example 1) 4-{4-[3-(5-chloro-2-methoxy-phenyl)-ureido]-phenyl}-piperazine-1-carboxylic acid tert-butyl ester (Compound No. 1-213)

N-tert-butyloxycarbonyl-N'-(4-aminophenyl)-piperazine (138 mg) and 5-chloro-2-methoxyphenyl isocyanate (138 mg) were dissolved in anhydrous tetrahydrofuran (5 mL) and stirred at room temperature for 14.5 hours. After methanol was added to the reaction solution, the mixed solution was concentrated. The obtained solid was vigorously stirred in hexane/isopropyl ether (5:1), collected by filtration and dried under reduced pressure, and 206 mg (89%) of the title compound was obtained as a pale pink crystal.
$^1$H-NMR spectrum (400MHz,DMSO-d6):δ(ppm)=9.15(1H, s), 8.27(1H, s), 8.21(1H, d, J=2.3Hz), 7.29(2H, d, J=9.0Hz), 7.00(1H, d, J=9.0Hz), 6.94(1H, dd, J=8.6 and 2.8Hz), 6.95(2H, d, J=2.8Hz), 3.87(3H, s), 3.44(4H, t, J=4.9Hz), 2.99(4H, t, J=5.1Hz), 1.42(9H, s).
MS(FAB) m/z:461 $(M + H)^+$.
Melting point: 205˚C.

(Example 2) 4- {4-[3-(2-methoxy-5-methyl-phenyl)-ureido]-phenyl}-piperazine-1-carboxylic acid tert-butyl ester (Compound No. 1-215)

885 mg (88%) of the title compound was obtained from N-tert-butyloxycarbonyl-N'-(4-aminophenyl)-piperazine (633 mg) and 2-methoxy-5-methylphenyl isocyanate (0.36 mL) as a light purple crystal in the same manner as in Example 1.
$^1$H-NMR spectrum (400MHz,$CDCl_3$):δ(ppm)=1.49(9H, s), 2.28(3H, s), 3.09(4H, t, J=5.1Hz), 3.58(4H, t, J=5.2Hz), 3.76 (3H, s), 6.48(1H, s), 6.71(1H, d, J=8.2Hz), 6.77(1H, dd, J=8.3 and 2.0Hz), 6.90(2H, d, J=8.6Hz), 7.10(1H, s), 7.24(1H, d, J=2.0Hz), 7.25(1H, s), 7.94(1H, d, J=2.0Hz).
MS(FAB) m/z:441 $(M + H)^+$.
Melting point: 157˚C.

(Example 3) 4-{4-[3-(3-phenoxy-phenyl)-ureido]-phenyl}-piperazine-1-carboxylic acid tert-butyl ester (Compound No. 1-225)

639 mg (78%) of the title compound was obtained from N-tert-butyloxycarbonyl-N'-(4-aminophenyl)-piperazine (467 mg) and 3-phenoxyphenyl isocyanate (0.33 mL) as a white powder in the same manner as in Example 1.
$^1$H-NMR spectrum (400MHz,$CDCl_3$):δ(ppm)=1.48(9H, s), 3.07(4H, t, J=4.5Hz), 3.57(4H, t, J=5.1Hz), 6.67-6.65(1H, m), 6.81(1H, s), 6.86(2H, d, J=8.6Hz), 6.98-6.96(2H, m), 7.02(1H, t, J=2.2Hz), 7.08-7.05(2H, m), 7.18(3H, d, J=8.3Hz), 7.31-7.27(3H, m).
MS(FAB) m/z:489 $(M + H)^+$.

Melting point: 172˚C.

(Example 4) 4- {4-[3-(2-ethoxy-phenyl)-ureido]-phenyl} -piperazine- I -carboxylic acid tert-butyl ester (Compound No. 1-217)

671 mg (95%) of the title compound was obtained from N-tert-butyloxycarbonyl-N'-(4-aminophenyl)-piperazine (445 mg) and 2-ethoxyphenyl isocyanate (0.25 mL) as a light purple amorphous material in the same manner as in Example 1.
$^1$H-NMR spectrum (400MHz,$CDCl_3$):δ(ppm)=1.29(3H, t, J=6.8Hz), 1.49(9H, s), 3.11(4H, brs), 3.62-3.60(4H, m), 3.98 (2H, q, J=6.9Hz), 6.30(1H, brs), 6.81-6.78(2H, m), 6.98-6.92(3H, m), 7.22(1H, s), 7.27(1H, s), 8.14-8.12(1H, m), 8.13 (1H, d, J=9.4Hz).
MS(FAB) m/z:441 $(M + H)^+$.
Melting point: 84˚C.

(Example 5) 4-{4-[3-(4-heptyloxy-phenyl)-ureido]-phenyl}-piperazine-1-carboxylic acid tert-butyl ester (Compound No. 1-226)

583 mg (83%) of the title compound was obtained from N-tert-butyloxycarbonyl-N'-(4-aminophenyl)-piperazine (382 mg) and 4-heptyloxyphenyl isocyanate (0.34 mL) as a pale pink powder in the same manner as in Example 1. $^1$H-NMR spectrum (400MHz,$CDCl_3$):δ(ppm)=7.25(1H, s), 7.23(2H, d, J=9.4Hz), 7.23(2H, d, J=9.4Hz), 6.88(2H, d, J=8.2Hz), 6.84(2H, d, J=9.0Hz), 6.42(1H, brs), 3.91(2H, t, J=6.7Hz), 3.58(4H, t, J=4.9Hz), 3.08(4H, brs), 1.76(2H, dt, J=10.8 and 4.6Hz), 1.48(9H, s), 1.46-1.40(2H, m), 1.38-1.31(6H, m), 0.89(3H, t, J=6.7Hz).
MS(FAB) m/z:511 $(M + H)^+$.
Melting point: 178˚C.

(Example 6) 4-{4-[3-(2-nitro-phenyl)-ureido]-phenyl}-piperazine-1-carboxylic acid tert-butyl ester (Compound No. 1-227)

1.23 g (95%) of the title compound was obtained from N-tert-butyloxycarbonyl-N'-(4-aminophenyl)-piperazine (818 mg) and 2-nitrophenyl isocyanate (0.508 mg) as an orange powder in the same manner as in Example 1. $^1$H-NMR spectrum (400MHz,$CDCl_3$):δ(ppm)=9.94(1H, s), 8.71(1H, d, J=8.6Hz), 8.18(1H, dd, J=8.6 and 1.6Hz), 7.61(1H, ddd, J=7.0, 7.0, 1.5 Hz), 7.30(2H, d, J=9.0Hz), 7.08(1H, dd, J=7.2 and 7.2Hz), 6.97(2H, d, J=8.6Hz), 6.55(1H, brs), 3.60(4H, t, J=5.1Hz), 3.16(4H, t, J=5.1Hz), 1.49(9H, s).
MS(FAB) m/z:442 $(M + H)^+$.
Melting point: 207˚C.

(Example 7) 4-{4-[3-(2-phenoxy-phenyl)-ureido]-phenyl}-piperazine-1-carboxylic acid tert-butyl ester (Compound No. 1-228)

212 mg (87%) of the title compound was obtained from N-tert-butyloxycarbonyl-N'-(4-aminophenyl)-piperazine (138 mg) and 2-phenoxyphenyl isocyanate (0.14 mL) as a white powder in the same manner as in Example 1.
$^1$H-NMR spectrum (400MHz,DMSO-d6):δ(ppm)=9.02(1H, s), 8.32(1H, s), 8.26(1H, dd, J=8.2 and 1.5Hz), 7.40(2H, dd, J=7.9 and 7.9Hz), 7.28(2H, d, J=9.0Hz), 7.14(1H, dd, J=7.2 and 7.2Hz), 7.10-7.06(1H, m), 7.02(2H, d, J=7.8Hz), 6.94-6.81 (4H, m), 3.44(4H, t, J=4.7Hz), 2.99(4H, t, J=5.1Hz), 1.41(9H, s).
MS(FAB) m/z:489 $(M + H)^+$.
Melting point: 186-187˚C.

(Example 8) 4-{4-[3-(5-chloro-2-phenoxy-phenyl)-ureido]-phenyl}-piperazine-1-carboxylic acid tert-butyl ester (Compound No. 1-229)

212 mg (87%) of the title compound was obtained from N-tert-butyloxycarbonyl-N'-(4-aminophenyl)-piperazine (138 mg) and 5-chloro-2-phenoxyphenyl isocyanate (0.14 mL) as a white powder in the same manner as in Example 1.
$^1$H-NMR spectrum (400MHz,DMSO-d6):δ(ppm)=9.12(1H, s), 8.53(1H, s), 8.38(1H, d, J=2.8Hz), 7.42(2H, dd, J=7.8 and 7.8Hz), 7.28(2H, d, J=8.6Hz), 7.18(1H, dd, J=7.2 and 7.2Hz), 7.06(2H, d, J=8.6Hz), 6.95(1H, dd, J=8.6 and 2.8Hz), 6.89 (2H, d, J=8.6Hz), 6.81(1H, d, J=8.6Hz), 3.43(4H, t, J=4.5Hz), 2.99(4H, t, J=4.9Hz), 1.40(9H, s).
MS(FAB) m/z:523 $(M + H)^+$.
Melting point: 94-96˚C.

(Example 9) 4-{4-[3-(2-difluoromethoxy-phenyl)-ureido]-phenyl}-piperazine-1-carboxylic acid tert-butyl ester (Compound No. 1-230)

67 mg (29%) of the title compound was obtained from N-tert-butyloxycarbonyl-N'-(4-aminophenyl)-piperazine (138 mg) and 2-difluoromethoxyphenyl isocyanate (0.11 mL) as a white powder in the same manner as in Example 1. $^1$H-NMR spectrum (400MHz,DMSO-d6):δ(ppm)=9.10(1H, s), 8.21(1H, d, J=1.5Hz), 8.19(1H, s), 7.30(2H, d, J=9.0Hz), 7.22(1H, t, J=74Hz), 7.19-7.14(2H, m), 6.98(1H, ddd J=7.8, 7.8 and 1.6Hz), 6.90(2H, d, J=8.7Hz), 3.44(4H, t, J=4.7Hz), 3.00(4H, t, J=5.1Hz), 1.42(9H, s).
MS(FAB) m/z:463 $(M + H)^+$.
Melting point: 154-155˚C.

(Example 10) 4-{4-[3-(5-chloro-2-nitro-phenyl)-ureido]-phenyl}-piperazine-1-carboxylic acid tert-butyl ester (Compound No. 1-231)

4.64 g (98%) of the title compound was obtained from N-tert-butyloxycarbonyl-N'-(4-aminophenyl)-piperazine (2.77 g) and 5-chloro-2-nitrophenyl isocyanate (2.58 g) as an orange powder in the same manner as in Example 1.
$^1$H-NMR spectrum (400MHz,DMSO-d6):δ(ppm)=9.81(1H, s), 9.70(1H, s), 8.54(1H, d, J=2.0Hz), 8.14(1H, d, J=9.0Hz), 7.34(2H, d, J=9.0Hz), 7.22(1H, dd, J=9.0 and 2.3Hz), 6.92(2H, d, J=9.0Hz), 3.45(4H, t, J=4.9Hz), 3.02(4H, t, J=5.0Hz), 1.42(9H, s).
MS(FAB) m/z:476 $(M + H)^+$.
Melting point: 205˚C.

(Example 11) 4-{4-[3-(2-amino-5-chloro-phenyl)-ureido]-phenyl}-piperazine-1-carboxylic acid tert-butyl ester (Compound No. 1-232)

4-{4-[3-(5-chloro-2-nitro-phenyl)-ureido]-phenyl}-piperazine-1-carboxylic acid tert-butyl ester and nickel (II) chloride hexahydrate (2.38 g) obtained in Example 10 was suspended in anhydrous tetrahydrofuran (40 mL). Sodium borohydride (757 mg) was slowly added to this suspension in small portions at room temperature. After 1.5 hours, the reaction mixture was concentrated and poured on powdered cellulose, followed by addition of a saturated sodium hydrogen carbonate aqueous solution. The reaction product was eluted from powdered cellulose with ethyl acetate. The organic layer of the obtained eluate was washed with saturated brine, dried over sodium sulfate and filtered and concentrated. The obtained solid was vigorously stirred in hexane, collected by filtration and dried under reduced pressure, and 950 mg (43%) of the title compound was obtained as a white powder. $^1$H-NMR spectrum (400MHz,DMSO-d6):δ (ppm)=8.62(1H, s), 7.73(1H, s), 7.53(1H, s), 7.29(2H, d, J=9.0Hz), 6.89(2H, d, J=9.0Hz), 6.81(1H, dd, J=8.6 and 2.3Hz), 6.71(1H, d, J=8.6Hz), 4.87(2H, s), 3.44(4H, t, J=4.3Hz), 2.99(4H, t, J=4.9Hz), 1.42(9H, s).
MS(FAB) m/z:446 $(M + H)^+$.
Melting point: 187-189˚C.

(Example 12) 4-{4-[3-(5-chloro-2-methanesulfonylamino-phenyl)-ureido]-phenyl}-piperazine-1-carboxylic acid tert-butyl ester (Compound No. 1-233)

Methanesulfonyl chloride (0.043 mL) was added to a pyridine solution (5 mL) of 4-{4-[3-(2-amino-5-chloro-phenyl)-ureido]-phenyl}-piperazine-1-carboxylic acid tert-butyl ester obtained in Example 11 at room temperature. After 1.5 hours, the reaction mixture was poured into water and vigorously stirred with isopropyl ether, followed by extraction with ethyl acetate. The separated organic layer was washed with water (twice) and saturated brine and dried over sodium sulfate and filtered and concentrated. The obtained residue was purified by column chromatography, and the obtained solid was vigorously stirred in a mixture of hexane and isopropyl ether, collected by filtration and dried under reduced pressure, and 214 mg (82%) of the title compound was obtained as a light brown powder.
$^1$H-NMR spectrum (400MHz,DMSO-d6):8(ppm)=9.40(1H, s), 9.11(1H, s), 8.25(2H, d, J=2.4Hz), 7.31-7.26(3H, m), 7.04 (1H, dd, J=8.4 and 2.6Hz), 6.90(2H, d, J=9Hz), 3.44(4H, t, J=4.SHz), 3.00(4H, t, J=4.0Hz), 3.00(3H, s), 1.42(9H, s).
MS(FAB) m/z:524 $(M + H)^+$.
Melting point: 127˚C.

(Example 13) 4-{4-[3-(2-methoxy-phenyl)-ureido]-phenyl}-piperazine-1-carboxylic acid tert-butyl ester (Compound No. 1-234)

764 mg (90%) of the title compound was obtained from N-tert-butyloxycarbonyl-N'-(4-aminophenyl)-piperazine (554 mg) and 2-methoxyphenyl isocyanate (0.40 mL) as a pale pink powder in the same manner as in Example 1. $^1$H-

NMR spectrum (400MHz,DMSO-d6):δ(ppm)=9.06(1H, s), 8.11(1H, d, J=2.0Hz), 8.11(1H, s), 7.29(2H, d, J=9.0Hz), 6.99 (1H, dd, J=8.0 and 1.8Hz), 6.91-6.84(4H, m), 3.86(3H, s), 3.44(4H, t, J=4.9Hz), 2.99(4H, t, J=5.2Hz), 1.42(9H, s).
MS(FAB) m/z:427 $(M + H)^+$.
Melting point: 184˚C.

(Example 14) 4-{4-[3-(2-fluoro-phenyl)-ureido]-phenyl}-piperazine-1-carboxylic acid tert-butyl ester (Compound No. 1-235)

779 mg (94%) of the title compound was obtained from N-tert-butyloxycarbonyl-N'-(4-aminophenyl)-piperazine (554 mg) and 2-fluorophenyl isocyanate (0.34 mL) as a pale yellow powder in the same manner as in Example 1. $^1$H-NMR spectrum (400MHz,DMSO-d6):8(ppm)=8.82(1H, s), 8.42(1H, d, J=2.4Hz), 8.13(1H, ddd, J=8.2, 8.2 and 1.5Hz), 7.30(2H, d, J=9.0Hz), 7.21 (1H, ddd, J=8.1, 8.1 and 1.3Hz), 7.11 (1H, dd, J=7.8 and 7.8Hz), 6.99-6.93 (1H, m), 6.90 (2H, d, J=9.OHz), 3.45(4H, t, J=4.9Hz), 3.00(4H, t, J=5.0Hz), 1.42(9H, s).
MS(FAB) m/z:415 $(M + H)^+$.
Melting point: 164-166˚C.

(Example 15) 4-(4-[3-(2-chloro-phenyl)-ureido]-phenyl}-piperazine-1-carboxylic acid tert-butyl ester (Compound No. 1-236)

765 mg (89%) of the title compound was obtained from N-tert-butyloxycarbonyl-N'-(4-aminophenyl)-piperazine (554 mg) and 2-chlorophenyl isocyanate (0.37 mL) as a grey white powder in the same manner as in Example 1. $^1$H-NMR spectrum (400MHz,DMSO-d6):δ(ppm)=9.17(1H, s), 8.18(1H, s), 8.15(1H, dd, J=8.2 and 1.6Hz), 7.43(1H, dd, J=7.9 and 1.5Hz), 7.30(2H, d, J=9.0Hz), 7.26(1H, ddd, J=8.4, 8.4 and 1.3Hz), 6.99(1H, ddd J=7.7, 7.7 and 1.6Hz), 6.90(2H, d, J=9.0Hz), 3.45(4H, t, J=5.0Hz), 3.00(4H, t, J=5.1Hz), 1.42(9H, s).
MS(FAB) m/z:431 $(M + H)^+$.
Melting point: 196-197˚C.

(Example 16) 4-[4-(3-o-tolyl-ureido)-phenyl]-piperazine-1-carboxylic acid tert-butyl ester (Compound No. 1-237)

741 mg (90%) of the title compound was obtained from N-tert-butyloxycarbonyl-N'-(4-aminophenyl)-piperazine (554 mg) and 2-methylphenyl isocyanate (0.37 mL) as a pale purple powder in the same manner as in Example 1. $^1$H-NMR spectrum (400MHz,DMSO-d6):δ(ppm)=8.76(1H, s), 7.82(1H, d, J=7.0Hz), 7.78(1H, s), 7.30(2H, d, J=9.0Hz), 7.13 (1H, d, J=7.8Hz), 7.09(1H, d, J=7.8Hz), 6.92-6.88(3H, m), 3.44(4H, t, J=4.9Hz), 2.99(4H, t, J=5.1Hz), 2.22(3H, s), 1.42 (9H, s).
MS(FAB) m/z:411 $(M + H)^+$.
Melting point: 188-191˚C.

(Example 17) 4-{4-[3-(2-ethyl-phenyl)-ureido]-phenyl}-piperazine-1-carboxylic acid tert-butyl ester (Compound No. 1-238)

781 mg (92%) of the title compound was obtained from N-tert-butyloxycarbonyl-N'-(4-aminophenyl)-piperazine (554 mg) and 2-ethylphenyl isocyanate (0.42 mL) as a light brown powder in the same manner as in Example 1. $^1$H-NMR spectrum (400MHz,DMSO-d6):δ(ppm)=8.76(1H, s), 7.79(1H, dd, J=8.0 and 1.2Hz), 7.77(1H, s), 7.31(2H, d, J=9.0Hz), 7.16-7.09(2H, m), 6.96(1H, ddd J=7.4, 7.4 and 1.2Hz), 6.89(2H, d, J=9.0Hz), 3.44(4H, t, J=4.9Hz), 2.99(4H, t, J=5.1Hz), 2.59(2H, q, J=7.4Hz), 1.42(9H, s), 1.17(3H, t, J=7.4Hz).
MS(FAB) m/z:425 $(M + H)^+$.
Melting point: 162˚C.

(Example 18) 4-{4-[3-(2-propyl-phenyl)-ureido]-phenyl}-piperazine-1-carboxylic acid tert-butyl ester (Compound No. 1-239)

1.03 g (94%) of the title compound was obtained from N-tert-butyloxycarbonyl-N'-(4-aminophenyl)-piperazine (693 mg) and 2-propylphenyl isocyanate (0.52 mL) as a purple white powder in the same manner as in Example 1. $^1$H-NMR spectrum (400MHz,DMSO-d6):δ(ppm)=8.76(1H, s), 7.76(1H, d, J=8.2Hz), 7.73(1H, s), 7.31(2H, d, J=9.0Hz), 7.14-7.10(2H, m), 6.95(1H, ddd J=7.4, 7.4 and 1.2Hz), 6.89(2H, d, J=9.0Hz), 3.44(4H, t, J=4.9Hz), 2.99(4H, t, J=5.1Hz), 2.55(2H, t, J=7.7Hz), 1.56(2H, sx, J=7.5Hz), 1.42(9H, s), 0.94(3H, t, J=7.2Hz).
MS(FAB) m/z:439 $(M + H)^+$.
Melting point: 177-181˚C.

(Example 19) 4-{4-[3-(2,5-dimethyl-phenyl)-ureido]-phenyl}-piperazine-1-carboxylic acid tert-butyl ester (Compound No. 1-240)

1.19 g (94%) of the title compound was obtained from N-tert-butyloxycarbonyl-N'-(4-aminophenyl)-piperazine (832 mg) and 2,5-dimethylphenyl isocyanate (0.55 mL) as a pale purple powder in the same manner as in Example 1. $^1$H-NMR spectrum (400MHz,DMSO-d6):δ(ppm)=8.82(1H, s), 7.78(1H, s), 7.70(1H, s), 7.33(2H, d, J=7.4Hz), 7.04(1H, d, J=7.4Hz), 6.91(2H, d, J=7.9Hz), 6.75(1H, d, J=7.8Hz), 3.46-3.45(4H, m), 3.00(4H, t, J=4.5Hz), 2.24(3H, s), 2.18(3H, s), 1.42(9H, s).
MS(FAB) m/z:425 $(M + H)^+$.
Melting point: 219-220˚C.

(Example 20) 4-{4-[3-(4-dimethylamino-phenyl)-ureido]-phenyl}-piperazine-1-carboxylic acid tert-butyl ester (Compound No. 1-241)

1.15 g (87%) of the title compound was obtained from N-tert-butyloxycarbonyl-N'-(4-aminophenyl)-piperazine (832 mg) and 4-(dimethylamino)phenyl isocyanate (633 mg) as a white powder in the same manner as in Example 1. $^1$H-NMR spectrum (400MHz,DMSO-d6):δ(ppm)=8.24(1H, s), 8.15(1H, s), 7.27(2H, d, J=9.0Hz), 7.22(2H, d, J=9.0Hz), 6.87(2H, d, J=9.0Hz), 6.67(2H, d, J=9.0Hz), 3.44(4H, t, J=4.7Hz), 2.98(4H, t, J=5.0Hz), 2.82(6H, s), 1.42(9H, s).
MS(FAB) m/z:440 $(M + H)^+$.
Melting point: 207˚C.

(Example 21) 4-{4-[3-(2-sec-butyl-phenyl)-ureido]-phenyl}-piperazine-1-carboxylic acid tert-butyl ester (Compound No. 1-242)

1.28 g (94%) of the title compound was obtained from N-tert-butyloxycarbonyl-N'-(4-aminophenyl)-piperazine (832 mg) and 2-sec-butylphenyl isocyanate (683 mg) as a white powder in the same manner as in Example 1. $^1$H-NMR spectrum (400MHz,DMSO-d6):δ(ppm)=8.68(1H, s), 7.77(1H, s), 7.64(1H, dd, J=8.0 and 1.4Hz), 7.30(2H, d, J=9.0Hz), 7.20(1H, dd, J=7.4 and 1.6Hz), 7.10(1H, ddd J=7.6, 7.6 and 1.6Hz), 7.03(1H, ddd J=7.5, 7.5 and 1.4Hz), 6.88 (2H, d, J=9.0Hz), 3.44(4H, t, J=5.1Hz), 2.99(4H, t, J=5.0Hz), 2.90(1H, q, J=6.2Hz), 1.57(2H, p, J=7.6Hz), 1.42(9H, s), 1.16(3H, d, J=7.1Hz), 0.80(3H, t, J=7.4Hz).
MS(FAB) m/z:453 $(M + H)^+$.
Melting point: 177˚C.

(Example 22) 4- {4-[3-(4-isopropyl-phenyl)-ureido]-phenyl}-piperazine-1-carboxylic acid tert-butyl ester (Compound No. 1-243)

1.24 g (94%) of the title compound was obtained from N-tert-butyloxycarbonyl-N'-(4-aminophenyl)-piperazine (832 mg) and 4-isopropylphenyl isocyanate (683 mg) as a pale purple powder in the same manner as in Example 1. $^1$H-NMR spectrum (400MHz,DMSO-d6):δ(ppm)=8.42(1H, s), 8.34(1H, s), 7.31(2H, d, J=8.6Hz), 7.29(2H, d, J=9.0Hz), 7.11 (2H, d, J=8.6Hz), 6.88(2H, d, J=9.0Hz), 3.44(4H, t, J=4.7Hz), 2.99(4H, t, J=5.1Hz), 2.86-2.76(1H, m), 1.42(9H, s), 1.17 (6H, d, J=7.1Hz).
MS(FAB) m/z:439 $(M + H)^+$.
Melting point: 210˚C.

(Example 23) 4-{4-[3-(4-ethoxy-phenyl)-ureido]-phenyl}-piperazine-1-carboxylic acid tert-butyl ester (Compound No. 1-244)

1.27 g (96%) of the title compound was obtained from N-tert-butyloxycarbonyl-N'-(4-aminophenyl)-piperazine (832 mg) and 4-ethoxyphenyl isocyanate (0.57 mL) as a pale purple powder in the same manner as in Example 1. $^1$H-NMR spectrum (400MHz,DMSO-d6):δ(ppm)=8.32(1H, s), 8.30(1H, s), 7.30(2H, d, J=8.8Hz), 7.28(2H, d, J=8.8Hz), 6.87 (2H, d, J=9.0Hz), 6.82(2H, d, J=9.0Hz), 3.95(2H, q, J=6.8Hz), 3.44(4H, t, J=4.9Hz), 2.98(4H, t, J=5.3Hz), 1.42(9H, s), 1.30(3H, t, J=7.1Hz).
MS(FAB) m/z:441 $(M + H)^+$.
Melting point: 198-200˚C.

(Example 24) 4-{4-[3-(4-ethyl-phenyl)-ureido]-phenyl}-piperazine-1-carboxylic acid tert-butyl ester (Compound No. 1-245)

1.23 g (96%) of the title compound was obtained from N-tert-butyloxycarbonyl-N'-(4-aminophenyl)-piperazine (832 mg) and 4-ethylphenyl isocyanate (0.56 mL) as a white powder in the same manner as in Example 1.
$^1$H-NMR spectrum (400MHz,DMSO-d6):δ(ppm)=8.46(1H, s), 8.37(1H, s), 7.33(2H, d, J=8.6Hz), 7.30(2H, d, J=8.6Hz), 7.10(2H, d, J=8.2Hz), 6.90(2H, d, J=9.0Hz), 3.46(4H, t, J=4.7Hz), 3.00(4H, t, J=4.9Hz), 2.57-2.50(2H, m), 1.42(9H, s), 1.15(3H, t, J=7.4Hz).
MS(FAB) m/z:425 $(M + H)^+$.
Melting point: 204-205˚C.

(Example 25) 4-{4-[3-(4-sec-butyl-phenyl)-ureido]-phenyl}-piperazine-1-carboxylic acid tert-butyl ester (Compound No. 1-246)

1.24 g (92%) of the title compound was obtained from N-tert-butyloxycarbonyl-N'-(4-aminophenyl)-piperazine (832 mg) and 4-sec-butylphenyl isocyanate (0.69 mL) as a white powder in the same manner as in Example 1.
$^1$H-NMR spectrum (400MHz,DMSO-d6):δ(ppm)=8.45(1H, s), 8.38(1H, s), 7.33(2H, d, J=8.2Hz), 7.30(2H, d, J=9.0Hz), 7.09(2H, d, J=8.6Hz), 6.90(2H, d, J=8.6Hz), 3.45(4H, t, J=4.7Hz), 3.00(4H, t, J=4.9Hz), 2.55-2.51(1H, m), 1.52(2H, dt J=7.2 and 2.5Hz), 1.42(9H, s), 1.16(3H, d, J=7.1Hz), 0.76(3H, t, J=7.2Hz).
MS(FAB) m/z:453 $(M + H)^+$.
Melting point: 201-202˚C.

(Example 26) 4-{4-[3-(4-butoxy-phenyl)-ureido]-phenyl}-piperazine-1-carboxylic acid tert-butyl ester (Compound No. 1-247)

1.32 g (94%) of the title compound was obtained from N-tert-butyloxycarbonyl-N'-(4-aminophenyl)-piperazine (832 mg) and 4-butoxyphenyl isocyanate (0.71 mL) as a pale purple powder in the same manner as in Example 1. $^1$H-NMR spectrum (400MHz,DMSO-d6):δ(ppm)=8.35(1H, s), 8.33(1H, s), 7.31(2H, d, J=6.6Hz), 7.29(2H, d, J=7.4Hz), 6.90 (2H, d, J=9.0Hz), 6.85(2H, d, J=8.6Hz), 3.91(2H, t, J=6.6Hz), 3.45(4H, t, J=3.5Hz), 2.99(4H, t, J=4.9Hz), 1.67(2H, p, J=7.2Hz), 1.46-1.37(11H, m), 0.93(3H, t, J=7.2Hz).
MS(FAB) m/z:469 $(M + H)^+$.
Melting point: 185-188˚C.

(Example 27) 4-{4-[3-(2-fluoro-5-trifluoromethyl-phenyl)-ureido]-phenyl}-piperazine-1-carboxylic acid tert-butyl ester (Compound No. 1-248)

1.32 g (91 %) of the title compound was obtained from N-tert-butyloxycarbonyl-N'-(4-aminophenyl)-piperazine (832 mg) and 2-fluoro-5-trifluoromethylphenyl isocyanate (0.56 mL) as a pale purple powder in the same manner as in Example 1.
$^1$H-NMR spectrum (400MHz,DMSO-d6):δ(ppm)=8.93(1H, s), 8.77(1H, d, J=2.7Hz), 8.60(1H, dd, J=7.4 and 2.3Hz), 7.49-7.44(1H, m), 7.36-7.33(1H, m), 7.31(2H, d, J=9.0Hz), 6.91(2H, d, J=9.4Hz), 3.45(4H, t, J=4.9Hz), 3.01(4H, t, J=5.0Hz), 1.42(9H, s).
MS(FAB) m/z:483 $(M + H)^+$.
Melting point: 241˚C.

(Example 28) 4- {4-[3-(2-chloro-5-trifluoromethyl-phenyl)-ureido]-phenyl}-piperazine-1-carboxylic acid tert-butyl ester (Compound No. 1-249)

1.41 g (95%) of the title compound was obtained from N-tert-butyloxycarbonyl-N'-(4-aminophenyl)-piperazine (832 mg) and 2-chloro-5-trifluoromethylphenyl isocyanate (0.58 mL) as a pale yellow powder in the same manner as in Example 1.
$^1$H-NMR spectrum (400MHz,DMSO-d6):δ(ppm)=9.33(1H, s), 8.63(1H, d, J=2.3Hz), 8.49(1H, s), 7.69(1H, d, J=8.2Hz), 7.34-7.31(3H, m), 6.92(2H, d, J=9.0Hz), 3.45(4H, t, J=4.7Hz), 3.01(4H, t, J=5.1Hz), 1.42(9H, s).
MS(FAB) m/z:499 $(M + H)^+$.
Melting point: 221-223˚C.

(Example 29) 4-{4-[3-(2-chloro-5-methyl-phenyl)-ureido]-phenyl}-piperazine-1-carboxylic acid tert-butyl ester (Compound No. 1-250)

1.26 g (95%) of the title compound was obtained from N-tert-butyloxycarbonyl-N'-(4-aminophenyl)-piperazine (832 mg) and 2-chloro-5-methylphenyl isocyanate (0.654 mg) as a light brown powder in the same manner as in Example 1.
$^1$H-NMR spectrum (400MHz,DMSO-d6):δ(ppm)=9.16(1H, s), 8.10(1H, s), 8.00(1H, d, J=1.5Hz), 7.30(3H, dd, J=8.4 and 8.4Hz), 6.90(2H, d, J=9.0Hz), 6.81(1H, dd, J=8.3 and 2.0Hz), 3.46-3.43(4H, m), 3.00(4H, t, J=5.3Hz), 2.27(3H, s), 1.42 (9H, s).
MS(FAB) m/z:445 $(M + H)^+$.
Melting point: 222-224˚C.

(Example 30) 4-{4-[3-(3-ethyl-phenyl)-ureido]-phenyl}-piperazine-1-carboxylic acid tert-butyl ester (Compound No. 1-251)

1.25 g (98%) of the title compound was obtained from N-tert-butyloxycarbonyl-N'-(4-aminophenyl)-piperazine (832 mg) and 3-ethylphenyl isocyanate (0.56 mL) as a white powder in the same manner as in Example 1.
$^1$H-NMR spectrum (400MHz,DMSO-d6):δ(ppm)=8.51(1H, s), 8.41(1H, s), 7.31(2H, d, J=7.9Hz), 7.30(1H, s), 7.22(1H, d, J=8.6Hz), 7.16(1H, dd, J=7.5 and 7.5Hz), 6.90(2H, d, J=7.8Hz), 6.80(1H, d, J=7.4Hz), 3.45-3.45(4H, m), 3.00(4H, t, J=4.5Hz), 2.56(2H, q, J=7.4Hz), 1.42(9H, s), 1.17(3H, t, J=7.4Hz).
MS(FAB) m/z:425 $(M + H)^+$.
Melting point: 160˚C.

(Example 31) 4-[4-(3-m-tolyl-ureido)-phenyl]-piperazine-1-carboxylic acid tert-butyl ester (Compound No. 1-252)

1.17 g (95%) of the title compound was obtained from N-tert-butyloxycarbonyl-N'-(4-aminophenyl)-piperazine (832 mg) and 3-methylphenyl isocyanate (0.50 mL) as a pale purple powder in the same manner as in Example 1.
$^1$H-NMR spectrum (400MHz,DMSO-d6):δ(ppm)=8.48(1H, s), 8.41(1H, s), 7.32-7.28(3H, m), 7.21(1H, d, J=7.5Hz), 7.14 (1H, ddd J=7.6, 7.6 and 1.8Hz), 6.91(2H, dd, J=8.6 and 1.5Hz), 6.77(1H, d, J=7.4Hz), 3.46-3.46(4H, m), 3.00(4H, m), 2.27(3H, s), 1.43(9H, s).
MS(FAB) m/z:411 $(M + H)^+$.
Melting point: 169-172˚C.

(Example 32) 4-{4-[3-(3-chloro-phenyl)-ureido]-phenyl}-piperazine-1-carboxylic acid tert-butyl ester (Compound No. 1-253)

1.21 g (94%) of the title compound was obtained from N-tert-butyloxycarbonyl-N'-(4-aminophenyl)-piperazine (832 mg) and 3-chlorophenyl isocyanate (0.51 mL) as a pale purple powder in the same manner as in Example 1.
$^1$H-NMR spectrum (400MHz,DMSO-d6):δ(ppm)=8.76(1H, s), 8.48(1H, s), 7.68(1H, d, J=2.0Hz), 7.30-7.21(4H, m), 6.97 (1H, dd, J=7.6 and 1.4Hz), 6.89(2H, d, J=7.8Hz), 3.43-3.43(4H, m), 2.98(4H, t, J=4.3Hz), 1.40(9H, s).
MS(FAB) m/z:431 $(M + H)^+$.
Melting point: 163˚C.

(Example 33) 4-{4-[3-(4-tert-butyl-phenyl)-ureido]-phenyl}-piperazine-1-carboxylic acid tert-butyl ester (Compound No. 1-254)

1.28 g (95%) of the title compound was obtained from N-tert-butyloxycarbonyl-N'-(4-aminophenyl)-piperazine (832 mg) and 4-tert-butylphenyl isocyanate (0.69 mL) as a pale purple powder in the same manner as in Example 1.
$^1$H-NMR spectrum (400MHz,DMSO-d6):δ(ppm)=8.47(1H, s), 8.37(1H, s), 7.36-7.27(6H, m), 6.90(2H, d, J=8.2Hz), 3.46-3.46(4H, m), 3.00(4H, t, J=4.9Hz), 1.42(9H, s), 1.26(9H, s).
MS(FAB) m/z:453 $(M + H)^+$.
Melting point: 140-141˚C.

(Example 34) 4-{4-[3-(4-butyl-phenyl)-ureido]-phenyl}-piperazine-1-carboxylic acid tert-butyl ester (Compound No. 1-255)

1.24 g (92%) of the title compound was obtained from N-tert-butyloxycarbonyl-N'-(4-aminophenyl)-piperazine (832 mg) and 4-butylphenyl isocyanate (0.69 mL) as a pale purple powder in the same manner as in Example 1.

$^1$H-NMR spectrum (400MHz,DMSO-d6):δ(ppm)=8.45(1H, s), 8.37(1H, s), 7.33(2H, d, J=7.8Hz), 7.30(2H, d, J=7.4Hz), 7.08(2H, d, J=7.0Hz), 6.90(2H, d, J=7.4Hz), 3.46-3.46(4H, m), 3.00(4H, t, J=4.1Hz), 2.52-2.49(2H, m), 1.56-1.48(2H, m), 1.42(9H, s), 1.29(2H, q, J=7.2Hz), 0.89(3H, dt J=7.5 and 1.6Hz).
MS(FAB) m/z:453 $(M + H)^+$.
Melting point: 167-170˚C.

(Example 35) 4-{4-[3-(5-chloro-2-methyl-phenyl)-ureido]-phenyl}-piperazine-1-carboxylic acid tert-butyl ester (Compound No. 1-256)

1.27 g (95%) of the title compound was obtained from N-tert-butyloxycarbonyl-N'-(4-aminophenyl)-piperazine (832 mg) and 5-chloro-2-methylphenyl isocyanate (0.53 mL) as a pale purple powder in the same manner as in Example 1.
$^1$H-NMR spectrum (400MHz,DMSO-d6):δ(ppm)=8.91(1H, s), 8.05(1H, d, J=2.3Hz), 7.92(1H, s), 7.31(2H, d, J=9.4Hz), 7.16(1H, d, J=8.6Hz), 6.93(1H, dd, J=8.2 and 2.4Hz), 6.90(2H, d, J=9.0Hz), 3.45(4H, t, J=4.7Hz), 3.00(4H, t, J=5.1Hz), 2.21(3H, s),1.42(9H,s).
MS(FAB) m/z:445 $(M + H)^+$.
Melting point: 200˚C.

(Example 36) 4-{4-[3-(5-fluoro-2-methyl-phenyl)-ureido]-phenyl}-piperazine-1-carboxylic acid tert-butyl ester (Compound No. 1-257)

1.24 g (96%) of the title compound was obtained from N-tert-butyloxycarbonyl-N'-(4-aminophenyl)-piperazine (832 mg) and 5-fluoro-2-methylphenyl isocyanate (0.50 mL) as a purple powder in the same manner as in Example 1.
$^1$H-NMR spectrum (400MHz,DMSO-d6):δ(ppm)=8.93(1H, s), 7.91(1H, s), 7.84(1H, dd, J=12.1 and 2.8Hz), 7.31 (2H, d, J=9.0Hz), 7.15(1H, dd, J=7.7 and 7.7Hz), 6.90(2H, d, J=9.4Hz), 6.70(1H, ddd, J=8.4, 8.4 and 2.5Hz), 3.45(4H, t, J=5.0Hz), 3.00(4H, t, J=5.0Hz), 2.20(3H, s), 1.42(9H, s).
MS(FAB) m/z:429 $(M + H)^+$.
Melting point: 206-208˚C.

(Example 37) 4-{4-[3-(2-fluoro-5-methyl-phenyl)-ureido]-phenyl}-piperazine-1-carboxylic acid tert-butyl ester (Compound No. 1-258)

1.23 g (96%) of the title compound was obtained from N-tert-butyloxycarbonyl-N'-(4-aminophenyl)-piperazine (832 mg) and 2-fluoro-5-methylphenyl isocyanate (0.51 mL) as a pale pink powder in the same manner as in Example 1.
$^1$H-NMR spectrum (400MHz,DMSO-d6):δ(ppm)=8.80(1H, s), 8.34(1H, d, J=2.8Hz), 7.97(1H, d, J=7.8Hz), 7.29(2H, d, J=8.9Hz), 7.07(1H, dd, J=11.5 and 8.4Hz), 6.89(2H, d, J=9.0Hz), 6.77-6.73(1H, m), 3.44(4H, t, J=4.7Hz), 3.00(4H, t, J=4.9Hz), 2.26(3H, s), 1.42(9H, s).
MS(FAB) m/z:429 $(M + H)^+$.
Melting point: 211-214˚C.

(Example 38) 4- {5-[3-(2-methoxy-5-methyl-phenyl)-ureido]-pyridin-2-yl}-piperazine-1-carboxylic acid tert-butyl ester (Compound No. 1-222) (38a) 4-(5-nitro-pyridin-2-yl)-piperazine-1-carboxylic acid tert-butyl ester

A suspension of 2-chloro-5-nitropyridine (7.90 g), piperazine-1-carboxylic acid tert-butyl ester (11.2 g) and potassium carbonate (6.90 g) in acetonitrile (250 mL) was heated under reflux for four hours. The reaction mixture was concentrated and diluted with ethyl acetate and washed with water and saturated brine and dried over sodium sulfate and filtered and concentrated. The residue was vigorously stirred in ethyl acetate/isopropyl ether, collected by filtration and dried under reduced pressure, and 16.1 g (87%) of the title compound was obtained as a yellow solid.
MS(FAB) m/z:309 $(M + H)^+$.
(38b) 4-(5-amino-pyridin-2-yl)-piperazine-1-carboxylic acid tert-butyl ester
A suspension of 4-(5-nitro-pyridin-2-yl)-piperazine-1-carboxylic acid tert-butyl ester (10.8 g) obtained in Example (38a) and 10% palladium-carbon catalyst (2.15 g) in ethanol (300 mL) was stirred at room temperature under a hydrogen atmosphere for five hours. The reaction mixture was filtered and concentrated. The residue was vigorously stirred in isopropyl ether, collected by filtration and dried under reduced pressure, and 6.59 g (68%) of the title compound was obtained as a pale pink solid.
MS(FAB) m/z:279 $(M + H)^+$.

(38c) 4-{5-[3-(2-methoxy-5-methyl-phenyl)-ureido]-pyridin-2-yl}-piperazine-1-carboxylic acid tert-butyl ester

A solution of 4-(5-amino-pyridin-2-yl)-piperazine-1-carboxylic acid tert-butyl ester (5.57 g) obtained in Example (38b) and 2-methoxy-5-methylphenyl isocyanate (3.52 mL) in anhydrous tetrahydrofuran (100 mL) was stirred at room temperature for three days. Methanol was added to the reaction mixture which was then concentrated. The residue was purified by column chromatography (dichloromethane/ethyl acetate 5:3 → 1:1), and 5.42 g (61 %) of the title compound was obtained as a pale pink solid.
$^1$H-NMR spectrum (400MHz,$CDCl_3$):δ(ppm)=9.07(1H, s), 8.13(1H, d, J=2.7Hz), 8.09(1H, s), 7.93(1H, d, J=1.9Hz), 7.72 (1H, dd, J=9.0 and 2.7Hz), 6.86(1H, d, J=8.2Hz), 6.82(1H, d, J=9.0Hz), 6.71(1H, dd, J=8.2 and 1.9Hz), 3.82(3H, s), 3.45-3.28(8H, m), 2.21 (3H, s), 1.42(9H, s).
MS(FAB) m/z:442 $(M + H)^+$.
Melting point: 185-186˚C.

(Example 39) 4-{5-[3-(2-ethoxy-phenyl)-ureido]-pyridin-2-yl}-piperazine-1-carboxylic acid tert-butyl ester (Compound No. 1-224)

1.66 g (75%) of the title compound was obtained from 4-(5-amino-pyridin-2-yl)-piperazine-1-carboxylic acid tert-butyl ester (1.39 g) obtained in Example (38b) and 2-ethoxyphenyl isocyanate (0.89 mL) as a pale pink solid in the same manner as in Example (38c).
$^1$H-NMR spectrum (400MHz,DMSO-d6):δ(ppm)=9.18(1H, s), 8.14(1H, d, J=1.9Hz), 8.08(1H, d, J=8.6Hz), 8.01(1H, s), 7.73(1H, dd, J=9.0 and 1.9Hz), 6.97(1H, d, J=8.6Hz), 6.89(1H, dd, J=8.6 and 8.6Hz), 6.83(2H, dd, J=9.0 and 9.0Hz), 4.11(2H, q, J=7.1Hz), 3.45-3.35(8H, m), 1.42(9H, s), 1.41(3H, t, J=7.1Hz).
MS(FAB) m/z:442 $(M + H)^+$.
Melting point: 162˚C.

(Example 40) 4-{5-[3-(5-chloro-2-methoxy-phenyl)-ureido]-pyridin-2-yl}-piperazine-1-carboxylic acid tert-butyl ester (Compound No. 1-220)

1.34 g (58%) of the title compound was obtained from 4-(5-amino-pyridin-2-yl)-piperazine-1-carboxylic acid tert-butyl ester (1.39 g) obtained in Example (38b) and 5-chloro-2-methoxyphenyl isocyanate (1.10 g) as a pale pink solid in the same manner as in Example (38c).
$^1$H-NMR spectrum (400MHz,DMSO-d6):δ(ppm)=9.15(1H, s), 8.33(1H, s), 8.19(1H, d,J=2.7Hz),8.13(1H,d,J=2.7Hz),7.71 (1H,dd,J=9.0and2.7Hz),7.01(1H,d, J=8.6Hz), 6.95(1H, dd, J=8.6 and 2.7Hz), 6.83(1H, d, J=9.OHz), 3.87(3H, s), 3.47-3.35(8H, m), 1.42(9H, s).
MS(ES) m/z:462 $(M + H)^+$.
Melting point: 155-157˚C.

(Example 41) 4-{4-[3-(2-ethoxy-5-methyl-phenyl)-ureido]-phenyl}-piperazine-1-carboxylic acid tert-butyl ester (Compound No. 1-216)

(41 a) 1-ethoxy-4-methyl-2-nitro-benzene

A suspension of 4-methyl-2-nitrophenol (3.06 g), sodium hydride (1.05 g) and iodoethane (2.08 mL) in dimethylacetamide (40 mL) was stirred at room temperature for 15 hours. The reaction mixture was diluted with ethyl acetate and washed with a saturated sodium hydrogen carbonate aqueous solution and saturated brine and dried over sodium sulfate and filtered and concentrated. The residue was purified by column chromatography (hexane/ethyl acetate 1:0 → 10:1) and 2.71 g (75%) of the title compound was obtained as a yellow oil.

(41b) 2-ethoxy-5-methylaniline

A suspension of 1-ethoxy-4-methyl-2-nitro-benzene (2.71 g) obtained in Example (41a) and a 10% palladium-carbon catalyst (0.27 g) in anhydrous tetrahydrofuran (40 mL) was stirred at room temperature under a hydrogen atmosphere for 25.5 hours. The reaction mixture was filtered through a cellulose powder, concentrated and dried under reduced pressure, and 2.26 g (100%) of the title compound was obtained as a dark brown solid.

(41 c) 4- {4-[3-(2-ethoxy-5-methyl-phenyl)-ureido]-phenyl} -piperazine-1-carboxylic acid tert-butyl ester

A solution of N-tert-butyloxycarbonyl-N'-(4-aminophenyl)-piperazine (555 mg) and diisopropylethylamine (0.77

mL) in anhydrous dichloromethane (5 mL) was added dropwise slowly to a solution of triphosgene (237 mg) in anhydrous dichloromethane (10 mL) at room temperature. After the dropwise addition was completed, a solution of 2-ethoxy-5-methylaniline (302 mg) obtained in Example (41b) and diisopropylethylamine (0.77 mL) in anhydrous dichloromethane (5 mL) was added dropwise slowly to the reaction mixture at room temperature. After 20 minutes, the reaction mixture was concentrated and diluted with ethyl acetate, washed with water and saturated brine, dried over sodium sulfate and filtered and concentrated. The residue was purified by column chromatography (dichloromethane/ethyl acetate 10:1 → 2:1), and the obtained solid was vigorously stirred in hexane and isopropyl ether, collected by filtration and dried under reduced pressure, and 691 mg (75%) of the title compound was obtained as a white powder. $^1$H-NMR spectrum (400MHz, DMSO-d6):δ(ppm)=9.12(1H, s), 7.95(1H, d, J=2.0Hz), 7.89(1H, s), 7.31 (2H, d, J=9.0Hz), 6.89(2H, dd, J=6.8 and 2.2Hz), 6.84(1H, d, J=8.3Hz), 6.68(1H, dd, J=8.2 and 1.5Hz), 4.07(2H, q, J=6.8Hz), 3.44(4H, t, J=4.9Hz), 2.99(4H, t, J=5.1Hz), 2.21(3H, s), 1.42(9H, s), 1.38(3H, t, J=7.0Hz).
MS(FAB) m/z:455 $(M + H)^+$.
Melting point: 105-107˚C.

(Example 42) 4-{4-[3-(2-ethoxy-5-fluoro-phenyl)-ureido]-phenyl}-piperazine-1-carboxylic acid tert-butyl ester (Compound No. 1-212)

(42a) 2-ethoxy-5-fluorobenzoic acid

A suspension of 5-fluorosalicylic acid (2.96 g), potassium carbonate (7.90 g) and iodoethane (4.6 mL) in dimethylacetamide (20 mL) was heated at 80˚C for 17 hours. The reaction mixture was diluted with ethyl acetate, washed with water and saturated brine, dried over sodium sulfate and concentrated and dried under reduced pressure to obtain 4.62 g of a yellow oil. This yellow oil (4.62 g) was heated under reflux in 1N sodium hydroxide (30 mL) aqueous solution and tetrahydrofuran (90 mL) for 3.5 hours. The organic solvent was removed under reduced pressure and 1N hydrochloric acid (40 mL) was added to the mixture. The deposited precipitate was collected by filtration, recrystallized from isopropyl ether/hexane again and 1.79 g (51 %) of the title compound was obtained as a white solid.

(42b) 4- {4-[3-(2-ethoxy-5-fluoro-phenyl)-ureido]-phenyl} -piperazine- I -carboxylic acid tert-butyl ester

A solution of 2-ethoxy-5-fluorobenzoic acid (1.03 g) obtained in Example (42a), diphenyl phosphoric acid azide (2.32 g) and triethylamine (1.6 mL) in anhydrous tetrahydrofuran (30 mL) was heated under reflux for 1.5 hours. After addition of N-tert-butyloxycarbonyl-N'-(4-aminophenyl)-piperazine (1.74 g), the reaction mixture was further heated under reflux for four hours, diluted with ethyl acetate and washed with water, a saturated sodium hydrogen carbonate aqueous solution and saturated brine and dried over sodium sulfate and filtered and concentrated. The residue was purified by column chromatography (dichloromethane/ethyl acetate 2:1), and the obtained solid was vigorously stirred in isopropyl ether, collected by filtration and dried under reduced pressure, and 2.29 g of the title compound was obtained as a white solid.
$^1$H-NMR spectrum (400MHz,DMSO-d6):δ(ppm)=9.26(1H, s), 8.14(1H, s), 8.00(1H, dd, J=11.8 and 3.2Hz), 7.31(2H, d, J=9.0Hz), 6.97(1H, dd, J=8.8 and 5.3Hz), 6.91 (2H, d, J=8.6Hz), 6.68(1H, ddd, J=8.4, 8.4, 3.2 Hz), 4.10(2H, q, J=6.9Hz), 3.45(4H, t, J=4.5Hz), 3.01(4H, t, J=4.9Hz), 1.42(9H, s), 1.40(3H, t, J=8.6Hz).
MS(FAB) m/z:459 $(M + H)^+$.
Melting point: 168-169˚C.

(Example 43) 4-{4-[3-(5-chloro-2-ethoxy-phenyl)-ureido]-phenyl}-piperazine-1-carboxylic acid tert-butyl ester (Compound No. 1-214)

(43a) 5-chloro-2-ethoxy benzoic acid

3.26 g (83%) of the title compound was obtained from 5-chlorosalicylic acid (3.03 g) as a white solid in the same manner as in Example (42a).

(43b) 4-{4-[3-(5-chloro-2-ethoxy-phenyl)-ureido]-phenyl}-piperazine-1-carboxylic acid tert-butyl ester

0.948 g (80%) of the title compound was obtained from 5-chloro-2-ethoxy benzoic acid (0.503 g) obtained in Example (43a) and N-tert-butyloxycarbonyl-N'-(4-aminophenyl)-piperazine (0.766 g) as a white solid in the same manner as in Example (42b).
$^1$H-NMR spectrum (400MHz,DMSO-d6):δ(ppm)=9.28(1H, s), 8.24(1H, d, J=2.3Hz), 8.13(1H, s), 7.33(2H, d, J=9.0Hz), 7.01(2H, d, J=8.6Hz), 6.95-6.92(1H, m), 6.93(1H, d, J=9.0Hz), 4.14(2H, q, J=7.1Hz), 3.46(4H, t, J=4.6Hz), 3.01(4H, t,

J=4.9Hz), 1.43(9H, s), 1.41(3H, t, J=7.1Hz).
MS(FAB) m/z:475 $(M + H)^+$.
Melting point: 165-167˚C.

(Example 44) 4-{4-[3-(5-fluoro-2-methoxy-phenyl)-ureido]-phenyl}-piperazine-1-carboxylic acid tert-butyl ester (Compound No. 1-211)

0.971 g (72%) of the title compound was obtained from 5-fluoro-2-methoxybenzoic acid (0.516 g) and N-tert-butyloxycarbonyl-N'-(4-aminophenyl)-piperazine (0.935 g) as a white solid in the same manner as in Example (42b).
$^1$H-NMR spectrum (400MHz,DMSO-d6):δ(ppm)=9.16(1H, s), 8.30(1H, s), 8.00(1H, dd, J=11.3 and 3.1Hz), 7.29(2H, d, J=9.0Hz), 6.97(1H, dd, J=9.0 and 5.0Hz), 6.90(2H, d, J=9.OHz), 6.70(1H, ddd, J=8.2, 8.2, 3.1Hz), 3.86(3H, s), 3.44(4H, t, J=4.7Hz), 3.00(4H, t, J==5.1Hz), 1.42(9H, s).
MS(FAB) m/z:445 $(M + H)^+$.
Melting point: 200-202˚C.

(Example 45) 4-{5-[3-(5-fluoro-2-methoxy-phenyl)-ureido]-pyridin-2-yl}-piperazine-1-carboxylic acid tert-butyl ester (Compound No. 1-218)

1.52 g (68%) of the title compound was obtained from 5-fluoro-2-methoxybenzoic acid (0.851 g) and 4-(5-amino-pyridin-2-yl)-piperazine-1-carboxylic acid tert-butyl ester (1.39 g) obtained in Example (38b) as a pale lavender solid in the same manner as in Example 42.
$^1$H-NMR spectrum (400MHz,DMSO-d6):δ(ppm)=9.17(1H, s), 8.35(1H, s), 8.13(1H, d, J=2.8Hz), 7.98(1H, dd, J=11.5 and 3.0Hz), 7.72(1H, dd, J=9.0 and 2.7Hz), 6.98(1H, dd, J=8.8 and 5.5Hz), 6.83(1H, d, J=9.0Hz), 6.71(1H, d d d , J=8.8, 8.8 and 3.0Hz), 3.86(3H, s), 3.44-3.35(8H, m), 1.42(9H, s).
MS(FAB) m/z:446 $(M + H)^+$.
Melting point: 200-201˚C.

(Example 46) 4-{5-[3-(2-ethoxy-5-fluoro-phenyl)-ureido]-pyridin-2-yl}-piperazine-1-carboxylic acid tert-butyl ester (Compound No. 1-219)

0.91 g (46%) of the title compound was obtained from 5-fluoro-2-ethoxybenzoic acid (0.800 g) and 4-(5-amino-pyridin-2-yl)-piperazine-1-carboxylic acid tert-butyl ester (1.21 g) obtained in Example (38b) as a lavender solid in the same manner as in Example 42.
$^1$H-NMR spectrum (400MHz,DMSO-d6):δ(ppm)=9.31(1H, s), 8.22(1H, s), 8.17(1H, d, J=2.7Hz), 8.02(1H, dd, J=11.7 and 3.1Hz), 7.76(1H, dd, J=9.0 and 2.7Hz), 7.01(1H, dd, J=9.0 and 5.1Hz), 6.87(1H, d, J=9.0Hz), 6.72(1H, ddd, J=9.0, 8.6 and 3.1Hz), 4.12(2H, q, J=6.9Hz), 3.47-3.35(8H, m), 1.43(9H, s), 1.41(3H, t, J=6.9Hz).
MS(FAB) m/z:460 $(M + H)^+$.
Melting point: 180˚C.

(Example 47) 4-{4-[3-(2-methoxy-5-methyl-phenyl)-ureido]-phenyl}-piperazine-1-carboxylic acid phenyl ester (Compound No. 1-259)

(47a) 1-(2-methoxy-5-methyl-phenyl)-3-(4-piperazin-1-yl-phenyl)-urea

Trifluoroacetic acid (20 mL) and anisole (two or three drops) were added to a solution of 4-{4-[3-(2-methoxy-5-methyl-phenyl)-ureido]-phenyl}-piperazine-1-carboxylic acid tert-butyl ester (7.10 g) obtained in Example 2 in anhydrous dichloromethane (80 mL) at room temperature. After one hour, the reaction mixture was concentrated and was neutralized with a saturated sodium hydrogen carbonate aqueous solution and stirred in a mixture of isopropyl ether/water. The deposited solid was collected by filtration and dried under reduced pressure and 5.28 g (96%) of the title compound was obtained as a pale pink solid.
MS(FAB) m/z:341 $(M + H)^+$.
Melting point: 140˚C.

(47b) 4-{4-[3-(2-methoxy-5-methyl-phenyl)-ureido]-phenyl}-piperazine-1-carboxylic acid phenyl ester

Phenyl chlorocarbonate (0.069 mL) was added to a solution of 1-(2-methoxy-5-methyl-phenyl)-3-(4-piperazin-1-yl-phenyl)-urea (170 mg) obtained in Example (47a) in dimethylacetamide (5 mL) at room temperature. After four hours, the reaction mixture was poured into a saturated sodium hydrogen carbonate solution and stirred at room temperature. The deposited solid was collected by filtration, washed with water and isopropyl ether, dried under reduced pressure and 223 mg (97%) of the title compound was obtained as a light grey powder.
$^1$H-NMR spectrum (400MHz,DMSO-d6):δ(ppm)=9.11(1H, s), 8.08(1H, s), 7.99(1H, d, J=1.5Hz), 7.41(2H, dd, J=7.7 and 7.7Hz), 7.35(2H, dd, J=6.0 and 2.9Hz), 7.24(1H, dd, J=7.4 and 7.4Hz), 7.16(2H, d, J=8.6Hz), 6.95(2H, d, J=9.0Hz), 6.88 (1H, d, J=8.2Hz), 6.73(1H, d, J=7.8Hz), 3.84(3H, s), 3.84-3.78(2H, m), 3.69-3.63(2H, m), 3.17-3.08(4H, m), 2.23(3H, s).
MS(FAB) m/z: 461 (M + H)+.
Melting point: 179˚C.

(Example 48) 1-[4-(4-cyclohexane carbonyl-piperazin-1-yl)-phenyl]-3-(2-methoxy-5-methyl-phenyl)-urea (Compound No. 1-260)

Cyclohexane carbonyl chloride (0.074 mL) was added to a solution of 1-(2-methoxy-5-methyl-phenyl)-3-(4-piperazin-1-yl-phenyl)-urea (170 mg) obtained in Example (47a) in dimethylacetamide (5 mL) at room temperature. After two hours, the reaction mixture was poured into a saturated sodium hydrogen carbonate solution, followed by addition of isopropyl ether. The deposited solid was collected by filtration, washed with water and isopropyl ether, dried under reduced pressure and 200 mg (94%) of the title compound was obtained as a pale pink crystal.
$^1$H-NMR spectrum (400MHz,DMSO-d6):δ(ppm)=9.09(1H, s), 8.07(1H, s), 7.98(1H, s), 7.32(2H, d, J=8.6Hz), 6.91(2H, d, J=9.4Hz), 6.88(1H, d, 8.2Hz), 6.73(1H, d, J=8.6Hz), 3.83(3H, s), 3.65-3.56(4H, m), 3.07-2.96(4H, m), 2.66-2.57(1H, m), 2.22(3H, s), 1.74-1.61(4H, m), 1.39-1.24(4H, m), 1.21-1.10(2H, m).
MS(FAB) m/z:451 $(M + H)^+$.
Melting point: 222˚C.

(Example 49) 4-{4-[3-(2-methoxy-5-methyl-phenyl)-ureido]-phenyl}-piperazine-1-carboxylic acid phenylamide (Compound No. 1-262)

Phenyl isocyanate (0.082 mL) was added to a solution of 1-(2-methoxy-5-methyl-phenyl)-3-(4-piperazin-1-yl-phenyl)-urea (170 mg) obtained in Example (47a) in anhydrous tetrahydrofuran (5 mL) at room temperature. After 15 hours, methanol was added to the reaction mixture which was then concentrated. The residue was stirred in ether, collected by filtration, dried under reduced pressure and 216 mg (94%) of the title compound was obtained as a pale pink crystal.
$^1$H-NMR spectrum (40OMHz,DMSO-d6):δ(ppm)=9.07(1H, s), 8.59(1H, s), 8.04(1H, s), 7.96(1H, d, J=2.0Hz), 7.46(2H, d, J=7.5Hz), 7.31(2H, d, J=9.0Hz), 7.22(1H, d, J=7.4Hz), 7.20(1H, d, J=8.6Hz), 6.94-6.90(3H, m), 6.86(1H, d, J=8.2Hz), 6.70(1H, dd, J=8.4 and 1.8Hz), 3.82(3H, s), 3.59(4H, t, J=4.9Hz), 3.07(4H, t, J=5.1Hz), 2.22(3H, s).
MS(FAB) m/z:460 $(M + H)^+$.
Melting point: 221˚C.

(Example 50) 4-{4-[3-(2-methoxy-5-methyl-phenyl)-ureido]-phenyl}-piperazine-1-carboxylic acid benzylamide (Compound No. 1-268)

Benzyl isocyanate (0.093 mL) was added to a solution of 1-(2-methoxy-5-methyl-phenyl)-3-(4-piperazin-1-yl-phenyl)-urea (170 mg) obtained in Example (47a) in anhydrous tetrahydrofuran (5 mL) at room temperature. After 15 hours, methanol was added to the reaction mixture which was then concentrated. The residue was stirred in hexane/isopropyl ether (5:1), collected by filtration, washed with ether, and dried under reduced pressure and 205 mg (87%) of the title compound was obtained as a light brown crystal.
$^1$H-NMR spectrum (40OMHz,DMSO-d6):δ(ppm)=9.08(1H, s), 8.06(1H, s), 7.98(1H, d, J=2.0Hz), 7.33-7.19(8H, m), 6.92 (2H, d, J=8.6Hz), 6.88(1H, d, J=8.2Hz), 6.72(1H, dd, J=8.0 and 1.7Hz), 4.25(2H, ABX, J=20 and 5.9Hz), 3.83(3H, s), 3.48(4H, t, J=4.6Hz), 3.02(4H, t, J=4.5Hz), 2.22(3H, s).
MS(FAB) m/z:474 $(M + H)^+$.
Melting point: 224˚C.

(Example 51) 4-{4-[3-(2-methoxy-5-methyl-phenyl)-ureido]-phenyl}-piperazine-1-carboxylic acid amide (Compound No. 1-273)

Trimethylsilyl isocyanate (0.10 mL) was added to a solution of 1-(2-methoxy-5-methyl-phenyl)-3-(4-piperazin-1-yl-phenyl)-urea (170 mg) obtained in Example (47a) in anhydrous tetrahydrofuran (5 mL) at room temperature. After 15.5 hours, methanol was added to the reaction mixture which was then concentrated. The residue was purified by column chromatography (dichloromethane/ethyl acetate 1:1 → dichloromethane/methanol 10:1 → 5:1). The obtained solid was stirred in hexane/isopropyl ether (5:1), collected by filtration and dried under reduced pressure and 133 mg

(69%) of the title compound was obtained as a white crystal.
$^1$H-NMR spectrum (400MHz,DMSO-d6):δ(ppm)=9.06(1H, s), 8.04(1H, s), 7.96(1H, d, J=1.9Hz), 7.29(2H, d, J=9.0Hz), 6.89(2H, d, J=9.0Hz), 6.86(1H, d, J=8.2Hz), 6.70(1H, dd, J=8.2 and 1.6Hz), 6.03(2H, s), 3.82(3H, s), 3.41(4H, t, J=5.1Hz), 2.98(4H, t, J=5.0Hz), 2.22(3H, s).
MS(FAB) m/z:384 (M + H)$^+$.
Melting point: 136˚C.

(Example 52) 4- {4-[3-(2-methoxy-5-methyl-phenyl)-ureido]-phenyl}-piperazine-1-carboxylic acid (2-fluoro-phenyl)-amide (Compound No. 1-275)

76 mg (80%) of the title compound was obtained from 1-(2-methoxy-5-methyl-phenyl)-3-(4-piperazin-1-yl-phenyl)-urea (68 mg) obtained in Example (47a) and 2-fluorophenyl isocyanate (0.034 mL) as a grey white powder in the same manner as in Example 49.
$^1$H-NMR spectrum (400MHz,DMSO-d6):δ(ppm)=9.09(1H, s), 8.41(1H, s), 8.06(1H, s), 7.99(1H, s), 7.48-7.43(1H, m), 7.33(2H, d, J=8.6Hz), 7.23-7.19(1H, m), 7.17-7.11(2H, m), 6.95(2H, d, J=8.6Hz), 6.88(1H, d, J=8.2Hz), 6.73(1H, ddd, J=4.7, 4.7 and 2.7Hz), 3.84(3H, s), 3.59(4H, t, J=4.9Hz), 3.08(4H, t, J=4.7Hz), 2.23(3H, s).
MS(FAB) m/z:478 (M + H)$^+$.
Melting point: 200-201˚C.

(Example 53) 4-{4-[3-(2-methoxy-5-methyl-phenyl)-ureido]-phenyl}-piperazine-1-carboxylic acid (2-trifluoromethyl-phenyl)-amide (Compound No. 1-201)

59 mg (56%) of the title compound was obtained from 1-(2-methoxy-5-methyl-phenyl)-3-(4-piperazin-1-yl-phenyl)-urea (68 mg) obtained in Example (47a) and 2-trifluoromethylphenyl isocyanate (0.045 mL) as a grey white powder in the same manner as in Example 49.
$^1$H-NMR spectrum (400MHz,DMSO-d6):δ(ppm)=9.06(1H, s), 8.32(1H, s), 8.04(1H, s), 7.96(1H, d, J=2.0Hz), 7.67(1H, dd, J=7.6 and 1.0Hz), 7.62(1H, dd, J=7.6 and 7.6Hz), 7.44(1H, d, J=7.8Hz), 7.38(1H, dd, J=7.2 and 7.2Hz), 7.31(2H, d, J=9.0Hz), 6.92(2H, d, J=9.0Hz), 6.86(1H, d, J=8.2Hz), 6.70(1H, dd, J=8.0 and 1.4Hz), 3.82(3H, s), 3.57(4H, t, J=4.9Hz), 3.06(4H, t, J=5.1Hz), 2.22(3H, s).
MS(FAB) m/z:528 (M + H)$^+$.
Melting point: 170-171˚C.

(Example 54) 4-{4-[3-(2-methoxy-5-methyl-phenyl)-ureido]-phenyl}-piperazine-1-carboxylic acid o-tolylamide (Compound No. 1-187)

84 mg (89%) of the title compound was obtained from 1-(2-methoxy-5-methyl-phenyl)-3-(4-piperazin-1-yl-phenyl)-urea (68 mg) obtained in Example (47a) and 2-methylphenyl isocyanate (0.037 mL) as a light brown powder in the same manner as in Example 49.
$^1$H-NMR spectrum (400MHz,DMSO-d6):δ(ppm)=9.06(1H, s), 8.12(1H, s), 8.04(1H, s), 7.96(1H, d, J=1.6Hz), 7.31(2H, d, J=9.0Hz), 7.17(2H, dd, J=6.8 and 6.8Hz), 7.11(1H, ddd, J=7.5, 7.5 and 1.7Hz), 7.03(1H, ddd, J=7.2, 7.2 and 1.5Hz), 6.92(2H, d, J=9.4Hz), 6.86(1H, d, J=8.2Hz), 6.70(1H, dd, J=8.2 and 1.6Hz), 3.82(3H, s), 3.58(4H, t, J=5.1Hz), 3.07(4H, t, J=4.9Hz), 2.22(3H, s), 2.17(3H, s).
MS(FAB) m/z:474 (M + H)$^+$.
Melting point: 209-211˚C.

(Example 55) 4-{4-[3-(2-methoxy-5-methyl-phenyl)-ureido]-phenyl}-piperazine-l-carboxylic acid (2,6-difluoro-phenyl)-amide (Compound No. 1-5)

88 mg (88%) of the title compound was obtained from 1-(2-methoxy-5-methyl-phenyl)-3-(4-piperazin-1-yl-phenyl)-urea (68 mg) obtained in Example (47a) and 2,6-difluorophenyl isocyanate (47 mg) as a light brown powder in the same manner as in Example 49.
$^1$H-NMR spectrum (400MHz,DMSO-d6):δ(ppm)=9.09(1H, s), 8.37(1H, s), 8.06(1H, s), 7.99(1H, s), 7.34(2H, d, J=8.9Hz), 7.28(1H, dd, J=8.4 and 8.4Hz), 7.12(1H, d, J=8.3Hz), 7.10(1H, d, J=8.2Hz), 6.95(2H, d, J=8.6Hz), 6.88(1H, d, J=8.6Hz), 6.73(1H, dd, J=8.6 and 1.2Hz), 3.84(3H, s), 3.60-3.58(4H, m), 3.08-3.07(4H, m), 2.23(3H, s).
MS(FAB) m/z:496 (M + H)$^+$.
Melting point: 224˚C.

(Example 56) 4-{4-[3-(2-methoxy-5-methyl-phenyl)-ureido]-phenyl}-piperazine-1-carboxylic acid (2-chloro-phenyl)-amide (Compound No. 1-281)

75 mg (76%) of the title compound was obtained from 1-(2-methoxy-5-methyl-phenyl)-3-(4-piperazin-1-yl-phenyl)-urea (68 mg) obtained in Example (47a) and 2-chlorophenyl isocyanate (0.36 mL) as a white powder in the same manner as in Example 49.
$^1$H-NMR spectrum (400MHz,DMSO-d6):δ(ppm)=9.09(1H, s), 8.31(1H, s), 8.07(1H, s), 7.99(1H, d, J=1.5Hz), 7.52(1H, dd, J=7.8 and 1.5Hz), 7.46(1H, dd, J=8.0 and 1.4Hz), 7.35-7.28(3H, m), 7.15(1H, ddd, J=7.8, 7.8 and 1.6Hz), 6.95(2H, d, J=9.0Hz), 6.88(1H, d, J=8.2Hz), 6.73(1H, dd, J=8.1 and 1.4Hz), 3.84(3H, s), 3.61(4H, t, J=4.9Hz), 3.09(4H, t, J=4.9Hz), 2.23(3H, s).
MS(FAB) m/z:494 $(M + H)^+$.
Melting point: >240˚C (dec).

(Example 57) 4-{4-[3-(2-methoxy-5-methyl-phenyl)-ureido]-phenyl}-piperazine-1-carboxylic acid (2-bromo-phenyl)-amide (Compound No. 1-287)

90 mg (84%) of the title compound was obtained from 1-(2-methoxy-5-methyl-phenyl)-3-(4-piperazin-1-yl-phenyl)-urea (68 mg) obtained in Example (47a) and 2-bromophenyl isocyanate (0.37 mL) as a white powder in the same manner as in Example 49.
$^1$H-NMR spectrum (400MHz,DMSO-d6):δ(ppm)=9.06(1H, s), 8.26(1H, s), 8.04(1H, s), 7.96(1H, d, J=2Hz), 7.60(1H, d, J=8.6Hz), 7.48(1H, d, J=7.9Hz), 7.34-7.30(3H, m), 7.06(1H, dd, J=7.5 and 7.5Hz), 6.92(2H, d, J=8.7Hz), 6.86(1H, d, J=7.8Hz), 6.70(1H, dd, J=8.4 and 1.8Hz), 3.82(3H, s), 3.60(4H, t, J=4.3Hz), 3.08(4H, t, J=4.7Hz), 2.22(3H, s).
MS(FAB) m/z:538 $(M + H)^+$.
Melting point: 212-214˚C.

(Example 58) 4-{4-[3-(2-methoxy-5-methyl-phenyl)-ureido]-phenyl}-piperazine-1-carboxylic acid (2-ethyl-phenyl)-amide (Compound No. 1-293)

81 mg (83%) of the title compound was obtained from 1-(2-methoxy-5-methyl-phenyl)-3-(4-piperazin-1-yl-phenyl)-urea (68 mg) obtained in Example (47a) and 2-ethylphenyl isocyanate (0.42 mL) as a white powder in the same manner as in Example 49.
$^1$H-NMR spectrum (400MHz,DMSO-d6):δ(ppm)=9.05(1H, s), 8.10(1H, s), 8.04(1H, s), 7.96(1H, s), 7.31(2H, d, J=8.6Hz), 7.20-7.10(4H, m), 6.92(2H, d, J=9.0Hz), 6.86(1H, d, J=8.2Hz), 6.70(1H, d, J=7.8Hz), 3.82(3H, s), 3.58(4H, brs), 3.06 (4H, brs), 2.56(2H, q, J=7.6Hz), 2.22(3H, s), 1.12(3H, t, J=7.4Hz).
MS(FAB) m/z:488 $(M + H)^+$.
Melting point: 220-221˚C.

(Example 59)4-{4-[3-(2-methoxy-5-methyl-phenyl)-ureido]-phenyl}-piperazine-1-carboxylic acid (2-chloro-6-methyl-phenyl)-amide (Compound No. 1-47)

76 mg (75%) of the title compound was obtained from 1-(2-methoxy-5-methyl-phenyl)-3-(4-piperazin-1-yl-phenyl)-urea (68 mg) obtained in Example (47a) and 2-chloro-6-methylphenyl isocyanate (0.38 mL) as a white powder in the same manner as in Example 49.
$^1$H-NMR spectrum (400MHz,DMSO-d6):δ(ppm)=9.06(1H, s), 8.21(1H, s), 8.04(1H, s), 7.96(1H, d, J=2.0Hz), 7.31(2H, d, J=9.4Hz), 7.30(1H, d, J=9.3Hz), 7.21-7.12(2H, m), 6.93(2H, dd, J=6.8 and 2.2Hz), 6.86(1H, d, J=8.2Hz), 6.70(1H, dd, J=8.2 and 1.6Hz), 3.82(3H, s), 3.60(4H, t, J=4.9Hz), 3.07(4H, t, J=4.9Hz), 2.22(3H, s), 2.20(3H, s).
MS(FAB) m/z:508 $(M + H)^+$.
Melting point: 143-145˚C.

(Example 60) 4-{4-[3-(2-methoxy-5-methyl-phenyl)-ureido]-phenyl}-piperazine-1-carboxylic acid (2-methyl-6-nitro-phenyl)-amide (Compound No. 1-299)

66 mg (63%) of the title compound was obtained from 1-(2-methoxy-5-methyl-phenyl)-3-(4-piperazin-1-yl-phenyl)-urea (68 mg) obtained in Example (47a) and 2-methyl-6-nitrophenyl isocyanate (53 mg) as a white powder in the same manner as in Example 49.
$^1$H-NMR spectrum (400MHz,DMSO-d6):δ(ppm)=9.06(1H, s), 8.43(1H, s), 8.04(1H, s), 7.96(1H, d, J=2.0Hz), 7.71(1H, d, J=9.0Hz), 7.56(1H, d, J=7.4Hz), 7.32-7.28(3H, m), 6.93(2H, dd, J=6.8 and 2.2Hz), 6.86(1H, d, J=8.2Hz), 6.70(1H, dd, J=8.4 and 1.8Hz), 3.82(3H, s), 3.59(4H, t, J=4.5Hz), 3.07(4H, t, J=4.9Hz), 2.29(3H, s), 2.22(3H, s).

MS(FAB) m/z:519 $(M + H)^+$.
Melting point: 213-215°C.

(Example 61) 4-{4-[3-(2-methoxy-5-methyl-phenyl)-ureido]-phenyl}-piperazine-1-carboxylic acid (2-cyano-phenyl)-amide (Compound No. 1-305)

85 mg (88%) of the title compound was obtained from 1-(2-methoxy-5-methyl-phenyl)-3-(4-piperazin-1-yl-phenyl)-urea (68 mg) obtained in Example (47a) and 2-cyanophenyl isocyanate (43 mg) as a yellow powder in the same manner as in Example 49.
$^1$H-NMR spectrum (400MHz,DMSO-d6):δ(ppm)=9.06(1H, s), 8.97(1H, brs), 8.04(1H, s), 7.96(1H, d, J=1.6Hz), 7.72(1H, dd, J=7.8 and 1.6Hz), 7.61(1H, ddd, J=7.9, 7.9 and 1.2Hz), 7.41(1H, d, J=8.2Hz), 7.31(2H, d, J=9.0Hz), 7.24(1H, dd, J=7.8 and 7.8Hz), 6.93(2H, d, J=9.0Hz), 6.86(1H, d, J=8.2Hz), 6.70(1H, dd, J=8.2 and 1.6Hz), 3.82(3H, s), 3.62(4H, t, J=4.9Hz), 3.09(4H, t, J=4.9Hz), 2.22(3H, s).
MS(FAB) m/z:485 $(M + H)^+$.
Melting point: 210-212°C.

(Example 62) 4-{4-[3-(2-methoxy-5-methyl-phenyl)-ureido]-phenyl}-piperazine-1-carboxylic acid (2,6-dimethyl-phenyl)-amide (Compound No. 1-61)

81 mg (83%) of the title compound was obtained from 1-(2-methoxy-5-methyl-phenyl)-3-(4-piperazin-1-yl-phenyl)-urea (68 mg) obtained in Example (47a) and 2,6-dimethylphenyl isocyanate (0.042 mL) as a yellow powder in the same manner as in Example 49.
$^1$H-NMR spectrum (400MHz,DMSO-d6):δ(ppm)=9.06(1H, s), 8.04(1H, s), 7.96(1H, d, J=2.0Hz), 7.94(1H, s), 7.31(2H, d, J=9.0Hz), 7.05-7.02(3H, m), 6.93(2H, d, J=9.4Hz), 6.86(1H, d, J=8.2Hz), 6.70(1H, dd, J=8.0 and 1.4Hz), 3.82(3H, s), 3.59(4H, t, J=4.7Hz), 3.06(4H, t, J=4.9Hz), 2.22(3H, s), 2.14(6H, s).
MS(FAB) m/z:488 $(M + H)^+$.
Melting point: 212-214°C.

(Example 63) 4-{4-[3-(2-methoxy-5-methyl-phenyl)-ureido]-phenyl}-piperazine-1-carboxylic acid (2-ethyl-6-methyl-phenyl)-amide (Compound No. 1-311)

89 mg (89%) of the title compound was obtained from 1-(2-methoxy-5-methyl-phenyl)-3-(4-piperazin-1-yl-phenyl)-urea (68 mg) obtained in Example (47a) and 2-ethyl-6-methylphenyl isocyanate (0.047 mL) as a light brown powder in the same manner as in Example 49.
$^1$H-NMR spectrum (400MHz,DMSO-d6):δ(ppm)=9.06(1H, s), 8.04(1H, s), 7.96(1H, d, J=2.4Hz), 7.93(1H, s), 7.31(2H, d, J=9.0Hz), 7.09-7.03(3H, m), 6.93(2H, d, J=9.0Hz), 6.86(1H, d, J=8.2Hz), 6.71(1H, dd, J=8.6 and 1.6Hz), 3.82(3H, s), 3.59(4H, t, J=4.9Hz), 3.06(4H, t, J=4.7Hz), 2.55-2.50(2H, m), 2.22(3H, s), 2.14(3H, s), 1.10(3H, t, J=7.7Hz).
MS(FAB) m/z:502 $(M + H)^+$.
Melting point: 207-209°C.

(Example 64) 4-{4-[3-(2-methoxy-5-methyl-phenyl)-ureido]-phenyl}-piperazine-1-carboxylic acid (2-fluoro-6-trifluoromethyl-phenyl)-amide (Compound No. 1-19)

76 mg (70%) of the title compound was obtained from 1-(2-methoxy-5-methyl-phenyl)-3-(4-piperazin-1-yl-phenyl)-urea (68 mg) obtained in Example (47a) and 2-fluoro-6-trifluoromethylphenyl isocyanate (0.043 mL) as a yellow powder in the same manner as in Example 49.
$^1$H-NMR spectrum (400MHz,DMSO-d6):δ(ppm)=9.08(1H, s), 8.34(1H, s), 8.06(1H, s), 7.99(1H, d, J=1.5Hz), 7.65-7.54 (3H, m), 7.34(2H, d, J=9.0Hz), 6.95(2H, d, J=9.0Hz), 6.89(1H, d, J=7.8Hz), 6.73(1H, dd, J=8.0 and 1.0Hz), 3.84(3H, s), 3.59(4H, t, J=4.7Hz), 3.06(4H, t, J=4.7Hz), 2.23(3H, s).
MS(FAB) m/z:546 $(M + H)^+$.
Melting point: 134-136°C.

(Example 65) 4-{4-[3-(2-methoxy-5-methyl-phenyl)-ureido]-phenyl}-piperazine-1-carboxylic acid (2,6-dichloro-phenyl)-amide (Compound No. 1-33)

93 mg (88%) of the title compound was obtained from 1-(2-methoxy-5-methyl-phenyl)-3-(4-piperazin-1-yl-phenyl)-urea (68 mg) obtained in Example (47a) and 2,6-dichlorophenyl isocyanate (56 mg) as a white powder in the same manner as in Example 49.

$^1$H-NMR spectrum (400MHz,DMSO-d6):δ(ppm)=9.08(1H, s), 8.52(1H, s), 8.06(1H, s), 7.99(1H, d, J=1.1Hz), 7.52(2H, d, J=8.2Hz), 7.32(2H, d, J=9.0Hz), 7.28(1H, d, J=8.2Hz), 6.95(2H, d, J=9.0Hz), 6.88(1H, d, J=8.2Hz), 6.73(1H, dd, J=8.0 and 1.8Hz), 3.84(3H, s), 3.61(4H, t, J=4.3Hz), 3.08(4H, t, J=4.3Hz), 2.23(3H, s).
MS(FAB) m/z:528 $(M + H)^+$.
Melting point: 210-211˚C.

(Example 66) 4- {4-[3-(2-methoxy-5-methyl-phenyl)-ureido]-phenyl}-piperazine-1 - carboxylic acid (2-chloro-6-trifluoromethyl-phenyl)-amide (Compound No. 1-317)

91 mg (81%) of the title compound was obtained from 1-(2-methoxy-5-methyl-phenyl)-3-(4-piperazin-1-yl-phenyl)-urea (68 mg) obtained in Example (47a) and 2-chloro-6-trifluoromethylphenyl isocyanate (67 mg) as a white powder in the same manner as in Example 49.
$^1$H-NMR spectrum (400MHz,DMSO-d6):δ(ppm)=9.06(1H, s), 8.48(1H, s), 8.04(IH, s), 7.97(1H, d, J=l.6Hz), 7.85(1H, d, J=7.5Hz), 7.71(1H, d, J=6.7Hz), 7.53-7.48(1H, m), 7.31 (2H, d, J=9.OHz), 6.93(2H, d, J=8.6Hz), 6.86(1H, d, J=9.0Hz), 6.71(1H, d, J=8.6Hz), 3.82(3H, s), 3.58(4H, brs), 3.05(4H, brs), 2.21(3H, s).
MS(FAB) m/z:562 $(M + H)^+$.
Melting point: 196-197˚C.

(Example 67) 4-{4-[3-(5-chloro-2-methoxy-phenyl)-ureido]-phenyl}-piperazine-1-carboxylic acid (2-fluoro-phenyl)-amide (Compound No. 1-322)

(67a) 1-(5-chloro-2-methoxy-phenyl)-3-(4-piperazin-1-yl-phenyl)-urea

7.01 g (100%) of the title compound was obtained from 4-{4-[3-(5-chloro-2-methoxyphenyl)-ureido]-phenyl}-piperazine-1-carboxylic acid tert-butyl ester (9.01 g) obtained in Example 1 as a pale pink solid in the same manner as in Example (47a).
MS(FAB) m/z:361 $(M + H)^+$.

(67b) 4-{4-[3-(5-chloro-2-methoxy-phenyl)-ureido]-phenyl}-piperazine-1-carboxylic acid (2-fluoro-phenyl)-amide

90 mg (90%) of the title compound was obtained from 1-(5-chloro-2-methoxy-phenyl)-3-(4-piperazin-1-yl-phenyl)-urea (72 mg) obtained in Example (67a) and 2-fluorophenyl isocyanate (0.034 mL) as a light brown powder in the same manner as in Example 49.
$^1$H-NMR spectrum (400MHz,DMSO-d6):δ(ppm)=9.15(1H, s), 8.37(1H, s), 8.27(1H, s), 8.22(1H, d, J=2.4Hz), 7.45-7.41 (1H, m), 7.31(2H, d, J=9.0Hz), 7.20-7.15(1H, m), 7.12-7.09(2H, m), 7.00(1H, d, J=8.6Hz), 6.94(1H, d, J=9.0Hz), 6.94 (2H, d, J=9.0Hz), 3.87(3H, s), 3.58(4H, t, J=4.9Hz), 3.08(4H, t, J=5.1Hz).
MS(FAB) m/z:498 $(M + H)^+$.
Melting point: 218-220˚C.

(Example 68) 4-{4-[3-(5-chloro-2-methoxy-phenyl)-ureido]-phenyl}-piperazine-1-carboxylic acid (2-trifluoromethyl-phenyl)-amide (Compound No. 1-199)

87 mg (79%) of the title compound was obtained from 1-(5-chloro-2-methoxy-phenyl)-3-(4-piperazin-1-yl-phenyl)-urea (72 mg) obtained in Example (67a) and 2-trifluorophenyl isocyanate (0.045 mL) as a dark brown powder in the same manner as in Example 49.
$^1$H-NMR spectrum (400MHz,DMSO-d6):δ(ppm)=9.15(1H, s), 8.31(1H, s), 8.27(1H, s), 8.22(1H, d, J=2.8Hz), 7.67(1H, d, J=7.9Hz), 7.62(1H, dd, J=7.7 and 7.7Hz), 7.43(1H, d, J=7.8Hz), 7.39(1H, dd, J=7.7 and 7.7Hz), 7.31(2H, d, J=9.0Hz), 7.00(1H, d, J=9.0Hz), 6.95-6.92(3H, m), 3.87(3H, s), 3.57(4H, t, J=4.9Hz), 3.07(4H, t, J=4.9Hz).
MS(FAB) m/z:548 $(M + H)^+$.
Melting point: 213-214˚C.

(Example 69) 4-{4-[3-(5-chloro-2-methoxy-phenyl)-ureido]-phenyl}-piperazine-1-carboxylic acid o-tolylamide (Compound No. 1-185)

86 mg (87%) of the title compound was obtained from 1-(5-chloro-2-methoxy-phenyl)-3-(4-piperazin-1-yl-phenyl)-urea (72 mg) obtained in Example (67a) and 2-methylphenyl isocyanate (0.037 mL) as a dark brown powder in the same manner as in Example 49.
$^1$H-NMR spectrum (400MHz,DMSO-d6):δ(ppm)=9.15(1H, s), 8.27(1H, s), 8.22(1H, d, J=2.4Hz), 8.12(1H, s), 7.31(2H,

d, J=9.0Hz), 7.17(2H, dd, J=7.2 and 7.2Hz), 7.11(1H, dd, J=7.5 and 7.5Hz), 7.04(1H, dd, J=7.2 and 1.3Hz), 7.00(1H, d, J=8.6Hz), 6.94(1H, d, J=9.0Hz), 6.94(2H, d, J=8.6Hz), 3.87(3H, s), 3.58(4H, t, J=4.9Hz), 3.08(4H, t, J=4.9Hz), 2.17 (3H, s).
MS(FAB) m/z:494 $(M + H)^+$.
Melting point: 230-232˚C.

(Example 70) 4-{4-[3-(2-methoxy-5-methyl-phenyl)-ureido]-phenyl}-piperazine-1-carboxylic acid (4-bromo-2-methyl-phenyl)-amide (Compound No. 1-324)

103 mg (93%) of the title compound was obtained from 1-(2-methoxy-5-methyl-phenyl)-3-(4-piperazin-1-yl-phenyl)-urea (68 mg) obtained in Example (47a) and 4-bromo-2-methylphenyl isocyanate (64 mg) as a white powder in the same manner as in Example 49.
$^1$H-NMR spectrum (400MHz,DMSO-d6):δ(ppm)=9.05(1H, s), 8.17(1H, s), 8.03(1H, s), 7.96(1H, d, J=1.6Hz), 7.38(1H, d, J=2.7Hz), 7.32-7.28(3H, m), 7.16(1H, d, J=8.6Hz), 6.92(2H, d, J=9.4Hz), 6.86(1H, d, J=8.2Hz), 6.70(1H, dd, J=8.6 and 1.6Hz), 3.82(3H, s), 3.57(4H, t, J=5.1Hz), 3.07(4H, t, J=4.6Hz), 2.22(3H, s), 2.16(3H, s).
MS(FAB) m/z:552 $(M + H)^+$.
Melting point: 191-193˚C.

(Example 71) 4-{4-[3-(2-methoxy-5-methyl-phenyl)-ureido]-phenyl}-piperazine-1-carboxylic acid (2,4-dimethyl-phenyl)-amide (Compound No. 1-330)

83 mg (85%) of the title compound was obtained from 1-(2-methoxy-5-methyl-phenyl)-3-(4-piperazin-1-yl-phenyl)-urea (68 mg) obtained in Example (47a) and 2,4-dimethylphenyl isocyanate (0.042 mL) as a white powder in the same manner as in Example 49.
$^1$H-NMR spectrum (400MHz,DMSO-d6):δ(ppm)=9.05(1H, s), 8.04(2H, brs), 7.96(1H, d, J=2.0Hz), 7.31(2H, d, J=9.0Hz), 7.03(1H, d, J=7.8Hz), 6.97(1H, brs), 6.94-6.90(3H, m), 6.86(1H, d, J=8.2Hz), 6.70(1H, dd, J=7.8 and 2.4Hz), 3.82(3H, s), 3.56(4H, t, J=4.9Hz), 3.06(4H, t, J=4.9Hz), 2.24(3H, s), 2.22(3H, s), 2.12(3H, s).
MS(FAB) m/z:488 $(M + H)^+$.
Melting point: 196-199˚C.

(Example 72) 4-{4-[3-(2-methoxy-5-methyl-phenyl)-ureido]-phenyl}-piperazine-1-carboxylic acid (2,4-difluoro-phenyl)-amide (Compound No. 1-336)

76 mg (77%) of the title compound was obtained from 1-(2-methoxy-5-methyl-phenyl)-3-(4-piperazin-1-yl-phenyl)-urea (68 mg) obtained in Example (47a) and 2,4-difluorophenyl isocyanate (0.036 mL) as an ochre powder in the same manner as in Example 49.
$^1$H-NMR spectrum (400MHz,DMSO-d6):δ(ppm)=9.06(1H, s), 8.39(1H, brs), 8.04(1H, s), 7.96(1H, d, J=1.6Hz), 7.43-7.37 (IH, m), 7.31(2H, d, J=9.0Hz), 7.23,(1H, ddd, J=9.0, 9.0 and 2.7Hz), 7.03-6.97(1H, m), 6.92(2H, d, J=9.4Hz), 6.86(1H, d, J=8.2Hz), 6.70(1H, dd, J=8.2 and 2Hz), 3.82(3H, s), 3.57(4H, t, J=5.0Hz), 3.07(4H, t, J=4.9Hz), 2.22(3H, s).
MS(FAB) m/z:496 $(M + H)^+$.
Melting point: 201-203˚C.

(Example 73) 4-{4-[3-(2-methoxy-5-methyl-phenyl)-ureido]-phenyl}-piperazine-1-carboxylic acid (4-methoxy-2-methyl-phenyl)-amide (Compound No. 1-145)

96 mg (95%) of the title compound was obtained from 1-(2-methoxy-5-methyl-phenyl)-3-(4-piperazin-1-yl-phenyl)-urea (68 mg) obtained in Example (47a) and 4-methoxy-2-methylphenyl isocyanate (0.044 mL) as an ochre powder in the same manner as in Example 49.
$^1$H-NMR spectrum (400MHz,DMSO-d6):δ(ppm)=9.06(1H, s), 8.04(1H, s), 8.01(1H, s), 7.96(1H, d, J=2.0Hz), 7.31(2H, d, J=9.0Hz), 7.03(1H, d, J=8.6Hz), 6.92(2H, d, J=9.0Hz), 6.86(1H, d, J=8.2Hz), 6.75(1H, d, J=3.1Hz), 6.69(2H, ddd, J=7.8, 7.8 and 2.4Hz), 3.82(3H, s), 3.71(3H, s), 3.55(4H, t, J=4.9Hz), 3.06(4H, t, J=4.7Hz), 2.22(3H, s), 2.13(3H, s).
MS(FAB) m/z:504 $(M + H)^+$.
Melting point: 193-195˚C.

(Example 74) 4-{4-[3-(2-methoxy-5-methyl-phenyl)-ureido]-phenyl}-piperazine-1-carboxylic acid (4-chloro-2-methyl-phenyl)-amide (Compound No. 1-342)

93 mg (92%) of the title compound was obtained from 1-(2-methoxy-5-methyl-phenyl)-3-(4-piperazin-1-yl-phenyl)-urea (68 mg) obtained in Example (47a) and 4-chloro-2-methylphenyl isocyanate (50 mg) as a light brown powder in the same manner as in Example 49.
$^1$H-NMR spectrum (400MHz,DMSO-d6):δ(ppm)=9.06(1H, s), 8.18(1H, s), 8.04(1H, s), 7.96(1H, d, J=2.0Hz), 7.31(2H, d, J=9.0Hz), 7.25(1H, d, J=2.3Hz), 7.21(1H, d, J=8.7Hz), 7.15(1H, dd, J=8.5 and 2.5Hz), 6.92(2H, d, J=9.0Hz), 6.86 (1H, d, J=8.2Hz), 6.70(1H, dd, J=8.0 and 1.4Hz), 3.82(3H, s), 3.57(4H, t, J=4.9Hz), 3.07(4H, t, J=4.9Hz), 2.22(3H, s), 2.17(3H, s).
MS(FAB) m/z:508 $(M + H)^+$.
Melting point: 199-201 ˚C.

(Example 75) 4-{4-[3-(2-methoxy-5-methyl-phenyl)-ureido]-phenyl}-piperazine-1-carboxylic acid (4-butyl-2-methyl-phenyl)-amide (Compound No. 1-348)

89 mg (84%) of the title compound was obtained from 1-(2-methoxy-5-methyl-phenyl)-3-(4-piperazin-1-yl-phenyl)-urea (68 mg) obtained in Example (47a) and 4-butyl-2-methylphenyl isocyanate (57 mg) as a light brown powder in the same manner as in Example 49.
$^1$H-NMR spectrum (400MHz,DMSO-d6):δ(ppm)=9.08(1H, s), 8.06(2H, s), 7.99(1H, d, J=1.9Hz), 7.33(2H, d, J=9.0Hz), 7.07(1H, d, J=7.9Hz), 7.00(1H, d, J=1.2Hz), 6.94(3H, d, J=9.0Hz), 6.94(1H, d, J=9.0Hz), 6.88(1H, d, J=8.2Hz), 6.73(1H, dd, J=8.6 and 1.9Hz), 3.84(3H, s), 3.58(4H, t, J=4.9Hz), 3.07(4H, t, J=4.3Hz), 2.53-2.50(2H, m), 2.23(3H, s), 2.14(3H, s), 1.55-1.52(2H, m), 1.30(2H, q, J=7.2Hz), 0.90(3H, t, J=7.5Hz).
MS(FAB) m/z:530 $(M + H)^+$.
Melting point: 172-173˚C.

(Example 76) 4-{4-[3-(2-methoxy-5-methyl-phenyl)-ureido]-phenyl}-piperazine-1-carboxylic acid (2-chloro-4-methyl-phenyl)-amide (Compound No. 1-117)

90 mg (88%) of the title compound was obtained from 1-(2-methoxy-5-methyl-phenyl)-3-(4-piperazin-1-yl-phenyl)-urea (68 mg) obtained in Example (47a) and 2-chloro-4-methylphenyl isocyanate (50 mg) as a pale pink powder in the same manner as in Example 49.
$^1$H-NMR spectrum (400MHz,DMSO-d6):δ(ppm)=9.06(1H, s), 8.20(1H, s), 8.04(1H, s), 7.96(1H, d, J=2.0Hz), 7.32(1H, d, J=8.2Hz), 7.30(2H, d, J=9.0Hz), 7.26(1H, d, J=2.0Hz), 7.27(2H, d, J=2.0Hz), 7.08(1H, dd, J=8.4 and 1.4Hz), 6.92 (2H, d, J=9.0Hz), 6.86(1H, d, J=8.2Hz), 6.70(1H, dd, J=8.4 and 2.6Hz), 3.82(3H, s), 3.58(4H, t, J=4.9Hz), 3.07(4H, t, J=4.9Hz), 2.28(3H, s), 2.22(3H, s).
MS(FAB) m/z:508 $(M + H)^+$.
Melting point: 186-187˚C.

(Example 77) 4-{4-[3-(5-chloro-2-methoxy-phenyl)-ureido]-phenyl}-piperazine-1-carboxylic acid (2,6-difluoro-phenyl)-amide (Compound No. 1-3)

96 mg (93%) of the title compound was obtained from 1-(5-chloro-2-methoxy-phenyl)-3-(4-piperazin-1-yl-phenyl)-urea (72 mg) obtained in Example (67a) and 2,6-difluorophenyl isocyanate (47 mg) as a dark brown powder in the same manner as in Example 49.
$^1$H-NMR spectrum (400MHz,DMSO-d6):δ(ppm)=9.18(1H, s), 8.36(1H, s), 8.30(1H, s), 8.25(1H, d, J=2.8Hz), 7.33(2H, d, J=9.0Hz), 7.31-7.25(1H, m), 7.12(2H, dd, J=8.0 and 8.0Hz), 7.03(1H, d, J=8.6Hz), 6.96(3H, d, J=8.6Hz), 3.89(3H, s), 3.60(4H, t, J=4.7Hz), 3.09(4H, t, J=4.9Hz).
MS(FAB) m/z:516 $(M + H)^+$.
Melting point: 230-232˚C.

(Example 78) 4-{4-[3-(5-chloro-2-methoxy-phenyl)-ureido]-phenyl}-piperazine-1-carboxylic acid (2-chloro-phenyl)-amide (Compound No. 1-353)

75 mg (73%) of the title compound was obtained from 1-(5-chloro-2-methoxy-phenyl)-3-(4-piperazin-1-yl-phenyl)-urea (72 mg) obtained in Example (67a) and 2-chlorophenyl isocyanate (0.036 mL) as a grey white powder in the same manner as in Example 49.
$^1$H-NMR spectrum (400MHz,DMSO-d6):δ(ppm)=9.18(1H, s), 8.30(2H, s), 8.25(1H, d, J=2.4Hz), 7.52(1H, dd, J=8.0 and 1.3Hz), 7.46(1H, dd, J=7.8 and 1.2Hz), 7.34(2H, d, J=8.9Hz), 7.29(1H, dd, J=7.7 and 1.3Hz), 7.16(1H, ddd, J=7.7, 7.7 and 1.4Hz), 7.03(1H, d, J=8.6Hz), 6.97(1H, d, J=9.0Hz), 6.96(2H, d, J=9.0Hz), 3.89(3H, s), 3.61(4H, t, J=5.1Hz), 3.10 (4H, t, J=4.9Hz).
MS(FAB) m/z:514 $(M + H)^+$.

Melting point: 231-233˚C.

(Example 79) 4-{4-[3-(5-chloro-2-methoxy-phenyl)-ureido]-phenyl}-piperazine-1-carboxylic acid (2-chloro-6-methyl-phenyl)-amide (Compound No. 1-45)

95 mg (90%) of the title compound was obtained from 1-(5-chloro-2-methoxy-phenyl)-3-(4-piperazin-1-yl-phenyl)-urea (72 mg) obtained in Example (67a) and 2-chloro-6-methylphenyl isocyanate (0.041 mL) as a grey white powder in the same manner as in Example 49.
$^1$H-NMR spectrum (400MHz,DMSO-d6):δ(ppm)=9.18(1H, s), 8.30(1H, s), 8.25(1H, d, J=2.8Hz), 8.23(1H, s), 7.33(2H, d, J=9.0Hz), 7.33(1H, d, J=9.0Hz), 7.20(1H, dd, J=7.8 and 1.2Hz), 7.16(1H, dd, J=7.8 and 7.8Hz), 7.03(1H, d, J=9.0Hz), 6.97(1H, d, J=8.6Hz), 6.96(2H, d, J=9.0Hz), 3.89(3H, s), 3.61(4H, t, J=4.9Hz), 3.09(4H, t, J=4.6Hz), 2.20(3H, s).
MS(FAB) m/z:528 $(M + H)^+$.
Melting point: 225-227˚C.

(Example 80) 4-{4-[3-(2-ethoxy-phenyl)-ureido]-phenyl}-piperazine-1-carboxylic acid (2-trifluoromethyl-phenyl)-amide (Compound No. 1-203)

(80a) 1-(2-ethoxy-phenyl)-3-(4-piperazin-1-yl-phenyl)-urea

8.30 g (98%) of the title compound was obtained from 4-{4-[3-(2-ethoxyphenyl)-ureido]-phenyl}-piperazine-1-carboxylic acid tert-butyl ester (11.0 g) obtained in Example 4 as a light brown powder in the same manner as in Example (47a).
MS(FAB) m/z:341 $(M + H)^+$.

(80b) 4-{4-[3-(2-ethoxy-phenyl)-ureido]-phenyl}-piperazine-1-carboxylic acid (2-trifluoromethyl-phenyl)-amide

33 mg (31%) of the title compound was obtained from 1-(2-ethoxy-phenyl)-3-(4-piperazin-1-yl-phenyl)-urea (68 mg) obtained in Example (80a) and 2-trifluoromethylphenyl isocyanate (0.045 mL) as a pale yellow powder in the same manner as in Example 49.
$^1$H-NMR spectrum (400MHz,DMSO-d6):δ(ppm)=9.14(1H, s), 8.32(1H, s), 8.10(1H, dd, J=7. 7 and 2.2Hz), 7.95(1H, s), 7.68(1H, d, J=7.5Hz), 7.63(1H, dd, J=7.6 and 7.6Hz), 7.43(1H, d, J=8.2Hz), 7.39(1H, dd, J=7.7 and 7.7Hz), 7.32(2H, d, J=9.0Hz), 6.97(1H, dd, J=7.8 and 1.6Hz), 6.93(1H, d, J=9.0Hz), 6.89(1H, ddd, J=7.6, 7.6 and 2.0Hz), 6.87(1H, d, J=2.3Hz), 6.85(1H, ddd, J=7.8, 7.8 and 1.9Hz), 4.12(2H, q, J=7.1Hz), 3.57(4H, t, J=5.0Hz), 3.07(4H, t, J=4.9Hz), 1.41 (3H, t, J=6.9Hz).
MS(FAB) m/z:528 $(M + H)^+$.
Melting point: 201-203˚C.

(Example 81) 4-{4-[3-(2-ethoxy-phenyl)-ureido]-phenyl}-piperazine-1-carboxylic acid o-tolylamide (Compound No. 1-189)

84 mg (89%) of the title compound was obtained from 1-(2-ethoxy-phenyl)-3-(4-piperazin-1-yl-phenyl)-urea (68 mg) obtained in Example (80a) and 2-methylphenyl isocyanate (0.037 mL) as an ochre powder in the same manner as in Example 49.
$^1$H-NMR spectrum (400MHz,DMSO-d6):δ(ppm)=9.15(1H, s), 8.12(1H, s), 8.10(1H, dd, J=7.7 and 2.2Hz), 7.95(1H, s), 7.32(2H, d, J=9.0Hz), 7.17(1H, dd, J=7.5 and 7.5Hz), 7.11(1H, ddd, J=7.5, 7.5 and 1.7Hz), 7.03(1H, ddd, J=7.3, 7.3 and 1.4Hz), 6.97(1H, dd, J=7.6 and 1.8Hz), 6.94(2H, d, J=9.0Hz), 6.89(1H, ddd, J=7.5, 7.5 and 2.0Hz), 6.87(1H, d, J=2.3Hz), 6.85(1H, ddd, J=7.7, 7.7 and 2.1Hz), 4.12(2H, q, J=7.1Hz), 3.58(4H, t, J=4.9Hz), 3.08(4H, t, J=4.9Hz), 2.17 (3H, s), 1.41(3H, t, J=6.9Hz).
MS(FAB) m/z:474 $(M + H)^+$.
Melting point: 206-208˚C.

(Example 82) 4-{4-[3-(2-ethoxy-phenyl)-ureido]-phenyl}-piperazine-1-carboxylic acid (2-chloro-6-methyl-phenyl)-amide (Compound No. 1-49)

92 mg (90%) of the title compound was obtained from 1-(2-ethoxy-phenyl)-3-(4-piperazin-1-yl-phenyl)-urea (68 mg) obtained in Example (80a) and 2-chloro-6-methylphenyl isocyanate (0.041 mL) as a brown powder in the same manner as in Example 49.
$^1$H-NMR spectrum (400MHz,DMSO-d6):δ(ppm)=9.15(1H, s), 8.21(1H, s), 8.10(1H, dd, J=7.9 and 2.0Hz), 7.95(1H, s),

7.33-7.29(3H, m), 7.20(1H, d, J=6.6Hz), 7.14(1H, dd, J=7.6 and 7.6Hz), 6.97(1H, dd, J=7.6 and 1.8Hz), 6.94(2H, d, J=9.0Hz), 6.89(1H, ddd, J=7.5, 7.5 and 2.1Hz), 6.85(1H, ddd, J=7.7, 7.7 and 2.1Hz), 4.12(2H, q, J=6.9Hz), 3.60(4H, t, J=4.9Hz), 3.07(4H, t, J=4.7Hz), 2.20(3H, s), 1.41(3H, t, J=7.1Hz).
MS(FAB) m/z:508 $(M + H)^+$.
Melting point: 215-217˚C.

(Example 83) 4-{4-[3-(2-ethoxy-5-methyl-phenyl)-ureido]-phenyl}-piperazine-1-carboxylic acid (2-trifluoromethyl-phenyl)-amide (Compound No. 1-202)

(83a) 1-(2-ethoxy-5-methylphenyl)-3-(4-piperazin-1-yl-phenyl)-urea

2.92 g (82%) of the title compound was obtained from 4-{4-[3-(2-ethoxy-5-methylphenyl)-ureido]-phenyl}-piperazine-1-carboxylic acid tert-butyl ester (4.55 g) obtained in Example 41 as a pale pink powder in the same manner as in Example (47a).
MS(FAB) m/z:355 $(M + H)^+$.

(83b) 4-{4-[3-(2-ethoxy-5-methyl-phenyl)-ureido]-phenyl}-piperazine-1-carboxylic acid (2-trifluoromethyl-phenyl)-amide

78 mg (72%) of the title compound was obtained from 1-(2-ethoxy-5-methylphenyl)-3-(4-piperazin-1-yl-phenyl)-urea (71 mg) obtained in Example (83a) and 2-trifluoromethylphenyl isocyanate (0.045 mL) as a pale flesh-coloured powder in the same manner as in Example 49.
$^1$H-NMR spectrum (400MHz,DMSO-d6):δ(ppm)=9.13(1H, s), 8.32(1H, s), 7.96(1H, d, J=2.0Hz), 7.89(1H, s), 7.68(1H, d, J=8.2Hz), 7.63(1H, dd, J=7.1 and 7.0Hz), 7.43(1H, d, J=7.8Hz), 7.39(1H, dd, J=7.7 and 7.7Hz), 7.32(2H, d, J=9.0Hz), 6.93(2H, d, J=9.0Hz), 6.85(1H, d, J=8.2Hz), 6.68(1H, dd, J=8.2 and 1.6Hz), 4.07(2H, q, J=7.1Hz), 3.57(4H, t, J=4.7Hz), 3.07(4H, t, J=4.9Hz), 2.21(3H, s), 1.38(3H, t, J=7.1Hz).
MS(FAB) m/z:542 $(M + H)^+$.
Melting point: 219-220˚C.

(Example 84) 4-{4-[3-(2-ethoxy-5-methyl-phenyl)-ureido]-phenyl}-piperazine-1-carboxylic acid o-tolylamide (Compound No. 1-188)

75 mg (77%) of the title compound was obtained from 1-(2-ethoxy-5-methylphenyl)-3-(4-piperazin-1-yl-phenyl)-urea (71 mg) obtained in Example (83a) and 2-methylphenyl isocyanate (0.037 mL) as a pale violet powder in the same manner as in Example 49.
$^1$H-NMR spectrum (400MHz,DMSO-d6):δ(ppm)=9.15(1H, s), 8.13(1H, s), 7.96(1H, d, J=2.0Hz), 7.90(1H, s), 7.34(2H, d, J=9.0Hz), 7.17(2H, dd, J=7.1 and 7.1Hz), 7.11(1H, dd, J=7.4 and 7.4Hz), 7.03(1H, ddd, J=7.2, 7.2 and 1.4Hz), 6.96 (2H, d, J=8.2Hz), 6.85(1H, d, J=8.2Hz), 6.68(1H, dd, J=8.2 and 1.6Hz), 4.07(2H, q, J=7.1Hz), 3.59(4H, brs), 3.10(4H, brs), 2.21(3H, s), 2.17(3H, s), 1.38(3H, t, J=6.9Hz).
MS(FAB) m/z:488 $(M + H)^+$.
Melting point: 190-192˚C.

(Example 85) 4-{4-[3-(2-ethoxy-5-methyl-phenyl)-ureido]-phenyl}-piperazine-1-carboxylic acid (2-chloro-6-methyl-phenyl)-amide (Compound No. 1-48)

75 mg (72%) of the title compound was obtained from 1-(2-ethoxy-5-methylphenyl)-3-(4-piperazin-1-yl-phenyl)-urea (71 mg) obtained in Example (83a) and 2-chloro-6-methylphenyl isocyanate (0.041 mL) as a pale violet powder in the same manner as in Example 49.
$^1$H-NMR spectrum (400MHz,DMSO-d6):δ(ppm)=9.15(1H, s), 8.22(1H, s), 7.96(1H, d, J=1.6Hz), 7.90(1H, s), 7.33(2H, d, J=9.0Hz), 7.31(1H, dd, J=7.8 and 1.2Hz), 7.18(1H, d, J=7.4Hz), 7.14(1H, dd, J=7.6 and 7.6Hz), 6.96(2H, d, J=9.0Hz), 6.85(1H, d, J=8.2Hz), 6.68(1H, dd, J=8.2 and 1.6Hz), 4.07(2H, q, J=6.9Hz), 3.61(4H, brs), 3.10(4H, brs), 2.21(3H, s), 2.20(3H, s), 1.38(3H, t, J=6.9Hz).
MS(FAB) m/z:522 $(M + H)^+$.
Melting point: 157-159˚C.

(Example 86) 4- {4-[3-(2-methoxy-5-methyl-phenyl)-ureido]-phenyl}-piperazine-1-carboxylic acid (4-chloro-2-trifluoromethyl-phenyl)-amide (Compound No. 1-355)

100 mg (89%) of the title compound was obtained from 1-(2-methoxy-5-methyl-phenyl)-3-(4-piperazin-1-yl-phenyl)-urea (68 mg) obtained in Example (47a) and 4-chloro-2-trifluoromethylphenyl isocyanate (0.045 mL) as a pale pink powder in the same manner as in Example 49.
$^1$H-NMR spectrum (400MHz,DMSO-d6):δ(ppm)=9.05(1H, s), 8.39(1H, s), 8.04(1H, s), 7.96(1H, d, J=2Hz), 7.72-7.67 (2H, m), 7.49(1H, d, J=7.9Hz), 7.31(2H, d, J=9.OHz), 6.92(2H, d, J=9.0Hz), 6.86(1H, d, J=8.2Hz), 6.70(1H, dd, J=8.0 and 1.8Hz), 3.82(3H, s), 3.56(4H, t, J=5.1Hz), 3.05(4H, t, J=4.7Hz), 2.22(3H, s).
MS(FAB) m/z:562 $(M + H)^+$.
Melting point: 186-187˚C.

(Example 87) 4-{4-[3-(2-methoxy-S-methyl-phenyl)-ureido]-phenyl}-piperazine-1-carboxylic acid (4-fluoro-2-trifluoromethyl-phenyl)-amide (Compound No. 1-103)

101 mg (93%) of the title compound was obtained from 1-(2-methoxy-5-methyl-phenyl)-3-(4-piperazin-1-yl-phenyl)-urea (68 mg) obtained in Example (47a) and 4-fluoro-2-trifluoromethylphenyl isocyanate (0.043 mL) as a pale pink powder in the same manner as in Example 49.
$^1$H-NMR spectrum (400MHz,DMSO-d6):δ(ppm)=9.05(1H, s), 8.35(1H, s), 8.03(1H, s), 7.96(1H, d, J=2.0Hz), 7.56(1H, d, J=7.9Hz), 7.50-7.47(2H, m), 7.31(2H, d, J=9.0Hz), 6.92(2H, d, J=9.0Hz), 6.86(1H, d, J=8.2Hz), 6.70(1H, dd, J=8.0 and 1.4Hz), 3.82(3H, s), 3.56(4H, t, J=4.6Hz), 3.05(4H, t, J=4.9Hz), 2.22(3H, s).
MS(FAB) m/z:546 $(M + H)^+$.
Melting point: 172-174˚C.

(Example 88) 4-{4-[3-(2-methoxy-5-methyl-phenyl)-ureido]-phenyl}-piperazine-1-carboxylic acid (2,4,6-trimethyl-phenyl)-amide (Compound No. 1-361)

92 mg (91 %) of the title compound was obtained from 1-(2-methoxy-5-methyl-phenyl)-3-(4-piperazin-1-yl-phenyl)-urea (68 mg) obtained in Example (47a) and 2,4,6-trimethylphenyl isocyanate (48 mg) as a white powder in the same manner as in Example 49.
$^1$H-NMR spectrum (400MHz,DMSO-d6):δ(ppm)=9.06(1H, s), 8.04(1H, s), 7.96(1H, d, J=1.6Hz), 7.85(1H, s), 7.31(2H, d, J=8.6Hz), 6.92(2H, d, J=9.0Hz), 6.86(1H, d, J=8.2Hz), 6.84(2H, s), 6.70(1H, dd, J=8.6 and 1.6Hz), 3.82(3H, s), 3.58 (4H, t, J=4.7Hz), 3.05(4H, t, J=4.9Hz), 2.22(3H, s), 2.21(3H, s), 2.10(6H, s).
MS(FAB) m/z:502 $(M + H)^+$.
Melting point: 215-217˚C.

(Example 89) 4-{4-[3-(2-ethoxy-5-fluoro-phenyl)-ureido]-phenyl}-piperazine-1-carboxylic acid (2-trifluoromethyl-phenyl)-amide (Compound No. 1-198)

(89a) 1-(2-ethoxy-5-fluoro)-3-(4-piperazin-1-yl-phenyl)-urea

1.64 g (95%) of the title compound was obtained from 4-{4-[3-(2-ethoxy-5-methylphenyl)-ureido]-phenyl}-piperazine-1-carboxylic acid tert-butyl ester (2.22 g) obtained in Example 42 as a greyish brown solid in the same manner as in Example (47a).
MS(FAB) m/z:359 $(M + H)^+$.

(89b) 4-{4-[3-(2-ethoxy-5-fluoro-phenyl)-ureido]-phenyl}-piperazine-1-carboxylic acid (2-trifluoromethyl-phenyl)-amide

92 mg (91%) of the title compound was obtained from 1-(2-ethoxy-5-fluorophenyl)-3-(4-piperazin-1-yl-phenyl)-urea (72 mg) obtained in Example (89a) and 2-trifluoromethylphenyl isocyanate (0.045 mL) as a white powder in the same manner as in Example 49.
$^1$H-NMR spectrum (400MHz,DMSO-d6):δ(ppm)=9.27(1H, s), 8.32(1H, brs), 8.14(1H, s), 8.00(1H, dd, J=11.7 and 3.1Hz), 7.65(1H, d, J=7.9Hz), 7.60(1H, dd, J=7.5 and 7.5Hz), 7.47(1H, d, J=8.2Hz), 7.36-7.31(1H, m), 7.32(2H, d, J=9.0Hz), 6.96(1H, dd, J=9.2 and 5.7Hz), 6.93(2H, d, J=9.4Hz), 6.68(1H, ddd, J=8.6, 8.6 and 3.4Hz), 4.10(2H, q, J=7.1Hz), 3.57 (4H, t, J=5.1Hz), 3.07(4H, t, J=4.9Hz), 1.40(3H, t, J=7.0Hz).
MS(FAB) m/z:546 $(M + H)^+$.
Melting point: 197-199˚C.

(Example 90) 4- {4-[3-(2-ethoxy-5-fluoro-phenyl)-ureido]-phenyl} -piperazine-1-carboxylic acid o-tolylamide (Compound No. 1-184)

87 mg (89%) of the title compound was obtained from 1-(2-ethoxy-5-fluorophenyl)-3-(4-piperazin-1-yl-phenyl)-urea (72 mg) obtained in Example (89a) and 2-methylphenyl isocyanate (0.037 mL) as a light brown powder in the same manner as in Example 49.
$^1$H-NMR spectrum (400MHz,DMSO-d6):δ(ppm)=9.29(1H, s), 8.14(1H, s), 8.12(1H, s), 8.00(1H, dd, J=11.7 and 3.1Hz), 7.32(2H, d, J=9.0Hz), 7.17(2H, dd, J=8.0 and 8.0Hz), 7.11(1H, ddd, J=7.6, 7.6 and 1.6Hz), 7.03(1H, ddd, J=7.2, 7.2 and 1.4Hz), 6.96(1H, dd, J=9.2 and 5.2Hz), 6.94(2H, d, J=9.0Hz), 6.68(1H, ddd, J=8.4, 8.4 and 3.2Hz), 4.10(2H, q, J=7.1Hz), 3.58(4H, t, J=4.9Hz), 3.08(4H, t, J=4.9Hz), 2.17(3H, s), 1.40(3H, t, J=7.0Hz).
MS(FAB) m/z:492 $(M + H)^+$.
Melting point: 186-187˚C.

(Example 91) 4-{4-[3-(2-ethoxy-5-fluoro-phenyl)-ureido]-phenyl}-piperazine-1-carboxylic acid (2-chloro-6-methyl-phenyl)-amide (Compound No. 1-44)

97 mg (93%) of the title compound was obtained from 1-(2-ethoxy-5-fluorophenyl)-3-(4-piperazin-1-yl-phenyl)-urea (72 mg) obtained in Example (89a) and 2-chloro-6-methylphenyl isocyanate (0.041 mL) as a light brown powder in the same manner as in Example 49.
$^1$H-NMR spectrum (400MHz,DMSO-d6):δ(ppm)=9.27(1H, s), 8.21(1H, s), 8.14(1H, s), 8.02(1H, d, J=3.1Hz), 7.99(1H, d, J=3.1Hz), 7.32(2H, d, J=9.0Hz), 7.31-7.28(1H, m), 7.19(1H, d, J=6.3Hz), 7.14(1H, dd, J=15.9 and 8.1Hz), 6.99-6.93 (1H, m), 6.94(1H, d, J=9.3Hz), 6.68(1H, ddd, J=8.5, 8.5 and 3.1Hz), 4.10(2H, q, J=7.1Hz), 3.60(4H, t, J=5.1Hz), 3.08 (4H, t, J=5.1Hz), 2.20(3H, s), 1.40(3H, t, J=6.8Hz).
MS(FAB) m/z:526 $(M + H)^+$.
Melting point: 197-199˚C.

(Example 92) 4-{4-[3-(2-ethoxy-5-fluoro-phenyl)-ureido]-phenyl}-piperazine-1-carboxylic acid (2,6-dimethyl-phenyl)-amide (Compound No. 1-58)

97 mg (96%) of the title compound was obtained from 1-(2-ethoxy-5-fluorophenyl)-3-(4-piperazin-1-yl-phenyl)-urea (72 mg) obtained in Example (89a) and 2,6-dimethylphenyl isocyanate (0.042 mL) as a light brown powder in the same manner as in Example 49.
$^1$H-NMR spectrum (400MHz,DMSO-d6):δ(ppm)=9.27(1H, s), 8.14(1H, s), 8.01(1H, dd, J=11.5 and 2.9Hz), 7.95(1H, s), 7.32(2H, d, J=9.4Hz), 7.02(2H, s), 7.00-6.92(2H, m), 6.95(2H, d, J=9.0Hz), 6.68(1H, ddd, J=8.5, 8.5 and 3.1Hz), 4.10 (2H, q, J=7.1Hz), 3.59(4H, t, J=5.1Hz), 3.07(4H, t, J=5.1Hz), 2.15(6H, s), 1.40(3H, t, J=6.9Hz).
MS(FAB) m/z:506 $(M + H)^+$.
Melting point: 205-206˚C.

(Example 93) 4-{4-[3-(2-ethoxy-5-fluoro-phenyl)-ureido]-phenyl}-piperazine-1-carboxylic acid (2-fluoro-6-trifluoromethyl-phenyl)-amide (Compound No. 1-16)

98 mg (87%) of the title compound was obtained from 1-(2-ethoxy-5-fluorophenyl)-3-(4-piperazin-1-yl-phenyl)-urea (72 mg) obtained in Example (89a) and 2-fluoro-6-trifluoromethylphenyl isocyanate (0.043 mL) as a light brownish green powder in the same manner as in Example 49.
$^1$H-NMR spectrum (400MHz,DMSO-d6):δ(ppm)=9.30(1H, s), 8.36(1H, brs), 8.17(1H, s), 8.03(1H, dd, J=11.6 and 2.9Hz), 7.61-7.47(3H, m), 7.35(2H, d, J=9.4Hz), 7.01-6.95(1H, m), 6.96(2H, d, J=8.6Hz), 6.71(1H, ddd, J=8.4 ,8.4 and 3.1Hz), 4.12(2H, q, J=7.1Hz), 3.59(4H, t, J=4.7Hz), 3.07(4H, t, J=4.9Hz), 1.40(3H, t, J=6.8Hz).
MS(FAB) m/z:564 $(M + H)^+$.
Melting point: 153-155˚C.

(Example 94) 4-{4-[3-(2-ethoxy-5-fluoro-phenyl)-ureido]-phenyl}-piperazine-1-carboxylic acid (2,6-dichloro-phenyl)-amide (Compound No. 1-30)

103 mg (94%) of the title compound was obtained from 1-(2-ethoxy-5-fluorophenyl)-3-(4-piperazin-1-yl-phenyl)-urea (72 mg) obtained in Example (89a) and 2,6-dichlorophenyl isocyanate (56 mg) as a light brownish green powder in the same manner as in Example 49.
$^1$H-NMR spectrum (400MHz,DMSO-d6):δ(ppm)=9.31(1H, s), 8.54(1H, brs), 8.17(1H, s), 8.03(1H, dd, J=11.9 and 3.4Hz), 7.51 (2H, d, J=8.2Hz), 7.35(2H, d, J=8.6Hz), 7.28(1H, ddd, J=8.5, 8.5 and 1.5Hz), 7.01-6.96(1H, m), 6.96(2H, d, J=9.0Hz), 6.71 (1H, ddd, J=8.5, 8.5 and 3.2Hz), 4.12(2H, q, J=7.1 Hz), 3.61 (4H, brs), 3.09(4H, t, J=4.5Hz), 1.40(3H, t, J=6.8Hz).
MS(FAB) m/z:546 $(M + H)^+$.
Melting point: 212-214˚C.

(Example 95) 1-(4-{4-[2-(2-fluoro-phenyl)-acetyl]-piperazin-1-yl}-phenyl)-3-(2-methoxy-5-methyl-phenyl)-urea (Compound No. 1-367)

87 mg (88%) of the title compound was obtained from 1-(2-methoxy-5-methyl-phenyl)-3-(4-piperazin-1-yl-phenyl)-urea (68 mg) obtained in Example (47a) and 2-fluorobenzyl isocyanate (0.038 mL) as a white powder in the same manner as in Example 49.
$^1$H-NMR spectrum (400MHz,DMSO-d6):δ(ppm)=9.06(1H, s), 8.04(1H, s), 7.95(1H, d, J=1.9Hz), 7.33-7.23(2H, m), 7.29 (2H, d, J=9.0Hz), 7.17-7.10(1H, m), 7.14(2H, dd, J=7.5 and 7.5Hz), 6.90(2H, d, J=9.0Hz), 6.86(1H, d, J=8.2Hz), 6.70 (1H, dd, J=8.5 and 1.3Hz), 4.29(2H, d, J=5.4Hz), 3.82(3H, s), 3.47(4H, t, J=4.9Hz), 3.01(4H, t, J=4.7Hz), 2.22(3H, s).
MS(FAB) m/z:492 $(M + H)^+$.
Melting point: 212-213˚C.

(Example 96) 4-{4-[3-(5-fluoro-2-methoxy-phenyl)-ureido]-phenyl}-piperazine-1-carboxylic acid (2-trifluoromethyl-phenyl)-amide (Compound No. 1-197)

(96a) 1-(5-fluoro-2-methoxy-phenyl)-3-(4-piperazin-1-yl-phenyl)-urea

0.689 g (100%) of the title compound was obtained from 4-{4-[3-(5-fluoro-2-methoxy-phenyl)-ureido]-phenyl}-piperazine-1-carboxylic acid tert-butyl ester (0.889 g) obtained in Example 44 as a light grey solid in the same manner as in Example (47a).
MS(FAB) m/z:345 $(M + H)^+$.

(96b) 4- {4-[3-(5-fluoro-2-methoxy-phenyl)-ureido]-phenyl} -piperazine- I -carboxylic acid (2-trifluoromethyl-phenyl)-amide

99 mg (93%) of the title compound was obtained from 1-(5-fluoro-2-methoxy-phenyl)-3-(4-piperazin-1-yl-phenyl)-urea (69 mg) obtained in Example (96a) and 2-trifluoromethylphenyl isocyanate (0.045 mL) as a white powder in the same manner as in Example 49.
$^1$H-NMR spectrum (400MHz,DMSO-d6):δ(ppm)=9.20(1H, s), 8.34(1H, s), 8.32(1H, s), 8.03(1H, dd, J=11.5 and 3.4Hz), 7.71-7.63(2H, m), 7.47-7.40(2H, m), 7.33(2H, d, J=8.2Hz), 7.02-6.97(1H, m), 6.96(2H, d, J=9.0Hz), 6.73(1H, ddd, J=8.4, 8.4 and 2.9Hz), 3.87(3H, s), 3.58(4H, t, J=4.5Hz), 3.08(4H, t, J=4.0Hz).
MS(FAB) m/z:532 $(M + H)^+$.
Melting point: 229-230˚C.

(Example 97) 4-{4-[3-(5-fluoro-2-methoxy-phenyl)-ureido]-phenyl}-piperazine-1-carboxylic acid o-tolylamide (Compound No. 1-183)

86 mg (90%) of the title compound was obtained from 1-(S-fluoro-2-methoxy-phenyl)-3-(4-piperazin-1-yl-phenyl)-urea (69 mg) obtained in Example (96a) and 2-methylphenyl isocyanate (0.037 mL) as a white powder in the same manner as in Example 49.
$^1$H-NMR spectrum (400MHz,DMSO-d6):δ(ppm)=9.17(1H, s), 8.30(1H, s), 8.13(1H, s), 8.01(1H, dd, J=11.7 and 3.1Hz), 7.31(2H, d, J=9.0Hz), 7.17(2H, dd, J=7.0 and 7.0Hz), 7.11(1H, dd, J=7.4 and 7.5Hz), 7.05-6.94(2H, m), 6.94(2H, d, J=8.6Hz), 6.70(1H, ddd, J=8.6, 8.6 and 3.6Hz), 3.86(3H, s), 3.58(4H, t, J=5.1Hz), 3.07(4H, t, J=4.1Hz), 2.17(3H, s).
MS(FAB) m/z:478 $(M + H)^+$.
Melting point: 222-224˚C.

(Example 98) 4-{4-[3-(5-fluoro-2-methoxy-phenyl)-ureido]-phenyl}-piperazine-1-carboxylic acid (2-chloro-6-methyl-phenyl)-amide (Compound No. 1-43)

89 mg (87%) of the title compound was obtained from 1-(5-fluoro-2-methoxy-phenyl)-3-(4-piperazin-1-yl-phenyl)-urea (69 mg) obtained in Example (96a) and 2-chloro-6-methylphenyl isocyanate (0.041 mL) as a white powder in the same manner as in Example 49.
$^1$H-NMR spectrum (400MHz,DMSO-d6):δ(ppm)=9.17(1H, s), 8.30(1H, s), 8.21(1H, s), 8.01(1H, dd, J=11.5 and 3.3Hz), 7.30(3H, d, J=9.0Hz), 7.19(1H, d, J=7.0Hz), 7.14(1H, dd, J=16.6 and 9.2Hz), 6.98(1H, dd, J=9.0 and 5.5Hz), 6.94(2H, d, J=9.0Hz), 6.70(1H, ddd, J=8.6, 8.6 and 3.2Hz), 3.86(3H, s), 3.60(4H, t, J=4.5Hz), 3.08(4H, t, J=4.3Hz), 2.20(3H, s).
MS(FAB) m/z:512 $(M + H)^+$.
Melting point: 240-241˚C.

(Example 99) 4-{4-[3-(5-fluoro-2-methoxy-phenyl)-ureido]-phenyl}-piperazine-1-carboxylic acid (2,6-dimethyl-phenyl)-amide (Compound No. 1-57)

90 mg (92%) of the title compound was obtained from 1-(5-fluoro-2-methoxy-phenyl)-3-(4-piperazin-1-yl-phenyl)-urea (69 mg) obtained in Example (96a) and 2,6-dimethylphenyl isocyanate (0.042 mL) as a white powder in the same manner as in Example 49.
$^1$H-NMR spectrum (400MHz,DMSO-d6):δ(ppm)=9.16(1H, s), 8.30(1H, s), 8.01(1H, dd, J=11.5 and 3.4Hz), 7.94(1H, s), 7.31(2H, d, J=9.0Hz), 7.02(3H, s), 6.98(1H, dd, J=8.6 and 5.5Hz), 6.94(2H, d, J=8.6Hz), 6.70,(1H, ddd, J=8.5, 8.5 and 3.2Hz), 3.86(3H, s), 3.59(4H, t, J=4.4Hz), 3.07(4H, t, J=4.7Hz), 2.14(6H, s).
MS(FAB) m/z:492 $(M + H)^+$.
Melting point: 226-228˚C.

(Example 100) 4-{4-[3-(5-fluoro-2-methoxy-phenyl)-ureido]-phenyl}-piperazine-1-carboxylic acid (2-fluoro-6-trifluoromethyl-phenyl)-amide (Compound No. 1-15)

96 mg (87%) of the title compound was obtained from 1-(5-fluoro-2-methoxy-phenyl)-3-(4-piperazin-1-yl-phenyl)-urea (69 mg) obtained in Example (96a) and 2-fluoro-6-trifluoromethylphenyl isocyanate (0.043 mL) as a white powder in the same manner as in Example 49.
$^1$H-NMR spectrum (400MHz,DMSO-d6):δ(ppm)=9.17(1H, s), 8.32(1H, s), 8.30(1H, s), 8.01(1H, dd, J=11.3 and 3.1Hz), 7.62-7.50(3H, m), 7.31(2H, d, J=9.0Hz), 6.98(1H, dd, J=9.2 and 5.3Hz), 6.94(2H, d, J=9.0Hz), 6.70(1H, ddd, J=8.6, 8.6 and 6.3Hz), 3.86(3H, s), 3.58(4H, t, J=4.5Hz), 3.06(4H, t, J=4.9Hz).
MS(FAB) m/z:550 $(M + H)^+$.
Melting point: 181-183˚C.

(Example 101) 4-{4-[3-(5-fluoro-2-methoxy-phenyl)-ureido]-phenyl}-piperazine-1-carboxylic acid (2,6-dichloro-phenyl)-amide (Compound No. 1-29)

96 mg (90%) of the title compound was obtained from 1-(5-fluoro-2-methoxy-phenyl)-3-(4-piperazin-1-yl-phenyl)-urea (69 mg) obtained in Example (96a) and 2,6-dichlorophenyl isocyanate (56 mg) as a light brown powder in the same manner as in Example 49.
$^1$H-NMR spectrum (400MHz,DMSO-d6):δ(ppm)=9.17(1H, s), 8.50(1H, s), 8.30(1H, s), 8.00(1H, dd, J=11.7 and 3.1Hz), 7.49(2H, d, J=8.2Hz), 7.31(2H, d, J=9.0Hz), 7.27(1H, d, J=8.2Hz), 6.98(1H, dd, J=9.0 and 5.1Hz), 6.94(2H, d, J=9.0Hz), 6.70,(1H, ddd, J=8.5, 8.5 and 3.3Hz), 3.86(3H, s), 3.60(4H, t, J=4.9Hz), 3.08(4H, t, J=4.9Hz).
MS(FAB) m/z:532 $(M + H)^+$.
Melting point: 235-237˚C.

(Example 102) 4-{4-[3-(5-chloro-2-ethoxy-phenyl)-ureido]-phenyl}-piperazine-1-carboxylic acid (2-trifluoromethyl-phenyl)-amide (Compound No. 1-200)

(102a) 1-(5-chloro-2-ethoxy-phenyl)-3-(4-piperazin-1-yl-phenyl)-urea

0.668 g (99%) of the title compound was obtained from 4-{4-[3-(5-chloro-2-ethoxy-phenyl)-ureido]-phenyl}-piperazine-1-carboxylic acid tert-butyl ester (0.855 g) obtained in Example 43 as a light brown solid in the same manner as in Example (47a).
MS(FAB) m/z:375 $(M + H)^+$.

(102b) 4-{4-[3-(5-chloro-2-ethoxy-phenyl)-ureido]-phenyl}-piperazine-1-carboxylic acid (2-trifluoromethyl-phenyl)-amide

101 mg (90%) of the title compound was obtained from 1-(5-chloro-2-ethoxyphenyl)-3-(4-piperazin-1-yl-phenyl)-urea (75 mg) obtained in Example (102a) and 2-trifluoromethylphenyl isocyanate (0.045 mL) as a light brown powder in the same manner as in Example 49.
$^1$H-NMR spectrum (400MHz,DMSO-d6):δ(ppm)=9.25(1H, s), 8.32(1H, s), 8.22(1H, d, J=2. 8Hz), 8.11(1H, s), 7.67(1H, d, J=7.9Hz), 7.63(1H, dd, J=7.9 and 7.8Hz), 7.43(1H, d, J=7.8Hz), 7.40(1H, dd, J=8.1 and 8.1Hz), 7.32(2H, d, J=9.0Hz), 7.00-6.90(4H, m), 4.13(2H, q, J=7.1Hz), 3.57(4H, t, J=4.9Hz), 3.08(4H, t, J=4.9Hz), 1.41(3H, t, J=7.0Hz).
MS(FAB) m/z:562 $(M + H)^+$.
Melting point: 219-221˚C.

(Example 103) 4-{4-[3-(5-chloro-2-ethoxy-phenyl)-ureido]-phenyl}-piperazine-1-carboxylic acid (2-chloro-6-methyl-phenyl)-amide (Compound No. 1-46)

98 mg (91%) of the title compound was obtained from 1-(5-chloro-2-ethoxyphenyl)-3-(4-piperazin-1-yl-phenyl)-urea (75 mg) obtained in Example (102a) and 2-chloro-6-methylphenyl isocyanate (0.041 mL) as a light brown powder in the same manner as in Example 49.
$^1$H-NMR spectrum (400MHz,DMSO-d6):δ(ppm)=9.25(1H, s), 8.22(1H, d, J=2.8Hz), 8.21(1H, s), 8.11(1H, s), 7.32(2H, d, J=9.0Hz), 7.30(1H, dd, J=7.1 and 1.6Hz), 7.18(1H, d, J=6.6Hz), 7.14(1H, dd, J=7.8 and 7.8Hz), 6.99(1H, d, J=8.6Hz), 6.95(2H, d, J=9.4Hz), 6.92(1H, dd, J=8.6 and 2.4Hz), 4.13 (2H, q, J=7.1Hz), 3.60(4H, t, J=4.1Hz), 3.09(4H, t, J=4.7Hz), 2.20(3H, s), 1.41(3H, t, J=7.0Hz).
MS(FAB) m/z:542 $(M + H)^+$.
Melting point: 159-161˚C.

(Example 104) 4-{4-[3-(5-chloro-2-ethoxy-phenyl)-ureido]-phenyl}-piperazine-1-carboxylic acid (2,6-dimethyl-phenyl)-amide (Compound No. 1-60)

95 mg (91%) of the title compound was obtained from 1-(5-chloro-2-ethoxyphenyl)-3-(4-piperazin-1-yl-phenyl)-urea (75 mg) obtained in Example (102a) and 2,6-dimethylphenyl isocyanate (0.042 mL) as a brownish grey powder in the same manner as in Example 49.
$^1$H-NMR spectrum (400MHz,DMSO-d6):δ(ppm)=9.25(1H, s), 8.22(1H, d, J=2.4Hz), 8.11(1H, s), 7.95(1H, s), 7.33(2H, d, J=9.0Hz), 7.02(2H, s), 7.03-7.00(1H, m), 6.99(1H, d, J=8.6Hz), 6.96(2H, d, J=9.0Hz), 6.92(1H, dd, J=8.6 and 2.8Hz), 4.13(2H, q, J=6.9Hz), 3.60(4H, t, J=4.7Hz), 3.08(4H, t, J=4.7Hz), 2.15(6H, s), 1.41(3H, t, J=7.0Hz).
MS(FAB) m/z:522 $(M + H)^+$.
Melting point: 144-146˚C.

(Example 105) 4-{4-[3-(5-chloro-2-ethoxy-phenyl)-ureido]-phenyl}-piperazine-1-carboxylic acid (2-fluoro-6-trifluoromethyl-phenyl)-amide (Compound No. 1-18)

101 mg (87%) of the title compound was obtained from 1-(5-chloro-2-ethoxyphenyl)-3-(4-piperazin-1-yl-phenyl)-urea (75 mg) obtained in Example (102a) and 2-fluoro-6-trifluoromethylphenyl isocyanate (0.043 mL) as a greyish purple powder in the same manner as in Example 49.
$^1$H-NMR spectrum (400MHz,DMSO-d6):δ(ppm)=9.25(1H, s), 8.33(1H, s), 8.22(1H, d, J=2.8Hz), 8.11(1H, s), 7.63-7.51 (3H, m), 7.33(2H, d, J=9.0Hz), 6.99(1H, d, J=8.6Hz), 6.95(2H, d, J=9.4Hz), 6.92(1H, dd, J=8.8 and 2.6Hz), 4.13(2H, q, J=7.1Hz), 3.58(4H, t, J=4.9Hz), 3.07(4H, t, J=4.9Hz), 1.41(3H, t, J=6.8Hz).
MS(FAB) m/z:580 $(M + H)^+$.
Melting point: 146-148˚C.

(Example 106) 4-{4-[3-(5-chloro-2-ethoxy-phenyl)-ureido]-phenyl}-piperazine-1-carboxylic acid (4-methoxy-2-methyl-phenyl)-amide (Compound No. 1-144)

98 mg (91%) of the title compound was obtained from 1-(5-chloro-2-ethoxyphenyl)-3-(4-piperazin-1-yl-phenyl)-urea (75 mg) obtained in Example (102a) and 4-methoxy-2-methylphenyl isocyanate (0.044 mL) as a greyish purple powder in the same manner as in Example 49.
$^1$H-NMR spectrum (400MHz,DMSO-d6):δ(ppm)=9.25(1H, s), 8.22(1H, d, J=2.8Hz), 8.11(1H, s), 8.02(1H, s), 7.32(2H, d, J=9.0Hz), 7.0 2 (1H, d, J=8.6Hz), 6.99(1H, d, J=9.0Hz), 6.94(2H, d, J=8.6Hz), 6.91(1H, dd, J=8.6 and 2.8Hz), 6.76 (1H, d, J=2.7Hz), 6.68(1H, dd, J=8.5 and 3.0Hz), 4.13(2H, q, J=7.1Hz), 3.71(3H, s), 3.56(4H, t, J=4.7Hz), 3.08(4H, t, J=4.9Hz), 2.13(3H, s), 1.41(3H, t, J=6.8Hz).
MS(FAB) m/z:538 $(M + H)^+$.
Melting point: 210-213˚C.

(Example 107) 4-{4-[3-(5-chloro-2-ethoxy-phenyl)-ureido]-phenyl}-piperazine-1-carboxylic acid (2-chloro-4-methyl-phenyl)-amide (Compound No. 1-116)

100 mg (92%) of the title compound was obtained from 1-(5-chloro-2-ethoxyphenyl)-3-(4-piperazin-1-yl-phenyl)-urea (75 mg) obtained in Example (102a) and 2-chloro-4-methylphenyl isocyanate (53 mg) as a light brown powder in the same manner as in Example 49.
$^1$H-NMR spectrum (400MHz,DMSO-d6):δ(ppm)=9.25(1H, s), 8.22(1H, d, J=2.4Hz), 8.21(1H, s), 8.11(1H, s), 7.33(1H, d, J=7.9Hz), 7.32(2H, d, J=9.0Hz), 7.27(1H, d, J=1.2Hz), 7.08(1H, dd, J=8.5 and 1.3Hz), 6.99(1H, d, J=8.6Hz), 6.95(2H, d, J=9.0Hz), 6.92(1H, dd, J=8.6 and 2.8Hz), 4.13(2H, q, J=7.1Hz), 3.58(4H, t, J=4.6Hz), 3.09(4H, t, J=4.6Hz), 2.28 (3H, s), 1.40(3H, t, J=7.1Hz).
MS(FAB) m/z:542 $(M + H)^+$.
Melting point: 124-126˚C.

(Example 108) 4-{4-[3-(2-methoxy-5-methyl-phenyl)-ureido]-phenyl}-piperazine-1-carboxylic acid (2,3-dimethyl-phenyl)-amide (Compound No. 1-373)

93 mg (95%) of the title compound was obtained from 1-(2-methoxy-5-methyl-phenyl)-3-(4-piperazin-1-yl-phenyl)-urea (68 mg) obtained in Example (47a) and 2,3-dimethylphenyl isocyanate (0.042 mL) as a pale orange powder in the same manner as in Example 49.
$^1$H-NMR spectrum (400MHz,DMSO-d6):δ(ppm)=9.10(1H, s), 8.19(1H, s), 8.07(1H, s), 7.99(1H, d, J=2.3Hz), 7.34(2H, d, J=8.9Hz), 7.02-6.92(4H, m), 6.88(2H, d, J=8.2Hz), 6.73(1H, dd, J=8.0 and 1.8Hz), 3.84(3H, s), 3.58(4H, t, J=2.8Hz), 3.08(4H, t, J=4.9Hz), 2.24(3H, s), 2.23(3H, s), 2.04(3H, s).
MS(FAB) m/z:488 $(M + H)^+$.
Melting point: 217-219˚C.

(Example 109) 4-{4-[3-(5-chloro-2-methoxy-phenyl)-ureido]-phenyl}-piperazine-1-carboxylic acid (4-methoxy-2-methyl-phenyl)-amide (Compound No. 1-143)

95 mg (90%) of the title compound was obtained from 1-(5-chloro-2-methoxy-phenyl)-3-(4-piperazin-1-yl-phenyl)-urea (72 mg) obtained in Example (67a) and 4-methoxy-2-methylphenyl isocyanate (0.047 mL) as a pale purple powder in the same manner as in Example 49.
$^1$H-NMR spectrum (400MHz,DMSO-d6):δ(ppm)=9.15(1H, s), 8.27(1H, s), 8.22(1H, d, J=2.8Hz), 8.01(1H, s), 7.30(2H, d, J=9.0Hz), 7.02(2H, dd, J=8.6 and 8.6Hz), 6.96-6.92(3H, m), 6.75(1H, d, J=3.1Hz), 6.68(1H, dd, J=9.0 and 2.8Hz), 3.87(3H, s), 3.71(3H, s), 3.56(4H, t, J=4.9Hz), 3.06(4H, t, J=4.9Hz), 2.13(3H, s).
MS(FAB) m/z:524 $(M + H)^+$.
Melting point: 225-226˚C.

(Example 110) 4-{4-[3-(2-ethoxy-phenyl)-ureido]-phenyl}-piperazine-1-carboxylic acid (4-methoxy-2-methyl-phenyl)-amide (Compound No. 1-147)

91 mg (90%) of the title compound was obtained from 1-(2-ethoxy-phenyl)-3-(4-piperazin-1-yl-phenyl)-urea (68 mg) obtained in Example (80a) and 4-methoxy-2-methylphenyl isocyanate (0.044 mL) as a pale orange powder in the same manner as in Example 49.
$^1$H-NMR spectrum (400MHz,DMSO-d6):δ(ppm)=9.15(1H, s), 8.10(1H, dd, J=7.4 and 1.9Hz), 8.01(1H, s), 7.95(1H, s), 7.32(2H, d, J=9.0Hz), 7.03(1H, d, J=8.6Hz), 6.97(1H, dd, J=7.8 and 2.0Hz), 6.93(2H, d, J=9.0Hz), 6.89(1H, ddd, J=7.5, 7.5 and 2.1Hz), 6.85(1H, ddd, J=7.7, 7.7 and 1.8Hz), 6.75(1H, d, J=2.7Hz), 6.68(1H, dd, J=8.2 and 3.2Hz), 4.12(2H, q, J=7.1Hz), 3.71(3H, s), 3.56(4H, t, J=4.9Hz), 3.06(4H, t, J=4.7Hz), 2.13(3H, s), 1.41(3H, t, J=7.1Hz).
MS(FAB) m/z:504 $(M + H)^+$.
Melting point: 202-203˚C.

(Example 111) 4-{4-[3-(2-ethoxy-5-methyl-phenyl)-ureido]-phenyl}-piperazine-1-carboxylic acid (4-methoxy-2-methyl-phenyl)-amide (Compound No. 1-146)

84 mg (82%) of the title compound was obtained from 1-(2-ethoxy-5-methylphenyl)-3-(4-piperazin-1-yl-phenyl)-urea (71 mg) obtained in Example (83a) and 4-methoxy-2-methylphenyl isocyanate (0.044 mL) as a pale purple powder in the same manner as in Example 49.
$^1$H-NMR spectrum (400MHz,DMSO-d6):δ(ppm)=9.14(1H, s), 8.02(1H, s), 7.96(1H, d, J=2.3Hz), 7.89(1H, s), 7.32(2H, d, J=9.0Hz), 7.03(1H, d, J=8.6Hz), 6.94(2H, d, J=9.0Hz), 6.85(1H, d, J=8.2Hz), 6.76(1H, d, J=2.7Hz), 6.68(2H, dd, J=8.2 and 2.4Hz), 4.07(2H, q, J=6.9Hz), 3.71(3H, s), 3.56(4H, t, J=4.9Hz), 3.07(4H, t, J=4.3Hz), 2.21(3H, s), 2.13(3H, s), 1.38 (3H, t, J=7.1Hz).
MS(FAB) m/z:518 $(M + H)^+$.
Melting point: 202-204˚C.

(Example 112) 4-{4-[3-(2-ethoxy-5-fluoro-phenyl)-ureido]-phenyl}-piperazine-1-carboxylic acid (4-methoxy-2-methyl-phenyl)-amide (Compound No. 1-142)

90 mg (89%) of the title compound was obtained from 1-(2-ethoxy-5-fluorophenyl)-3-(4-piperazin-1-yl-phenyl)-urea (72 mg) obtained in Example (89a) and 4-methoxy-2-methylphenyl isocyanate (0.044 mL) as a pale pink powder in the same manner as in Example 49.
$^1$H-NMR spectrum (400MHz,DMSO-d6):δ(ppm)=9.30(1H, s), 8.16(1H, s), 8.02(1H, d, J=2.8Hz), 7.99(1H, d, J=3.1Hz), 7.32(2H, d, J=9.0Hz), 7.03(1H, d, J=8.2Hz), 6.97(1H, dd, J=8.8 and 5.3Hz), 6.94(2H, d, J=9.4Hz), 6.76(1H, d, J=3.1Hz), 6.71-6.65(2H, m), 4.10(2H, q, J=7.1Hz), 3.71(3H, s), 3.56(4H, t, J=4.7Hz), 3.07(4H, t, J=4.9Hz), 2.13(3H, s), 1.40(3H, t, J=6.8Hz).
MS(FAB) m/z:522 $(M + H)^+$.
Melting point: 186-189˚C.

(Example 113) 4-{4-[3-(5-fluoro-2-methoxy-phenyl)-ureido]-phenyl}-piperazine-l-carboxylic acid (4-methoxy-2-methyl-phenyl)-amide (Compound No. 1-141)

90 mg (89%) of the title compound was obtained from 1-(5-fluoro-2-methoxy-phenyl)-3-(4-piperazin-1-yl-phenyl)-urea (69 mg) obtained in Example (96a) and 4-methoxy-2-methylphenyl isocyanate (0.044 mL) as a pale pink powder in the same manner as in Example 49.
$^1$H-NMR spectrum (400MHz,DMSO-d6):δ(ppm)=9.16(1H, s), 8.30(1H, s), 8.02(1H, d, J=3.1Hz), 7.99(1H, d, J=3.1Hz), 7.31(2H, d, J=9.0Hz), 7.03(1H, d, J=8.6Hz), 6.98(1H, dd, J=8.8 and 5.3Hz), 6.93(2H, d, J=9.0Hz), 6.75(1H, d, J=2.7Hz), 6.73-6.67(2H, m), 3.86(3H, s), 3.71(3H, s), 3.56(4H, t, J=4.9Hz), 3.06(4H, t, J=4.9Hz), 2.13(3H, s).
MS(FAB) m/z:508 $(M + H)^+$.
Melting point: 216-218˚C.

(Example 114) 4-{4-[3-(5-chloro-2-methoxy-phenyl)-ureido]-phenyl}-piperazine-1-carboxylic acid (2,6-dimethyl-phenyl)-amide (Compound No. 1-59)

90 mg (89%) of the title compound was obtained from 1-(5-chloro-2-methoxy-phenyl)-3-(4-piperazin-1-yl-phenyl)-urea (72 mg) obtained in Example (67a) and 2,6-dimethylphenyl isocyanate (0.042 mL) as a pale purple powder in the same manner as in Example 49.
$^1$H-NMR spectrum (400MHz,DMSO-d6):δ(ppm)=9.15(1H, s), 8.28(1H, s), 8.22(IH, d, J=2.4Hz), 7.94(1H, s), 7.31 (2H, d, J=9.OHz), 7.03(2H, s), 7.01 (2H, d, J=8.6Hz), 6.95(1H, d, J=8.7Hz), 6.94(2H, d, J=8.2Hz), 3.88(3H, s), 3.59(4H, t, J=4.9Hz), 3.07(4H, t, J=4.9Hz), 2.14(6H, s).
MS(FAB) m/z:508 $(M + H)^+$.
Melting point: 216-218˚C.

(Example 115) 4-{4-[3-(5-chloro-2-methoxy-phenyl)-ureido]-phenyl}-piperazine-1-carboxylic acid (2-fluoro-6-trifluoromethyl-phenyl)-amide (Compound No. 1-17)

97 mg (86%) of the title compound was obtained from 1-(5-chloro-2-methoxy-phenyl)-3-(4-piperazin-1-yl-phenyl)-urea (72 mg) obtained in Example (67a) and 2-fluoro-6-trifluoromethylphenyl isocyanate (0.043 mL) as a grey white powder in the same manner as in Example 49.
$^1$H-NMR spectrum (400MHz,DMSO-d6):δ(ppm)=9.17(1H, s), 8.33(1H, s), 8.28(1H, s), 8.22(1H, d, J=2.4Hz), 7.63-7.51 (3H, m), 7.31(2H, d, J=9.4Hz), 7.00(1H, d, J=8.6Hz), 6.95(1H, d, J=8.6Hz), 6.94(2H, d, J=9.4Hz), 3.87(3H, s), 3.58(4H, t, J=4.7Hz), 3.06(4H, t, J=4.7Hz).
MS(FAB) m/z:566 $(M + H)^+$.
Melting point: 156-158˚C.

(Example 116) 4-{4-[3-(2-ethoxy-phenyl)-ureido]-phenyl}-piperazine-1-carboxylic acid (2,6-dimethyl-phenyl)-amide (Compound No. 1-63)

90 mg (93%) of the title compound was obtained from 1-(2-ethoxy-phenyl)-3-(4-piperazin-1-yl-phenyl)-urea (68 mg) obtained in Example (80a) and 2,6-dimethylphenyl isocyanate (0.042 mL) as a pale pink powder in the same manner as in Example 49.
$^1$H-NMR spectrum (400MHz,DMSO-d6):δ(ppm)=9.15(1H, s), 8.10(1H, dd, J=7.9 and 2.0Hz), 7.95(2H, d, J=4.6Hz), 7.32 (2H, d, J=9.0Hz), 7.04-7.02(1H, m), 7.02(2H, s), 6.97(1H, dd, J=7.8 and 1.6Hz), 6.94(2H, d, J=9.0Hz), 6.89 (1H, ddd, J=7.7, 7.7 and 2.1Hz), 6.85(1H, ddd, J=7.6, 7.6 and 1.7Hz), 4.12(2H, q, J=7.1Hz), 3.59(4H, t, J=4.6Hz), 3.07(4H, t,

J=4.9Hz), 2.15(6H, s), 1.41(3H, t, J=6.9Hz).
MS(FAB) m/z:488 $(M + H)^+$.
Melting point: 204-205˚C.

(Example 117) 4-{4-[3-(2-ethoxy-phenyl)-ureido]-phenyl}-piperazine-1-carboxylic acid (2-fluoro-6-trifluoromethyl-phenyl)-amide (Compound No. 1-21)

104 mg (93%) of the title compound was obtained from 1-(2-ethoxy-phenyl)-3-(4-piperazin-1-yl-phenyl)-urea (68 mg) obtained in Example (80a) and 2-fluoro-6-trifluoromethylphenyl isocyanate (0.043 mL) as a yellow powder in the same manner as in Example 49.
$^1$H-NMR spectrum (400MHz,DMSO-d6):δ(ppm)=9.15(1H, s), 8.32(1H, s), 8.10(1H, dd, J=7.7 and 2.2Hz), 7.95(1H, s), 7.62-7.49(3H, m), 7.32(2H, d, J=9.0Hz), 6.97(1H, dd, J=8.0 and 1.8Hz), 6.93(2H, d, J=9.0Hz), 6.89(1H, ddd, J=7.6, 7.6 and 2.0Hz), 6.85(1H, ddd, J=7.6, 7.6 and 1.5Hz), 4.12(2H, q, J=6.9Hz), 3.58(4H, t, J=4.9Hz), 3.06(4H, t, J=4.5Hz), 1.41 (3H, t, J=6.9Hz).
MS(FAB) m/z:546 $(M + H)^+$.
Melting point: 139-141˚C.

(Example 118) 4-{4-[3-(2-ethoxy-5-methyl-phenyl)-ureido]-phenyl}-piperazine-1-carboxylic acid (2,6-dimethyl-phenyl)-amide (Compound No. 1-62)

88 mg (87%) of the title compound was obtained from 1-(2-ethoxy-5-methylphenyl)-3-(4-piperazin-1-yl-phenyl)-urea (71 mg) obtained in Example (83a) and 2,6-dimethylphenyl isocyanate (0.042 mL) as a pale pink powder in the same manner as in Example 49.
$^1$H-NMR spectrum (400MHz,DMSO-d6):δ(ppm)=9.14(1H, s), 7.96(1H, d, J=2.4Hz), 7.94(1H, s), 7.90(1H, s), 7.33(2H, d, J=9.4Hz), 7.04-7.02(3H, m), 6.94(2H, d, J=9.0Hz), 6.85(1H, d, J=8.2Hz), 6.68(1H, dd, J=8.2 and 2.4Hz), 4.07(2H, q, J=6.9Hz), 3.60(4H, t, J=4.7Hz), 3.08(4H, t, J=4.7Hz), 2.21(3H, s), 2.15(6H, s), 1.38(3H, t, J=6.9Hz).
MS(FAB) m/z:502 $(M + H)^+$.
Melting point: 222-225˚C.

(Example 119) 4-{4-[3-(2-ethoxy-5-methyl-phenyl)-ureido]-phenyl}-piperazine-1-carboxylic acid (2-fluoro-6-trifluoromethyl-phenyl)-amide (Compound No. 1-20)

101 mg (91%) of the title compound was obtained from 1-(2-ethoxy-5-methylphenyl)-3-(4-piperazin-1-yl-phenyl)-urea (71 mg) obtained in Example (83a) and 2-fluoro-6-trifluoromethylphenyl isocyanate (0.043 mL) as a pale pink powder in the same manner as in Example 49.
$^1$H-NMR spectrum (400MHz,DMSO-d6):δ(ppm)=9.15(1H, s), 8.33(1H, s), 7.96(1H, d, J=2.4Hz), 7.90(1H, s), 7.63-7.51 (3H, m), 7.33(2H, d, J=9.0Hz), 6.94(2H, d, J=9.0Hz), 6.85(1H, d, J=8.2Hz), 6.68(1H, dd, J=9.0 and 2.0Hz), 4.07(2H, q, J=7.1Hz), 3.58(4H, t, J=4.6Hz), 3.07(4H, t, J=4.5Hz), 2.21(3H, s), 1.38(3H, t, J=6.9Hz).
MS(FAB) m/z:560 $(M + H)^+$.
Melting point: 144-146˚C.

(Example 120) 4-{4-[3-(2-ethoxy-5-methyl-phenyl)-ureido]-phenyl}-piperazine-1-carboxylic acid (2-chloro-4-methyl-phenyl)-amide (Compound No. 1-118)

88 mg (84%) of the title compound was obtained from 1-(2-ethoxy-5-methylphenyl)-3-(4-piperazin-1-yl-phenyl)-urea (71 mg) obtained in Example (83a) and 2-chloro-4-methylphenyl isocyanate (50 mg) as a pale pink powder in the same manner as in Example 49.
$^1$H-NMR spectrum (400MHz,DMSO-d6):δ(ppm)=9.17(1H, s), 8.24(1H, s), 7.99(1H, s), 7.93(1H, s), 7.35(3H, d, J=7.8Hz), 7.30(1H, s), 7.11(1H, d, J=8.6Hz), 6.96(2H, d, J=9.0Hz), 6.87(1H, d, J=8.2Hz), 6.71(1H, d, J=8.6Hz), 4.08(2H, q, J=7.1Hz), 3.60(4H, brs), 3.10(4H, brs), 2.28(3H, s), 2.22(3H, s), 1.39(3H, t, J=6.8Hz).
MS(FAB) m/z:522 $(M + H)^+$.
Melting point: 119-122˚C.

(Example 121) 4-{4-[3-(2-ethoxy-phenyl)-ureido]-phenyl}-piperazine-1-carboxylic acid (2-chloro-4-methyl-phenyl)-amide (Compound No. 1-119)

90 mg (88%) of the title compound was obtained from 1-(2-ethoxy-phenyl)-3-(4-piperazin-1-yl-phenyl)-urea (68 mg) obtained in Example (80a) and 2-chloro-4-methylphenyl isocyanate (50 mg) as a pale yellow powder in the

same manner as in Example 49.
$^1$H-NMR spectrum (400MHz,DMSO-d6):δ(ppm)=9.20(1H, s), 8.24(1H, s), 8.13(1H, d, J=7.9Hz), 7.99(1H, s), 7.35(2H, d, J=7.8Hz), 7.35(1H, d, J=9.4Hz), 7.30(1H, s), 7.10(1H, d, J=8.6Hz), 7.01-6.85(5H, m), 4.13(2H, q, J=6.6Hz), 3.59(4H, t, J=5.3Hz), 3.09(4H, t, J=4.3Hz), 2.28(3H, s), 1.41(3H, t, J=6.9Hz).
MS(FAB) m/z:508 $(M + H)^+$.
Melting point: 179-181˚C.

(Example 122) 4-{4-[3-(2-ethoxy-5-fluoro-phenyl)-ureido]-phenyl}-piperazine-1-carboxylic acid (2-chloro-4-methyl-phenyl)-amide (Compound No. 1-114)

96 mg (91%) of the title compound was obtained from 1-(2-ethoxy-5-fluorophenyl)-3-(4-piperazin-1-yl-phenyl)-urea (72 mg) obtained in Example (89a) and 2-chloro-4-methylphenyl isocyanate (50 mg) as a yellowish brown powder in the same manner as in Example 49.
$^1$H-NMR spectrum (400MHz,DMSO-d6):δ(ppm)=9.34(1H, s), 8.25(1H, brs), 8.19(1H, s), 8.03(1H, dd, J=12.3 and 2.6Hz), 7.35(3H, d, J=9.4Hz), 7.29(1H, s), 7.10(1H, d, J=8.2Hz), 6.99(1H, dd, 9.0Hz and 5.0Hz), 6.95(2H, d, J=8.6Hz), 6.70 (1H, ddd, J=8.1, 8.1 and 3.0Hz), 4.12(2H, q, J=7.1Hz), 3.59(4H, t, J=4.5Hz), 3.09(4H, t, J=4.5Hz), 2.29(3H, s), 1.40(3H, t, J=6.8Hz).
MS(FAB) m/z:526 $(M + H)^+$.
Melting point: 155-157˚C.

(Example 123) 4-{4-[3-(3-fluoro-2-methoxy-phenyl)-ureido]-phenyl}-piperazine-1-carboxylic acid (2-chloro-4-methyl-phenyl)-amide (Compound No. 1-113)

94 mg (92%) of the title compound was obtained from 1-(5-fluoro-2-methoxy-phenyl)-3-(4-piperazin-1-yl-phenyl)-urea (69 mg) obtained in Example (96a) and 2-chloro-4-methylphenyl isocyanate (50 mg) as a pale pink powder in the same manner as in Example 49.
$^1$H-NMR spectrum (400MHz,DMSO-d6):δ(ppm)=9.21(1H, s), 8.34(1H, s), 8.24(1H, s), 8.03(1H,dd, J=11.8 and 3.1Hz), 7.35(1H,d, J=7.4Hz), 7.33(2H,d, J=8.6Hz), 7.28(1H, s), 7.10(1H, dd, J=8.4 and 1.8Hz), 7.00(1H, dd, J=8.8 and 5.3Hz), 6.96(2H, d, J=8.6Hz), 6.73(1H, ddd, J=8.4, 8.4 and 3.8Hz), 3.87(3H, s), 3.59(4H, t, J=4.9Hz), 3.09(4H, t, J=4.7Hz), 2.28 (3H, s).
MS(FAB) m/z:512 $(M + H)^+$.
Melting point: 233-235˚C.

(Example 124) 4-{4-[3-(2-methoxy-phenyl)-ureido]-phenyl}-piperazine-1-carboxylic acid (2-chloro-6-methyl-phenyl)-amide (Compound No. 1-378)

(124a) 1-(2-methoxy-phenyl)-3-(4-piperazin-1-yl-phenyl)-urea

0.467 g (96%) of the title compound was obtained from 4-{4-[3-(2-methoxy-phenyl)-ureido]-phenyl}-piperazine-1-carboxylic acid tert-butyl ester (0.640 g) obtained in Example 13 as a light brown powder in the same manner as in Example (47a).
MS(FAB) m/z:327 $(M + H)^+$.

(124b) 4-{4-[3-(2-methoxy-phenyl)-ureido]-phenyl}-piperazine-1-carboxylic acid (2-chloro-6-methyl-phenyl)-amide

78 mg (79%) of the title compound was obtained from 1-(2-methoxy-phenyl)-3-(4-piperazin-1-yl-phenyl)-urea (65 mg) obtained in Example (124a) and 2-chloro-6-methylphenyl isocyanate (0.041 mL) as a brown powder in the same manner as in Example 49.
$^1$H-NMR spectrum (400MHz,DMSO-d6):δ(ppm)=9.11(1H, s), 8.24(1H, s), 8.13(1H, dd, J=7.6 and 1.8Hz), 8.13(1H, s), 7.33(3H, d, J=9.0Hz), 7.22(1H, d, J=6.6Hz), 7.17(1H, dd, J=15.2 and 7.4Hz), 7.01(1H, dd, J=7.8 and 1.2Hz), 6.97-6.87 (2H, m), 6.95(2H, d, J=8.9Hz), 3.88(3H, s), 3.61(4H, t, J=4.3Hz), 3.08(4H, t, J=4.6Hz), 2.20(3H, s).
MS(FAB) m/z:494 $(M + H)^+$.
Melting point: 245-247˚C.

(Example 125) 4-{4-[3-(2-fluoro-phenyl)-ureido]-phenyl}-piperazine-1-carboxylic acid (2-chloro-6-methyl-phenyl)-amide (Compound No. 1-379)

(125a) 1-(2-fluoro-phenyl)-3-(4-piperazin-1-yl-phenyl)-urea

0.471 g (100%) of the title compound was obtained from 4-{4-[3-(2-fluorophenyl)-ureido]-phenyl}-piperazine-1-carboxylic acid tert-butyl ester (0.622 g) obtained in Example 14 as a white powder in the same manner as in Example (47a).
MS(FAB) m/z:315 $(M + H)^+$.

(125b) 4-{4-[3-(2-fluoro-phenyl)-ureido]-phenyl}-piperazine-1-carboxylic acid (2-chloro-6-methyl-phenyl)-amide

80 mg (83%) of the title compound was obtained from 1-(2-fluoro-phenyl)-3-(4-piperazin-1-yl-phenyl)-urea (63 mg) obtained in Example (125a) and 2-chloro-6-methylphenyl isocyanate (0.041 mL) as a white powder in the same manner as in Example 49.
$^1$H-NMR spectrum (400MHz,DMSO-d6):δ(ppm)=8.93(1H, s), 8.52(1H, s), 8.24(1H, s), 8.16(1H, ddd, J=8.2, 8.2 and 1.4Hz), 7.34(2H, d, J=9.0Hz), 7.34-7.32(1H, m), 7.25-7.11(4H, m), 7.02-6.97(1H, m), 6.96(2H, d, J=9.OHz), 3.61(4H, t, J=4.6Hz), 3.09(4H, t, J=4.7Hz), 2.20(3H, s).
MS(FAB) m/z:482 $(M + H)^+$.
Melting point: 232-234˚C.

(Example 126) 4-{4-[3-(2-chloro-phenyl)-ureido]-phenyl}-piperazine-1-carboxylic acid (2-chloro-6-methyl-phenyl)-amide (Compound No. 1-380)

(126a) 1-(2-chloro-phenyl)-3-(4-piperazin-1-yl-phenyl)-urea

0.496 g (100%) of the title compound was obtained from 4-{4-[3-(2-chlorophenyl)-ureido]-phenyl}-piperazine-1-carboxylic acid tert-butyl ester (0.646 g) obtained in Example 15 as a light brown powder in the same manner as in Example (47a).
MS(FAB) m/z:331 $(M + H)^+$.

(126b) 4-{4-[3-(2-chloro-phenyl)-ureido]-phenyl}-piperazine-1-carboxylic acid (2-chloro-6-methyl-phenyl)-amide

90 mg (90%) of the title compound was obtained from 1-(2-chloro-phenyl)-3-(4-piperazin-1-yl-phenyl)-urea (66 mg) obtained in Example (126a) and 2-chloro-6-methylphenyl isocyanate (0.041 mL) as a white powder in the same manner as in Example 49.
$^1$H-NMR spectrum (400MHz,DMSO-d6):δ(ppm)=9.26(1H, s), 8.25(1H, s), 8.23(1H, s), 8.18(1H, dd, J=8.6 and 1.6Hz), 7.45(1H, dd, J=8.0 and 1.4Hz), 7.35(2H, d, J=9.0Hz), 7.32(1H, d, J=9.4Hz), 7.28(1H, d, J=7.0Hz), 7.21(1H, d, J=6.6Hz), 7.17(1H, dd, J=15.2 and 7.8Hz), 7.02(1H, ddd, J=7.6, 7.6 and 1.4Hz), 6.97(2H, d, J=9.4Hz), 3.61(4H, t, J=4.5Hz), 3.09 (4H, t, J=4.9Hz), 2.20(3H, s).
MS(FAB) m/z:498 $(M + H)^+$.
Melting point: >260˚C (dec).

(Example 127) 4-[4-(3-o-tolyl-ureido)-phenyl]-piperazine-1-carboxylic acid (2-chloro-6-methyl-phenyl)-amide (Compound No. 1-381)

(127a) 1-(4-piperazin-1-yl-phenyl)-3-o-tolyl-urea

0.451 g (97%) of the title compound was obtained from 4-{4-[3-o-tolyl-ureido]-phenyl}-piperazine-1-carboxylic acid tert-butyl ester (0.616 g) obtained in Example 16 as a light brown powder in the same manner as in Example (47a).
MS(FAB)m/z:311 $(M+H)^+$.

(127b) 4-[4-(3-o-tolyl-ureido)-phenyl]-piperazine-1-carboxylic acid (2-chloro-6-methyl-phenyl)-amide

83 mg (87%) of the title compound was obtained from 1-(4-piperazin-1-yl-phenyl)-3-o-tolyl-urea (62 mg) obtained in Example (127a) and 2-chloro-6-methylphenyl isocyanate (0.041 mL) as a pale pink powder in the same manner as in Example 49.
$^1$H-NMR spectrum (400MHz,DMSO-d6):δ(ppm)=8.90(1H, s), 8.24(1H, s), 7.91(1H, brs), 7.85(1H, d, J=8.2Hz), 7.35(2H,

d, J=9.0Hz), 7.34-7.32(1H, m), 7.23-7.12(4H, m), 6.95(2H, d, J=8.6Hz), 6.94-6.91(1H, m), 3.61(4H, t, J=4.5Hz), 3.08 (4H, t, J=4.7Hz), 2.24(3H, s), 2.20(3H, s).
MS(FAB) m/z:478 $(M + H)^+$.
Melting point: >260˚C (dec).

(Example 128) 4-(4-[3-(2-ethyl-phenyl)-ureido]-phenyl)-piperazine-1-carboxylic acid (2-chloro-6-methyl-phenyl)-amide (Compound No. 1-382)

(128a) 1-(2-ethyl-phenyl)-3-(4-piperazin-1-yl-phenyl)-urea

0.486 g (100%) of the title compound was obtained from 4-{4-[3-(2-ethylphenyl)-ureido]-phenyl}-piperazine-1-carboxylic acid tert-butyl ester (0.637 g) obtained in Example 17 as a white powder in the same manner as in Example (47a).
MS(FAB) m/z:325 $(M + H)^+$.

(128b) 4-{4-[3-(2-ethyl-phenyl)-ureido]-phenyl}-piperazine-1-carboxylic acid (2-chloro-6-methyl-phenyl)-amide

85 mg (87%) of the title compound was obtained from 1-(2-ethyl-phenyl)-3-(4-piperazin-1-yl-phenyl)-urea (65 mg) obtained in Example (128a) and 2-chloro-6-methylphenyl isocyanate (0.041 mL) as a brown powder in the same manner as in Example 49.
$^1$H-NMR spectrum (400MHz,DMSO-d6):δ(ppm)=8.89(1H, s), 8.24(1H, s), 7.89(1H, s), 7.81(1H, d, J=8.2Hz), 7.35(2H, d, J=9.0Hz), 7.34-7.32(1H, m), 7.23-7.12(4H, m), 7.01-6.97(1H, m), 6.95(2H, d, J=8.9Hz), 3.61(4H, t, J=4.5Hz), 3.08 (4H, t, J=4.7Hz), 2.61(2H, q, J=7.4Hz), 2.20(3H, s), 1.17(3H, t, J=7.4Hz).
MS(FAB) m/z:492 $(M + H)^+$.
Melting point: 217-219˚C.

(Example 129) 4-{4-[3-(2-methoxy-5-methyl-phenyl)-ureido]-phenyl}-piperazine-1-carboxylic acid (4-bromo-2-chloro-phenyl)-amide (Compound No. 1-384)

109 mg (95%) of the title compound was obtained from 1-(2-methoxy-5-methyl-phenyl)-3-(4-piperazin-1-yl-phenyl)-urea (68 mg) obtained in Example (47a) and 4-bromo-2-chlorophenyl isocyanate (70 mg) as a grey powder in the same manner as in Example 49.
$^1$H-NMR spectrum (400MHz,DMSO-d6):δ(ppm)=9.08(1H, s), 8.36(1H, brs), 8.05(1H, s), 7.96(1H, s), 7.70(1H, s), 7.47 (2H, s), 7.31(2H, d, J=9.0Hz), 6.92(2H, d, J=9.0Hz), 6.86(1H, d, J=8.2Hz), 6.70(1H, d, J=8.2Hz), 3.81(3H, s), 3.58(4H, t, J=4.7Hz), 3.06(4H, t, J=4.5Hz), 2.20(3H, s).
MS(FAB) m/z:572 $(M + H)^+$.
Melting point: 140-142˚C.

(Example 130) 4- {4-[3-(2-propyl-phenyl)-ureido]-phenyl}-piperazine-1-carboxylic acid (2-chloro-6-methyl-phenyl)-amide (Compound No. 1-389)

(130a) 1-(2-propyl-phenyl)-3-(4-piperazin-1-yl-phenyl)-urea

0.677 g (100%) of the title compound was obtained from 4-{4-[3-(2-propylphenyl)-ureido]-phenyl}-piperazine-1-carboxylic acid tert-butyl ester (0.877 g) obtained in Example 18 as a white powder in the same manner as in Example (47a).
MS(FAB) m/z:339 $(M + H)^+$.

(130b) 4-{4-[3-(2-propyl-phenyl)-ureido]-phenyl}-piperazine-1-carboxylic acid (2-chloro-6-methyl-phenyl)-amide

179 mg (88%) of the title compound was obtained from 1-(2-propyl-phenyl)-3-(4-piperazin-1-yl-phenyl)-urea (135 mg) obtained in Example (130a) and 2-chloro-6-methylphenyl isocyanate (0.082 mL) as a light brown powder in the same manner as in Example 49.
$^1$H-NMR spectrum (400MHz,DMSO-d6):δ(ppm)=8.78(1H, s), 8.21(1H, s), 7.77(1H, d, J=9.4Hz), 7.75(1H, s), 7.32(2H, d, J=9.OHz), 7.33-7.29(1H, m), 7.21-7.10(4H, m), 6.97-6.92(1H, m), 6.93(2H, d, J=9.OHz), 3.60(4H, t, J=4.9Hz), 3.07 (4H, t, J=4.3Hz), 2.55(2H, t, J=7.4Hz), 2.20(3H, s), 1.59-1.54(2H, m), 0.94(3H, t, J=7.4Hz).
MS(FAB) m/z:506 $(M + H)^+$.
Melting point: 216-217˚C.

(Example 131)4-{4-[3-(2,5-dimethyl-phenyl)-ureido]-phenyl}-piperazine-1-carboxylic acid (2-chloro-6-methyl-phenyl)-amide (Compound No. 1-390)

(131a) 1-(2,5-dimethyl-phenyl)-3-(4-piperazin-1-yl-phenyl)-urea

0.649 g (100%) of the title compound was obtained from 4-{4-[3-(2,5-dimethyl-phenyl)-ureido]-phenyl}-piperazine-1-carboxylic acid tert-butyl ester (0.849 g) obtained in Example 19 as a light brownish purple powder in the same manner as in Example (47a).
MS(FAB) m/z:325 $(M + H)^+$.

(131b) 4-{4-[3-(2,5-dimethyl-phenyl)-ureido]-phenyl}-piperazine-1-carboxylic acid (2-chloro-6-methyl-phenyl)-amide

81 mg (83%) of the title compound was obtained from 1-(2,5-dimethylphenyl)-3-(4-piperazin-1-yl-phenyl)-urea (65 mg) obtained in Example (131a) and 2-chloro-6-methylphenyl isocyanate (0.041 mL) as a yellowish brown powder in the same manner as in Example 49.
$^1$H-NMR spectrum (400MHz,DMSO-d6):δ(ppm)=8.80(1H, s), 8.21(1H, s), 7.76(1H, s), 7.68(1H, s), 7.32(2H, d, J=9.0Hz), 7.32-7.29(1H, m), 7.19(1H, d, J=6.2Hz), 7.15(1H, dd, J=15.2 and 7.4Hz), 7.01(1H, d, J=7.9Hz), 6.93(2H, d, J=9.0Hz), 6.72(1H, dd, J=7.4 and 1.9Hz), 3.60(4H, t, J=5.1Hz), 3.07(4H, t, J=4.3Hz), 2.24(3H, s), 2.20(3H, s), 2.18(3H, s).
MS(FAB) m/z:492 $(M + H)^+$.
Melting point: 251-253˚C.

(Example 132) 4- {4-[3-(5-chloro-2-methoxy-phenyl)-ureido]-phenyl}-piperazine-1-carboxylic acid (2,6-dichloro-phenyl)-amide (Compound No. 1-31)

94 mg (86%) of the title compound was obtained from 1-(5-chloro-2-methoxy-phenyl)-3-(4-piperazin-1-yl-phenyl)-urea (72 mg) obtained in Example (67a) and 2,6-dichlorophenyl isocyanate (56 mg) as a grey white powder in the same manner as in Example 49.
$^1$H-NMR spectrum (400MHz,DMSO-d6):δ(ppm)=9.16(1H, s), 8.51(1H, s), 8.27(1H, s), 8.22(1H, d, J=2.0Hz), 7.49(2H, d, J=7.8Hz), 7.30(2H, dd, J=8.8 and 8.8Hz), 7.31-7.26(1H, m), 7.02-6.93(2H, m), 6.94(2H, d, J=11.0Hz), 3.87(3H, s), 3.60(4H, t, J=5.1Hz), 3.09(4H, t, J==5.1Hz).
MS(FAB) m/z:548 $(M + H)^+$.
Melting point: 189-190˚C.

(Example 133) 4-{4-[3-(2-ethoxy-phenyl)-ureido]-phenyl}-piperazine-1-carboxylic acid (2,6-dichloro-phenyl)-amide (Compound No. 1-35)

96 mg (90%) of the title compound was obtained from 1-(2-ethoxy-phenyl)-3-(4-piperazin-1-yl-phenyl)-urea (68 mg) obtained in Example (80a) and 2,6-dichlorophenyl isocyanate (56 mg) as a pale yellowish green powder in the same manner as in Example 49.
$^1$H-NMR spectrum (400MHz,DMSO-d6):δ(ppm)=9.16(1H, s), 8.51(1H, s), 8.10(1H, dd, J=7.5 and 2.4Hz), 7.96(1H, s), 7.49(2H, d, J=8.2Hz), 7.32(2H, d, J=9.0Hz), 7.27(1H, dd, J=8.6 and 7.9Hz), 6.99-6.93(1H, m), 6.94(2H, d, J=9.0Hz), 6.89(1H, ddd, J=7.8, 7.8 and 2.2Hz), 6.85(1H, ddd, J=7.6, 7.6 and 1.5Hz), 4.12(2H, q, J=6.9Hz), 3.60(4H, t, J=4.7Hz), 3.08(4H, t, J=4.8Hz), 1.41(3H, t, J=6.9Hz).
MS(FAB) m/z:528 $(M + H)^+$.
Melting point: 164-166˚C.

(Example 134) 4-{4-[3-(2-ethoxy-5-methyl-phenyl)-ureido]-phenyl}-piperazine-1-carboxylic acid (2,6-dichloro-phenyl)-amide (Compound No. 1-34)

91 mg (84%) of the title compound was obtained from 1-(2-ethoxy-5-methylphenyl)-3-(4-piperazin-1-yl-phenyl)-urea (71 mg) obtained in Example (83a) and 2,6-dichlorophenyl isocyanate (56 mg) as a pale purple powder in the same manner as in Example 49.
$^1$H-NMR spectrum (400MHz,DMSO-d6):δ(ppm)=9.15(1H, s), 8.51(1H, s), 7.96(1H, d, J=2.0Hz), 7.90(1H, s), 7.49(2H, d, J=8.2Hz), 7.33(2H, d, J=8.6Hz), 7.28(1H, dd, J=8.0 and 8.0Hz), 6.95(2H, d, J=9.4Hz), 6.85(1H, d, J=8.2Hz), 6.69 (1H, dd, J=9.0 and 2.8Hz), 4.07(2H, q, J=7.1Hz), 3.61(4H, t, J=5.5Hz), 3.09(4H, t, J=4.5Hz), 2.21(3H, s), 1.38(3H, t, J=6.9Hz).
MS(FAB) m/z:542 $(M + H)^+$.
Melting point: 159-161˚C.

(Example 135) 4-{4-[3-(5-chloro-2-ethoxy-phenyl)-ureido]-phenyl}-piperazine-1-carboxylic acid (2,6-dichloro-phenyl)-amide (Compound No. 1-32)

104 mg (92%) of the title compound was obtained from 1-(5-chloro-2-ethoxyphenyl)-3-(4-piperazin-1-yl-phenyl)-urea (75 mg) obtained in Example (102a) and 2,6-dichlorophenyl isocyanate (56 mg) as a pale purple powder in the same manner as in Example 49.
$^1$H-NMR spectrum (400MHz,DMSO-d6):δ(ppm)=9.26(1H, s), 8.51(1H, s), 8.22(1H, d, J=2.4Hz), 8.11(1H, s), 7.49(2H, d, J=8.2Hz), 7.33(2H, d, J=8.6Hz), 7.28(1H, dd, J=8.2 and 8.2Hz), 7.00(1H, d, J=9.0Hz), 6.95(2H, d, J=9.0Hz), 6.91 (1H, dd, J=8.8 and 2.6Hz), 4.13(2H, q, J=6.9Hz), 3.61(4H, t, J=4.9Hz), 3.09(4H, t, J=4.9Hz), 1.41(3H, t, J=7.0Hz).
MS(FAB) m/z:562 $(M + H)^+$.
Melting point: 135-137˚C.

(Example 136) 4- {4-[3-(2-methoxy-5-methyl-phenyl)-ureido]-phenyl}-piperazine-l - carboxylic acid (3-chloro-2-methyl-phenyl)-amide (Compound No. 1-392)

94 mg (92%) of the title compound was obtained from 1-(2-methoxy-5-methyl-phenyl)-3-(4-piperazin-1-yl-phenyl)-urea (68 mg) obtained in Example (47a) and 3-chloro-2-methylphenyl isocyanate (0.040 mL) as a flesh colour powder in the same manner as in Example 49.
$^1$H-NMR spectrum (400MHz,DMSO-d6):δ(ppm)=9.06(1H, s), 8.40(1H, s), 8.04(1H, s), 7.96(1H, d, J=2.4Hz), 7.31(2H, d, J=9.0Hz), 7.23(1H, dd, J=4.7 and 4.7Hz), 7.14(2H, d, J=4.7Hz), 6.93(2H, d, J=8.6Hz), 6.86(1H, d, J=8.6Hz), 6.71 (1H, dd, J=7.4 and 2.4Hz), 3.82(3H, s), 3.59(4H, t, J=5.1Hz), 3.07(4H, t, J=4.5Hz), 2.22(3H, s), 2.18(3H, s).
MS(FAB) m/z:508 $(M + H)^+$.
Melting point: 228-230˚C.

(Example 137) 4-{5-[3-(2-methoxy-5-methyl-phenyl)-ureido]-pyridin-2-yl}-piperazine-1-carboxylic acid (2-trifluoromethyl-phenyl)-amide (Compound No. 1-208)

(137a) 1-(2-methoxy-5-methyl-phenyl)-3-(6-piperazin-1-yl-pyridin-3-yl)-urea

0.651 g (83%) of the title compound was obtained from 4-{5-[3-(2-methoxy-5-methyl-phenyl)-ureido]-pyridin-2-yl}-piperazine-l-carboxylic acid tert-butyl ester (1.04 g) obtained in Example 38 as a white solid in the same manner as in Example (47a).
MS(FAB) m/z:342 $(M + H)^+$.

(137b) 4-{5-[3-(2-methoxy-5-methyl-phenyl)-ureido]-pyridin-2-yl}-piperazine-1-carboxylic acid (2-trifluoromethyl-phenyl)-amide

48 mg (47%) of the title compound was obtained from 1-(2-methoxy-5-methyl-phenyl)-3-(6-piperazin-1-yl-pyridin-3-yl)-urea (66 mg) obtained in Example (137a) and 2-trifluoromethylphenyl isocyanate (0.045 mL) as a beige solid in the same manner as in Example 49.
$^1$H-NMR spectrum (400MHz,DMSO-d6):δ(ppm)=9.10(1H, s), 8.32(1H, s), 8.13(1H, d, J=2.5Hz), 8.10(1H, s), 7.94(1H, d, J=1.6Hz), 7.74(1H, dd, J=9.2 and 2.5Hz), 7.68(1H, d, J=7.8Hz), 7.63(1H, dd, J=7.8 and 7.8Hz), 7.44(1H, d, J=7.8Hz), 7.40(1H, dd, J=7.8 and 7.8Hz), 6.90-6.85(1H, m), 6.87(1H, d, J=8.2Hz), 6.72(1H, dd, J=8.2 and 1.6Hz), 3.82(3H, s), 3.57-3.52(4H, m), 3.47-3.41(4H, m), 2.22(3H, s).
MS(FAB) m/z:529 $(M + H)^+$.
Melting point: 213-214˚C.

(Example 138) 4- {5-[3-(2-methoxy-5-methyl-phenyl)-ureido]-pyridin-2-yl}-piperazine-1-carboxylic acid (2,6-dimethyl-phenyl)-amide (Compound No. 1-68)

76 mg (80%) of the title compound was obtained from 1-(2-methoxy-5-methyl-phenyl)-3-(6-piperazin-1-yl-pyridin-3-yl)-urea (66 mg) obtained in Example (137a) and 2,6-dimethylphenyl isocyanate (0.042 mL) as a beige solid in the same manner as in Example 49.
$^1$H-NMR spectrum (400MHz,DMSO-d6):δ(ppm)=9.07(1H, s), 8.15(1H, d, J=2.8Hz), 8.10(1H, s), 7.94(1H, s), 7.94(1H, d, J=2.3Hz), 7.73(1H, dd, J=9.0 and 2.8Hz), 7.02(2H, s), 7.05-7.00(1H, m), 6.98-6.90(1H, m), 6.87(1H, d, J=8.2Hz), 6.71(1H, dd, J=8.2 and 2.3Hz), 3.82(3H, s), 3.58-3.54(4H, m), 3.46-3.41(4H, m), 2.22(3H, s), 2.14(6H, s).
MS(FAB) m/z:489 $(M + H)^+$.
Melting point: 221-223˚C.

(Example 139) 4-{5-[3-(2-methoxy-5-methyl-phenyl)-ureido]-pyridin-2-yl}-piperazine-1-carboxylic acid (2-fluoro-6-trifluoromethyl-phenyl)-amide (Compound No. 1-26)

63 mg (74%) of the title compound was obtained from 1-(2-methoxy-5-methyl-phenyl)-3-(6-piperazin-1-yl-pyridin-3-yl)-urea (66 mg) obtained in Example (137a) and 2-fluoro-6-trifluoromethylphenyl isocyanate (0.043 mL) as a white solid in the same manner as in Example 49.
$^1$H-NMR spectrum (400MHz,DMSO-d6):δ(ppm)=9.08(1H, s), 8.33(1H, s), 8.15(1H, d, J=2.4Hz), 8.10(1H, s), 7.94(1H, d, J=1.9Hz), 7.74(1H, dd, J=9.0 and 2.4Hz), 7.64-7.50(3H, m), 6.87(1H, d, J=9.0Hz), 6.87(1H, d, J=8.2Hz), 6.72(1H, dd, J=8.2 and 1.9Hz), 3.83(3H, s), 3.56(4H, t, J=5.0Hz), 3.43(4H, t, J=5.0Hz), 2.22(3H, s).
MS(FAB) m/z:547 $(M + H)^+$.
Melting point: 221-222˚C.

(Example 140) 4-{5-[3-(2-methoxy-5-methyl-phenyl)-ureido]-pyridin-2-yl}-piperazine-1-carboxylic acid (2,6-dichlorophenyl)-amide (Compound No. 1-40)

63 mg (74%) of the title compound was obtained from 1-(2-methoxy-5-methyl-phenyl)-3-(6-piperazin-1-yl-pyridin-3-yl)-urea (71 mg) obtained in Example (137a) and 2,6-dichlorophenyl isocyanate (56 mg) as a beige solid in the same manner as in Example 49.
$^1$H-NMR spectrum (400MHz,DMSO-d6):δ(ppm)=9.19(1H, s), 8.51(1H, s), 8.20(1H, d, J=2.8Hz), 8.15(1H, s), 7.94(1H, d, J=1.6Hz), 7.77(1H, dd, J=9.0 and 2.8Hz), 7.49(2H, d, J=8.2Hz), 7.30(1H, dd, J=8.2 and 8.2Hz), 6.89(1H, d, J=9.0Hz), 6.89(1H, d, J=8.2Hz), 6.73(1H, dd, J=8.2 and 1.6Hz), 3.82(3H, s), 3.59-3.55(4H, m), 3.47-3.43(4H, m), 2.22(3H, s).
MS(FAB) m/z:529 $(M + H)^+$.
Melting point: 222-224˚C.

(Example 141) 4-{5-[3-(2-ethoxy-phenyl)-ureido]-pyridin-2-yl}-piperazine-1-carboxylic acid (2-chloro-6-methyl-phenyl)-amide (Compound No. 1-56)

(141a) 1-(2-ethoxy-phenyl)-3-(6-piperazin-1-yl-pyridin-3-yl)-urea

1.27 g (100%) of the title compound was obtained from 4-{5-[3-(2-ethoxyphenyl)-ureido]-pyridin-2-yl}-piperazine-1-carboxylic acid tert-butyl ester (1.51 g) obtained in Example 39 as a pale pink solid in the same manner as in Example (47a).
MS(FAB) m/z:342 $(M + H)^+$.

(141b) 4-{5-[3-(2-ethoxy-phenyl)-ureido]-pyridin-2-yl}-piperazine-1-carboxylic acid (2-chloro-6-methyl-phenyl)-amide

195 mg (96%) of the title compound was obtained from 1-(2-ethoxy-phenyl)-3-(6-piperazin-1-yl-pyridin-3-yl)-urea (137 mg) obtained in Example (141a) and 2-chloro-6-methylphenyl isocyanate (0.082 mL) as a white solid in the same manner as in Example 49.
$^1$H-NMR spectrum (400MHz,DMSO-d6):δ(ppm)=9.20(1H, s), 8.22(1H, s), 8.17(1H, d, J=2.4Hz), 8.09(1H, dd, J=7.8 and 1.9Hz), 8.02(1H, s), 7.76(1H, dd, J=9.0 and 2.4Hz), 7.31(1H, d, J=7.6Hz), 7.20(1H, d, J=7.6Hz), 7.15(1H, dd, J=7.6 and 7.6Hz), 6.98(1H, dd, J=7.8 and 1.6Hz), 6.94-6.83(3H, m), 4.12(2H, q, J=6.8Hz), 3.61-3.55(4H, m), 3.49-3.44(4H, m), 2.20(3H, s), 1.41(3H, t, J=6.8Hz).
MS(FAB) m/z:509 $(M + H)^+$.
Melting point: 185-186˚C.

(Example 142) 4-{5-[3-(2-ethoxy-phenyl)-ureido]-pyridin-2-yl}-piperazine-1-carboxylic acid (2,6-dimethyl-phenyl)-amide (Compound No. 1-70)

182 mg (93%) of the title compound was obtained from 1-(2-ethoxy-phenyl)-3-(6-piperazin-l-yl-pyridin-3-yl)-urea (137 mg) obtained in Example (141a) and 2,6-dichlorophenyl isocyanate (0.084 mL) as a white solid in the same manner as in Example 49.
$^1$H-NMR spectrum (400MHz,DMSO-d6):δ(ppm)=9.20(1H, s), 8.17(1H, d, J=2.3Hz), 8.09(1H, dd, J=7.8 and 1.5Hz), 8.02 (1H, s), 7.95(1H, s), 7.75(1H, dd, J=9.0 and 2.3Hz), 7.02(2H, s), 7.06-7.01(1H, m), 6.98(1H, dd, J=7.8 and 1.5Hz), 6.93-6.82(3H, m), 4.12(2H, q, J=6.8Hz), 3.59-3.54(4H, m), 3.48-3.43(4H, m), 2.14(6H, s), 1.41(3H, t, J=6.8Hz).
MS(FAB) m/z:489 $(M + H)^+$.
Melting point: 188-190˚C.

(Example 143) 4-{5-[3-(2-ethoxy-phenyl)-ureido]-pyridin-2-yl}-piperazine-1-carboxylic acid (2-fluoro-6-trifluoromethyl-phenyl)-amide (Compound No. 1-28)

164 mg (75%) of the title compound was obtained from 1-(2-ethoxy-phenyl)-3-(6-piperazin-1-yl-pyridin-3-yl)-urea (137 mg) obtained in Example (141a) and 2-fluoro-6-trifluoromethylphenyl isocyanate (0.086 mL) as a white solid in the same manner as in Example 49.
$^1$H-NMR spectrum (400MHz,DMSO-d6):δ(ppm)=9.21 (1H, s), 8.34(1H, s), 8.17(1H, d, J=2.6Hz), 8.09(1H, dd, J=7.8 and 1.6Hz), 8.10(1H, s), 7.76(1H, dd, J=8.8 and 2.6Hz), 7.64-7.50(3H, m), 6.98(1H, dd, J=7.8 and 1.6Hz), 6.94-6.82(3H, m), 4.12(2H, q, J=7.0Hz), 3.59-3.52(4H, m), 3.48-3.41(4H, m), 1.41(3H, t, J=7.0Hz).
MS(FAB) m/z:547 $(M + H)^+$.
Melting point: 177-179°C.

(Example 144) 4-{5-[3-(2-ethoxy-phenyl)-ureido]-pyridin-2-yl}-piperazine-1-carboxylic acid (2,6-dichloro-phenyl)-amide (Compound No. 1-42)

131 mg (82%) of the title compound was obtained from 1-(2-ethoxy-phenyl)-3-(6-piperazin-1-yl-pyridin-3-yl)-urea (102 mg) obtained in Example (141a) and 2,6-dichlorophenyl isocyanate (85 mg) as a white solid in the same manner as in Example 49.
$^1$H-NMR spectrum (400MHz,DMSO-d6):δ(ppm)=9.24(1H, s), 8.55(1H, s), 8.20(1H, d, J=2.7Hz), 8.12(1H, dd, J=7.8 and 1.6Hz), 8.05(1H, s), 7.78(1H, dd, J=9.2 and 2.7Hz), 7.52(2H, d, J=8.1Hz), 7.30(1H, dd, J=8.1 and 8.1Hz), 7.01(1H, dd, J=7.8 and 1.6Hz), 6.97-6.85(3H, m), 4.13(2H, q, J=7.0Hz), 3.62-3.56(4H, m), 3.51-3.45(4H, m), 1.42(3H, t, J=7.0Hz).
MS(FAB) m/z:529 $(M + H)^+$.
Melting point: 170-172°C.

(Example 145) 4-{5-[3-(2-ethoxy-phenyl)-ureido]-pyridin-2-yl}-piperazine-1-carboxylic acid (4-methoxy-2-methyl-phenyl)-amide (Compound No. 1-154)

114 mg (75%) of the title compound was obtained from 1-(2-ethoxy-phenyl)-3-(6-piperazin-1-yl-pyridin-3-yl)-urea (102 mg) obtained in Example (141a) and 4-methoxy-2-methylphenyl isocyanate (0.066 mL) as a white solid in the same manner as in Example 49.
$^1$H-NMR spectrum (400MHz,DMSO-d6):δ(ppm)=9.23(1H, s), 8.20(1H, d, J=2.7Hz), 8.12(1H, d, J=7.8Hz), 8.05(2H, s), 7.78(1H, dd, J=9.1 and 2.7Hz), 7.05(1H, d, J=8.2Hz), 7.01(1H, d, J=7.8Hz), 6.96-6.85(3H, m), 6.78(1H, s), 6.71(1H, d, J=8.2Hz), 4.13(2H, q, J=6.9Hz), 3.72(3H, s), 3.58-3.52(4H, m), 3.50-3.43(4H, m), 2.14(3H, s), 1.42(3H, t, J=6.9Hz).
MS(FAB) m/z:505 $(M + H)^+$.
Melting point: 166-168°C.

(Example 146) 4-{5-[3-(2-ethoxy-phenyl)-ureido]-pyridin-2-yl}-piperazine-1-carboxylic acid (2-chloro-4-methyl-phenyl)-amide (Compound No. 1-126)

125 mg (82%) of the title compound was obtained from 1-(2-ethoxy-phenyl)-3-(6-piperazin-1-yl-pyridin-3-yl)-urea (102 mg) obtained in Example (141a) and 2-chloro-4-methylphenyl isocyanate (75 mg) as a white solid in the same manner as in Example 49.
$^1$H-NMR spectrum (400MHz,DMSO-d6):δ(ppm)=9.22(1H, s), 8.24(1H, s), 8.20(1H, d, J=2.7Hz), 8.11(1H, dd, J=8.0 and 1.8Hz), 8.05(1H, s), 7.78(1H, dd, J=9.0 and 2.7Hz), 7.35(1H, d, J=8.2Hz), 7.29(1H, s), 7.11(1H, d, J=8.2Hz), 7.01(1H, dd, J=8.0 and 1.5Hz), 6.96-6.85(3H, m), 4.13(2H, q, J=6.9Hz), 3.60-3.54(4H, m), 3.51-3.45(4H, m), 2.28(3H, s), 1.41 (3H, t, J=6.9Hz).
MS(FAB) m/z:509 $(M + H)^+$.
Melting point: 136-137°C.

(Example 147) 4-{5-[3-(2-methoxy-5-methyl-phenyl)-ureido]-pyridin-2-yl}-piperazine-1-carboxylic acid (2-chloro-4-methyl-phenyl)-amide (Compound No. 1-124)

78 mg (38%) of the title compound was obtained from 1-(2-methoxy-5-methyl-phenyl)-3-(6-piperazin-1-yl-pyridin-3-yl)-urea (137 mg) obtained in Example (137a) and 2-chloro-4-methylphenyl isocyanate (101 mg) as a white solid in the same manner as in Example 49.
$^1$H-NMR spectrum (400MHz,DMSO-d6):δ(ppm)=9.07(1H, s), 8.21(1H, s), 8.14(1H, d, J=3.1Hz), 8.09(1H, s), 7.94(1H, d, J=2.0Hz), 7.73(1H, dd, J=9.0 and 3.1Hz), 7.33(1H, d, J=7.8Hz), 7.27(1H, s), 7.08(1H, d, J=7.8Hz), 6.87(1H, d, J=8.2Hz), 6.86(1H, d, J=9.0Hz), 6.71(1H, dd, J=8.2 and 2.0Hz), 3.82(3H, s), 3.58-3.52(4H, m), 3.48-3.42(4H, m), 2.28(3H, s), 2.22(3H, s).
MS(FAB) m/z:509 $(M + H)^+$.
Melting point: 214-215˚C.

(Example 148) 4-{5-[3-(5-fluoro-2-methoxy-phenyl)-ureido]-pyridin-2-yl}-piperazine-1-carboxylic acid (2-chloro-6-methyl-phenyl)-amide (Compound No. 1-50)

(148a) 1-(5-fluoro-2-methoxy-phenyl)-3-(6-piperazin-1-yl-pyridin-3-yl)-urea

1.10 g (100%) of the title compound was obtained from 4-{5-[3-(5-fluoro-2-methoxy-phenyl)-ureido]-pyridin-2-yl}-piperazine-1-carboxylic acid tert-butyl ester (1.43 g) obtained in Example 44 as a white solid in the same manner as in Example (47a).
MS(FAB) m/z:346 $(M + H)^+$.

(148b) 4-{5-[3-(5-fluoro-2-methoxy-phenyl)-ureido]-pyridin-2-yl}-piperazine-1-carboxylic acid (2-chloro-6-methyl-phenyl)-amide

73 mg (47%) of the title compound was obtained from 1-(5-fluoro-2-methoxy-phenyl)-3-(6-piperazin-1-yl-pyridin-3-yl)-urea (106 mg) obtained in Example (148a) and 2-chloro-6-methylphenyl isocyanate (0.061 mL) as a white solid in the same manner as in Example 49.
$^1$H-NMR spectrum (400MHz,DMSO-d6):δ(ppm)=9.21(1H, s), 8.39(1H, s), 8.24(1H, s), 8.17(1H, d, J=3.1Hz), 8.01(1H, dd, J=11.6 and 3.0Hz), 7.76(1H, dd, J=9.0 and 3.1Hz), 7.33(1H, d, J=7.0Hz), 7.22(1H, d, J=7.0Hz), 7.17(1H, dd, J=7.0 and 7.0Hz), 7.01(1H, dd, J=8.8 and 4.9Hz), 6.91(1H, d, J=9.0Hz), 6.74(1H, ddd, J=8.8, 8.8 and 3.0Hz), 3.88(3H, s), 3.61-3.55(4H, m), 3.49-3.44(4H, m), 2.20(3H, s).
MS(FAB) m/z:513 $(M + H)^+$.
Melting point: 227-228˚C.

(Example 149) 4-{5-[3-(5-fluoro-2-methoxy-phenyl)-ureido]-pyridin-2-yl}-piperazine-1-carboxylic acid (2,6-dimethyl-phenyl)-amide (Compound No. 1-64)

102 mg (69%) of the title compound was obtained from 1-(5-fluoro-2-methoxy-phenyl)-3-(6-piperazin-1-yl-pyridin-3-yl)-urea (106 mg) obtained in Example (148a) and 2,6-dimethylphenyl isocyanate (0.063 mL) as a white solid in the same manner as in Example 49.
$^1$H-NMR spectrum (400MHz,DMSO-d6):δ(ppm)=9.18(1H, s), 8.36(1H, s), 8.14(1H, d, J=2.7Hz), 7.99(1H, dd, J=11.4 and 3.1Hz), 7.95(1H, s), 7.74(1H, dd, J=9.0 and 2.7Hz), 7.05-7.01(1H, m), 7.02(2H, s), 6.99(1H, dd, J=8.8 and 5.2Hz), 6.88(1H, d, J=9.0Hz), 6.72(1H, ddd, J=8.8, 8.8 and 3.1Hz), 3.86(3H, s), 3.62-3.54(4H, m), 3.47-3.42(4H, m), 2.14(6H, s).
MS(FAB) m/z:493 $(M + H)^+$.
Melting point: 226-228˚C.

(Example 150) 4- {5-[3-(5-fluoro-2-methoxy-phenyl)-ureido]-pyridin-2-yl} - piperazine-1-carboxylic acid (2-fluoro-6-trifluoromethyl-phenyl)-amide (Compound No. 1-22)

78 mg (47%) of the title compound was obtained from 1-(5-fluoro-2-methoxy-phenyl)-3-(6-piperazin-1-yl-pyridin-3-yl)-urea (106 mg) obtained in Example (148a) and 2-fluoro-6-trifluoromethylphenyl isocyanate (0.063 mL) as a white solid in the same manner as in Example 49.
$^1$H-NMR spectrum (400MHz,DMSO-d6):δ(ppm)=9.19(1H, s), 8.37(1H, s), 8.33(IH, s), 8.15(IH, d, J=2.7Hz), 7.99(1H, dd, J=11.2 and 3.1Hz), 7.74(1H, dd, J=9.0 and 2.7Hz), 7.64-7.50(3H, m), 6.99(1H, dd, J=8.8 and 5.4Hz), 6.89(IH, d, J=9.OHz), 6.72(IH, ddd, J=8.8, 8.8 and 3.1Hz), 3.86(3H, s), 3.58-3.52(4H, m), 3.46-3.41(4H, m).
MS(FAB) m/z:551 $(M + H)^+$.
Melting point: 212-213˚C.

(Example 151) 4-{5-[3-(5-fluoro-2-methoxy-phenyl)-ureido]-pyridin-2-yl}-piperazine-1-carboxylic acid (2,6-dichloro-phenyl)-amide (Compound No. 1-36)

77 mg (72%) of the title compound was obtained from 1-(5-fluoro-2-methoxy-phenyl)-3-(6-piperazin-1-yl-pyridin-3-yl)-urea (69 mg) obtained in
Example (148a) and 2,6-dimethylphenyl isocyanate (56 mg) as a white solid in the same manner as in Example 49.

$^1$H-NMR spectrum (400MHz,DMSO-d6):δ(ppm)=9.21(1H, s), 8.54(1H, s), 8.39(1H, s), 8.17(1H, d, J=2.8Hz), 8.01(1H, dd, J=12.1 and 3.3Hz), 7.76(1H, dd, J=9.0 and 2.8Hz), 7.52(2H, d, J=8.2Hz), 7.30(1H, dd, J=8.2 and 8.2Hz), 7.01(1H, dd, J=8.2 and 5.9Hz), 6.91(1H, d, J=9.0Hz), 6.74(1H, ddd, J=8.2, 8.2 and 3.3Hz), 3.88(3H, s), 3.62-3.56(4H, m), 3.51-3.44 (4H, m).
MS(ES) m/z:533 $(M + H)^+$.
Melting point: 228˚C.

(Example 152) 4-{5-[3-(2-methoxy-5-methyl-phenyl)-ureido]-pyridin-2-yl}-piperazine-1-carboxylic acid (2,6-difluoro-phenyl)-amide (Compound No. 1-12)

56 mg (57%) of the title compound was obtained from 1-(2-methoxy-5-methyl-phenyl)-3-(6-piperazin-1-yl-pyridin-3-yl)-urea (68 mg) obtained in Example (137a) and 2,6-difluoro-phenyl isocyanate (34 mg) as a white solid in the same manner as in Example 49.
$^1$H-NMR spectrum (400MHz,DMSO-d6):δ(ppm)=9.20(1H, s), 8.39(1H, s), 8.22(1H, s), 8.15(1H, s), 7.96(1H, d, J=1.8Hz), 7.79(1H, dd, J=9.0 and 2.3Hz), 7.35-7.24(1H, m), 7.13(2H, dd, J=7.2 and 7.3Hz), 7.08-6.96(1H, m), 6.90(1H, d, J=8.3Hz), 6.75(1H, dd, J=8.3 and 1.8Hz), 3.84(3H, s), 3.64-3.60(4H, m), 3.59-3.47(4H, m), 2.23(3H, s). MS(ES) m/z:497 $(M + H)^+$.
Melting point: 237-239˚C.

(Example 153) 4-{5-[3-(5-fluoro-2-methoxy-phenyl)-ureido]-pyridin-2-yl}-piperazine-1-carboxylic acid (4-methoxy-2-methyl-phenyl)-amide (Compound No. 1-148)

28 mg (27%) of the title compound was obtained from 1-(5-fluoro-2-methoxy-phenyl)-3-(6-piperazin-1-yl-pyridin-3-yl)-urea (69 mg) obtained in Example (148a) and 4-methoxy-2-methylphenyl isocyanate (0.044 mL) as a white solid in the same manner as in Example 49.
$^1$H-NMR spectrum (400MHz,DMSO-d6):δ(ppm)=9.17(1H, s), 8.36(1H, s), 8.14(1H, d, J=2.7Hz), 8.01(1H, s), 7.98(1H, dd, J=11.3 and 3.1Hz), 7.73(1H, dd, J=9.0 and 2.7Hz), 7.03(1H, d, J=8.2Hz), 6.98(1H, dd, J=8.8 and 4.9Hz), 6.87(1H, d, J=9.0Hz), 6.78-6.66(3H, m), 3.86(3H, s), 3.71(3H, s), 3.71-3.57(4H, m), 3.47-3.41(4H, m), 2.13(3H, s).
MS(ES) m/z:509 $(M + H)^+$.
Melting point: 212-214˚C.

(Example 154) 4-{5-[3-(5-fluoro-2-methoxy-phenyl)-ureido]-pyridin-2-yl}-piperazine-1-carboxylic acid (2-chloro-4-methyl-phenyl)-amide (Compound No. 1-120)

61 mg (59%) of the title compound was obtained from 1-(5-fluoro-2-methoxy-phenyl)-3-(6-piperazin-1-yl-pyridin-3-yl)-urea (69 mg) obtained in Example (148a) and 2-chloro-4-methylphenyl isocyanate (50 mg) as a white solid in the same manner as in Example 49.
$^1$H-NMR spectrum (400MHz,DMSO-d6):δ(ppm)=9.18(1H, s), 8.36(1H, s), 8.21(1H, s), 8.14(1H, d, J=2.7Hz), 7.98(1H, dd, J=11.3 and 3.1Hz), 7.73(1H, dd, J=9.0 and 2.7Hz), 7.33(1H, d, J=8.2Hz), 7.27(1H, d, J=1.6Hz), 7.08(1H, dd, J=8.2 and 1.6Hz), 6.99(1H, dd, J=9.0 and 5.0Hz), 6.87(1H, d, J=9.0Hz), 6.72(1H, ddd, J=9.0, 8.6 and 3.1Hz), 3.86(3H, s), 3.57-3.53(4H, m), 3.48-3.43(4H, m), 2.28(3H, s).
MS(FAB) m/z:513 $(M + H)^+$.
Melting point: 217-218˚C.

(Example 155) 4-{5-[3-(2-methoxy-5-methyl-phenyl)-ureido]-pyridin-2-yl}-piperazine-1-carboxylic acid (4-fluoro-2-trifluoromethyl-phenyl)-amide (Compound No. 1-110)

69 mg (42%) of the title compound was obtained from 1-(2-methoxy-5-methyl-phenyl)-3-(6-piperazin-1-yl-pyridin-3-yl)-urea (102 mg) obtained in Example (137a) and 4-fluoro-2-trifluoromethylphenyl isocyanate (0.065 mL) as a white solid in the same manner as in Example 49.
$^1$H-NMR spectrum (500MHz,DMSO-d6):δ(ppm)=9.09(1H, s), 8.36(1H, s), 8.16(1H, d, J=2.8Hz), 8.11(1H, s), 7.95(1H, d, J=1.9Hz), 7.75(1H, dd, J=9.0 and 2.8Hz), 7.59(1H, dd, J=9.0 and 2.8Hz), 7.56-7.44(2H, m), 6.88(2H, d, J=7.8Hz), 6.73(1H, dd, J=7.8 and 1.9Hz), 3.83(3H, s), 3.57-3.52(4H, m), 3.46-3.41(4H, m), 2.22(3H, s). MS(ES) m/z:547 $(M + H)^+$.
Melting point: 186-187˚C.

(Example 156) 4-{4-[3-(4-dimethylamino-phenyl)-ureido]-phenyl}-piperazine-1-carboxylic acid (2-chloro-6-methyl-phenyl)-amide (Compound No. 1-397)

(156a) 1-(4-dimethylamino-phenyl)-3-(4-piperazin-1-yl-phenyl)-urea

0.679 g (100%) of the title compound was obtained from 4-{4-[3-(4-dimethylamino-phenyl)-ureido]-phenyl}-piperazine-1-carboxylic acid tert-butyl ester (0.880 g) obtained in Example 20 as a white solid in the same manner as in Example (47a).
MS(FAB) m/z:340 $(M + H)^+$.

(156b) 4-{4-[3-(4-dimethylamino-phenyl)-ureido]-phenyl}-piperazine-1-carboxylic acid (2-chloro-6-methyl-phenyl)-amide

88 mg (87%) of the title compound was obtained from 4-{4-[3-(4-dimethylamino-phenyl)-ureido]-phenyl}-piperazine-1-carboxylic acid (2-chloro-6-methyl-phenyl)-amide (68 mg) obtained in Example (156a) and 2-chloro-6-methylphenyl isocyanate (0.041 mL) as a pale purple solid in the same manner as in Example 49.
$^1$H-NMR spectrum (400MHz,DMSO-d6):δ(ppm)=8.34(2H, brs), 8.23(1H, s), 7.33-7.31(5H, m), 7.24-7.15(4H, m), 6.95 (2H, d, J=7.8Hz), 3.61(4H, t, J=3.5Hz), 3.08(4H, t, J=3.9Hz), 2.89(6H, brs), 2.20(3H, s).
MS(FAB) m/z:507 $(M + H)^+$.
Melting point: 214-217˚C.

(Example 157) 4-{4-[3-(2-sec-butyl-phenyl)-ureido]-phenyl}-piperazine-1-carboxylic acid (2-chloro-6-methyl-phenyl)-amide (Compound No. 1-398)

(157a) 1-(2-sec-butyl-phenyl)-3-(4-piperazin-1-yl-phenyl)-urea

0.705 g (100%) of the title compound was obtained from 4-{4-[3-(2-sec-butyl-phenyl)-ureido]-phenyl}-piperazine-1-carboxylic acid tert-butyl ester (0.905 g) obtained in Example 21 as a white solid in the same manner as in Example (47a).
MS(FAB) m/z:353 $(M + H)^+$.

(157b) 4-{4-[3-(2-sec-butyl-phenyl)-ureido]-phenyl}-piperazine-1-carboxylic acid (2-chloro-6-methyl-phenyl)-amide

82 mg (79%) of the title compound was obtained from 1-(2-sec-butylphenyl)-3-(4-piperazin-1-yl-phenyl)-urea (71 mg) obtained in Example (157a) and 2-chloro-6-methylphenyl isocyanate (0.041 mL) as a pale purple solid in the same manner as in Example 49.
$^1$H-NMR spectrum (400MHz,DMSO-d6):δ(ppm)=8.72(1H, s), 8.23(1H, s), 7.80(1H, s), 7.67(1H, d, J=7.9Hz), 7.34(2H, d, J=8.2Hz), 7.33(1H, d, J=5.1Hz), 7.22(2H, d, J=8.2Hz), 7.18(1H, d, J=7.0Hz), 7.13(1H, dd, J=6.6 and 6.6Hz), 7.06 (1H, dd, J=7.8 and 7.8Hz), 6.95(2H, d, J=8.6Hz), 3.61(4H, t, J=4.0Hz), 3.08(4H, t, J=4.3Hz), 2.92(1H, q, J=7.5Hz), 2.20 (3H, s), 1.61-1.54(2H, m), 1.17(3H, d, J=6.3Hz), 0.81(3H, t,J=7.1Hz).
MS(FAB) m/z:520 $(M + H)^+$.
Melting point: 232-234˚C.

(Example 158) 4-{4-[3-(4-isopropyl-phenyl)-ureido]-phenyl}-piperazine-1-carboxylic acid (2-chloro-6-methyl-phenyl)-amide (Compound No. 1-399)

(158a) 1-(4-isopropyl-phenyl)-3-(4-piperazin-1-yl-phenyl)-urea

0.677 g (100%) of the title compound was obtained from 4-{4-[3-(4-isopropyl-phenyl)-ureido]-phenyl}-piperazine-1-carboxylic acid tert-butyl ester (0.877 g) obtained in Example 22 as a white solid in the same manner as in Example (47a).
MS(FAB) m/z:339 $(M + H)^+$.

(158b) 4-{4-[3-(4-isopropyl-phenyl)-ureido]-phenyl}-piperazine-1-carboxylic acid (2-chloro-6-methyl-phenyl)-amide

87 mg (86%) of the title compound was obtained from 1-(4-isopropylphenyl)-3-(4-piperazin-1-yl-phenyl)-urea (67 mg) obtained in Example (158a) and 2-chloro-6-methylphenyl isocyanate (0.041 mL) as a white powder in the same manner as in Example 49.

$^1$H-NMR spectrum (400MHz,DMSO-d6):δ(ppm)=8.43(1H, s), 8.35(1H, s), 8.20(1H, s), 7.32(2H, d, J=8.6Hz), 7.29(3H, d, J=8.2Hz), 7.19(1H, d, J=7.4Hz), 7.14(1H, d, J=7.8Hz), 7.11(2H, d, J=8.2Hz), 6.92(2H, d, J=8.2Hz), 3.59(4H, t, J=4.5Hz), 3.05(4H, t, J=4.7Hz), 2.83-2.76(1H, m), 2.18(3H, s), 1.15(6H, d, J=7.1Hz).
MS(FAB) m/z:506 $(M + H)^+$.
Melting point: 278-280˚C.

(Example 159) 4-{4-[3-(4-ethoxy-phenyl)-ureido]-phenyl}-piperazine-1-carboxylic acid (2-chloro-6-methyl-phenyl)-amide (Compound No. 1-400)

(159a) 1-(4-ethoxy-phenyl)-3-(4-piperazin-1-yl-phenyl)-urea

0.681 g (100%) of the title compound was obtained from 4-{4-[3-(4-ethoxyphenyl)-ureido]-phenyl}-piperazine-1-carboxylic acid tert-butyl ester (0.881 g) obtained in Example 23 as a white solid in the same manner as in Example (47a).
MS(FAB) m/z:341 $(M + H)^+$.

(159b) 4-{4-[3-(4-ethoxy-phenyl)-ureido]-phenyl}-piperazine-1-carboxylic acid (2-chloro-6-methyl-phenyl)-amide

80 mg (79%) of the title compound was obtained from 1-(4-ethoxy-phenyl)-3-(4-piperazin-1-yl-phenyl)-urea (68 mg) obtained in Example (159a) and 2-chloro-6-methylphenyl isocyanate (0.041 mL) as a white powder in the same manner as in Example 49.
$^1$H-NMR spectrum (400MHz,DMSO-d6):δ(ppm)=8.32(1H, s), 8.31(1H, s), 8.20(1H, s), 7.30(2H, d, J=8.6Hz), 7.29(2H, d, J=8.6Hz), 7.19(1H, d, J=7.OHz), 7.16(1H, d, J=10.2Hz), 7.13(1H, dd, J=7.6 and 7.6Hz), 6.91(2H, d, J=8.9Hz), 6.82 (2H, d, J=8.6Hz), 3.95(2H, q, J=7.1Hz), 3.58(4H, brs), 3.05(4H, t, J=4.7Hz), 2.18(3H, s), 1.28(3H, t, J=7.1Hz).
MS(FAB) m/z:508 $(M + H)^+$.
Melting point: 229-230˚C.

(Example 160) 4-{4-[3-(4-ethyl-phenyl)-ureido]-phenyl}-piperazine-1-carboxylic acid (2-chloro-6-methyl-phenyl)-amide (Compound No. 1-401)

(160a) 1-(4-ethyl-phenyl)-3-(4-piperazin-1-yl-phenyl)-urea

0.649 g (100%) of the title compound was obtained from 4-{4-[3-(4-ethylphenyl)-ureido]-phenyl}-piperazine-1-carboxylic acid tert-butyl ester (0.849 g) obtained in Example 24 as a white solid in the same manner as in Example (47a).
MS(FAB) m/z:325 $(M + H)^+$.

(160b) 4-{4-[3-(4-ethyl-phenyl)-ureido]-phenyl}-piperazine-1-carboxylic acid (2-chloro-6-methyl-phenyl)-amide

87 mg (89%) of the title compound was obtained from 1-(4-ethyl-phenyl)-3-(4-piperazin-1-yl-phenyl)-urea (65 mg) obtained in Example (160a) and 2-chloro-6-methylphenyl isocyanate (0.041 mL) as a white powder in the same manner as in Example 49.
$^1$H-NMR spectrum (400MHz,DMSO-d6):δ(ppm)=8.44(1H, s), 8.36(1H, s), 8.20(1H, s), 7.32(2H, d, J=8.0Hz), 7.30(3H, d, J=8.0Hz), 7.19(1H, d, J=7.8Hz), 7.14(1H, ddd, J=7.5, 7.5 and 1.5Hz), 7.08(2H, d, J=7.4Hz), 6.92(2H, d, J=7.4Hz), 3.58(4H, brs), 3.05(4H, brs), 2.54-2.48(2H, m), 2.18(3H, s), 1.13(3H, t, J=7.6Hz).
MS(FAB) m/z:492 $(M + H)^+$.
Melting point: 239-241˚C.

(Example 161)4-{4-[3-(4-sec-butyl-phenyl)-ureido]-phenyl}-piperazine-1-carboxylic acid (2-chloro-6-methyl-phenyl)-amide (Compound No. 1-402)

(161 a) 1-(4-sec-butyl-phenyl)-3-(4-piperazin-1-yl-phenyl)-urea

0.705 g (100%) of the title compound was obtained from 4-{4-[3-(4-sec-butyl-phenyl)-ureido]-phenyl}-piperazine-1-carboxylic acid tert-butyl ester (0.905 g) obtained in Example 25 as a white solid in the same manner as in Example (47a).
MS(FAB) m/z:353 $(M + H)^+$.

(161b) 4-{4-[3-(4-sec-butyl-phenyl)-ureido]-phenyl}-piperazine-1-carboxylic acid (2-chloro-6-methyl-phenyl)-amide

87 mg (84%) of the title compound was obtained from 1-(4-sec-butylphenyl)-3-(4-piperazin-1-yl-phenyl)-urea (71 mg) obtained in Example (161a) and 2-chloro-6-methylphenyl isocyanate (0.041 mL) as a white powder in the same manner as in Example 49.
$^1$H-NMR spectrum (400MHz,DMSO-d6):δ(ppm)=8.44(1H, s), 8.36(1H, s), 8.21(1H, s),7.32(2H, d, J=8.6Hz), 7.30(3H, d, J=9.OHz), 7.20(1H, d, J=7.0Hz), 7.13(1H, dd, J=7.6 and 7.6Hz), 7.07(2H, d, J=8.2Hz), 6.92(2H, d, J=8.6Hz), 3.58 (4H, t, J=4.3Hz), 3.05(4H, t, J=4.7Hz), 3.02-2.98(1H, m), 2.18(3H, s), 1.54-1.47(2H, m), 1.14(3H, d, J=6.7Hz), 0.74(3H, t, J=7.1Hz).
MS(FAB) m/z:520 $(M + H)^+$.
Melting point: 259-261˚C.

(Example 162) 4-{4-[3-(4-butoxy-phenyl)-ureido]-phenyl}-piperazine-1-carboxylic acid (2-chloro-6-methyl-phenyl)-amide (Compound No. 1-403)

(162a) 1-(4-butoxy-phenyl)-3-(4-piperazin-1-yl-phenyl)-urea

0.737 g (100%) of the title compound was obtained from 4-{4-[3-(4-butoxyphenyl)-ureido]-phenyl}-piperazine-1-carboxylic acid tert-butyl ester (0.937 g) obtained in Example 26 as a white solid in the same manner as in Example (47a).
MS(FAB) m/z:369 $(M + H)^+$.

(162b) 4-{4-[3-(4-butoxy-phenyl)-ureido]-phenyl}-piperazine-1-carboxylic acid (2-chloro-6-methyl-phenyl)-amide

102 mg (95%) of the title compound was obtained from 1-(4-butoxy-phenyl)-3-(4-piperazin-1-yl-phenyl)-urea (74 mg) obtained in Example (162a) and 2-chloro-6-methylphenyl isocyanate (0.041 mL) as a pale pink powder in the same manner as in Example 49.
$^1$H-NMR spectrum (400MHz,DMSO-d6):δ(ppm)=8.35(1H, s), 8.33(1H, s), 8.20(1H, s), 7.31-7.28(5H, m), 7.19(1H, d, J=6.6Hz), 7.14(1H, dd, J=7.6 and 7.6Hz), 6.91(2H, d, J=9.4Hz), 6.82(2H, d, J=9.0Hz), 3.90(2H, t, J=6.2Hz), 3.59(4H, t, J=4.7Hz), 3.06(4H, t, J=5.5Hz), 2.19(3H, s), 1.67(2H, t, J=7.6Hz), 1.42(2H, q, J=7.6Hz), 0.93(3H, t, J=7.4Hz).
MS(FAB) m/z:536 $(M + H)^+$.
Melting point: 218-219˚C.

(Example 163) 4-{4-[3-(2-fluoro-5-trifluoromethyl-phenyl)-ureido]-phenyl}-piperazine-1-carboxylic acid (2-chloro-6-methyl-phenyl)-amide (Compound No. 1-404)

(163a) 1-(2-fluoro-5-trifluoromethyl-phenyl-phenyl)-3-(4-piperazin-1-yl-phenyl)-urea

0.765 g (100%) of the title compound was obtained from 4-{4-[3-(2-fluoro-5-trifluoromethyl-phenyl)-ureido]-phenyl}-piperazine-1-carboxylic acid tert-butyl ester (0.965 g) obtained in Example 27 as a white solid in the same manner as in Example (47a).
MS(FAB) m/z:383 $(M + H)^+$.

(163b) 4-{4-[3-(2-fluoro-5-trifluoromethyl-phenyl)-ureido]-phenyl}-piperazine-1-carboxylic acid (2-chloro-6-methyl-phenyl)-amide

103 mg (94%) of the title compound was obtained from 1-(2-fluoro-5-trifluoromethyl-phenyl-phenyl)-3-(4-piperazin-1-yl-phenyl)-urea (77 mg) obtained in Example (163a) and 2-chloro-6-methylphenyl isocyanate (0.041 mL) as a light brown powder in the same manner as in Example 49.
$^1$H-NMR spectrum (400MHz,DMSO-d6):8(ppm)=8.84(1H, brs), 8.59(1H, s), 8.21(1H, brs), 7.46(1H, dd, J=9.6 and 9.6Hz), 7.32(4H, d, J=9.0Hz), 7.30(1H, d, J=6.7Hz), 7.19(1H, d, J=6.2Hz), 7.14(1H, dd, J=7.8 and 7.8Hz), 6.95(2H, d, J=9.4Hz), 3.60(4H, t, J=4.7Hz), 3.09(4H, t, J=4.9Hz), 2.20(3H, s).
MS(FAB) m/z:550 $(M + H)^+$.
Melting point: 228-230˚C.

(Example 164) 4-{4-[3-(2-chloro-5-trifluoromethyl-phenyl)-ureido]-phenyl}-piperazine-1-carboxylic acid (2-chloro-6-methyl-phenyl)-amide (Compound No. 1-405)

(164a) 1-(2-chloro-5-trifluoromethyl-phenyl-phenyl)-3-(4-piperazin-1-yl-phenyl)-urea

0.798 g (100%) of the title compound was obtained from 4-{4-[3-(2-chloro-5-trifluoromethyl-phenyl)-ureido]-phenyl}-piperazine-1-carboxylic acid tert-butyl ester (0.998 g) obtained in Example 28 as a white solid in the same manner as in Example (47a).
MS(FAB) m/z:399 $(M + H)^+$.

(164b) 4-{4-[3-(2-chloro-5-trifluoromethyl-phenyl)-ureido]-phenyl}-piperazine-1-carboxylic acid (2-chloro-6-methyl-phenyl)-amide

100 mg (88%) of the title compound was obtained from 1-(2-chloro-5-trifluoromethyl-phenyl-phenyl)-3-(4-piperazin-1-yl-phenyl)-urea (77 mg) obtained in Example (164a) and 2-chloro-6-methylphenyl isocyanate (0.041 mL) as a flesh colour powder in the same manner as in Example 49.
$^1$H-NMR spectrum (400MHz,DMSO-d6):δ(ppm)=9.33(1H, s), 8.63(1H, d, J=2.3Hz), 8.49(1H, s), 8.21(1H, s), 7.69(2H, d, J=8.6Hz), 7.34(2H, d, J=9.0Hz), 7.31(1H, dd, J=9.2 and 1.8Hz), 7.20(1H, d, J=6.2Hz), 7.14(1H, dd, J=7.6 and 7.6Hz), 6.96(2H, d, J=9.0Hz), 3.60(4H, t, J=4.6Hz), 3.09(4H, t, J=4.5Hz), 2.20(3H, s).
MS(FAB) m/z:566 $(M + H)^+$.
Melting point: 249-251˚C.

(Example 165) 4-{4-[3-(2-chloro-5-methyl-phenyl)-ureido]-phenyl}-piperazine-1-carboxylic acid (2-chloro-6-methyl-phenyl)-amide (Compound No. 1-406)

(165a) 1-(2-chloro-5-methyl-phenyl-phenyl)-3-(4-piperazin-1-yl-phenyl)-urea

0.690 g (100%) of the title compound was obtained from 4-{4-[3-(2-chloro-5-methyl-phenyl)-ureido]-phenyl}-piperazine-1-carboxylic acid tert-butyl ester (0.890 g) obtained in Example 29 as a white solid in the same manner as in Example (47a).
MS(FAB) m/z:345 $(M + H)^+$.

(165b) 4-{4-[3-(2-chloro-5-methyl-phenyl)-ureido]-phenyl}-piperazine-l-carboxylic acid (2-chloro-6-methyl-phenyl)-amide

87 mg (84%) of the title compound was obtained from 1-(2-chloro-5-methylphenyl-phenyl)-3-(4-piperazin-1-yl-phenyl)-urea (69 mg) obtained in Example (165a) and 2-chloro-6-methylphenyl isocyanate (0.041 mL) as a pale orange powder in the same manner as in Example 49.
$^1$H-NMR spectrum (400MHz,DMSO-d6):δ(ppm)=8.20(2H, brs), 8.00(1H, s), 7.32(2H, d, J=9.0Hz), 7.31-7.28(1H, m), 7.28(1H, d, J=8.2Hz), 7.20(1H, d, J=6.2Hz), 7.14(1H, dd, J=7.6 and 7.6Hz), 6.94(2H, d, J=9.0Hz), 6.81(2H, d, J=8.2Hz), 3.60(4H, t, J=4.7Hz), 3.08(4H, t, J=5.1Hz), 2.27(3H, s), 2.20(3H, s).
MS(FAB) m/z:512 $(M + H)^+$.
Melting point: >260˚C (dec).

(Example 166) 4- {4-[3-(2-methoxy-5-methyl-phenyl)-ureido]-phenyl}-piperazine-1-carboxylic acid (4-amino-2-trifluoromethyl-phenyl)-amide (Compound No. 1-408)

(166a) 4-{4-[3-(2-methoxy-5-methyl-phenyl)-ureido]-phenyl}-piperazine-1-carboxylic acid (4-nitro-2-trifluoromethyl-phenyl)-amide

1.10 g (96%) of the title compound was obtained from 1-(2-methoxy-5-methyl-phenyl)-3-(4-piperazin-1-yl-phenyl)-urea (680 mg) obtained in Example (47a) and 4-nitro-2-trifluoromethylphenyl isocyanate (604 mg) as an orange powder in the same manner as in Example 49.
MS(FAB) m/z:573 $(M + H)^+$.

(166b) 4-{4-[3-(2-methoxy-5-methyl-phenyl)-ureido]-phenyl}-piperazine-1-carboxylic acid (4-amino-2-trifluoromethyl-phenyl)-amide

A suspension of 4-{4-[3-(2-methoxy-5-methyl-phenyl)-ureido]-phenyl}-piperazine-1-carboxylic acid (4-nitro-2-trifluoromethyl-phenyl)-amide (1.10 g) obtained in Example (166a) and 10% palladium-carbon catalyst (0.11 g) in anhydrous tetrahydrofuran (20 mL) was stirred at room temperature under a hydrogen atmosphere. Dimethylacetamide was added to the reaction mixture which was then filtered. The filtrate was diluted with ethyl acetate and washed with water and saturated brine and dried over sodium sulfate and filtered and concentrated.

The residue was purified by column chromatography (dichloromethane:ethyl acetate 5:1 → 1:1 and dichloromethane:methanol 20:1 → 5:1). The obtained solid was recrystallized from ethyl acetate again and 712 mg (69%) of the title compound was obtained as a yellow powder.
$^1$H-NMR spectrum (400MHz,DMSO-d6):δ(ppm)=9.08(1H, s), 8.06(1H, s), 7.98(2H, d, J=3.9Hz), 7.33(2H, d, J=8.2Hz), 6.98(1H, d, J=8.6Hz), 6.93(2H, d, J=8.2Hz), 6.88(1H, d, J=8.2Hz), 6.84(1H, s), 6.73(2H, dd, J=9.6 and 9.6Hz), 5.47(2H, s), 3.84(3H, s), 3.53(4H, brs), 3.03(4H, t, J=4.1Hz), 2.23(3H, s).
MS(FAB) m/z:543 $(M + H)^+$.
Melting point: 244-246˚C.

(Example 167) 4-{4-[3-(2-methoxy-5-methyl-phenyl)-ureido]-phenyl}-piperazine-1-carboxylic acid (2-amino-6-methyl-phenyl)-amide (Compound No. 1-414)

667 mg (73%) of the title compound was obtained as a pale yellow powder from 4-{4-[3-(2-methoxy-S-methyl-phenyl)-ureido]-phenyl}-piperazine-1-carboxylic acid (2-methyl-6-nitro-phenyl)-amide (975 mg) obtained in Example 60 in the same manner as in Example (166b).
$^1$H-NMR spectrum (400MHz,DMSO-d6):δ(ppm)=9.05(1H, s), 8.04(1H, s), 7.96(1H, d, J=2.0Hz), 7.67(1H, s), 7.31(2H, d, J=9.0Hz), 6.92(2H, d, J=8.6Hz), 6.86(1H, d, J=8.2Hz), 6.81(1H, dd, J=7.4 and 7.4Hz), 6.70(1H, dd, J=8.8 and 2.2Hz), 6.54(1H, d, J=8.2Hz), 6.41(1H, d, J=7.5Hz), 4.66(2H, s), 3.82(3H, s), 3.58(4H, t, J=4.9Hz), 3.07(4H, t, J=4.9Hz), 2.22 (3H, s), 2.05(3H, s).
MS(FAB) m/z:489 $(M + H)^+$.
Melting point: 198-199˚C.

(Example 168) 4-{4-[3-(2-methoxy-5-methyl-phenyl)-ureido]-phenyl}-piperazine-1-carboxylic acid (4-amino-2-chloro-phenyl)-amide (Compound No. 1-420)

(168a) 4- {4-[3-(2-methoxy-5-methyl-phenyl)-ureido]-phenyl} -piperazine-1 - carboxylic acid (4-nitro-2-chloro-phenyl)-amide

1.07 g (100%) of the title compound was obtained from 1-(2-methoxy-5-methyl-phenyl)-3-(4-piperazin-1-yl-phenyl)-urea (680 mg) obtained in Example (47a) and 2-chloro-4-nitrophenyl isocyanate (516 mg) as an orange powder in the same manner as in Example 49.
MS(FAB) m/z:539 $(M + H)^+$.

(168b) 4-{4-[3-(2-methoxy-5-methyl-phenyl)-ureido]-phenyl}-piperazine-1-carboxylic acid (4-amino-2-chloro-phenyl)-amide

201 mg (20%) of the title compound was obtained as a yellow powder from 4-{4-[3-(2-methoxy-5-methyl-phenyl)-ureido]-phenyl}-piperazine-1-carboxylic acid (4-nitro-2-chloro-phenyl)-amide (1.07 g) obtained in Example (168a) in the same manner as in Example (166b).
$^1$H-NMR spectrum (400MHz,DMSO-d6):δ(ppm)=9.05(1H, s), 8.03(1H, s), 7.98(1H, s), 7.96(1H, d, J=2.4Hz), 7.30(2H, d, J=9.0Hz), 6.95(2H, d, J=9.0Hz), 6.91(1H, d, J=9.0Hz), 6.86(1H, d, J=8.2Hz), 6.70(1H, d, J=7.0Hz), 6.61(1H, d, J=2.3Hz), 6.45(1H, dd, J=8.4 and 2.6Hz), 5.23(2H, s), 3.82(3H, s), 3.53(4H, t, J=4.3Hz), 3.04(4H, t, J==5.1Hz), 2.22 (3H, s).
MS(FAB) m/z:509 $(M + H)^+$.
Melting point: 226-228˚C.

(Example 169) 4-{4-[3-(2-methoxy-5-methyl-phenyl)-ureido]-phenyl}-piperazine-1-carboxylic acid (4-acetylamino-2-chloro-phenyl)-amide (Compound No. 1-426)

Acetyl chloride (0.08 mL) was added to a solution of dimethylacetamide (5 mL) of 4-{4-[3-(2-methoxy-5-methyl-phenyl)-ureido]-phenyl}-piperazine-1-carboxylic acid (4-amino-2-chloro-phenyl)-amide (56 mg) obtained in Example (168b) at room temperature. After four days, the reaction mixture was diluted with ethyl acetate and washed with water and concentrated. The residue was purified by preparative TLC (dichloromethane/methanol 10:1). The obtained solid was vigorously stirred with hexane/isopropyl ether (5:1), collected by filtration and dried under reduced pressure, and 24 mg of the title compound was obtained as a yellow powder.
$^1$H-NMR spectrum (400MHz,DMSO-d6):δ(ppm)=10.0(1H, s), 9.06(1H, s), 8.23(1H, s), 8.04(1H, s), 7.96(1H, d, J=1.1 Hz), 7.81(1H, s), 7.34(2H, s), 7.31(2H, d, J=8.2Hz), 6.92(2H, d, J=9.0Hz), 6.86(1H, d, J=8.2Hz), 6.70(1H, d, J=8.6Hz), 3.81(3H, s), 3.56(4H, brs), 3.05(4H, t, J=4.1Hz), 2.20(3H, s), 2.02(3H, s).
MS(FAB) m/z:551 $(M + H)^+$.
Melting point: 155-157˚C.

(Example 170) 4- {4-[3-(2-methoxy-5-methyl-phenyl)-ureido]-phenyl}-piperazine-1-carboxylic acid (2-methoxy-6-methyl-phenyl)-amide (Compound No. 1-89)

A solution of 2-methoxy-6-methylphenylaniline (137 mg) and diisopropylethylamine (0.38 mL) in anhydrous dichloromethane (5 mL) was added dropwise slowly to a solution of triphosgene (119 mg) in anhydrous dichloromethane (5 mL) at room temperature. After the dropwise addition was completed, a solution of 1-(2-methoxy-5-methyl-phenyl)-3-(4-piperazin-1-yl-phenyl)-urea (340 mg) obtained in Example (47a) and diisopropylethylamine (0.38 mL) in anhydrous dichloromethane (5 mL) was added dropwise slowly to the reaction mixture at room temperature. After 3 hours, the reaction mixture was concentrated and diluted with ethyl acetate and water. The deposited solid was collected by filtration, washed with hexane and ethyl acetate and dried under reduced pressure, and 361 mg (72%) of the title compound was obtained as a white powder.
$^1$H-NMR spectrum (400MHz,DMSO-d6):δ(ppm)=9.10(1H, s), 8.07(1H, s), 7.99(1H, d, J=1.5Hz), 7.77(1H, s), 7.34(2H, d, J=8.6Hz), 7.10(1H, dd, J=7.9 and 7.9Hz), 6.96(2H, d, J=9.0Hz), 6.96(1H, d, J=9Hz), 6.88(1H, dd, J=7.4 and 7.4Hz), 6.82(1H, dd, J=8.4 and 8.4Hz), 6.72(1H, d, J=8.2Hz), 3.84(3H, s), 3.73(3H, s), 3.59(4H, t, J=4.1Hz), 3.07(4H, t, J=5.1Hz), 2.23(3H, s), 2.12(3H, s).
MS(FAB) m/z:504 $(M + H)^+$.
Melting point: 200-203˚C.

(Example 171) 4-{4-[3-(2-methoxy-5-methyl-phenyl)-ureido]-phenyl}-piperazine-1-carboxylic acid (4-bromo-2-cyano-phenyl)-amide (Compound No. 1-432)

77 mg (14%) of the title compound was obtained as a pale yellow powder from 1-(2-methoxy-5-methyl-phenyl)-3-(4-piperazin-1-yl-phenyl)-urea (340 mg) obtained in Example (47a) and 4-bromo-2-cyanoaniline (197 mg) in the same manner as in Example 170.
$^1$H-NMR spectrum (400MHz,DMSO-d6):δ(ppm)=9.08(1H, s), 9.07(1H, s), 8.06(1H, s), 8.04(1H, d, J=2.3Hz), 7.99(1H, d, J=1.9Hz), 7.82(1H, dd, J=9.0 and 2.3Hz), 7.39(1H, d, J=8.9Hz), 7.34(2H, d, J=9.0Hz), 6.95(2H, d, J=9.0Hz), 6.88 (1H, d, J=8.6Hz), 6.73(1H, dd, J=7.8 and 1.9Hz), 3.84(3H, s), 3.62(4H, t, J=4.9Hz), 3.10(4H, t, J=4.6Hz), 2.23(3H, s)
MS(FAB) m/z:563 $(M + H)^+$.
Melting point: 149-151˚C.

(Example 172) 4-{4-[3-(2-methoxy-5-methyl-phenyl)-ureido]-phenyl}-piperazine-1-carboxylic acid (4-fluoro-2-methyl-phenyl)-amide (Compound No. 1-438)

203 mg (83%) of the title compound was obtained as a white powder from 1-(2-methoxy-5-methyl-phenyl)-3-(4-piperazin-1-yl-phenyl)-urea (170 mg) obtained in Example (47a) and 4-fluoro-2-methylaniline (63 mg) in the same manner as in Example 170.
$^1$H-NMR spectrum (400MHz,DMSO-d6):δ(ppm)=9.06(1H, s), 8.14(1H, s), 8.04(1H, s), 7.96(1H, d, J=2.0Hz), 7.31(2H, d, J=9.0Hz), 7.16(1H, dd, J=8.8 and 5.7Hz), 7.03(1H, dd, J=9.4 and 2.7Hz), 6.96-6.93(1H, m), 6.92(2H, d, J=8.7Hz), 6.86(1H, d, J=8.2Hz), 6.70(1H, dd, J=8.0 and 1.4Hz), 3.82(3H, s), 3.57(4H, t, J=4.5Hz), 3.07(4H, t, J=4.7Hz), 2.22(3H, s), 2.16(3H, s).
MS(FAB) m/z:492 $(M + H)^+$.
Melting point: >270˚C (dec).

(Example 173) 4-{4-[3-(2-methoxy-5-methyl-phenyl)-ureido]-phenyl}-piperazine-1-carboxylic acid (4-acetylamino-2-trifluoromethyl-phenyl)-amide (Compound No. 1-173)

191 mg (66%) of the title compound was obtained as a white powder from 1-(2-methoxy-5-methyl-phenyl)-3-(4-piperazin-1-yl-phenyl)-urea (170 mg) obtained in Example (47a) and 2-amino-5-acetamino-benzotrifluoride (109 mg) in the same manner as in Example 170.
$^{1}$H-NMR spectrum (400MHz,DMSO-d6):δ(ppm)=10.2(1H, s), 9.06(1H, s), 8.24(1H, s), 8.04(1H, s), 7.99(1H, d, J=2.4Hz), 7.96(1H, d, J=2.0Hz), 7.72(1H, dd, J=8.6 and 2.0Hz), 7.31(1H, dd, J=7.9 and 7.9Hz), 7.30(2H, d, J=8.6Hz), 6.92(2H, d, J=9.0Hz), 6.86(1H, d, J=8.2Hz), 6.70(1H, dd, J=8.0 and 1.4Hz), 3.82(3H, s), 3.55(4H, t, J=4.5Hz), 3.05(4H, t, J=4.3Hz), 2.22(3H, s), 2.07(3H, s).
MS(FAB) m/z:585 $(M + H)^+$.
Melting point: 152-154˚C.

(Example 174) 4-{4-[3-(2-methoxy-5-methyl-phenyl)-ureido]-phenyl}-piperazine-1-carboxylic acid (4-chloro-2,6-dimethyl-phenyl)-amide (Compound No. 1-450)

218 mg (83%) of the title compound was obtained as a white powder from 1-(2-methoxy-5-methyl-phenyl)-3-(4-piperazin-1-yl-phenyl)-urea (170 mg) obtained in Example (47a) and 4-chloro-2,6-dimethylaniline (78 mg) in the same manner as in Example 170.
$^{1}$H-NMR spectrum (400MHz,DMSO-d6):δ(ppm)=9.05(1H, s), 8.04(1H, s), 7.97(1H, s), 7.96(1H, d, J=1.6Hz), 7.31(2H, d, J=9.0Hz), 7.12(2H, s), 6.92(2H, d, J=9.0Hz), 6.86(1H, d, J=8.2Hz), 6.70(1H, dd, J=8.2 and 2.0Hz), 3.82(3H, s), 3.59 (4H, t, J=4.5Hz), 3.06(4H, t, J=5.1Hz), 2.22(3H, s), 2.14(6H, s).
MS(FAB) m/z:522 $(M + H)^+$.
Melting point: 238-240˚C.

(Example 175) 4-{4-[3-(2-methoxy-5-methyl-phenyl)-ureido]-phenyl}-piperazine-1-carboxylic acid (4-methoxy-2-trifluoromethyl-phenyl)-amide (Compound No. 1-456)

224 mg (80%) of the title compound was obtained as a light brown powder from 1-(2-methoxy-5-methyl-phenyl)-3-(4-piperazin-1-yl-phenyl)-urea (170 mg) obtained in Example (47a) and 4-methoxy-2-trifluoromethylaniline (96 mg) in the same manner as in Example 170.
$^{1}$H-NMR spectrum (400MHz,DMSO-d6):δ(ppm)=9.05(1H, s), 8.20(1H, s), 8.04(1H, s), 7.96(1H, d, J=1.6Hz), 7.31(3H, d, J=9.0Hz), 7.19(1H, dd, J=8.6 and 2.7Hz), 7.15(1H, d, J=3.1Hz), 6.92(2H, d, J=9.0Hz), 6.86(1H, d, J=8.6Hz), 6.70 (1H, dd, J=8.2 and 1.6Hz), 3.82(6H, s), 3.55(4H, t, J=4.9Hz), 3.04(4H, t, J=4.9Hz), 2.22(3H, s).
MS(FAB) m/z:558 $(M + H)^+$.
Melting point: 245-247˚C.

(Example 176) 4-{4-[3-(2-methoxy-5-methyl-phenyl)-ureido]-phenyl}-piperazine-1-carboxylic acid (5,6,7,8,-tetrahydronaphthalen-1-yl)-amide (Compound No. 1-462)

221 mg (86%) of the title compound was obtained as a pale purple powder from 1-(2-methoxy-5-methyl-phenyl)-3-(4-piperazin-1-yl-phenyl)-urea (170 mg) obtained in Example (47a) and 5,6,7,8-tetrahydro-1-naphthylamine (74 mg) in the same manner as in Example 170.
$^{1}$H-NMR spectrum (400MHz,DMSO-d6):δ(ppm)=9.05(1H, s), 8.04(1H, s), 7.97(1H, d, J=1.9Hz), 7.96(1H, s), 7.31(2H, d, J=9.0Hz), 7.03-6.98(2H, m), 6.92(2H, d, J=9.4Hz), 6.86(2H, d, J=8.2Hz), 6.70(1H, dd, J=8.2 and 1.6Hz), 3.82(3H, s), 3.56(4H, t, J=4.9Hz), 3.06(4H, t, J=5.1Hz), 2.72(2H, brs), 2.57(2H, brs), 2.22(3H, s), 1.69(4H, t, J=3.4Hz).
MS(FAB) m/z:514 $(M + H)^+$.
Melting point: 205-208˚C.

(Example 177) 4-{4-[3-(2-methoxy-5-methyl-phenyl)-ureido]-phenyl}-piperazine-l-carboxylic acid (2-bromo-6-methylphenyl)-amide (Compound No. 1-468)

228 mg (83%) of the title compound was obtained as a white powder from 1-(2-methoxy-5-methyl-phenyl)-3-(4-piperazin-1-yl-phenyl)-urea (170 mg) obtained in Example (47a) and 2-bromo-6-methylaniline (93 mg) in the same manner as in Example 170.
$^{1}$H-NMR spectrum (400MHz,DMSO-d6):δ(ppm)=9.05(1H, s), 8.23(1H, s), 8.04(1H, s), 7.96(1H, d, J=2.0Hz), 7.46(1H, dd, J=7.4 and 1.2Hz), 7.31(2H, d, J=9.0Hz), 7.23(1H, d, J=7.0Hz), 7.08(1H, dd, J=7.8 and 7.8Hz), 6.93(2H, d, J=9.4Hz), 6.86(1H, d, J=8.2Hz), 6.71(1H, dd, J=7.8 and 1.6Hz), 3.82(3H, s), 3.60(4H, t, J=4.9Hz), 3.07(4H, t, J=5.1Hz), 2.22(3H, s), 2.21(3H, s).
MS(FAB) m/z:552 $(M + H)^+$.
Melting point: 221-222˚C.

(Example 178) 4- {4-[3-(2-methoxy-5-methyl-phenyl)-ureido]-phenyl} -piperazine-1-carboxylic acid (4-isopropyl-2-trifluoromethyl-phenyl)-amide (Compound No. 1-474)

239 mg (84%) of the title compound was obtained as a light brown powder from 1-(2-methoxy-5-methyl-phenyl)-3-(4-piperazin-1-yl-phenyl)-urea (170 mg) obtained in Example (47a) and 4-isopropyl-2-(trifluoromethyl) aniline (0.087 mL) in the same manner as in Example 170.
$^1$H-NMR spectrum (400MHz,DMSO-d6):δ(ppm)=9.06(1H, s), 8.23(1H, s), 8.04(1H, s), 7.96(1H, d, J=2.0Hz), 7.50(1H, d, J=7.1Hz), 7.49(1H, s), 7.33(1H, d, J=6.7Hz), 7.31(2H, d, J=9.0Hz), 6.92(2H, d, J=9.4Hz), 6.86(1H, d, J=8.2Hz), 6.70 (1H, dd, J=8.2 and 1.6Hz), 3.82(3H, s), 3.56(4H, t, J=4.9Hz), 3.05(4H, t, J=4.9Hz), 3.02-2.95(1H, m), 2.22(3H, s), 1.23 (6H, d, J=6.7Hz).
MS(FAB) m/z:570 $(M + H)^+$.
Melting point: 134-135˚C.

(Example 179) 2-[(4-{4-[3-(2-methoxy-5-methyl-phenyl)-ureido]-phenyl}-piperazine-1-carbonyl)-amino]-3-methyl-benzoic acid (Compound No. 1-480)

(179a) 2-amino-3-methylbenzoic acid methyl ester

A solution of 2-amino-3-methylbenzoic acid (5.10 g) and concentrated sulfuric acid (5 mL) in methanol (200 mL) was heated under reflux for 24 hours.

The reaction mixture was concentrated and was neutralized with a saturated sodium hydrogen carbonate aqueous solution. Insolubles were mixed with ethyl acetate and filtered. The filtrate was partitioned in a separatory funnel and the separated organic layer was washed with saturated brine, dried over sodium sulfate, filtered and concentrated. The residue was purified by column chromatography (dichloromethane/ethyl acetate 4:1) and 1.31 g (24%) of the title compound was obtained as a light brown oil.

(179b) 2-[(4-{4-[3-(2-methoxy-5-methyl-phenyl)-ureido]-phenyl}-piperazine-1-carbonyl)-amino]-3-methyl-benzoic acid methyl ester

452 mg (85%) of the title compound was obtained as a flesh colour powder from 1-(2-methoxy-5-methyl-phenyl)-3-(4-piperazin-1-yl-phenyl)-urea (340 mg) obtained in Example (47a) and 2-amino-3-methylbenzoic acid methyl ester (165 mg) obtained in Example (179a) in the same manner as in Example 170.
MS(FAB) m/z:532 $(M + H)^+$.

(179c) 2-[(4-{4-[3-(2-methoxy-5-methyl-phenyl)-ureido]-phenyl}-piperazine-l-carbonyl)-amino]-3-methyl-benzoic acid

2-[(4-{4-[3-(2-methoxy-5-methyl-phenyl)-ureido]-phenyl}-piperazine-1-carbonyl)-amino]-3-methyl-benzoic acid methyl ester (266 mg) obtained in Example (179b) was stirred in 1N aqueous sodium hydroxide (1 1 mL)/tetrahydrofuran (4 mL)/methanol (2 mL) at room temperature for seven days. The reaction mixture was neutralized with 1N hydrochloric acid aqueous solution (1 mL), followed by addition of water. The deposited solid was collected by filtration, washed with water and dried under reduced pressure, and 235 mg (91%) of the title compound was obtained as a white powder.
$^1$H-NMR spectrum (400MHz,DMSO-d6):δ(ppm)=9.06(1H, s), 8.04(1H, s), 7.96(1H, d, J=2.0Hz), 7.64(1H, dd, J=7.2 and 1.3Hz), 7.37-7.34(1H, m), 7.36(1H, dd, J=7.1 and 1.2Hz), 7.31(2H, d, J=9.0Hz), 7.11(1H, dd, J=8.0 and 8.0Hz), 6.92 (2H, d, J=9.0Hz), 6.86(1H, d, J=8.2Hz), 6.71(1H, dd, J=8.2 and 2.4Hz), 3.82(3H, s), 3.60(4H, t, J=5.1Hz), 3.08(4H, t, J=4.9Hz), 2.22(3H, s), 2.19(3H, s).
MS(FAB) m/z:518 $(M + H)^+$.
Melting point: 159-160˚C.

(Example 180) 4-[(4-{4-[3-(2-methoxy-5-methyl-phenyl)-ureido]-phenyl}-piperazine-1-carbonyl)-amino]-3-methyl-benzoic acid (Compound No. 1-159)

(180a) 4-amino-3-methylbenzoic acid methyl ester

257 mg (24%) of the title compound was obtained as a beige solid from 4-amino-3-methylbenzoic acid (1.00 g) in the same manner as in Example (179a).

(180b) 4-[(4-{4-[3-(2-methoxy-5-methyl-phenyl)-ureido]-phenyl}-piperazine-1-carbonyl)-amino]-3-methyl-benzoic acid methyl ester

429 mg (81 %) of the title compound was obtained as a light brown powder from 1-(2-methoxy-5-methyl-phenyl)-3-(4-piperazin-1-yl-phenyl)-urea (340 mg) obtained in Example (47a) and 4-amino-3-methylbenzoic acid methyl ester (165 mg) obtained in Example (180a) in the same manner as in Example 170.
$^1$H-NMR spectrum (400MHz,DMSO-d6):δ(ppm)=9.09(1H, s), 8.28(1H, s), 8.06(1H, s), 7.99(1H, d, J=1.6Hz), 7.80(1H, d, J=2.0Hz), 7.75(1H, dd, J=8.2 and 2.0Hz), 7.49(1H, d, J=8.6Hz), 7.34(2H, d, J=9.0Hz), 6.95(2H, d, J=9.0Hz), 6.89 (1H, d, J=8.2Hz), 6.73(1H, dd, J=8.4 and 1.4Hz), 3.84(3H, s), 3.83(3H, s), 3.62(4H, t, J=4.9Hz), 3.09(4H, t, J=4.9Hz), 2.26(3H, s), 2.23(3H, s).
MS(FAB) m/z:532 $(M + H)^+$.
Melting point: 131-132˚C.

(180c) 4-[(4-{4-[3-(2-methoxy-5-methyl-phenyl)-ureido]-phenyl}-piperazine-1-carbonyl)-amino]-3-methyl-benzoic acid

238 mg (92%) of the title compound was obtained as a yellowish grey powder from 4-[(4-{4-[3-(2-methoxy-5-methyl-phenyl)-ureido]-phenyl}-piperazine-1-carbonyl)-amino]-3-methyl-benzoic acid methyl ester (266 mg) obtained in Example (180b) in the same manner as in Example (179c).
$^1$H-NMR spectrum (400MHz,DMSO-d6):δ(ppm)=9.07(1H, s), 8.24(1H, s), 8.04(1H, s), 7.96(1H, d, J=2.4Hz), 7.74(1H, d, J=2.0Hz), 7.69(1H, dd, J=8.0 and 2.2Hz), 7.41(1H, d, J=8.2Hz), 7.31(2H, d, J=8.6Hz), 6.93(2H, d, J=9.0Hz), 6.86 (1H, d, J=8.6Hz), 6.70(1H, dd, J=8.0 and 1.8Hz), 3.82(3H, s), 3.60(4H, t, J=4.9Hz), 3.08(4H, t, J=4.7Hz), 2.24(3H, s), 2.22(3H, s).
MS(FAB) m/z:518 $(M + H)^+$.
Melting point: 178-180˚C.

(Example 181) 3-chloro-4-[(4-{4-[3-(2-methoxy-5-methyl-phenyl)-ureido]-phenyl}-piperazine-1-carbonyl)-amino]-benzoic acid (Compound No. 1-131)

(181a) 3-chloro-4-[(4-{4-[3-(2-methoxy-5-methyl-phenyl)-ureido]-phenyl}-piperazine-1-carbonyl)-amino]-benzoic acid methyl ester

318 mg (58%) of the title compound was obtained as a white powder from 1-(2-methoxy-5-methyl-phenyl)-3-(4-piperazin-1-yl-phenyl)-urea (340 mg) obtained in Example (47a) and 4-amino-3-chlorobenzoic acid methyl ester (185 mg) in the same manner as in Example 170.
MS(FAB) m/z:552 $(M + H)^+$.

(181b) 3-chloro-4-[(4-{4-[3-(2-methoxy-5-methyl-phenyl)-ureido]-phenyl}-piperazine-1-carbonyl)-amino]-benzoic acid

140 mg (87%) of the title compound was obtained as a purple grey powder from 3-chloro-4-[(4-{4-[3-(2-methoxy-5-methyl-phenyl)-ureido]-phenyl}-piperazine-1-carbonyl)-amino]-benzoic acid methyl ester (166 mg) obtained in Example (181a) in the same manner as in Example (179b).
$^1$H-NMR spectrum (400MHz,DMSO-d6):δ(ppm)=9.08(1H, s), 8.42(1H, s), 8.05(1H, s), 7.96(1H, d, J=2.0Hz), 7.91(1H, d, J=1.5Hz), 7.83(1H, dd, J=8.6 and 2.0Hz), 7.76(1H, d, J=8.3Hz), 7.32(2H, d, J=9.0Hz), 6.92(2H, d, J=9.0Hz), 6.86 (1H, d, J=8.2Hz), 6.70(1H, dd, J=8.8 and 2.2Hz), 3.82(3H, s), 3.62(4H, t, J=5.1Hz), 3.10(4H, t, J=4.7Hz), 2.22(3H, s).
MS(FAB) m/z:538 $(M + H)^+$.
Melting point: 209-210˚C.

(Example 182) 4-{4-[3-(5-chloro-2-methoxy-phenyl)-ureido]-phenyl}-piperazine-1-carboxylic acid (2-methoxy-6-methyl-phenyl)-amide (Compound No. 1-87)

464 mg (89%) of the title compound was obtained as a light brownish tan powder from 1-(5-chloro-2-methoxy-phenyl)-3-(4-piperazin-1-yl-phenyl)-urea (360 mg) obtained in Example (67a) and 2-methoxy-6-methylaniline (137 mg) in the same manner as in Example 170.
$^1$H-NMR spectrum (400MHz,DMSO-d6):δ(ppm)=9.15(1H, s), 8.28(1H, s), 8.22(1H, d, J=2.4Hz), 7.74(1H, s), 7.31(2H, d, J=9.0Hz), 7.07(1H, dd, J=7.9 and 7.8Hz), 7.00(1H, d, J=8.6Hz), 6.96-6.92(3H, m), 6.82(1H, d, J=8.6Hz), 6.78(1H, d, J=7.4Hz), 3.87(3H, s), 3.72(3H, s), 3.57(4H, t, J=4.7Hz), 3.06(4H, t, J=4.7Hz), 2.11(3H, s).
MS(FAB) m/z:524 $(M + H)^+$.
Melting point: 165-167˚C.

(Example 183) 4-{4-[3-(2-ethoxy-phenyl)-ureido]-phenyl}-piperazine-1-carboxylic acid (2-methoxy-6-methyl-phenyl)-amide (Compound No. 1-91)

117 mg (23%) of the title compound was obtained as a white powder from 1-(2-ethoxy-phenyl)-3-(4-piperazin-1-yl-phenyl)-urea (340 mg) obtained in Example (80a) and 2-methoxy-6-methylaniline (137 mg) in the same manner as in Example 170.
$^1$H-NMR spectrum (400MHz,DMSO-d6):δ(ppm)=9.14(1H, s), 8.10(1H, d, J=5.5Hz), 7.95(1H, s), 7.74(1H, s), 7.32(2H, d, J=9.0Hz), 7.07(1H, dd, J=7.8 and 7.8Hz), 6.99-6.77(5H, m), 6.93(2H, d, J=8.6Hz), 4.12(2H, q, J=7.2Hz), 3.72(3H, s), 3.57(4H, t, J=5.0Hz), 3.06(4H, t, J=5.5Hz), 2.11(3H, s), 1.41(3H, t, J=6.9Hz).
MS(FAB) m/z:504 $(M + H)^+$.
Melting point: 169-171˚C.

(Example 184) 4-{4-[3-(2-ethoxy-5-methyl-phenyl)-ureido]-phenyl}-piperazine-1-carboxylic acid (2-methoxy-6-methyl-phenyl)-amide (Compound No. 1-90)

340 mg (66%) of the title compound was obtained as a yellowish flesh colour powder from 1-(2-ethoxy-5-methylphenyl)-3-(4-piperazin-1-yl-phenyl)-urea (354 mg) obtained in Example (83a) and 2-methoxy-6-methylaniline (137 mg) in the same manner as in Example 170.
$^1$H-NMR spectrum (400MHz,DMSO-d6):δ(ppm)=9.13(1H, s), 7.96(1H, d, J=2.0Hz), 7.89(1H, s), 7.74(1H, s), 7.32(2H, d, J=9.0Hz), 7.07(1H, dd, J=8.1 and 8.0Hz), 6.93(2H, d, J=9.0Hz), 6.85(1H, d, J=8.2Hz), 6.82(IH, d, J=10.2Hz), 6.78 (1H, d, J=7.4Hz), 6.68(1H, dd, J=8.2 and 2.0Hz), 4.07(2H, q, J=6.9Hz), 3.72(3H, s), 3.57(4H, t, J=4.7Hz), 3.06(4H, t, J=5.1Hz), 2.21(3H, s), 2.11(3H, s), 1.38(3H, t, J=6.9Hz).
MS(FAB) m/z:518 $(M + H)^+$.
Melting point: 142-144˚C.

(Example 185) 4-{4-[3-(2-methoxy-5-methyl-phenyl)-ureido]-phenyl}-piperazine-1-carboxylic acid (2-hydroxymethyl-6-methyl-phenyl)-amide (Compound No. 1-75)

(185a) 2-(tert-butyl-dimethyl-silanoxymethyl)-6-methyl-phenylamine

Imidazole (817 mg) and tert-butyldimethylsilyl chloride (1.09 g) were added to a solution of (2-amino-3-methyl-phenyl)-methanol (823 mg) in dimethylformamide (10 mL) at room temperature. The reaction mixture was stirred at room temperature for one hour, diluted with ethyl acetate and washed with a saturated sodium hydrogen carbonate aqueous solution and brine, dried over sodium sulfate and concentrated, and 1.51 g (100%) of the title compound was obtained as a colourless transparent oil.
MS(FAB) m/z:252 $(M + H)^+$.

(185b) 4-{4-[3-(2-methoxy-5-methyl-phenyl)-ureido]-phenyl}-piperazine-1-carboxylic acid [2-(tert-butyl-dimethyl-silanoxymethyl)-6-methyl-phenyl]-amide

1.01 g (82%) of the title compound was obtained as a white powder from 1-(2-methoxy-5-methyl-phenyl)-3-(4-piperazin-1-yl-phenyl)-urea (681 mg) obtained in Example (47a) and 2-(tert-butyl-dimethyl-silanoxymethyl)-6-methyl-phenylamine (502 mg) obtained in Example (185a) in the same manner as in Example 170.
$^1$H NMR(400MHz,DMSO-d6):δ(ppm)=9.09(1H, s), 8.07(1H, s), 7.99(1H, d, J=2.3Hz), 7.93(1H, s), 7.34(2H, d, J=9.0Hz), 7.28(1H, d, J=7.4Hz), 7.18(1H, d, J=7.4Hz), 7.16-7.12(1H, m), 6.95(1H, d, J=9.0Hz), 6.89(2H, d, J=8.2Hz), 6.73(1H, dd, J=8.4 and 1.8Hz), 4.65(2H, s), 3.84(3H, s), 3.60(4H, t, J=4.1Hz), 3.07(4H, t, J=4.5Hz), 2.23(3H, s), 2.16(3H, s), 0.90 (9H, s), 0.05(6H, s).

(185c) 4-{4-[3-(2-methoxy-5-methyl-phenyl)-ureido]-phenyl}-piperazine-1-carboxylic acid (2-hydroxymethyl-6-methyl-phenyl)

A tetrabutylammonium fluoride solution (in anhydrous tetrahydrofuran, 1 mol/L, 3.3 mL) was added to a solution of 4-{4-[3-(2-methoxy-5-methyl-phenyl)-ureido]-phenyl}-piperazine-1-carboxylic acid [2-(tert-butyl-dimethyl-silanoxymethyl)-6-methyl-phenyl]-amide (1.01 g) obtained in Example (185b) in anhydrous tetrahydrofuran (15 mL) at room temperature. The reaction mixture was stirred at room temperature for four hours and diluted with ethyl acetate and washed with potassium hydrogensulfate solution and concentrated. The residue was purified by column chromatography (dichloromethane/methanol 20:1 → 10:1). The obtained solid was recrystallized from ethyl acetate and 363 mg (44%) of the title compound was obtained as a beige powder.

$^1$H-NMR spectrum (400MHz,DMSO-d6):δ(ppm)=9.06(1H, s), 8.04(1H, s), 7.96(1H, d, J=1.6Hz), 7.93(1H, s), 7.31(2H, d, J=9.0Hz), 7.27(1H, dd, J=7.1 and 2.3Hz), 7.14-7.08(2H, m), 6.93(2H, d, J=9.0Hz), 6.86(1H, d, J=8.6Hz), 6.71(1H, dd, J=8.2 and 2.4Hz), 5.03(1H, t, J=5.5Hz), 4.44(2H, d, J=5.5Hz), 3.82(3H, s), 3.59(4H, t, J=4.9Hz), 3.07(4H, t, J=4.7Hz), 2.22(3H, s), 2.15(3H, s).
MS(FAB) m/z:504 $(M + H)^+$.
Melting point: 192-194˚C.

(Example 186) 4-{5-[3-(2-methoxy-5-methyl-phenyl)-ureido]-pyridin-2-yl}-piperazine-1-carboxylic acid (2-methoxy-6-methyl-phenyl)-amide (Compound No. 1-96)

38 mg (38%) of the title compound was obtained as a white powder from 1-(2-methoxy-5-methyl-phenyl)-3-(6-piperazin-1-yl-pyridin-3-yl)-urea (64 mg) obtained in Example (137a) and 2-methoxy-6-methylaniline (28 mg) in the same manner as in Example 170.
$^1$H-NMR spectrum (400MHz,DMSO-d6):δ(ppm)=9.07(1H, s), 8.14(1H, d, J=2.7Hz), 8.09(1H, s), 7.94(1H, d, J=2.0Hz), 7.73(1H, dd, J=8.6 and 2.7Hz), 7.07(1H, dd, J=7.8 and 8.2Hz), 6.87(2H, d, J=8.6Hz), 6.83(1H, d, J=8.2Hz), 6.78(1H, d, J=7.8Hz), 6.72(1H, dd, J=8.6 and 2.0Hz), 3.83(3H, s), 3.72(3H, s), 3.56-3.51(4H, m), 3.45-3.40(4H, m), 2.22(3H, s), 2.11(3H, s).
MS(ES) m/z:505 $(M + H)^+$.
Melting point: 198-200˚C.

(Example 187) 4-{5-[3-(2-ethoxy-phenyl)-ureido]-pyridin-2-yl}-piperazine-1-carboxylic acid (2-methoxy-6-methyl-phenyl)-amide (Compound No. 1-98)

62 mg (41%) of the title compound was obtained as a white powder from 1-(2-ethoxy-phenyl)-3-(4-piperazin-1-yl-pyridin-3-yl)-urea (102 mg) obtained in Example (141a) and 2-methoxy-6-methylaniline (41 mg) in the same manner as in Example 170.
$^1$H-NMR spectrum (400MHz,DMSO-d6):δ(ppm)=9.20(1H, s), 8.19(1H, d, J=2.7Hz), 8.12(1H, dd, J=7.8 and 1.9Hz), 8.03 (1H, s), 7.77(1H, dd, J=8.6 and 2.7Hz), 7.77(1H, s), 7.09(1H, dd, J=8.0 and 8.2Hz), 7.00(1H, dd, J=7.8 and 2.0Hz), 6.95-6.78(5H, m), 4.13(2H, q, J=6.9Hz), 3.73(3H, s), 3.58-3.52(4H, m), 3.48-3.41(4H, m), 2.12(3H, s), 1.42(3H, t, J=6.9Hz).
MS(FAB) m/z:505 $(M + H)^+$.
Melting point: 129-130˚C.

(Example 188) 4-{5-[3-(2-methoxy-5-methyl-phenyl)-ureido]-pyridin-2-yl}-piperazine-1-carboxylic acid (4-acetylamino-2-trifluoromethyl-phenyl)-amide (Compound No. 1-180)

56 mg (31%) of the title compound was obtained as a white powder from 1-(2-methoxy-5-methyl-phenyl)-3-(6-piperazin-1-yl-pyridin-3-yl)-urea (64 mg) obtained in Example (137a) and 4-acetylamino-1-amino-2-trifluoromethyl benzene (65 mg) in the same manner as in Example 170.
$^1$H-NMR spectrum (400MHz,DMSO-d6):δ(ppm)= 10.20(1H, s), 9.07(1H, s), 8.24(1H, s), 8.14(1H, d, J=2.3Hz), 8.09(1H, s), 7.99(1H, d, J=2.7Hz), 7.94(1H, d, J=2.7Hz), 7.77-7.69(2H, m), 7.33(1H, d, J=9.0Hz), 6.87(1H, d, J=8.0Hz), 6.87(1H, d, J=9.0Hz), 6.71(1H, dd, J=8.0 and 2.7Hz), 3.83(3H, s), 3.55-3.49(4H, m), 3.45-3.39(4H, m), 2.22(3H, s), 2.07(3H, s).
MS(ES) m/z:586 $(M + H)^+$.
Melting point: 175-177˚C.

(Example 189)

1-{4-[4-(bicyclo[2.2.1]heptane-2-carbonyl)-piperazin-1-yl]-phenyl}-3-(2-methoxy-5-methyl-phenyl)-urea (Compound No. 1-485)

Dimethylformamide (one drop) and oxalyl chloride (0.1 mL) were added dropwise to a solution of bicyclo[2.2.1] heptane-2-carboxylic acid (31 mg) in anhydrous tetrahydrofuran (1 mL) at room temperature. After one hour, the reaction mixture was concentrated and dissolved in dimethylacetamide (1 mL). 1-(2-methoxy-5-methyl-phenyl)-3-(4-piperazin-1-yl-phenyl)-urea (68 mg) obtained in Example (47a) was added dropwise to this mixture at room temperature. After it was stirred at room temperature for 20 hours, it was heated at 80˚C for five hours. The reaction mixture was diluted with a sodium hydrogen carbonate aqueous solution, followed by extraction with ethyl acetate. The organic layer was washed with water and saturated brine and dried over sodium sulfate and filtered and concentrated. The residue was purified by column chromatography (dichloromethane/ethyl acetate 5:3). The obtained solid was vigorously stirred in hexane/isopropyl ether (5:1), collected by filtration and dried under reduced pressure, and 37 mg (40%) of the title compound was obtained as a white solid.
$^1$H-NMR spectrum (400MHz,DMSO-d6):δ(ppm)=9.09(1H, s), 8.06(1H, s), 7.98(1H, s), 7.33(2H, d, J=8.2Hz), 6.90(2H, d, J=8.2Hz), 6.88(1H, d, J=8.2Hz), 6.73(1H, d, J=8.2Hz), 3.83(3H, s), 3.75-3.52(4H, m), 3.15-2.92(5H, m), 2.45-2.40 (1H, m), 2.25-2.19(1H, m), 2.23(3H, s), 1.80-1.73(1H, m), 1.56-1.39(3H, m), 1.33-1.19(4H, m).
MS(FAB) m/z:463 $(M + H)^+$.
Melting point: 126-128˚C.

(Example 190) 1-(2-methoxy-5-methyl-phenyl)-3-{4-[4-(2-methyl-cyclohexanecarbonyl)-piperazin-1-yl]-phenyl}-urea (Compound No. 1-486)

A solution of 2-methyl-cyclohexanecarboxylic acid (0.042 mL), 1-(2-methoxy-5-methyl-phenyl)-3-(4-piperazin-1-yl-phenyl)-urea (102 mg) obtained in Example (47a), triethylamine (0.084 mL) and 1-propanephosphonic acid cyclic anhydride (50% ethyl acetate solution, 0.23 mL) in ethyl acetate (1 mL) was stirred at room temperature for one hour. A saturated sodium hydrogen carbonate aqueous solution was added to the reaction mixture, stirred at room temperature for 3.5 hours, followed by extraction with ethyl acetate. The organic layer was washed with water and saturated brine and dried over sodium sulfate and filtered and concentrated.

The residue was purified by column chromatography (dichloromethane/ethyl acetate 5:3). The obtained solid was vigorously stirred in a dichloromethane/hexane (5:1) solution, collected by filtration and dried under reduced pressure, and 66 mg (48%) of the title compound was obtained as a white solid.
$^1$H-NMR spectrum (400MHz,$CDCl_3$):δ(ppm)=7.94(1H, d, J=2.0Hz), 7.26(2H, d, J=8.8Hz), 7.09(1H, brs), 6.90(2H, d, J=8.8Hz), 6.77(1H, dd, J=8.2 and 2.0Hz), 6.72(1H, d, J=8.2Hz), 6.49(1H, brs), 3.92-3.83(1H, m), 3.77(3H, s), 3.74-3.57 (3H, m), 3.24-2.98(4H, m), 2.75-2.68(1H, m), 2.29(3H, s), 2.07-1.99(1H, m), 1.93-1.73(2H, m), 1.71-1.64(1H, m), 1.60-1.39(4H, m), 1.33-1.22(1H, m), 0.94(3H, d, J=7.0Hz).
MS(FAB) m/z:465 $(M + H)^+$.
Melting point: 208-209˚C.

(Example 191) 1-[4-(4-benzyl-piperazin-1-yl)-phenyl]-3-(2-methoxy-5-methylphenyl)-urea (Compound No. 1-487)

A solution of 1-(2-methoxy-5-methyl-phenyl)-3-(4-piperazin-1-yl-phenyl)-urea (102 mg) obtained in Example (47a) and benzaldehyde (0.037 mL) in acetic acid (1 mL) was stirred at room temperature for 20 minutes. Anhydrous tetrahydrofuran (3 mL) was added to the reaction mixture and cooled with an ice bath, followed by addition of triacetoxysodium borohydride (70 mg). After one hour, the reaction mixture was warmed to room temperature, followed by gradual addition of triacetoxysodium borohydride till the reaction was finished. After the reaction was completed, 1N aqueous sodium hydroxide solution was added to the reaction mixture, followed by extraction with ethyl acetate. The organic layer was washed with water and saturated brine, dried over sodium sulfate, filtered and concentrated. The residue was purified by column chromatography (dichloromethane/ethyl acetate 5:3). The obtained solid was vigorously stirred in isopropyl ether, collected by filtration and dried under reduced pressure, and 39 mg (31 %) of the title compound was obtained as a white solid.
$^1$H-NMR spectrum (400MHz,DMSO-d6):δ(ppm)=9.02(1H, s), 8.02(1H, s), 7.95(1H, d, J=2.4Hz), 7.34-7.29(1H, m), 7.31 (2H, s), 7.27(2H, d, J=9.0Hz), 7.26(2H, d, J=9.0Hz), 7.28-7.22(1H, m), 6.85(1H, d, J=8.4Hz), 6.85(2H, d, J=9.0Hz), 6.70 (1H, dd, J=8.4 and 2.4Hz), 3.82(3H, s), 3.51(2H, s), 3.07-3.02(4H, m), 2.53-2.47(4H, m), 2.21(3H, s).
MS(FAB) m/z:431 $(M + H)^+$.
Melting point: 184-186˚C.

(Example 192) 1-(4-{4-[2-(2,6-difluoro-phenyl)-acetyl]-piperazin-1-yl}-phenyl)-3-(2-methoxy-5-methyl-phenyl)-urea (Compound No. 1-488)

42 mg (42%) of the title compound was obtained as a white crystal from 2,6-difluorophenyl acetic acid (38 mg) and 1-(2-methoxy-5-methyl-phenyl)-3-(4-piperazin-1-yl-phenyl)-urea (102 mg) obtained in Example (47a) in the same manner as in Example 189.
$^1$H-NMR spectrum (400MHz,DMSO-d6):δ(ppm)=9.09(1H, s), 8.07(1H, s), 7.99(1H, d, J=1.9Hz), 7.39-7.31(1H, m), 7.34 (2H, d, J=9.0Hz), 7.08(2H, dd, J=7.8 and 7.9Hz), 6.94(2H, d, J=9.0Hz), 6.89(1H, d, J=8.3Hz), 6.73(1H, dd, J=8.3 and 1.9Hz), 3.84(3H, s), 3.82(2H, s), 3.79-3.72(2H, m), 3.65-3.58(2H, m), 2.23-3.10(2H, m), 3.04-3.00(2H, m), 2.23(3H, s).
MS(FAB) m/z:495 $(M)^+$.
Melting point: 213-215˚C.

(Example 193) 1-(2-methoxy-5-methyl-phenyl)-3-[4-(4-pyrimidin-2-yl-piperazin-1-yl)-phenyl]-urea (Compound No. 1-489)

A solution of 1-(2-methoxy-5-methyl-phenyl)-3-(4-piperazin-1-yl-phenyl)-urea (68 mg) obtained in Example (47a) and 2-chloropyrimidine (69 mg) in dimethylacetamide (10 mL) was stirred at room temperature for 17 hours. The reaction mixture was diluted with ethyl acetate and washed with a saturated sodium hydrogen carbonate aqueous solution and dried over sodium sulfate and filtered and concentrated. The residue was purified by column chromatography (dichloromethane/methanol 30:1 → 12:1). The obtained solid was vigorously stirred in the hexane/water and was collected by filtration and dried under reduced pressure, and 57 mg (27%) of the title compound was obtained as a pale orange powder.
$^1$H-NMR spectrum (400MHz,DMSO-d6):δ(ppm)=9.06(1H, s), 8.37(2H, d, J=4.7Hz), 8.04(1H, s), 7.96(1H, d, J=2.0Hz), 7.31 (2H, d, J=9.0Hz), 6.93(2H, d, J=9.0Hz), 6.86(1H, d, J=8.2Hz), 6.70(1H, dd, J=8.4 and 1.8Hz), 6.64(1H, dd, J=4.7 and 4.7Hz), 3.86(4H, t, J=5.1Hz), 3.82(3H, s), 3.11(4H, t, J=5.3Hz), 2.22(3H, s).
MS(FAB) m/z:419 $(M + H)^+$.
Melting point: 220-221 ˚C.

(Example 194) 4-{4-[3-(2-methoxy-5-methyl-phenyl)-ureido]-3-methyl-phenyl}-piperazine-1-carboxylic acid tert-butyl ester (Compound No. 2-41)

(194a) 4-(3-methyl-4-nitro-phenyl)-piperazine-1-carboxylic acid tert-butyl ester

A suspension of 5-fluoro-2-nitro-toluene (1.55 g), piperazine-1-carboxylic acid tert-butyl ester (1.86 g) and potassium carbonate (1.38 g) in dimethylacetamide (40 mL) was heated under reflux for 15.5 hours. The reaction mixture was diluted with ethyl acetate and washed with a saturated sodium hydrogen carbonate aquous solution, water and saturated brine and dried over sodium sulfate and filtered and concentrated. The residue was purified by column chromatography (hexane/ethyl acetate 10:1 → 2:1) and 2.57 g (80%) of the title compound was obtained as a yellow solid (following a method of Tetrahedron Lett. 38, 4091 (1997)).

(194b) 4-(4-amino-3-methyl-phenyl)-piperazine-1-carboxylic acid tert-butyl ester

A suspension of 4-(3-methyl-4-nitro-phenyl)-piperazine-1-carboxylic acid tert-butyl ester (2.57 g) obtained in Example (194a) and 10% palladium-carbon catalyst (0.26 g) in anhydrous tetrahydrofuran (40 mL) was stirred at room temperature under a hydrogen atmosphere for two hours. The reaction mixture was filtered in KC flock and concentrated. The residue was vigorously stirred in hexane/isopropyl ether and was collected by filtration and dried under reduced pressure, and 2.25 g (97%) of the title compound was obtained as a pale pink solid.
MS(FAB) m/z:292 $(M + H)^+$.

(194c) 4-{4-[3-(2-methoxy-5-methyl-phenyl)-ureido]-3-methyl-phenyl}-piperazine-1-carboxylic acid tert-butyl ester

A solution of 4-(4-amino-3-methyl-phenyl)-piperazine-1-carboxylic acid tert-butyl ester (58 mg) obtained in Example (194b) and 2-methoxy-5-methylphenyl isocyanate (0.3 mL) in anhydrous tetrahydrofuran (5 mL) was stirred at room temperature for two hours. Methanol was added to the reaction mixture which was then concentrated. The residue was purified by column chromatography (dichloromethane/ethyl acetate 10:1 → 1:1), and 48 mg (53%) of the title compound was obtained as a white solid.
$^1$H-NMR spectrum (400MHz,DMSO-d6):δ(ppm)=8.32(1H, s), 8.31(1H, s), 7.95(1H, d, J=1.6Hz), 7.46(1H, d, J=8.6Hz), 6.85(1H, d, J=8.2Hz), 6.79(1H, d, J=2.3Hz), 6.73(1H, dd, J=8.8 and 2.5Hz), 6.69(1H, d, J=8.2Hz), 3.81(3H, s), 3.45-3.41 (4H, t, J=4.9Hz), 3.00(4H, t, J=4.9Hz), 2.19(3H, s), 2.17(3H, s), 1.40(9H, s).
MS(FAB) m/z:455 $(M + H)^+$.
Melting point: 169-171 ˚C.

(Example 195) 4-{4-[3-(2-methoxy-5-methyl-phenyl)-ureido]-2-methyl-phenyl}-piperazine-1-carboxylic acid tert-butyl ester (Compound No. 2-43)

(195a) 4-(2-methyl-4-nitro-phenyl)-piperazine-1-carboxylic acid tert-butyl ester

1.15 g (36%) of the title compound was obtained as a yellow solid from 2-fluoro-5-nitro-toluene (1.55 g) and piperazine-1-carboxylic acid tert-butyl ester (1.86 g) in the same manner as in Example (194a).
$^1$H NMR(400MHz, $CDCl_3$):δ(ppm)=8.04(1H, dd, J=5.1 and 2.8Hz), 8.02(1H, d, J=2.8Hz), 6.98(1H, d, J=8.6Hz), 3.60(4H, t, J=5.1Hz), 2.96(4H, t, J=4.9Hz), 2.38(3H, s), 1.49(9H, s).

(195b) 4-(4-amino-2-methyl-phenyl)-piperazine-1-carboxylic acid tert-butyl ester

0.870 g (83%) of the title compound was obtained as a pale pink solid from 4-(2-methyl-4-nitro-phenyl)-piperazine-1-carboxylic acid tert-butyl ester (1.15 g) obtained in Example (195a) in the same manner as in Example (194b).
MS(FAB) m/z:292 $(M + H)^+$.

(195c) 4-{4-[3-(2-methoxy-5-methyl-phenyl)-ureido]-2-methyl-phenyl}-piperazine-1-carboxylic acid tert-butyl ester

24 mg (26%) of the title compound was obtained as a pale pink solid from 4-(4-amino-2-methyl-phenyl)-piperazine-1-carboxylic acid tert-butyl ester (58 mg) obtained in Example (195b) and 2-methoxy-5-methylphenyl isocyanate (0.3 mL) in the same manner as in Example (194c).
$^1$H-NMR spectrum (400MHz,DMSO-d6):δ(ppm)=9.10(1H, s), 8.07(1H, s), 7.97(1H, d, J=1.2Hz), 7.27(1H, d, J=1.9Hz), 7.18(1H, dd, J=8.6 and 2.4Hz), 6.93(1H, d, J=8.6Hz), 6.86(1H, d, J=8.2Hz), 6.71(1H, d, J=7.8Hz), 3.81(3H, s), 3.43(4H, brs), 2.71(4H, t, J=4.5Hz), 2.22(3H, s), 2.20(3H, s), 1.40(9H, s).
MS(FAB) m/z:455 $(M + H)^+$.
Melting point: 103-105˚C.

(Example 196) 1-{4-[3-(2-methoxy-5-methyl-phenyl)-ureido]-phenyl}-piperidine-4-carboxylic acid (2-chloro-6-methyl-phenyl)-amide (Compound No. 2-19)

(196a) 1-(4-nitro-phenyl)-piperidine-4-carboxylic acid ethyl ester

A suspension of isonipecotic acid ethyl ester (5.04 g), 4-fluoronitrobenzene (2.30 g) and potassium carbonate (2.26 g) in acetonitrile (100 mL) was heated under reflux for 22.5 hours. The reaction mixture was diluted with ethyl acetate and washed with water, a saturated sodium hydrogen carbonate aqueous solution and saturated brine and dried over sodium sulfate and filtered and concentrated. The residue was purified by column chromatography (dichloromethane/ethyl acetate 10:1) and 4.08 g (90%) of the title compound was obtained as a yellow solid.
MS(EI) m/z: 278 $(M^+)$.

(196b) 1-(4-amino-phenyl)-piperidine-4-carboxylic acid ethyl ester

A suspension of 1-(4-nitro-phenyl)-piperidine-4-carboxylic acid ethyl ester (2.83 g) obtained in Example (196a) and 10% palladium-carbon catalyst (0.542 g) in anhydrous tetrahydrofuran (60 mL) was stirred under a hydrogen atmosphere at room temperature for 24 hours. The reaction mixture was filtered, concentrated and dried under reduced pressure, and the title compound (3.08 g) was obtained as black oil.
MS(FAB) m/z:249 $(M + H)^+$.

(196c) 1-{4-[3-(2-methoxy-5-methylphenyl)-ureido]-phenyl}-piperidine-4-carboxylic acid ethyl ester

A solution of 2-methoxy-5-methylphenyl isocyanate (1.94 g) in anhydrous tetrahydrofuran (10 mL) was added to a solution of 1-(4-amino-phenyl)-piperidine-4-carboxylic acid ethyl ester (2.69 g) obtained in Example (196b) in anhydrous tetrahydrofuran (20 mL). After five hours, methanol was added to the reaction mixture which was then concentrated. The residue was vigorously stirred in isopropyl ether and was collected by filtration and dried under reduced pressure, and 3.98 g (90%) of the title compound was obtained as a pale grey solid.
MS(FAB) m/z:412 $(M + H)^+$.

(196d) 1-{4-[3-(2-methoxy-5-methylphenyl)-ureido]-phenyl}-piperidine-4-carboxylic acid

A solution of 1-{4-[3-(2-methoxy-5-methylphenyl)-ureido]-phenyl}-piperidine-4-carboxylic acid ethyl ester (3.91 g) obtained in Example (196c) in a 1N aqueous sodium hydroxide solution (20 mL) and tetrahydrofuran (60 mL) was heated under reflux for 2.5 hours. 1N hydrochloric acid (30 mL) was added to the reaction mixture, and the organic solvent was removed under reduced pressure. The residue was mixed with isopropyl ether and stirred till a solid deposited. The deposited solid was collected by filtration and washed with water and isopropyl ether and dried under reduced pressure, and 3.84 g (100%) of the title compound was obtained as a grey solid.
MS(FAB) m/z:384 $(M + H)^+$.

(196e) 1-{4-[3-(2-methoxy-5-methyl-phenyl)-ureido]-phenyl}-piperidine-4-carboxylic acid (2-chloro-6-methyl-phenyl)-amide

A solution of 1-{4-[3-(2-methoxy-5-methylphenyl)-ureido]-phenyl}-piperidine-4-carboxylic acid (103 mg) obtained in Example (196d), 2-chloro-6-methylaniline (42 mg), 1-propanephosphonic acid cyclic anhydride (50% ethyl acetate solution, 0.32 mL) and triethylamine (0.11 mL) in dimethylacetamide (5 mL) was stirred at room temperature for 17 hours, and then heated at 80˚C for nine hours. The reaction mixture was diluted with ethyl acetate and washed with a saturated sodium hydrogen carbonate aqueous solution and saturated brine and dried over sodium sulfate and concentrated. The residue was purified by column chromatography (dichloromethane/ethyl acetate 2:1 $\rightarrow$ 0:1 and dichloromethane/methanol 5:1) and 26 mg (19%) of the title compound was obtained as a light brown solid.
$^1$H-NMR spectrum (400MHz,DMSO-d6):δ(ppm)=9.51(1H, s), 9.06(1H, s), 8.05(1H, s), 7.99(1H, s), 7.37-7.27(2H, m), 7.31(1H, d, J=9.0Hz), 7.26-7.18(m, 2H), 6.92(2H, d, J=8.6Hz), 6.88(1H, d, J=8.2Hz), 6.72(1H, d, J=10.9Hz), 3.84(3H, s), 3.66(2H, d, J=11.4Hz), 3.34-3.27(1H, m), 2.73-2.63(2H, m), 2.23(3H, s), 2.18(3H, s), 1.97-1.89(2H, m), 1.87-1.75 (2H, m).
MS(FAB) m/z:507 $(M + H)^+$.
Melting point: >250˚C (dec).

(Example 197) 4-{4-[3-(2-methoxy-5-methyl-phenyl)-ureido]-phenyl}-piperazine-1-carboxylic acid 2-chloro-6-methylphenyl ester (Compound No. 1-490)

(197a) Chloroformic acid (2-chloro-6-methylphenyl)

Triphosgene (0.736 g) and pyridine (0.60 mL) were added subsequently to a solution of 2-chloro-6-methylphenol (1.06 g) in anhydrous dichloromethane (10 mL) at 0˚C. After 10 minutes, the reaction mixture was warmed to room temperature and stirred for 15 hours and filtered. The filtrate was diluted with ethyl acetate and washed with water, 1N hydrochloric acid, 1N aqueous sodium hydroxide solution and saturated brine and dried over sodium sulfate and concentrated, and the title compound was obtained as a purple oil.
MS(EI) m/z:204 $(M^+)$.

(197b) 4-{4-[3-(2-methoxy-5-methyl-phenyl)-ureido]-phenyl}-piperazine-1-carboxylic acid 2-chloro-6-methyl-phenyl ester

A suspension (10 mL) of 1-(2-methoxy-5-methyl-phenyl)-3-(4-piperazin-1-yl-phenyl)-urea (86 mg) obtained in Example (47a) and chloroformic acid (2-chloro-6-methylphenyl) (63 mg) obtained in Example (197a) in anhydrous tetrahydrofuran was stirred at room temperature for three hours. The reaction mixture was diluted with ethyl acetate and washed with a saturated sodium hydrogen carbonate aqueous solution and saturated brine and dried over sodium sulfate and filtered and concentrated. The residue was purified by column chromatography (dichloromethane/ethyl acetate 4:1). The obtained colourless oil was vigorously stirred in hexane/isopropyl ether (5:1), the deposited solid was collected by filtration and dried under reduced pressure, and 94 mg (72%) of the title compound was obtained as a white solid.
$^1$H-NMR spectrum (400MHz,DMSO-d6):δ(ppm)=9.13(1H, s), 8.08(1H, s), 7.99(1H, s), 7.39(1H, d, J=7.9Hz), 7.35(2H, d, J=9.0Hz), 7.28(1H, d, J=7.4Hz), 7.19(1H, dd, J=7.7 and 7.7Hz), 6.96(2H, d, J=9.0Hz), 6.89(1H, d, J=8.2Hz), 6.73 (1H, d, J=7.8Hz), 3.84(3H, s), 3.61(4H, brs), 3.14(4H, brs), 2.23(3H, s), 2.20(3H, s).
MS(FAB) m/z:509 $(M + H)^+$.
Melting point: 146-148˚C.

(Example 198) 4-{4-[3-(2-methoxy-5-methyl-phenyl)-ureido]-phenyl}-2-methylpiperazine-1-carboxylic acid (2-chloro-6-methyl-phenyl)-amide (Compound No. 2-13)

(198a) 3-methyl-1-(4-nitro-phenyl)-piperazine

A suspension of 2-methylpiperazine (1.03 g), 4-fluoronitrobenzene (0.55 mL) and potassium carbonate (0.745 g) in acetonitrile (20 mL) was heated under reflux for 3.5 hours. The reaction mixture was diluted with ethyl acetate and washed with water and saturated brine and dried over sodium sulfate and filtered and concentrated. The residue was vigorously stirred in isopropyl ether, and the deposited solid was collected by filtration and dried under reduced pressure to obtain 884 mg (77%) of the title compound as a yellow solid.
MS(FAB) m/z:222 $(M + H)^+$.

(198b) 2-methyl-4-(4-nitro-phenyl)-piperazine-1-carboxylic acid tert-butyl ester

A solution of 3-methyl-1-(4-nitro-phenyl)-piperazine (0.662 g) obtained in Example (198a) and di-tert-butyl dicarbonate (0.780 g) in anhydrous tetrahydrofuran (20 mL) was stirred at room temperature for 18.5 hours. The reaction mixture was diluted with ethyl acetate and washed with a saturated sodium hydrogen carbonate aqueous solution and saturated brine and dried over sodium sulfate and filtered and concentrated and dried under reduced pressure to obtain 1.18 g of the title compound as a yellow solid.
MS(FAB) m/z:322 $(M + H)^+$.

(198c) 2-methyl-4-(4-amino-phenyl)-piperazine-1-carboxylic acid tert-butyl ester

A suspension of 2-methyl-4-(4-nitro-phenyl)-piperazine-1-carboxylic acid tert-butyl ester (1.18 g) obtained in Example (198b) and 10% palladium-carbon catalyst (160 mg) in anhydrous tetrahydrofuran (20 mL) was stirred under a hydrogen atmosphere at room temperature for 13.5 hours and filtered and concentrated. The residue was purified by column chromatography (dichloromethane/ethyl acetate 1: 1) and 476 mg of the title compound was obtained as a dark purple oil.
MS(EI) m/z:291 $(M^+)$.

(198d) 4- {4-[3-(2-methoxy-5-methyl-phenyl)-ureido]-phenyl}-2-methyl-piperazine-1-carboxylic acid tert-butyl ester

A solution of 2-methyl-4-(4-amino-phenyl)-piperazine-1-carboxylic acid tert-butyl ester (0.476 g) obtained in Example (198c) and 2-methoxy-5-methylphenyl isocyanate (0.39 g) in anhydrous tetrahydrofuran (10 mL) was stirred at room temperature for 12 hours. Methanol was added to the reaction mixture which was then concentrated. The residue was purified by column chromatography (dichloromethane/ethyl acetate 4:1) to obtain brown oil. The obtained oil was stirred vigorously in isopropyl ether. The deposited solid was collected by filtration and dried under reduced pressure, and 99 mg of the title compound was obtained as a pale purple solid.
$^1$H-NMR spectrum (400MHz,DMSO-d6):δ(ppm)=9.08(1H, s), 8.05(1H, s), 7.98(1H, d, J=1.9Hz), 7.32(2H, d, J=8.6Hz), 6.89(1H, d, J=2.0Hz), 6.87(1H, s), 6.72(1H, dd, J=8.2 and 1.9Hz), 4.20(1H, brs), 3.83(3H, s), 3.80(1H, d, J=12.5Hz), 3.46(1H, d, J=12.0Hz), 3.37(1H, d, J=12.1Hz), 3.32(1H, d, J=9.4Hz), 3.14(1H, ddd, J=12.0, 12.0, 4.0Hz), 2.71(1H, dd, J=11.8 and 3.9Hz), 2.54-2.51(1H, m), 2.22(3H, s), 1.42(9H, s), 1.22(3H, d, J==7.0Hz).
MS(FAB) m/z:455 $(M + H)^+$.
Melting point: 167-168˚C.

(198e) 1-(2-methoxy-5-methyl-phenyl)-3-[4-(3-methyl-piperazin-1-yl)-phenyl]-urea

Trifluoroacetic acid (1.2 mL) was added dropwise to a solution of 4-{4-[3-(2-methoxy-5-methyl-phenyl)-ureido]-phenyl}-2-methyl-piperazine-1-carboxylic acid tert-butyl ester (370 mg) obtained in Example (198d) and anisole (1.3 mL) in anhydrous dichloromethane (10 mL) at room temperature. After 18 hours, the reaction mixture was concentrated and was neutralized with a saturated sodium hydrogen carbonate aqueous solution and stirred in isopropyl ether. The supernatant was removed and allowed to stand till the residue solidified. The deposited solid was collected by filtration and dried under reduced pressure and 283 mg (98%) of the title compound was obtained as a brown solid.
MS(FAB) m/z:355 $(M + H)^+$.

(198f) 4-{4-[3-(2-methoxy-5-methyl-phenyl)-ureido]-phenyl}-2-methyl-piperazine-1-carboxylic acid (2-chloro-6-methyl-phenyl)-amide

A solution of 1-(2-methoxy-5-methyl-phenyl)-3-[4-(3-methyl-piperazin-1-yl)-phenyl]-urea (60 mg) obtained in Example (198e) and 2-chloro-6-methylphenyl isocyanate (59 mg) in anhydrous tetrahydrofuran (5 mL) was stirred at room temperature for 14 hours. Methanol was added to the reaction mixture which was then concentrated. The residue was purified by column chromatography (dichloromethane/ethyl acetate 2:1) to obtain brown oil. The obtained oil was vigorously stirred in isopropyl ether/ethyl acetate (5:1), and the deposited solid was collected by filtration and dried under reduced pressure, and 38 mg (43%) of the title compound was obtained as a light brown solid.
$^1$H-NMR spectrum (400MHz,DMSO-d6):δ(ppm)=9.10(1H, s), 8.16(1H, s), 8.07(1H, s), 7.99(1H, s), 7.33(3H, d, J=8.6Hz), 7.22(1H, d, J=7.5Hz), 7.17(1H, dd, J=7.6 and 7.6Hz), 6.94(2H, d, J=9.0Hz), 6.89(1H, d, J=8.6Hz), 6.73(1H, d, J=8.2Hz), 4.41(1H, brs), 3.97(1H, d, J=12.0Hz), 3.84(3H, s), 3.54(1H, d, J=9.2Hz), 3.45(1H, d, J=9.2Hz), 3.22(1H, t, J=12.2Hz), 2.78(1H, d, J=8.2Hz), 2.60(1H, t, J=10.6Hz), 2.23(3H, s), 2.20(3H, s), 1.33(3H, d, J=6.2Hz).
MS(FAB) m/z:522 $(M + H)^+$.
Melting point: 161-163˚C.

(Example 199) 4-{4-[3-(2-methoxy-5-methyl-phenyl)-ureido]-phenyl}-2-methylpiperazine-1-carboxylic acid (2,6-dimethyl-phenyl)-amide (Compound No. 2-33)

36 mg (42%) of the title compound was obtained as a light brown solid from 1-(2-methoxy-5-methyl-phenyl)-3-[4-(3-methyl-piperazin-1-yl)-phenyl]-urea (60 mg) obtained in Example (198e) and 2,6-dimethylphenyl isocyanate (45 mg) in the same manner as in Example (198f).
$^1$H-NMR spectrum (400MHz,DMSO-d6):δ(ppm)=9.10(1H, s), 8.07(1H, s), 7.99(1H, s), 7.89(1H, s), 7.33(2H, d, J=9.0Hz), 7.05(3H, s), 6.94(2H, d, J=9.0Hz), 6.89(1H, d, J=8.2Hz), 6.73(1H, dd, J=10.4 and 3.8Hz), 4.40(1H, brs), 3.97(1H, d, J=14.3Hz), 3.84(3H, s), 3.53(2H, d, J=8.0Hz), 3.45-3.17(1H, m), 2.78(1H, d, J=12.1Hz), 2.65-2.51(1H, m), 2.23(6H, s), 2.15(3H, s), 1.31(3H, d, J=6.7Hz).
MS(FAB) m/z:502 $(M + H)^+$.
Melting point: 204-206˚C.

(Example 200) 4-{4-[3-(2-methoxy-5-methyl-phenyl)-ureido]-phenyl}-perhydro-1,4-diazepine-1-carboxylic acid (2-chloro-6-methyl-phenyl)-amide (Compound No. 2-9) (200a) 4-(4-nitro-phenyl)-perhydro-1,4-diazepine-carboxylic acid tert-butyl ester

3.13 g (75%) of the title compound was obtained as a yellow solid from 1-homopiperazine carboxylic acid tert-butyl ester (4.96 g) and 4-fluoronitrobenzene (1.84 g) in the same manner as in Example (198a).
MS(FAB) m/z:322 $(M + H)^+$.

(200b) 4-(4-amino-phenyl)-perhydro-1,4-diazepine-carboxylic acid tert-butyl ester

2.97 g (100%) of the title compound was obtained as a dark brown oil from 4-(4-nitro-phenyl)-perhydro-1,4-diazepine-carboxylic acid tert-butyl ester 3.13 g obtained in Example (200a) in the same manner as in Example (198c).
MS(FAB) m/z:292 $(M + H)^+$.

(200c) 4-{4-[3-(2-methoxy-5-methyl-phenyl)-ureido]-phenyl}-perhydro-1,4-diazepine-1-carboxylic acid tert-butyl ester

1.27 g (75%) of the title compound was obtained as an orange oil from 4-(4-amino-phenyl)-perhydro-1,4-diazepine-carboxylic acid tert-butyl ester (1.08 g) obtained in Example (200b) and 2-methoxy-5-methylphenyl isocyanate in the same manner as in Example (198d).
MS(FAB) m/z:455 $(M + H)^+$.

(200 d) 1-(2-methoxy-5-methyl-phenyl)-3-(4-perhydro-1,4-diazepin-1-yl-phenyl)-urea

1.07 g (100%) of the title compound was obtained as a dark brown amorphous material from 4-{4-[3-(2-methoxy-5-methyl-phenyl)-ureido]-phenyl}-perhydro-1,4-diazepine-1-carboxylic acid tert-butyl ester (1.27 g) obtained in Example (200c) in the same manner as in Example (198e).
MS(FAB) m/z:355 $(M + H)^+$.

(200e) 4-{4-[3-(2-methoxy-5-methyl-phenyl)-ureido]-phenyl}-perhydro-1,4-diazepine-1-carboxylic acid (2-chloro-6-methyl-phenyl)-amide

172 mg (95%) of the title compound was obtained as a beige solid from 1-(2-methoxy-5-methyl-phenyl)-3-(4-perhydro-1,4-diazepin-1-yl-phenyl)-urea (123 mg) obtained in Example (200d) and 2-chloro-6-methylphenyl isocyanate (80 mg) in the same manner as in Example (198f).
$^1$H NMR(400MHz,DMSO-d6):δ(ppm)=8.92(1H, s), 8.01(1H, s), 7.99(1H, d, J=2.0Hz), 7.94(1H, s), 7.30(1H, d, J=6.3Hz), 7.25(2H, d, J=9.0Hz), 7.18(1H, d, J=5.5Hz), 7.15(1H, d, J=7.5Hz), 6.88(1H, d, J=8.3Hz), 6.71(3H, d, J=9.0Hz), 3.83(3H, s), 3.63-3.50(4H, m), 3.40-3.26 (4H, m), 2.23(3H, s), 2.09(3H, s), 2.05-1.95(2H, m).
MS(ES) m/z:522 $(M + H)^+$.
Melting point: >240˚C (dec).

(Example 201)4-{4-[3-(2-methoxy-5-methyl-phenyl)-ureido]-phenyl}-perhydro-1,4-diazepine-1-carboxylic acid (2,6-dimethyl-phenyl)-amide (Compound No. 2-29)

106 mg (58%) of the title compound was obtained as a beige solid from 1-(2-methoxy-5-methyl-phenyl)-3-(4-perhydro-1,4-diazepin-1-yl-phenyl)-urea (129 mg) obtained in Example (200d) and 2,6-dimethylphenyl isocyanate (73 mg) in the same manner as in Example (198f).
$^1$H NMR(400MHz,DMSO-d6):δ(ppm)=8.89(1H, s), 7.98(1H, s), 7.97(1H, s), 7.64(1H, s), 7.22(2H, d, J=9.0Hz), 6.98(3H, s), 6.85(1H, d, J=9.0Hz), 6.68(3H, d, J=9.0Hz), 3.80(3H, s), 3.61-3.47(4H, m), 3.38-3.22(4H, m), 2.20(3H, s), 2.03(6H, s), 1.98-1.89(2H, m).
MS(ES) m/z:502 $(M + H)^+$.
Melting point: >240˚C (dec).

(Example 202) 6-[3-(2-methoxy-5-methyl-phenyl)-ureido]-3,4-dihydrido-1H-isoquinoline-2-carboxylic acid (2-chloro-6-methyl-phenyl)-amide (Compound No. 3-14)

(202a) 6-[3-(2-methoxy-5-methyl-phenyl)-ureido]-3,4-dihydrido-1H-isoquinoline-2-carboxylic acid tert-butyl ester

A solution of 6-amino-3,4-dihydro-1H-isoquinoline-2-carboxylic acid tert-butyl ester (0.0451 g) and 2-methoxy-5-methylphenyl isocyanate (0.0392 g) in anhydrous tetrahydrofuran (10 mL) was stirred at room temperature for 15 hours. Methanol was added to the reaction mixture which was then concentrated. The residue was purified by column chromatography (dichloromethane/ethyl acetate 10:1). The obtained solid was vigorously stirred in hexane/isopropyl ether and was collected by filtration and dried under reduced pressure, and 548 mg (73%) of the title compound was obtained as a white solid.
$^1$H-NMR spectrum (400MHz,DMSO-d6):δ(ppm)=9.24(1H, s), 8.14(1H, s), 7.99(1H, s), 7.35(1H, s), 7.19(1 H, d, J=7.1 Hz), 7.07(1H, d, J=8.2Hz), 6.89(1H, d, J=8.2Hz), 6.74(1H, d, J=8.2Hz), 4.43(2H, brs), 3.84(3H, s), 3.54(2H, t, J=5.5Hz), 2.75(2H, t, J=5.7Hz), 2.23(3H, s), 1.43(9H, s).
MS(FAB) m/z:412 $(M + H)^+$.
Melting point: 183-185˚C.

(202b) 1-(2-methoxy-5-methyl-phenyl)-3-(1,2,3,4-tetrahydro-isoquinolin-6-yl)-urea

Trifluoroacetic acid (1.5 mL) was added dropwise to a solution of 6-[3-(2-methoxy-5-methyl-phenyl)-ureido]-3,4-dihydrido-1H-isoquinoline-2-carboxylic acid tert-butyl ester (0.406 g) obtained in Example (202a) and anisole (1.6 mL) in anhydrous dichloromethane (10 mL) at room temperature. After 24 hours, the reaction mixture was concentrated and was neutralized with a saturated sodium hydrogen carbonate aqueous solution, followed by addition of hexane and stirring at room temperature. The supernatant was removed, and it was vigorously stirred in hexane and further vigorously stirred in a saturated sodium hydrogen carbonate aqueous solution. The deposited solid was collected by filtration, washed with water and hexane and dried under reduced pressure, and 308 mg (100%) of the title compound was obtained as a white solid.
MS(FAB) m/z:312 $(M + H)^+$.

(202c) 6-[3-(2-methoxy-5-methyl-phenyl)-ureido]-3,4-dihydrido-1H-isoquinoline-2-carboxylic acid (2-chloro-6-methyl-phenyl)-amide

A solution of 1-(2-methoxy-5-methyl-phenyl)-3-(1,2,3,4-tetrahydro-isoquinolin-6-yl)-urea (70 mg) obtained in Example (202b) and 2-chloro-6-methylphenyl isocyanate (44 mg) in anhydrous tetrahydrofuran (5 mL) was stirred at room temperature for two hours. Methanol was added to the reaction mixture which was then concentrated. The residue was purified by column chromatography (dichloromethane/ethyl acetate 4:1 → 2:1) and 97 mg (91 %) of the title compound was obtained as a white solid.
$^1$H-NMR spectrum (400MHz,DMSO-d6):δ(ppm)=9.25(1H, s), 8.19(1H, s), 8.16(1H, s), 8.00(1H, d, J=1.9Hz), 7.39(1H, d, J=2.3Hz), 7.32(1H, dd, J=6.9 and 0.2Hz), 7.22-7.17(2H, m), 7.17(1H, dd, J=13.8 and 6.2Hz), 7.07(1H, d, J=8.2Hz), 6.90(1H, d, J=8.2Hz), 6.75(1H, dd, J=8.2 and 1.5Hz), 4.58(2H, s), 3.84(3H, s), 3.70(2H, t, J=5.9Hz), 2.84(2H, t, J=5.5Hz), 2.23(3H, s), 2.18(3H, s).
MS(FAB) m/z:479 $(M + H)^+$.
Melting point: 216-218˚C.

(Example 203) 6-[3-(2-methoxy-5-methyl-phenyl)-ureido]-3,4-dihydrido-1H-isoquinoline-2-carboxylic acid (2,6-dimethyl-phenyl)-amide (Compound No. 3-20)

90 mg (91 %) of the title compound was obtained from 1-(2-methoxy-5-methyl-phenyl)-3-(1,2,3,4-tetrahydro-isoquinolin-6-yl)-urea (68 mg) obtained in Example (202b) and 2,6-dimethylphenyl isocyanate (39 mg) as a white solid.
$^1$H-NMR spectrum (400MHz,DMSO-d6):δ(ppm)=9.25(1H, s), 8.16(1H, s), 8.00(1H, s), 7.93(1H, s), 7.39(1H, s), 7.21(1H, d, J=8.2Hz), 7.07(1H, d, J=9.4Hz), 7.05(3H, s), 6.90(1H, dd, J=8.2 and 1.5Hz), 6.75(1H, d, J=8.2Hz), 4.58(2H, s), 3.85(3H, s), 3.70(2H, t, J=5.4Hz), 2.84(2H, t, J=5.8Hz), 2.23(3H, s), 2.13(6H, s).
MS(FAB) m/z:459 $(M + H)^+$.
Melting point: 204-206˚C.

(Example 204) [4-(3-{4-[4-(2-chloro-6-methyl-phenylcarbamoyl)-piperazin-1-yl]-phenyl}-ureido)-benzyl]-phosphonic acid diethyl ester (Compound No. 1-491) (204a) 4-[(diethoxy-phosphoryl) methyl]-benzoic acid

A suspension of phosphonic acid triethyl ester (17.5 mL) of 4-bromomethyl benzoic acid (11.9 g) in acetonitrile (40 mL) was heated under reflux for two days. The reaction mixture was concentrated and the deposited solid was collected by filtration and washed with isopropyl ether and dried under reduced pressure, and 11.0 g (73%) of the title compound was obtained as a white solid.

(204b) 4-(4-{3-[4-(diethoxy-phosphorylmethyl)-phenyl]-ureido}-phenyl)-piperazine-1-carboxylic acid tert-butyl ester

1.65 g (80%) of the title compound was obtained from 4-(diethoxy-phosphoryl methyl)-benzoic acid (1.03 g) obtained in Example (204a) and N-tert-butyloxycarbonyl-N'-(4-aminophenyl)-piperazine (1.11 g) as a white solid in the same manner as in Example 42.
MS(FAB) m/z:547 $(M + H)^+$.

(204c) {4-[3-(4-piperazin-1-yl-phenyl)-ureido]-benzyl}-phosphonic acid diethyl ester

1.23 g (91%) of the title compound was obtained from 4-(4-{3-[4-(diethoxy-phosphorylmethyl)-phenyl]-ureido}-phenyl)-piperazine-1-carboxylic acid tert-butyl ester (1.65 g) obtained in Example (204b) in the same manner as in Example (47a).
MS(FAB) m/z:447 $(M + H)^+$.

(204d) [4-(3-{4-[4-(2-chloro-6-methyl-phenylcarbamoyl)-piperazin-1-yl]-phenyl}-ureido)-benzyl]-phosphonic acid diethyl ester

61 mg (62%) of the title compound was obtained as a white solid from {4-[3-(4-piperazin-1-yl-phenyl)-ureido]-benzyl}-phosphonic acid diethyl ester (78 mg) obtained in Example (204c) and 2-chloro-6-methylphenyl isocyanate (33 mg) in the same manner as in Example 49.
$^1$H-NMR spectrum (400MHz,DMSO-d6):δ(ppm)=8.54(1H, s), 8.43(1H, s), 8.24(1H, s), 7.38(2H, d, J=8.7Hz), 7.33(3H, d, J=8.2Hz), 7.20(1H, d, J=11.0Hz), 7.18(2H, d, J= 7.8Hz), 7.15(d, 1H, d,J=6.6Hz), 6.95(2H, d, J=8.2Hz), 3.95(2H, q, J=7.5Hz), 3.93(2H, q, J=7.5Hz), 3.61(4H, t, J=4.6Hz), 3.14(2H, d, J=21Hz), 3.08(4H, t, J=4.5Hz), 2.20(3H, s), 1.18(6H, t, J=7.0Hz).
MS(FAB) m/z:614 $(M + H)^+$.
Melting point: 218-220˚C.

(Example 205) 1-(4-benzoyl-3,4,5,6-tetrahydro-2H-[1,2']bipyridinyl-5'-yl)-3-(2-methoxy-5-methyl-phenyl)-urea (Compound No. 2-80)

(205a) (5'-nitro-3,4,5,6-tetrahydro-2H-[1,2']bipyridyl-4-yl)-phenyl-methanone

A suspension of 4-benzoyl piperidine hydrochloride (0.96 g), 2-chloro-5-nitropyridine (0.674 g) and potassium carbonate (1.18 g) in acetonitrile (20 mL) was heated under reflux for 7.5 hours. The reaction mixture was diluted with ethyl acetate and washed with water and saturated brine and dried over sodium sulfate and filtered and concentrated. The residue was vigorously stirred in isopropyl ether/ethyl acetate (5:1) and was collected by filtration and dried under reduced pressure, and 1.18 g (89%) of the title compound was obtained as a yellow solid.
MS(FAB) m/z:312 $(M + H)^+$.

(205b) (5'-amino-3,4,5,6-tetrahydro-2H-[1,2'] bipyridyl-4-yl)-phenyl-methanone

A suspension of (5'-nitro-3,4,5,6-tetrahydro-2H-[1,2']bipyridyl-4-yl)-phenyl-methanone (0.620 g) obtained in Example (205a) and 10% palladium-carbon catalyst (0.124 g) in anhydrous tetrahydrofuran (15 mL) and ethanol (15 mL) was stirred under a hydrogen atmosphere for three hours. The reaction mixture was filtered and concentrated. The residue was purified by column chromatography (dichloromethane/ethyl acetate 1:2). The obtained solid was vigorously

stirred in hexane/ethyl acetate (5: 1) and was collected by filtration and dried under reduced pressure, and 424 mg (75%) of the title compound was obtained as a pale pink solid.
MS(FAB) m/z:282 $(M + H)^+$.

(205c) 1-(4-benzoyl-3,4,5,6-tetrahydro-2H-[1,2']bipyridinyl-5'-yl)-3-(2-methoxy-5-methyl-phenyl)-urea

A solution of (5'-amino-3,4,5,6-tetrahydro-2H-[1,2']bipyridyl-4-yl)-phenyl-methanone (403 mg) obtained in Example (205b) and 2-methoxy-5-methylphenyl isocyanate (0.25 mL) in anhydrous tetrahydrofuran (10 mL) was stirred at room temperature for four hours. Methanol was added to the reaction mixture which was then concentrated. The residue was vigorously stirred in dichloromethane/ethyl acetate (5:1), collected by filtration and dried under reduced pressure, and 537 mg (84%) of the title compound was obtained as a pale pink solid.
$^1$H-NMR spectrum (400MHz,DMSO-d6):δ(ppm)=9.05(1H, s), 8.13(1H, d, J=2.8Hz), 8.11(1H, s), 8.03(2H, d, J=7.8Hz), 7.97(1H, s), 7.72(1H, dd, J=9.4 and 2.8Hz), 7.67(1H, t, J=7.8Hz), 7.56(2H, t, J=7.8Hz), 6.89(1H, d, J=8.2Hz), 6.85(1H, d, J=9.4Hz), 6.73(1H, d, J=8.2Hz), 4.25(2H, d, J=12.2Hz), 3.84(3H, s), 3.78-3.65(1H, m), 2.97(2H, dd, J=12.2 and 11.8Hz), 2.22(3H, s), 1.84(2H, d, J=12.5Hz), 1.59(2H, dd, J=11.8 and 11.2Hz).
MS(ES) m/z:445 $(M + H)^+$.
Melting point: 193-194˚C.

(Example 206) 4-[4-(3-hexyl-ureido)-phenyl]-piperazine-1-carboxylic acid (2-chloro-6-methyl-phenyl)-amide (Compound No. 1-495)

(206a) 4-[4-(3-hexyl-ureido)-phenyl]-piperazine-1-carboxylic acid tert-butyl ester

3.91 g (97%) of the title compound was obtained as a light brown powder from N-tert-butyloxycarbonyl-N'-(4-aminophenyl)-piperazine (2.77 g) and n-hexyl isocyanate (1.75 mL) in the same manner as in Example 1.
$^1$H NMR(400MHz,DMSO-d6):δ(ppm)=8.09(1H, s), 7.21(2H, d, J=9.0Hz), 6.82(2H, d, J=9.0Hz), 5.95(1H, brs), 3.43(4H, t, J=4.7Hz), 3.04(2H, q, J=11.4Hz), 2.96(4H, t, J=6.0Hz), 1.41(9H, s), 1.41-1.38(2H, m), 1.29-1.25(6H, m), 0.87(3H, t, J=6.8Hz).

(206b) 1-hexyl-3-(4-piperazin-1-yl-phenyl)-urea

2.94 g (100%) of the title compound was obtained from 4-[4-(3-hexyl-ureido)-phenyl]-piperazine-1-carboxylic acid tert-butyl ester (3.91 g) obtained in Example (206a) as a white powder in the same manner as in Example (47a).
MS(FAB) m/z:305 $(M + H)^+$.

(206c) 4-[4-(3-hexyl-ureido)-phenyl]-piperazine-1-carboxylic acid (2-chloro-6-methyl-phenyl)-amide

75 mg (85%) of the title compound was obtained as an ochre powder from 1-hexyl-3-(4-piperazin-1-yl-phenyl)-urea (69 mg) obtained in Example (206b) and 2-chloro-6-methylphenyl isocyanate (0.034 mL) in the same manner as in Example 49. $^1$H-NMR spectrum (400MHz,DMSO-d6):δ(ppm)=8.19(1H, s), 8.10(1H, s), 7.30(1H, d, J=8.2Hz), 7.23 (2H, d, J=9.0Hz), 7.18(1H, dd, J=9.8 and 7.4Hz), 7.15(2H, dd, J=16.1 and 8.2Hz), 6.86(2H, d, J=9.0Hz), 3.58(4H, t, J=4.4Hz), 3.03(6H, t, J=5.3Hz), 2.19(3H, s), 1.40(2H, brs), 1.27(6H, brs), 0.87(3H, t, J=6.8Hz).
MS(ES) m/z:472 $(M)^+$.
Melting point: 206-210˚C.

(Example 207) 4-[4-(3-cyclohexyl-ureido)-phenyl]-piperazine-1-carboxylic acid (2-chloro-6-methyl-phenyl)-amide (Compound No. 1-511)

(207a) 4-[4-(3-cyclohexyl)-phenyl]-piperazine-1-carboxylic acid tert-butyl ester

3.94 g (98%) of the title compound was obtained as an ochre powder from N-tert-butyloxycarbonyl-N'-(4-aminophenyl)-piperazine (2.77 g) and cyclohexylisocyanate (1.53 mL) in the same manner as in Example 1.
$^1$H NMR(400MHz,DMSO-d6):δ(ppm)=8.04(1H, s), 7.23(2H, d, J=8.2Hz), 6.85(2H, d, J=8.2Hz), 5.92(1H, d, J=7.5Hz), 3.44-3.44(5H, m), 2.96(4H, t, J=4.7Hz), 1.81-1.78(2H, m), 1.67-1.64(2H, m), 1.56-1.51(2H, m), 1.41(9H, s), 1.31-1.25 (2H, m), 1.21-1.09(2H, m).

(207b) 1-cyclohexyl-3-(4-piperazin-1-yl-phenyl)-urea

2.96 g (100%) of the title compound was obtained as a white powder from 4-[4-(3-hexyl-ureido)-phenyl]-piperazine-1-carboxylic acid tert-butyl ester (3.94 g) obtained in Example (207a) in the same manner as in Example (47a).
MS(FAB) m/z:303 $(M + H)^+$.

(207c) 4-[4-(3-cyclohexyl-ureido)-phenyl]-piperazine-1-carboxylic acid (2-chloro-6-methyl-phenyl)-amide

843 mg (90%) of the title compound was obtained as a white powder from 1-cyclohexyl-3-(4-piperazin-1-yl-phenyl)-urea (605 mg) obtained in Example (207b) and 2-chloro-6-methylphenyl isocyanate (0.33 mL) in the same manner as in Example 49.
$^1$H-NMR spectrum (400MHz,DMSO-d6):δ(ppm)=8.22(1H, s), 8.04(1H, s), 7.33(1H, d, J=7.8Hz), 7.24(2H, d, J=8.6Hz), 7.22(1H, d, J=10.2Hz), 7.17(1H, dd, J=16.8 and 9.4Hz), 6.89(2H, d, J=8.6Hz), 5.92(1H, d, J=7.4Hz), 3.60(4H, t, J=4.3Hz), 3.43(1H, brs), 3.04(4H, t, J=4.3Hz), 2.20(3H, s), 1.79(2H, d, J=9.0Hz), 1.65(2H, d, J=12.9Hz), 1.54(2H, d, J=9.8Hz), 1.29(2H, t, J=11.7Hz), 1.16(2H, t, J=12.3Hz).
MS(ES) m/z:470 $(M)^+$.
Melting point: >260 ˚C (dec).

(Example 208) 4-{4-[3-(2-methoxy-5-methyl-phenyl)-ureido]-phenyl}-piperazine-1-carboxylic acid [4-(2-hydroxy-ethoxy)-2-methyl-phenyl]-amide (Compound No. 1-692)

(208a) 2-(3-methyl-4-nitro-phenoxy)-ethanol (Cotts, Ian G.C. et al, J. Chem. Soc. Perkin Trans. 1, 1985, 1829-1836)
Potassium carbonate (3.32 g) and 2-bromoethanol (1.6 mL) were added to a solution of 4-nitro-meta-cresol (3.06 g) in dimethylacetamide (40 mL) at room temperature. The reaction mixture was heated at 80˚C for 24 hours and diluted with ethyl acetate and washed with water and brine and dried over sodium sulfate and concentrated. The residue was purified by column chromatography (dichloromethane:ethyl acetate 10:1 → 5:1) and 1.81 g (46%) of the title compound was obtained as a pale yellow solid.
$^1$H NMR(400MHz,$CDCl_3$):δ(ppm)=8.07(1H, d, J=9.4Hz), 6.83(1H, d, J=2.8Hz), 6.80(1H, s), 4.52(1H, s), 4.15(2H, dd, J=4.5 and 4.5Hz), 4.00(2H, dd, J=10.3 and 4.9Hz), 2.63(3H, s).

(208b) tert-butyl-dimethyl-[2-(3-methyl-4-nitro-phenoxy)-ethoxy]-silane

2-(3-methyl-4-nitro-phenoxy)-ethanol (1.81 g) obtained in Example (208a) was dissolved in dimethylformamide (30 mL). Imidazole (1.25 g) and tert-butyldimethylsilyl chloride (1.66 g) were added to this solution at room temperature. The reaction mixture was stirred for 45 minutes and diluted with ethyl acetate and washed with water and saturated brine and dried over sodium sulfate and concentrated. The residue was dried under reduced pressure and 2.85 g (quantitative yield) of the title compound was obtained as a pale yellow solid.
$^1$H NMR(400MHz,$CDCl_3$):δ(ppm)=8.09(1H, d, J=8.6Hz), 6.83(1H, d, J=2.3Hz), 6.81(1H, s), 4.12(2H, t, J=4.7Hz), 3.99 (2H, t, J=4.6Hz), 2.64(3H, s), 0.91(9H, s), 0.10(6H, s).

(208c) 4-[2-(tert-butyl-dimethyl-silanyloxy)-ethoxy]-2-methyl-phenylamine

A suspension of tert-butyl-dimethyl-[2-(3-methyl-4-nitro-phenoxy)-ethoxy]-silane (2.85 g) obtained in Example (208b) and 10% palladium-carbon catalyst (0.29 g) in anhydrous tetrahydrofuran (30 mL) was stirred at room temperature under a hydrogen atmosphere for 27 hours. The reaction mixture was filtered and concentrated. The residue was purified by column chromatography (dichloromethane:ethyl acetate 1:0 → 5:1) and 2.57 g (quantitative yield) of the title compound was obtained as a purple oil.
$^1$H NMR(400MHz,$CDCl_3$):δ(ppm)=6.66(1H, d, J=2.3Hz), 6.61(1H, d, J=2.7Hz), 6.60(1H, s), 3.96-3.90(4H, m), 3.34(2H, brs), 2.15(3H, s), 0.91(9H, s), 0.10(6H, s). (208d) 4-{4-[3-(2-methoxy-5-methyl-phenyl)-ureido]-phenyl}-piperazine-1-carboxylic acid [4-(2-hydroxy-ethoxy)-2-methyl-phenyl]-amide
1.32 g (80%) of silyl ether was obtained as a white powder from 1-(2-methoxy-5-methyl-phenyl)-3-(4-piperazin-1-yl-phenyl)-urea (865 mg) obtained in Example (47a) and 4-[2-(tert-butyl-dimethyl-silanyloxy)-ethoxy]-2-methylphenylamine (715 mg) obtained in Example (208c) in the same manner as in Example 170. Tetrabutylammonium fluoride (1M tetrahydrofuran solution, 4.1 mL) was added to a solution of this silyl ether in anhydrous tetrahydrofuran (30 mL) at room temperature. After 4.5 hours, the reaction mixture was concentrated and stirred with water and methanol (5:1). The deposited solid was collected by filtration, washed with water and methanol and dried under reduced pressure, and 1.09 g (quantitative yield) of the title compound was obtained as a white solid.

$^1$H NMR(400MHz,DMSO-d6):δ(ppm)=9.05(1H, s), 8.03(1H, s), 8.01(1H, s), 7.96(1H, d, J=2.0Hz), 7.31(2H, d, J=9.0Hz), 7.02(1H, d, J=8.6Hz), 6.92(2H, d, J=9.0Hz), 6.86(1H, d, J=8.2Hz), 6.76(1H, d, J=2.7Hz), 6.70(1H, dd, J=7.8 and 2.0Hz), 6.68(1H, dd, J=8.4 and 2.6Hz), 4.83(1H, t, J=5.5Hz), 3.93(2H, dd, J=4.9 and 4.9Hz), 3.82(3H, s), 3.69(2H, dd, J=10.1 and 5.0Hz), 3.56(4H, t, J=4.9Hz), 3.06(4H, t, J=4.9Hz), 2.22(3H, s), 2.13(3H, s).
MS(ES$^+$) m/z:534 (M + H)$^+$.
Melting point: 174-176˚C.

(Example 209) 4-{4-[3-(2-methoxy-5-methyl-phenyl)-ureido]-phenyl}-piperazine-1-carboxylic acid [4-(2-hydroxy-ethoxy)-2-trifluoromethyl-phenyl]-amide (Compound No. 1-603)

(209a) 2-(4-nitro-3-trifluoromethyl-phenoxy)-ethanol

1.32 g (26%) of the title compound was obtained as a yellow oil from 4-nitro-3-(trifluoromethyl) phenol (4.14 g) and 2-bromoethanol (1.6 mL) in the same manner as in Example (208a).
$^1$H NMR(400MHz,$CDCl_3$):δ(ppm)=8.01(1H, d, J=9.0Hz), 7.33(1H, d, J=2.8Hz), 7.14(1H, dd, J=9.0 and 2.7Hz), 4.52(1H, s), 4.21(2H, t, J=4.5Hz), 4.04(2H, t, J=4.5Hz).

(209b) tert-butyl-dimethyl-[2-(4-nitro-3-trifluoromethyl-phenoxy)-ethoxy]-silane

The title compound (2.02 g) was obtained as a yellow oil from 2-(4-nitro-3-trifluoromethyl-phenoxy)-ethanol (1.32 g) obtained in Example (209a) and tert-butyldimethylsilyl chloride (0.951 g) in the same manner as in Example (208b).
$^1$H NMR(400MHz,$CDCl_3$):δ(ppm)=7.94(1H, s), 7.24(1H, d, J=2.4Hz), 7.06(1H, dd, J=9.0 and 2.7Hz), 4.09(2H, t, J=4.9Hz), 3.92(2H, t, J=4.9Hz), 0.81(9H, s), 0.01(6H, s).

(209c) 4-[2-(tert-butyl-dimethyl-silanyloxy)-ethoxy]-2-trifluoromethyl-phenylamine

1.56 g (88%) of the title compound was obtained as pale yellow oil from tert-butyl-dimethyl-[2-(4-nitro-3-trifluoromethyl-phenoxy)-ethoxy]-silane (1.92 g) obtained in Example (209b) in the same manner as in Example (208c).
$^1$H NMR(400MHz,$CDCl_3$):δ(ppm)=6.98(1H, d, J=2.7Hz), 6.91(1H, dd, J=8.8 and 2.9Hz), 6.68(1H, d, J=8.6Hz), 3.99-3.91 (4H, m), 3.86(2H, brs), 0.90(9H, s), 0.09(6H, s).

(209d) 4-{4-[3-(2-methoxy-5-methyl-phenyl)-ureido]-phenyl}-piperazine-1-carboxylic acid [4-(2-hydroxy-ethoxy)-2-trifluoromethyl-phenyl]-amide

662 mg (79%) of silyl ether was obtained as a beige powder from 1-(2-methoxy-5-methyl-phenyl)-3-(4-piperazin-1-yl-phenyl)-urea (407 mg) obtained in Example (47a) and 4-[2-(tert-butyl-dimethyl-silanyloxy)-ethoxy]-2-trifluoromethyl-phenylamine (401 mg) obtained in Example (209c) in the same manner as in Example 170. Tetrabutylammonium fluoride (1M tetrahydrofuran solution, 1.9 mL) was added to a solution of this silyl ether in anhydrous tetrahydrofuran (20 mL) at room temperature. After 4.5 hours, the reaction mixture was concentrated and stirred with water and methanol (5:1). The deposited solid was collected by filtration, washed with water and methanol and dried under reduced pressure, and 424 mg (77%) of the title compound was obtained as a white solid.
$^1$H NMR(400MHz,DMSO-d6):δ(ppm)=9.09(1H, s), 8.23(1H, s), 8.07(1H, s), 7.99(1H, s), 7.33(2H, d, J=7.1Hz), 7.32(1H, d, J=7.8Hz), 7.22(1H, d, J=9.0Hz), 7.18(1H, s), 6.94(2H, d, J=7.9Hz), 6.89(1H, d, J=8.2Hz), 6.73(1H, d, J=8.2Hz), 4.92 (1H, t, J=4.9Hz), 4.07(2H, dd, J=4.2 and 4.2Hz), 3.84(3H, s), 3.73(2H, d, J=3.9Hz), 3.56(4H, brs), 3.06(4H, brs), 2.23 (3H, s).
MS(ES$^+$) m/z:588 (M + H)$^+$.
Melting point: 175-177˚C.

(Example 210) 4-{4-[3-(2-methoxy-5-methyl-phenyl)-ureido]-phenyl}-piperazine-1-carboxylic acid [2-fluoro-4-(2-hydroxy-ethoxy)-phenyl]-amide (Compound No. 1-761)

(210a) 2-[2-(3-fluoro-4-nitro-phenoxy)-ethoxy]-tetrahydro-pyran

Potassium carbonate (1.66 g) and 3-fluoro-4-nitrophenol (1.57 g) were added to a solution of 2-(2-bromoethoxy) tetrahydro-2H-pyran (2.09 g) in acetonitrile (20 mL) at room temperature. The reaction mixture was heated under reflux for 22 hours and diluted with ethyl acetate and washed with water and saturated brine and dried over sodium sulfate and concentrated. The residue was purified by column chromatography (dichloromethane: ethyl acetate 1:0 → 10:1)

and 973 mg (36%) of the title compound was obtained as a yellow oil.
$^1$H NMR(400MHz,$CDCl_3$):δ(ppm)=8.06(1H, d, J=9.0Hz), 6.80(1H, s), 6.77(1H, d, J=2.7Hz), 4.68 (1H, dd, J=4.2 and 3.0Hz), 4.24(1H, dt, J=5.1 and 3.1Hz), 4.10(1H, ddd, J=11.6, 4.9 and 4.0Hz), 3.87-3.80(1H, m), 3.56-3.50(1H, m), 1.83-1.71(2H, m), 1.63-1.51(5H, m).

(210b) 2-fluoro-4-[2-(tetrahydro-pyran-2-yloxy)-ethoxy]-phenylamine

A suspension of 2-[2-(3-fluoro-4-nitro-phenoxy)-ethoxy]-tetrahydro-pyran (972 mg) obtained in Example (210a) and 10% palladium-carbon catalyst (0.1 g) in anhydrous tetrahydrofuran (20 mL) was stirred at room temperature under a hydrogen atmosphere for 9 hours. The reaction mixture was filtered and concentrated. The residue was purified by column chromatography (dichloromethane:ethyl acetate 5:1 → 3:1) and 571 mg (62%) of the title compound was obtained as a yellow oil.
$^1$H NMR(400MHz,$CDCl_3$):δ(ppm)=6.74-6.66(2H, m), 6.58(1H, dd, J=8.6 and 2.7Hz), 4.70(1H, t, J=3.5Hz), 4.09-3.99 (3H, m), 3.92-3.87(1H, m), 3.81-3.76(1H, m), 3.56-3.51(1H, m), 1.89-1.81(1H, m), 1.78-1.71(1H, m), 1.67-1.52(5H, m).

(210c) 4- {4-[3-(2-methoxy-5-methyl-phenyl)-ureido]-phenyl}-piperazine-1-carboxylic acid [2-fluoro-4-(2-hydroxy-ethoxy)-phenyl]-amide

THP (= tetrahydropyranyl) ether (876 mg, 67%) was obtained as a white powder from 1-(2-methoxy-5-methyl-phenyl)-3-(4-piperazin-1-yl-phenyl)-urea (722 mg) obtained in Example (47a) and 2-fluoro-4-[2-(tetrahydro-pyran-2-yloxy)-ethoxy]-phenylamine (571 mg) obtained in Example (210b) in the same manner as in Example 170. This THP ether was deprotected with p-toluenesulfonic acid in a methanol solution and 652 mg (86%) of the title compound was obtained as a white solid.
$^1$H NMR(400MHz,DMSO-d6):δ(ppm)=9.08(1H, s), 8.22(1H, s), 8.06(1H, s), 7.99(1H, s), 7.33(2H, d, J=8.2Hz), 7.24(1H, dd, J=8.8 and 8.8Hz), 6.94(2H, d, J=8.6Hz), 6.89(1H, d, J=8.6Hz), 6.84(1H, dd, J=12.3 and 2.1Hz), 6.73(2H, d, J=8.2Hz), 4.88(1H, dd, J=5.3 and 5.3Hz), 3.98(2H, dd, J=4.7 and 4.7Hz), 3.84(3H, s), 3.70(2H, dd, J=4.6 and 10.2Hz), 3.57(4H, t, J=4.1Hz), 3.07(4H, t, J=4.1Hz), 2.23(3H, s)
MS($ES^+$) m/z:538 $(M + H)^+$.
Melting point: 214-215˚C.

(Example 211) 4-{4-[3-(2-fluoro-phenyl)-ureido]-phenyl}-piperazine-1-carboxylic acid [4-(2-hydroxy-ethoxy)-2-trifluoromethyl-phenyl]-amide (Compound No. 1-596)

375 mg (56%) of silyl ether was obtained as a white solid from 1-(2-fluorophenyl)-3-(4-piperazin-1-yl-phenyl)-urea (314 mg) obtained in Example (125a) and 4-[2-(tert-butyl-dimethyl-silanyloxy)-ethoxy]-2-trifluoromethyl-phenylamine (335 mg) obtained in Example (209c) in the same manner as in Example 170. This silyl ether was deprotected with tetrabutylammonium fluoride in the same manner as in Example (209d) and 260 mg (83%) of the title compound was obtained as a white solid.
$^1$H NMR(400MHz,DMSO-d6):δ(ppm)=8.83(1H, s), 8.42(1H, d, J=2.7Hz), 8.20(1H, s), 8.14(1H, ddd, J=8.4 ,8.4 and 1.7Hz), 7.31(2H, d, J=8.6Hz), 7.29(1H, d, J=7.8Hz), 7.23-7.15(2H, m), 7.11(1H, dd, J=8.0 and 8.0Hz), 6.99-6.95(1H, m), 6.93(2H, d, J=9.0Hz), 4.90(2H, dd, J=5.5 and 5.5Hz), 4.05(2H, dd, J=4.9 and 4.9Hz), 3.72(2H, dd, J=10.0 and 5.3Hz), 3.55(4H, t, J=4.7Hz), 3.05(4H, t, J=4.9Hz).
MS($ES^+$) m/z:562 $(M + H)^+$.
Melting point: 219-220˚C.

(Example 212) 4- {4-[3-(5-fluoro-2-methoxy-phenyl)-ureido]-phenyl}-piperazine-1-carboxylic acid [4-(2-hydroxy-ethoxy)-2-trifluoromethyl-phenyl]-amide (Compound No. 1-599)

1.02 g (72%) of silyl ether was obtained as a white solid from 1-(5-fluoro-2-methoxy-phenyl)-3-(4-piperazin-1-yl-phenyl)-urea (689 mg) obtained in Example (96a) and 4-[2-(tert-butyl-dimethyl-silanyloxy)-ethoxy]-2-trifluoromethyl-phenylamine (671 mg) obtained in Example (209c) in the same manner as in Example 170. This silyl ether was deprotected with tetrabutylammonium fluoride in the same manner as in Example (209d) and 704 mg (83%) of the title compound was obtained as a grey solid.
$^1$H NMR(400MHz,DMSO-d6):δ(ppm)=9.17(1H, s), 8.30(1H, s), 8.19(1H, s), 8.00(1H, dd, J=11.7 and 3.1Hz), 7.31(2H, d, J=9.0Hz), 7.29(1H, d, J=7.0Hz), 7.20(1H, dd, J=8.6 and 2.8Hz), 7.15(1H, d, J=2.7Hz), 6.98(1H, dd, J=8.8 and 5.3Hz), 6.93(2H, d, J=9.0Hz), 6.70(1H, ddd, J=8.6, 8.6 and 3.3Hz), 4.89(1H, dd, J=5.5 and 5.5Hz), 4.05(2H, t, J=4.9 and 4.9Hz), 3.86(3H, s), 3.72(2H, dd, J=9.8 and 5.1Hz), 3.55(4H, t, J=4.9Hz), 3.05 (4H, t, J=4.9Hz).
MS($ES^+$) m/z:592 $(M + H)^+$.

Melting point: 140-141˚C.

(Example 213) 4-{4-[3-(5-methyl-thiazol-2-yl)-ureido]-phenyl}-piperazine-1-carboxylic acid [4-(2-hydroxy-ethoxy)-2-trifluoromethyl-phenyl]-amide (Compound No. 1-606)

(213a) 4-{4-[3-(5-methyl-thiazol-2-yl)-ureido]-phenyl}-piperazine-1-carboxylic acid tert-butyl ester

691 mg (83%) of the title compound was obtained as a pale purple solid from N-tert-butyloxycarbonyl-N'-(4-aminophenyl)-piperazine (555 mg) and 2-amino-5-methylthiazol (228 mg) in the same manner as in Example (41c).
$^1$H NMR(400MHz,DMSO-d6):δ(ppm)=10.2(1H, s), 8.68(1H, s), 7.30(2H, d, J=9.0Hz), 6.99(1H, s), 6.91(2H, d, J=9.0Hz), 3.43(4H, t, J=5.1Hz), 3.00(4H, t, J=5.0Hz), 2.29(3H, s), 1.40(9H, s).

(213b) 1-(5-methyl-thiazol-2-yl)-3-(4-piperazin-1-yl-phenyl)-urea

317 mg (quantitative yield) of the title compound was obtained as a white solid from 4-{4-[3-(5-methyl-thiazol-2-yl)-ureido]-phenyl}-piperazine-1-carboxylic acid tert-butyl ester (417 mg) obtained in Example (213a) in the same manner as in Example (47a).
$^1$H NMR(400MHz,DMSO-d6):δ(ppm)=8.79(1H, s), 8.31(1H, brs), 7.33(2H, d, J=8.2Hz), 6.99(1H, s), 6.93(2H, d, J=8.2Hz), 3.20(4H, brs), 3.18(4H, brs), 2.28(3H, s).

(213c) 4-{4-[3-(5-methyl-thiazol-2-yl)-ureido]-phenyl} -piperazine-1 -carboxylic acid [4-(2-hydroxy-ethoxy)-2-trifluoromethyl-phenyl]-amide

314 mg (77%) of a silyl ether was obtained as a white solid from 1-(5-methyl-thiazol-2-yl)-3-(4-piperazin-1-yl-phenyl)-urea (190 mg) obtained in Example (213b) and 4-[2-(tert-butyl-dimethyl-silanyloxy)-ethoxy]-2-trifluoromethyl-phenylamine (202 mg) obtained in Example (209c) in the same manner as in Example 170. This silyl ether was deprotected with tetrabutylammonium fluoride in the same manner as in Example (209d) and 178 mg (68%) of the title compound was obtained as a white solid.
$^1$H NMR(400MHz,DMSO-d6):δ(ppm)=10.2(1H, brs), 8.70(1H, s), 8.20(1H, s), 7.31(2H, d, J=8.6Hz), 7.29(1H, d, J=8.6Hz), 7.20(1H, dd, J=8.8 and 3.0Hz), 7.15(1H, d, J=2.7Hz), 7.00(1H, s), 6.94(2H, d, J=9.0Hz), 4.89(1H, dd, J=5.6 and 5.6Hz), 4.05(2H, dd, J=4.9 and Hz), 3.72(2H, dd, J=9.8 and 5.1Hz), 3.55(4H, t, J=4.7Hz), 3.06(4H, t, J=4.7Hz), 2.30(3H, s).
MS(ES$^+$) m/z:565 (M + H)$^+$.
Melting point: 118-120˚C.

(Example 214) 4-{4-[3-(3-methoxy-phenyl)-ureido]-phenyl}-piperazine-1-carboxylic acid [4-(2-hydroxy-ethoxy)-2-trifluoromethyl-phenyl]-amide (Compound No. 1-844) (214a) 4- {4-[3-(3-methoxy-phenyl)-ureido]-phenyl}-piperazine-1-carboxylic acid tert-butyl ester

4.27 g (quantitative yield) of the title compound was obtained as a white solid from N-tert-butyloxycarbonyl-N'-(4-aminophenyl)-piperazine (2.77 g) and 3-methoxyphenyl isocyanate (1.7 mL) in the same manner as in Example 1.
$^1$H NMR(400MHz,$CDCl_3$):δ(ppm)=7.20(2H, d, J=7.9Hz), 7.16(1H, dd, J=8.1 and 8.1Hz), 7.06(1H, dd, J=2.3 and 2.4Hz), 6.87(2H, brs), 6.79(1H, dd, J=8.0 and 1.4Hz), 6.74(1H, brs), 6.64(1H, brs), 6.60(1H, dd, J=8.0 and 2.2Hz), 3.77(3H, s), 3.57(4H, t, J=5.1Hz), 3.08(4H, brs), 1.49(9H, s).

(214b) 1-(3-methoxy-phenyl)-3-(4-piperazin-1-yl-phenyl)-urea

3.26 g (quantitative yield) of the title compound was obtained as a white solid from 4-{4-[3-(3-methoxy-phenyl)-ureido]-phenyl}-piperazine-1-carboxylic acid tert-butyl ester (4.27 g) obtained in Example (214a) in the same manner as in Example (47a).
$^1$H NMR(400MHz, DMSO-d6):δ(ppm)=8.78(1H, s), 8.59(1H, brs), 7.30(2H, d, J=9.0Hz), 7.19(1H, s), 7.16(1H, dd, J=15.0 and 7.2Hz), 6.92(1H, d, J=9.0Hz), 6.86(2H, d, J=9.0Hz), 6.52(1H, dd, J=8.2 and 2.8Hz), 3.72(3H, s), 3.47(1H, brs), 2.96 (4H, brs), 2.83(4H, brs).

(214c) 4-{4-[3-(3-methoxy-phenyl)-ureido]-phenyl}-piperazine-1-carboxylic acid [4-(2-hydroxy-ethoxy)-2-trifluoromethyl-phenyl]-amide

1.47 g (quantitative yield) of silyl ether was obtained as a white solid from 1-(3-methoxy-phenyl)-3-(4-piperazin-1-yl-phenyl)-urea (326 mg) obtained in Example (214b) and 4-[2-(tert-butyl-dimethyl-silanyloxy)-ethoxy]-2-trifluoromethyl-phenylamine (671 mg) obtained in Example (209c) in the same manner as in Example 170. This silyl ether was deprotected with tetrabutylammonium fluoride in the same manner as in Example (209d) and 545 mg (48%) of the title compound was obtained as a white solid.
$^1H$ NMR(400MHz,DMSO-d6):δ(ppm)=8.62(1H, s), 8.45(1H, s), 8.22(1H, s), 7.33(2H, d, J=8.2Hz), 7.31(1H, d, J=8.2Hz), 7.24-7.15(2H, m), 7.19(2H, s), 6.94(2H, d, J=8.6Hz), 6.92(1H, d, J=7.1Hz), 6.54(1H, d, J=9.4Hz), 4.91(1H, dd, J=5.5 and 5.5Hz), 4.07(2H, dd, J=4.5 and 4.5Hz), 3.75-3.71(2H, m), 3.73(3H, s), 3.56(4H, t, J=3.9Hz), 3.06(4H, t, J=3.9Hz).
MS($ES^+$) m/z:575 $(M + H)^+$.
Melting point: 214-215˚C.

(Example 215) 4-{4-[3-(3-ethoxy-phenyl)-ureido]-phenyl}-piperazine-1-carboxylic acid [4-(2-hydroxy-ethoxy)-2-trifluoromethyl-phenyl]-amide (Compound No. 1-846)

(215a) 4-{4-[3-(3-ethoxy-phenyl)-ureido]-phenyl}-piperazine-1-carboxylic acid tert-butyl ester

2.18 g (99%) of the title compound was obtained as a white solid from N-tert-butyloxycarbonyl-N'-(4-aminophenyl)-piperazine (1.39 g) and 3-ethoxyphenyl isocyanate (0.88 mL) in the same manner as in Example 1. $^1H$ NMR(400MHz, $CDCl_3$):δ(ppm)=7.24(2H, d, J=7.8Hz), 7.19(1H, dd, J=8.4 and 8.4Hz), 7.05(1H, s), 6.92(2H, brs), 6.82(1H, d, J=7.5Hz), 6.64(1H, d, J=6.3Hz), 6.48(1H, brs), 6.39(1H, brs), 4.02(2H, q, J=6.8Hz), 3.59(4H, t, J=4.9Hz), 3.11(4H, brs), 1.57(9H, s), 1.39(3H, t, J=6.8Hz).

(215b) 1-(3-ethoxy-phenyl)-3-(4-piperazin-1-yl-phenyl)-urea

1.68 g (quantitative yield) of the title compound was obtained as a white solid from 4- {4-[3-(3-ethoxy-phenyl)-ureido]-phenyl}-piperazine-1-carboxylic acid tert-butyl ester (2.17 g) obtained in Example (215a) in the same manner as in Example (47a).
$^1H$ NMR(400MHz,DMSO-d6):δ(ppm)=8.59(1H, s), 8.46(1H, s), 7.34(2H, d, J=7.4Hz), 7.17(1H, s), 7.14(1H, d, J=7.8Hz), 6.94(2H, d, J=8.6Hz), 6.89(1H, d, J=7.OHz), 6.52(lH, d, J=8.2Hz), 3.99(2H, q, J=6.5Hz), 3.32(1H, brs), 3.21(8H, brs), 1.32(3H, t, J=6.2Hz).

(215c) 4-{4-[3-(3-ethoxy-phenyl)-ureido]-phenyl}-piperazine-1-carboxylic acid [4-(2-hydroxy-ethoxy)-2-trifluoromethyl-phenyl]-amide

470 mg (67%) of silyl ether was obtained as a white solid from 1-(3-ethoxyphenyl)-3-(4-piperazin-l-yl-phenyl)-urea (335 mg) obtained in Example (215b) and 4-[2-(tert-butyl-dimethyl-silanyloxy)-ethoxy]-2-trifluoromethyl-phenylamine (340 mg) obtained in Example (209c) in the same manner as in Example 170. This silyl ether was deprotected with tetrabutylammonium fluoride in the same manner as in Example (209d) and 321 mg (82%) of the title compound was obtained as a white solid.
$^1H$ NMR(400MHz,DMSO-d6):δ(ppm)=8.60(1H, s), 8.46(1H, s), 8.24(1H, s), 7.34(2H, d, J=9.0Hz), 7.33(1H, d, J=8.6Hz), 7.24(1H, dd, J=8.8 and 2.9Hz), 7.20(2H, d, J=2.0Hz), 7.16(1H, dd, J=8.2 and 8.2Hz), 6.96(2H, d, J=9.0Hz), 6.92(1H, d, J=7.8Hz), 6.53(1H, dd, J=8.0 and 2.2Hz), 4.94(1H, dd, J=5.5 and 5.5Hz), 4.09(2H, dd, J=4.9 and 4.9Hz), 4.02(2H, q, J=6.8Hz), 3.76(2H, dd, J=10.0 and 5.2Hz), 3.59(4H, t, J=4.3Hz), 3.09(4H, t, J=4.5Hz), 1.36(3H, t, J=7.0Hz).
MS($ES^+$) m/z:589 $(M + H)^+$.
Melting point: 108-110˚C.

(Example 216) 4-{4-[3-(3,5-dimethoxy-phenyl)-ureido]-phenyl}-piperazine-1-carboxylic acid [4-(2-hydroxy-ethoxy)-2-trifluoromethyl-phenyl]-amide (Compound No. 1-605)

(216a) 4-{4-[3-(3,5-dimethoxy-phenyl)-ureido]-phenyl}-piperazine-1-carboxylic acid tert-butyl ester

4.52 g (99%) of the title compound was obtained as a white solid from N-tert-butyloxycarbonyl-N'-(4-aminophenyl)-piperazine (2.77 g) and 3,5-dimethoxyphenyl isocyanate (1.92 g) in the same manner as in Example 1.
$^1H$ NMR(400MHz,$CDCl_3$):δ(ppm)=7.23(2H, d, J=8.6Hz), 7.03(1H, s), 6.95(1H, s), 6.87(2H, d, J=8.6Hz), 6.59(2H, d, J=2.0Hz), 6.18(1H, dd, J=1.8 and 1.8Hz), 3.74(6H, s), 3.58(4H, t, J=4.9Hz), 3.06(4H, t, J=4.5Hz), 1.49(9H, s).

(216b) 1-(3,5-dimethoxy-phenyl)-3-(4-piperazin-1-yl-phenyl)-urea

2.88 g (82%) of the title compound was obtained as a white solid from 4-{4-[3-(3,5-dimethoxy-phenyl)-ureido]-phenyl}-piperazine-1-carboxylic acid tert-butyl ester (4.52 g) obtained in Example (216a) in the same manner as in

Example (47a).
$^1$H NMR(400MHz,DMSO-d6):δ(ppm)=8.84(1H, brs), 8.63(1H, brs), 7.30(2H, d, J=8.2Hz), 6.86(2H, d, J=8.6Hz), 6.67 (2H, d, J=13.3Hz), 6.11(1H, s), 3.71(6H, s), 3.48(1H, brs), 2.96(4H, brs), 2.83(4H, brs).

(216c) 4-{4-[3-(3,5-dimethoxy-phenyl)-ureido]-phenyl}-piperazine-1-carboxylic acid [4-(2-hydroxy-ethoxy)-2-trifluoromethyl-phenyl]-amide

577 mg (81%) of silyl ether was obtained as a white solid from 1-(3,5-dimethoxy-phenyl)-3-(4-piperazin-1-yl-phenyl)-urea (356 mg) obtained in Example (216b) and 4-[2-(tert-butyl-dimethyl-silanyloxy)-ethoxy]-2-trifluoromethyl-phenylamine (335 mg) obtained in Example (209c) in the same manner as in Example 170. This silyl ether was deprotected with tetrabutylammonium fluoride in the same manner as in Example (209d) and 422 mg (87%) of the title compound was obtained as a white solid.
$^1$H NMR(400MHz,DMSO-d6):δ(ppm)=8.59(1H, s), 8.42(1H, s), 8.20(1H, s), 7.29(2H, d, J=8.9Hz), 7.29(1H, d, J=8.9Hz), 7.20(1H, dd, J=9.0 and 2.8Hz), 7.15(1H, d, J=2.7Hz), 6.91(2H, d, J=9.0Hz), 6.65(2H, d, J=1.9Hz), 6.10(1H, t, J=2.2Hz), 4.90(1H, dd, J=5.6 and 5.6Hz), 4.05(2H, dd, J=4.9 and 4.9Hz), 3.73-3.70(2H, m), 3.70(6H, s), 3.55(4H, t, J=4.9Hz), 3.05 (4H, t, J=4.9Hz).
MS($ES^+$) m/z:604 $(M + H)^+$.
Melting point: 209-210˚C.

(Example 217) 4-{4-[3-(2-methoxy-phenyl)-ureido]-phenyl}-piperazine-1-carboxylic acid [4-(2-hydroxy-ethoxy)-2-trifluoromethyl-phenyl]-amide (Compound No. 1-597)

561 mg (82%) of silyl ether was obtained as a beige solid from 1-(2-methoxy-phenyl)-3-(4-piperazin-1-yl-phenyl)-urea (326 mg) obtained in Example (124a) and 4-[2-(tert-butyl-dimethyl-silanyloxy)-ethoxy]-2-trifluoromethyl-phenylamine (335 mg) obtained in Example (209c) in the same manner as in Example 170. This silyl ether was deprotected with tetrabutylammonium fluoride in the same manner as in Example (209d) and 436 mg (93%) of the title compound was obtained as a white solid.
$^1$H NMR(400MHz,DMSO-d6):δ(ppm)=9.10(1H, s), 8.22(1H, s), 8.14(1H, d, J=7.8Hz), 8.13(1H, s), 7.34(2H, d, J=7.9Hz), 7.32(1H, d, J=8.2Hz), 7.22(1H, d, J=9.4Hz), 7.18(1H, s), 7.02(1H, d, J=7.8Hz), 6.94(2H, d, J=8.6Hz), 6.92-6.87(2H, m), 4.91(1H, dd, J=4.7 and 4.7Hz), 4.07(2H, dd, J=4.3 and 4.3Hz), 3.88(3H, s), 3.73(2H, dd, J=9.6 and 4.1Hz), 3.56(4H, brs), 3.06(4H, brs).
MS($ES^+$) m/z:574 $(M + H)^+$.
Melting point: 118-120˚C.

(Example 218) 4-{4-[3-(2-methoxy-5-methyl-phenyl)-ureido]-phenyl}-perhydro-1,4-diazepine-1-carboxylic acid [4-(2-hydroxy-ethoxy)-2-trifluoromethyl-phenyl]-amide (Compound No. 2-107)

507 mg (82%) of silyl ether was obtained as a yellow solid from 1-(2-methoxy-5-methyl-phenyl)-3-(4-perhydro-1,4-diazepin-1-yl-phenyl)-urea (354 mg) obtained in Example (200d) and 4-[2-(tert-butyl-dimethyl-silanyloxy)-ethoxy]-2-trifluoromethyl-phenylamine (335 mg) obtained in Example (209c) in the same manner as in Example 170. This silyl ether was deprotected with tetrabutylammonium fluoride in the same manner as in Example (209d) and 390 mg (92%) of the title compound was obtained as a yellow solid.
$^1$H NMR(400MHz,DMSO-d6):8(ppm)=8.89(1H, s), 7.98(1H, s), 7.97(1H, d, J=1.9Hz), 7.88(1H, s), 7.22(2H, d, J=9.0Hz), 7.16(2H, s), 7.15(1H, d, J=7.0Hz), 6.85(1H, d, J=8.6Hz), 6.70(2H, d, J=9.4Hz), 6.69(1H, d, J=8.6Hz), 4.88(1H, dd, J=5.4 and 5.4Hz), 4.03(2H, dd, J=4.8 and 4.8Hz), 3.82(3H, s), 3.70(2H, dd, J=10.1 and 5.1Hz), 3.58-3.55(2H, m), 3.53-3.49 (4H, m), 3.30-3.27(2H, m), 2.22(3H, s), 1.98-1.94(2H, m).
MS($ES^+$) m/z:602 $(M + H)^+$.
Melting point: 118-120˚C.

(Example 219) 4-{5-[3-(2-methoxy-5-methyl-phenyl)-ureido]-pyridin-2-yl}-piperazine-1-carboxylic acid [4-(2-hydroxy-ethoxy)-2-methyl-phenyl]-amide (Compound No. 1-693)

(133 mg) (41%) of silyl ether was obtained as a white solid from 1-(2-methoxy-5-methyl-phenyl)-3-(6-piperazin-1-yl-pyridin-3-yl)-urea (171 mg) obtained in Example (137a) and 4-[2-(tert-butyl-dimethyl-silanyloxy)-ethoxy]-2-methyl-phenylamine (141 mg) obtained in Example (208c) in the same manner as in Example 170. This silyl ether was deprotected with tetrabutylammonium fluoride in the same manner as in Example (209d) and 96 mg (93%) of the title compound was obtained as a white solid.
$^1$H NMR(400MHz,DMSO-d6):δ(ppm)=9.06(1H, s), 8.14(1H, d, J=2.7Hz), 8.09(1H, s), 8.01(1H, s), 7.94(1H, d, J=2.0Hz),

7.73(1H, dd, J=9.4 and 2.7Hz), 7.01(1H, d, J=8.6Hz), 6.87(1H, d, J=8.2Hz), 6.86(1H, d, J=9.4Hz), 6.75(1H, d, J=2.7Hz), 6.71(1H, dd, J=8.2 and 2.0Hz), 6.68(1H, dd, J=8.6 and 2.7Hz), 4.83(1H, t, J=5.5Hz), 3.93(2H, t, J=5.1Hz), 3.82(3H, s), 3.68(2H, dt, J=5.5 and 5.1Hz), 3.56-3.49(4H, m), 3.46-3.40(4H, m), 2.22(3H, s), 2.12(3H, s).
MS($ES^+$) m/z:535 $(M + H)^+$.
Melting point: 189-190˚C.

(Example 220) 4- {5-[3-(2-methoxy-5-methyl-phenyl)-ureido]-pyridin-2-yl}-piperazine-1-carboxylic acid [4-(2-hydroxy-ethoxy)-2-trifluoromethyl-phenyl]-amide (Compound No. 1-615)

(123 mg) (35%) of silyl ether was obtained as a white solid from 1-(2-methoxy-5-methyl-phenyl)-3-(6-piperazin-l-yl-pyridin-3-yl)-urea (171 mg) obtained in Example (137a) and 4-[2-(tert-butyl-dimethyl-silanyloxy)-ethoxy]-2-trifluoromethyl-phenylamine (168 mg) obtained in Example (209c) in the same manner as in Example 170. This silyl ether was deprotected with tetrabutylammonium fluoride and 62.2 mg (66%) of the title compound was obtained as a white solid.
$^1$H NMR(400MHz,DMSO-d6):δ(ppm)=9.07(1H, s), 8.19(1H, s), 8.14(1H, d, J=2.7Hz), 8.09(1H, s), 7.94(1H, d, J=2.0Hz), 7.73(1H, dd, J=9.2 and 2.7Hz), 7.29(1H, d, J=8.2Hz), 7.20(1H, dd, J=8.2 and 2.7Hz), 7.15(1H, d, J=2.7Hz), 6.87(1H, d, J=8.0Hz), 6.86(1H, d, J=9.2Hz), 6.71(1H, dd, J=8.0 and 2.0Hz), 4.90(1H, t, J=5.7Hz), 4.05(2H, t, J=4.7Hz), 3.82(3H, s), 3.71(2H, dt, J=5.7 and 4.7Hz), 3.55-3.49(4H, m), 3.44-3.39(4H, m), 2.22(3H, s).
MS($ES^+$) m/z:589 $(M + H)^+$.
Melting point: 198-200˚C.

(Example 221) 4- {2-chloro-4-[3-(2-fluoro-phenyl)-ureido]-phenyl}-piperazine-1-carboxylic acid [4-(2-hydroxy-ethoxy)-2-trifluoromethyl-phenyl]-amide (Compound No. 2-121)

(221a) 4-(2-chloro-4-nitro-phenyl)-piperazine-1-carboxylic acid tert-butyl ester

5.74 g (84%) of the title compound was obtained as a yellow solid from 3-chloro-4-fluoronitrobenzene (3.52 g) and piperazine-1-carboxylic acid tert-butyl ester (4.46 g) in the same manner as in Example (38a). $^1$H NMR(40OMHz, $CDCl_3$):δ(ppm)=8.26(1H, d, J=2.7Hz), 8.17(1H, dd, J=9.0 and 2.7Hz), 7.31(1H, d, J=9.0Hz), 3.51(4H, t, J=4.9Hz), 3.15 (4H, t, J=4.9Hz), 1.43(9H, s).

(221b) 4-(4-amino-2-chloro-phenyl)-piperazine-1-carboxylic acid tert-butyl ester

Zinc powder (1.96 g) and acetic acid (1.0 mL) were added slowly to a solution of 4-(2-chloro-4-nitro-phenyl)-piperazine-1-carboxylic acid tert-butyl ester (1.02 g) obtained in Example (221 a) in methanol (20 mL) and anhydrous tetrahydrofuran (20 mL) at room temperature. The reaction mixture was stirred for one hour, followed by addition of a saturated sodium hydrogen carbonate aqueous solution. The mixture was stirred for another hour and filtered through Celite, followed by extraction with ethyl acetate. The organic layer was washed with saturated brine and dried over sodium sulfate and concentrated. The residue was purified by column chromatography (dichloromethane:ethyl acetate 4:1). The obtained solid was vigorously stirred in hexane/isopropanol (5:1), collected by filtration and dried under reduced pressure, and 684 mg (73%) of the title compound was obtained as a pale yellow solid.
$^1$H NMR(400MHz,$CDCl_3$):δ(ppm)=6.86(1H, d, J=8.6Hz), 6.61(1H, d, J=2.8Hz), 6.46(1H, dd, J=8.6 and 2.8Hz), 5.06(2H, s), 3.41(4H, t, J=4.9Hz), 2.73(4H, t, J=4.9Hz), 1.41(9H, s).

(221c) 4-{2-chloro-4-[3-(2-fluoro-phenyl)-ureido]-phenyl}-piperazine-1-carboxylic acid tert-butyl ester

749 mg (77%) of the title compound was obtained as a pale yellow solid from 4-(4-amino-2-chloro-phenyl)-piperazine-1-carboxylic acid tert-butyl ester (672 mg) obtained in Example (221b) and 2-fluorophenyl isocyanate (0.29 mL) in the same manner as in Example 1.
$^1$H NMR(400MHz,DMSO-d6):δ(ppm)=9.09(1H, s), 8.52(1H, s), 8.10(1H, ddd, J=8.2, 8.2 and 1.7Hz), 7.69(1H, d, J=2.4Hz), 7.26-7.18(2H, m), 7.16-7.08(2H, m), 7.16-7.08(1H, m), 3.48-3.41(4H, m), 2.87-2.81(4H, m), 1.41(9H, s).
MS($ES^+$) m/z:449 $(M + H)^+$.

(221d) 1-(3-chloro-4-piperazin-1-yl-phenyl)-3-(2-fluoro-phenyl)-urea

655 mg (quantitative yield) of the title compound was obtained as a white solid from 4-{2-chloro-4-[3-(2-fluoro-phenyl)-ureido]-phenyl}-piperazine-l-carboxylic acid tert-butyl ester (690 mg) obtained in Example (22 1 c) in the same manner as in Example (47a).
MS($ES^+$) m/z:349 $(M + H)^+$.

(221e) 4-{2-chloro-4-[3-(2-fluoro-phenyl)-ureido]-phenyl}-piperazine-1-carboxylic acid [4-(2-hydroxy-ethoxy)-2-trifluoromethyl-phenyl)-amide

224 mg (63%) of silyl ether was obtained as a pale yellow solid from 1-(3-chloro-4-piperazin-1-yl-phenyl)-3-(2-fluoro-phenyl)-urea (175 mg) obtained in Example (221d) and 4-[2-(tert-butyl-dimethyl-silanyloxy)-ethoxy]-2-trifluoromethyl-phenylamine (168 mg) obtained in Example (209c) in the same manner as in Example 170. This silyl ether was deprotected with tetrabutylammonium fluoride in the same manner as in Example (209d) and 92 mg (77%) of the title compound was obtained as a white solid.
$^1$H NMR(400MHz,DMSO-d6):δ(ppm)=9.14(1H, s), 8.56(1H, d, J=2.3Hz), 8.23(1H, s), 8.14(1H, ddd, J=8.2, 8.2 and 1.5Hz), 7.74(1H, d, J=2.3Hz), 7.33(1H, d, J=8.6Hz), 7.29-7.22(3H, m), 7.22-7.14(3H, m), 7.07-7.00(1H, m), 4.95(1H, t, J=5.5Hz), 4.10(2H, t, J=4.9Hz), 3.76(2H, dt, J=5.5 and 4.9Hz), 3.62-3.57(4H, m), 2.98-2.92(4H, m).
MS(ES$^+$) m/z:596 (M + H)$^+$.
Melting point: 199-200˚C.

(Example 222) 4-{5-[3-(2-fluoro-phenyl)-ureido]-pyridin-2-yl}-piperazine-1-carboxylic acid [4-(2-hydroxy-ethoxy)-2-trifluoromethyl-phenyl]-amide (Compound No. 1-608)

(222a) 4-{5-[3-(2-fluoro-phenyl)-ureido]-pyridin-2-yl}-piperazine-1-carboxylic acid tert-butyl ester

16.0 g (36%) of the title compound was obtained as a purple solid from 4-(5-amino-pyridin-2-yl)-piperazine-1-carboxylic acid tert-butyl ester (29.2 g) obtained in Example (38b) and 2-fluorophenyl isocyanate (14.1 mL) in the same manner as in Example (38c).
$^1$H NMR(400MHz,DMSO-d6):δ(ppm)=8.80(1H, s), 8.48(1H, d, J=2.4Hz), 8.14(1H, d, J=2.7Hz), 8.10(1H, ddd, J=8.4, 8.4 and 1.7Hz), 7.71(1H, dd, J=9.0 and 2.7Hz), 7.21(1H, ddd, J=11.6, 8.1 and 1.4Hz), 7.11(1H, ddd, J=8.4, 7.8 and 1.4Hz), 7.01-6.94(1H, m), 6.83(1H, d, J=9.0Hz), 3.45-3.35(8H, m), 1.42(9H, s).
MS(ES$^+$) m/z:416 (M + H)$^+$.
Melting point: 178-179˚C.

(222b) 1-(2-fluoro-phenyl)-3-(6-piperazin-1-yl-pyridin-3-yl)-urea

11.4 g (quantitative yield) of the title compound was obtained as a purple solid from 4-{5-[3-(2-fluoro-phenyl)-ureido]-pyridin-2-yl}-piperazine-1-carboxylic acid tert-butyl ester (15.0 g) obtained in Example (222a) in the same manner as in Example (47a).
MS(ES$^+$) m/z:316 (M + H)$^+$.

(222c) 4-{5-[3-(2-fluoro-phenyl)-ureido]-pyridin-2-yl}-piperazine-1-carboxylic acid [4-(2-hydroxy-ethoxy)-2-trifluoromethyl-phenyl)-amide

(174 mg) (5 1 %) of silyl ether was obtained as a pale yellow solid from 1-(2-fluoro-phenyl)-3-(6-piperazin-1-yl-pyridin-3-yl)-urea (158 mg) obtained in Example (222b) and 4-[2-(tert-butyl-dimethyl-silanyloxy)-ethoxy]-2-trifluoromethyl-phenylamine (168 mg) obtained in Example (209c) in the same manner as in Example 170. This silyl ether was deprotected with tetrabutylammonium fluoride in the same manner as in Example (209d) and 67.8 mg (60%) of the title compound was obtained as a white solid.
$^1$H NMR(400MHz,DMSO-d6):δ(ppm)=9.06(1H, s), 8.64(1H, s), 8.26(1H, s), 8.25(1H, s), 8.04(1H, dd, J=7.6 and 7.6Hz), 7.85(1H, d, J=9.0Hz), 7.29(1H, d, J=8.6Hz), 7.26-7.18(3H, m), 7.18-7.10(2H, m), 7.07-6.98(1H, m), 4.05(2H, t, J=4.9Hz), 3.72(2H, t, J=4.9Hz), 3.86-3.32(8H, m).
MS(ES$^+$) m/z:563 (M + H)$^+$.
Melting point: 193-194˚C.

(Example 223) 4- {5-[3-(5-fluoro-2-methoxy-phenyl)-ureido]-pyridin-2-yl} - piperazine-1-carboxylic acid [4-(2-hydroxy-ethoxy)-2-trifluoromethyl-phenyl]-amide (Compound No. 1-611)

(144 mg) (41%) of silyl ether was obtained as a white solid from 1-(5-fluoro-2-methoxy-phenyl)-3-(6-piperazin-1-yl-pyridin-3-yl)-urea (173 mg) obtained in Example (148a) and 4-[2-(tert-butyl-dimethyl-silanyloxy)-ethoxy]-2-trifluoromethyl-phenylamine (168 mg) obtained in Example (209c) in the same manner as in Example 170. This silyl ether was deprotected with tetrabutylammonium fluoride in the same manner as in Example (209d) and 72.2 mg (66%) of the title compound was obtained as a white solid.
$^1$H NMR(400MHz,DMSO-d6):δ(ppm)=9.21 (1H, s), 8.39(1H, s), 8.23(1H, s), 8.17(1H, d, J=2.7Hz), 8.01(1H, dd, J=11.3

and 3.1Hz), 7.76(1H, dd, J=9.0 and 2.7Hz), 7.32(1H, d, J=8.6Hz), 7.22(1H, dd, J=8.6 and 2.4Hz), 7.18(1H, d, J=2.4Hz), 7.01(1H, dd, J=8.4 and 5.3Hz), 6.90(1H, d, J=9.0Hz), 6.74(1H, ddd, J=8.6, 8.4 and 3.1Hz), 4.91(1H, t, J=5.7Hz), 4.07 (2H, t, J=4.7Hz), 3.88(3H, s), 3.73(2H, dt, J=4.7 and 5.7Hz), 3.56-3.50(4H, m), 3.48-3.40(4H, m).
MS(ES$^+$) m/z:593 (M + H)$^+$.
Melting point: 172-173˚C.

(Example 224) 4-{4-[3-(4-tert-butyl-thiazol-2-yl)-ureido]-phenyl}-piperazine-1-carboxylic acid [4-(2-hydroxy-ethoxy)-2-trifluoromethyl-phenyl]-amide (Compound No. 1-607)

(224a) 4-[4-(4-nitro-phenoxycarbonylamino)-phenyl]-piperazine-1-carboxylic acid tert-butyl ester

p-Nitrophenyl chloroformate (4.16 g) and pyridine (2.0 mL) were added to a solution of N-tert-butyloxycarbonyl-N'-(4-aminophenyl)-piperazine (5.54 g) in anhydrous tetrahydrofuran (40 mL) at 0˚C. The reaction mixture was stirred at 0˚C for one hour and stirred at room temperature for three hours and diluted with 1N hydrochloric acid, followed by extraction with ethyl acetate. The organic layer was washed with a saturated sodium hydrogen carbonate aqueous solution and saturated brine, dried over sodium sulfate and concentrated. The obtained solid was collected by filtration and dried and 6.42 g (72%) of the title compound was obtained as a pale yellow solid.
$^1$H NMR(400MHz,DMSO-d6):δ(ppm)=8.26(2H, d, J=9.OHz), 7.37(2H, d, J=9.0Hz), 7.34(2H, d, J=9.OHz), 6.97(IH, brs), 6.92(2H, d, J=9.OHz), 3.58(4H, t, J=5.1Hz), 3.10(4H, t, J=5.1Hz), 1.48(9H, s).

(224b) 4-{4-[3-(4-tert-butyl-thiazol-2-yl)-ureido]-phenyl}-piperazine-1-carboxylic acid tert-butyl ester

4-tert-butyl-thiazol-2-ylamine (469 mg) was added to a solution of 4-[4-(4-nitro-phenoxycarbonylamino)-phenyl]-piperazine-1-carboxylic acid tert-butyl ester (885 mg) obtained in Example (224a) in acetonitrile (10 mL). The reaction mixture was heated under reflux for four hours and concentrated. The residue was purified by column chromatography (hexane/ethyl acetate 2:1 → 1:1). The obtained solid was vigorously stirred in isopropyl ether, collected by filtration and dried and 716 mg (78%) of the title compound was obtained as a white solid.
$^1$H NMR(400MHz,DMSO-d6):δ(ppm)=10.5(1H, s), 8.61(1H, s), 7.31(2H, d, J=8.6Hz), 6.93(2H, d, J=8.6Hz), 6.63(1H, s), 3.46(4H, t, J=4.6Hz), 3.02(4H, t, J=4.6Hz), 1.42(9H, s), 1.25(9H, s).
MS(ES$^+$) m/z:460 (M + H)$^+$.
Melting point: 123-124˚C.

(224c) 1-(4-tert-butyl-thiazol-2-yl)-3-(4-piperazin-1-yl-phenyl)-urea

521 mg (95%) of the title compound was obtained as a white solid from 4-{4-[3-(4-tert-butyl-thiazol-2-yl)-ureido]-phenyl}-piperazine-1-carboxylic acid tert-butyl ester (704 mg) obtained in Example (224b) in the same manner as in Example (47a). MS(ES$^+$) m/z:360 (M + H)$^+$.

(224d) 4-{4-[3-(4-tert-butyl-thiazol-2-yl)-ureido]-phenyl}-piperazine-1-carboxylic acid [4-(2-hydroxy-ethoxy)-2-trifluoromethyl-phenyl]-amide

(125 mg) (35%) of silyl ether was obtained as a white solid from 1-(4-tert-butyl-thiazol-2-yl)-3-(4-piperazin-1-yl-phenyl)-urea (180 mg) obtained in Example (224c) and 4-[2-(tert-butyl-dimethyl-silanyloxy)-ethoxy]-2-trifluoromethyl-phenylamine (168 mg) obtained in Example (209c) in the same manner as in Example 170. This silyl ether was deprotected with tetrabutylammonium fluoride and 45.5 mg (46%) of the title compound was obtained as a grey white solid.
$^1$H NMR(400MHz,DMSO-d6):δ(ppm)=10.5(1H, s), 8.63(1H, s), 8.23(1H, s), 7.33(2H, d, J=8.6Hz), 7.32(1H, d, J=9.0Hz), 7.22(1H, d, J=9.0Hz), 7.18(1H, s), 6.97(2H, d, J=8.6Hz), 6.63(1H, s), 4.91(1H, t, J=5.7Hz), 4.07(2H, t, J=4.3Hz), 3.73 (2H, dt, J=5.7 and 4.3Hz), 3.61-3.51(4H, m), 3.13-3.03(4H, m), 1.25(9H, s). MS(ES$^+$) m/z:607 (M + H)$^+$.
Melting point: 130-132˚C.

(Example 225) 4-{4-[3-(4,5-dimethyl-thiazol-2-yl)-ureido]-phenyl}-piperazine-1-carboxylic acid [4-(2-hydroxy-ethoxy)-2-trifluoromethyl-phenyl]-amide (Compound No. 1-848)

(225a) 4-{4-[3-(4,5-dimethyl-thiazol-2-yl)-ureido]-phenyl}-piperazine-1-carboxylic acid tert-butyl ester

333 mg (38%) of the title compound was obtained as a white solid from 4-[4-(4-nitro-phenoxycarbonylamino)-phenyl]-piperazine-1-carboxylic acid tert-butyl ester (885 mg) obtained in Example (224a) and 4,5-dimethyl-thiazol-2-ylamine hydrochloride (494 mg) and triethylamine (0.42 mL) in the same manner as in Example (224b).

$^1$H NMR(400MHz,DMSO-d6):δ(ppm)=10.2(1H, s), 8.82(1H, s), 7.33(2H, d, J=8.8Hz), 6.92(2H, d, J=8.8Hz), 3.45(4H, t, J=4.9Hz), 3.02(4H, t, J=4.9Hz), 2.20(3H, s), 2.11(3H, s), 1.42(9H, s).
MS(ES$^+$) m/z:432 (M + H)$^+$.
Melting point: 103-105˚C.

(225b) 1-(4,5-dimethyl-thiazol-2-yl)-3-(4-piperazin-1-yl-phenyl)-urea

243 mg (quantitative yield) of the title compound was obtained as a white solid from 4-{4-[3-(4,5-dimethyl-thiazol-2-yl)-ureido]-phenyl}-piperazine-1-carboxylic acid tert-butyl ester (316 mg) obtained in Example 225a in the same manner as in Example (47a).
MS(ES$^+$) m/z:332 (M + H)$^+$.

(225c) 4-{4-[3-(4,5-dimethyl-thiazol-2-yl)-ureido]-phenyl}-piperazine-1-carboxylic acid [4-(2-hydroxy-ethoxy)-2-trifluoromethyl-phenyl]-amide

(175 mg) (51%) of silyl ether was obtained as a white solid from 1-(4,5-dimethyl-thiazol-2-yl)-3-(4-piperazin-1-yl-phenyl)-urea (166 mg) obtained in Example (225b) and 4-[2-(tert-butyl-dimethyl-silanyloxy)-ethoxy]-2-trifluoromethyl-phenylamine (168 mg) obtained in Example (209c) in the same manner as in Example 170. This silyl ether was deprotected with tetrabutylammonium fluoride and 89.0 mg (29%) of the title compound was obtained as a white solid.
$^1$H NMR(400MHz,DMSO-d6):δ(ppm)=10.1(1H, brs), 8.75(1H, s), 8.22(1H, s), 7.34(2H, d, J=9.3Hz), 7.32(1H, d, J=8.6Hz), 7.22(1H, dd, J=8.6 and 3.1Hz), 7.18(1H, d, J=3.1Hz), 6.95(2H, d, J=9.3Hz), 4.91(1H, t, J=5.5Hz), 4.07(2H, t, J=4.7Hz), 3.73(2H, dt, J=5.5 and 4.7Hz), 3.56(4H, t, J=4.5Hz), 3.07(4H, t, J=4.5Hz), 2.20(3H, s), 2.11(3H, s).
MS(ES$^+$) m/z:579 (M + H)$^+$.
Melting point: 166-167˚C.

(Example 226) 6-[3-(2-methoxy-5-methyl-phenyl)-ureido]-3,4-dihydro-1H-isoquinoline-2-carboxylic acid [4-(2-hydroxy-ethoxy)-2-trifluoromethyl-phenyl]-amide (Compound No. 3-94)

Silyl ether (185 mg) was obtained as a white solid from 1-(2-methoxy-5-methyl-phenyl)-3-(1,2,3,4-tetrahydro-isoquinolin-6-yl)-urea (225 mg) obtained in Example (202b) and 4-[2-(tert-butyl-dimethyl-silanyloxy)-ethoxy]-2-trifluoromethyl-phenylamine (234 mg) obtained in Example (209c) in the same manner as in Example 170. This silyl ether was deprotected with tetrabutylammonium fluoride in the same manner as in Example (209d) and 83 mg (43%) of the title compound was obtained as a white solid.
$^1$H NMR(400MHz,MeOH-d4):δ(ppm)=8.00(1H, s), 7.90(1H, s), 7.33(1H, s), 7.31(1H, s), 7.26(1H, s), 7.22(1H, s), 7.19 (2H, d, J=2.8Hz), 7.12(1H, d, J=8.6Hz), 7.12(1H, d, J=8.6Hz), 6.80(1H, d, J=8.2Hz), 6.75(1H, d, J=8.2Hz), 4.87(1H, s), 4.58(2H, s), 4.03-4.02(2H, m), 3.86(2H, t, J=4.4Hz), 3.81(3H, s), 3.68(2H, t, J=5.3Hz), 2.84(2H, brs), 2.25(3H, s).
MS(FAB$^+$) m/z:559 (M + H)$^+$.
Melting point: 122-124˚C.

(Example 227) 6-[3-(2-fluoro-phenyl)-ureido]-3,4-dihydro-1H-isoquinoline-2-carboxylic acid [4-(2-hydroxy-ethoxy)-2-trifluoromethyl-phenyl]-amide (Compound No. 3-91)

(227a) 6-[3-(2-fluoro-phenyl)-ureido]-3,4-dihydrido-1H-isoquinoline-2-carboxylic acid tert-butyl ester

559 mg (95%) of the title compound was obtained as a white solid from 6-amino-3,4-dihydro-1H-isoquinoline-2-carboxylic acid tert-butyl ester (380 mg) and 2-fluorophenyl isocyanate (250 mg) in the same manner as in Example (202a).
$^1$H NMR(400MHz,$CDCl_3$):δ(ppm)=8.12(1H, dd, J=8.2 and 8.2Hz), 7.13-6.95(8H, m), 4.51(2H, s), 3.62(2H, t, J=5.7Hz), 2.78(2H, t, J=5.5Hz), 1.50(9H, s).

(227b) 6-[3-(2-fluoro-phenyl)-ureido]-3,4-dihydro-1H-isoquinoline-2-carboxylic acid [4-(2-hydroxy-ethoxy)-2-trifluoromethyl-phenyl]-amide

Secondary amine was obtained from 6-[3-(2-fluoro-phenyl)-ureido]-3,4-dihydrido-1H-isoquinoline-2-carboxylic acid tert-butyl ester (100 mg) obtained in Example (227a) in the same manner as in Example (202b). 101 mg (60%) of silyl ether was obtained as a white solid from the secondary amine and 4-[2-(tert-butyldimethyl-silanyloxy)-ethoxy]-2-trifluoromethyl-phenylamine (104 mg) obtained in Example (209c) in the same manner as in Example 170. This silyl ether was deprotected with tetrabutylammonium fluoride in the same manner as in Example (209d) and 20 mg (24%)

of the title compound was obtained as a white solid.
$^1$H NMR(400MHz,MeOH-d4):δ(ppm)=8.05(1H, dd, J=8.0 and 8.0Hz), 7.33(1H, d, J=9.0Hz), 7.24(1H, s), 7.23-7.20(2H, m), 7.15(1H, dd, J=8.8 and 2.5Hz), 7.12-7.05(3H, m), 7.01-6.96(1H, m), 4.59(2H, s), 4.06(2H, t, J=4.3Hz), 3.91-3.85 (2H, m), 3.70(2H, t, J=5.6Hz), 2.88(2H, t, J=5.9Hz).
MS(ES$^+$)m/z:533 (M + H)$^+$.
Melting point: 162-164˚C.

(Example 228) 4-{4-[3-(2-methoxy-5-methyl-phenyl)-ureido]-phenyl}-piperidine-1-carboxylic acid (2-chloro-6-methyl-phenyl)-amide (Compound No. 4-14)

(228a) 4-trifluoromethanesulfonyloxy-3,6-dihydro-2H-pyridine-1-carboxylic acid tert-butyl ester

A solution of 4-oxo-piperidine-1-carboxylic acid tert-butyl ester (3.0 g) in anhydrous tetrahydrofuran (20 mL) was added dropwise slowly to a solution of lithium diisopropylamide (1.8M tetrahydrofuran solution, 9.5 mL) in anhydrous tetrahydrofuran (20 mL) under a nitrogen atmosphere at -78˚C. After 30 minutes, a solution of N-phenyl trifluoromethanesulfonimide (5.97 g) in anhydrous tetrahydrofuran (20 mL) was added dropwise. After 30 minutes, the reaction mixture was warmed to 0˚C and stirred for two hours. The reaction mixture was concentrated, dissolved in a hexane: ethyl acetate (9:1, 300 mL) solution and filtered through Celite. The filtrate was concentrated and the title compound (4.85 g) was obtained as an orange oil.
$^1$H NMR(400MHz,$CDCl_3$):δ(ppm)=5.75(1H, brs), 4.03(2H, dd, J=6.1 and 2.9Hz), 3.62(2H, t, J=5.7Hz), 2.45-2.40(2H, m), 1.47(9H, s).

(228b) 4-(4-benzyloxycarbonylaminophenyl)-3,6-dihydro-2H-pyridine-1-carboxylic acid tert-butyl ester

A solution of 4-trifluoromethanesulfonyloxy-3,6-dihydro-2H-pyridine-1-carboxylic acid tert-butyl ester (0.92 g) obtained in Example (228a), tetrakis triphenylphosphine palladium (160 mg), potassium carbonate (1.3 g) and (4-benzyloxycarbonylaminophenyl)boric acid (0.80 g) in dimethylformamide (50 mL) was heated at 100˚C for three hours. The reaction mixture was diluted with ethyl acetate and washed with saturated brine and dried over sodium sulfate and concentrated. The residue was purified by column chromatography (hexane: ethyl acetate 2:1) and 442 mg of the title compound was obtained as a pink solid.
MS(ES$^+$) m/z:409 (M + H)$^+$.

(228c) 4-(4-amino-phenyl)-piperidine-1-carboxylic acid tert-butyl ester

A solution of 4-(4-benzyloxycarbonylaminophenyl)-3,6-dihydro-2H-pyridine-1-carboxylic acid tert-butyl ester (100 mg) obtained in Example (228b) and palladium-carbon (15 mg) in methanol (30 ml) was stirred at room temperature under a hydrogen atmosphere for one hour. The reaction solution was filtered through Celite and concentrated. The residue was purified by chromatography (hexane: ethyl acetate 5:2) and 57 mg (84%) of the title compound was obtained as a yellow oil.
MS(ES$^+$) m/z:277 (M + H)$^+$.

(228d) 4-{4-[3-(2-methoxy-5-methyl-phenyl)-ureido]-phenyl}-piperidine-1-carboxylic acid tert-butyl ester

94 mg (quantitative yield) of the title compound was obtained as a pink oil from 4-(4-amino-phenyl)-piperidine-1-carboxylic acid tert-butyl ester (57 mg) obtained in Example (228c) and 2-methoxy-5-methylphenyl isocyanate (37 mg) in the same manner as in Example 1.
MS(ES$^+$) m/z:440 (M + H)$^+$.

(228e) 1-(2-methoxy-5-methyl-phenyl)-3-(4-piperidin-4-yl-phenyl)-urea

86 mg of the title compound obtained in the same manner as in Example (47a) from 4-{4-[3-(2-methoxy-5-methyl-phenyl)-ureido]-phenyl}-piperidine-1-carboxylic acid tert-butyl ester (94 mg) obtained in Example (228d) was used in Example (228f) without purification.

(228f) 4-{4-[3-(2-methoxy-5-methyl-phenyl)-ureido]-phenyl}-piperidine-1-carboxylic acid (2-chloro-6-methyl-phenyl)-amide

26 mg (26%) of the title compound was obtained as a white solid from 1-(2-methoxy-5-methyl-phenyl)-3-(4-piperidin-4-yl-phenyl)-urea (86 mg) obtained in Example (228e) and 2-chloro-6-methylphenyl isocyanate (45 mg) in the same manner as in Example 49.
$^1$H NMR(400MHz,DMSO-d6):δ(ppm)=9.22(1H, s), 8.12(2H, s), 7.97(1H, s), 7.37(2H, d, J=8.6Hz), 7.31(1H, dd, J=6.8 and 6.9Hz), 7.23-7.11(4H, m), 6.87(1H, dd, J=8.2 and 1.6Hz), 6.71(1H, d, J=7.4Hz), 4.20(2H, d, J=12.2Hz), 3.81(3H, s), 2.88(2H, t, J=12.8Hz), 2.76-2.65(1H, m), 2.26(3H, s), 2.20(3H, s), 1.75(2H, d, J=11.7Hz), 1.59-1.48(2H, m).
MS(ES$^+$) m/z:507 (M + H)$^+$.
Melting point: 234-236˚C.

(Example 229) 4-{4-[3-(2-fluoro-phenyl)-ureido]-phenyl}-3,6-dihydro-2H-pyridine-1-carboxylic acid [4-(2-hydroxy-ethoxy)-2-trifluoromethyl-phenyl]-amide (Compound No. 5-41)

(229a) 4-(4-aminophenyl)-3,6-dihydro-2H-pyridine-1-carboxylic acid tert-butyl ester

A solution of 4-(4-benzyloxycarbonylaminophenyl)-3,6-dihydro-2H-pyridine-1-carboxylic acid tert-butyl ester (200 mg) obtained in Example (228b) in 2N aqueous sodium hydroxide solution (5 mL) and methanol (15 ml) was heated at 60-70˚C for five hours. The reaction solution was concentrated and diluted with dichloromethane and washed with saturated brine and dried over sodium sulfate and concentrated, and the title compound was obtained as a pink oil.
$^1$H NMR(400MHz,$CDCl_3$):δ(ppm)=7.17(2H, d, J=8.6Hz), 6.63(2H, d, J=8.6Hz), 5.87(1H, brs), 4.03(2H, s), 3.68(2H, brs), 3.60(2H, t, J=5.7Hz), 2.46(2H, brs), 1.48(9H, s).

(229b) 4-{4-[3-(2-fluoro-phenyl)-ureido]-phenyl}-3,6-dihydro-2H-pyridine-1-carboxylic acid tert-butyl ester

559 mg (95%) of the title compound was obtained as a white solid from 4-(4-aminophenyl)-3,6-dihydro-2H-pyridine-1-carboxylic acid tert-butyl ester (380 mg) obtained in Example (229a) and 2-fluorophenyl isocyanate (253 mg) in the same manner as in Example 1.
$^1$H NMR(400MHz,DMSO-d6):δ(ppm)=8.12(1H, dd, J=8.1 and 8.0Hz), 8.03(1H, s), 7.68(1H, s), 7.29(2H, d, J=8.2Hz), 7.19(2H, d, J=8.6Hz), 7.06-6.89(2H, m), 5.90(1H, brs), 4.06(2H, brs), 3.62(2H, t, J=5.5Hz), 2.43(2H, brs), 1.51(9H, s).

(229c) 1-(2-fluoro-phenyl)-3-[4-(1,2,3,6-tetrahydro-pyridin-4-yl)-phenyl]-urea

The title compound obtained in the same manner as in Example (47a) from 4-{4-[3-(2-fluoro-phenyl)-ureido]-phenyl}-3,6-dihydro-2H-pyridine-1-carboxylic acid tert-butyl ester (100 mg) obtained in Example (229b) was used in Example (229d) without purification.

(229d) 4-{4-[3-(2-fluoro-phenyl)-ureido]-phenyl}-3,6-dihydro-2H-pyridine-1-carboxylic acid [4-(2-hydroxy-ethoxy)-2-trifluoromethyl-phenyl]-amide

36 mg (33%) of silyl ether was obtained as a white solid from 1-(2-fluorophenyl)-3-[4-(1,2,3,6-tetrahydro-pyridin-4-yl)-phenyl]-urea (50 mg) obtained in Example (229c) and 4-[2-(tert-butyl-dimethyl-silanyloxy)-ethoxy]-2-trifluoromethyl-phenylamine (59 mg) obtained in Example (209c) in the same manner as in Example 170. This silyl ether was deprotected with tetrabutylammonium fluoride in the same manner as in Example (209d) and 18 mg (61 %) of the title compound was obtained as a white solid.
$^1$H NMR(400MHz,MeOH-d4):δ(ppm)=8.09-8.04(1H, m), 7.44-7.38(2H, m), 7.42(2H, d, J=6.2Hz), 7.34(1H, d, J=8.7Hz), 7.23(1H, d, J=2.7Hz), 7.19(1H, dd, J=8.6 and 2.8Hz), 7.16-7.11(1H, m), 7.10(1H, d, J=7.8Hz), 7.04-6.97(1H, m), 5.44 (1H, dd, J=6.1 and 2.2Hz), 4.16(2H, d, J=3.1Hz), 4.09(2H, t, J=4.7Hz), 3.91-3.86(2H, m), 3.73(2H, t, J=5.5Hz), 2.63-2.57 (2H, m).
MS(ES$^+$) m/z:559 (M + H)$^+$.
Melting point: 110-112˚C.

(Example 230) 4-{4-[3-(2-methoxy-5-methyl-phenyl)-ureido]-phenyl}-3,6-dihydro-2H-pyridine-1-carboxylic acid [4-(2-hydroxy-ethoxy)-2-trifluoromethyl-phenyl]-amide (Compound No. 5-44)

(230a) 4-{4-[3-(2-methoxy-5-methyl-phenyl)-ureido]-phenyl}-3,6-dihydro-2H-pyridine-1-carboxylic acid tert-butyl ester

4.82 g (quantitative yield) of the title compound was obtained as a pale red solid from 4-(4-aminophenyl)-3,6-dihydro-2H-pyridine-1-carboxylic acid tert-butyl ester (3.00 g) obtained in Example (229a) and 2-methoxy-5-methylphenyl isocyanate (1.76 ml) in the same manner as in Example 1.
MS(APCI) m/z:438 (M + H)$^+$.

(230b) 1-(2-methoxy-5-methyl-phenyl)-3-[4-(1,2,3,6-tetrahydro-pyridin-4-yl)-phenyl]-urea

2.69 g (two steps, 73%) of the title compound was obtained as a yellow solid from 4- {4-[3-(2-methoxy-5-methyl-phenyl)-ureido]-phenyl}-piperidine- I -carboxylic acid tert-butyl ester (4.82 g) obtained in Example (228d) in the same manner as in Example (47a).
MS(APCI) m/z:338 $(M + H)^+$.

(230c) 4-{4-[3-(2-methoxy-5-methyl-phenyl)-ureido]-phenyl}-3,6-dihydro-2H-pyridine-1-carboxylic acid [4-(2-hydroxy-ethoxy)-2-trifluoromethyl-phenyl]-amide

Silyl ether (104 mg) was obtained from 1-(2-methoxy-5-methyl-phenyl)-3-[4-(1,2,3,6-tetrahydro-pyridin-4-yl)-phenyl]-urea (74 mg) obtained in Example (230b) and 4-[2-(tert-butyl-dimethyl-silanyloxy)-ethoxy]-2-trifluoromethyl-phenylamine (74 mg) obtained in Example (209c) in the same manner as in Example 170. This silyl ether was deprotected with tetrabutylammonium fluoride in an anhydrous tetrahydrofuran solution and 6.5 mg (7.5%) of the title compound was obtained as a light brown solid.
$^1$H NMR(400MHz,DMSO-d6):δ(ppm)=2.23(3H, s), 2.58-2.41(2H, m), 3.62(2H, t, J=6.1Hz), 3.71(2H, t, J=4.9Hz), 3.83 (3H, s), 4.05(2H, t, J=4.7Hz), 4.09(2H, s), 6.14(1H, s), 6.73(1H, d, J=7.8Hz), 6.88(1H, d, J=8.3Hz), 7.15(1H, d, J=3.2Hz), 7.20(1H, d, J=9.0Hz), 7.31(1H, d, J=9.8Hz), 7.38(2H, d, J=8.6Hz), 7.43(2H, d, J=8.6Hz), 7.96(1H, s), 8.10(1H, s), 8.15 (1H, s), 9.33(1H, s).
MS(APCI) m/z:585 $(M + H)^+$.
Melting point: 150-153˚C.

(Example 231) 4-{4-[3-(2-methoxy-5-methyl-phenyl)-ureido]-phenyl}-piperazine-1-carboxylic acid [2-chloro-5-(2-hydroxy-ethoxy)-phenyl]-amide (Compound No. 1-651)

(231 a) 2-(4-chloro-3-nitro-phenoxy)-ethanol

5.48 g (50%) of the title compound was obtained as a yellow oil from 4-chloro-3-nitrophenol (8.67 g) and 2-bromoethanol (3.9 mL) in the same manner as in Example (208a).
$^1$H NMR(400MHz,$CDCl_3$): δ(ppm)=7.44(1H, s), 7.41(1H, d, J=3.1Hz), 7.09(1H, dd, J=8.8 and 3.0Hz), 4.12(2H, t, J=4.5Hz), 4.00(2H, brs), 2.04(1H, brs).

(231b) tert-butyl-[2-(4-chloro-3-nitro-phenoxy)-ethoxy]-dimethylsilane

8.52 g (quantitative yield) of the title compound was obtained as a yellow oil from 2-(4-chloro-3-nitro-phenoxy)-ethanol (5.48 g) obtained in Example (231a) and tert-butyldimethylsilyl chloride (4.56 g) in the same manner as in Example (208b).
$^1$H NMR(400MHz,$CDCl_3$):δ(ppm)=7.31(1H, d, J=3.9Hz), 7.30(1H, s), 6.98(1H, dd, J=8.8 and 2.9Hz), 3.98(2H, t, J=4.9Hz), 3.88(2H, t, J=4.7Hz), 0.81(9H, s), 0.00(6H, s).

(231c) 5-[2-(tert-butyl-dimethyl-silanyloxy)-ethoxy]-2-chloro-phenylamine

tert-butyl-[2-(4-chloro-3-nitro-phenoxy)-ethoxy]-dimethylsilane(2.29 g) obtained in Example (231b) and nickel (II) chloride hexahydrate (3.28 g) were suspended in anhydrous tetrahydrofuran (40 mL). Sodium borohydride (1.02 g) was added slowly to this suspension in small portions at room temperature. After one hour, the reaction mixture was concentrated and poured on powdered cellulose, followed by addition of a saturated sodium hydrogen carbonate aqueous solution. The reaction product was eluted from powdered cellulose with ethyl acetate and water. The organic layer of the obtained eluate was washed with a saturated sodium hydrogen carbonate aqueous solution and saturated brine and dried over sodium sulfate and filtered and concentrated. The residue was dried under reduced pressure and 1.85 g (89%) of the title compound was obtained as a yellow oil.
$^1$H NMR(40OMHz,$CDCl_3$):δ(ppm)=7.02(1H, d, J=8.6Hz), 6.25(1H, d, J=2.7Hz), 6.19(1H, dd, J=9.0 and 2.7Hz), 3.92(2H, brs), 3.88-3.83(4H, m), 0.81(9H, s), 0.00(6H, s).

(231d) 4-{4-[3-(2-methoxy-5-methyl-phenyl)-ureido]-phenyl}-piperazine-1-carboxylic acid [2-chloro-5-(2-hydroxy-ethoxy)-phenyl]-amide

1.09 g (50%) of silyl ether was obtained as a white solid from 1-(2-methoxy-5-methyl-phenyl)-3-(4-piperazin-1-yl-phenyl)-urea (1.12 g) obtained in Example (47a) and 5-[2-(tert-butyl-dimethyl-silanyloxy)-ethoxy]-2-chloro-phenylamine (990 mg) obtained in Example (231c) in the same manner as in Example 170. This silyl ether was deprotected with tetrabutylammonium fluoride in an anhydrous tetrahydrofuran solution and 821 mg (91 %) of the title compound was obtained as a white solid.

$^1$H NMR(400MHz,DMSO-d6):δ(ppm)=9.09(1H, s), 8.20(1H, s), 8.07(1H, s), 7.99(1H, s), 7.34(2H, d, J=8.2Hz), 7.34(1H, d, J=8.2Hz), 7.21(1H, s), 6.95(2H, d, J=8.2Hz), 6.89(1H, d, J=8.2Hz), 6.76-6.72(2H, m), 4.88(1H, dd, J=4.9 and 4.9Hz), 3.96(2H, dd, J=4.3 and 4.3Hz), 3.84(3H, s), 3.71(2H, dd, J=9.6 and 4.5Hz), 3.60(4H, t, J=4.3Hz), 3.09(4H, t, J=3.7Hz), 2.23(3H, s).

MS(ES$^+$) m/z:555 (M + H)$^+$.

Melting point: 121-124˚C.

(Example 232) 4-{4-[3-(2-methoxy-phenyl)-ureido]-phenyl}-piperazine-1-carboxylic acid [2-chloro-5-(2-hydroxy-ethoxy)-phenyl]-amide (Compound No. 1-645)

519 mg (79%) of silyl ether was obtained as a white solid from 1-(2-methoxy-phenyl)-3-(4-piperazin-1-yl-phenyl)-urea (326 mg) obtained in Example (124a) and 5-[2-(tert-butyl-dimethyl-silanyloxy)-ethoxy]-2-chloro-phenylamine (331 mg) obtained in Example (231c) in the same manner as in Example 170. This silyl ether was deprotected with tetrabutylammonium fluoride in the same manner as in Example (209d) and 364 mg (85%) of the title compound was obtained as a white solid.

$^1$H NMR(400MHz,DMSO-d6):δ(ppm)=9.06(1H, s), 8.16(1H, s), 8.12-8.09(1H, m), 8.09(1H, s), 7.31(2H, d, J=8.6Hz), 7.31(1H, d, J=8.6Hz), 7.19(1H, d, J=2.8Hz), 6.99(1H, dd, J=7.9 and 1.5Hz), 6.92(2H, d, J=9.3Hz), 6.89-6.84(2H, m), 6.71(1H, dd, J=8.8 and 3.0Hz), 4.86(1H, dd, J=5.5 and 5.5Hz), 3.95(2H, t, J=4.9Hz), 3.86(3H, s), 3.69(2H, dd, J=10.2 and 5.1Hz), 3.59(4H, t, J=4.9Hz), 3.08(4H, t, J=4.9Hz).

MS(ES$^+$) m/z:542 (M + H)$^+$.

Melting point: 179-180˚C.

(Example 233) 4-{4-[3-(3-ethoxy-phenyl)-ureido]-phenyl}-piperazine-1-carboxylic acid [2-chloro-5-(2-hydroxy-ethoxy)-phenyl]-amide (Compound No. 1-858)

600 mg (90%) of silyl ether was obtained as a white solid from 1-(3-ethoxyphenyl)-3-(4-piperazin-1-yl-phenyl)-urea (340 mg) obtained in Example (215b) and 5-[2-(tert-butyl-dimethyl-silanyloxy)-ethoxy]-2-chloro-phenylamine (331 mg) obtained in Example (231c) in the same manner as in Example 170. This silyl ether was deprotected with tetrabutylammonium fluoride in the same manner as in Example (209d) and 234 mg (47%) of the title compound was obtained as a white solid.

$^1$H NMR(400MHz,DMSO-d6):δ(ppm)=8.61(1H, s), 8.46(1H, s), 8.16(1H, s), 7.31(2H, d, J=9.0Hz), 7.30(1H, d, J=9.4Hz), 7.19(1H, d, J=3.2Hz), 7.16(1H, dd, J=2.1 and 2.1Hz), 7.12(1H, dd, J=7.8 and 7.8Hz), 6.92(2H, d, J=9.0Hz), 6.88(1H, d, J=7.5Hz), 6.71(1H, dd, J=8.8 and 3.0Hz), 6.49(1H, dd, J=7.9 and 2.8Hz), 4.86(1H, dd, J=5.5 and 5.5Hz), 4.00-3.93 (4H, m), 3.70(2H, t, J=4.7Hz), 3.59(4H, t, J=4.9Hz), 3.08(4H, t, J=4.9Hz), 1.32(3H, t, J=7.1Hz).

MS(ES$^+$) m/z:555 (M + H)$^+$.

Melting point: 196-197˚C.

(Example 234) 4-{4-[3-(3,5-dimethoxy-phenyl)-ureido]-phenyl}-piperazine-1-carboxylic acid [2-chloro-5-(2-hydroxy-ethoxy)-phenyl]-amide (Compound No. 1-653)

518 mg (96%) of silyl ether was obtained as a white solid from 1-(3,5-dimethoxy-phenyl)-3-(4-piperazin-1-yl-phenyl)-urea (356 mg) obtained in Example (216b) and 5-[2-(tert-butyl-dimethyl-silanyloxy)-ethoxy]-2-chloro-phenylamine (331 mg) obtained in Example (231c) in the same manner as in Example 170. This silyl ether was deprotected with tetrabutylammonium fluoride in the same manner as in Example (209d) and 234 mg (47%) of the title compound was obtained as a white solid.

$^1$H NMR(400MHz,DMSO-d6):δ(ppm)=8.54(1H, s), 8.36(1H, s), 8.16(1H, s), 7.31(2H, d, J=9.0Hz), 7.29(1H, d, J=8.9Hz), 7.19(1H, d, J=3.2Hz), 6.92(2H, d, J=9.4Hz), 6.71(1H, dd, J=9.0 and 2.8Hz), 6.65(2H, d, J=2.4Hz), 6.10(1H, dd, J=2.2 and 2.2Hz), 4.86(1H, dd, J=5.5 and 5.5Hz), 3.95(2H, dd, J=4.9 and 4.9Hz), 3.70(6H, s), 3.70-3.67(2H, m), 3.59(4H, t, J=4.7Hz), 3.08(4H, t, J=4.7Hz).

MS(ES$^+$) m/z:571 (M + H)$^+$.

Melting point: 191-192˚C.

(Example 235) 4-{5-[3-(2-methoxy-phenyl)-ureido]-pyridin-2-yl}-piperazine-1-carboxylic acid [2-chloro-5-(2-hydroxy-ethoxy)-phenyl]-amide (Compound No. 1-657)

(235a) 4-{5-[3-(2-methoxy-phenyl)-ureido]-pyridin-2-yl}-piperazine-1-carboxylic acid tert-butyl ester

8.83 g (quantitative yield) of the title compound was obtained as a purple solid from 4-(5-amino-pyridin-2-yl)-piperazine-1-carboxylic acid tert-butyl ester (5.57 g) obtained in Example (38b) and 2-methoxyphenyl isocyanate (3.2 mL) in the same manner as in Example (38c).
$^1$H NMR(400MHz,$CDCl_3$):δ(ppm)=8.10(1H, s), 8.08(1H, d, J=3.9Hz), 7.71(1H, dd, J=9.0 and 2.7Hz), 7.08(1H, s), 7.03-6.94(2H, m), 6.86(1H, d, J=8.2Hz), 6.66(1H, d, J=9.0Hz), 6.49(1H, s), 3.81(3H, s), 3.55-3.48(8H, m), 1.49(9H, s).

(235b) 1-(2-methoxy-phenyl)-3-(6-piperazin-1-yl-pyridin-3-yl)-urea

6.28 g (96%) of the title compound was obtained as a purple solid from 4-{5-[3-(2-methoxy-phenyl)-ureido]-pyridin-2-yl}-piperazine-1-carboxylic acid tert-butyl ester (8.55 g) obtained in Example (235a) in the same manner as in Example (47a).
$^1$H NMR(400MHz,DMSO-d6):δ(ppm)=9.03(1H, s), 8.14-8.07(3H, m), 7.68(1H, d, J=8.6Hz), 6.99(1H, d, J=7.8Hz), 6.91 (1H, dd, J=7.6 and 7.6Hz), 6.86(1H, dd, J=7.2 and 7.2Hz), 6.76(1H, d, J=8.6Hz), 3.85(3H, s), 3.39(1H, brs), 3.28(4H, brs), 2.76(4H, brs).

(235c) 4-{5-[3-(2-methoxy-phenyl)-ureido]-pyridin-2-yl}-piperazine-1-carboxylic acid [2-chloro-5-(2-hydroxy-ethoxy)-phenyl]-amide

414 mg (63%) of silyl ether was obtained as a purple solid from 1-(2-methoxy-phenyl)-3-(6-piperazin-1-yl-pyridin-3-yl)-urea (327 mg) obtained in Example (235b) and 5-[2-(tert-butyl-dimethyl-silanyloxy)-ethoxy]-2-chloro-phenylamine (301 mg) obtained in Example (231c) in the same manner as in Example 170. This silyl ether was deprotected with tetrabutylammonium fluoride in the same manner as in Example (209d) and 312 mg (92%) of the title compound was obtained as a white solid.
$^1$H NMR(400MHz,DMSO-d6):δ(ppm)=9.08(1H, s), 8.16(1H, d, J=4.3Hz), 8.15(1H, d, J=3.2Hz), 8.09(1H, dd, J=7.8 and 2.0Hz), 7.73(1H, dd, J=9.0 and 2.7Hz), 7.31(1H, d, J=9.0Hz), 7.19(1H, d, J=3 .2Hz), 7.00(1H, dd, J=8.2 and 1.6Hz), 6.92(1H, ddd, J=7.6, 7.6 and 2.0Hz), 6.90(1H, d, J=1.9Hz), 6.89-6.84(2H, m), 6.72(1H, dd, J=8.6 and 3.2Hz), 4.87(1H, dd, J=5.5 and 5.5Hz), 3.94(2H, dd, J=4.9 and 4.9Hz), 3.87(3H, s), 3.69(2H, dd, J=10.1 and 5.5Hz), 3.56(4H, t, J=5.1Hz), 3.46(4H, t, J=3.5Hz). MS($ES^+$) m/z:542 $(M + H)^+$.
Melting point: 139-141 ˚C.

(Example 236) 4-{5-[3-(3-ethoxy-phenyl)-ureido]-pyridin-2-yl}-piperazine-1-carboxylic acid [2-chloro-5-(2-hydroxy-ethoxy)-phenyl]-amide (Compound No. 1-859)

(236a) 4-{5-[3-(3-ethoxy-phenyl)-ureido]-pyridin-2-yl}-piperazine-1-carboxylic acid tert-butyl ester

8.37 g (95%) of the title compound was obtained as a purple solid from 4-(5-amino-pyridin-2-yl)-piperazine-1-carboxylic acid tert-butyl ester (5.57 g) obtained in Example (38b) and 3-ethoxyphenyl isocyanate (3.5 mL) in the same manner as in Example (38c).
$^1$H NMR(400MHz,$CDCl_3$):δ(ppm)=8.08(1H, d, J=2.3Hz), 7.65(1H, dd, J=9.0 and 2.4Hz), 7.20(1H, dd, J=8.0 and 8.0Hz), 7.03(1H, s), 6.82(1H, d, J=7.9Hz), 6.66(2H, d, J=9.0Hz), 6.46(1H, s), 6.37(1H, s), 4.02(2H, q, J=6.9Hz), 3.55-3.50(8H, m), 1.49(9H, s), 1.40(3H, t, J=7.1Hz).

(236b) 1-(3-ethoxy-phenyl)-3-(6-piperazin-1-yl-pyridin-3-yl)-urea

6.47 g (quantitative yield) of the title compound was obtained as a purple solid from 4- {5-[3-(3-ethoxy-phenyl)-ureido]-pyridin-2-yl}-piperazine-1-carboxylic acid tert-butyl ester (8.37 g) obtained in Example (236a) in the same manner as in Example (47a).
$^1$H NMR(400MHz,DMSO-d6):δ(ppm)=8.74(1H, s), 8.48(1H, s), 8.11(1H, d, J=2.3Hz), 7.66(1H, dd, J=9.4 and 2.7Hz), 7.14(1H, d, J=2.3Hz), 7.11(1H, d, J=7.8Hz), 6.89(1H, dd, J=7.8 and 1.6Hz), 6.76(1H, d, J=8.6Hz), 6.49(1H, dd, J=8.2 and 1.6Hz), 3.97(2H, q, J=7.1Hz), 3.43(1H, brs), 3.30(4H, t, J=4.5Hz), 2.78(4H, t, J=4.3Hz), 1.31 (3H, t, J=6.9Hz).

(236c) 4-{5-[3-(3-ethoxy-phenyl)-ureido]-pyridin-2-yl}-piperazine-1-carboxylic acid [2-chloro-5-(2-hydroxy-ethoxy)-phenyl]-amide

527 mg (77%) of silyl ether was obtained as a pale purple solid from 1-(3-ethoxy-phenyl)-3-(6-piperazin-1-yl-pyridin-3-yl)-urea (341 mg) obtained in Example (235b) and 5-[2-(tert-butyl-dimethyl-silanyloxy)-ethoxy]-2-chloro-phenylamine (301 mg) obtained in Example (231c) in the same manner as in Example 170. This silyl ether was deprotected with tetrabutylammonium fluoride in the same manner as in Example (209d) and 334 mg (78%) of the title compound was obtained as a pale purple solid.
$^1$H NMR(400MHz,DMSO-d6):δ(ppm)=8.65(1H, s), 8.41(1H, s), 8.18(1H, s), 8.18(1H, dd, J=2.2 and 2.2Hz), 7.73(1H, dd, J=8.8 and 2.2Hz), 7.34(1H, d, J=8.9Hz), 7.22(1H, dd, J=1.9 and 1.9Hz), 7.17-7.13(2H, m), 6.90(2H, dd, J=9.4 and 9.4Hz), 6.74(1H, dd, J=8.6 and 2.7Hz), 6.53(1H, d, J=7.9Hz), 4.88(1H, dd, J=5.5 and 5.5Hz), 4.00-3.95(4H, m), 3.70 (2H, dd, J=10.3 and 5.2Hz), 3.58(4H, brs), 3.48(4H, brs), 1.32(3H, t, J=6.9Hz).
MS(ES$^+$) m/z:556 (M + H)$^+$.
Melting point: 140-143˚C.

(Example 237) 4-{5-(3-(4,5-dimethyl-thiazol-2-yl)-ureido)-pyridin-2-yl}-piperazine-1-carboxylic acid {2-chloro-5-(2-hydroxy-ethoxy)-phenyl}-amide (Compound No. 1-863)

(237a) 4-[5-(4-nitro-phenoxycarbonylamino)-pyridin-2-yl]-piperazine-1-carboxylic acid tert-butyl ester

10.8 g (81 %) of the title compound was obtained as a purple solid from 4-(5-amino-pyridin-2-yl)-piperazine-1-carboxylic acid tert-butyl ester (8.34 g) obtained in Example (38b) and p-nitrophenyl chloroformate (6.24 g) in the same manner as in Example (224a).
MS(FAB) m/z:444 (M + H)$^+$.

(237b) 4-{5-[3-(4,5-dimethyl-thiazol-2-yl)-ureido]-pyridin-2-yl}-piperazine-1-carboxylic acid tert-butyl ester

1.37 g (63%) of the title compound was obtained as a pale purple solid from 4-[5-(4-nitro-phenoxycarbonylamino)-pyridin-2-yl]-piperazine-1-carboxylic acid tert-butyl ester (2.22 g) obtained in Example (237a) and 4,5-dimethyl-thiazol-2-ylamine (988 mg) in the same manner as in Example (224b).
$^1$H NMR(400MHz,DMSO-d6):δ(ppm)=10.2(1H, s), 8.76(1H, s), 8.17(1H, d, J=2.7Hz), 7.71(1H, dd, J=9.0 and 2.7Hz), 6.84(1H, d, J=9.0Hz), 3.46-3.38(4H, m), 3.36-3.29(4H, m), 2.19(3H, s), 2.11(3H, s), 1.42(9H, s)
MS(ES$^+$) m/z:461 (M + H)$^+$.

(237c) 1-(4,5-dimethyl-thiazol-2-yl)-3-(6-piperazin-1-yl-pyridin-3-yl)-urea

1.02 mg (98%) of the title compound was obtained as a pale yellow solid from 4- {5-[3-(4,5-dimethyl-thiazol-2-yl)-ureido]-pyridin-2-yl}-piperazine-1-carboxylic acid tert-butyl ester (1.35 g) obtained in Example (237b) in the same manner as in Example (47a).
MS(ES$^+$) m/z:361 (M + H)$^+$.

(237d) 4-{5-(3-(4,5-dimethyl-thiazol-2-yl)-ureido)-pyridin-2-yl}-piperazine-1-carboxylic acid {2-chloro-5-(2-hydroxy-ethoxy)-phenyl}-amide

Silyl ether (149 mg) (45%) was obtained as a white solid from 1-(4,5-dimethyl-thiazol-2-yl)-3-(6-piperazin-1-yl-pyridin-3-yl)-urea (166 mg) obtained in Example (237c) and 5-[2-(tert-butyl-dimethyl-silanyloxy)-ethoxy]-2-chloro-phenylamine (151 mg) obtained in Example (231c) in the same manner as in Example 170. This silyl ether was deprotected with tetrabutylammonium fluoride in the same manner as in Example (209d) and 66 mg (58%) of the title compound was obtained as a pale pink solid.
$^1$H NMR(400MHz,DMSO-d6):δ(ppm)=10.3(1H, s), 8.76(1H, s), 8.20(1H, s), 8.19(1H, s), 7.74(1H, d, J=9.0Hz), 7.34(1H, d, J=9.0Hz), 7.21(1H, d, J=2.7Hz), 6.89(1H, d, J=9.0Hz), 6.74(1H, dd, J=9.0 and 2.7Hz), 4.88(1H, t, J=5.2Hz), 3.96(2H, t, J=5.0Hz), 3.70(2H, dt, J=5.2 and 5.0Hz), 3.60-3.54(4H, m), 3.52-3.45(4H, m), 2.19(3H, s), 2.11(3H, s).
MS(ES$^+$) m/z:546 (M + H)$^+$.
Melting point: 128-129˚C.

(Example 238) 4-{5-[3-(2-methoxy-5-methyl-phenyl)-ureido]-pyridin-2-yl}-piperazine-1-carboxylic acid [2-chloro-5-(2-hydroxy-ethoxy)-phenyl]-amide (Compound No. 1-663)

Silyl ether (149 mg) (44%) was obtained as a white solid from 1-(2-methoxy-5-methyl-phenyl)-3-(6-piperazin-1-yl-pyridin-3-yl)-urea (171 mg) obtained in Example (137a) and 5-[2-(tert-butyl-dimethyl-silanyloxy)-ethoxy]-2-chloro-phenylamine (151 mg) obtained in Example (231c) in the same manner as in Example 170. This silyl ether was deprotected with tetrabutylammonium fluoride in the same manner as in Example (209d) and 33 mg (30%) of the title compound was obtained as a white solid.
$^1$H NMR(400MHz,DMSO-d6):δ(ppm)=9.10(1H, s), 8.19(1H, s), 8.18(1H, d, J=2.7Hz), 8.12(1H, s), 7.97(1H, d, J=1.6Hz), 7.76(1H, dd, J=9.0 and 2.7Hz), 7.34(1H, d, J=9.0Hz), 7.21(1H, d, J=2.4Hz), 6.89(1H, d, J=9.0Hz), 6.89(1H, d, J=8.6Hz), 6.74(1H, dd, J=9.0 and 2.4Hz), 6.74(1H, dd, J=8.6 and 1.6Hz), 4.88(1H, t, J=5.7Hz), 3.96(2H, t, J=4.9Hz), 3.84(3H, s), 3.70(2H, dt, J=5.7 and 4.9Hz), 3.72-3.71(4H, m), 3.50-3.44(4H, m), 2.23(3H, s).
MS(ES$^+$) m/z:555 (M + H)$^+$.
Melting point: 132-134˚C.

(Example 239) 4-{4-[3-(2-methoxy-5-methyl-phenyl)-ureido]-2-methyl-phenyl}-piperazine-1-carboxylic acid [4-(2-hydroxy-ethoxy)-2-trifluoromethyl-phenyl]-amide (Compound No. 2-103)

(239a) 1-(2-methoxy-5-methyl-phenyl)-3-(3-methyl-4-piperazin-1-yl-phenyl)-urea

848 mg (87%) of the title compound was obtained as a white solid from 4-{4-[3-(2-methoxy-5-methyl-phenyl)-ureido]-2-methyl-phenyl}-piperazine-1-carboxylic acid tert-butyl ester (1.38 g) obtained in Example 195 in the same manner as in Example (47a).
MS(ES$^+$) m/z: 355 (M + H)$^+$.

(239b) 4-{4-[3-(2-methoxy-5-methyl-phenyl)-ureido]-2-methyl-phenyl}-piperazine-1-carboxylic acid [4-(2-hydroxy-ethoxy)-2-trifluoromethyl-phenyl]-amide

509 mg (71%) of silyl ether was obtained as a white solid from 1-(2-methoxy-5-methyl-phenyl)-3-(3-methyl-4-piperazin-l-yl-phenyl)-urea (354 mg) obtained in Example (239a) and 4-[2-(tert-butyl-dimethyl-silanyloxy)-ethoxy]-2-trifluoromethyl-phenylamine (335 mg) obtained in Example (209c) in the same manner as in Example 170. This silyl ether was deprotected with tetrabutylammonium fluoride in the same manner as in Example (209d) and 385 mg (90%) of the title compound was obtained as a white solid.
$^1$H NMR(400MHz,DMSO-d6):δ(ppm)=9.13(1H, s), 8.18(1H, s), 8.10(1H, s), 7.99(1H, s), 7.32(2H, d, J=8.2Hz), 7.31(1H, s), 7.22(1H, d, J=8.6Hz), 7.19(1H, s), 6.98(1H, d, J=8.6Hz), 6.89(1H, d, J=8.6Hz), 6.73(1H, d, J=7.8Hz), 4.91(1H, t, J=5.6Hz), 4.07(2H, t, J=4.7Hz), 3.84(3H, s), 3.73(2H, q, J=5.1Hz), 3.55(4H, brs), 2.79(4H, brs), 2.27(3H, s), 2.23(3H, s).
MS(ES$^+$) m/z:602 (M + H)$^+$.
Melting point: 225-228˚C.

(Example 240) 4-{4-[3-(2-trifluoromethoxy-phenyl)-ureido]-phenyl}-piperazine-1-carboxylic acid [4-(2-hydroxy-ethoxy)-2-trifluoromethyl-phenyl]-amide (Compound No. 1-868)

(240a) 4- {4-[3-(2-trifluoromethoxy-phenyl)-ureido]-phenyl}-piperazine-1-carboxylic acid tert-butyl ester

2.31 g (96%) of the title compound was obtained as a white solid from N-tert-butyloxycarbonyl-N'-(4-aminophenyl)-piperazine (1.39 g) and 2-trifluoromethoxyphenyl isocyanate (0.98 mL) in the same manner as in Example 1. $^1$H NMR(400MHz,$CDCl_3$):δ(ppm)=8.30(1H, d, J=8.2Hz), 7.32-7.18(3H, m), 7.04-6.89(6H, m), 3.60(4H, brs), 3.13(4H, brs), 1.50(9H, s).

(240b) 1-(2-trifluoromethoxy-phenyl)-3-(4-piperazin-1-yl-phenyl)-urea

1.83 g (quantitative yield) of the title compound was obtained as a white solid from 4-{4-[3-(2-trifluoromethoxy-phenyl)-ureido]-phenyl}-piperazine-1-carboxylic acid tert-butyl ester (2.31 g) obtained in Example (240a) in the same manner as in Example (47a).
$^1$H NMR(400MHz,DMSO-d6):δ(ppm)=9.14(1H, s), 8.47(1H, s), 8.24(1H, dd, J=8.2 and 1.5Hz), 7.33(1H, d, J=9.8Hz), 7.29(2H, d, J=9.0Hz), 7.29(1H, d, J=9.0Hz), 7.04(1H, ddd, J=7.8, 7.8 and 2.0Hz), 6.86(2H, d, J=9.3Hz), 3.41(1H, brs), 2.95(4H, t, J=4.7Hz), 2.81 (4H, t, J=4.9Hz).

(240c) 4-{4-[3-(2-trifluoromethoxy-phenyl)-ureido]-phenyl}-piperazine-1-carboxylic acid [4-(2-hydroxy-ethoxy)-2-trifluoromethyl-phenyl]-amide

537 mg (73%) of silyl ether was obtained as a white solid from 1-(2-trifluoromethoxy-phenyl)-3-(4-piperazin-1-yl-phenyl)-urea (381 mg) obtained in Example (240b) and 4-[2-(tert-butyl-dimethyl-silanyloxy)-ethoxy]-2-trifluoromethyl-phenylamine (335 mg) obtained in Example (209c) in the same manner as in Example 170. This silyl ether was deprotected with tetrabutylammonium fluoride in the same manner as in Example (209d) and 382 mg (84%) of the title compound was obtained as a white solid.
$^1$H NMR(400MHz,DMSO-d6):δ(ppm)=9.08(1H, s), 8.37(1H, s), 8.29(1H, d, J=8.2Hz), 8.22(1H, s), 7.38-7.31(3H, m), 7.35(2H, d, J=9.0Hz), 7.22(1H, d, J=9.0Hz), 7.18(1H, s), 7.07(1H, dd, J=7.6 and 7.6Hz), 6.97(2H, d, J=8.6Hz), 4.91(1H, t, J=5.7Hz), 4.07(2H, t, J=4.6Hz), 3.73(2H, dd, J=9.8 and 4.7Hz), 3.56(4H, brs), 3.07(4H, brs).
MS(ES$^+$) m/z:628 (M + H)$^+$.
Melting point: 112-114˚C.

(Example 241) 4-{4-[3-(2-methylsulfanyl-phenyl)-ureido]-phenyl}-piperazine-l-carboxylic acid [4-(2-hydroxy-ethoxy)-2-trifluoromethyl-phenyl]-amide (Compound No. 1-870)

(241a) 4-{4-[3-(2-methylsulfanyl-phenyl)-ureido]-phenyl}-piperazine-1-carboxylic acid tert-butyl ester

1.04 g (94%) of the title compound was obtained as a white solid from N-tert-butyloxycarbonyl-N'-(4-aminophenyl)-piperazine (693 mg) and 2-(methylthio)phenyl isocyanate (0.44 mL) in the same manner as in Example 1.
$^1$H NMR(400MHz,DMSO-d6):δ(ppm)=9.18(1H, s), 8.03(1H, s), 7.92(1H, d, J=7.1Hz), 7.38(1H, dd, J=7.8 and 1.5Hz), 7.31(2H, d, J=9.0Hz), 7.19(1H, dd, J=7.7 and 7.6Hz), 7.01(1H, dd, J=6.9 and 6.9Hz), 6.89(2H, d, J=9.0Hz), 3.44(4H, t, J=4.9Hz), 3.00(4H, t, J=5.1Hz), 2.41(3H, s), 1.42(9H, s).

(241b) 1-(2-methylsulfanyl-phenyl)-3-(4-piperazin-1-yl-phenyl)-urea

685 mg (quantitative yield) of the title compound was obtained as a white solid from 4-{4-[3-(2-methylsulfanyl-phenyl)-ureido]-phenyl}-piperazine-1-carboxylic acid tert-butyl ester (885 mg) obtained in Example (241a) in the same manner as in Example (47a).
$^1$H NMR(400MHz,DMSO-d6):δ(ppm)=9.15(1H, s), 8.04(1H, s), 7.92(1H, d, J=8.2Hz), 7.38(1H, dd, J=7.7 and 1.3Hz), 7.29(2H, d, J=8.6Hz), 7.19(1H, ddd, J=7.7, 7.7 and 1.3Hz), 7.00(1H, ddd, J=7.5, 7.5 and 1.2Hz), 6.85(2H, d, J=9.0Hz), 3.46(1H, brs), 2.95(4H, brs), 2.82(4H, brs), 2.41(3H, s).

(241c) 4-{4-[3-(2-methylsulfanyl-phenyl)-ureido]-phenyl}-piperazine-1-carboxylic acid [4-(2-hydroxy-ethoxy)-2-trifluoromethyl-phenyl]-amide

427 mg (61 %) of silyl ether was obtained as a white solid from 1-(2-methylsulfanyl-phenyl)-3-(4-piperazin-1-yl-phenyl)-urea (342 mg) obtained in Example (241b) and 4-[2-(tert-butyl-dimethyl-silanyloxy)-ethoxy]-2-trifluoromethyl-phenylamine (335 mg) obtained in Example (209c) in the same manner as in Example 170. This silyl ether was deprotected with tetrabutylammonium fluoride in the same manner as in Example (209d) and 333 mg (93%) of the title compound was obtained as a white solid.
$^1$H NMR(400MHz,DMSO-d6):δ(ppm)=9.21(1H, s), 8.22(1H, s), 8.06(1H, s), 7.96(1H, d, J=8.2Hz), 7.41(1H, d, J=7.8Hz), 7.35(2H, d, J=7.8Hz), 7.32(1H, d, J=9.4Hz), 7.24-7.18(3H, m), 7.03(1H, dd, J=7.6 and 7.6Hz), 6.95(2H, d, J=7.9Hz), 4.90(1H, t, J=5.5Hz), 4.07(2H, t, J=4.3Hz), 3.73(2H, dd, J=9.6 and 4.1Hz), 3.56(4H, brs), 3.07(4H, brs), 2.43(3H, s).
MS(ES$^+$) m/z:590 (M + H)$^+$.
Melting point: 115-116˚C.

(Example 242) 4-{2-cyano-4-[3-(2-methoxy-5-methyl-phenyl)-ureido]-phenyl}-piperazine-1-carboxylic acid [4-(2-hydroxy-ethoxy)-2-trifluoromethyl-phenyl]-amide (Compound No. 2-108)

(242a) 4-(2-cyano-4-nitro-phenyl)-piperazine-1-carboxylic acid tert-butyl ester

1.94 g (99%) of the title compound was obtained as a yellow solid from 2-fluoro-5-nitrobenzonitrile (985 mg) and piperazine-1-carboxylic acid tert-butyl ester (1.10 g) in the same manner as in Example (194a). $^1$H NMR(400MHz, $CDCl_3$):δ(ppm)=8.44(1H, d, J=2.8Hz), 8.28(1H, dd, J=9.0 and 2.7Hz), 6.98(1H, d, J=9.0Hz), 3.67(4H, t, J=5.1Hz), 3.46 (4H, t, J=5.0Hz), 1.49(9H, s).

(242b) 4-(4-amino-2-cyano-phenyl)-piperazine-1-carboxylic acid tert-butyl ester

Zinc powder (7.63 g) was added to a solution of isopropanol (120 mL) and 1N hydrochloric acid (60 mL) of 4-(2-cyano-4-nitro-phenyl)-piperazine-1-carboxylic acid tert-butyl ester (1.94 g) obtained in Example (242a). After stirring at room temperature for five hours, the reaction mixture was neutralized with a saturated sodium hydrogen carbonate aqueous solution and stirred for 30 minutes and filtered through cellulose powder. The filtrate was subjected to extraction with ethyl acetate. The organic layer was washed with saturated brine and concentrated. The residue was purified by column chromatography (hexane:ethyl acetate 5:1 → 1:1). The obtained solid was vigorously stirred in hexane, collected by filtration and dried under reduced pressure, and 1.23 g (70%) of the title compound was obtained as a yellow solid.
$^1$H NMR(400MHz,$CDCl_3$):δ(ppm)=6.88(1H, s), 6.86(1H, d, J=9.3Hz), 6.81(1H, dd, J=8.6 and 2.7Hz), 3.60(4H, t, J=5.1Hz), 3.67(2H, brs), 2.97(4H, t, J=4.9Hz), 1.48(9H, s).

(242c) 4- {2-cyano-4-[3-(2-methoxy-5-methyl-phenyl)-ureido]-phenyl}-piperazine-1-carboxylic acid tert-butyl ester

871 mg (94%) of the title compound was obtained as an orange solid from 4-(4-amino-2-cyano-phenyl)-piperazine-1-carboxylic acid tert-butyl ester (605 mg) obtained in Example (242b) and 2-methoxy-5-methylphenyl isocyanate (0.38 mL) in the same manner as in Example (194c).
$^1$H NMR(400MHz,$CDCl_3$):δ(ppm)=7.84(1H, d, J=1.9Hz), 7.61(1H, d, J=2.7Hz), 7.56(1H, dd, J=8.7 and 2.8Hz), 7.02(1H, s), 6.95(1H, d, J=9.0Hz), 6.83(1H, dd, J=8.0 and 1.8Hz), 6.76(2H, d, J=8.2Hz), 3.83(3H, s), 3.62(4H, t, J=4.9Hz), 3.07 (4H, t, J=4.9Hz), 2.30(3H, s), 1.48(9H, s).

(242d) 1-(3-cyano-4-piperazin-1-yl-phenyl)-3-(2-methoxy-5-methyl-phenyl)-urea

658 mg (96%) of the title compound was obtained as a pale beige solid from 4-{2-cyano-4-[3-(2-methoxy-5-methyl-phenyl)-ureido]-phenyl}-piperazine-1-carboxylic acid tert-butyl ester (870 mg) obtained in Example (242c) in the same manner as in Example (47a).
$^1$H NMR(400MHz,DMSO-d6):δ(ppm)=9.41(1H, s), 8.15(1H, s), 7.94(1H, d, J=2.0Hz), 7.85(1H, d, J=2.4Hz), 7.49(1H, dd, J=9.0 and 2.7Hz), 7.10(1H, d, J=8.7Hz), 6.88(1H, d, J=8.3Hz), 6.74(1H, dd, J=8.2 and 2.0Hz), 3.83(3H, s), 2.96(4H, t, J=4.7Hz), 2.84(4H, t, J=4.7Hz), 2.22(3H, s).

(242e) 4-{2-cyano-4-[3-(2-methoxy-5-methyl-phenyl)-ureido]-phenyl}-piperazine-1-carboxylic acid [4-(2-hydroxy-ethoxy)-2-trifluoromethyl-phenyl]-amide

648 mg (89%) of silyl ether was obtained as a yellow solid from 1-(3-cyano-4-piperazin-1-yl-phenyl)-3-(2-methoxy-5-methyl-phenyl)-urea (365 mg) obtained in Example (242d) and 4-[2-(tert-butyl-dimethyl-silanyloxy)-ethoxy]-2-trifluoromethyl-phenylamine (335 mg) obtained in Example (209c) in the same manner as in Example 170. This silyl ether was deprotected with tetrabutylammonium fluoride in the same manner as in Example (209d) and 453 mg (83%) of the title compound was obtained as a pale yellow solid.
$^1$H NMR(400MHz,DMSO-d6):δ(ppm)=9.44(1H, s), 8.22(1H, s), 8.16(1H, s), 7.95(1H, s), 7.90(1H, s), 7.51(1H, dd, J=9.0 and 2.8Hz), 7.30(1H, d, J=8.2Hz), 7.22-7.16(1H, m), 7.18(2H, d, J=8.6Hz), 6.88(1H, d, J=8.2Hz), 6.74(1H, dd, J=8.6 and 2.3Hz), 4.90(1H, t, J=5.6Hz), 4.06(2H, t, J=4.7Hz), 3.83(3H, s), 3.72(2H, dd, J=10.0 and 5.3Hz), 3.58(4H, t, J=4.5Hz), 3.05(4H, t, J=4.7Hz), 2.23(3H, s).
MS($ES^+$) m/z:613 $(M + H)^+$.
Melting point: 135-137˚C.

(Example 243) 4-{4-[3-(2,3-dihydro-benzofuran-5-yl)-ureido]-phenyl}-piperazine-1-carboxylic acid [4-(2-hydroxy-ethoxy)-2-trifluoromethyl-phenyl]-amide (Compound No. 1-876)

(243a) 4-{4-[3-(2,3-dihydro-benzofuran-5-yl)-ureido]-phenyl}-piperazine-1-carboxylic acid tert-butyl ester

1.68 g (96%) of the title compound was obtained as a pale purple solid from N-tert-butyloxycarbonyl-N'-(4-aminophenyl)-piperazine (1.11 g) and 2,3-dihydro-1-benzofuran-5-yl isocyanate (838 mg) in the same manner as in Example 1.
$^1$H NMR(400MHz,$CDCl_3$):δ(ppm)=7.26-7.21(2H, m), 6.96-6.84(4H, m), 6.71(2H, d, J=8.2Hz), 6.22(1H, brs), 4.57(2H, t, J=8.6Hz), 3.58(4H, brs), 3.19(2H, t, J=8.8Hz), 3.07(4H, brs), 1.48(9H, s).

(243b) 1-(2,3-dihydro-benzofuran-5-yl)-3-(4-piperazin-1-yl-phenyl)-urea

1.29 g (quantitative yield) of the title compound was obtained as a white solid from 4-{4-[3-(2,3-dihydro-benzofuran-5-yl)-ureido]-phenyl}-piperazine-1-carboxylic acid tert-butyl ester (1.68 g) obtained in Example (243a) in the same manner as in Example (47a).
$^1$H NMR(400MHz,DMSO-d6):δ(ppm)=8.45(2H, s), 7.34(1H, s), 7.26(2H, d, J=8.2Hz), 7.04(1H, dd, J=8.6 and 2.3Hz), 6.82(2H, d, J=8.6Hz), 6.63(1H, d, J=8.2Hz), 4.46(2H, t, J=8.8Hz), 3.45(1H, brs), 3.14(2H, t, J=8.6Hz), 2.94(4H, brs), 2.82(4H, brs).

(243c) 4-{4-[3-(2,3-dihydro-benzofuran-5-yl)-ureido]-phenyl}-piperazine-1-carboxylic acid [4-(2-hydroxy-ethoxy)-2-trifluoromethyl-phenyl]-amide

508 mg (73%) of silyl ether was obtained as a pale pink solid from 1-(2,3-dihydro-benzofuran-5-yl)-3-(4-piperazin-1-yl-phenyl)-urea (338 mg) obtained in Example (243b) and 4-[2-(tert-butyl-dimethyl-silanyloxy)-ethoxy]-2-trifluoromethyl-phenylamine (335 mg) obtained in Example (209c) in the same manner as in Example 170. This silyl ether was deprotected with tetrabutylammonium fluoride in the same manner as in Example (209d) and 367 mg (86%) of the title compound was obtained as a white solid.
$^1$H NMR(400MHz,DMSO-d6):δ(ppm)=8.30(1H, s), 8.27(1H, s), 8.19(1H, s), 7.34(1H, s), 7.29(2H, d, J=9.0Hz), 7.29(1H, d, J=9.0Hz), 7.20(1H, dd, J=8.9 and 3.1Hz), 7.15(1H, d, J=3.2Hz), 7.03(1H, dd, J=8.2 and 2.4Hz), 6.90(2H, d, J=9.0Hz), 6.64(1H, d, J=8.2Hz), 4.89(1H, t, J=5.5Hz), 4.47(2H, t, J=8.8Hz), 4.05(2H, t, J=4.9Hz), 3.71(2H, dd, J=9.8 and 5.1Hz), 3.54(4H, t, J=4.5Hz), 3.14(2H, t, J=8.4Hz), 3.04(4H, t, J=4.9Hz)
MS(ES$^+$) m/z:586 (M + H)$^+$.
Melting point: 212-214˚C.

(Example 244) 4-{4-[3-(2,3-dihydro-1,4-benzodioxin-5-yl)-ureido]-phenyl}-piperazine-1-carboxylic acid [4-(2-hydroxy-ethoxy)-2-trifluoromethyl-phenyl]-amide (Compound No. 1-874)

(244a) 4-{4-[3-(2,3-dihydro-1,4-benzodioxin-5-yl)-ureido]-phenyl}-piperazine-1-carboxylic acid tert-butyl ester

9.64 g (75%) of the title compound was obtained as a white solid from 1,4-benzodioxan-5-carboxylic acid (5.0 g) and N-tert-butyloxycarbonyl-N'-(4-aminophenyl)-piperazine (7.78 g) in the same manner as in Example (42b).
$^1$H NMR(400MHz,DMSO-d6):δ(ppm)=9.04(1H, s), 8.04(1H, s), 7.67(1H, d, J=8.2Hz), 7.28(2H, d, J=9.0Hz), 6.88(2H, d, J=9.0Hz), 6.73-6.70(1H, m), 6.47-6.44(1H, m), 4.34(2H, brs), 4.34(2H, brs), 3.45(4H, brs), 3.00(4H, brs), 1.42(9H, s).
MS(ES$^+$) m/z:455 (M + H)$^+$.
Melting point: 187-189˚C.

(244b) 1-(2,3-dihydro-1,4-benzodioxin-5-yl)-3-(4-piperazin-1-yl-phenyl)-urea

8.01 g (quantitative yield) of the title compound was obtained as a white solid from 4-{4-[3-(2,3-dihydro-l,4-benzodioxin-5-yl)-ureido]-phenyl}-piperazine-l-carboxylic acid tert-butyl ester (9.64 g) obtained in Example (244a) in the same manner as in Example (47a).
MS(ES$^+$) m/z:355 (M + H)$^+$.

(244c) 4-{4-[3-(2,3-dihydro-1,4-benzodioxin-5-yl)-ureido]-phenyl}-piperazine-1-carboxylic acid [4-(2-hydroxy-ethoxy)-2-trifluoromethyl-phenyl]-amide

545 mg (76%) of silyl ether was obtained as a white solid from 1-(2,3-dihydro-1,4-benzodioxin-5-yl)-3-(4-piperazin-1-yl-phenyl)-urea (354 mg) obtained in Example (244b) and 4-[2-(tert-butyl-dimethyl-silanyloxy)-ethoxy]-2-trifluoromethyl-phenylamine (335 mg) obtained in Example (209c) in the same manner as in Example 170. This silyl ether was deprotected with tetrabutylammonium fluoride in the same manner as in Example (209d) and 373 mg (81 %) of the title compound was obtained as a white solid.
$^1$H NMR(400MHz,DMSO-d6):δ(ppm)=9.05(1H, s), 8.19(1H, s), 8.05(1H, s), 7.67(1H, d, J=8.2Hz), 7.30(2H, d, J=9.0Hz), 7.29(1H, d, J=9.8Hz), 7.21-7.15(2H, m), 6.92(2H, d, J=9.0Hz), 6.71(1H, dd, J=8.2 and 8.2Hz), 6.46(1H, d, J=7.0Hz), 4.89(1H, t, J=5.4Hz), 4.34(2H, t, J=3.5Hz), 4.26(2H, t, J=3.7Hz), 4.05(2H, t, J=4.9Hz), 3.71(2H, dd, J=10.2 and 5.1Hz), 3.55(4H, t, J=4.7Hz), 3.04(4H, t, J=4.7Hz).
MS(ES$^+$) m/z:602 (M + H)$^+$.
Melting point: 127-129˚C.

(Example 245) 4-{4-[3-(2-difluoromethoxy-phenyl)-ureido]-phenyl}-piperazine-1-carboxylic acid [4-(2-hydroxy-ethoxy)-2-trifluoromethyl-phenyl]-amide (Compound No. 1-873)

(245a) 1-(2-difluoromethoxy-phenyl)-3-(4-piperazin-1-yl-phenyl)-urea

2.44 g (quantitative yield) of the title compound was obtained as a white solid from 4-{4-[3-(2-difluoromethoxy-phenyl)-ureido]-phenyl}-piperazine-1-carboxylic acid tert-butyl ester (3.12 g) obtained in Example 9 in the same manner as in Example (47a).
$^1$H NMR(400MHz,DMSO-d6):δ(ppm)=9.09(1H, s), 8.20(1H, dd, J=8.0 and 1.8Hz), 8.20(1H, s), 7.22(1H, t, J=73.6Hz), 7.28(2H, d, J=8.6Hz), 7.19-7.14(2H, m), 6.98(1H, ddd, J=7.8, 7.8 and 1.6Hz), 6.85(2H, d, J=8.6Hz), 3.45(1H, brs), 2.95 (4H, t, J=4.7Hz), 2.81(4H, t, J=4.5Hz).

(245b) 4-{4-[3-(2-difluoromethoxy-phenyl)-ureido]-phenyl}-piperazine-1-carboxylic acid [4-(2-hydroxy-ethoxy)-2-trifluoromethyl-phenyl]-amide

625 mg (86%) of silyl ether was obtained as a pale beige solid from 1-(2-difluoromethoxy-phenyl)-3-(4-piperazin-1-yl-phenyl)-urea (362 mg) obtained in Example (245a) and 4-[2-(tert-butyl-dimethyl-silanyloxy)-ethoxy]-2-trifluoromethyl-phenylamine (335 mg) obtained in Example (209c) in the same manner as in Example 170. This silyl ether was deprotected with tetrabutylammonium fluoride in the same manner as in Example (209d) and 458 mg (87%) of the title compound was obtained as a white solid.
$^1$H NMR(400MHz,DMSO-d6):δ(ppm)=9.11(1H, s), 8.21(1H, d, J=8.6Hz), 8.19(2H, s), 7.23(1H, t, J=73.8Hz), 7.32(2H, d, J=9.0Hz), 7.29(1H, d, J=10.5Hz), 7.21-7.15(4H, m), 6.98(1H, dd, J=8.0 and 8.0Hz), 6.93(2H, d, J=9.0Hz), 4.89(1H, t, J=5.2Hz), 4.05(2H, t, J=5.1Hz), 3.72(2H, dd, J=9.8 and 5.5Hz), 3.55(4H, t, J=4.3Hz), 3.05(4H, t, J=4.7Hz).
MS(ES$^+$) m/z:610 (M + H)$^+$.
Melting point: 118-120˚C.

(Example 246) 4-{4-[3-(4-ethyl-thiazol-2-yl)-ureido]-phenyl}-piperazine-1-carboxylic acid [4-(2-hydroxy-ethoxy)-2-trifluoromethyl-phenyl]-amide (Compound No. 1-900)

(246a) 4-ethyl-thiazol-2-ylamine

A mixture of 1-bromo-2-butanone (1 g) and thiourea (0.594 g) in methanol (20 mL) was heated under reflux for five hours and concentrated. The residue was made basic with a saturated sodium hydrogen carbonate aqueous solution, followed by extraction with ethyl acetate. The organic layer was washed with saturated brine and dried over sodium sulfate and concentrated and dried under reduced pressure, and 0.66 g (78%) of the title compound was obtained as a yellow oil.
MS(ES$^+$) m/z:129 (M + H)$^+$.

(246b) 4- {4-[3-(4-ethyl-thiazol-2-yl)-ureido]-phenyl}-piperazine-1-carboxylic acid tert-butyl ester

1.75 g (79%) of the title compound was obtained as a beige solid from 4-[4-(4-nitro-phenoxycarbonylamino)-phenyl]-piperazine-1-carboxylic acid tert-butyl ester (2.26 g) obtained in Example (224a) and 4-ethyl-thiazol-2-ylamine (0.66 g) obtained in Example (246a) in the same manner as in Example (224b).
$^1$H NMR(400MHz,DMSO-d6):δ(ppm)=10.4(1H, brs), 8.72(1H, brs), 7.33(2H, d, J=8.2Hz), 6.93(2H, d, J=9.0Hz), 6.63 (1H, brs), 3.54-3.42(4H, m), 3.08-2.98(4H, m), 2.58-2.51(2H, m), 1.42(9H, s), 1.18(3H, t, J=7.4Hz).
MS(ES$^+$) m/z:432 (M + H)$^+$.
Melting point: 190-192˚C.

(246c) 1-(4-ethyl-thiazol-2-yl)-3-(4-piperazin-1-yl-phenyl)-urea

1.70 g (quantitative yield) of the title compound was obtained as a white solid from 4-{4-[3-(4-ethyl-thiazol-2-yl)-ureido]-phenyl}-piperazine-1-carboxylic acid tert-butyl ester (1.70 g) obtained in Example (246b) in the same manner as in Example (47a).
MS(ES$^+$) m/z:129 (M + H)$^+$.

(246d) 4-{4-[3-(4-ethyl-thiazol-2-yl)-ureido]-phenyl}-piperazine-1-carboxylic acid [4-(2-hydroxy-ethoxy)-2-trifluoromethyl-phenyl]-amide

441 mg (64%) of silyl ether was obtained as a yellow solid from 1-(4-ethyl-thiazol-2-yl)-3-(4-piperazin-1-yl-phenyl)-urea (331 mg) obtained in Example (246c) and 4-[2-(tert-butyl-dimethyl-silanyloxy)-ethoxy]-2-trifluoromethyl-phenylamine (335 mg) obtained in Example (209c) in the same manner as in Example 170. This silyl ether was deprotected with tetrabutylammonium fluoride in the same manner as in Example (209d) and 316 mg (86%) of the title compound was obtained as a beige solid.
$^1$H NMR(400MHz,DMSO-d6):δ(ppm)=10.3(1H, s), 8.70(1H, s), 8.20(1H, s), 7.31(1H, d, J=9.8Hz), 7.29(2H, d, J=9.0Hz), 7.21-7.15(2H, m), 6.94(2H, d, J=9.0Hz), 6.61(1H, s), 4.89(1H, t, J=5.3Hz), 4.05(2H, t, J=4.9Hz), 3.71(2H, dd, J=10.0 and 5.3Hz), 3.55(4H, t, J=4.7Hz), 3.07(4H, t, J=4.9Hz), 2.56(2H, q, J=7.2Hz), 1.18(3H, t, J=7.4Hz).
MS(ES$^+$) m/z:579 (M + H)$^+$.
Melting point: 124-125˚C.

(Example 247) 4-{4-[3-(2,3-dimethoxy-phenyl)-ureido]-phenyl}-piperazine-1-carboxylic acid [4-(2-hydroxy-ethoxy)-2-trifluoromethyl-phenyl]-amide (Compound No. 1-881)

(247a) 4-{4-[3-(2,3-dimethoxy-phenyl)-ureido]-phenyl}-piperazine-1-carboxylic acid tert-butyl ester

3.32 g (73%) of the title compound was obtained as a white solid from N-tert-butyloxycarbonyl-N'-(4-aminophenyl)-piperazine (2.77 g) and 2,3-dimethoxy aniline (1.53 g) in the same manner as in Example (41c).
$^1$H NMR(400MHz,$CDCl_3$):δ(ppm)=1.49(9H, s), 3.10(4H, brs), 3.59(4H, t, J=5.1Hz), 3.74(3H, s), 3.84(3H, s), 6.60(1H, brs), 6.59(2H, d, J=8.6Hz), 6.92(2H, brs), 7.01(1H, dd, J=8.4 and 8.4Hz), 7.27-7.23(1H, m), 7.31(1H, s), 7.77(1H, d, J=8.6Hz).

(247b) 1-(2,3-dimethoxy-phenyl)-3-(4-piperazin-1-yl-phenyl)-urea

2.43 g (94%) of the title compound was obtained as a white solid from 4-{4-[3-(2,3-dimethoxy-phenyl)-ureido]-phenyl}-piperazine-1-carboxylic acid tert-butyl ester (3.32 g) obtained in Example (247a) in the same manner as in Example (47a).
$^1$H NMR(400MHz,DMSO-d6):δ(ppm)=9.15(1H, s), 8.57(1H, brs), 8.26(1H, s), 7.78(1H, d, J=7.4Hz), 7.33(2H, d, J=8.6Hz), 6.94(1H, dd, J=8.4 and 8.4Hz), 6.93(2H, d, J=8.6Hz), 6.64(1H, d, J=7.5Hz), 3.79(3H, s), 3.74(3H, s), 3.33(1H, brs), 3.22 (8H, brs).

(247c) 4-{4-[3-(2,3-dimethoxy-phenyl)-ureido]-phenyl}-piperazine-1-carboxylic acid [4-(2-hydroxy-ethoxy)-2-trifluoromethyl-phenyl]-amide

502 mg (70%) of silyl ether was obtained as a pale yellow solid from 1-(2,3-dimethoxy-phenyl)-3-(4-piperazin-1-yl-phenyl)-urea (356 mg) obtained in Example (247b) and 4-[2-(tert-butyl-dimethyl-silanyloxy)-ethoxy]-2-trifluoromethyl-phenylamine (335 mg) obtained in Example (209c) in the same manner as in Example 170. This silyl ether was deprotected with tetrabutylammonium fluoride in the same manner as in Example (209d) and 335 mg (80%) of the title compound was obtained as a white solid.
$^1$H NMR(400MHz,DMSO-d6):δ(ppm)=9.12(1H, s), 8.27(1H, s), 8.23(1H, s), 7.82(1H, d, J=7.4Hz), 7.34(2H, d, J=9.0Hz), 7.32(1H, d, J=9.4Hz), 7.22(1H, dd, J=8.8 and 2.6Hz), 7.18(1H, d, J=2.7Hz), 6.97(1H, dd, J=8.2 and 8.2Hz), 6.95(2H, d, J=9.0Hz), 6.66(1H, dd, J=8.4 and 1.4Hz), 4.91(1H, t, J=5.5Hz), 4.07(2H, t, J=4.9Hz), 3.81(3H, s), 3.76(3H, s), 3.73 (2H, dd, J=10.0 and 5.3Hz), 3.56(4H, t, J=4.7Hz), 3.06(4H, t, J=4.5Hz).
MS(ES$^+$) m/z:604 (M + H)$^+$.
Melting point: 135-138˚C.

(Example 248) 4-{4-[3-(2-methoxy-5-methyl-phenyl)-ureido]-phenyl}-piperazine-1-carboxylic acid [2-chloro-4-(2-hydroxy-ethoxy)-phenyl]-amide (Compound No. 1-555)

(248a) 2-chloro-4-hydroxy-benzoic acid methyl ester

A solution of 2-chloro-4-hydroxybenzoic acid (4.20 g) and concentrated sulfuric acid (2.5 mL) in methanol (50 mL) was heated under reflux for three days. The reaction mixture was neutralized with a saturated sodium hydrogen carbonate aqueous solution, followed by extraction with ethyl acetate. The organic layer was washed with saturated brine and dried over sodium sulfate and concentrated and dried under reduced pressure, and 4.18 g (92%) of the title

compound was obtained as a brown solid.

$^1$H NMR(400MHz,$CDCl_3$):δ(ppm)=7.83(1H, d, J=8.6Hz), 6.94(1H, d, J=2.7Hz), 6.76(1H, dd, J=8.6 and 2.4Hz), 5.63(1H, s), 3.89(3H, s).

(248b) 2-chloro-4-(2-hydroxy-ethoxy)-benzoic acid methyl ester

5.16 g (quantitative yield) of the title compound was obtained as a brown oil from 2-chloro-4-hydroxy-benzoic acid methyl ester (4.18 g) obtained in Example (248a) and 2-bromoethanol (5.2 mL) in the same manner as in Example (208a).
$^1$H NMR(400MHz,$CDCl_3$):δ(ppm)=7.90(1H, dd, J=9.0 and 2.0Hz), 7.01(1H, dd, J=2.1 and 2.1Hz), 6.86(1H, ddd, J=5.4, 5.4 and 2.9Hz), 4.54(1H, s), 4.13(2H, t, J=5.5Hz), 4.00(2H, dd, J=9.2 and 4.2Hz), 3.91(3H, s).

(248c) 4-[2-(tert-butyl-dimethyl-silanyloxy)-ethoxy]-2-chloro-benzoic acid methyl ester

7.38 g (96%) of the title compound was obtained as a yellow oil from 2-chloro-4-(2-hydroxy-ethoxy)-benzoic acid methyl ester (5.16 g) obtained in Example (248b) in the same manner as in Example (208b).
$^1$H NMR(400MHz,$CDCl_3$):δ(ppm)=7.76(1H, d, J=8.6Hz), 6.89(1H, d, J=2.8Hz), 6.73(1H, dd, J=8.8 and 2.6Hz), 3.98(2H, t, J=4.9Hz), 3.87(2H, t, J=4.9Hz), 3.80(3H, s), 0.81 (9H, s), 0.01(6H, s).

(248d) 4-[2-(tert-butyl-dimethyl-silanyloxy)-ethoxy]-2-chloro-benzoic acid

Potassium trimethylsilylsilanolate (3.57 g) was added to a solution of 4-[2-(tert-butyl-dimethyl-silanyloxy)-ethoxy]-2-chloro-benzoic acid methyl ester (7.38 g) obtained in Example (248c) in anhydrous tetrahydrofuran (80 mL) at room temperature. The reaction mixture was stirred at room temperature for 18 hours and was neutralized with a citric acid aqueous solution, followed by extraction with ethyl acetate. The organic layer was washed with water and saturated brine and dried over sodium sulfate and concentrated. The obtained solid was vigorously stirred in hexane and was collected by filtration and dried under reduced pressure, and 3.98 g (56%) of the title compound was obtained as a white solid.
$^1$H NMR(400MHz,$CDCl_3$):δ(ppm)=7.92(1H, d, J=9.0Hz), 6.91 (1H, d, J=2.8Hz), 6.75(1H, dd, J=8.8 and 2.5Hz), 4.00 (2H, t, J=4.9Hz), 3.88(2H, t, J=4.9Hz), 0.81(9H, s), 0.00(6H, s).

(248e) 4-{4-[3-(2-methoxy-5-methyl-phenyl)-ureido]-phenyl}-piperazine-1-carboxylic acid [2-chloro-4-(2-hydroxy-ethoxy)-phenyl]-amide

Diphenylphosphoric acid azide (1.3 mL) and triethylamine (1.0 mL) were added to 4-[2-(tert-butyl-dimethyl-silanyloxy)-ethoxy]-2-chloro-benzoic acid (1.72 g) obtained in Example (248d) in anhydrous tetrahydrofuran (30 mL) at room temperature. The reaction mixture was heated under reflux for four hours, followed by addition of 1-(2-methoxy-5-methyl-phenyl)-3-(4-piperazin-1-yl-phenyl)-urea (1.70 g) obtained in Example (47a). The reaction mixture was heated for a further five hours and concentrated. The residue was diluted with ethyl acetate and washed with a saturated sodium hydrogen carbonate aqueous solution and saturated brine and concentrated. The residue was purified by column chromatography (dichloromethane:ethyl acetate 5:1 → 2:1) to obtain 2.40 g (72%) of silyl ether as a yellow solid. This yellow solid was dissolved in anhydrous tetrahydrofuran (40 mL), followed by addition of tetrabutylammonium fluoride (7.2 mL) at room temperature. The reaction mixture was stirred at room temperature for 18 hours and concentrated. The residue was purified by column chromatography (dichloromethane:methanol 30:1 → 10:1). The obtained solid was recrystallized (from ethanol) and 1.48 g (74%) of the title compound was obtained as a white solid. $^1$H NMR(400MHz,DMSO-d6):δ (ppm)=9.05(1H, s), 8.19(1H, s), 8.03(1H, s), 7.96(1H, d, J=2.0Hz), 7.31(2H, d, J=9.0Hz), 7.28(1H, d, J=9.0Hz), 7.03 (1H, d, J=2.7Hz), 6.92(2H, d, J=9.0Hz), 6.87(1H, dd, J=9.0 and 2.7Hz), 6.86(1H, d, J=8.2Hz), 6.70(1H, dd, J=8.2 and 1.6Hz), 4.86(1H, t, J=5.5Hz), 3.98(2H, dd, J=4.9 and 4.9Hz), 3.82(3H, s), 3.69(2H, dd, J=10.0 and 5.3Hz), 3.57(4H, t, J=4.9Hz), 3.06(4H, t, J=4.7Hz), 2.22(3H, s).
MS($ES^+$) m/z:554 $(M + H)^+$.
Melting point: 152-154˚C.

(Example 249) 4-{4-[3-(3-methoxy-phenyl)-ureido]-phenyl}-piperazine-1-carboxylic acid [2-chloro-4-(2-hydroxy-ethoxy)-phenyl]-amide (Compound No. 1-852)

427 mg (62%) of silyl ether was obtained as a white solid from 1-(3-methoxy-phenyl)-3-(4-piperazin-1-yl-phenyl)-urea (356 mg) obtained in Example (214b) and 4-[2-(tert-butyl-dimethyl-silanyloxy)-ethoxy]-2-chloro-benzoic acid

(330 mg) obtained in Example (248d) in the same manner as in Example (248e). This silyl ether was deprotected with tetrabutylammonium fluoride in the same manner as in Example (248e) and 219 mg (76%) of the title compound was obtained as a white solid.
$^1$H NMR(400MHz,DMSO-d6):δ(ppm)=8.58(1H, s), 8.41(1H, s), 8.22(1H, s), 7.32(2H, d, J=7.9Hz), 7.30(1H, d, J=10.1Hz), 7.19(1H, s), 7.16(1H, dd, J=8.0 and 1.4Hz), 7.06(1H, dd, J=2.0 and 2.0Hz), 6.94(2H, d, J=9.3Hz), 6.93-6.88(2H, m), 6.54(1H, d, J=8.6Hz), 4.89(1H, t, J=5.3Hz), 4.00(2H, dd, J=4.5 and 4.5Hz), 3.73(3H, s), 3.71(2H, q, J=6.0Hz), 3.58(4H, t, J=3.9Hz), 3.08(4H, brs).
MS(ES$^+$) m/z:541 (M + H)$^+$.
Melting point: 198-201˚C.

(Example 250) 4-{4-[3-(3,5-dimethoxy-phenyl)-ureido]-phenyl}-piperazine-1-carboxylic acid [2-chloro-4-(2-hydroxy-ethoxy)-phenyl]-amide (Compound No. 1-557)

427 mg (62%) of silyl ether was obtained as a white solid from 1-(3,5-dimethoxy-phenyl)-3-(4-piperazin-1-yl-phenyl)-urea (356 mg) obtained in Example (216b) and 4-[2-(tert-butyl-dimethyl-silanyloxy)-ethoxy]-2-chloro-benzoic acid (330 mg) obtained in Example (248d) in the same manner as in Example (248e). This silyl ether was deprotected with tetrabutylammonium fluoride in the same manner as in Example (248e) and 286 mg (81%) of the title compound was obtained as a white solid.
$^1$H NMR(400MHz,DMSO-d6):δ(ppm)=8.57(1H, s), 8.38(1H, s), 8.21(1H, s), 7.31(2H, d, J=7.9Hz), 7.30(1H, d, J=6.6Hz), 7.05(1H, d, J=1.6Hz), 6.94(2H, d, J=8.2Hz), 6.89(1H, dd, J=9.3 and 2.0Hz), 6.67(2H, s), 6.12(1H, d, J=1.6Hz), 4.88(1H, t, J=5.3Hz), 3.99(2H, dd, J=4.5 and 4.5Hz), 3.71(6H, s), 3.71-3.68(2H, m), 3.58(4H, brs), 3.07(4H, t, J=3.7Hz).
MS(ES$^+$) m/z:571 (M + H)$^+$.
Melting point: 114-116˚C.

(Example 251)4-{4-[3-(2-methoxy-phenyl)-ureido]-phenyl}-piperazine-1-carboxylic acid [2-chloro-4-(2-hydroxy-ethoxy)-phenyl]-amide (Compound No. 1-549)

516 mg (66%) of silyl ether was obtained as a brown solid from 1-(2-methoxy-phenyl)-3-(4-piperazin-1-yl-phenyl)-urea (392 mg) obtained in Example (124a) and 4-[2-(tert-butyl-dimethyl-silanyloxy)-ethoxy]-2-chloro-benzoic acid (400 mg) obtained in Example (248d) in the same manner as in Example (248e). This silyl ether was deprotected with tetrabutylammonium fluoride in the same manner as in Example (248e) and 376 mg (88%) of the title compound was obtained as a white solid.
$^1$H NMR(400MHz,DMSO-d6):δ(ppm)=9.09(1H, s), 8.22(1H, s), 8.13(1H, d, J=7.8Hz), 8.12(1H, s), 7.33(2H, d, J=7.5Hz), 7.30(1H, d, J=9.7Hz), 7.06(1H, s), 7.02(1H, d, J=7.4Hz), 6.95(2H, d, J=7.4Hz), 6.95(1H, d, J=7.4Hz), 6.90(2H, d, J=8.6Hz), 4.88(1H, t, J=5.7Hz), 4.00(2H, d, J=4.7Hz), 3.88(3H, s), 3.70(2H, dd, J=10.1 and 5.1Hz), 3.58(4H, t, J=4.5Hz), 3.08(4H, t, J=4.9Hz).
MS(ES$^+$) m/z:541 (M + H)$^+$.
Melting point: 134-136˚C.

(Example 252) 4-{4-[3-(2-methoxy-5-methyl-phenyl)-ureido]-phenyl}-2-methylpiperazine-1-carboxylic acid [2-chloro-4-(2-hydroxy-ethoxy)-phenyl]-amide (Compound No. 2-99)

202 mg (30%) of silyl ether was obtained as a pale yellow solid from 1-(2-methoxy-5-methyl-phenyl)-3-[4-(3-methyl-piperazin-1-yl)-phenyl]-urea (354 mg) obtained in Example (198e) and 4-[2-(tert-butyl-dimethyl-silanyloxy)-ethoxy]-2-chloro-benzoic acid (330 mg) obtained in Example (248d) in the same manner as in Example (248e). This silyl ether was deprotected with tetrabutylammonium fluoride in the same manner as in Example (248e) and 156 mg (93%) of the title compound was obtained as a white solid.
$^1$H NMR(400MHz,DMSO-d6):δ(ppm)=9.07(1H, s), 8.12(1H, s), 8.04(1H, s), 7.96(1H, d, J=2.0Hz), 7.31(2H, d, J=9.0Hz), 7.27(1H, d, J=9.0Hz), 7.04(1H, d, J=2.7Hz), 6.91(2H, d, J=8.6Hz), 6.87(1H, dd, J=8.8 and 3.0Hz), 6.86(1H, d, J=8.2Hz), 6.70(1H, dd, J=8.4 and 1.8Hz), 4.87(1H, t, J=4.9Hz), 4.39-4.33(1H, m), 3.98(2H, t, J=4.9Hz), 3.95-3.90(1H, m), 3.82 (3H, s), 3.69(2H, dd, J=8.6 and 3.9Hz), 3.54-3.50(1H, m), 3.45-3.41(1H, m), 3.22-3.16(1H, m), 2.79-2.75(1H, m), 2.61-2.55(1H, m), 2.22(3H, s), 1.30(3H, d, J=6.6Hz).
MS(ES$^+$) m/z:569 (M + H)$^+$.
Melting point: 129-131˚C.

(Example 253) 4- {2-fluoro-4-[3-(2-methoxy-5-methyl-phenyl)-ureido]-phenyl}-piperazine-1-carboxylic acid [2-chloro-4-(2-hydroxy-ethoxy)-phenyl]-amide (Compound No. 2-95)

(253a) 4-(2-fluoro-4-nitro-phenyl)-piperazine-1-carboxylic acid tert-butyl ester

4.85 g (74%) of the title compound was obtained as a yellow solid from 3,4-difluoronitrobenzene (3.18 g) and piperazine-1-carboxylic acid tert-butyl ester (4.46 g) in the same manner as in Example (38a).
MS($ES^+$) m/z:326 $(M + H)^+$.

(253b) 4-(4-amino-2-fluoro-phenyl)-piperazine-1-carboxylic acid tert-butyl ester

4.29 g (98%) of the title compound was obtained as a white solid from 4-(2-fluoro-4-nitro-phenyl)-piperazine-1-carboxylic acid tert-butyl ester (4.84 g) obtained in Example (253a) in the same manner as in Example (194b).
$^1$H NMR(400MHz,$CDCl_3$):δ(ppm)=6.78(1H, dd, J=9.0 and 9.0Hz), 6.45-6.38(2H, m), 3.56(4H, t, J=4.9Hz), 2.90(4H, t, J=4.9Hz), 1.47(9H, s).

(253c) 4-{2-fluoro-4-[3-(2-methoxy-5-methyl-phenyl)-ureido]-phenyl}-piperazine-1-carboxylic acid tert-butyl ester

2.73 g (99%) of the title compound was obtained as a pale pink solid from 4-(4-amino-2-fluoro-phenyl)-piperazine-1-carboxylic acid tert-butyl ester (1.77 g) obtained in Example (253b) and 2-methoxy-5-methylphenyl isocyanate (1.2 mL) in the same manner as in Example 1.
$^1$H NMR(400MHz,$CDCl_3$):δ(ppm)=7.90(1H, d, J=1.9Hz), 7.28(1H, d, J=2.4Hz), 7.07(1H, s), 7.00(1H, dd, J=8.2 and 2.0Hz), 6.87(1H, dd, J=8.8 and 8.8Hz), 6.82(1H, dd, J=8.4 and 1.3Hz), 6.76(1H, d, J=8.2Hz), 6.62(1H, s), 3.82(3H, s), 3.60(4H, t, J=5.0Hz), 2.99(4H, t, J=4.9Hz), 2.31(3H, s), 1.50(9H, s).

(253d) 1-(3-fluoro-4-piperazin-1-yl-phenyl)-3-(2-methoxy-5-methyl-phenyl)-urea

2.13 g (quantitative yield) of the title compound was obtained as a white solid from 4-{2-fluoro-4-[3-(2-methoxy-5-methyl-phenyl)-ureido]-phenyl}-piperazine-1-carboxylic acid tert-butyl ester (2.73 g) obtained in Example (253c) in the same manner as in Example (47a).
$^1$H NMR(400MHz,DMSO-$d_6$):δ(ppm)=9.29(1H, s), 8.10(1H, s), 7.94(1H, d, J=2.0Hz), 7.42(1H, d, J=14.9Hz), 6.99-6.90 (2H, m), 6.87(1H, d, J=8.2Hz), 6.72(1H, dd, J=8.4 and 2.1Hz), 3.82(3H, s), 3.46(1H, brs), 2.83(8H, brs), 2.22(3H, s).

(253e) 4-{2-fluoro-4-[3-(2-methoxy-5-methyl-phenyl)-ureido]-phenyl}-piperazine-1-carboxylic acid [2-chloro-4-(2-hydroxy-ethoxy)-phenyl]-amide

462 mg (67%) of silyl ether was obtained as a light brown solid from 1-(3-fluoro-4-piperazin-1-yl-phenyl)-3-(2-methoxy-5-methyl-phenyl)-urea (462 mg) obtained in Example (253d) and 4-[2-(tert-butyl-dimethyl-silanyloxy)-ethoxy]-2-chloro-benzoic acid (330 mg) obtained in Example (248d) in the same manner as in Example (248e). This silyl ether was deprotected with tetrabutylammonium fluoride in the same manner as in Example (248e) and 365 mg (95%) of the title compound was obtained as a white solid.
$^1$H NMR(400MHz,DMSO-d6):δ(ppm)=9.36(1H, s), 8.22(1H, s), 8.15(1H, s), 7.99(1H, d, J=1.9Hz), 7.53(1H, d, J=2.4Hz), 7.32(1H, d, J=9.0Hz), 7.08(1H, d, J=2.7Hz), 7.04(1H, dd, J=4.9 and 2.1Hz), 7.03(2H, d, J=1.5Hz), 6.91(2H, d, J=8.6Hz), 6.77(1H, dd, J=8.2 and 1.6Hz), 4.91(1H, t, J=5.5Hz), 4.03(2H, dd, J=4.9 and 4.9Hz), 3.87(3H, s), 3.73(2H, dd, J=10.0 and 5.2Hz), 3.62(4H, t, J=4.5Hz), 2.99(4H, t, J=4.7Hz), 2.27(3H, s).
MS($ES^+$) m/z:573 $(M + H)^+$.
Melting point: 118-120˚C.

(Example 254) 4-{4-[3-(2-ethoxy-phenyl)-ureido]-phenyl}-piperazine-1-carboxylic acid [2-chloro-4-(2-hydroxy-ethoxy)-phenyl]-amide (Compound No. 1-866)

219 mg (40%) of the title compound was obtained as a light brown solid from 1-(2-ethoxy-phenyl)-3-(4-piperazin-1-yl-phenyl)-urea (340 mg) obtained in Example (80a) and 4-[2-(tert-butyl-dimethyl-silanyloxy)-ethoxy]-2-chloro-benzoic acid (330 mg) obtained in Example (248d) in the same manner as in Example (248e).
$^1$H NMR(400MHz,DMSO-d6):δ(ppm)=8.54(1H, s), 8.40(1H, s), 8.19(1H, s), 7.30(2H, d, J=9.4Hz), 7.27(1H, d, J=9.0Hz), 7.23(1H, dd, J=7.8 and 7.8Hz), 7.15(1H, dd, J=2.3 and 2.3Hz), 7.12(1H, dd, J=8.2 and 8.2Hz), 7.03(1H, d, J=3.1Hz), 6.92(2H, d, J=9.0Hz), 6.87(1H, dd, J=8.8 and 2.6Hz), 6.49(1H, dd, J=8.8 and 2.2Hz), 4.87(1H, t, J=5.6Hz), 4.00-3.95 (4H, m), 3.69(2H, dd, J=10.1 and 5.5Hz), 3.57(4H, t, J=4.7Hz), 3.06(4H, t, J=4.7Hz), 1.32(3H, t, J=6.9Hz).

MS($ES^+$) m/z:555 $(M + H)^+$.
Melting point: 112-114˚C.

(Example 255) 4-{4-[3-(2-fluoro-phenyl)-ureido]-phenyl}-piperazine-1-carboxylic acid [2-chloro-4-(2-hydroxy-ethoxy)-phenyl]-amide (Compound No. 1-548)

331 mg (52%) of silyl ether was obtained as a white solid from 1-(2-fluorophenyl)-3-(4-piperazin-1-yl-phenyl)-urea (314 mg) obtained in Example (125a) and 4-[2-(tert-butyl-dimethyl-silanyloxy)-ethoxy]-2-chloro-benzoic acid (330 mg) obtained in Example (248d) in the same manner as in Example (248e). This silyl ether was deprotected with tetrabutylammonium fluoride in the same manner as in Example (248e) and 257 mg (94%) of the title compound was obtained as a white solid.
$^1$H NMR(400MHz,DMSO-d6):δ(ppm)=8.85(1H, s), 8.45(1H, d, J=2.8Hz), 8.23(1H, s), 8.17(1H, ddd, J=8.3, 8.3 and 1.7Hz), 7.33(2H, d, J=9.0Hz), 7.30(1H, d, J=9.0Hz), 7.24(1H, ddd, J=11.7 ,8.2 and 1.5Hz), 7.13(1H, t, J=7.2Hz), 7.06 (1H, d, J=2.8Hz), 7.02-6.97(1H, m), 6.96(2H, d, J=9.0Hz), 6.89(1H, dd, J=8.8 and 3.0Hz), 4.89(1H, t, J=5.5Hz), 4.00 (2H, t, J=4.9Hz), 3.70(2H, dd, J=10.0 and 5.2Hz), 3.58(4H, t, J=4.7Hz), 3.08(4H, t, J=4.9Hz).
MS($ES^+$) m/z:528 $(M + H)^+$.
Melting point: 213-217˚C.

(Example 256) 4- {4-[3-(2-methoxy-5-methyl-phenyl)-ureido]-phenyl}-3,6-dihydro-2H-pyridine-1-carboxylic acid [2-chloro-4-(2-hydroxy-ethoxy)-phenyl]-amide (Compound No. 5-24)

90 mg (61%) of silyl ether was obtained from 1-(2-methoxy-5-methylphenyl)-3-[4-(1,2,3,6-tetrahydro-pyridin-4-yl)-phenyl]-urea (74 mg) obtained in Example (230b) and 4-[2-(tert-butyl-dimethyl-silanyloxy)-ethoxy]-2-chloro-benzoic acid (73 mg) obtained in Example (248d) in the same manner as in Example (248e). This silyl ether was deprotected with tetrabutylammonium fluoride in the same manner as in Example (248e) and 14 mg (19%) of the title compound was obtained as a red white solid.
$^1$H NMR(400MHz,DMSO-d6):δ(ppm)=2.23(3H, s), 2.58-2.41(2H, m), 3.64(2H, t, J=5.5Hz), 3.69(2H, t, J=4.9Hz), 3.83 (3H, s), 3.98(2H, t, J=4.7Hz), 4.12(2H, s), 6.13(1H, s), 6.73(1H, d, J=8.6Hz), 6.83-6.93 (2H, m), 7.03(1H, d, J=2.7Hz), 7.30(1H, d, J=9.0Hz), 7.38(2H, d, J=9.3Hz), 7.43(2H, d, J=8.6Hz), 7.97(1H, s), 8.10(1H, s), 8.15(1H, s), 9.33(1H, s).
MS(APCI) m/z:551, 553 $(M + H)^+$.
Melting point: 117-120˚C.

(Example 257) 4-{4-[3-(2-methoxy-5-methyl-phenyl)-ureido]-phenyl}-piperidine-1-carboxylic acid [2-chloro-4-(2-hydroxy-ethoxy)-phenyl]-amide (Compound No. 4-24)

48 mg (36%) of silyl ether was obtained from 1-(2-methoxy-5-methylphenyl)-3-(4-piperidin-4-yl-phenyl)-urea (68 mg) obtained in Example (228e) and 4-[2-(tert-butyl-dimethyl-silanyloxy)-ethoxy]-2-chloro-benzoic acid (66 mg) obtained in Example (248d) in the same manner as in Example (248e). This silyl ether was deprotected with tetrabutylammonium fluoride in the same manner as in Example (248e) and 40 mg (quantitative yield) of the title compound was obtained as a white solid.
$^1$H NMR(400MHz,DMSO-d6):δ(ppm)=1.61-1.47(2H, m), 1.77(2H, d, J=12.9Hz), 2.22(3H, s), 2.73-2.63(1H, m), 2.87(2H, t, J=13.3Hz), 3.69(2H, dd, J=10.0 and 5.3Hz), 3.83(3H, s), 3.98(2H, t, J=4.7Hz), 4.19(2H, d, J=11.3Hz), 4.87(1H, t, J=5.3Hz), 6.72(1H, dd, J=8.1 and 1 .7Hz), 6.85(1H, s), 6.87(1H, s), 7.03(1H, d, J=3.1Hz), 7.14(2H, d, J=8.6Hz), 7.28 (1H, d, J=9.0Hz), 7.37(2H, d, J=8.6Hz), 7.96(1H, d, J=1.9Hz), 8.08(1H, s), 8.11(1H, s), 9.21(1H, s).
MS(APCI) m/z:553, 555 $(M + H)^+$.
Melting point: 134-137˚C.

(Example 258) 4-{4-[3-(2-methoxy-phenyl)-ureido]-phenyl}-3,6-dihydro-2H-pyridine-1-carboxylic acid [2-chloro-4-(2-hydroxy-ethoxy)-phenyl]-amide (Compound No. 5-22)

(258a) 4-{4-[3-(2-methoxy-phenyl)-ureido]-phenyl}-3,6-dihydro-2H-pyridine-1-carboxylic acid tert-butyl ester

785 mg (quantative yield) of the title compound was obtained as a reddish purple solid from 4-(4-aminophenyl)-3,6-dihydro-2H-pyridine-1-carboxylic acid tert-butyl ester (500 mg) obtained in Example (229a) and 2-methoxyphenyl isocyanate (258 μl) in the same manner as in Example 1.
MS(APCI) m/z:424 $(M + H)^+$.

(258b) 1-(2-methoxy-phenyl)-3-[4-(1,2,3,6-tetrahydro-pyridin-4-yl)-phenyl]-urea

674 mg (quantitative yield) of the title compound was obtained as a yellow brown solid from 4-{4-[3-(2-methoxy-phenyl)-ureido]-phenyl}-3,6-dihydro-2H-pyridine-1-carboxylic acid tert-butyl ester (785 mg) obtained in Example (258a) in the same manner as in Example (47a).
MS(APCI) m/z:324 $(M + H)^+$.

(258c) 4- (4-[3-(2-methoxy-phenyl)-ureido]-phenyl) -3,6-dihydro-2H-pyridine-1 - carboxylic acid [2-chloro-4-(2-hydroxy-ethoxy)-phenyl]-amide

69 mg (53%) of silyl ether was obtained from 1-(2-methoxy-phenyl)-3-[4-(1,2,3,6-tetrahydro-pyridin-4-yl)-phenyl]-urea (65 mg) obtained in Example (258b) and 4-[2-(tert-butyl-dimethyl-silanyloxy)-ethoxy]-2-chloro-benzoic acid (66 mg) obtained in Example (248d) in the same manner as in Example (248e). This silyl ether was deprotected with tetrabutylammonium fluoride in the same manner as in Example (248e) and 39 mg (57%) of the title compound was obtained as a white solid.
$^1$H NMR(400MHz,DMSO-d6):δ(ppm)=2.41-2.58(2H, m), 3.64(2H, t, J=5.5Hz), 3.69(2H, dd, J=9.6 and 4.5Hz), 3.87(3H, s), 3.98(2H, t, J=4.9Hz), 4.11(2H, d, J=2.0Hz), 4.87(1H, t, J=5.5Hz), 6.13(1H, brs), 6.91-6.85(2H, m), 6.96-6.91(1H, m), 7.01(1H, dd, J=8.0 and 1.4Hz), 7.03(1H, d, J=2.7Hz), 7.30(1H, d, J=9.0Hz), 7.39(2H, d, J=9.0Hz), 7.43(2H, d, J=9.0Hz), 8.10(1H, s), 8.14-8.08(1H, m), 8.22(1H, s), 9.36(1H, s).
MS(APCI) m/z:537, 539 $(M + H)^+$.
Melting point: 120-125˚C.

(Example 259) 4-{4-[3-(3,5-dimethoxy-phenyl)-ureido]-phenyl}-3,6-dihydro-2H-pyridine-1-carboxylic acid [2-chloro-4-(2-hydroxy-ethoxy)-phenyl]-amide (Compound No. 5-25)

(259a) 4-{4-[3-(3,5-dimethoxy-phenyl)-ureido]-phenyl}-3,6-dihydro-2H-pyridine-1-carboxylic acid tert-butyl ester

820 mg (99%) of the title compound was obtained as a beige solid from 4-(4-aminophenyl)-3,6-dihydro-2H-pyridine-1-carboxylic acid tert-butyl ester (500 mg) obtained in Example (229a) and 3,5-dimethoxyphenyl isocyanate (375 mg) in the same manner as in Example 1.
MS(APCI) m/z:454 $(M + H)^+$.

(259b) 1-(3,5-dimethoxy-phenyl)-3-[4-(1,2,3,6-tetrahydro-pyridin-4-yl)-phenyl]-urea

791 mg (quantitative yield) of the title compound was obtained as a yellow brown solid from 4-{4-[3-(3,5-dimethoxy-phenyl)-ureido]-phenyl}-3,6-dihydro-2H-pyridine-1-carboxylic acid tert-butyl ester (820 mg) obtained in Example (259b) in the same manner as in Example (47a).
MS(APCI) m/z:354 $(M + H)^+$.

(259c) 4-{4-[3-(3,5-dimethoxy-phenyl)-ureido]-phenyl}-3,6-dihydro-2H-pyridine-1-carboxylic acid [2-chloro-4-(2-hydroxy-ethoxy)-phenyl]-amide

43 mg (32%) of silyl ether was obtained from 1-(3,5-dimethoxy-phenyl)-3-[4-(1,2,3,6-tetrahydro-pyridin-4-yl)-phenyl]-urea (71 mg) obtained in Example (259b) and 4-[2-(tert-butyl-dimethyl-silanyloxy)-ethoxy]-2-chloro-benzoic acid (66 mg) obtained in Example (248d) in the same manner as in Example (248e). This silyl ether was deprotected with tetrabutylammonium fluoride in the same manner as in Example (248e) and 30 mg (84%) of the title compound was obtained as a white solid.
$^1$H NMR(400MHz, DMSO-d6):δ(ppm)= 2.41-2.57 (2H, m), 3.71(6H, s), 3.73-3.62(4H, m), 3.98(2H, t, J=4.9Hz), 4.11(2H, brs), 4.87(1H, t, J=5.4Hz), 6.15-6.11(2H, m), 6.67(2H, d, J=2.3Hz), 6.87(1H, dd, J=8.6 and 2.8Hz), 7.03(1H, d, J=2.7Hz), 7.30(1H, d, J=9.0Hz), 7.38(2H, d, J=9.0Hz), 7.42(2H, d, J=8.6Hz), 8.10(1H, s), 8.74(1H, s), 8.75(1H, s).
MS(APCI) m/z:567, 569 $(M + H)^+$.
Melting point: 135-139˚C.

(Example 260) 4-{4-[3-(2-fluoro-phenyl)-ureido]-phenyl}-3,6-dihydro-2H-pyridine-1-carboxylic acid [2-chloro-4-(2-hydroxy-ethoxy)-phenyl]-amide (Compound No. 5-21)

(260a) 4-{4-[3-(2-fluoro-phenyl)-ureido]-phenyl}-3,6-dihydro-2H-pyridine-1-carboxylic acid tert-butyl ester

599 mg (quantitative yield) of the title compound was obtained as a yellow brown solid from 4-(4-aminophenyl)-3,6-dihydro-2H-pyridine-1-carboxylic acid tert-butyl ester (400 mg) obtained in Example (229a) and 2-fluorophenyl isocyanate (188 μl) in the same manner as in Example 1.
MS(APCI) m/z:412 $(M + H)^+$.

(260b) 1-(2-fluoro-phenyl)-3-[4-(1,2,3,6-tetrahydro-pyridin-4-yl)-phenyl]-urea

407 mg (90%) of the title compound was obtained as a yellow brown solid from 4-4-{4-[3-(2-fluoro-phenyl)-ureido]-phenyl}-3,6-dihydro-2H-pyridine-1-carboxylic acid tert-butyl ester (599 mg) obtained in Example (260b) in the same manner as in Example (47a).
MS(APCI) m/z:312 $(M + H)^+$.

(260c) 4-{4-[3-(2-fluoro-phenyl)-ureido]-phenyl}-3,6-dihydro-2H-pyridine-1-carboxylic acid [2-chloro-4-(2-hydroxy-ethoxy)-phenyl]-amide

Silyl ether was obtained from 1-(2-fluoro-phenyl)-3-[4-(1,2,3,6-tetrahydro-pyridin-4-yl)-phenyl]-urea (62 mg) obtained in Example (260b) and 4-[2-(tert-butyldimethyl-silanyloxy)-ethoxy]-2-chloro-benzoic acid (66 mg) obtained in Example (248d) in the same manner as in Example (248e). This silyl ether was deprotected with tetrabutylammonium fluoride in the same manner as in Example (248e) and 62 mg (two steps, 59%) of the title compound was obtained as a pale yellow solid.
$^1$H NMR(400MHz,DMSO-d6):δ(ppm)=2.55-2.47(2H, m), 3.72-3.61(4H, m), 3.98(2H, t, J=4.9Hz), 4.11(2H, brd, J=2.8Hz), 4.87(1H, t, J=5.5Hz), 6.14(1H, brs), 6.87(1H, dd, J=9.0 and 2.8Hz), 7.06-6.96(2H, m), 7.13(1H, brt like, J=7.2Hz), 7.22 (1H, ddd, J=11.5, 8.2 and 1.2Hz), 7.30(1H, d, J=8.7Hz), 7.40(2H, d, J=9.0Hz), 7.43(2H, d, J=9.0Hz), 8.10(1H, s), 8.14 (1H, ddd, J=11.5, 11.5 and 4.1Hz), 8.53(1H, d, J=2.7Hz), 9.10(1H, s).
MS(APCI) m/z:525, 527 $(M + H)^+$.
Melting point: 173-178°C.

(Example 261) 4-{4-[3-(2-methoxy-phenyl)-ureido]-phenyl}-piperidine-1-carboxylic acid [2-chloro-4-(2-hydroxy-ethoxy)-phenyl]-amide (Compound No. 4-22) (261a) 4-{4-[3-(2-methoxy-phenyl)-ureido]-phenyl}-piperidine-1-carboxylic acid tert-butyl ester

785 mg (quantitative yield) of the title compound was obtained as a white solid from 4-(4-amino-phenyl)-piperidine-1-carboxylic acid tert-butyl ester (500 mg) obtained in Example (228c) and 2-methoxyphenyl isocyanate (268 μl) in the same manner as in Example 1.
MS(APCI) m/z:426 $(M + H)^+$.

(261b) 1-(2-methoxy-phenyl)-3-(4-piperidin-4-yl-phenyl)-urea

541 mg (two steps, 92%) of the title compound was obtained as a white solid from 4-{4-[3-(2-methoxy-phenyl)-ureido]-phenyl}-piperidine-1-carboxylic acid tert-butyl ester (785 mg) obtained in Example (261 a) in the same manner as in Example (47a).
MS(APCI) m/z:326 $(M + H)^+$.

(261c) 4-{4-[3-(2-methoxy-phenyl)-ureido]-phenyl}-piperidine-1-carboxylic acid [2-chloro-4-(2-hydroxy-ethoxy)-phenyl]-amide

59 mg (45%) of silyl ether was obtained from 1-(2-methoxy-phenyl)-3-(4-piperidin-4-yl-phenyl)-urea (65 mg) obtained in Example (261b) and 4-[2-(tert-butyl- _ dimethyl-silanyloxy)-ethoxy]-2-chloro-benzoic acid (66 mg) obtained in Example (248d) in the same manner as in Example (248e). This silyl ether was deprotected with tetrabutylammonium fluoride in the same manner as in Example (248e) and 43 mg (49%) of the title compound was obtained as a white solid.
$^1$H NMR(400MHz,DMSO-d6):δ(ppm)=1.61-1.48(2H, m), 1.81-1.71(2H, m), 2.73-2.63(1H, m), 2.87(2H, t, J=12.2Hz), 3.69(2H, dd, J=10.2 and 5.1Hz), 3.87(3H, s), 3.98(2H, t, J=4.9Hz), 4.20(2H, brd, J=13.3Hz), 4.87(1H, t, J=5.5Hz), 6.89-6.84(2H, m), 6.95-6.89(1H, m), 7.00(1H, dd, J=8.0 and 1.4Hz), 7.03(1H, d, J=3.2Hz), 7.14(2H, d, J=8.6Hz), 7.28(1H, d, J=9.0Hz), 7.37(2H, d, J=8.6Hz), 8.08(1H, s), 8.11(1H, dd, J=7.7 and 1.8Hz), 8.18(1H, s), 9.24(1H, s).
MS(APCI) m/z:539, 541 $(M + H)^+$.
Melting point: 166-168˚C.

(Example 262) 4-{4-[3-(3,5-dimethoxy-phenyl)-ureido]-phenyl}-piperidine-1-carboxylic acid [2-chloro-4-(2-hydroxy-ethoxy)-phenyl]-amide (Compound No. 4-25)

(262a) 4-{4-[3-(3,5-dimethoxy-phenyl)-ureido]-phenyl}-piperidine-1-carboxylic acid tert-butyl ester

877 mg (quantitative yield) of the title compound was obtained as a white solid from 4-(4-amino-phenyl)-piperidine-1-carboxylic acid tert-butyl ester (500 mg) obtained in Example (228c) and 3,5-dimethoxyphenyl isocyanate (373 mg) in the same manner as in Example 1.
MS(APCI) m/z:456 $(M + H)^+$.

(262b) 1-(3,5-dimethoxy-phenyl)-3-(4-piperidin-4-yl-phenyl)-urea

858 mg (quantitative yield) of the title compound was obtained as a light brownish white solid from 4-{4-[3-(3,5-dimethoxy-phenyl)-ureido]-phenyl}-piperidine-1-carboxylic acid tert-butyl ester (877 mg) obtained in Example (262a) in the same manner as in Example (47a).
MS(APCI) m/z:356 $(M + H)^+$.

(262c) 4-{4-[3-(3,5-dimethoxy-phenyl)-ureido]-phenyl}-piperidine-1-carboxylic acid [2-chloro-4-(2-hydroxy-ethoxy)-phenyl]-amide

42 mg (31%) of silyl ether was obtained from 1-(3,5-dimethoxy-phenyl)-3-(4-piperidin-4-yl-phenyl)-urea (71 mg) obtained in Example (262b) and 4-[2-(tert-butyldimethyl-silanyloxy)-ethoxy]-2-chloro-benzoic acid (66 mg) obtained in Example (248d) in the same manner as in Example (248e). This silyl ether was deprotected with tetrabutylammonium fluoride in the same manner as in Example (248e) and 33 mg (94%) of the title compound was obtained as a white solid.
$^1$H NMR(400MHz,DMSO-d6):δ(ppm)=1.60-1.51(2H, m), 1.81-1.72(2H, m), 2.75-2.62(1H, m), 2.87(2H, t, J=12.4Hz), 3.70(6H, s), 3.72-3.67(2H, m), 3.98(2H, t, J=4.9Hz), 4.19(2H, brd, J=13.3Hz), 4.87(1H, t, J=5.4Hz), 6.11(1H, dd, J=2.2 and 2.2Hz), 6.66(2H, d, J=1.9Hz), 6.87(1H, dd, J=8.8 and 2.9Hz), 7.03(1H, d, J=2.8Hz), 7.14(2H, d, J=8.6Hz), 7.28(1H, d, J=8.6Hz), 7.35(2H, d, J=8.6Hz), 8.08(1H, s), 8.61(1H, s), 8.69(1H, s).
MS(APCI) m/z:569, 571 $(M + H)^+$.
Melting point: 101-105˚C.

(Example 263) 4-{4-[3-(2-fluoro-phenyl)-ureido]-phenyl}-piperidine-1-carboxylic acid [2-chloro-4-(2-hydroxy-ethoxy)-phenyl]-amide (Compound No. 4-21) (263a) 4-{4-[3-(2-fluoro-phenyl)-ureido]-phenyl}-piperidine-1-carboxylic acid tert-butyl ester

586 mg (98%) of the title compound was obtained as a light brown solid from 4-(4-amino-phenyl)-piperidine-1-carboxylic acid tert-butyl ester (400 mg) obtained in Example (228c) and 2-fluorophenyl isocyanate (187 μl) in the same manner as in Example 1.
MS(APCI) m/z:414 $(M + H)^+$.

(263b) 1-(2-fluoro-phenyl)-3-(4-piperidin-4-yl-phenyl)-urea

430 mg (97%) of the title compound was obtained as a light brownish yellow solid from 4-{4-[3-(2-fluoro-phenyl)-ureido]-phenyl}-piperidine-1-carboxylic acid tert-butyl ester (586 mg) obtained in Example (263a) in the same manner as in Example (47a).
MS(APCI) m/z:314 $(M + H)^+$.

(263c) 4-{4-[3-(2-fluoro-phenyl)-ureido]-phenyl}-piperidine-1-carboxylic acid [2-chloro-4-(2-hydroxy-ethoxy)-phenyl]-amide

Silyl ether was obtained from 1-(2-fluoro-phenyl)-3-(4-piperidin-4-yl-phenyl)-urea (62 mg) obtained in Example (263b) and 4-[2-(tert-butyl-dimethyl-silanyloxy)-ethoxy]-2-chloro-benzoic acid (66 mg) obtained in Example (248d) in the same manner as in Example (248e). This silyl ether was deprotected with tetrabutylammonium fluoride in the same manner as in Example (248e) and 48 mg (46%) of the title compound was obtained as a light brownish white solid.

$^1$H NMR(400MHz,DMSO-d6):δ(ppm)=1.61-1.48(2H, m), 1.81-1.73(2H, m), 2.74-2.64(1H, m), 2.87(2H, brt, J=11.9Hz), 3.69(2H, dd, J=9.8 and 5.1Hz), 3.98(2H, t, J=4.9Hz), 4.20(2H, brd, J=12.9Hz), 4.87(1H, t, J=5.5Hz), 6.87(1H, dd, J=8.8 and 2.9Hz), 7.06-6.94(2H, m), 7.12(1H, t, J=7.8Hz), 7.16(2H, d, J=8.6Hz), 7.21(1H, ddd, J=11.5, 8.2 and 1.3Hz), 7.28 (1H, d, J=8.6Hz), 7.37(2H, d, J=8.2Hz), 8.08(1H, s), 8.14(1H, ddd, J=11.6, 11.6 and 4.1Hz), 8.49(1H, d, J=2.3Hz), 8.98 (1H, s). MS(APCI) m/z:527, 529 $(M + H)^+$.
Melting point: 166-171°C.

(Example 264) 4-{4-[3-(3-ethoxy-phenyl)-ureido]-phenyl}-piperidine-1-carboxylic acid [2-chloro-4-(2-hydroxy-ethoxy)-phenyl]-amide (Compound No. 4-23) (264a) 4-{4-[3-(3-ethoxy-phenyl)-ureido]-phenyl}-piperidine-1-carboxylic acid tert-butyl ester

719 mg (quantitative yield) of the title compound was obtained as a light brown solid from 4-(4-amino-phenyl)-piperidine-1-carboxylic acid tert-butyl ester (400 mg) obtained in Example (228c) and 3-ethoxyphenyl isocyanate (246 μl) in the same manner as in Example 1.
MS(APCI) m/z:440 $(M + H)^+$.

(264b) 1-(3-ethoxy-phenyl)-3-(4-piperidin-4-yl-phenyl)-urea

536 mg (quantitative yield) of the title compound was obtained as a light brownish solid 4-{4-[3-(3-ethoxy-phenyl)-ureido]-phenyl}-piperidine-1-carboxylic acid tert-butyl ester (719 mg) obtained in Example (264a) in the same manner as in Example (47a).
MS(APCI) m/z:340 $(M + H)^+$.

(264c) 4-{4-[3-(3-ethoxy-phenyl)-ureido]-phenyl}-piperidine-1-carboxylic acid [2-chloro-4-(2-hydroxy-ethoxy)-phenyl]-amide

Silyl ether was obtained from 1-(3-ethoxy-phenyl)-3-(4-piperidin-4-yl-phenyl)-urea (67 mg) obtained in Example (264b) and 4-[2-(tert-butyl-dimethyl-silanyloxy)-ethoxy]-2-chloro-benzoic acid (66 mg) obtained in Example (248d) in the same manner as in Example (248e). This silyl ether was deprotected with tetrabutylammonium fluoride in the same manner as in Example (248e) and 41 mg (37%) of the title compound was obtained as a light brownish white solid.
$^1$H NMR(400MHz,DMSO-d6):δ(ppm)=1.32(3H, t, J=6.8Hz), 1.61-1.48(2H, m), 1.80-1.72(2H, m), 2.74-2.63(1H, m), 2.87 (2H, brt, J=12.3Hz), 3.69(2H, dd, J=10.0 and 5.3Hz), 4.02-3.94(4H, m), 4.20(2H, brd, J=13.3Hz), 4.87(1H, t, J=5.5Hz), 6.51(1H, dd, J=8.2 and 1.9Hz), 6.91-6.84(2H, m), 7.03(1H, d, J=2.8Hz), 7.14(2H, d, J=8.6Hz), 7.18-7.10(2H, m), 7.28 (1H, d, J=9.0Hz), 7.36(2H, d, J=8.6Hz), 8.08(1H, s), 8.55(1H, s), 8.60(1H, s).
MS(APCI) m/z:553, 555 $(M + H)^+$.
Melting point: 129-134°C.

(Example 265) 6-[3-(2-methoxy-phenyl)-ureido]-3,4-dihydro-1H-isoquinoline-2-carboxylic acid [2-chloro-4-(2-hydroxy-ethoxy)-phenyl]-amide (Compound No. 3-72)

(265a) 6-[3-(2-methoxy-phenyl)-ureido]-3,4-dihydrido-1H-isoquinoline-2-carboxylic acid tert-butyl ester

784 mg (98%) of the title compound was obtained as a white solid from 6-amino-3,4-dihydro-1H-isoquinoline-2-carboxylic acid tert-butyl ester (500 mg) and 2-methoxyphenyl isocyanate (285 μl) in the same manner as in Example (202a).
MS(APCI) m/z:398 $(M + H)^+$.

(265b) 1-(2-methoxy-phenyl)-3-(1,2,3,4-tetrahydro-isoquinolin-6-yl)-urea

659 mg (quantitative yield) of the title compound was obtained as a pale yellow solid from 6-[3-(2-methoxy-phenyl)-ureido]-3,4-dihydrido-1H-isoquinoline-2-carboxylic acid tert-butyl ester (784 mg) obtained in Example (265a) in the same manner as in Example (202b).
MS(APCI) m/z:298 $(M + H)^+$.

(265c) 6-[3-(2-methoxy-phenyl)-ureido]-3,4-dihydro-1H-isoquinoline-2-carboxylic acid [2-chloro-4-(2-hydroxy-ethoxy)-phenyl]-amide

Silyl ether was obtained from 1-(2-methoxy-phenyl)-3-(1,2,3,4-tetrahydro-isoquinolin-6-yl)-urea (60 mg) obtained in Example (265b) and 4-[2-(tert-butyldimethyl-silanyloxy)-ethoxy]-2-chloro-benzoic acid (66 mg) obtained in Example (248d) in the same manner as in Example (248e). This silyl ether was deprotected with tetrabutylammonium fluoride in the same manner as in Example (248e) and 52 mg (51%) of the title compound was obtained as a white solid.
$^1$H NMR(400MHz,DMSO-d6):δ(ppm)=2.82(2H, t, J=5.9Hz), 3.72-3.64(4H, m), 3.87(3H, s), 3.98(2H, t, J=4.9Hz), 4.54 (2H, s), 4.87(1H, t, J=5.5Hz), 6.89-6.84(2H, m), 6.95-6.89(1H, m), 7.00(1H, dd, J=8.0 and 1.3Hz), 7.03(1H, d, J=2.8Hz), 7.06(1H, d, J=8.2Hz), 7.20(1H, dd, J=8.2 and 1.9Hz), 7.29(1H, d, J=8.6Hz), 7.34(1H, d, J=1.5Hz), 8.12(1H, s), 8.13-8.08 (1H, m), 8.19(1H, s), 9.24(1H, s).
MS(APCI) m/z:511, 513 $(M + H)^+$.
Melting point: 130-135˚C.

(Example 266) 6-[3-(3,5-dimethoxy-phenyl)-ureido]-3,4-dihydro-1H-isoquinoline-2-carboxylic acid [2-chloro-4-(2-hydroxy-ethoxy)-phenyl]-amide (Compound No. 3-75)

(266a) 6-[3-(3,5-dimethoxy-phenyl)-ureido]-3,4-dihydrido-1H-isoquinoline-2-carboxylic acid tert-butyl ester

846 mg (98%) of the title compound was obtained as a white solid from 6-amino-3,4-dihydro-1H-isoquinoline-2-carboxylic acid tert-butyl ester (500 mg) and 3,5-dimethoxyphenyl isocyanate (396 mg) in the same manner as in Example (202a).
MS(APCI) m/z:428 $(M + H)^+$.

(266b) 1-(3,5-dimethoxy-phenyl)-3-(1,2,3,4-tetrahydro-isoquinolin-6-yl)-urea

735 mg (quantitative yield) of the title compound was obtained as a light brown solid from 6-[3-(3,5-dimethoxy-phenyl)-ureido]-3,4-dihydrido-1H-isoquinoline-2-carboxylic acid tert-butyl ester (846 mg) obtained in Example (266a) in the same manner as in Example (202b).
MS(APCI) m/z:328 $(M + H)^+$.

(266c) 6-[3-(3,5-dimethoxy-phenyl)-ureido]-3,4-dihydro-1H-isoquinoline-2-carboxylic acid [2-chloro-4-(2-hydroxy-ethoxy)-phenyl]-amide

67 mg (51%) of silyl ether was obtained from 1-(3,5-dimethoxy-phenyl)-3-(1,2,3,4-tetrahydro-isoquinolin-6-yl)-urea (65 mg) obtained in Example (266b) and 4-[2-(tert-butyl-dimethyl-silanyloxy)-ethoxy]-2-chloro-benzoic acid (66 mg) obtained in Example (248d) in the same manner as in Example (248e). This silyl ether was deprotected with tetrabutylammonium fluoride in the same manner as in Example (248e) and 53 mg (96%) of the title compound was obtained as a white solid.
$^1$H NMR(400MHz,DMSO-d6):δ(ppm)=2.82(2H, t, J=5.6Hz), 3.71(6H, s), 3.72-3.63(4H, m), 3.98(2H, t, J=4.9Hz), 4.54 (2H, s), 4.87(1H, t, J=5.5Hz), 6.12(1H, dd, J=2.2Hz and 2.2Hz), 6.66(2H, d, J=2.4Hz), 6.87(1H, dd, J=9.0 and 2.7Hz), 7.03(1H, d, J=2.8Hz), 7.05(1H, d, J=8.2Hz), 7.19(1H, dd, J=8.2 and 1.9Hz), 7.29(1H, d, J=8.6Hz), 7.33(1H, d, J=1.9Hz), 8.12(1H, s), 8.60(1H, s), 8.68(1H, s).
MS(APCI) m/z:541, 543 $(M + H)^+$.
Melting point: 105-110˚C.

(Example 267) 6-[3-(2-fluoro-phenyl)-ureido]-3,4-dihydro-1H-isoquinoline-2-carboxylic acid [2-chloro-4-(2-hydroxy-ethoxy)-phenyl]-amide (Compound No. 3-71)

(267a) 6-[3-(2-fluoro-phenyl)-ureido]-3,4-dihydrido-1H-isoquinoline-2-carboxylic acid tert-butyl ester

678 mg (quantitative yield) of the title compound was obtained as a white solid from 6-amino-3,4-dihydro-1H-isoquinoline-2-carboxylic acid tert-butyl ester (400 mg) and 2-fluorophenyl isocyanate (199 μl) in the same manner as in Example (202a).
MS(APCI) m/z:386 $(M + H)^+$.

(267b) 1-(2-fluoro-phenyl)-3-(1,2,3,4-tetrahydro-isoquinolin-6-yl)-urea

216 mg (47%) of the title compound was obtained as a pale yellow solid from 6-[3-(2-fluoro-phenyl)-ureido]-3,4-dihydrido-1H-isoquinoline-2-carboxylic acid tert-butyl ester (678 mg) obtained in Example (267a) in the same manner as in Example (202b).
MS(APCI) m/z:286 $(M + H)^+$.

(267c) 6-[3-(2-fluoro-phenyl)-ureido]-3,4-dihydro-1H-isoquinoline-2-carboxylic acid [2-chloro-4-(2-hydroxy-ethoxy)-phenyl]-amide

Silyl ether was obtained from 1-(2-fluoro-phenyl)-3-(1,2,3,4-tetrahydro-isoquinolin-6-yl)-urea (57 mg) obtained in Example (267b) and 4-[2-(tert-butyldimethyl-silanyloxy)-ethoxy]-2-chloro-benzoic acid (66 mg) obtained in Example (248d) in the same manner as in Example (248e). This silyl ether was deprotected with tetrabutylammonium fluoride in the same manner as in Example (248e) and 62 mg (59%) of the title compound was obtained as a white solid.
$^1$H NMR(400MHz,DMSO-d6):δ(ppm)=2.84(2H, t, J=5.6Hz), 3.63-3.74(4H, m), 4.00(2H, t, J=4.SHz), 4.57(2H, brs), 4.88 (1H, t, J=5.5Hz), 6.89(1H, dd, J=8.6 and 2.0Hz), 7.18-6.97(4H, m), 7.28-7.20(2H, m), 7.32(1H, d, J=9.0Hz), 7.36(1H, s), 8.21-8.12(2H, m), 8.54(1H, s), 9.02(1H, s).
MS(APCI) m/z:499, 501 (M + H)$^+$.
Melting point: 183-186˚C.

(Example 268) 4-{5-[3-(2-methoxy-5-methyl-phenyl)-ureido]-pyridin-2-yl}-piperazine-1-carboxylic acid [2-chloro-4-(2-hydroxy-ethoxy)-phenyl]-amide (Compound No. 1-567)

153 mg (23%) of urea compound was obtained as a white solid from 1-(2-methoxy-5-methyl-phenyl)-3-(6-piperazin-1-yl-pyridin-3-yl)-urea (341 mg) obtained in Example (137a) and 4-[2-(tert-butyl-dimethyl-silanyloxy)-ethoxy]-2-chlorobenzoic acid (331 mg) obtained in Example (248d) in the same manner as in

Example (248e). This urea compound was deprotected with tetrabutylammonium fluoride in the same manner as in Example (248e) and 31.2 mg (28%) of the title compound was obtained as a white solid.
$^1$H NMR(400MHz,DMSO-d6):δ(ppm)=9.07(1H, s), 8.19(1H, s), 8.14(1H, d, J=2.0Hz), 8.09(1H, s), 7.93(1H, s), 7.73(1H, dd, J=8.8 and 2.0Hz), 7.27(1H, d, J=8.6Hz), 7.03(1H, d, J=2.3Hz), 6.89-6.83(1H, m), 6.86(1H, d, J=8.3Hz), 6.86(1H, d, J=8.8Hz), 6.71(1H, d, J=8.3Hz), 4.88(1H, t, J=4.6Hz), 3.98(2H, t, J=4.9Hz), 3.82(3H, s), 3.69(2H, dt, J=4.9 and 4.6Hz), 3.57-3.51 (4H, m), 3.46-3.41(4H, m), 2.22(3H, s). MS(ES$^+$) m/z:555 (M + H)$^+$.
Melting point: 196-197˚C.

(Example 269) 4-{2-chloro-4-[3-(2-methoxy-phenyl)-ureido]-phenyl}-piperazine-1-carboxylic acid [2-chloro-4-(2-hydroxy-ethoxy)-phenyl]-amide (Compound No. 2-133)

(269a) 4-{2-chloro-4-[3-(2-methoxy-phenyl)-ureido]-phenyl}-piperazine-1-carboxylic acid tert-butyl ester

2.30 g (quantitative yield) of the title compound was obtained as a pale pink solid from 4-(4-amino-2-chloro-phenyl)-piperazine-1-carboxylic acid tert-butyl ester (1.56 g) obtained in Example (221b) and 2-methoxyphenyl isocyanate (0.80 mL) in the same manner as in Example 1.
$^1$H NMR(400MHz,DMSO-d6):δ(ppm)=9.37(1H, s), 8.20(1H, s), 8.11(1H, d, J=7.8Hz), 7.71(1H, d, J=2.3Hz), 7.22(1H, dd, J=8.6 and 2.3Hz), 7.12(1H, d, J=8.6Hz), 7.03(1H, d, J=7.8Hz), 6.96(1H, dd, J=7.8 and 7.8Hz), 6.90(1H, dd, J=7.8 and 7.8Hz), 3.88(3H, s), 3.47(4H, t, J=4.5Hz), 2.86(4H, t, J=4.5Hz), 1.43(9H, s). MS(ES$^+$) m/z:461 (M + H)$^+$.

(269b) 1-(3-chloro-4-piperazin-1-yl-phenyl)-3-(2-methoxy-phenyl)-urea

1.04 g (58%) of the title compound was obtained as a white solid from 4-{2-chloro-4-[3-(2-methoxy-phenyl)-ureido]-phenyl}-piperazine-1-carboxylic acid tert-butyl ester (2.28 g) obtained in Example (269a) in the same manner as in Example (47a).
MS(ES$^+$) m/z:361 (M + H)$^+$.

(269c) 4-{2-chloro-4-[3-(2-methoxy-phenyl)-ureido]-phenyl}-piperazine-1-carboxylic acid [2-chloro-4-(2-hydroxy-ethoxy)-phenyl]-amide

201 mg (58%) of urea compound was obtained as a pale yellow solid from 1-(3-chloro-4-piperazin-1-yl-phenyl)-3-(2-methoxy-phenyl)-urea (180 mg) obtained in Example (269b) and 4-[2-(tert-butyl-dimethyl-silanyloxy)-ethoxy]-2-chloro-benzoic acid (165 mg) obtained in Example (248d) in the same manner as in Example (248e). This urea compound was deprotected with tetrabutylammonium fluoride in the same manner as in Example (248e) and 52.9 mg (34%) of the title compound was obtained as a white solid.
$^1$H NMR(400MHz,DMSO-d6):δ(ppm)=9.35(1H, s), 8.17(2H, s), 8.09(1H, dd, J=8.1 and 1.8Hz), 7.71(1H, d, J=2.7Hz), 7.28(1H, d, J=9.0Hz), 7.21(1H, dd, J=8.6 and 2.7Hz), 7.13(1H, d, J=8.6Hz), 7.04(1H, d, J=3.1Hz), 7.01(1H, dd, J=8.2 and 1. 8Hz), 6.94(1H, ddd, J=8.2, 7.8 and 1.8Hz), 6.91-6.85(2H, m), 4.86(1H, t, J=5.7Hz), 3.99(2H, t, J=4.8Hz), 3.87 (3H, s), 3.69(2H, dt, J=5.7 and 4.8Hz), 3.58(4H, t, J=4.2Hz), 2.92(4H, t, J=4.2Hz).

MS(ES$^+$) m/z:574 (M + H)$^+$.
Melting point: 173-175˚C.

(Example 270) 4-{4-[3-(3-methoxy-pyridin-2-yl)-ureido]-phenyl}-piperazine-1-carboxylic acid [2-chloro-4-(2-hydroxy-ethoxy)-phenyl]-amide (Compound No. 1-860)

(270a) 4-{4-[3-(3-methoxy-pyridin-2-yl)-ureido]-phenyl}-piperazine-1-carboxylic acid tert-butyl ester

1.61 g (75%) of the title compound was obtained as a pale yellow solid from 4-[4-(4-nitro-phenoxycarbonylamino)-phenyl]-piperazine-1-carboxylic acid tert-butyl ester (2.21 g) obtained in Example (224a) and 2-amino-3-methoxypyridine (745 mg) in the same manner as in Example (224b).
$^1$H NMR(400MHz,DMSO-d6):δ(ppm)=11.5(1H, s), 8.06(1H, s), 7.91(1H, d, J=5.0Hz), 7.45(2H, d, J=9.0Hz), 7.43(1H, d, J=8.0Hz), 7.05(1H, dd, J=8.0 and 5.0Hz), 6.95(2H, d, J=9.0Hz), 3.89(3H, s), 3.46(4H, t, J=4.6Hz), 3.03(4H, t, J=4.6Hz), 1.42(9H, s).
MS(ES$^+$) m/z:428 (M + H)$^+$.

(270b) 1-(3-methoxy-pyridin-2-yl)-3-(4-piperazin-1-yl-phenyl)-urea

1.21 g (quantitative yield) of the title compound was obtained as a white solid from 4-{4-[3-(3-methoxy-pyridin-2-yl)-ureido]-phenyl}-piperazine-1-carboxylic acid tert-butyl ester (1.58 g) obtained in Example (271a) in the same manner as in Example (47a).
MS(ES$^+$) m/z:328 (M + H)$^+$.

(270c) 4-{4-[3-(3-methoxy-pyridin-2-yl)-ureido]-phenyl}-piperazine-1-carboxylic acid [2-chloro-4-(2-hydroxy-ethoxy)-phenyl]-amide

214 mg (65%) of urea compound was obtained as a white solid from 1-(3-methoxy-pyridin-2-yl)-3-(4-piperazin-1-yl-phenyl)-urea (164 mg) obtained in Example (270b) and 4-[2-(tert-butyl-dimethyl-silanyloxy)-ethoxy]-2-chloro-benzoic acid (165 mg) obtained in Example (248d) in the same manner as in Example (248e). This urea compound was deprotected with tetrabutylammonium fluoride in the same manner as in Example (248e) and 118 mg (71%) of the title compound was obtained as a white solid.
$^1$H NMR(400MHz,DMSO-d6):δ(ppm)=11.5(1H, s), 8.19(1H, s), 8.04(1H, s), 7.89(1H, dd, J=5.2 and 1.3Hz), 7.43(2H, d, J=9.0Hz), 7.41(1H, dd, J=8.0, 1.3Hz), 7.28(1H, d, J=8.8Hz), 7.06-7.00(2H, m), 6.96(2H, d, J=9.0Hz), 6.87(1H, dd, J=8.8 and 2.9Hz), 4.86(1H, t, J=5.5Hz), 3.98(2H, t, J=4.9Hz), 3.88(3H, s), 3.69(2H, dt, J=5.5 and 4.9Hz), 3.58(4H, t, J=4.7Hz), 3.10(4H, t, J=4.7Hz).
MS(ES$^+$) m/z:541 (M + H)$^+$.
Melting point: 215-216˚C.

(Example 271) 4-{5-[3-(4,5-dimethyl-thiazol-2-yl)-ureido]-pyridin-2-yl}-piperazine-1-carboxylic acid [2-chloro-4-(2-hydroxy-ethoxy)-phenyl]-amide (Compound No. 1-865)

179 mg (54%) of urea compound was obtained as a white solid from 1-(4,5-dimethyl-thiazol-2-yl)-3-(6-piperazin-1-yl-pyridin-3-yl)-urea (166 mg) obtained in Example (237c) and 4-[2-(tert-butyl-dimethyl-silanyloxy)-ethoxy]-2-chlorobenzoic acid (165 mg) obtained in Example (248d) in the same manner as in Example (248e). This urea compound was deprotected with tetrabutylammonium fluoride in the same manner as in Example (248e) and 96.8 mg (72%) of the title compound was obtained as a white solid.
$^1$H NMR(400MHz,DMSO-d6):δ(ppm)=10.2(1H, s), 8.72(1H, s), 8.19(1H, s), 8.18(1H, s), 7.72(1H, d, J=9.0Hz), 7.27(1H, d, J=8.6Hz), 7.03(1H, d, J=2.7Hz), 6.87(1H, dd, J=8.6 and 2.7Hz), 6.87(1H, d, J=9.0Hz), 4.87(1H, t, J=5.7Hz), 3.98(2H, t, J=4.7Hz), 3.69(2H, dt, J=5.7 and 4.7Hz), 3.56-3.50(4H, m), 3.49-3.41(4H, m), 2.19(3H, s), 2.10(3H, s).
MS(ES$^+$) m/z:546 (M + H)$^+$.
Melting point: 184-185˚C.

(Example 272) 4-{4-[3-(4,5-dimethyl-thiazol-2-yl)-ureido]-phenyl}-piperazine-1-carboxylic acid [2-chloro-4-(2-hydroxy-ethoxy)-phenyl]-amide (Compound No. 1-864)

176 mg (53%) of urea compound was obtained as a white solid from 1-(4,5-dimethyl-thiazol-2-yl)-3-(4-piperazin-1-yl-phenyl)-urea (166 mg) obtained in Example (225b) and 4-[2-(tert-butyl-dimethyl-silanyloxy)-ethoxy]-2-chloro-benzoic acid (165 mg) obtained in Example (248d) in the same manner as in Example (248e). This urea compound was

deprotected with tetrabutylammonium fluoride in the same manner as in Example (248e) and 72.3 mg (55%) of the title compound was obtained as a white solid.
$^{1}$H NMR(400MHz,DMSO-d6):δ(ppm)=10.1(1H, s), 8.72(1H, s), 8.20(1H, s), 7.31(2H, d, J=9.0Hz), 7.27(1H, d, J=8.6Hz), 7.03(1H, d, J=2.7Hz), 6.93(2H, d, J=9.0Hz), 6.87(1H, dd, J=8.6 and 2.7Hz), 4.87(1H, t, J=5.5Hz), 3.98(2H, t, J=4.9Hz), 3.69(2H, dt, J=5.5 and 4.9Hz), 3.70(4H, t, J=4.9Hz), 3.08(4H, t, J=4.9Hz), 2.19(3H, s), 2.11(3H, s).
MS(ES$^{+}$) m/z:545 (M + H)$^{+}$.
Melting point: 186-187˚C.

(Example 273) 4-{4-[3-(2-methoxy-5-methyl-phenyl)-ureido]-phenyl}-3,6-dihydro-2H-pyridine-1-carboxylic acid [2-chloro-5-(2-hydroxy-ethoxy)-phenyl]-amide (Compound No. 5-64)

100 mg (68%) of silyl ether was obtained from 1-(2-methoxy-5-methylphenyl)-3-[4-(1,2,3,6-tetrahydro-pyridin-4-yl)-phenyl]-urea (74 mg) obtained in Example (230b) and 5-[2-(tert-butyl-dimethyl-silanyloxy)-ethoxy]-2-chloro-phenylamine (66 mg) obtained in Example (231c) in the same manner as in Example 170. This silyl ether was deprotected with tetrabutylammonium fluoride in an anhydrous tetrahydrofuran solution and 6 mg (7%) of the title compound was obtained as a pale pink solid.
$^{1}$H NMR(400MHz,DMSO-d6):δ(ppm)=2.23(3H, s), 2.59-2.40(2H, m), 3.73-3.62(4H, m), 3.83(3H, s), 3.97-3.90(2H, m), 4.23-4.12(2H, m), 6.13(1H, s), 6.81-6.65(2H, m), 6.88(1H, d, J=8.3Hz), 7.46-7.15(6H, m), 7.96(1H, s), 8.05(1H, s), 8.15 (1H, s), 9.34(1H, s).
MS(APCI) m/z:551, 553 (M + H)$^{+}$.
Melting point: 139-142˚C.

(Example 274) 4-{4-[3-(2-methoxy-5-methyl-phenyl)-ureido]-phenyl}-piperidine-1-carboxylic acid [2-chloro-5-(2-hydroxy-ethoxy)-phenyl]-amide (Compound No. 4-64)

86 mg (59%) of silyl ether was obtained as a white solid from 1-(2-methoxy-5-methyl-phenyl)-3-(4-piperidin-4-yl-phenyl)-urea (74 mg) obtained in Example (228e) and 5-[2-(tert-butyl-dimethyl-silanyloxy)-ethoxy]-2-chloro-phenylamine (67 mg) obtained in Example (231c) in the same manner as in Example 170. This silyl ether was deprotected with tetrabutylammonium fluoride in an anhydrous tetrahydrofuran solution and 63 mg (88%) of the title compound was obtained as a white solid.
$^{1}$H NMR(400MHz,DMSO-d6):δ(ppm)=1.62-1.50(2H, m), 1.78(2H, d, J=12.9Hz), 2.22(3H, s), 2.74-2.64(1H, m), 2.91(2H, t, J=11.9Hz), 3.70(2H, dd, J=9.8 and 5.1Hz), 3.82(3H, s), 3.95(2H, t, J=4.9Hz), 4.20(2H, d, J=13.3Hz), 4.87(1H, t, J=4.5Hz), 6.74-6.68(2H, m), 6.87(1H, d, J=8.2Hz), 7.15(2H, d, J=8.3Hz), 7.20(1H, d, J=3.1Hz), 7.30(1H, d, J=8.6Hz), 7.36(2H, d, J=8.6Hz), 7.96(1H, d, J=1.9Hz), 8.06(1H, s), 8.11(1H, s), 9.21(1H, s).
MS(APCI) m/z:553, 555 (M + H)$^{+}$.
Melting point: 202-205˚C.

(Example 275) 4-{4-[3-(2-methoxy-5-methyl-phenyl)-ureido]-phenyl}-piperazine-1-carboxylic acid [4-(2,3-dihydroxy-propoxy)-2-trifluoromethyl-phenyl]-amide (Compound No. 1-627)

(275a) 3-(4-nitro-3-trifluoromethyl-phenoxy)-propane-1,2-diol

Potassium carbonate (3.32 g) and 3-bromo-1,2-propanediol (4.0 mL) were added to a solution of 4-nitro-3-(trifluoromethyl)phenol (4.14 g) in acetonitrile (40 mL) at room temperature. The reaction mixture was heated under reflux for 23 hours, diluted with ethyl acetate and washed with water and saturated brine and dried over sodium sulfate and concentrated. The residue was purified by column chromatography (dichloromethane:ethyl acetate 5:1 → 1:1) and the title compound was obtained 5.40 g (96%) as a pale yellow solid.
$^{1}$H NMR(400MHz,$CDCl_3$):δ(ppm)=8.03(1H, d, J=8.9Hz), 7.35(1H, d, J=2.7Hz), 7.17(1H, dd, J=9.0 and 2.8Hz), 4.21-4.15 (2H, m), 3.99-3.96(1H, m), 3.88(1H, dd, J=5.3 and 3.7Hz), 3.82-3.76(1H, m), 2.56(1H, brs), 1.97(1H, t, J=5.7Hz).

(275b) 2,2-dimethyl-4-(4-nitro-3-trifluoromethyl-phenoxymethyl)-1,3-dioxolane

2,2-dimethoxypropane (47 mL) and p-toluenesulfonic acid (483 mg) were added to a solution of 3-(4-nitro-3-trifluoromethyl-phenoxy)-propane-1,2-diol (5.40 g) obtained in Example (275a) in anhydrous tetrahydrofuran (50 mL) at room temperature. The reaction mixture was stirred at room temperature for 24 hours and concentrated and diluted with ethyl acetate and washed with water and brine and dried over sodium sulfate and concentrated. The residue was purified by column chromatography (dichloromethane:ethyl acetate 1:0 → 3:1) and 6.06 g (98%) of the title compound was obtained as a yellow oil.

$^1$H NMR(400MHz,CDCl3):δ(ppm)=8.00(1H, d, J=9.0Hz), 7.33(1H, d, J=2.7Hz), 7.14(1H, dd, J=9.0 and 2.7Hz), 4.54-4.48 (1H, m), 4.19(1H, dd, J=8.6 and 6.6Hz), 4.17-4.07(2H, m), 3.91(1H, dd, J=8.6 and 5.5Hz), 1.46(3H, s), 1.41(3H, s).

(275c) 4-(2,2-dimethyl-1,3-dioxolan-4-ylmethoxy)-2-trifluoromethyl-phenylamine

A suspension of anhydrous tetrahydrofuran (60 mL) of 2,2-dimethyl-4-(4-nitro-3-trifluoromethyl-phenoxymethyl)-1,3-dioxolane (6.06 g) obtained in Example (275b) and palladium-carbon (10%, 0.60 g) was stirred under hydrogen gas atmosphere at room temperature for five days. The reaction mixture was filtered and concentrated. The residue was purified by column chromatography (hexane:ethyl acetate from 5:1 to 3:1) and 5.39 g (98%) of the title compound was obtained as a yellow oil.
$^1$H NMR(400MHz,$CDCl_3$):δ(ppm)=7.01(1H, d, J=3.1Hz), 6.94(1H, dd, J=8.8 and 2.9Hz), 6.70(1H, d, J=9.0Hz), 4.48-4.42 (1H, m), 4.18-4.10(1H, m), 4.00(1H, dd, J=9.4 and 5.4Hz), 3.91-3.86(4H, m), 1.46(3H, s), 1.40(3H, s).

(275d) 4-{4-[3-(2-methoxy-5-methyl-phenyl)-ureido]-phenyl}-piperazine-1-carboxylic acid [4-(2,3-dihydroxy-propoxy)-2-trifluoromethyl-phenyl]-amide

341 mg (65%) of urea compound was obtained as a white solid from 1-(2-methoxy-5-methyl-phenyl)-3-(4-piperazin-1-yl-phenyl)-urea (273 mg) obtained in Example (47a) and 4-(2,2-dimethyl-1,3-dioxolan-4-ylmethoxy)-2-trifluoromethyl-phenylamine (234 mg) obtained in Example (275c) in the same manner as in Example 170. p-Toluenesulfonic acid (10 mg) was added to a solution of this urea compound (340 mg) in methanol (10 mL) at room temperature. The reaction mixture was stirred at room temperature for two weeks and concentrated. The residue was purified by column chromatography (dichloromethane:ethyl acetate 3:1 → 1:1, further dichloromethane:methanol 30:1 → 5:1). The obtained solid was vigorously stirred in hexane/diisopropyl ether (5:1), collected by filtration and dried under reduced pressure, and 175 mg (55%) of the title compound was obtained as a pale pink solid.
$^1$H NMR(400MHz,DMSO-d6):δ(ppm)=9.09(1H, s), 8.22(1H, s), 8.07(1H, s), 7.99(1H, s), 7.33(2H, d, J=7.8Hz), 7.31(1H, d, J=7.5Hz), 7.24-7.15(2H, m), 6.94(2H, d, J=8.2Hz), 6.89(1H, d, J=7.8Hz), 6.73(1H, d, J=8.6Hz), 5.00(1H, d, J=4.7Hz), 4.70(1H, t, J=5.4Hz), 4.08(1H, dd, J=3.5 and 9.8Hz), 3.94(1H, dd, J=6.7 and 9.4Hz), 3.84(3H, s), 3.81(1H, dd, J=7.8 and 7.9Hz), 3.56(4H, brs), 3.45(2H, t, J=5.5Hz), 3.06(4H, brs), 2.23(3H, s).
MS($ES^+$) m/z:618 $(M + H)^+$.
Melting point: 187-190˚C.

(Example 276) 4-{4-[3-(2-methoxy-5-methyl-phenyl)-ureido]-phenyl}-piperazine-1-carboxylic acid [4-(2,3-dihydroxy-propoxy)-2-methyl-phenyl]-amide (Compound No. 1-755)

(276a) 3-(3-methyl-4-nitro-phenoxy)-propane-1,2-diol

4.54 g (quantitative yield) of the title compound was obtained as a yellow oil from 4-nitro-m-cresol (3.06 g) and 3-bromo-1,2-propanediol (4.0 mL) in the same manner as in Example (275a).
$^1$H NMR(400MHz,$CDCl_3$):δ(ppm)=8.06(1H, d, J=9.4Hz), 6.82-6.80(1H, m), 6.80(1H, s), 4.12-4.09(3H, m), 3.86(1H, d, J=11.3Hz), 3.76(1H, d, J=11.3Hz), 2.72(1H, d, J=3.9Hz), 2.62(3H, s), 2.18(1H, brs).

(276b) 2,2-dimethyl-4-(4-nitro-3-methyl-phenoxymethyl)-1,3-dioxolane

5.01 g (94%) of the title compound was obtained as a yellow oil from 3-(3-methyl-4-nitro-phenoxy)-propane-1,2-diol (4.54 g) obtained in Example (276a) in the same manner as in Example (275b).
$^1$H NMR(400MHz,$CDCl_3$):δ(ppm)=8.09(1H, d, J=9.0Hz), 6.83(1H, d, J=7.5Hz), 6.82(1H, s), 4.53-4.47(1H, m), 4.19(1H, dd, J=7.4 and 7.5Hz), 4.11(1H, dd, J=10.2 and 4.7Hz), 4.03(1H, dd, J=9.6 and 5.6Hz), 3.91(1H, dd, J=8.6 and 5.9Hz), 2.63(3H, s), 1.47(3H, s), 1.41 (3H, s).

(276c) 4-(2,2-dimethyl-1,3-dioxolan-4-ylmethoxy)-2-methyl-phenylamine

4.45 g (quantitative yield) of the title compound was obtained as reddish brown oil from 2,2-dimethyl-4-(4-nitro-3-methyl-phenoxymethyl)-1,3-dioxolane (5.01 g) obtained in Example (276b) in the same manner as in Example (275c).
$^1$H NMR(400MHz,$CDCl_3$):δ(ppm)=6.67(1H, d, J=2.3Hz), 6.61(1H, d, J=2.7Hz), 6.60(1H, s), 4.46-4.40(1H, m), 4.16-4.09 (1H, m), 3.99(1H, dd, J=9.4 and 5.5Hz), 3.88-3.83(2H, m), 3.36(2H, brs), 2.15(3H, s), 1.45(3H, s), 1.39(3H, s).

(276d) 4-{4-[3-(2-methoxy-5-methyl-phenyl)-ureido]-phenyl}-piperazine-1-carboxylic acid [4-(2,3-dihydroxy-propoxy)-2-methyl-phenyl]-amide

615 mg (91%) of urea compound was obtained as a light brown solid from 1-(2-methoxy-5-methyl-phenyl)-3-(4-piperazin-1-yl-phenyl)-urea (380 mg) obtained in Example (47a) and 4-(2,2-dimethyl-1,3-dioxolan-4-ylmethoxy)-2-methylphenylamine (265 mg) obtained in Example (276c) in the same manner as in Example 170. This urea compound was deprotected in the same manner as in Example (275d) and 180 mg (74%) of the title compound was obtained as a white solid.
$^1$H NMR(400MHz,DMSO-d6):δ(ppm)=9.10(1H, s), 8.07(1H, s), 8.03(1H, s), 7.99(1H, s), 7.33(2H, d, J=8.6Hz), 7.04(1H, d, J=9.0Hz), 6.94(2H, d, J=9.0Hz), 6.88(1H, d, J=8.6Hz), 6.78(1H, d, J=2.8Hz), 6.72(2H, dd, J=8.4 and 8.4Hz), 4.92 (1H, d, J=4.7Hz), 4.65(1H, t J=5.7Hz), 3.95(1H, dd, J=3.7 and 9.6Hz), 3.83(3H, s), 3.82-3.75(2H, m), 3.57(4H, t, J=4.3Hz), 3.44(2H, t, J=5.5Hz), 3.06(4H, t, J=3.7Hz), 2.23(3H, s), 2.13(3H, s).
MS(ES$^+$) m/z:564 (M + H)$^+$.
Melting point: 148-150˚C.

(Example 277) 4- {4-[3-(2-methoxy-5-methyl-phenyl)-ureido]-phenyl} -piperazine-1 - carboxylic acid [2-chloro-4-(2,3-dihydroxy-propoxy)-phenyl]-amide (Compound No. 1-579)

(277a) 2-chloro-4-(2,3-dihydro-propoxy)-benzoic acid methyl ester

4.15 g (58%) of the title compound was obtained as a yellow oil from 2-chloro-4-hydroxy-benzoic acid methyl ester (4.18 g) obtained in Example (248a) and 3-bromo-1,2-propanediol (5.5 mL) in the same manner as in Example (275a).
$^1$H NMR(400MHz,$CDCl_3$):δ(ppm)=7.89(1H, d, J=8.6Hz), 7.01(1H, d, J=1.6Hz), 6.85(1H, dd, J=9.3 and 2.0Hz), 4.12-4.08 (3H, m), 3.91(3H, s), 3.85(1H, brs), 3.78(1H, brs), 2.58(1H, brs), 2.00(1H, brs).

(277b) 2-chloro-4-(2,2-dimethyl-1,3-dioxolan-4-ylmethoxy)-benzoic acid methyl ester

3.69 g (77%) of the title compound was obtained as an orange solid from 2-chloro-4-(2,3-dihydro-propoxy)-benzoic acid methyl ester (4.15 g) obtained in Example (277a) in the same manner as in Example (275b).
$^1$H NMR(400MHz,$CDCl_3$):δ(ppm)=7.86(1H, d, J=9.0Hz), 6.98(1H, d, J=2.7Hz), 6.83(1H, dd, J=8.8 and 2.6Hz), 4.50-4.44 (1H, m), 4.16(1H, dd, J=8.4 and 6.5Hz), 4.07(1H, dd, J=9.4 and 5.5Hz), 3.98(1H, dd, J=9.4 and 5.5Hz), 3.89(3H, s), 3.90-3.87(1H, m), 1.46(3H, s), 1.40(3H, s).

(277c) 2-chloro-4-(2,2-dimethyl-1,3-dioxolan-4-ylmethoxy)-benzoic acid

3.52 g (82%) of the title compound was obtained as a white solid from 2-chloro-4-(2,2-dimethyl-1,3-dioxolan-4-ylmethoxy)-benzoic acid methyl ester (3.69 g) obtained in Example (277b) in the same manner as in Example (248d).
$^1$H NMR(400MHz,$CDCl_3$):δ(ppm)=8.04(1H, d, J=8.6Hz), 7.03(1H, s), 6.88(1H, d, J=8.6Hz), 4.53-4.47(1H, m), 4.19(1H, dd, J=8.2 and 7.0Hz), 4.10(1H, dd, J=9.3 and 5.5Hz), 4.02(1H, dd, J=10.0 and 5.7Hz), 3.91(1H, dd, J=8.6 and 5.8Hz), 3.50(1H, s), 1.47(3H, s), 1.41(3H, s).

(277d) 4-{4-[3-(2-methoxy-5-methyl-phenyl)-ureido]-phenyl}-piperazine-1-carboxylic acid [2-chloro-4-(2,3-dihydroxy-propoxy)-phenyl]-amide

1.06 g (85%) of urea compound was obtained as a beige solid from 1-(2-methoxy-5-methyl-phenyl)-3-(4-piperazin-1-yl-phenyl)-urea (681 mg) obtained in Example (47a) and 2-chloro-4-(2,2-dimethyl-1,3-dioxolan-4-ylmethoxy)-benzoic acid (573 mg) obtained in Example (277c) in the same manner as in Example (248e). This urea compound was deprotected in the same manner as in Example (275d) and 807 mg (81 %) of the title compound was obtained as a white solid.
$^1$H NMR(400MHz,DMSO-d6):δ(ppm)==9.09(1H, s), 8.22(1H, s), 8.06(1H, s), 7.99(1H, s), 7.33(2H, d, J=9.0Hz), 7.30 (1H, d, J=9.0Hz), 7.05(1H, d, J=2.7Hz), 6.94(2H, d, J=7.8Hz), 6.89(2H, dd, J=8.8 and 2.9Hz), 6.73(1H, d, J=8.2Hz), 4.97(1H, d, J=5.4Hz), 4.68(1H, t, J=5.6Hz), 4.01(1H, dd, J=9.4 and 3.5Hz), 3.87(1H, dd, J=9.8 and 6.7Hz), 3.84(3H, s), 3.78(1H, dd, J=10.7 and 5.7Hz), 3.58(4H, brs), 3.44(2H, t, J=5.6Hz), 3.08(4H, brs), 2.23(3H, s).
MS(ES$^+$) m/z:584 (M + H)$^+$.
Melting point: 134-136˚C.

(Example 278) 4- {4-[3-(2-fluoro-phenyl)-ureido]-phenyl}-piperazine-1-carboxylic acid [2-chloro-4-(2,3-dihydroxy-propoxy)-phenyl]-amide (Compound No. 1-572)

401 mg (67%) of urea compound was obtained as a white solid from 1-(2-fluoro-phenyl)-3-(4-piperazin-1-yl-phenyl)-urea (314 mg) obtained in Example (125a) and 2-chloro-4-(2,2-dimethyl-1,3-dioxolan-4-ylmethoxy)-benzoic acid (286 mg) obtained in Example (277c) in the same manner as in Example (248e). This urea compound (401 mg) was deprotected in the same manner as in Example (275d) and 374 mg (quantitative yield) of the title compound was obtained as a light brown solid.
$^1$H NMR(400MHz,DMSO-d6):δ(ppm)=8.97(1H, s), 8.51(1H, d, J=2.3Hz), 8.24(1H, s), 8.17(1H, dd, J=8.2 and 8.2Hz), 7.34(2H, d, J=8.6Hz), 7.30(1H, d, J=8.6Hz), 7.23(1H, dd, J=11.8 and 8.2Hz), 7.13(1H, dd, J=7.6 and 7.6Hz), 7.05(1H, d, J=2.7Hz), 7.03-6.97(1H, m), 6.96(2H, d, J=8.6Hz), 6.89(1H, dd, J=8.8 and 3.0Hz), 4.99(1H, d, J=5.1Hz), 4.70(1H, t, J=5.4Hz), 4.11(1H, dd, J=10.6 and 5.0Hz), 4.01(1H, dd, J=10.0 and 4.1Hz), 3.87(1H, dd, J=9.8 and 6.3Hz), 3.78(1H, dd, J=10.1 and 5.1Hz), 3.59(2H, t, J=2.1Hz), 3.44(4H, t, J=5.6Hz), 3.08(4H, t, J=4.7Hz). MS(ES$^+$) m/z:558 (M + H)$^+$.
Melting point: 189-190˚C.

(Example 279) 4-{5-[3-(5-fluoro-2-methoxy-phenyl)-ureido]-pyridin-2-yl}-piperazine-1-carboxylic acid [4-(2,3-dihydroxy-propoxy)-2-trifluoromethyl-phenyl]-amide (Compound No. 1-635)

149 mg (45%) of urea compound was obtained as a white solid from 1-(5-fluoro-2-methoxy-phenyl)-3-(6-piperazin-1-yl-pyridin-3-yl)-urea (173 mg) obtained in Example (148a) and 4-(2,2-dimethyl-1,3-dioxolan-4-ylmethoxy)-2-trifluoromethyl-phenylamine (146 mg) obtained in Example (275c) in the same manner as in Example 170. This urea compound was deprotected in the same manner as in Example (275d) and 112 mg (90%) of the title compound was obtained as a white solid.
$^1$H NMR(400MHz,DMSO-d6):δ(ppm)=9.23(1H, s), 8.40(1H, s), 8.23(1H, s), 8.17(1H, d, J=2.6Hz), 8.01(1H, dd, J=11.9 and 3.0Hz), 7.76(1H, dd, J=9.0 and 2.6Hz), 7.32(1H, d, J=8.8Hz), 7.22(1H, d, J=8.8Hz), 7.18(1H, s), 7.01(1H, dd, J=8.6 and 5.5Hz), 6.90(1H, d, J=9.0Hz), 6.74(1H, ddd, J=8.8, 8.6 and 3.0Hz), 5.14-4.92(1H, m), 4.82-4.64(1H, m), 4.11-4.00 (2H, m), 3.98-3.91(1H, m), 3.98(3H, s), 3.84-3.77(1H, m), 3.57-3.50(4H, m), 3.49-3.41(5H, m).
MS(ES$^+$) m/z:623 (M + H)$^+$.
Melting point: 195-195˚C.

(Example 280) 6-[3-(2-methoxy-5-methyl-phenyl)-ureido]-3,4-dihydro-1H-isoquinoline-2-carboxylic acid [2-chloro-4-(2,3-dihydroxy-propoxy)-phenyl]-amide (Compound No. 3-84)

140 mg (94%) of urea compound was obtained as a yellow brown solid from 1-(2-methoxy-5-methyl-phenyl)-3-(1,2,3,4-tetrahydro-isoquinolin-6-yl)-urea (87 mg) obtained in Example (202b) and 2-chloro-4-(2,2-dimethyl-1,3-dioxolan-4-ylmethoxy)-benzoic acid (72 mg) obtained in Example (277c) in the same manner as in Example (248e). This urea compound (140 mg) was deprotected in the same manner as in Example (275d) and 95 mg (73%) of the title compound was obtained as a white solid.
$^1$H NMR(400MHz,DMSO-d6):δ(ppm)=2.22(3H, s), 2.82(2H, brs), 3.42(2H, t, J=5.5Hz), 3.66(2H, t, J=6.2Hz), 3.83(3H, s), 3.89-3.72(2H, m), 3.99(1H, dd, J=9.6 and 4.1Hz), 4.54(2H, s), 4.66(1H, t, J=6.0Hz), 4.95(1H, d, J=5.1Hz), 6.72(1H, d, J=8.3Hz), 6.90-6.84(2H, m), 7.08-7.01(2H, m), 7.18(1H, d, J=7.9Hz), 7.29(1H, d, J=8.6Hz), 7.36(1H, s), 7.97(1H, brs), 8.14-8.10(2H, m), 9.22(1H, s).
MS(APCI) m/z:555, 557 (M + H)$^+$.
Melting point: 119-121˚C.

(Example 281)4-{4-[3-(2-methoxy-5-methyl-phenyl)-ureido]-phenyl}-3,6-dihydro-2H-pyridine-1-carboxylic acid [2-chloro-4-(2,3-dihydroxy-propoxy)-phenyl]-amide (Compound No. 5-34)

82 mg (60%) of urea compound was obtained from 1-(2-methoxy-5-methylphenyl)-3-[4-(1,2,3,6-tetrahydro-pyridin-4-yl)-phenyl]-urea (74 mg) obtained in Example (230b) and 2-chloro-4-(2,2-dimethyl-1,3-dioxolan-4-ylmethoxy)-benzoic acid (63 mg) obtained in Example (277c) in the same manner as in Example (248e). This urea compound (82 mg) was deprotected in the same manner as in Example (275d) and 20 mg (26%) of the title compound was obtained as an orange white solid. $^1$H NMR(400MHz,DMSO-d6):δ(ppm)=2.23(3H, s), 2.61-2.45(2H, m), 3.64(2H, t, J=5.5Hz), 3.80-3.74(1H, m), 3.90-3.81(2H, m), 3.83(3H, s), 4.00(2H, dd, J=10.4 and 4.1Hz), 4.11(2H, s), 6.13(1H, brs), 6.73(1H, d, J=8.3Hz), 6.90-6.85(2H, m), 7.03(1H, d, J=2.8Hz), 7.30(1H, d, J=9.0Hz), 7.38(2H, d, J=8.6Hz), 7.43(2H, d, J=9.0Hz), 7.97(1H, s), 8.10(1H, s), 8.15(1H, s), 9.33(1H, s).
MS(APCI) m/z:581, 583 (M + H)$^+$.
Melting point: 125-128˚C.

(Example 282) 4-{4-[3-(2-methoxy-5-methyl-phenyl)-ureido]-phenyl}-3,6-dihydro-2H-pyridine-1-carboxylic acid [4-(2,3-dihydroxy-propoxy)-2-trifluoromethylphenyl]-amide (Compound No. 5-54)

95 mg (66%) of urea compound was obtained from 1-(2-methoxy-5-methylphenyl)-3-[4-(1,2,3,6-tetrahydro-pyridin-4-yl)-phenyl]-urea (74 mg) obtained in Example (230b) and 4-(2,2-dimethyl-1,3-dioxolan-4-ylmethoxy)-2-trifluoromethyl-phenylamine (64 mg) obtained in Example (275c) in the same manner as in Example 170. This urea compound was deprotected in the same manner as in Example (275d) and 11 mg (12%) of the title compound was obtained as a yellow brown solid. $^1$H NMR(400MHz,DMSO-d6):δ(ppm)=2.23(3H, s), 2.57-2.39(2H, m), 3.62(2H, t, J=5.7Hz), 3.82-3.75 (1H, m), 3.83(3H, s), 3.92(2H, dd, J=11.8 and 5.8Hz), 4.12-4.03(4H, m), 6.13(1H, s), 6.73(1H, d, J=9.4Hz), 6.88(1H, d, J=8.3Hz), 7.26-7.14(2H, m), 7.30(1H, d, J=8.7Hz), 7.38(2H, d, J=9.0Hz), 7.43(2H, d, J=8.2Hz), 7.97(1H, s), 8.10(1H, s), 8.15(1H, s), 9.33(1H, s).
MS(APCI) m/z:615 $(M + H)^+$.
Melting point: 135-137˚C.

(Example 283) 4-{4-[3-(2-methoxy-5-methyl-phenyl)-ureido]-phenyl}-piperidine-1-carboxylic acid [2-chloro-4-(2,3-dihydroxy-propoxy)-phenyl]-amide (Compound No. 4-34)

90 mg (72%) of urea compound was obtained from 1-(2-methoxy-5-methylphenyl)-3-(4-piperidin-4-yl-phenyl)-urea (68 mg) obtained in Example (228e) and 2-chloro-4-(2,2-dimethyl-1,3-dioxolan-4-ylmethoxy)-benzoic acid (57 mg) obtained in Example (277c) in the same manner as in Example (248e). This urea compound (90 mg) was deprotected in the same manner as in Example (275d) and 66 mg (78%) of the title compound was obtained as a white solid.
$^1$H NMR(400MHz,DMSO-d6):δ(ppm)=1.61-1.47(2H, m), 1.77(2H, d, J=11.8Hz), 2.22(3H, s), 2.73-2.63(1H, m), 2.87(2H, t, J=11.4Hz), 3.43(2H, t, J=5.1Hz), 3.83(3H, s), 3.90-3.73(2H, m), 4.04-3.96(1H, m), 4.20(2H, d, J=12.5Hz), 4.67(1H, t, J=5.3Hz), 4.96(1H, d, J=4.7Hz), 6.72(1H, dd, J=8.4 and 1.8Hz), 6.85(1H, s), 6.87(1H, s), 7.02(1H, d, J=2.7Hz), 7.14 (2H, d, J=8.2Hz), 7.28(1H, d, J=9.0Hz), 7.37(2H, d, J=8.2Hz), 7.96(1H, s), 8.08(1H, s), 8.11(1H, s), 9.21(1H, s).
MS(APCI) m/z:583, 585 $(M + H)^+$.
Melting point: 179-181˚C.

(Example 284) 6-[3-(2-methoxy-phenyl)-ureido]-3,4-dihydro-1H-isoquinoline-2-carboxylic acid [2-chloro-4-(2,3-dihydroxy-propoxy)-phenyl]-amide (Compound No. 3-82)

43 mg (37%) of urea compound was obtained from 1-(2-methoxy-phenyl)-3-(1,2,3,4-tetrahydro-isoquinolin-6-yl)-urea (60 mg) obtained in Example (265b) and 2-chloro-4-(2,2-dimethyl-1,3-dioxolan-4-ylmethoxy)-benzoic acid (57 mg) obtained in Example (277c) in the same manner as in Example (248e). This urea compound (43 mg) was deprotected in the same manner as in Example (275d) and 36 mg (90%) of the title compound was obtained as a white solid.
$^1$H NMR(400MHz,DMSO-d6):δ(ppm)=2.82(2H, t, J=6.2Hz), 3.42(2H, t, J=5.6Hz), 3.66(2H, t, J=5.6Hz), 3.76(1H, dd, J=9.6 and 5.7Hz), 3.87(3H, s), 3.89-3.83(1H, m), 3.99(1H, dd, J=10.2 and 4.3Hz), 4.54(2H, s), 4.66(1H, t, J=5.7Hz), 4.95(1H, d, J=5.1Hz), 6.90-6.84(2H, m), 6.96-6.90(1H, m), 7.00(1H, dd, J=8.0 and 1.3Hz), 7.02(1H, d, J=2.7Hz), 7.06 (1H, d, J=8.2Hz), 7.20(1H, dd, J=8.4 and 2.1Hz), 7.29(1H, d, J=8.6Hz), 7.34(1H, d, J=1.9Hz), 8.12(1H, s), 8.14-8.07 (1H, m), 8.18(1H, s), 9.23(1H, s).
MS(APCI) m/z:541, 543 $(M + H)^+$.
Melting point: 107-111˚C.

(Example 285) 6-[3-(3,5-dimethoxy-phenyl)-ureido]-3,4-dihydro-1H-isoquinoline-2-carboxylic acid [2-chloro-4-(2,3-dihydroxy-propoxy)-phenyl]-amide (Compound No. 3-85)

38 mg (31%) of a urea compound was obtained from 1-(3,5-dimethoxyphenyl)-3-(1,2,3,4-tetrahydro-isoquinolin-6-yl)-urea (65 mg) obtained in Example (266b) and 2-chloro-4-(2,2-dimethyl-1,3-dioxolan-4-ylmethoxy)-benzoic acid (57 mg) obtained in Example (277c) in the same manner as in Example (248e). This urea compound (38 mg) was deprotected in the same manner as in Example (275d) and 32 mg (90%) of the title compound was obtained as a white solid.
$^1$H NMR(400MHz,DMSO-d6):δ(ppm)=2.82(2H, brs), 3.42(2H, t, J=5.6Hz), 3.71(6H, s), 3.79-3.62(3H, m), 3.85(1H, dd, J=10.6 and 6.2Hz), 3.99(1H, dd, J=10.2 and 4.3Hz), 4.54(2H, brs), 4.66(1H, t, J=5.7Hz), 4.95(1H, d, J=5.8Hz), 6.12(1H, brs), 6.66(2H, d, J=2.0Hz), 6.87(1H, dd, J=7.8 and 1.9Hz), 7.02(1H, d, J=1.9Hz), 7.05(1H, brd, J=7.4Hz), 7.19(1H, brd, J=9.4Hz), 7.29(1H, d, J=9.8Hz), 7.32(1H, brs), 8.12(1H, s), 8.55(1H, s), 8.63(1H, s).
MS(APCI) m/z:571, 573 $(M + H)^+$.
Melting point: 108-110˚C.

(Example286)4-{4-[3-(2-methoxy-phenyl)-ureido]-phenyl}-3,6-dihydro-2H-pyridine-1-carboxylicacid[2-chloro-4-(2,3-dihydroxy-propoxy)-phenyl]-amide (Compound No. 5-32)

69 mg (57%) of urea compound was obtained from 1-(2-methoxy-phenyl)-3-[4-(1,2,3,6-tetrahydro-pyridin-4-yl)-phenyl]-urea (65 mg) obtained in Example (258b) and 2-chloro-4-(2,2-dimethyl-1,3-dioxolan-4-ylmethoxy)-benzoic acid (57 mg) obtained in Example (277c) in the same manner as in Example (248e). This urea compound (69 mg) was deprotected in the same manner as in Example (275d) and 60 mg (93%) of the title compound was obtained as a pale yellow solid.
$^1$H NMR(400MHz,DMSO-d6):δ(ppm)=2.42-2.59(2H, m), 3.43(2H, t, J=5.6Hz), 3.67-3.59(2H, m), 3.80-3.73(1H, m), 3.87 (3H, s), 3.89-3.83(1H, m), 4.00(1H, dd, J=9. and 3.9Hz), 4.11(2H, brs), 4.67(1H, t, J=5.6Hz), 4.96(1H, d, J=5.1Hz), 6.13 (1H, brs), 6.90-6.85(2H, m), 6.96-6.90(1H, m), 7.00(1H, dd, J=7.8 and 1.6Hz), 7.03(1H, d, J=3.1Hz), 7.30(1H, d, J=9.0Hz), 7.38(2H, d, J=9.0Hz), 7.43(2H, d, J=9.0Hz), 8.14-8.09(2H, m), 8.21(1H, s), 9.34(1H, s).
MS(APCI) m/z:567, 569 $(M + H)^+$.
Melting point: 103-107˚C.

(Example 287) 4-{4-[3-(3,5-dimethoxy-phenyl)-ureido]-phenyl}-3,6-dihydro-2H-pyridine-1-carboxylic acid [2-chloro-4-(2,3-dihydroxy-propoxy)-phenyl]-amide (Compound No. 5-35)

27 mg (21%) of urea compound was obtained from 1-(3,5-dimethoxyphenyl)-3-[4-(1,2,3,6-tetrahydro-pyridin-4-yl)-phenyl]-urea (71 mg) obtained in Example (259b) and 2-chloro-4-(2,2-dimethyl-1,3-dioxolan-4-ylmethoxy)-benzoic acid (57 mg) obtained in Example (277c) in the same manner as in Example (248e). This urea compound (27 mg) was deprotected in the same manner as in Example (275d) and 23 mg (91%) of the title compound was obtained as a white solid.
$^1$H NMR(400MHz,DMSO-d6):δ(ppm)= 2.42-2.55 (2H, m), 3.42(2H, t, J=5.6Hz), 3.64(2H, t, J=5.5Hz), 3.71(6H, s), 3.79-3.74(1H, m), 3.86(1H, dd, J=9.6 and 6.4Hz), 4.00(1H, dd, J=9.8 and 3.5Hz), 4.11(2H, brd, J=3.1Hz), 4.67(1H, t, J=5.6Hz), 4.96(1H, d, J=5.1Hz), 6.16-6.10(2H, m), 6.66(2H, d, J=2.4Hz), 6.87(1H, dd, J=8.8 and 2.9Hz), 7.03(1H, d, J=2.7Hz), 7.30(1H, d, J=9.0Hz), 7.38(2H, d, J=8.6Hz), 7.42(2H, d, J=9.0Hz), 8.10(1H, s), 8.66(1H, s), 8.67(1H, s).
MS(APCI) m/z:597, 599 $(M + H)^+$.
Melting point: 108-111˚C.

(Example 288) 4-{4-[3-(2-methoxy-phenyl)-ureido]-phenyl}-piperidine-1-carboxylic acid [2-chloro-4-(2,3-dihydroxy-propoxy)-phenyl]-amide (Compound No. 4-32)

59 mg (48%) of urea compound was obtained from 1-(2-methoxy-phenyl)-3-(4-piperidin-4-yl-phenyl)-urea (65 mg) obtained in Example (261b) and 2-chloro-4-(2,2-dimethyl-1,3-dioxolan-4-ylmethoxy)-benzoic acid (57 mg) obtained in Example (277c) in the same manner as in Example (248e). This urea compound (59 mg) was deprotected in the same manner as in Example (275d) and 49 mg (89%) of the title compound was obtained as a white solid.
$^1$H NMR(400MHz,DMSO-d6):δ(ppm)=1.61-1.47(2H, m), 1.81-1.72(2H, m), 2.73-2.61(1H, m), 2.87(2H, brt, J=12.9Hz), 3.43(2H, t, J=5.6Hz), 3.80-3.73(1H, m), 3.87(3H, s), 3.89-3.82(1H, m), 4.00(1H, dd, J=9.8 and 3.9Hz), 4.19(2H, brd, J=12.9Hz), 4.67(1H, t, J=5.7Hz), 4.96(1H, d, J=5.1Hz), 6.89-6.84(2H, m), 6.95-6.90(1H, m), 7.00(1H, dd, J=8.2 and 1.6Hz), 7.02(1H, d, J=2.7Hz), 7.14(2H, d, J=8.6Hz), 7.28(1H, d, J=8.6Hz), 7.37(2H, d, J=8.6Hz), 8.08(1H, s), 8.11(1H, dd, J=7.9 and 1.5Hz), 8.17(1H, s), 9.22(1H, s).
MS(APCI) m/z:569, 571 $(M + H)^+$.
Melting point: 158-161˚C.

(Example 289) 4- {4-[3-(3,5-dimethoxy-phenyl)-ureido]-phenyl}-piperidine-1-carboxylic acid [2-chloro-4-(2,3-dihydroxy-propoxy)-phenyl]-amide (Compound No. 4-35)

47 mg (37%) of urea compound was obtained from 1-(3,5-dimethoxyphenyl)-3-(4-piperidin-4-yl-phenyl)-urea (71 mg) obtained in Example (262b) and 2-chloro-4-(2,2-dimethyl-1,3-dioxolan-4-ylmethoxy)-benzoic acid (57 mg) obtained in Example (277c) in the same manner as in Example (248e). This urea compound (47 mg) was deprotected in the same manner as in Example (275d) and 33 mg (75%) of the title compound was obtained as a white solid.
$^1$H NMR(400MHz,DMSO-d6):δ(ppm)=1.61-1.47(2H, m), 1.81-1.71(2H, m), 2.74-2.61(1H, m), 2.87(2H, brt, J=11.6Hz), 3.43(2H, t, J=5.6Hz), 3.70(6H, s), 3.79-3.73(1H, m), 3.86(1H, dd, J=10.0 and 6.0Hz), 4.00(1H, dd, J=9.8 and 3.9Hz), 4.19(2H, brd, J=12.2Hz), 4.67(1H, t, J=5.6Hz), 4.96(1H, d, J=5.1Hz), 6.12(1H, dd, J=2.1Hz and 2.1Hz), 6.65(2H, d, J=2.3Hz), 6.87(1H, dd, J=8.6 and 2.7Hz), 7.02(1H, d, J=2.7Hz), 7.14(2H, d, J=8.6Hz), 7.28(1H, d, J=9.0Hz), 7.35(2H, d, J=8.6Hz), 8.08(1H, s), 8.54(1H, s), 8.62(1H, s).
MS(APCI) m/z:599, 601 $(M + H)^+$.

Melting point: 105-108˚C.

(Example 290) 6-[3-(2-fluoro-phenyl)-ureido]-3,4-dihydro-1H-isoquinoline-2-carboxylic acid [2-chloro-4-(2,3-dihydroxy-propoxy)-phenyl]-amide (Compound No. 3-81)

A urea compound was obtained from 1-(2-fluoro-phenyl)-3-(1,2,3,4-tetrahydro-isoquinolin-6-yl)-urea (57 mg) obtained in Example (267b) and 2-chloro-4-(2,2-dimethyl-1,3-dioxolan-4-ylmethoxy)-benzoic acid (57 mg) obtained in Example (277c) in the same manner as in Example (248e). This urea compound was deprotected in the same manner as in Example (275d) and 50 mg (two steps, 47%) of the title compound was obtained as a white solid.
$^1$H NMR(400MHz,DMSO-d6):δ(ppm)=2.84(2H, t, J=5.0Hz), 3.44(2H, t, J=5.2Hz), 3.68(2H, t, J=5.6Hz), 3.81-3.73(1H, m), 3.91-3.83(1H, m), 4.04-3.96(1H, m), 4.57(2H, s), 4.68(1H, t, J=5.3Hz), 4.97(1H, d, J=5.1Hz), 6.95-6.86(1H, m), 7.18-6.97(4H, m), 7.28-7.21(2H, m), 7.32(1H, d, J=9.0Hz), 7.36(1H, brs), 8.21-8.12(2H, m), 8.54(1H, s), 9.02(1H, s).
MS(APCI) m/z:529, 531 $(M + H)^+$.
Melting point: 164-167˚C.

(Example 291) 6-[3-(3-ethoxy-phenyl)-ureido]-3,4-dihydro-1H-isoquinoline-2-carboxylic acid [2-chloro-4-(2,3-dihydroxy-propoxy)-phenyl]-amide (Compound No. 3-83)

(291a) 6-[3-(3-ethoxy-phenyl)-ureido]-3,4-dihydrido-1H-isoquinoline-2-carboxylic acid tert-butyl ester

727 mg (quantitative yield) of the title compound was obtained as a white solid from 6-amino-3,4-dihydro-1H-isoquinoline-2-carboxylic acid tert-butyl ester (400 mg) and 3-ethoxyphenyl isocyanate (260 μl) in the same manner as in Example (202a).
MS(APCI) m/z:412 $(M + H)^+$.

(291b) 1-(3-ethoxy-phenyl)-3-(1,2,3,4-tetrahydro-isoquinolin-6-yl)-urea

536 mg (two steps, quantitative yield) of the title compound was obtained as a pale yellow solid from 6-[3-(3-ethoxy-phenyl)-ureido]-3,4-dihydrido-1H-isoquinoline-2-carboxylic acid tert-butyl ester (727 mg) obtained in Example (291a) in the same manner as in Example (202b).
MS(APCI) m/z:312 $(M + H)^+$.

(291c) 6-[3-(3-ethoxy-phenyl)-ureido]-3,4-dihydro-1H-isoquinoline-2-carboxylic acid [2-chloro-4-(2,3-dihydroxy-propoxy)-phenyl]-amide

A urea compound was obtained from 1-(3-ethoxy-phenyl)-3-(1,2,3,4-tetrahydro-isoquinolin-6-yl)-urea (62 mg) obtained in Example (291b) and 2-chloro-4-(2,2-dimethyl-1,3-dioxolan-4-ylmethoxy)-benzoic acid (57 mg) obtained in Example (277c) in the same manner as in Example (248e). This urea compound was deprotected in the same manner as in Example (275d) and 34 mg (two steps, 34%) of the title compound was obtained as a pale grey white solid.
$^1$H NMR(400MHz,DMSO-d6):δ(ppm)=1.33(3H, t, J=6.7Hz), 2.83(2H, brs), 3.44(2H, brs), 3.67(2H, brs), 3.82-3.74(1H, m), 3.90-3.83(1H, m), 4.05-3.95(3H, m), 4.56(2H, s), 4.68(1H, brs), 4.97(1H, brs), 6.54(1H, brd, J=6.3Hz), 6.95-6.86 (2H, m), 7.11-7.03(2H, m), 7.25-7.13(3H, m), 7.32(1H, brd, J=10.2Hz), 7.36(1H, s), 8.15(1H, s), 8.60(1H, s), 8.65(1H, s).
MS(APCI) m/z:555, 557 $(M + H)^+$.
Melting point: 134-139˚C.

(Example 292) 4-{4-[3-(2-fluoro-phenyl)-ureido]-phenyl}-piperidine-l-carboxylic acid [2-chloro-4-(2,3-dihydroxy-propoxy)-phenyl]-amide (Compound No. 4-31)

A urea compound was obtained from 1-(2-fluoro-phenyl)-3-(4-piperidin-4-yl-phenyl)-urea (62 mg) obtained in Example (263b) and 2-chloro-4-(2,2-dimethyl-1,3-dioxolan-4-ylmethoxy)-benzoic acid (57 mg) obtained in Example (277c) in the same manner as in Example (248e). This urea compound was deprotected in the same manner as in Example (275d) and 57 mg (two steps, 51 %) of the title compound was obtained as a white solid.
$^1$H NMR(400MHz,DMSO-d6):δ(ppm)=1.59-1.45(2H, m), 1.79-1.70(2H, m), 2.72-2.62(1H, m), 2.85(2H, brt, J=11.8Hz), 3.41(2H, t, J=5.3Hz), 3.79-3.71(1H, m), 3.85(1H, dd, J=9.8 and 6.3Hz), 3.98(1H, dd, J=10.4 and 3.7Hz), 4.19(2H, brd, J=12.9Hz), 4.66(1H, t, J=5.0Hz), 4.95(1H, d, J=5.4Hz), 6.92-6.83(1H, m), 7.05-6.94(2H, m), 7.25-7.08(4H, m), 7.28(1H, d, J=9.0Hz), 7.37(2H, d, J=8.6Hz), 8.09(1H, s), 8.14(1H, dd, J=8.6, 8.6Hz), 8.49(1H, s), 8.99(1H, s).
MS(APCI) m/z:557, 559 $(M + H)^+$.
Melting point: 170-175˚C.

(Example 293) 6-[3-(2-methoxy-5-methyl-phenyl)-ureido]-3,4-dihydro-1H-isoquinoline-2-carboxylic acid [4-(2,3-dihydroxy-propoxy)-2-trifluoromethylphenyl]-amide (Compound No. 3-104)

73 mg (30%) of urea compound was obtained as a white solid from 1-(2-methoxy-5-methyl-phenyl)-3-(1,2,3,4-tetrahydro-isoquinolin-6-yl)-urea (122 mg) obtained in Example (202b) and 4-(2,2-dimethyl-1,3-dioxolan-4-ylmethoxy)-2-trifluoromethyl-phenylamine (126 mg) obtained in Example (275c) in the same manner as in Example 170. This urea compound was deprotected in the same manner as in Example (275d) and 37 mg (56%) of the title compound was obtained as a white solid.
$^1$H NMR(400MHz,DMSO-d6):δ(ppm)=9.23(1H, s), 8.13(2H, s), 7.97(1H, s), 7.36(1H, s), 7.30(1H, d, J=9.0Hz), 7.21-7.14 (3H, m), 7.04(1H, d, J=8.2Hz), 6.87(1H, d, J=8.2Hz), 6.73(1H, d, J=8.6Hz), 4.99(1H, d, J=5.1Hz), 4.69(1H, t, J=5.6Hz), 4.52(2H, s), 4.07(1H, dd, J=10.4 and 4.1Hz), 3.92(1H, dd, J=10.0 and 8.4Hz), 3.83(3H, s), 3.81-3.76(1H, m), 3.64(2H, t, J=5.9Hz), 3.44(2H, t, J=5.7Hz), 2.80(2H, t, J=4.5Hz), 2.23(3H, s).
MS(ES$^+$) m/z:589 (M + H)$^+$.
Melting point: 174-176˚C.

(Example 294) 4- {4-[3-(2-methoxy-5-methyl-phenyl)-ureido]-phenyl}-piperazine-1-carboxylic acid (3-methyl-pyridin-2-yl)-amide (Compound No. 1-531) (294a) (3-methyl-pyridin-2-yl)-thiocarbamic acid methyl ester

n-Butyl lithium (35 mL, 2.6M hexane solution) was added to a solution of 2-aminopicoline (4.41 g) and 1,1,1,3,3,3-hexamethyldisilazane (9.5 mL) in anhydrous tetrahydrofuran (35 mL) at -78˚C under a nitrogen atmosphere. After 30 minutes, a solution of S,S-dimethyl dithiocarbonate (5 g) in anhydrous tetrahydrofuran (10 mL) was added to the reaction mixture. The reaction mixture was warmed to room temperature slowly and stirred for three days and poured into water, followed by extraction with dichloromethane (twice). The combined organic layers were washed with saturated brine and dried over sodium sulfate and concentrated. The residue was purified by column chromatography (dichloromethane: ethyl acetate 4:1) and 1.81 g (24%) of the title compound was obtained as a light green oil.
$^1$H NMR(400MHz,$CDCl_3$):δ(ppm)=8.49(1H, brs), 8.25(1H, d, J=3.5Hz), 7.26(1H, d, J=4.6Hz), 7.06(1H, dd, J=7.4 and 5.1Hz), 2.38(3H, s), 2.28(3H, s).

(294b) 4-{4-[3-(2-methoxy-5-methyl-phenyl)-ureido]-phenyl}-piperazine-1-carboxylic acid (3-methyl-pyridin-2-yl)-amide

A solution of (3-methyl-pyridin-2-yl)-thiocarbamic acid methyl ester (416 mg) obtained in Example (294a) and 1-(2-methoxy-5-methyl-phenyl)-3-(4-piperazin-1-yl-phenyl)-urea (775 mg) obtained in Example (47a) in acetonitrile (20 mL) was heated under reflux for 19 hours. The precipitate was collected by filtration, washed well with acetonitrile and dried under reduced pressure, and 783 mg (73%) of the title compound was obtained as a white solid.
$^1$H NMR(400MHz,DMSO-d6):δ(ppm)=9.06(1H, s), 8.84(1H, s), 8.16(1H, d, J=5.9Hz), 8.04(1H, s), 7.96(1H, d, J=2.0Hz), 7.57(1H, d, J=6.7Hz), 7.31(2H, d, J=9.0Hz), 7.08(1H, dd, J=7.5 and 4.6Hz), 6.92(2H, d, J=9.0Hz), 6.86(1H, d, J=8.2Hz), 6.70(1H, d, J=8.0 Hz), 3.82(3H, s), 3.59(4H, t, J=4.7Hz), 3.06(4H, t, J=4.7Hz), 2.22(3H, s), 2.13(3H, s).
MS(ES$^+$) m/z:475 (M + H)$^+$.
Melting point: 209-211˚C.

(294c) 2-tert-butoxycarbonylamino-3-methylpyridine

A solution of di-tert-butyl dicarbonate (50.0 g) in n-hexane (55 mL) was heated to 58˚C and stirred. To this solution, a solution of 2-aminopicoline (15.5 g) in ethyl acetate (20 mL) was added dropwise. After heated at 58˚C for three hours, it was cooled to room temperature. After addition of n-hexane (30 mL), it was stirred for 1.5 hours. The deposited precipitate was collected by filtration and dried and 25.8 g (86%) of the title compound was obtained as a white solid.
MS(FAB) m/z:209 (M + H)$^+$.

(294d) 4-{4-[3-(2-methoxy-5-methyl-phenyl)-ureido]-phenyl}-piperazine-1-carboxylic acid (3-methyl-pyridin-2-yl)-amide

A solution of 2-tert-butoxycarbonylamino-3-methylpyridine (2.30 g) obtained in Example (294c) and 1-(2-methoxy-5-methyl-phenyl)-3-(4-piperazin-1-yl-phenyl)-urea (3.09 g) obtained in and Example (47a) in anhydrous tetrahydrofuran (100 mL) was heated under reflux for 17 hours. The reaction mixture was concentrated and the obtained gum-like substance was vigorously stirred in acetonitrile. The deposited precipitate was collected by filtration and was recrystallized from acetonitrile and 3.73 g (87%) of the title compound was obtained as a white solid.

(Example 295) 4- {4-[3-(2-fluoro-phenyl)-ureido]-phenyl}-piperazine-1-carboxylic acid (3-methyl-pyridin-2-yl)-amide (Compound No. 1-524)

137 mg (12%) of the title compound was obtained as a white solid from 1-(2-fluoro-phenyl)-3-(4-piperazin-1-yl-phenyl)-urea (802 mg) obtained in Example (125a) and (3-methyl-pyridin-2-yl)-thiocarbamic acid methyl ester (480 mg) obtained in Example (294a) in the same manner as in Example (294b).
$^1$H NMR(400MHz,DMSO-d6):δ(ppm)=8.93(1H, s), 8.84(1H, s), 8.52(1H, s), 8.16(1H, d, J=5.5Hz), 8.12(1H, d, J=7.8Hz), 7.57(1H, d, J=6.7Hz), 7.32(2H, d, J=8.6Hz), 7.20(1H, dd, J=11.1 and 8.4Hz), 7.10(2H, dd, J=15.2 and 7.4Hz), 6.99-6.92 (1H, m), 6.93(2H, d, J=8.6Hz), 3.59(4H, brs), 3.32(4H, brs), 2.12(3H, s).
MS(ES$^+$) m/z:449 (M + H)$^+$.
Melting point: >250˚C (dec).

(Example 296) 4-{4-[3-(5-fluoro-2-methoxy-phenyl)-ureido]-phenyl}-piperazine-1-carboxylic acid (3-methyl-pyridin-2-yl)-amide (Compound No. 1-527)

383 mg (34%) of the title compound was obtained as a white solid from 1-(5-fluoro-2-methoxy-phenyl)-3-(4-piperazin-1-yl-phenyl)-urea (798 mg) obtained in Example (96a) and (3-methyl-pyridin-2-yl)-thiocarbamic acid methyl ester (0.45 g) obtained in Example (294a) in the same manner as in Example (294b).
$^1$H NMR(400MHz,DMSO-d6):δ(ppm)=9.20(1H, s), 8.87(1H, s), 8.33(1H, s), 8.19(1H, d, J=5.0Hz), 8.04(1H, d, J=11.8Hz), 7.60(1H, d, J=7.4Hz), 7.34(2H, d, J=7.5Hz), 7.14-7.09(1H, m), 7.03-6.91(1H, m), 6.96(2H, d, J=7.4Hz), 6.77-6.68(1H, m), 3.87(3H, s), 3.61(4H, brs), 3.08(4H, brs), 2.13(3H, s).
MS(ES$^+$) m/z:479 (M + H)$^+$.
Melting point: 206-208˚C.

(Example 297) 6-[3-(2-methoxy-5-methyl-phenyl)-ureido]-3,4-dihydro-1H-isoquinoline-2-carboxylic acid (3-methyl-pyridin-2-yl)-amide (Compound No. 3-54)

A solution of 1-(2-methoxy-5-methyl-phenyl)-3-(1,2,3,4-tetrahydro-isoquinolin-6-yl)-urea (106 mg, hydrochloride) obtained in Example (202b), triethylamine (0.045 mL) and (3-methyl-pyridin-2-yl)-thiocarbamic acid methyl ester (0.45 g) obtained in Example (294a) in acetonitrile (20 mL) was heated under reflux for 18 hours in the same manner as in Example (294b). The reaction mixture was concentrated and diluted with ethyl acetate and washed with a saturated sodium hydrogen carbonate aqueous solution and saturated brine and dried over sodium sulfate and concentrated. The residue was purified by column chromatography (basic silica gel, dichloromethane:methanol 20:1) and further by preparative TLC (dichloromethane:methanol 20:1) and 76 mg (56%) of the title compound was obtained as a light brown amorphous material.
$^1$H NMR(400MHz,DMSO-d6):δ(ppm)=9.23(1H, s), 8.77(1H, s), 8.16(1H, d, J=4.7Hz), 8.13(1H, s), 7.98(1H, d, J=1.5Hz), 7.57(1H, d, J=7.4Hz), 7.37(1H, d, J=2.0Hz), 7.18(1H, dd, J=8.2 and 2.3Hz), 7.08(1H, dd, J=7.5 and 4.7Hz), 7.05(1H, d, J=8.2Hz), 6.87(1H, d, J=8.2Hz), 6.73(1H, dd, J=8.0 and 1.4Hz), 4.56(2H, s), 3.83(3H, s), 3.68(2H, t, J=5.6Hz), 2.82 (2H, t, J=5.6Hz), 2.23(3H, s), 2.10(3H, s).
MS(ES$^+$) m/z:446 (M + H)$^+$.

(Example 298) 4- {5-[3-(5-fluoro-2-methoxy-phenyl)-ureido]-pyridin-2-yl} - piperazine-1-carboxylic acid (3-methyl-pyridin-2-yl)-amide (Compound No. 1-539)

320 mg (36%) of the title compound was obtained as a white solid from 1-(5-fluoro-2-methoxy-phenyl)-3-(6-piperazin-1-yl-pyridin-3-yl)-urea (632 mg) obtained in Example (148a) and (3-methyl-pyridin-2-yl)-thiocarbamic acid methyl ester (0.45 g) obtained in Example (294a) in the same manner as in Example (294b).
$^1$H NMR(400MHz,DMSO-d6):δ(ppm)=9.18(1H, s), 8.84(1H, s), 8.36(1H, s), 8.16-8.14(1H, m), 8.14(1H, d, J=2.7Hz), 7.99(1H, dd, J=11.3 and 3.2Hz), 7.73(1H, dd, J=9.0 and 2.8Hz), 7.57(1H, d, J=7.5Hz), 7.09(1H, dd, J=7.5 and 5.1Hz), 6.99(1H, dd, J=9.0 and 5.0Hz), 6.88(1H, d, J=9.3Hz), 6.72(1H, ddd, J=8.5, 8.5, 3.3Hz), 3.86(3H, s), 3.57-3.54(4H, m), 3.46-3.43(4H, m), 2.12(3H, s).
MS(ES$^+$) m/z:480 (M + H)$^+$.
Melting point: 196-198˚C.

(Example 299) 4-{5-[3-(5-chloro-2-ethoxy-phenyl)-ureido]-pyridin-2-yl}-piperazine-1-carboxylic acid (3-methyl-pyridin-2-yl)-amide (Compound No. 1-542)

(299a) 4-{5-[3-(5-chloro-2-ethoxy-phenyl)-ureido]-pyridin-2-yl}-piperazine-1-carboxylic acid tert-butyl ester

1.39 g (59%) of the title compound was obtained as a pink crystal from 4-(5-amino-pyridin-2-yl)-piperazine-1-carboxylic acid tert-butyl ester (1.39 g) obtained in Example (38b) and 5-chloro-2-ethoxy benzoic acid (1.00 g) obtained in Example (43a) in the same manner as in Example (42b).
$^1$H NMR(400MHz,DMSO-d6):δ(ppm)=9.29(1H, s), 8.22(1H, d, J=2.4Hz), 8.19(1H, s), 8.17(1H, d, J=2.7Hz), 7.75(1H, dd, J=9.0 and 2.7Hz), 7.02(1H, d, J=8.9Hz), 6.95(1H, dd, J=8.9 and 2.4Hz), 6.86(1H, d, J=9.0Hz), 4.14(2H, q, J=6.9Hz), 3.46-3.36(8H, m), 1.43(9H, s), 1.41(3H, t, J=6.9Hz).
MS(ES$^+$) m/z:476 (M + H)$^+$.
Melting point: 197-198˚C.

(299b) 1-(5-chloro-2-ethoxy-phenyl)-3-(6-piperazin-1-yl-pyridin-3-yl)-urea

513 mg (49%) of the title compound was obtained as a pale pink crystal from 4- {5-[3-(5-chloro-2-ethoxy-phenyl)-ureido]-pyridin-2-yl} -piperazine-1-carboxylic acid tert-butyl ester (1.32 g) obtained in Example (299a) in the same manner as in Example (47a).
MS(ES$^+$) m/z:376 (M + H)$^+$.

(299c) 4- {5-[3-(5-chloro-2-ethoxy-phenyl)-ureido]-pyridin-2-yl}-piperazine-1-carboxylic acid (3-methyl-pyridin-2-yl)-amide

503 mg (53%) of the title compound was obtained as a white solid from 1-(5-chloro-2-ethoxy-phenyl)-3-(6-piperazin-1-yl-pyridin-3-yl)-urea (701 mg) obtained in Example (299b) and (3-methyl-pyridin-2-yl)-thiocarbamic acid methyl ester (1.35 g) obtained in Example (294a) in the same manner as in Example (294b).
$^1$H NMR(400MHz,DMSO-d6):δ(ppm)=9.29(1H, s), 8.84(1H, s), 8.20(1H, d, J=2.3Hz), 8.18-8.15(1H, m), 8.16 (1H, d, J=7.5Hz), 7.74(1H, dd, J=9.0 and 2.7Hz), 7.57(1H, d, J=7.9Hz), 7.09(1H, dd, J=7.5 and 5.1Hz), 7.00(2H, d, J=8.6Hz), 6.94(1H, d, J=2.4Hz), 6.88(1H, d, J=9.0Hz), 4.13(2H, q, J=6.7Hz), 3.58-3.55(4H, m), 3.46-3.44(4H, m), 2.13(3H, s), 1.41(3H, t, J=6.9Hz).
MS(ES$^+$) m/z:510 (M + H)$^+$.
Melting point: 214-215˚C.

(Example 300) 4-{5-[3-(2-ethoxy-5-fluoro-phenyl)-ureido]-pyridin-2-yl}-piperazine-1-carboxylic acid (3-methyl-pyridin-2-yl)-amide (Compound No. 1-540)

(300a) 1-(2-ethoxy-5-fluoro-phenyl)-3-(6-piperazin-1-yl-pyridin-3-yl)-urea

3.14 g (100%) of the title compound was obtained as a white solid from 4-{5-[3-(2-ethoxy-5-fluoro-phenyl)-ureido]-pyridin-2-yl}-piperazine-1-carboxylic acid tert-butyl ester (4.01 g) obtained in Example 46 in the same manner as in Example (47a).
MS(ES$^+$) m/z:360 (M + H)$^+$.

(300b) 4-{5-[3-(2-ethoxy-5-fluoro-phenyl)-ureido]-pyridin-2-yl}-piperazine-1-carboxylic acid (3-methyl-pyridin-2-yl)-amide

64 mg (7%) of the title compound was obtained as a pale red solid from 1-(2-ethoxy-5-fluoro-phenyl)-3-(6-piperazin-l-yl-pyridin-3-yl)-urea (672 mg) obtained in Example (300a) and (3-methyl-pyridin-2-yl)-thiocarbamic acid methyl ester (0.45 g) obtained in Example (294a) in the same manner as in Example (294b).
$^1$H NMR(400MHz,DMSO-d6):8(ppm)=9.27(1H, s), 8.83(1H, s), 8.19(1H, s), 8.15(2H, s), 7.98(1H, d, J=12.1Hz), 7.74 (1H, d, J=9.0Hz), 7.57(1H, d, J=6.7Hz), 7.08(1H, dd, J=6.2 and 6.2Hz), 6.97(1H, dd, J=6.9 and 6.8Hz), 6.88(1H, d, J=8.6Hz), 6.69(1H, dd, J=8.1 and 8.0Hz), 4.10(2H, q, J=7.3Hz), 3.56(4H, brs), 3.44(4H, brs), 2.12(3H, s), 1.39(3H, t, J=6.7Hz).
MS(ES$^+$) m/z:494 (M + H)$^+$.
Melting point: 180-185˚C.

(Example 301) 4-{5-[3-(2-methoxy-5-methyl-phenyl)-ureido]-pyridin-2-yl}-piperazine-1-carboxylic acid (3-methyl-pyridin-2-yl)-amide (Compound No. 1-543)

19 mg (3%) of the title compound was obtained as a white solid from 1-(2-methoxy-5-methyl-phenyl)-3-(6-piperazin-1-yl-pyridin-3-yl)-urea (503 mg) obtained in Example (137a) and (3-methyl-pyridin-2-yl)-thiocarbamic acid methyl ester (0.60 g) obtained in Example (294a) in the same manner as in Example (294b).
$^1$H NMR(400MHz,DMSO-d6):δ(ppm)=9.07(1H, s), 8.83(1H, s), 8.83-8.17(2H, m), 8.09(1H, s), 7.94(1H, s), 7.74(1H, d, J=9.0Hz), 7.57(1H, d, J=8.6Hz), 7.11-7.05(1H, m), 6.87(2H, d, J=8.3Hz), 6.74-6.69(1H, m), 3.82(3H, s), 3.59-3.53(4H, m), 3.46-3.40(4H, m), 2.22(3H, s), 2.12(3H, s).
MS(ES$^+$) m/z:476 (M + H)$^+$.
Melting point: 216-218˚C.

(Example 302) 4-{5-[3-(2-fluoro-phenyl)-ureido]-pyridin-2-yl}-piperazine-1-carboxylic acid (3-methyl-pyridin-2-yl)-amide (Compound No. 1-536)

295 mg (41%) of the title compound was obtained as a pale red solid from 1-(2-fluoro-phenyl)-3-(6-piperazin-1-yl-pyridin-3-yl)-urea (508 mg) obtained in Example (222b) and (3-methyl-pyridin-2-yl)-thiocarbamic acid methyl ester (0.65 g) in the same manner as in Example (294b).
$^1$H NMR(400MHz,DMSO-d6):δ(ppm)=8.84(1H, s), 8.82(1H, s), 8.49(1H, s), 8.16(2H, d, J=2.7Hz), 8.11(1H, dd, J=9.0 and 9.0Hz), 7.73(1H, d, J=8.6Hz), 7.57(1H, d, J=7.5Hz), 7.21(1H, dd, J=10.6 and 10.6Hz), 7.14-7.06(2H, m), 7.01-6.95 (1H, m), 6.88(1H, d, J=8.6Hz), 3.58-3.54(4H, m), 3.47-3.42(4H, m), 2.12(3H, s).
MS(ES$^+$)m/z:450 (M + H)$^+$.
Melting point: 213-215˚C.

(Example 303) 4-{4-[3-(3-methoxy-phenyl)-ureido]-phenyl}-piperazine-1-carboxylic acid (3-methyl-pyridin-2-yl)-amide (Compound No. 1-854)

46 mg (6%) of the title compound was obtained as a white solid from 1-(3-methoxy-phenyl)-3-(4-piperazin-1-yl-phenyl)-urea (503 mg) obtained in Example (214b) and (3-methyl-pyridin-2-yl)-thiocarbamic acid methyl ester (0.65 g) obtained in Example (294a) in the same manner as in Example (294b).
$^1$H NMR(400MHz,DMSO-d6):δ(ppm)=8.84(1H, s), 8.56(1H, s), 8.39(1H, s), 8.16(1H, d, J=4.3Hz), 7.57(1H, d, J=9.0Hz), 7.30(2H, d, J=8.2Hz), 7.19-7.06(3H, m), 6.96-6.87(3H, m), 6.51(1H, d, J=9.8Hz), 3.72(3H, s), 3.62-3.57(4H, m), 3.09-3.02 (4H, m), 2.12(3H, s).
MS(ES$^+$) m/z:461 (M + H)$^+$.
Melting point: 191-193˚C.

(Example 304) 4-{4-[3-(2-ethoxy-phenyl)-ureido]-phenyl}-piperazine-1-carboxylic acid (3-methyl-pyridin-2-yl)-amide (Compound No. 1-526)

556 mg (47%) of the title compound was obtained as a white solid from 1-(2-ethoxy-phenyl)-3-(4-piperazin-1-yl-phenyl)-urea (400 mg) obtained in Example (80a) and (3-methyl-pyridin-2-yl)-thiocarbamic acid methyl ester (0.70 g) obtained in Example (294a) in the same manner as in Example (294b).
$^1$H NMR(400MHz,DMSO-d6):δ(ppm)=9.15(1H, s), 8.84(1H, s), 8.16(1H, d, J=4.7Hz), 8.10(1H, d, J=7.4Hz), 7.95(1H, s), 7.57(1H, d, J=7.5Hz), 7.32(2H, d, J=8.2Hz), 7.09(1H, dd, J=7.3 and 5.3Hz), 6.98-6.83(5H, m), 4.12(2H, q, J=6.6Hz), 3.63-3.58(4H, m), 3.08-3.05(4H, m), 2.13(3H, s), 1.41(3H, t, J=6.8Hz).
MS(ES$^+$) m/z:475 (M + H)$^+$.
Melting point: 110-112˚C.

(Example 305) 4-{4-[3-(3-ethoxy-phenyl)-ureido]-phenyl}-piperazine-1-carboxylic acid (3-methyl-pyridin-2-yl)-amide (Compound No. 1-856)

26 mg (5%) of the title compound was obtained as a white solid from 1-(3-ethoxy-phenyl)-3-(4-piperazin-1-yl-phenyl)-urea (400 mg) obtained in Example (215b) and (3-methyl-pyridin-2-yl)-thiocarbamic acid methyl ester (0.65 g) obtained in Example (294a) in the same manner as in Example (294b).
$^1$H NMR(400MHz,DMSO-d6):δ(ppm)=8.84(1H, s), 8.53(1H, s), 8.39(1H, s), 8.17-8.15(1H, m), 7.57(1H, d, J=9.0Hz), 7.30(2H, d, J=9.0Hz), 7.15-7.08(3H, m), 6.92(2H, d, J=9.0Hz), 6.87(1H, d, J=9.4Hz), 6.50(1H, d, J=9.4Hz), 3.98(2H, q, J=7.2Hz), 3.59(4H, brs), 3.06(4H, brs), 2.12(3H, s), 1.32(3H, t, J=6.9Hz).
MS(ES$^+$) m/z:475 (M + H)$^+$.

Melting point: 171-173˚C.

(Example 306) 4-{4-[3-(3,5-dimethoxy-phenyl)-ureido]-phenyl}-piperazine-1-carboxylic acid (3-methyl-pyridin-2-yl)-amide (Compound No. 1-533)

189 mg (26%) of the title compound was obtained as a white solid from 1-(3,5-dimethoxy-phenyl)-3-(4-piperazin-1-yl-phenyl)-urea (522 mg) obtained in Example (216b) and (3-methyl-pyridin-2-yl)-thiocarbamic acid methyl ester (1.84 g) obtained in Example (294a) in the same manner as in Example (294b).
$^1$H NMR(400MHz,DMSO-d6):δ(ppm)=8.84(1H, s), 8.59(1H, s), 8.41(1H, s), 8.17-8.13(1H, m), 7.57(1H, d, J=6.7Hz), 7.30(2H, d, J=9.0Hz), 7.08(1H, dd, J=7.5 and 4.6Hz), 6.92(2H, d, J=9.0Hz), 6.65(2H, d, J=1.9Hz), 6.10(1H, dd, J=2.3 and 2.3Hz), 3.70(6H, s), 3.59(4H, t, J=4.3Hz), 3.06(4H, t, J=5.1Hz), 2.12(3H, s).
MS(ES$^+$) m/z:491 (M + H)$^+$.
Melting point: 188-190˚C.

(Example 307) 4- {5-[3-(2-ethoxy-5-methyl-phenyl)-ureido]-pyridin-2-yl} - piperazine-1-carboxylic acid (3-methyl-pyridin-2-yl)-amide (Compound No. 1-544)

(307a) 4-{5-[3-(2-ethoxy-5-methyl-phenyl)-ureido]-pyridin-2-yl}-piperazine-1-carboxylic acid tert-butyl ester

960 mg (40%) of the title compound was obtained as a pale pink solid from 4-(5-amino-pyridin-2-yl)-piperazine-1-carboxylic acid tert-butyl ester (1.39 g) obtained in Example (38b) and 2-ethoxy-5-methylaniline (746 mg) obtained in Example (41b) in the same manner as in Example (41c).
$^1$H NMR(400MHz,DMSO-d6):δ(ppm)=9.15 (1H, s), 8.14(1H, d, J=2.7Hz), 7.94(1H, s), 7.94(1H, d, J=2.4Hz), 7.73(1H, dd, J=9.0 and 2.7Hz), 6.85(1H, d, J=8.2Hz), 6.83(1H, d, J=9.0Hz), 6.69(1H, dd, J=8.2 and 2.4Hz), 4.07(2H, q, J=7.1Hz), 3.44-3.35(8H, m), 2.21(3H, s), 1.42(9H, s), 1.38(3H, t, J=7.1Hz)
MS(ES$^+$) m/z:456 (M + H)$^+$.
Melting point: 192-193˚C

(307b) 1-(2-ethoxy-5-methyl-phenyl)-3-(6-piperazin-1-yl-pyridin-3-yl)-urea

500 mg (71%) of the title compound was obtained as a purple solid from 4-{5-[3-(2-ethoxy-5-methyl-phenyl)-ureido]-pyridin-2-yl}-piperazine-1-carboxylic acid tert-butyl ester (900 mg) obtained in Example (307a) in the same manner as in Example (47a).
MS(ES$^+$) m/z:356 (M + H)$^+$.

(307c) 4-{5-[3-(2-ethoxy-5-methyl-phenyl)-ureido]-pyridin-2-yl}-piperazine-1-carboxylic acid (3-methyl-pyridin-2-yl)-amide

109 mg (66%) of the title compound was obtained as a pale lavender-coloured solid from 1-(2-ethoxy-5-methyl-phenyl)-3-(6-piperazin-1-yl-pyridin-3-yl)-urea (121 mg) obtained in Example (307b) and (3-methyl-pyridin-2-yl)-thiocarbamic acid methyl ester (0.40 g) obtained in Example (294a) in the same manner as in Example (294b).
$^1$H NMR(400MHz,DMSO-d6):δ(ppm)=9.19(1H, s), 8.87(1H, s), 8.19(2H, s), 7.98(2H, s), 7.78(1H, d, J=6.6Hz), 7.60(1H, d, J=7.8Hz), 7.11(1H, dd, J=5.3 and 5.3Hz), 6.89(2H, dd, J=7.4 and 7.5Hz), 6.72(1H, d, J=8.2Hz), 4.09(2H, q, J=6.8Hz), 3.61-3.54(4H, m), 3.48-3.39(4H, m), 2.22(3H, s), 2.14(3H, s), 1.39(3H, t, J=7.8Hz).
MS(ES$^+$) m/z:490 (M + H)$^+$.
Melting point: 198-200˚C.

(Example 308) 4- {5-[3-(5-chloro-2-methoxy-phenyl)-ureido]-pyridin-2-yl} - piperazine-1-carboxylic acid (3-methyl-pyridin-2-yl)-amide (Compound No. 1-541)

(308a) 1-(5-chloro-2-methoxy-phenyl)-3-(6-piperazin-1-yl-pyridin-3-yl)-urea

6.90 g (quantitative yield) of the title compound was obtained as a pale pink solid from 4-{5-[3-(5-chloro-2-methoxy-phenyl)-ureido]-pyridin-2-yl}-piperazine-1-carboxylic acid tert-butyl ester (8.81 g) obtained in Example 40 in the same manner as in Example (47a).
MS(ES$^+$) m/z:362 (M + H)$^+$.

(308b) 4-{5-[3-(5-chloro-2-methoxy-phenyl)-ureido]-pyridin-2-yl}-piperazine-1-carboxylic acid (3-methyl-pyridin-2-yl)-amide

72 mg (68%) of the title compound was obtained as a white solid from 1-(5-chloro-2-methoxy-phenyl)-3-(6-piperazin-1-yl-pyridin-3-yl)-urea (81 mg) obtained in Example (308a) and (3-methyl-pyridin-2-yl)-thiocarbamic acid methyl ester (0.40 g) obtained in Example (294a) in the same manner as in Example (294b).
$^1$H NMR(400MHz,DMSO-d6):δ(ppm)=9.21(1H, s), 8.87(1H, s), 8.38(1H, s), 8.23(1H, d, J=2.7Hz), 8.18(2H, s), 7.76(1H, d, J=9.4Hz), 7.60(1H, d, J=7.0Hz), 7.15-7.10(2H, m), 7.05-6.95(1H, m), 6.90(1H, d, J=8.2Hz), 3.89(3H, s), 3.61-3.54 (4H, m), 3.50-3.41(4H, m), 2.13(3H, s).
MS(ES$^+$) m/z:496 (M + H)$^+$.
Melting point: 203-205˚C.

(Example 309) 4-{4-[3-(2-methoxy-phenyl)-ureido]-phenyl}-piperazine-1-carboxylic acid (3-methyl-pyridin-2-yl)-amide (Compound No. 1-525)

134 mg (69%) of the title compound was obtained as a white solid from 1-(2-methoxy-phenyl)-3-(4-piperazin-1-yl-phenyl)-urea (137 mg) obtained in Example (124a) and (3-methyl-pyridin-2-yl)-thiocarbamic acid methyl ester (0.40 g) obtained in Example (294a) in the same manner as in Example (294b).
$^1$H NMR(400MHz,DMSO-d6):δ(ppm)=9.08(1H, s), 8.84(1H, s), 8.16(1H, d, J=5.1Hz), 8.12-8.08(2H, m), 7.57(1H, d, J=6.3Hz), 7.31(2H, d, J=8.6Hz), 7.08(1H, dd, J=7.4 and 5.1Hz), 7.01-6.97(4H, m), 6.94-6.84(1H, m), 3.86(3H, s), 3.62-3.56(4H, m), 3.08-3.02(4H, m), 2.12(3H, s).
MS(ES$^+$) m/z:461 (M + H)$^+$.
Melting point: 207-209˚C.

(Example 310) 4-{4-[3-(5-chloro-2-methoxy-phenyl)-ureido]-phenyl}-piperazine-1-carboxylic acid (3-methyl-pyridin-2-yl)-amide (Compound No. 1-529)

95 mg (53%) of the title compound was obtained as a white solid from 1-(5-chloro-2-methoxy-phenyl)-3-(4-piperazin-1-yl-phenyl)-urea (131 mg) obtained in Example (67a) and (3-methyl-pyridin-2-yl)-thiocarbamic acid methyl ester (0.40 g) obtained in Example (294a) in the same manner as in Example (294b).
$^1$H NMR(400MHz,DMSO-d6):8(ppm)=9.17(1H, s), 8.84(1H, s), 8.29(1H, s), 8.23-8.21(1H, m), 8.17-8.15(1H, m), 7.58 (1H, d, J=7.8Hz), 7.31 (2H, d, J=9.0Hz), 7.11-7.06(1H, m), 7.03-6.98(1H, m), 6.97-6.90(3H, m), 3.87(3H, s), 3.63-3.56 (4H, m), 3.10-3.03(4H, m), 2.12(3H, s).
MS(ES$^+$) m/z:495 (M + H)$^+$.
Melting point: 163-165˚C.

(Example 311) 4-{4-[3-(2-ethoxy-5-methyl-phenyl)-ureido]-phenyl}-piperazine-1-carboxylic acid (3-methyl-pyridin-2-yl)-amide (Compound No. 1-532)

100 mg (60%) of the title compound was obtained as a white solid from 1-(2-ethoxy-5-methylphenyl)-3-(4-piperazin-1-yl-phenyl)-urea (122 mg) obtained in Example (83a) and (3-methyl-pyridin-2-yl)-thiocarbamic acid methyl ester (0.40 g) obtained in Example (294a) in the same manner as in Example (294b).
$^1$H NMR(400MHz,DMSO-d6):δ(ppm)=9.15(1H, s), 8.84(1H, s), 8.16(1H, d, J=4.3Hz), 7.96(1H, s), 7.91(1H, s), 7.57(1H, d, J=6.3Hz), 7.32(2H, d, J=9.0Hz), 7.08(1H, dd, J=7.5 and 4.7Hz), 6.93(2H, d, J=9.0Hz), 6.85(1H, d, J=8.2Hz), 6.68 (1H, d, J=10.2Hz), 4.07(2H, q, J=6.9Hz), 3.59(4H, brs), 3.06(4H, brs), 2.21(3H, s), 2.13(3H, s), 1.38(3H, t, J=6.8Hz).
MS(ES$^+$) m/z:489 (M + H)$^+$.
Melting point: 206-208˚C.

(Example 312) 4-{2-fluoro-4-[3-(2-methoxy-5-methyl-phenyl)-ureido]-phenyl}-piperazine-1-carboxylic acid (3-methyl-pyridin-2-yl)-amide (Compound No. 2-85)

118 mg (72%) of the title compound was obtained as a white solid from 1-(3-fluoro-4-piperazin-1-yl-phenyl)-3-(2-methoxy-5-methyl-phenyl)-urea (120 mg) obtained in Example (253d) and 2-tert-butoxycarbonylamino-3-methyl-pyridine (97 mg) obtained in Example (294c) in the same manner as in Example (294d).
$^1$H NMR(400MHz,DMSO-d6):δ(ppm)=9.32(1H, s), 8.83(1H, s), 8.17(1H, s), 8.11(1H, s), 7.95(1H, s), 7.60-7.54(1H, m), 7.46(1H, d, J=13.3Hz), 7.12-7.05(1H, m), 7.00(2H, s), 6.91-6.83(1H, m), 6.76-6.68(1H, m), 3.83(3H, s), 3.60(4H, brs), 2.94(4H, brs), 2.22(3H, s), 2.13(3H, s).
MS(ES$^+$) m/z:493 (M + H)$^+$.

Melting point: 188-190˚C.

(Example 313) 4- {4-[3-(2-methoxy-5-methyl-phenyl)-ureido]-2-methyl-phenyl}-piperazine-1-carboxylic acid (3-methyl-pyridin-2-yl)-amide (Compound No. 2-83)

51 mg (30%) of the title compound was obtained as a white solid from 1-(2-methoxy-5-methyl-phenyl)-3-(3-methyl-4-piperazin-1-yl-phenyl)-urea (118 mg) obtained in Example (239a) and 2-tert-butoxycarbonylamino-3-methyl-pyridine (89 mg) obtained in Example (294c) in the same manner as in Example (294d).
$^1$H NMR(400MHz,DMSO-d6):δ(ppm)=9.14(1H, s), 8.83(1H, s), 8.19(1H, dd, J=4.7 and 1.5Hz), 8.11(1H, s), 7.99(1H, d, J=1.6Hz), 7.60(1H, d, J=6.6Hz), 7.31(1H, d, J=1.9Hz), 7.22(1H, dd, J=8.4 and 2.6Hz), 7.11(1H, dd, J=7.4 and 4.7Hz), 6.99(1H, d, J=8.6Hz), 6.89(1H, d, J=8.2Hz), 6.73(1H, dd, J=8.2 and 2.3Hz), 3.84(3H, s), 3.59(4H, brs), 2.79(4H, brs), 2.27(3H, s), 2.23(3H, s), 2.15(3H, s)
MS(ES$^+$) m/z:489 (M + H)$^+$.
Melting point: 144-146˚C.

(Example 314) 4-{4-[3-(2-difluoromethoxy-phenyl)-ureido]-phenyl}-piperazine-1-carboxylic acid (3-methyl-pyridin-2-yl)-amide (Compound No. 1-871)

37 mg (25%) of the title compound was obtained as a beige solid from 1-(2-difluoromethoxy-phenyl)-3-(4-piperazin-1-yl-phenyl)-urea (109 mg) obtained in Example (245a) and 2-tert-butoxycarbonylamino-3-methylpyridine (88 mg) obtained in Example (294c) in the same manner as in Example (294d).
$^1$H NMR(400MHz,DMSO-d6):δ(ppm)==9.11(1H, s), 8.84(1H, s), 8.24-8.13(3H, m), 7.57(1H, d, J=7.5Hz), 7.23(1H, t, J=73.2Hz), 7.32(2H, d, J=8.6Hz), 7.23-6.86(6H, m), 3.59(4H, brs), 3.06(4H, brs), 2.12(3H, s).
MS(ES) m/z:497 (M + H)$^+$.
Melting point: 190-192˚C.

(Example 315)4-{4-[3-(2-trifluoromethoxy-phenyl)-ureido]-phenyl}-piperazine-1-carboxylic acid (3-methyl-pyridin-2-yl)-amide (Compound No. 1-893)

38 mg (23%) of the title compound was obtained as a white solid from 1-(2-trifluoromethoxy-phenyl)-3-(4-piperazin-1-yl-phenyl)-urea (120 mg) obtained in Example (240b) and 2-tert-butoxycarbonylamino-3-methylpyridine (89 mg) obtained in Example (294c) in the same manner as in Example (294d).
$^1$H NMR(400MHz,DMSO-d6):δ(ppm)=9.07(1H, s), 8.84(1H, s), 8.37(1H, s), 8.26(1H, dd, J=8.2 and 1.5Hz), 8.16(1H, dd, J=4.8 and 1.8Hz), 7.57(1H, d, J=8.6Hz), 7.35(2H, d, J=7.4Hz), 7.30(2H, d, J=11.0Hz), 7.10-7.03(2H, m), 6.94(2H, d, J=9.0Hz), 3.59(4H, t, J=5.9Hz), 3.07(4H, t, J=5.1Hz), 2.12(3H, s).
MS(ES$^+$) m/z:515 (M + H)$^+$.
Melting point: 112-114˚C.

(Example 316) 4-{4-[3-(2,3-dihydro-1,4-benzodioxin-5-yl)-ureido]-phenyl}-piperazine-1-carboxylic acid (3-methyl-pyridin-2-yl)-amide (Compound No. 1-896)

65 mg (38%) of the title compound was obtained as a white solid from 1-(2,3-dihydro-l,4-benzodioxin-5-yl)-3-(4-piperazin-l-yl-phenyl)-urea (121 mg) obtained in Example (244b) and 2-tert-butoxycarbonylamino-3-methylpyridine (78 mg) obtained in Example (294c) in the same manner as in Example (294d).
$^1$H NMR(400MHz,DMSO-d6):δ(ppm)=9.08(1H, s), 8.87(1H, s), 8.19(1H, d, J=4.3Hz), 8.08(1H, s), 7.70(1H, d, J=8.2Hz), 7.60(1H, d, J=7.4Hz), 7.33(2H, d, J=8.2Hz), 7.11(1H, dd, J=7.6 and 5.2Hz), 6.95(2H, d, J=8.6Hz), 6.74(1H, dd, J=8.4 and 8.4Hz), 6.48(1H, d, J=7.8Hz), 4.40-4.32(2H, m), 4.32-4.22(2H, m), 3.61(4H, brs), 3.07(4H, brs), 2.13(3H, s).
MS(ES$^+$) m/z:489 (M + H)$^+$.
Melting point: 138-140˚C.

(Example 317) 4-{4-[3-(2-ethoxy-5-fluoro-phenyl)-ureido]-phenyl}-piperazine-1-carboxylic acid (3-methyl-pyridin-2-yl)-amide (Compound No. 1-528)

90 mg (51%) of the title compound was obtained as a white solid from 1-(2-ethoxy-5-fluoro-phenyl)-3-(4-piperazin-1-yl-phenyl)-urea (129 mg) obtained in Example (89a) and 2-tert-butoxycarbonylamino-3-methylpyridine (92 mg) obtained in Example (294c) in the same manner as in Example (294d).
$^1$H NMR(400MHz,DMSO-d6):δ(ppm)=9.29(1H, s), 8.88(1H, s), 8.18(1H, dd, J=5.9 and 4.3Hz), 8.17(1H, s), 8.03(1H, dd, J=11.8 and 3.2Hz), 7.60(1H, d, J=6.3Hz), 7.35(2H, d, J=9.0Hz), 7.11(1H, dd, J=7.2 and 4.8Hz), 7.00-6.95(3H, m),

6.72(1H, ddd, J=6.6, 2.9 and 2.9Hz), 4.12(2H, q, J=7.1Hz), 3.61(4H, t, J=4.9Hz), 3.08(4H, t, J=4.7Hz), 2.13(3H, s), 1.40 (3H, t, J=7.1Hz).
MS($ES^+$) m/z:493 $(M + H)^+$.
Melting point: 193-195˚C.

(Example 318) 4- {4-[3-(5-chloro-2-ethoxy-phenyl)-ureido]-phenyl}-piperazine-1-carboxylic acid (3-methyl-pyridin-2-yl)-amide (Compound No. 1-530)

92 mg (57%) of the title compound was obtained as a light brown solid from 1-(5-chloro-2-ethoxy-phenyl)-3-(4-piperazin-1-yl-phenyl)-urea (119 mg) obtained in Example (102a) and 2-tert-butoxycarbonylamino-3-methylpyridine (89 mg) obtained in Example (294c) in the same manner as in Example (294d).
$^1H$ NMR(400MHz,DMSO-d6):δ(ppm)=9.29(1H, s), 8.88(1H, s), 8.25(1H, dd, J=2.8 and 2.8Hz), 8.19(1H, dd, J=1.9 and 2.0Hz), 8.15(1H, s), 7.60(1H, d, J=7.4Hz), 7.35(2H, dd, J=9.0 and 2.4Hz), 7.14-7.08(1H, m), 7.03-6.93(4H, m), 4.17-4.12 (2H, m), 3.61(4H, brs), 3.09(4H, brs), 2.14(3H, s), 1.45-1.39(3H, m).
MS($ES^+$) m/z:509 $(M + H)^+$.
Melting point: 216-218˚C.

(Example 319) 4- {4-[3-(4-tert-butyl-thiazol-2-yl)-ureido]-phenyl}-piperazine-1-carboxylic acid (3-methyl-pyridin-2-yl)-amide (Compound No. 1-535)

33.1 mg (34%) of the title compound was obtained as a white solid from 2-tert-butoxycarbonylamino-3-methylpyridine (83.3 mg) obtained in Example (294c) and 1-(4-tert-butyl-thiazol-2-yl)-3-(4-piperazin-1-yl-phenyl)-urea (71.9 mg) obtained in Example (224c) in the same manner as in Example (294d).
$^1H$ NMR(400MHz,DMSO-d6):δ(ppm)=10.5(1H, s), 8.87(IH, s), 8.61(IH, s), 8.19(1H, dd, J=4.6 and 1.1Hz), 7.60(IH, dd, J=6.8 and 1.1Hz), 7.33(2H, d, J=8.8Hz), 7.11 (1H, dd, J=6.8 and 4.6Hz), 6.97(2H, d, J=8.8Hz), 6.63 (1H, s), 3.61 (4H, t, J=4.0Hz), 3.09(4H, t, J=4.OHz), 2.13(3H, s), 1.26(9H, s)
MS($ES^+$) m/z:494 $(M + H)^+$.
Melting point: 158-159˚C

(Example 320) 4- {5-[3-(4-tert-butyl-thiazol-2-yl)-ureido]-pyridin-2-yl}-piperazine-1-carboxylic acid (3-methyl-pyridin-2-yl)-amide (Compound No. 1-547)

(320a) 4-{5-[3-(4-tert-butyl-thiazol-2-yl)-ureido]-pyridin-2-yl}-piperazine-1-carboxylic acid tert-butyl ester

471 mg (30%) of the title compound was obtained as a pale pink solid from 4-[5-(4-nitro-phenoxycarbonylamino)-pyridin-2-yl]-piperazine-1-carboxylic acid tert-butyl ester (1.49 g) obtained in Example (237a) and 4-tert-butyl-thiazol-2-ylamine (630 mg) in the same manner as in Example (224b).
$^1H$ NMR(400MHz,$CDCl_3$):δ(ppm)=9.03(1H, s), 8.22(1H, s), 7.87-7.80(1H, m), 7.13-7.05(1H, m), 7.13(1H, s), 3.55-3.49 (4H, m), 3.49-3.43(4H, m), 1.43(9H, s), 1.25(9H, s).
MS($ES^+$) m/z:461 $(M + H)^+$.

(320b) 1-(4-tert-butyl-thiazol-2-yl)-3-(6-piperazin-1-yl-pyridin-3-yl)-urea

320 mg (89%) of the title compound was obtained as a pale yellow solid from 4-{5-[3-(4-tert-butyl-thiazol-2-yl)-ureido]-pyridin-2-yl}-piperazine-1-carboxylic acid tert-butyl ester (458 mg) obtained in Example (320a) in the same manner as in Example (47a).
MS($ES^+$) m/z:361 $(M + H)^+$.

(320c) 4- {5-[3-(4-tert-butyl-thiazol-2-yl)-ureido]-pyridin-2-yl}-piperazine-1-carboxylic acid (3-methyl-pyridin-2-yl)-amide

40 mg (40%) of the title compound was obtained as a white solid from 2-tert-butoxycarbonylamino-3-methylpyridine (83 mg) obtained in Example (294c) and 1-(4-tert-butyl-thiazol-2-yl)-3-(6-piperazin-1-yl-pyridin-3-yl)-urea (72 mg) obtained in Example (320b) in the same manner as in Example (294d).
$^1H$ NMR(400MHz,$CDCl_3$):δ(ppm)=10.6(1H, s), 8.86(1H, s), 8.61(1H, s), 8.21-8.17(1H, m), 8.19(1H, d, J=2.3Hz), 7.73 (1H, dd, J=9.0 and 2.3Hz), 7.60(1H, d, J=6.7Hz), 7.11(1H, dd, J=6.7 and 5.3Hz), 6.91(1H, d, J=9.0Hz), 6.63(1H, s), 3.60-3.54(4H, m), 3.50-3.45(4H, m), 2.13(3H, s), 1.25(9H, s).
MS($ES^+$) m/z:495 $(M + H)^+$.
Melting point: 157-159˚C.

(Example 321) 4-{5-[3-(3-ethoxy-phenyl)-ureido]-pyridin-2-yl}-piperazine-l-carboxylic acid (3-methyl-pyridin-2-yl)-amide (Compound No. 1-857)

(321a) 4-{S-[3-(3-ethoxy-phenyl)-ureido]-pyridin-2-yl}-piperazine-1-carboxylic acid tert-butyl ester

8.37 g (95%) of the title compound was obtained as a purple solid from 4-(5-amino-pyridin-2-yl)-piperazine-1-carboxylic acid tert-butyl ester (5.57 g) obtained in Example (38b) and 3-ethoxyphenyl isocyanate (3.54 mL) in the same manner as in Example (38c).
$^1$H NMR(400MHz,$CDCl_3$):δ(ppm)=8.08(1H, d, J=2.3Hz), 7.65(1H, dd, J=9.0 and 2.4Hz), 7.20(1H, dd, J=8.0 and 8.0Hz), 7.03(1H, s), 6.82(1H, d, J=7.9Hz), 6.66(2H, d, J=9.0Hz), 6.46(1H, s), 6.37(1H, s), 4.02(2H, q, J=6.9Hz), 3.55-3.50(8H, m), 1.49(9H, s), 1.40(3H, t, J=7.1Hz).

(321b) 1-(3-ethoxy-phenyl)-3-(6-piperazin-1-yl-pyridin-3-yl)-urea

6.47 g (quantitative yield) of the title compound was obtained as a pale purple solid from 4-{5-[3-(3-ethoxy-phenyl)-ureido]-pyridin-2-yl}-piperazine-1-carboxylic acid tert-butyl ester (8.37 g) obtained in Example (321a) in the same manner as in Example (47a).
$^1$H NMR(400MHz,DMSO-d6):δ(ppm)=8.74(1H, s), 8.48(1H, s), 8.11(1H, d, J=2.3Hz), 7.66(1H, d, J=7.8Hz), 7.15-7.10 (2H, m), 6.89(1H, d, J=7.4Hz), 6.76(1H, d, J=8.6Hz), 6.49(1H, dd, J=8.2 and 1.6Hz), 3.97(2H, q, J=7.1Hz), 3.43(1H, brs), 3.30(4H, t, J=4.5Hz), 2.78(4H, t, J=4.3Hz), 1.31(3H, t, J=6.9Hz).

(321c) 4-{5-[3-(3-ethoxy-phenyl)-ureido]-pyridin-2-yl}-piperazine-1-carboxylic acid (3-methyl-pyridin-2-yl)-amide

53 mg (55%) of the title compound was obtained as a pale yellow solid from 2-tert-butoxycarbonylamino-3-methylpyridine (83 mg) obtained in Example (294c) and 1-(3-ethoxy-phenyl)-3-(6-piperazin-1-yl-pyridin-3-yl)-urea (68 mg) obtained in Example (321b) in the same manner as in Example (294d).
$^1$H NMR(400MHz,DMSO-d6):δ(ppm)=8.83(1H, s), 8.62(1H, s), 8.39(1H, s), 8.16(1H, dd, J=4.6 and 1.6Hz), 8.15(1H, d, J=2.7Hz), 7.71(1H, dd, J=9.0 and 2.7Hz), 7.57(1H, dd, J=7.8 and 1.6Hz), 7.14(1H, dd, J=2.5 and 2.5Hz), 7.13(1H, dd, J=8.0 and 8.0Hz), 7.08(1H, dd, J=7.8 and 4.6Hz), 6.88(1H, dd, J=8.0 and 2.5Hz), 6.86(1H, d, J=9.0Hz), 6.50(1H, dd, J=8.0 and 2.5Hz), 3.97(2H, q, J=6.9Hz), 3.59-3.53(4H, m), 3.46-3.41(4H, m), 2.12(3H, s), 1.32(3H, t, J=6.9Hz).
MS($ES^+$) m/z:476 $(M + H)^+$.
Melting point: 99-100˚C.

(Example 322) 4-{2-chloro-4-[3-(2-methoxy-phenyl)-ureido]-phenyl}-piperazine-1-carboxylic acid (3-methyl-pyridin-2-yl)-amide (Compound No. 2-127)

31 mg (31%) of the title compound was obtained as a white solid from 2-tert-butoxycarbonylamino-3-methyl-pyridine (50 mg) obtained in Example (294c) and 1-(3-chloro-4-piperazin-1-yl-phenyl)-3-(2-methoxy-phenyl)-urea (72 mg) obtained in Example (269b) in the same manner as in Example (294d).
$^1$H NMR(400MHz,DMSO-d6):δ(ppm)=9.38(1H, s), 8.84(1H, s), 8.18(1H, s), 8.17(1H, d, J=4.8Hz), 8.12(1H, d, J=7.8Hz), 7.73(1H, s), 7.60(1H, d, J=7.0Hz), 7.24(1H, d, J=8.6Hz), 7.15(1H, d, J=8.6Hz), 7.11(1H, dd, J=7.0 and 4.8Hz), 7.03(1H, d, J=7.8Hz), 6.96(1H, dd, J=7.8 and 7.8Hz), 6.90(1H, dd, J=7.8 and 7.8Hz), 3.88(3H, s), 3.65-3.56(4H, m), 2.97-2.86 (4H, m), 2.15(3H, s).
MS($ES^+$) m/z:495 $(M + H)^+$.
Melting point: 190-191˚C.

(Example 323) 4-{2-chloro-4-[3-(2-fluoro-phenyl)-ureido]-phenyl}-piperazine-1-carboxylic acid (3-methyl-pyridin-2-yl)-amide (Compound No. 2-145)

37 mg (38%) of the title compound was obtained as a white solid from 2-tert-butoxycarbonylamino-3-methyl-pyridine (50 mg) obtained in Example (294c) and 1-(3-chloro-4-piperazin-1-yl-phenyl)-3-(2-fluoro-phenyl)-urea (70 mg) obtained in Example (221d) in the same manner as in Example (294d).
$^1$H NMR(400MHz,DMSO-d6):δ(ppm)=9.12(1H, s), 8.85(1H, s), 8.55(1H, s), 8.19(1H, d, J=5.1Hz), 8.13(1H, ddd, J=8.2,8.2 and 1.4Hz), 7.73(1H, s), 7.60(1H, d, J=6.6Hz), 7.30-6.98(6H, m), 3.66-3.58(4H, m), 2.97-2.89(4H, m), 2.15(3H, s).
MS($ES^+$) m/z:483 $(M + H)^+$.
Melting point: 240-242˚C (dec).

(Example 324) 4-{4-[3-(2-methoxy-5-methyl-phenyl)-ureido]-phenyl}-3,6-dihydro-2H-pyridine-1-carboxylic acid (3-methyl-pyridin-2-yl)-amide (Compound No. 5-4)

68 mg (66%) of the title compound was obtained as a white solid from 2-tert-butoxycarbonylamino-3-methylpyridine (92 mg) obtained in Example (294c) and 1-(2-methoxy-5-methyl-phenyl)-3-[4-(1,2,3,6-tetrahydro-pyridin-4-yl)-phenyl]-urea (74 mg) obtained in Example (230b) in the same manner as in Example (294d).
$^1$H NMR(400MHz,DMSO-d6):δ(ppm)=2.12(3H, s), 2.23(3H, s), 2.57-2.43(2H, m), 3.66(2H, t, J=5.3Hz), 3.83(3H, s), 4.13 (2H, brs), 6.13(1H, brs), 6.73(1H, dd, J=8.3 and 1.6Hz), 6.88(1H, d, J=8.2Hz), 7.08(1H, dd, J=7.4 and 4.7Hz), 7.38(2H, d, J-9.0Hz), 7.43(2H, d, J=9.0Hz), 7.57(1H, d, J=7.1Hz), 7.97(1H, brs), 8.17-8.13(2H, m), 8.74(1H, s), 9.32(1H, s).
MS(APCI) m/z:472 $(M + H)^+$.
Melting point: 195-198˚C.

(Example 325) 4-{4-[3-(2-methoxy-5-methyl-phenyl)-ureido]-phenyl}-piperidine-1- carboxylic acid (3-methyl-pyridin-2-yl)-amide (Compound No. 4-4)

50 mg (53%) of the title compound was obtained as a white solid from 2-tert-butoxycarbonylamino-3-methylpyridine (63 mg) obtained in Example (294c) and 1-(2-methoxy-5-methyl-phenyl)-3-(4-piperidin-4-yl-phenyl)-urea (68 mg) obtained in Example (228e) in the same manner as in Example (294d).
$^1$H NMR(400MHz,DMSO-d6):δ(ppm)=1.60-1.47(2H, m), 1.77(2H, d, J=11.8Hz), 2.14(3H, s), 2.22(3H, s), 2.69(1H, t, J=12.2Hz), 2.88(2H, t, J=12.1Hz), 3.83(3H, s),
4.22(2H, d, J=12.9Hz), 6.72(1H, dd, J=8.2 and 2.0Hz), 6.87(1H, d, J=8.2Hz), 7.07(1H, dd, J=7.3 and 4.5Hz), 7.14(2H, d, J=8.6Hz), 7.37(2H, d, J=8.6Hz), 7.56(1H, dd, J=7.8 and 0.8Hz), 7.96(1H, d, J=1.5Hz), 8.11(1H, s), 8.15(1H, dd, J=4.7 and 1.2Hz), 8.71(1H, s), 9.21(1H, s).
MS(APCI) m/z:474 $(M + H)^+$.
Melting point: 199-201˚C.

(Example 326) 6-[3-(3,5-dimethoxy-phenyl)-ureido]-3,4-dihydro-1H-isoquinoline-2-carboxylic acid (3-methyl-pyridin-2-yl)-amide (Compound No. 3-55)

43 mg (42%) of the title compound was obtained as a lemon yellow solid from 2-tert-butoxycarbonylamino-3-methylpyridine (91 mg) obtained in Example (294c) and 1-(3,5-dimethoxy-phenyl)-3-(1,2,3,4-tetrahydro-isoquinolin-6-yl)-urea (72 mg) obtained in Example (266b) in the same manner as in Example (294d).
$^1$H NMR(400MHz,DMSO-d6):δ(ppm)=2.15(3H, s), 2.83(2H, t, J=5.4Hz), 3.71(6H, s), 3.74-3.66(2H, m), 4.58(2H, s), 6.12 (1H, dd, J=2.3Hz and 2.3Hz), 6.66(2H, d, J=2.0Hz), 7.06(1H, d, J=8.7Hz), 7.14(1H, brs), 7.20(1H, dd, J=8.2 and 2.0Hz), 7.34(1H, s), 7.68(1H, brd, J=6.6Hz), 8.18(1H, brs), 8.58(1H, s), 8.66(1H, s), 8.90(1H, brs).
MS(APCI) m/z:462 $(M + H)^+$.
Melting point: 132-137˚C.

(Example 327) 4-{4-[3-(2-methoxy-phenyl)-ureido]-phenyl}-3,6-dihydro-2H-pyridine-1-carboxylic acid (3-methyl-pyridin-2-yl)-amide (Compound No. 5-2)

51 mg (51 %) of the title compound was obtained as a pale yellow solid from 2-tert-butoxycarbonylamino-3-methylpyridine (91 mg) obtained in Example (294c) and 1-(2-methoxy-phenyl)-3-[4-(1,2,3,6-tetrahydro-pyridin-4-yl)-phenyl]-urea (71 mg) obtained in Example (258b) in the same manner as in Example (294d).
$^1$H NMR(400MHz,DMSO-d6):δ(ppm)=2.12(3H, s), 2.41-6.62(2H, m), 3.66(2H, t, J=5.4Hz), 3.87(3H, s), 4.13(2H, brs), 6.13(1H, brs), 6.96-6.85(2H, m), 7.00(1H, dd, J=8.2 and 1.2Hz), 7.08(1H, dd, J=7.4 and 5.1Hz), 7.39(2H, d, J=8.6Hz), 7.43(2H, d, J=8.6Hz), 7.57(1H, d, J=7.4Hz), 8.18-8.09(2H, m), 8.21(1H, s), 8.75(1H, s), 9.34(1H, s).
MS(APCI) m/z:458 $(M + H)^+$.
Melting point: 133-135˚C.

(Example 328) 4- {4-[3-(3,5-dimethoxy-phenyl)-ureido]-phenyl}-3,6-dihydro-2H-pyridine-1-carboxylic acid (3-methyl-pyridin-2-yl)-amide (Compound No. 5-5)

48 mg (45%) of the title compound was obtained as a white solid from 2-tert-butoxycarbonylamino-3-methylpyridine (91 mg) obtained in Example (294c) and 1-(3,5-dimethoxy-phenyl)-3-[4-(1,2,3,6-tetrahydro-pyridin-4-yl)-phenyl]-urea (78 mg) obtained in Example (259b) in the same manner as in Example (294d).
$^1$H NMR(400MHz, DMSO-d6):δ(ppm)=2.11(3H, s), 2.37-2.63(2H, m), 3.71(6H, s), 3.80-3.56(2H, m), 4.13(2H, brs), 6.13 (2H, brs), 6.67(2H, brs), 7.09(1H, brs), 7.41(4H, brs), 7.58(1H, brs), 8.17(1H, brs), 8.66(2H, brs), 8.76(1H, brs).

MS(APCI) m/z:488 $(M + H)^+$.
Melting point: 181-184˚C.

(Example 329) 4- {4-[3-(2-methoxy-phenyl)-ureido]-phenyl} -piperidine-1 -carboxylic acid (3-methyl-pyridin-2-yl)-amide (Compound No. 4-2)

27 mg (27%) of the title compound was obtained as a white solid from 2-tert-butoxycarbonylamino-3-methyl-pyridine (91 mg) obtained in Example (294c) and 1-(2-methoxy-phenyl)-3-(4-piperidin-4-yl-phenyl)-urea (71 mg) obtained in Example (261b) in the same manner as in Example (294d).
$^1$H NMR(400MHz,DMSO-d6):δ(ppm)=1.60-1.47(2H, m), 1.81-1.73(2H, m), 2.14(3H, s), 2.74-2.65(1H, m), 2.88(2H, brt, J=12.7Hz), 3.87(3H, s), 4.22(2H, brd, J=12.5Hz), 6.95-6.84(2H, m), 7.00(1H, dd, J=7.8 and 1.2Hz), 7.07(1H, dd, J=6.8 and 4.9Hz), 7.15(2H, d, J=8.2Hz), 7.37(2H, d, J=8.7Hz), 7.56(1H, brd, J=7.1Hz), 8.11(1H, dd, J=8.1 and 1.4Hz), 8.17 (1H, s), 8.20-8.13(1H, m), 8.72(1H, s), 9.23(1H, s).
MS(APCI) m/z:460 $(M + H)^+$.
Melting point: >280˚C.

(Example 330) 4-{4-[3-(3,5-dimethoxy-phenyl)-ureido]-phenyl}-piperidine-1-carboxylic acid (3-methyl-pyridin-2-yl)-amide (Compound No. 4-5)

38 mg (35%) of the title compound was obtained as a white solid from 2-tert-butoxycarbonylamino-3-methyl-pyridine (91 mg) obtained in Example (294c) and 1-(3,5-dimethoxy-phenyl)-3-(4-piperidin-4-yl-phenyl)-urea (78 mg) obtained in Example (262b) in the same manner as in Example (294d).
$^1$H NMR(400MHz,DMSO-d6):δ(ppm)=1.61-1.46(H, m), 1.81-1.72(2H, m), 2.14(2H, s), 2.74-2.63(1H, m), 2.88(2H, brt, J=12.1Hz), 3.70(3H, s), 4.22(2H, brd, J=11.7Hz), 6.12(1H, brs), 6.66(2H, d, J=1.2Hz), 7.10-7.04(1H, m), 7.14(2H, d, J=7.8Hz), 7.36(2H, d, J=7.4Hz), 7.56(1H, brd, J=7.4Hz), 8.18-8.13(1H, m), 8.54(1H, s), 8.62(1H, s), 8.72(1H, s).
MS(APCI) m/z:490 $(M + H)^+$.
Melting point: 207-210˚C.

(Example 331) 4-{4-[3-(2-fluoro-phenyl)-ureido]-phenyl}-3,6-dihydro-2H-pyridine-1-carboxylic acid (3-methyl-pyridin-2-yl)-amide (Compound No. 5-1)

50 mg (53%) of the title compound was obtained as a white solid from 2-tert-butoxycarbonylamino-3-methyl-pyridine (83 mg) obtained in Example (294c) and 1-(2-fluoro-phenyl)-3-[4-(1,2,3,6-tetrahydro-pyridin-4-yl)-phenyl]-urea (62 mg) obtained in Example (260b) in the same manner as in Example (294d).
$^1$H NMR(400MHz,DMSO-d6):δ(ppm)=2.12(3H, s), 2.57-2.43(2H, m), 3.66(2H, brt, J=5.1Hz), 4.13(2H, brs), 6.14(1H, brs), 7.04-6.95(1H, m), 7.16-7.05(2H, m), 7.26-7.18(1H, m), 7.40(2H, d, J=8.2Hz), 7.44(2H, d, J=8.6Hz), 7.57(1H, brd, J=8.6Hz), 8.19-8.11(2H, m), 8.54(1H, brs), 8.75(1H, brs), 9.10(1H, brs).
MS(APCI) m/z:446 $(M + H)^+$.
Melting point: 185-188˚C.

(Example 332) 4-{4-[3-(2-fluoro-phenyl)-ureido]-phenyl}-piperidine-1-carboxylic acid (3-methyl-pyridin-2-yl)-amide (Compound No. 4-1)

58 mg (64%) of the title compound was obtained as a white solid from 2-tert-butoxycarbonylamino-3-methyl-pyridine (83 mg) obtained in Example (294c) and 1-(2-fluoro-phenyl)-3-(4-piperidin-4-yl-phenyl)-urea (63 mg) obtained in Example (263b) in the same manner as in Example (294d).
$^1$H NMR(400MHz,DMSO-d6):δ(ppm)=1.61-1.46(2H, m), 1.82-1.71(2H, m), 2.14(3H, s), 2.75-2.65(1H, m), 2.88(2H, brt, J=12.7Hz), 4.22(2H, brd, J=12.5Hz), 7.03-6.92(1H, m), 7.16(2H, d, J=8.2Hz), 7.26-7.03(3H, m), 7.37(2H, d, J=7.8Hz), 7.57(1H, brd, J=8.2Hz), 8.21-8.09(2H, m), 8.50(1H, s), 8.72(1H, s), 8.99(1H, s).
MS(APCI) m/z:448 $(M + H)^+$.
Melting point: 191-194˚C.

(Example 333) 4-{4-[3-(2-methylsulfanyl-phenyl)-ureido]-phenyl}-piperazine-1-carboxylic acid (3-methyl-pyridin-2-yl)-amide (Compound No. 1-895)

109 mg (46%) of the title compound was obtained as a white solid from 1-(2-methylsulfanyl-phenyl)-3-(4-piperazin-1-yl-phenyl)-urea (171 mg) obtained in Example (241b) and 2-tert-butoxycarbonylamino-3-methylpyridine (55 mg) obtained in Example (294c) in the same manner as in Example (294d).

$^1$H NMR(400MHz,DMSO-d6):δ(ppm)=9.18(1H, s), 8.84(1H, s), 8.16(1H, d, J=5.1Hz), 8.04(1H, s), 7.93(1H, d, J=8.2Hz), 7.57(1H, d, J=7.0Hz), 7.38(1H, d, J=8.2Hz), 7.33(2H, d, J=9.0Hz), 7.19(1H, dd, J=7.7 and 7.7Hz), 7.08(1H, dd, J=4.8 and 7.7Hz), 7.01(1H, dd, J=7.6 and 7.6Hz), 6.93(2H, d, J=9.OHz), 3.59(4H, t, J=4.5Hz), 3.06(4H, t, J=4.7Hz), 2.42(3H, s), 2.12(3H, s).
MS(ES$^+$) m/z:477 (M + H)$^+$.
Melting point: 196-198˚C.

(Example 334) 4-{4-[3-(2,3-dimethoxy-phenyl)-ureido]-phenyl}-piperazine-1-carboxylic acid (3-methyl-pyridin-2-yl)-amide (Compound No. 1-882)

32 mg (13%) of the title compound was obtained as a pale reddish brown solid from 1-(2,3-dimethoxy-phenyl)-3-(4-piperazin-1-yl-phenyl)-urea (178 mg) obtained in Example (247b) and 2-tert-butoxycarbonylamino-3-methylpyridine (55 mg) obtained in Example (294c) in the same manner as in Example (294d).
$^1$H NMR(400MHz,DMSO-d6):δ(ppm)=9.12(1H, s), 8.87(1H, s), 8.27(1H, s), 8.19(1H, d, J=3.5Hz), 7.82(1H, d, J=8.2Hz), 7.60(1H, d, J=6.7Hz), 7.34(2H, d, J=9.0Hz), 7.11(1H, dd, J=7.2 and 4.9Hz), 6.97(1H, dd, J=9.9 and 10.0Hz), 6.96(2H, d, J=8.6Hz), 6.66(1H, d, J=8.2Hz), 3.81(3H, s), 3.76(3H, s), 3.60(4H, t, J=4.5Hz), 3.07(4H, t, J=4.7Hz), 2.13(3H, s).
MS(ES$^+$) m/z:491 (M + H)$^+$.
Melting point: 186-187˚C.

(Example 335) 4-{5-[3-(2-ethoxy-5-fluoro-phenyl)-ureido]-pyndin-2-yl}-piperazine-1-carboxylic acid (3-trifluoromethyl-pyridin-2-yl)-amide (Compound No. 1-877) (335a) 3-trifluoromethyl-pyridin-2-ylamine

2,4-dimethoxybenzylamine (8.5 g) and potassium carbonate (5.77 g) were added to a solution of 2-chloro-3-trifluoromethylpyridine (6.89 g) in dimethylacetamide (70 mL) at room temperature. The reaction mixture was heated at 70˚C for 24 hours and diluted with ethyl acetate and washed with a saturated sodium hydrogen carbonate aqueous solution and saturated brine and concentrated. The residue was purified by column chromatography (hexane:ethyl acetate 10:1 → 5:1) to obtain a yellow oil (5.23 g). Trifluoroacetic acid (20 mL) was added to a solution of this oil in dichloromethane (70 mL) at room temperature. The reaction mixture was stirred for four days and concentrated and was neutralized with a saturated sodium hydrogen carbonate aqueous solution, followed by extraction with ethyl acetate. The organic layer was concentrated and purified by column chromatography (hexane:ethyl acetate 5:1 → 1:1) and 1.02 g (17%, two steps) of the title compound was obtained as a white solid.
$^1$H NMR(400MHz,$CDCl_3$):δ(ppm)=8.21(1H, d, J=5.1Hz), 7.69(1H, d, J=7.4Hz), 6.72(1H, dd, J=7.7 and 4.9Hz), 4.95(2H, brs).

(335b) (3-trifluoromethyl-pyridin-2-yl)-carbamic acid tert-butyl ester

Sodium hexamethyldisilazide (38% tetrahydrofuran solution, 3.8 mL) was added to a solution of 3-trifluoromethyl-pyridin-2-ylamine (578 mg) obtained in Example (335a) in anhydrous tetrahydrofuran (10 mL) at room temperature. After 15 minutes, a solution of di-tert-butyl dicarbonate (778 mg) in anhydrous tetrahydrofuran (10 mL) was added thereto. The reaction mixture was stirred at room temperature for three days and concentrated. The residue was purified by column chromatography (dichloromethane: ethyl acetate 5:1 → 1:1) and 672 mg (72%) of the title compound was obtained as a pale yellow solid.
$^1$H NMR(400MHz,$CDCl_3$):δ(ppm)=8.65(1H, d, J=4.7Hz), 7.90(1H, d, J=7.9Hz), 7.13(1H, dd, J=7.1 and 4.7Hz), 7.05(1H, brs), 1.54(9H, s).

(335c) 4-{5-[3-(2-ethoxy-5-fluoro-phenyl)-ureido]-pyridin-2-yl}-piperazine-1-carboxylic acid (3-trifluoromethyl-pyridin-2-yl)-amide

284 mg (82%) of the title compound was obtained as a white solid from 1-(2-ethoxy-5-fluoro-phenyl)-3-(6-piperazin-1-yl-pyridin-3-yl)-urea (226 mg) obtained in Example (300b) and (3-trifluoromethyl-pyridin-2-yl)-carbamic acid tert-butyl ester (197 mg) obtained in Example (335b) in the same manner as in Example (294d).
$^1$H NMR(400MHz,DMSO-d6):δ(ppm)=9.28(1H, s), 9.08(1H, s), 8.62(1H, s), 8.19(1H, s), 8.15(1H, d, J=2.4Hz), 8.12(1H, s), 7.99(1H, dd, J=11.6 and 3.3Hz), 7.74(1H, d, J=9.8Hz), 7.41(1H, brs), 6.98(1H, dd, J=8.8 and 5.3Hz), 6.88(1H, d, J=8.6Hz), 6.69(1H, ddd, J=8.5, 8.5 and 3.2Hz), 4.11(2H, q, J=7.1Hz), 3.55(4H, brs), 3.44(4H, brs), 1.40(3H, t, J=7.1Hz).
MS(ES$^+$) m/z:548 (M + H)$^+$.
Melting point: 206-208˚C.

(Example 336) 4-{4-[3-(2-methoxy-5-methyl-phenyl)-ureido]-phenyl}-piperazine-1-carboxylic acid (3-chloro-pyridin-2-yl)-amide (Compound No. 1-898)

(336a) 3-chloro-pyridin-2-ylamine

2,3-dichloropyridine (2.96 g), palladium acetate (46 mg), BINAP (rac-2,2'-bis(diphenylphosphino)-1,1'-binaphthyl, 184 mg) and sodium tert-butoxide (2.88 g) were added to a solution of 2,4-dimethoxybenzylamine (2.04 g) in anhydrous tetrahydrofuran (40 mL) at room temperature. The reaction mixture was heated under reflux for ten hours and diluted with ethyl acetate and washed with a saturated sodium hydrogen carbonate aqueous solution and dried over sodium sulfate and concentrated. The residue was purified by column chromatography (hexane:ethyl acetate 20:1 → 5:1) to obtain 4.05 g of yellow oil. Trifluoroacetic acid (15 mL) was added to a solution of this oil in dichloromethane (40 mL) at room temperature. The reaction mixture was stirred at room temperature for three hours and concentrated and neutralized with a saturated sodium hydrogen carbonate aqueous solution, followed by extraction with ethyl acetate. The organic layer was concentrated and purified by column chromatography (hexane:ethyl acetate 10:1 → 1:1) and 1.09 g (85%, two steps) of the title compound was obtained as a white solid. $^1H$ NMR(404MHz,$CDCl_3$):δ(ppm)=7.99 (1H, d, J=3.9Hz), 7.50(1H, dd, J=7.8 and 1.5Hz), 6.63(1H, dd, J=7.8 and 5.1Hz), 4.89(2H, brs).

(336b) (3-chloro-pyridin-2-yl)-carbamic acid tert-butyl ester

1.53 g (79%) of the title compound was obtained as a yellow solid from 3-chloro-pyridine-ylamine (1.09 g) obtained in Example (336a) and di-tert-butyl dicarbonate (1.84 g) in the same manner as in Example (335b).
$^1H$ NMR(400MHz,$CDCl_3$):δ(ppm)=8.39(1H, dd, J=4.7 and 1.6Hz), 7.67(1H, dd, J=7.9 and 1.5Hz), 7.29(1H, brs), 6.97 (1H, dd, J=7.8 and 4.7Hz), 1.55(9H, s). (336c) 4-{4-[3-(2-methoxy-5-methyl-phenyl)-ureido]-phenyl}-piperazine-1-carboxylic acid (3-chloro-pyridin-2-yl)-amide

134 mg (54%) of the title compound was obtained as a white solid from 1-(2-methoxy-5-methyl-phenyl)-3-(4-piperazin-1-yl-phenyl)-urea (170 mg) obtained in Example (47a) and (3-chloro-pyridin-2-yl)-carbamic acid tert-butyl ester (134 mg) obtained in Example (336b) in the same manner as in Example (294d).
$^1H$ NMR(400MHz,DMSO-d6):δ(ppm)=9.12(1H, s), 9.06(1H, s), 8.30(1H, dd, J=4.7 and 1.6Hz), 8.04(1H, s), 7.97(1H, d, J=2.0Hz), 7.89(1H, dd, J=7.9 and 1.5Hz), 7.31(2H, d, J=9.0Hz), 7.20(1H, dd, J=8.0 and 4.9Hz), 6.92(2H, d, J=9.4Hz), 6.86(1H, d, J=8.3Hz), 6.71(1H, dd, J=8.2 and 1.6Hz), 3.81(3H, s), 3.58(4H, t, J=4.6Hz), 3.05(4H, t, J=4.7Hz), 2.20(3H, s).
MS($ES^+$) m/z:495 $(M + H)^+$.
Melting point: 113-115°C.

(Example 337) 4-{4-[3-(2-methoxy-S-methyl-phenyl)-ureido]-phenyl}-piperazine-1-carboxylic acid (3-fluoro-pyridin-2-yl)-amide (Compound No. 1-899)

(337a) 3-fluoro-pyridin-2-ylamine

114 mg (two steps, 27%) of the title compound was obtained as a pale yellow solid from 2-chloro-3-fluoropyridine (589 mg) and 2,4-dimethoxybenzylamine (912 mg) in the same manner as in Example (336a).
$^1H$ NMR(400MHz,$CDCl_3$):δ(ppm)=7.87(1H, d, J=S.1Hz), 7.23(1H, ddd, J=10.8, 7.9 and 1.3Hz), 6.65(1H, ddd, J=8.2, 4.7 and 3.1Hz), 4.63(2H, brs).

(337b) (3-fluoro-pyridin-2-yl)-carbamic acid tert-butyl ester

115 mg (50%) of the title compound was obtained as a pale yellow solid from 3-fluoro-pyridin-2-ylamine (114 mg) obtained in Example (337a) and di-tert-butyl dicarbonate (218 mg) in the same manner as in Example (335b).
$^1H$ NMR(400MHz,$CDCl_3$):δ(ppm)=8.24(1H, d, J=5.1Hz), 7.41(1H, ddd, J=10.1, 8.3 and 1.7Hz), 7.05(1H, ddd, J=8.2, 4.7 and 3.7Hz), 6.92(1H, brs), 1.54(9H, s).

(337c) 4-{4-[3-(2-methoxy-5-methyl-phenyl)-ureido]-phenyl}-piperazine-1-carboxylic acid (3-fluoro-pyridin-2-yl)-amide

38 mg (19%) of the title compound was obtained as a white solid from 1-(2-methoxy-5-methyl-phenyl)-3-(4-piperazin-1-yl-phenyl)-urea (143 mg) obtained in Example (47a) and (3-fluoro-pyridin-2-yl)-carbamic acid tert-butyl ester (115 mg) obtained in Example (337b) in the same manner as in Example (294d).
$^1H$ NMR(400MHz,DMSO-d6):δ(ppm)=9.16(1H, s), 9.06(1H, s), 8.15(1H, d, J=4.7Hz), 8.04(1H, s), 7.96(1H, d, J=1.6Hz), 7.66(1H, ddd, J=10.5, 8.1 and 1.5Hz), 7.31(2H, d, J=9.0Hz), 7.22(1H, ddd, J=8.2, 4.7 and 3.5Hz), 6.92(2H, d, J=9.0Hz), 6.86(1H, d, J=8.2Hz), 6.70(1H, dd, J=8.2 and 1.6Hz), 3.82(3H, s), 3.60(4H, t, J=4.7Hz), 3.06(4H, t, J=4.9Hz), 2.22(3H, s).

MS(ES$^+$) m/z:479 (M + H)$^+$.
Melting point: 131-133˚C.

(Example 338) 4-{4-[3-(2-methoxy-5-methyl-phenyl)-ureido]-phenyl}-piperazine-1-carboxylic acid (3,5-difluoro-pyridin-2-yl)-amide (Compound No. 1-787)

(338a) (3,5-difluoro-pyridin-2-yl)-carbamic acid tert-butyl ester

525 mg (28%) of the title compound was obtained as a white solid from 3,5-difluoro-pyridin-2-ylamine (1.14 g) and di-tert-butyl dicarbonate (1.75 g) in the same manner as in Example (335b).
$^1$H NMR(400MHz,$CDCl_3$):δ(ppm)=8.14(1H, d, J=2.8Hz), 7.25(1H, ddd, J=9.8, 7.6 and 2.8Hz), 6.89(1H, brs), 1.53(9H, s).

(338b) 4-{4-[3-(2-methoxy-5-methyl-phenyl)-ureido]-phenyl}-piperazine-1-carboxylic acid (3,5-difluoro-pyridin-2-yl)-amide

40.4 mg (41%) as of the title compound was obtained as a lemon yellow solid from 1-(2-methoxy-5-methyl-phenyl)-3-(4-piperazin-1-yl-phenyl)-urea (68.1 mg) obtained in Example (47a) and (3,5-difluoro-pyridin-2-yl)-carbamic acid tert-butyl ester (55.2 mg) obtained in Example (338a) in the same manner as in Example (294d).
$^1$H NMR(400MHz,DMSO-d6):δ(ppm)=9.20(1H, s), 9.06(1H, s), 8.25(1H, d, J=2. 8Hz), 8.04(1H, s), 7.96(1H, d, J=2.0Hz), 7.91 (1H, ddd, J=11.1, 8.6 and 2.8Hz), 7.31(2H, d, J=9.0Hz), 6.92(2H, d, J=9.0Hz), 6.86(1H, d, J=8.2Hz), 6.71(1H, dd, J=8.2 and 2.0Hz), 3.82(3H, s), 3.59(4H, t, J=4.8Hz), 3.06(4H, t, J=4.8Hz), 2.22(3H, s).
MS(ES$^+$) m/z:497 (M + H)$^+$.
Melting point: 120-122˚C.

(Example 339) 4-{4-[3-(2-ethoxy-phenyl)-ureido]-phenyl}-piperazine-1-carboxylic acid (3,5-difluoro-pyridin-2-yl)-amide (Compound No. 1-884)

100 mg (50%) of the title compound was obtained as a lemon yellow solid from 1-(2-ethoxy-phenyl)-3-(4-piperazin-1-yl-phenyl)-urea (136 mg) obtained in Example (80a) and (3,5-difluoro-pyridin-2-yl)-carbamic acid tert-butyl ester (202 mg) obtained in Example (338a) in the same manner as in Example (294d).
$^1$H NMR(400MHz,DMSO-d6):δ(ppm)=9.24(1H, s), 9.18(1H, s), 8.28(1H, d, J=2.8Hz), 8.13(1H, dd, J=8.0 and 2.0Hz), 7.98(1H, s), 7.94(1H, ddd, J=11.0, 8.6 and 2.8Hz), 7.35(2H, d, J=9.0Hz), 7.00(1H, dd, J=7.8 and 2.0Hz), 6.96(2H, d, J=9.0Hz), 6.91(1H, ddd, J=8.0, 8.0 and 2.0Hz), 6.87(1H, ddd, J=8.0, 7.8 and 2.0Hz), 4.13 (2H, q, J=6.9Hz), 3.61(4H, t, J=5.0Hz), 3.08(4H, t, J=5.0Hz), 1.41(3H, t, J=6.9Hz).
MS(ES$^+$) m/z:497 (M + H)$^+$.
Melting point: 134-135˚C.

(Example 340) 4-{4-[3-(3-ethoxy-phenyl)-ureido]-phenyl}-piperazine-1-carboxylic acid (3,5-difluoro-pyridin-2-yl)-amide (Compound No. 1-885)

22 mg (11%) of the title compound was obtained as a white solid from 1-(3-ethoxy-phenyl)-3-(4-piperazin-1-yl-phenyl)-urea (136 mg) obtained in Example (215b) and (3,5-difluoro-pyridin-2-yl)-carbamic acid tert-butyl ester (202 mg) obtained in Example (338a) in the same manner as in Example (294d).
$^1$H NMR(400MHz,DMSO-d6):δ(ppm)=9.23(1H, s), 8.57(1H, s), 8.42(1H, s), 8.28(1H, d, J=2.4Hz), 7.94(1H, ddd, J=9.2, 9.2 and 2.4Hz), 7.32(2H, d, J=9.0Hz), 7.17(1H, d, J=2.2Hz), 7.15(1H, dd, J=8.0 and 8.0Hz), 6.94(2H, d, J=9.0Hz), 6.89 (1H, d, J=8.0Hz), 6.52(1H, dd, J=8.0 and 2.2Hz), 3.99(2H, q, J=6.9Hz), 3.60(4H, t, J=4.7Hz), 3.07(4H, t, J=4.7Hz), 1.32 (3H, t, J=6.9Hz).
MS(ES$^+$) m/z:497 (M + H)$^+$.
Melting point: 185-186˚C.

(Example 341) 4-{4-[3-(3,5-dimethoxy-phenyl)-ureido]-phenyl}-piperazine-1-carboxylic acid (3,5-difluoro-pyridin-2-yl)-amide (Compound No. 1-886)

50.5 mg (25%) of the title compound was obtained as a white solid from 1-(3,5-dimethoxy-phenyl)-3-(4-piperazin-1-yl-phenyl)-urea (143 mg) obtained in Example (216b) and (3,5-difluoro-pyridin-2-yl)-carbamic acid tert-butyl ester (184 mg) obtained in Example (338a) in the same manner as in Example (294d).
$^1$H NMR(400MHz,DMSO-d6):δ(ppm)=9.24(1H, s), 8.59(1H, s), 8.40(1H, s), 8.28(1H, d, J=2.3Hz), 7.94(1H, ddd, J=9.2, 9.2 and 2.3Hz), 7.32(2H, d, J=9.0Hz), 6.94(2H, d, J=9.0Hz), 6.67(2H, s), 6.13(1H, s), 3.71(6H, s), 3.63-3.56(4H, m),

3.11-3.03(4H, m).
MS($ES^+$) m/z:513 $(M + H)^+$.
Melting point: 204-205˚C.

(Example 342) 4-{5-[3-(2-ethoxy-5-fluoro-phenyl)-ureido]-pyridin-2-yl}-piperazine-1-carboxylic acid (3,5-difluoro-pyridin-2-yl)-amide (Compound No. 1-888)

159 mg (77%) of the title compound was obtained as a pink solid from 1-(2-ethoxy-5-fluoro-phenyl)-3-(6-piperazin-1-yl-pyridin-3-yl)-urea (144 mg) obtained in Example (300b) and (3,5-difluoro-pyridin-2-yl)-carbamic acid tert-butyl ester (234 mg) obtained in Example (338a) in the same manner as in Example (294d).
$^1$H NMR(400MHz,DMSO-d6):δ(ppm)=9.31(1H, s), 9.24(1H, s), 8.28(1H, d, J=2.3Hz), 8.22(1H, s), 8.18(1H, d, J=2.7Hz), 8.02(1H, dd, J=11.5 and 3.1Hz), 7.94(1H, ddd, J=9.2 9.2 and 2.3Hz), 7.77(1H, dd, J=9.0 and 2.7Hz), 7.00(1H, dd, J=9.0 and 5.5Hz), 6.91(1H, d, J=9.0Hz), 6.72(1H, ddd, J=9.0, 8.4 and 3.1Hz), 4.12(2H, q, J=6.9Hz), 3.57(4H, t, J=4.7Hz), 3.46 (4H, t, J=4.7Hz), 1.40(3H, t, J=6.9Hz).
MS($ES^+$) m/z:516 $(M + H)^+$.
Melting point: 198-199˚C.

(Example 343) 4-{4-[3-(2-trifluoromethoxy-phenyl)-ureido]-phenyl}-piperazine-1-carboxylic acid (3,5-difluoro-pyridin-2-yl)-amide (Compound No. 1-889)

130 mg (60%) of the title compound was obtained as a white solid from 1-(2-trifluoromethoxy-phenyl)-3-(4-piperazin-1-yl-phenyl)-urea (152 mg) obtained in Example (240b) and (3,5-difluoro-pyridin-2-yl)-carbamic acid tert-butyl ester (234 mg) obtained in Example (338a) in the same manner as in Example (294d).
$^1$H NMR(400MHz,DMSO-d6):δ(ppm)=9.24(1H, s), 9.09(1H, s), 8.38(1H, s), 8.31-8.26(1H, m), 8.28(1H, d, J=2.4Hz), 7.94(1H, ddd, J=9.2, 9.2 and 2.4Hz), 7.44-7.29(2H, m), 7.35(2H, d, J=9.0Hz), 7.10-7.04(1H, m), 6.97(2H, d, J=9.0Hz), 3.60(4H, t, J=4.7Hz), 3.09(4H, t, J=4.7Hz).
MS($ES^+$) m/z:537 $(M + H)^+$.
Melting point: 128-130˚C.

(Example 344) 4-{4-[3-(2-difluoromethoxy-phenyl)-ureido]-phenyl}-piperazine-1-carboxylic acid (3,5-difluoro-pyridin-2-yl)-amide (Compound No. 1-890)

113 mg (54%) of the title compound was obtained as a pale pink solid from 1-(2-difluoromethoxy-phenyl)-3-(4-piperazin-1-yl-phenyl)-urea (145 mg) obtained in Example (245a) and (3,5-difluoro-pyridin-2-yl)-carbamic acid tert-butyl ester (234 mg) obtained in Example (338a) in the same manner as in Example (294d).
$^1$H NMR(400MHz,DMSO-d6):δ(ppm)=9.24(1H, s), 9.15(1H, s), 8.28(1H, d, J=2.8Hz), 8.26-8.21(1H, m), 8.23(1H, s), 7.94(1H, ddd, J=11.0, 8.6 and 2.8Hz), 7.34(2H, d, J=9.4Hz), 7.25(1H, t, J=73.7Hz), 7.24-7.16(2H, m), 7.01(1H, ddd, J=7.8, 7.8 and 1.5Hz), 6.96(2H, d, J=9.4Hz), 3.60(4H, t, J=4.8Hz), 3.08(4H, t, J=4.8Hz). MS($ES^+$) m/z:519 $(M + H)^+$.
Melting point: 179-180˚C.

(Example 345) 4-{4-[3-(2-methylsulfanyl-phenyl)-ureido]-phenyl}-piperazine-1-carboxylic acid (3,5-difluoro-pyridin-2-yl)-amide (Compound No. 1-891)

122 mg (81%) of the title compound was obtained as a white solid from 1-(2-methylsulfanyl-phenyl)-3-(4-piperazin-l-yl-phenyl)-urea (103 mg) obtained in Example (241b) and (3,5-difluoro-pyridin-2-yl)-carbamic acid tert-butyl ester (138 mg) obtained in Example (338a) in the same manner as in Example (294d).
$^1$H NMR(400MHz,DMSO-d6):δ(ppm)=9.20(1H, s), 9.19(1H, s), 8.25(1H, d, J=2.4Hz), 8.04(1H, s), 7.93(1H, dd, J=8.4 and 1.3Hz), 7.91(1H, ddd, J=11.3, 8.2 and 2.4Hz), 7.39(1H, dd, J=7.8 and 1.3Hz), 7.33(2H, d, J=9.0Hz), 7.19(1H, ddd, J=7.8,7.6 and 1.3Hz), 7.01(1H, ddd, J=8.4, 7.6 and 1.3Hz), 6.93(2H, d, J=9.0Hz), 3.59(4H, t, J=4.8Hz), 3.07(4H, t, J=4.8Hz), 2.42(3H, s).
MS($ES^+$) m/z:499 $(M + H)^+$.
Melting point: 197-198˚C.

(Example 346) 4-{4-[3-(2,3-dihydro-1,4-benzodioxin-5-yl)-ureido]-phenyl}-piperazine-1-carboxylic acid (3,5-difluoro-pyridin-2-yl)-amide (Compound No. 1-892)

86.9 mg (42%) of the title compound was obtained as a pale yellow solid from 1-(2,3-dihydro-1,4-benzodioxin-5-yl)-3-(4-piperazin-1-yl-phenyl)-urea (142 mg) obtained in Example (244b) and (3,5-difluoro-pyridin-2-yl)-carbamic acid

tert-butyl ester (184 mg) obtained in Example (338a) in the same manner as in Example (294d). $^1$H NMR(400MHz, DMSO-d6):δ(ppm)=9.20(1H, s), 9.05(1H, s), 8.25(1H, d, J=2.4Hz), 8.05(1H, s), 7.91(1H, ddd, J=9.0, 9.0 and 2.4Hz), 7.67(1H, dd, J=8.0 and 1.4Hz), 7.30(2H, d, J=9.0Hz), 6.92(2H, d, J=9.0Hz), 6.71(1H, dd, J=8.0 and 8.0Hz), 6.46(1H, dd, J=8.0 and 1.4Hz), 4.36-4.31(2H, m), 4.28-4.24(2H, m), 3.62-3.56(4H, m), 3.09-3.03(4H, m).
MS(ES$^+$) m/z:511 (M + H)$^+$.
Melting point: 189-190˚C.

(Example 347) 4-{4-[3-(2,3-dimethoxy-phenyl)-ureido]-phenyl}-piperazine-1-carboxylic acid (3,5-difluoro-pyridin-2-yl)-amide (Compound No. 1-883)

30.3 mg (15%) of the title compound was obtained as a white solid from 1-(2,3-dimethoxy-phenyl)-3-(4-piperazin-1-yl-phenyl)-urea (143 mg) obtained in Example (334b) and (3,5-difluoro-pyridin-2-yl)-carbamic acid tert-butyl ester (221 mg) obtained in Example (338a) in the same manner as in Example (294d).
$^1$H NMR(400MHz,DMSO-d6):δ(ppm)=9.20(1H, s), 9.09(1H, s), 8.25(1H, d, J=2.4Hz), 8.24(1H, s), 7.91(1H, ddd, J=9.0, 9.0 and 2.4Hz), 7.79(1H, d, J=8.2Hz), 7.32(2H, d, J=9.0Hz), 6.95(1H, dd, J=8.2 and 8.2Hz), 6.93(2H, d, J=9.0Hz), 6.64 (1H, d, J=8.2Hz), 3.79(3H, s), 3.75(3H, s), 3.59(4H, t, J=4.5Hz), 3.07(4H, t, J=4.5Hz).
MS(ES$^+$) m/z:513 (M + H)$^+$.
Melting point: 175-177˚C.

(Example 348) 4-{4-[3-(2-methoxy-5-methyl-phenyl)-ureido]-phenyl}-3,6-dihydro-2H-pyridine-1-carboxylic acid (3,5-difluoro-pyridin-2-yl)-amide (Compound No. 5-84)

46 mg (47%) of the title compound was obtained as a yellow solid from 1-(2-methoxy-phenyl)-3-[4-(1,2,3,6-tetrahydro-pyridin-4-yl)-phenyl]-urea (67 mg) obtained in Example (258b) and (3,5-difluoro-pyridin-2-yl)-carbamic acid tert-butyl ester (69 mg) obtained in Example (338a) in the same manner as in Example (294d). $^1$H NMR(400MHz,DMSO-d6):δ(ppm)=2.21(3H, s), 2.53-2.46(2H, m), 3.65(2H, t, J=5.4Hz), 3.82(3H, s), 4.12(2H, brs), 6.12(1H, brs), 6.73(1H, dd, J=8.2 and 1.6Hz), 6.88(1H, d, J=8.2Hz), 7.38(2H, d, J=9.0Hz), 7.43(2H, d, J=9.0Hz), 7.90(1H, ddd, J=9.8, 8.6 and 2.7Hz), 7.97(1H, d, J=1.9Hz), 8.15(1H, s), 8.26(1H, d, J=2.7Hz), 9.14(1H, s), 9.34(1H, s).
MS(APCI) m/z:494 (M + H)$^+$.
Melting point: >250˚C.

(Example 349) 4-{4-[3-(2-methoxy-phenyl)-ureido]-phenyl}-3,6-dihydro-2H-pyridine-1-carboxylic acid (3,5-difluoro-pyridin-2-yl)-amide (Compound No. 5-82)

25 mg (26%) of the title compound was obtained as a yellow solid from 1-(2-methoxy-5-methyl-phenyl)-3-[4-(1,2,3,6-tetrahydro-pyridin-4-yl)-phenyl]-urea (65 mg) obtained in Example (230b) and (3,5-difluoro-pyridin-2-yl)-carbamic acid tert-butyl ester (69 mg) obtained in Example (338a) in the same manner as in Example (294d).
$^1$H NMR(400MHz,DMSO-d6):δ(ppm)=2.56-2.45(2H, m), 3.65(2H, t, J=5.2Hz), 3.86(3H, s), 4.12(2H, brs), 6.12(1H, brs), 6.96-6.85(2H, m), 7.00(1H, d, J=7.9Hz), 7.39(2H, d, J=8.6Hz), 7.43(2H, d, J=8.6Hz), 7.94-7.87(1H, m), 8.11(1H, brd, J=7.9Hz), 8.21(1H, s), 8.26(1H, d, J=2.3Hz), 9.15(1H, s), 9.35(1H, s).
MS(APCI) m/z:480 (M + H)$^+$.
Melting point: 186-189˚C.

(Example 350) 4- {4-[3 -(2-fluoro-phenyl)-ureido] -phenyl} -3,6-dihydro-2H-pyridine-1-carboxylic acid (3,5-difluoro-pyridin-2-yl)-amide (Compound No. 5-81)

14 mg (15%) of the title compound was obtained as a dark reddish-brown solid from 1-(2-fluoro-phenyl)-3-[4-(1,2,3,6-tetrahydro-pyridin-4-yl)-phenyl]-urea (62 mg) obtained in Example (260b) and (3,5-difluoro-pyridin-2-yl)-carbamic acid tert-butyl ester (69 mg) obtained in Example (338a) in the same manner as in Example (294d).
$^1$H NMR(400MHz,DMSO-d6):δ(ppm)=2.53-2.45(2H, m), 3.65(2H, brt, J=5.1Hz), 4.12(2H, brs), 6.13(1H, brs), 6.99(1H, dd, J=13.1 and 7.6Hz), 7.13(1H, dd, J=7.6 and 7.6Hz), 7.22(1H, dd, J=11.7 and 8.2Hz), 7.40(2H, d, J=9.0Hz), 7.44(2H, d, J=8.6Hz), 7.91(1H, dd like, J=9.2 and 9.2Hz), 8.14(1H, dd, J=8.2 and 8.2Hz), 8.25(1H, brs), 8.54(1H, brs), 9.11(1H, s), 9.15(1H, s).
MS(APCI) m/z:468 (M + H)$^+$.
Melting point: 200-204˚C.

(Example 351) 4-{4-[3-(3-ethoxy-phenyl)-ureido]-phenyl}-3,6-dihydro-2H-pyridine-1-carboxylic acid (3,5-difluoro-pyridin-2-yl)-amide (Compound No. 5-83)

(351a) 4-{4-[3-(3-ethoxy-phenyl)-ureido]-phenyl}-3,6-dihydro-2H-pyridine-1-carboxylic acid tert-butyl ester

745 mg (quantitative yield) of the title compound was obtained as a brown solid from 4-(4-aminophenyl)-3,6-dihydro-2H-pyridine-1-carboxylic acid tert-butyl ester (400 mg) obtained in Example (229a) and 3-ethoxyphenyl isocyanate (248 $\mu$l) in the same manner as in Example 1.
MS(APCI) m/z:438 $(M + H)^+$.

(351b) 1-(3-ethoxy-phenyl)-3-[4-(1,2,3,6-tetrahydro-pyridin-4-yl)-phenyl]-urea

474 mg (two steps, 96%) of the title compound was obtained as a yellow brown solid from 4-{4-[3-(3-ethoxy-phenyl)-ureido]-phenyl}-3,6-dihydro-2H-pyridine-1-carboxylic acid tert-butyl ester (745 mg) obtained in Example (351a) in the same manner as in Example (47a).
MS(APCI) m/z:338 $(M + H)^+$.

(351c) 4-{4-[3-(3-ethoxy-phenyl)-ureido]-phenyl]-3,6-dihydro-2H-pyridine-1-carboxylic acid (3,5-difluoro-pyridin-2-yl)-amide

10 mg (10%) of the title compound was obtained as a beige solid from 1-(3-ethoxy-phenyl)-3-[4-(1,2,3,6-tetrahydro-pyridin-4-yl)-phenyl]-urea (67 mg) obtained in Example (351b) and (3,5-difluoro-pyridin-2-yl)-carbamic acid tert-butyl ester (69 mg) obtained in Example (338a) in the same manner as in Example (294d).
$^1$H NMR(400MHz,DMSO-d6):$\delta$(ppm)=1.30(3H, t, J=6.2Hz), 2.54-2.44(2H, m), 3.65(2H, t, J=5.2Hz), 3.97(2H, q, J=6.3Hz), 4.12(2H, brs), 6.12(1H, brs), 6.52(1H, brd, J=8.2Hz), 6.89(1H, brd, J=8.6Hz), 7.18-7.11(2H, m), 7.38(2H, d, J=8.6Hz), 7.43(2H, brd, J=7.4Hz), 7.91(1H, dd like, J=9.2 and 9.2Hz), 8.26(1H, brs), 8.65(1H, s), 8.69(1H, s), 9.15(1H, s).
MS(APCI) m/z:494 $(M + H)^+$.
Melting point: 188-191˚C.

(Example 352) 4-{4-[3-(2-methoxy-5-methyl-phenyl)-ureido]-phenyl}-piperidine-1-carboxylic acid (3,5-difluoro-pyridin-2-yl)-amide (Compound No. 4-84)

22 mg (22%) of the title compound was obtained as a pale beige solid from 1-(2-methoxy-5-methyl-phenyl)-3-(4-piperidin-4-yl-phenyl)-urea (68 mg) obtained in Example (228e) and (3,5-difluoro-pyridin-2-yl)-carbamic acid tert-butyl ester (69 mg) obtained in Example (338a) in the same manner as in Example (294d).
$^1$H NMR(400MHz,DMSO-d6):$\delta$(ppm)=1.61-1.47(2H, m), 1.83-1.73(2H, m), 2.23(3H, s), 2.75-2.65(1H, m), 2.91(2H, brt, J=11.9Hz), 3.84(3H, s), 4.22(2H, brd, J=12.9Hz), 6.74(1H, dd, J=8.0 and 1.4Hz), 6.89(1H, d, J=8.2Hz), 7.17(2H, d, J=8.6Hz), 7.40(2H, d, J=8.2Hz), 7.91(1H, ddd like, J=10.6, 8.2 and 2.7Hz), 7.99(1H, d, J=1.6Hz), 8.14(1H, s), 8.27(1H, d, J=2.7Hz), 9.12(1H, s), 9.24(1H, s).
MS(APCI) m/z:496 $(M + H)^+$.
Melting point: 136-140˚C.

(Example 353)4-{4-[3-(2-methoxy-phenyl)-ureido]-phenyl}-piperidine-1-carboxylic acid (3,5-difluoro-pyridin-2-yl)-amide (Compound No. 4-82)

14 mg (15%) of the title compound was obtained as a pale pink solid from 1-(2-methoxy-phenyl)-3-(4-piperidin-4-yl-phenyl)-urea (65 mg) obtained in Example (261b) and (3,5-difluoro-pyridin-2-yl)-carbamic acid tert-butyl ester (69 mg) obtained in Example (338a) in the same manner as in Example (294d).
$^1$H NMR(400MHz,DMSO-d6):$\delta$(ppm)=1.62-1.48(2H, m), 1.84-1.74(2H, m), 2.75-2.65(1H, m), 2.91(2H, t, J=12.5Hz), 3.88(3H, s), 4.22(2H, brd, J=12.5Hz), 6.98-6.87(2H, m), 7.02(1H, d, J=7.8Hz), 7.18(2H, d, J=7.8Hz), 7.40(2H, d, J=7.4Hz), 7.93(1H, dd, J=9.0 and 9.0Hz), 8.14(1H, d, J=7.9Hz), 8.20(1H, s), 8.27(1H, s), 9.12(1H, s), 9.26(1H, s).
MS(APCI) m/z:482 $(M + H)^+$.
Melting point: >250˚C.

(Example 354) 4-{4-[3-(3,5-dimethoxy-phenyl)-ureido]-phenyl}-piperidine-1-carboxylic acid (3,5-difluoro-pyridin-2-yl)-amide (Compound No. 4-85)

8.9 mg (8.7%) of the title compound was obtained as a pink solid from 1-(3,5-dimethoxy-phenyl)-3-(4-piperidin-4-yl-phenyl)-urea (71 mg) obtained in Example (262b) and (3,5-difluoro-pyridin-2-yl)-carbamic acid tert-butyl ester (69 mg) obtained in Example (338a) in the same manner as in Example (294d).
$^1$H NMR(400MHz,DMSO-d6):δ(ppm)=1.61-1.47(2H, m), 1.82-1.73(2H, m), 2.76-2.63(1H, m), 2.90(2H, t, J=12.7Hz), 3.72(6H, s), 4.22(2H, brd, J=12.9Hz), 6.14(1H, dd, J=2.0 and 2.0Hz), 6.68(2H, d, J=2.0Hz), 7.17(2H, d, J=8.6Hz), 7.38 (2H, d, J=8.2Hz), 7.91(1H, ddd, J=10.5, 8.2 and 2.3Hz), 8.27(1H, d, J=2.3Hz), 8.56(1H, s), 8.64(1H, s), 9.12(1H, s).
MS(APCI) m/z:512 $(M + H)^+$.
Melting point: 193-196˚C.

(Example 355) 4-{4-[3-(3-ethoxy-phenyl)-ureido]-phenyl}-piperidine-1-carboxylic acid (3,5-difluoro-pyridin-2-yl)-amide (Compound No. 4-83)

22 mg (22%) of the title compound was obtained as a light brown solid from 1-(3-ethoxy-phenyl)-3-(4-piperidin-4-yl-phenyl)-urea (68 mg) obtained in Example (264b) and (3,5-difluoro-pyridin-2-yl)-carbamic acid tert-butyl ester (69 mg) obtained in Example (338a) in the same manner as in Example (294d).
$^1$H NMR(400MHz,DMSO-d6):δ(ppm)=1.33(3H, t, J=6.8Hz), 1.62-1.47(2H, m), 1.83-1.73(2H, m), 2.75-2.64(IH, m), 2.91 (2H, t, J=11.9Hz), 4.00(2H, q, J=6.9Hz), 4.23(2H, brd, J=12.9Hz), 6.54(IH, dd, J=7.8 and 2.4Hz), 6.91(IH, brd, J=7.9Hz), 7.22-7.13(4H, m), 7.39(2H, d, J=8.2Hz), 7.93(1H, ddd, J=10.6, 8.2 and 2.4Hz), 8.27(IH, d, J=2.7Hz), 8.59(IH, s), 8.63 (IH, s), 9.13(1H, s).
MS(APCI) m/z:496 $(M + H)^+$.
Melting point: 189-193˚C.

(Example 356)4-{4-[3-(2-methoxy-5-methyl-phenyl)-ureido]-phenyl}-piperazine-1-carboxylic acid (3-cyano-pyridin-2-yl)-amide (Compound No. 1-713)

(356a) (3-cyano-pyridin-2-yl)-carbamic acid tert-butyl ester

2.90 g (66%) of the title compound was obtained as a yellow solid from 3-cyano-pyridin-2-ylamine (2.62 g) and di-tert-butyl dicarbonate (4.36 g) in the same manner as in Example (335b).
$^1$H NMR(400MHz,$CDCl_3$):δ(ppm)=8.59(1H, dd, J=5.0 and 1.8Hz), 7.94(1H, dd, J=7.8 and 1.8Hz), 7.55(1H, brs), 7.13 (1H, dd, J=7.8 and 5.0Hz), 1.56(9H, s) (356b) 4-{4-[3-(2-methoxy-5-methyl-phenyl)-ureido]-phenyl}-piperazine-1-carboxylic acid (3-cyano-pyridin-2-yl)-amide
163 mg (81 %) of the title compound was obtained as a pale yellow solid from 1-(2-methoxy-5-methyl-phenyl)-3-(4-piperazin-1-yl-phenyl)-urea (136 mg) obtained in Example (47a) and (3-cyano-pyridin-2-yl)-carbamic acid tert-butyl ester (105 mg) obtained in Example (356a) in the same manner as in Example (294d).
$^1$H NMR(400MHz,DMSO-d6):δ(ppm)=9.81(1H, s), 9.06(1H, s), 8.60-8.53(1H, m), 8.21(1H, dd, J=7.4 and 1.9Hz), 8.04 (1H, s), 7.96(1H, d, J=2.0Hz), 7.31(2H, d, J=9.0Hz), 7.29-7.22(1H, m), 6.92(2H, d, J=9.0Hz), 6.86(1H, d, J=8.2Hz), 6.70 (1H, dd, J=8.2 and 2.0Hz), 3.82(3H, s), 3.64(4H, t, J=4.7Hz), 3.08(4H, t, J=4.7Hz), 2.22(3H, s).
MS($ES^+$) m/z:486 $(M + H)^+$.
Melting point: 196-197˚C.

(Example 357) 4-{4-[3-(2-ethoxy-phenyl)-ureido]-phenyl}-piperazine-1-carboxylic acid (3-cyano-pyridin-2-yl)-amide (Compound No. 1-708)

129 mg (66%) of the title compound was obtained as a white solid from 1-(2-ethoxy-phenyl)-3-(4-piperazin-1-yl-phenyl)-urea (136 mg) obtained in Example (80a) and (3-cyano-pyridin-2-yl)-carbamic acid tert-butyl ester (105 mg) obtained in Example (356a) in the same manner as in Example (294d).
$^1$H NMR(400MHz,DMSO-d6):δ(ppm)=9.84(1H, s), 9.18(1H, s), 8.60(IH, dd, J=4.9 and 1.8Hz), 8.24(1H, dd, J=7.6 and 1.8Hz), 8.13 (1H, dd, J=7. 8 and 1.7Hz), 7.98(1H, s), 7.35(2H, d, J=9.OHz), 7.30(1H, dd, J=7.6 and 4.9Hz), 7.00(1H, dd, J=7.8 and 1.7Hz), 6.95(2H, d, J=9.OHz), 6.91(1H, ddd, J=7.8, 7.6 and 1.7Hz), 6.87(1H, ddd, J=7.8, 7.6 and 1.7Hz), 4.13(2H, q, J=6.9Hz), 3.65(4H, t, J=4.7Hz), 3.10(4H, t, J=4.7Hz), 1.41 (3H, t, J=6.9Hz).
MS($ES^+$) m/z:486 $(M + H)^+$.
Melting point: 181-182˚C.

(Example 358) 4-{4-[3-(3-ethoxy-phenyl)-ureido]-phenyl}-piperazine-1-carboxylic acid (3-cyano-pyridin-2-yl)-amide (Compound No. 1-879)

15.9 mg (8%) of the title compound was obtained as a grey solid from 1-(3-ethoxy-phenyl)-3-(4-piperazin-1-yl-phenyl)-urea (136 mg) obtained in Example (215b) and (3-cyano-pyridin-2-yl)-carbamic acid tert-butyl ester (105 mg)

obtained in Example (356a) in the same manner as in Example (294d).
$^1$H NMR(400MHz,DMSO-d6):8(ppm)=9.84(1H, s), 8.63-8.58(1H, m), 8.57(1H, s), 8.43(1H, s), 8.24(1H, dd, J=7.7 and 1.8Hz), 7.33(2H, d, J=9.0Hz), 7.31-7.27(1H, m), 7.18(1H, dd, J=2.2 and 1.2Hz), 7.15(1H, dd, J=8.0 and 8.0Hz), 6.94 (2H, d, J=9.0Hz), 6.89(1H, dd, J=8.0 and 1.2Hz), 6.52(1H, dd, J=8.0 and 2.2Hz), 3.99(2H, q, J=6.9Hz), 3.65(4H, t, J=4.9Hz), 3.09(4H, t, J=4.9Hz), 1.32(3H, t, J=6.9Hz).
MS(ES$^+$) m/z:486 (M + H)$^+$.
Melting point: 121-123˚C.

(Example 359) 4-{4-[3-(3,5-dimethoxy-phenyl)-ureido]-phenyl}-piperazine-1-carboxylic acid (3-cyano-pyridin-2-yl)-amide (Compound No. 1-715)

96.7 mg (48%) of the title compound was obtained as a light brown solid from 1-(3,5-dimethoxy-phenyl)-3-(4-piperazin-1-yl-phenyl)-urea (143 mg) obtained in Example (216b) and (3-cyano-pyridin-2-yl)-carbamic acid tert-butyl ester (105 mg) obtained in Example (356a) in the same manner as in Example (294d).
$^1$H NMR(400MHz,DMSO-d6):δ(ppm)=9.81(1H, s), 8.57(1H, dd, J=4.9 and 1.7Hz), 8.55(1H, s), 8.37(1H, s), 8.21(1H, dd, J=7.6 and 1.7Hz), 7.29(2H, d, J=9.OHz), 7.27(1H, dd, J=7.6 and 4.9Hz), 6.91(2H, d, J=9.OHz), 6.65(2H, d, J=2.3Hz), 6.10(1H, t, J=2.3Hz), 3.70(6H, s), 3.64(4H, t, J=4.9Hz), 3.08(4H, t, J=4.9Hz).
MS(ES$^+$) m/z:502 (M + H)$^+$.
Melting point: 182-184˚C.

(Example 360) 4- {4-[3-(2-trifluoromethoxy-phenyl)-ureido]-phenyl}-piperazine-1-carboxylic acid (3-cyano-pyridin-2-yl)-amide (Compound No. 1-869)

165 mg (78%) of the title compound was obtained as a white solid from 1-(2-trifluoromethoxy-phenyl)-3-(4-piperazin-1-yl-phenyl)-urea (152 mg) obtained in Example (240b) and (3-cyano-pyridin-2-yl)-carbamic acid tert-butyl ester (105 mg) obtained in Example (356a) in the same manner as in Example (294d).
$^1$H NMR(400MHz,DMSO-d6):δ(ppm)=9.85(1H, s), 9.09(1H, s), 8.60(1H, dd, J=5.1 and 1.4Hz), 8.38(1H, s), 8.29(1H, dd, J=8.4 and 1.4Hz), 8.24(1H, dd, J=7.8 and 2.0Hz), 7.40-7.27(3H, m), 7.35(2H, d, J=9.0Hz), 7.07(1H, ddd, J=7.8, 7.8 and 2.0Hz), 6.96(2H, d, J=9.0Hz), 3.65(4H, t, J=4.6Hz), 3.11(4H, t, J=4.6Hz).
MS(ES$^+$) m/z:526 (M + H)$^+$.
Melting point: 200-202˚C.

(Example 361) 4-{4-[3-(2-difluoromethoxy-phenyl)-ureido]-phenyl}-piperazine-1-carboxylic acid (3-cyano-pyridin-2-yl)-amide (Compound No. 1-872)

178 mg (88%) of the title compound was obtained as a white solid from 1-(2-difluoromethoxy-phenyl)-3-(4-piperazin-1-yl-phenyl)-urea (145 mg) obtained in Example (245a) and (3-cyano-pyridin-2-yl)-carbamic acid tert-butyl ester (105 mg) obtained in Example (356a) in the same manner as in Example (294d).
$^1$H NMR(400MHz,DMSO-d6):δ(ppm)=9.84(1H, s), 9.15(1H, s), 8.60(1H, dd, J=4.9 and 1.4Hz), 8.26-8.21(3H, m), 7.25 (1H, t, J=73.7Hz), 7.34(2H, d, J=9.0Hz), 7.29(1H, dd, J=7.2 and 4.9Hz), 7.20(1H, dd, J=7.8 and 7.8Hz), 7.18(1H, d, J=7.8Hz), 7.01(1H, dd, J=7.8 and 7.8Hz), 6.96(2H, d, J=9.0Hz), 3.65(4H, t, J=4.7Hz), 3.10(4H, t, J=4.7Hz).
MS(ES$^+$) m/z:508 (M + H)$^+$.
Melting point: 173-175˚C.

(Example 362) 4-{4-[3-(2,3-dihydro-1,4-benzodioxin-5-yl)-ureido]-phenyl}-piperazine-1-carboxylic acid (3-cyano-pyridin-2-yl)-amide (Compound No. 1-875)

80.4 mg (40%) of the title compound was obtained as a grey solid from 1-(2,3-dihydro-1,4-benzodioxin-5-yl)-3-(4-piperazin-1-yl-phenyl)-urea (142 mg) obtained in Example (244b) and (3-cyano-pyridin-2-yl)-carbamic acid tert-butyl ester (105 mg) obtained in Example (356a) in the same manner as in Example (294d).
$^1$H NMR(400MHz,DMSO-d6):δ(ppm)=9.85(1H, s), 9.09(1H, s), 8.60(1H, dd, J=4.9 and 1.8Hz), 8.24(1H, dd, J=7.8 and 1.8Hz), 8.08(1H, s), 7.70(1H, dd, J=8.2 and 1.6Hz), 7.33(2H, d, J=9.0Hz), 7.30(1H, dd, J=7.8 and 4.9Hz), 6.94(2H, d, J=9.0Hz), 6.74(1H, dd, J=8.2 and 8.2Hz), 6.48(1H, dd, J=8.2 and 1.6Hz), 4.37-4.33(2H, m), 4.29-4.25(2H, m), 3.65(4H, t, J=4.7Hz), 3.09(4H, t, J=4.7Hz).
MS(ES$^+$) m/z:500 (M + H)$^+$.
Melting point: 163-165˚C.

(Example 363) 4-{5-[3-(2-ethoxy-5-fluoro-phenyl)-ureido]-pyridin-2-yl}-piperazine-1-carboxylic acid (3-cyano-pyridin-2-yl)-amide (Compound No. 1-742)

139 mg (69%) of the title compound was obtained as a white solid from 1-(2-ethoxy-5-fluoro-phenyl)-3-(6-piperazin-1-yl-pyridin-3-yl)-urea (144 mg) obtained in Example (300b) and (3-cyano-pyridin-2-yl)-carbamic acid tert-butyl ester (105 mg) obtained in Example (356a) in the same manner as in Example (294d).
$^1$H NMR(400MHz,DMSO-d6):δ(ppm)=9.85(1H, s), 9.32(1H, s), 8.60(1H, dd, J=4.8 and 1.8Hz), 8.25 (1H, dd, J=7.6 and 1.8Hz), 8.23 (1H, s), 8.18 (1H, d, J=2.7Hz), 8.02(1H, dd, J=11.3 and 3.1Hz), 7.78(1H, dd, J=9.0 and 2.7Hz), 7.30(1H, dd, J=7.6 and 4.8Hz), 7.00(1H, dd, J=9.0 and 5.5Hz), 6.91(1H, d, J=9.0Hz), 6.72(1H, ddd, J=9.0, 8.6 and 3.1Hz), 4.12 (2H, q, J=7.0Hz), 3.62(4H, t, J=4.9Hz), 3.48(4H, t, J=4.9Hz), 1.40(3H, t, J=7.0Hz).
MS(ES$^+$) m/z:505 (M + H)$^+$.
Melting point: 198-199˚C.

(Example 364) 4-{4-[3-(2,3-dimethoxy-phenyl)-ureido]-phenyl}-piperazine-1-carboxylic acid (3-cyano-pyridin-2-yl)-amide (Compound No. 1-880)

36.4 mg (18%) of the title compound was obtained as a grey solid from 1-(2,3-dimethoxy-phenyl)-3-(4-piperazin-1-yl-phenyl)-urea (143 mg) obtained in Example (334b) and (3-cyano-pyridin-2-yl)-carbamic acid tert-butyl ester (210 mg) obtained in Example (356a) in the same manner as in Example (294d).
$^1$H NMR(400MHz,DMSO-d6):δ(ppm)=9.81(1H, s), 9.09(1H, s), 8.57(1H, dd, J=4.9 and 1.8Hz), 8.24(1H, s), 8.22(1H, dd, J=7.6 and 1.8Hz), 7.79(1H, dd, J=8.6 and 1.2Hz), 7.32(2H, d, J=9.0Hz), 7.28(1H, dd, J=7.6 and 4.9Hz), 6.95(1H, dd, J=8.6 and 8.2Hz), 6.93(2H, d, J=9.0Hz), 6.64(1H, dd, J=8.2 and 1.2Hz), 3.80(3H, s), 3.75(3H, s), 3.68-3.61(4H, m), 3.68-3.61(4H, m).
MS(ES$^+$) m/z:502 (M + H)$^+$.
Melting point: 109-111˚C.

(Example 365) 4-{4-[3-(2-methoxy-5-methyl-phenyl)-ureido]-phenyl}-3,6-dihydro-2H-pyridine-1-carboxylic acid (3-cyano-pyridin-2-yl)-amide (Compound No. 5-94)

23 mg (24%) of the title compound was obtained as a yellow solid from 1-(2-methoxy-5-methyl-phenyl)-3-[4-(1,2,3,6-tetrahydro-pyridin-4-yl)-phenyl]-urea (67 mg) obtained in Example (230b) and (3-cyano-pyridin-2-yl)-carbamic acid tert-butyl ester (66 mg) obtained in Example (356a) in the same manner as in Example (294d). $^1$H NMR (400MHz,DMSO-d6):δ(ppm)=2.23(3H, s), 2.58-2.47(2H, m), 3.71(3H, t, J=5.5Hz), 3.84(2H, s), 4.18(1H, brs), 6.15(1H, brs), 6.75(1H, d, J=7.4Hz), 6.90(1H, d, J=8.3Hz), 7.30(1H, dd, J=7.5 and 4.7Hz), 7.41(2H, d, J=9.0Hz), 7.45(2H, d, J=8.7Hz), 7.99(1H, s), 8.18(1H, s), 8.24(1H, d, J=7.8Hz), 8.60(1H, d, J=5.0Hz), 9.36(1H, s), 9.74(1H, s).
MS(APCI) m/z:483 (M + H)$^+$.
Melting point: 190-194˚C.

(Example 366) 4-{4-[3-(2-methoxy-phenyl)-ureido]-phenyl}-3,6-dihydro-2H-pyridine-1-carboxylic acid (3-cyano-pyridin-2-yl)-amide (Compound No. 5-92)

11 mg (14%) of the title compound was obtained as a beige solid from 1-(2-methoxy-phenyl)-3-[4-(1,2,3,6-tetrahydro-pyridin-4-yl)-phenyl]-urea (55 mg) obtained in Example (258b) and (3-cyano-pyridin-2-yl)-carbamic acid tert-butyl ester (51 mg) obtained in Example (356a) in the same manner as in Example (294d).
$^1$H NMR(400MHz,DMSO-d6):δ(ppm)=2.57-2.48(2H, m), 3.71(2H, t, J=5.2Hz), 3.89(3H, s), 4.19(2H, brs), 6.15(1H, brs), 6.98-6.87(2H, m), 7.03(1H, dd, J=8.0 and 1.3Hz), 7.30(1H, dd, J=7.1 and 5.4Hz), 7.41(2H, d, J=8.6Hz), 7.46(2H, d, J=8.6Hz), 8.14(1H, dd, J=7.8 and 1.9Hz), 8.26-8.22(2H, m), 8.60(1H, brd, J=2.8Hz), 9.37(1H, s), 9.75(1H, brs).
MS(APCI) m/z:469 (M + H)$^+$.
Melting point: 174-178˚C.

(Example 367) 4- {4-[3-(3,5-dimethoxy-phenyl)-ureido]-phenyl}-3,6-dihydro-2H-pyridine-1-carboxylic acid (3-cyano-pyridin-2-yl)-amide (Compound No. 5-95)

4 mg (5%) of the title compound was obtained as a beige solid from 1-(3,5-dimethoxy-phenyl)-3-[4-(1,2,3,6-tetrahydro-pyridin-4-yl)-phenyl]-urea (60 mg) obtained in Example (259b) and (3-cyano-pyridin-2-yl)-carbamic acid tert-butyl ester (51 mg) obtained in Example (356a) in the same manner as in Example (294d).
$^1$H NMR(400MHz,DMSO-d6):δ(ppm)=2.57-2.44(2H, m), 3.72(6H, s), 3.76-3.66(2H, m), 4.18(2H, brs), 6.15(2H, brs), 6.69(2H, s), 7.33-7.27(1H, m), 7.40(2H, d, J=8.6Hz), 7.45(2H, d, J=8.6Hz), 8.24(1H, d, J=7.5Hz), 8.63-8.58(1H, m),

8.69(1H, s), 8.71(1H, s), 9.75(1H, brs).
MS(APCI) m/z:499 $(M + H)^+$.
Melting point: 150-153˚C.

(Example 368) 4-{4-[3-(2-fluoro-phenyl)-ureido]-phenyl}-3,6-dihydro-2H-pyridine-1-carboxylic acid (3-cyano-pyridin-2-yl)-amide (Compound No. 5-91)

26 mg (33%) of the title compound was obtained as a beige solid from 1-(2-fluoro-phenyl)-3-[4-(1,2,3,6-tetrahydro-pyridin-4-yl)-phenyl]-urea (53 mg) obtained in Example (260b) and (3-cyano-pyridin-2-yl)-carbamic acid tert-butyl ester (51 mg) obtained in Example (356a) in the same manner as in Example (294d).
$^1$H NMR(400MHz,DMSO-d6):δ(ppm)=2.57-2.48(2H, m), 3.71(2H, t, J=5.3Hz), 4.19(2H, brs), 6.16(1H, brs), 7.05-6.98 (1H, m), 7.15(1H, dd, J=7.8 and 7.8Hz), 7.33-7.21(2H, m), 7.43(2H, d, J=9.0Hz), 7.46(2H, d, J=8.6Hz), 8.16(1H, ddd, J=8.2, 8.2 and 1.6Hz), 8.24(1H, dd, J=7.9 and 1.5Hz), 8.63-8.55(2H, m), 9.13(1H, brs), 9.75(1H, brs).
MS(APCI) m/z:457 $(M + H)^+$.
Melting point: 185-187˚C.

(Example 369) 4-{4-[3-(2-methoxy-5-methyl-phenyl)-ureido]-phenyl}-piperidine-l-carboxylic acid (3-cyano-pyridin-2-yl)-amide (Compound No. 4-94)

18 mg (22%) of the title compound was obtained as a pale beige solid from 1-(2-methoxy-5-methyl-phenyl)-3-(4-piperidin-4-yl-phenyl)-urea (58 mg) obtained in Example (228e) and (3-cyano-pyridin-2-yl)-carbamic acid tert-butyl ester (51 mg) obtained in Example (356a) in the same manner as in Example (294d).
$^1$H NMR(400MHz,DMSO-d6):δ(ppm)=1.69-1.56(2H, m), 1.81-1.72(2H, m), 2.23(3H, s), 2.79-2.69(1H, m), 2.96(2H, t, J=12.6Hz), 3.84(3H, s), 4.25(2H, brd, J=12.9Hz), 6.74(1H, dd, J=8.4 and 1.8Hz), 6.89(1H, d, J=8.2Hz), 7.16(2H, d, J=8.6Hz), 7.28(1H, dd, J=7.6 and 4.9Hz), 7.38(2H, d, J=8.6Hz), 7.99(1H, d, J=2.0Hz), 8.14(1H, s), 8.24(1H, dd, J=7.7 and 1.8Hz), 8.59(1H, brd, J=4.3Hz), 9.24(1H, s), 9.78(1H, brs).
MS(APCI) m/z:485 $(M + H)^+$.
Melting point: 147-151˚C.

(Example 370) 4-{4-[3-(2-methoxy-phenyl)-ureido]-phenyl}-piperidine-1-carboxylic acid (3-cyano-pyridin-2-yl)-amide (Compound No. 4-92)

11 mg (14%) of the title compound was obtained as a lemon yellow solid from 1-(2-methoxy-phenyl)-3-[4-(1,2,3,6-tetrahydro-pyridin-4-yl)-phenyl]-urea (55 mg) obtained in Example (258b) and (3-cyano-pyridin-2-yl)-carbamic acid tert-butyl ester (51 mg) obtained in Example (356a) in the same manner as in Example (294d). $^1$H NMR (400MHz,DMSO-d6):δ(ppm)=1.69-1.55(2H, m), 1.81-1.72(2H, m), 2.80-2.69(1H, m), 2.96(2H, t, J=12.3Hz), 3.88(3H, s), 4.24(2H, brd, J=12.9Hz), 6.97-6.86(2H, m), 7.02(1H, dd, J=8.0 and 1.3Hz), 7.16(2H, d, J=8.6Hz), 7.28(1H, dd, J=7.4 and 5.1Hz), 7.38(2H, d, J=8.6Hz), 8.14(1H, dd, J=7.8 and 1.6Hz), 8.20(1H, s), 8.24(1H, dd, J=7.6 and 1.8Hz), 8.59(1H, dd, J=5.0 and 1.5Hz), 9.25(1H, s), 9.78(1H, brs)
MS(APCI) m/z:471 $(M + H)^+$.
Melting point: 197-200˚C.

(Example 371) 4-{4-[3-(2-fluoro-phenyl)-ureido]-phenyl}-piperidine-1-carboxylic acid (3-cyano-pyridin-2-yl)-amide (Compound No. 4-91)

10 mg (13%) of the title compound was obtained as a white solid from 1-(2-fluoro-phenyl)-3-(4-piperidin-4-yl-phenyl)-urea (53 mg) obtained in Example (263b) and (3-cyano-pyridin-2-yl)-carbamic acid tert-butyl ester (51 mg) obtained in Example (356a) in the same manner as in Example (294d).

$^l$H NMR(400MHz,DMSO-d6):b(ppm)=1.66-1.51(2H, m), 1.79-1.69(2H, m), 2.76-2.65(1H, m), 2.88(2H, t, J=12.3Hz), 4.32(2H, brd, J=13.3Hz), 7.05-6.96(1H, m), 7.17(2H, d, J=8.6Hz), 7.29-7.10(3H, m), 7.39(2H, d, J=8.6Hz), 8.06(1H, brs), 8.15(IH, dd, J=7.4 and 7.4Hz), 8.44(1H, brs), 8.65(1H, s), 9.14(1H, s), 9.81(IH, brs).
MS(APCI) m/z:459 $(M + H)^+$.
Melting point: >250˚C.

(Example 372) 4-{4-[3-(3-ethoxy-phenyl)-ureido]-phenyl}-piperidine-1-carboxylic acid (3-cyano-pyridin-2-yl)-amide (Compound No. 4-93)

8.5 mg (10%) of the title compound was obtained as a pale beige solid from 1-(3-ethoxy-phenyl)-3-(4-piperidin-4-yl-phenyl)-urea (58 mg) obtained in Example (264b) and (3-cyano-pyridin-2-yl)-carbamic acid tert-butyl ester (51 mg) obtained in Example (356a) in the same manner as in Example (294d).
$^1$H NMR(400MHz,DMSO-d6):δ(ppm)=1.32(3H, t, J=6.9Hz), 1.70-1.55(2H, m), 1.82-1.72(2H, m), 2.80-2.66(1H, m), 2.96 (2H, t, J=12.4Hz), 3.99(2H, q, J=6.8Hz), 4.24(2H, brd, J=12.5Hz), 6.53(1H, dd, J=8.2 and 2.3Hz), 6.91(1H, d, J=7.8Hz), 7.23-7.13(4H, m), 7.31-7.25(1H, m), 7.37(2H, d, J=8.6Hz), 8.24(1H, dd, J=7.7 and 1.8Hz), 8.66-8.55(3H, m), 9.77(1H, brs).
MS(APCI) m/z:485 $(M + H)^+$.
Melting point: 163-167˚C.

(Example 373) 4-{4-[3-(2-methoxy-4-methyl-phenyl)-ureido]-phenyl}-piperazine-1-carboxylic acid (3-methyl-pyridin-2-yl)-amide (Compound No. 1-902)

(373a) 2-methoxy-4-methyl-aniline (Org. Lett. 2000, 2, 277-280)

A suspension of 5-methyl-2-nitroanisole (5.02 g) and palladium-carbon (10%, 1.0 g) in anhydrous tetrahydrofuran (50 mL) was stirred under a hydrogen atmosphere at 70˚C for 39 hours. The reaction mixture was filtered and concentrated. The residue was purified by column chromatography (hexane:ethyl acetate 4:1) and 992 mg (24%) of the title compound was obtained as a dark brown oil.
MS($ES^+$) m/z:138 $(M+H)^+$.

(373b) 4-{4-[3-(2-methoxy-4-methyl-phenyl)-ureido]-phenyl}-piperazine-1-carboxylic acid tert-butyl ester

982 mg (77%) of the title compound was obtained as a pale beige solid from N-tert-butyloxycarbonyl-N'-(4-aminophenyl)-piperazine (1.99 g) and 2-methoxy-4-methyl-aniline obtained in Example (373a) in the same manner as in Example (41c). $^1$H NMR(400MHz,$CDCl_3$):δ(ppm)=7.91(1H, d, J=8.2Hz), 7.28-7.26(2H, m), 6.94(3H, brs), 6.78(1H, d, J=8.2Hz), 6.68(1H, s), 6.37(1H, brs), 3.79(3H, s), 3.59(4H, t, J=5.1Hz), 3.10(4H, brs), 2.32(3H, s), 1.49(9H, s).

(373c) 1-(2-methoxy-4-methyl-phenyl)-3-(4-piperazin-1-yl-phenyl)-urea

1.89 g (quantitative yield) of the title compound was obtained as a white solid from 4-{4-[3-(2-methoxy-4-methyl-phenyl)-ureido]-phenyl}-piperazine-1-carboxylic acid tert-butyl ester (2.44 g) obtained in Example (373b) in the same manner as in Example (47a).
$^1$H NMR(400MHz,DMSO-d6):δ(ppm)=9.00(1H, brs), 7.99(1H, s), 7.94(1H, d, J=7.8Hz), 7.27(2H, d, J=8.2Hz), 6.83(2H, d, J=8.2Hz), 6.81(1H, s), 6.66(1H, d, J=7.8Hz), 3.83(3H, s), 3.41(1H, brs), 2.92(4H, brs), 2.80(4H, brs), 2.23(3H, s).
(373d) 4-{4-[3-(2-methoxy-4-methyl-phenyl)-ureido]-phenyl}-piperazine-1-carboxylic acid (3-methyl-pyridin-2-yl)-amide
135 mg (44%) of the title compound was obtained as a white solid from 1-(2-methoxy-4-methyl-phenyl)-3-(4-piperazin-1-yl-phenyl)-urea (219 mg) obtained in Example (373c) and 2-tert-butoxycarbonylamino-3-methylpyridine (71 mg) obtained in Example (294c) in the same manner as in Example (294d).
$^1$H NMR(400MHz,DMSO-d6):δ(ppm)=9.02(1H, s), 8.86(1H, s), 8.19(1H, d, J=3.1Hz), 8.01(1H, s), 7.98(1H, d, J=8.2Hz), 7.60(1H, d, J=7.5Hz), 7.32(2H, d, J=9.0Hz), 7.11(1H, dd, J=7.4 and 5.1Hz), 6.94(2H, d, J=9.0Hz), 6.84(1H, d, J=1.6Hz), 6.69(1H, d, J=7.9Hz), 3.86(3H, s), 3.60(4H, t, J=4.7Hz), 3.06(4H, t, J=4.9Hz), 2.26(3H, s), 2.13(3H, s).
MS($ES^+$) m/z:475 $(M + H)^+$.
Melting point: 116-120˚C.

(Example 374) 4-{2-fluoro-4-[3-(2-methoxy-phenyl)-ureido]-phenyl}-piperazine-1-carboxylic acid (3-methyl-pyridin-2-yl)-amide (Compound No. 2-129)

(374a) 4- {2-fluoro-4-[3-(2-methoxy-phenyl)-ureido]-phenyl}-piperazine-1-carboxylic acid tert-butyl ester

2.67 g (97%) of the title compound was obtained as a pale pink solid from 4-(4-amino-2-fluoro-phenyl)-piperazine-1-carboxylic acid tert-butyl ester (1.77 g) obtained in Example (253b) and 2-methoxy-phenyl isocyanate (1.23 mL) in the same manner as in Example 1.
$^1$H NMR(400MHz,$CDCl_3$):δ(ppm)=8.04(1H, dd, J=7.8 and 1.5Hz), 7.28(1H, brs), 7.06(1H, brs), 7.04-6.94(4H, m), 6.86 (1H, dd, J=8.0 and 1.4Hz), 6.51(1H, brs), 3.84(3H, s), 3.60(4H, t, J=4.9Hz), 2.99(4H, t, J=4.5Hz), 1.49(9H, s).

(374b) 1-(3-fluoro-4-piperazin-1-yl-phenyl)-3-(2-methoxy-5-methyl-phenyl)-urea

2.00 g (99%) of the title compound was obtained as a pale purple solid from 4-{2-fluoro-4-[3-(2-methoxy-phenyl)-ureido]-phenyl}-piperazine-1-carboxylic acid tert-butyl ester (2.58 g) obtained in Example (374a) in the same manner as in Example (47a).
$^1$H NMR(400MHz,DMSO-d6):δ(ppm)=9.29(1H, brs), 8.16(1H, s), 8.09(1H, dd, J=7.8 and 2.0Hz), 7.42(1H, d, J=14.9Hz), 7.01-6.85(5H, m), 3.86(3H, s), 3.46(1H, brs), 2.83(8H, brs).

(374c) 4-{2-fluoro-4-[3-(2-methoxy-phenyl)-ureido]-phenyl}-piperazine-1-carboxylic acid (3-methyl-pyridin-2-yl)-amide

128 mg (54%) of the title compound was obtained as a light brown solid from 1-(3-fluoro-4-piperazin-1-yl-phenyl)-3-(2-methoxy-5-methyl-phenyl)-urea (172 mg) obtained in Example (374b) and 2-tert-butoxycarbonylamino-3-methylpyridine (55 mg) obtained in Example (294c) in the same manner as in Example (294d).
$^1$H NMR(400MHz,DMSO-d6):δ(ppm)=9.33(1H, s), 8.82(1H, s), 8.17(1H, s), 8.16(1H, d, J=7.4Hz), 8.09(1H, dd, J=7.8 and 1.6Hz), 7.57 (1H, d, J=6.3Hz), 7.46(1H, d, J=14.1Hz), 7.09(1H, dd, J=7.5 and 4.7Hz), 7.01-6.99(3H, m), 6.93(1H, ddd, J=7.6, 7.6 and 1.9Hz), 6.88(1H, ddd, J=7.5, 7.5 and 1.8Hz), 3.87(3H, s), 3.60(4H, t, J=4.SHz), 2.94(4H, t, J=4.5Hz), 2.13(3H, s).
MS(ES$^+$) m/z:479 (M + H)$^+$.
Melting point: 118-121˚C.

(Example 375) 4-{4-[3-(2-methoxy-3-methyl-phenyl)-ureido]-phenyl}-piperazine-1-carboxylic acid (3-methyl-pyridin-2-yl)-amide (Compound No. 1-897)

(375a) 2-methoxy-3-methylbenzoic acid methyl ester (J. Chem. Sic. 1937, 260-262)

A suspension of 2-hydroxy-3-methylbenzoic acid (10.1 g), iodomethane (9.1 mL) and potassium carbonate (18.4 g) in dimethylacetamide (100 mL) was heated at 60˚C for 20 hours and diluted with water, followed by extraction with ethyl acetate.
The organic layer was washed with water and saturated brine and dried over sodium sulfate and concentrated. The residue was purified by column chromatography (hexane:ethyl acetate 20:1) and 6.29 g (53%) of the title compound was obtained as pale yellow oil.
$^1$H NMR(400MHz,$CDCl_3$):δ(ppm)=7.62(1H, d, J=8.2Hz), 7.33(1H, d, J=7.4Hz), 7.05(1H, dd, J=7.7 and 7.7Hz), 3.92(3H, s), 3.92(3H, s), 2.33(3H, s).
(375b) 2-methoxy-3-methylbenzoic acid (Chem. Ber.1879, 12, 818 - 821.)
1N aqueous sodium hydroxide solution (70 mL) was added to a solution of 2-methoxy-3-methylbenzoic acid methyl ester (6.29 g) obtained in Example (375a) in tetrahydrofuran (140 mL) at room temperature. The reaction mixture was heated at 50˚C for 30 hours and concentrated and acidified with 1N hydrochloric acid. The deposited precipitate was collected by filtration, washed with water and dried under reduced pressure, and 5.15 g (89%) of the title compound was obtained as a white solid.
$^1$H NMR(400MHz,DMSO-d6):δ(ppm)=7.49(1H, d, J=8.2Hz), 7.37(1H, d, J=7.5Hz), 7.07(1H, dd, J=7.7 and 7.7Hz), 3.73 (3H, s), 3.73(3H, s).

(375c) 4-{4-[3-(2-methoxy-3-methyl-phenyl)-ureido]-phenyl}-piperazine-1-carboxylic acid tert-butyl ester

397 mg (8%) of the title compound was obtained as a brown amorphous material from 2-methoxy-3-methyl-benzoic acid (1.93 g) obtained in Example (375b) and N-tert-butyloxycarbonyl-N'-(4-aminophenyl)-piperazine (3.25 g) in the same manner as in Example (42b).
$^1$H NMR(400MHz,DMSO-d6):δ(ppm)=9.07(1H, s), 8.18(IH, s), 7.99(1H, d, J=7.8Hz), 7.31(2H, d, J=9.OHz), 6.93-6.88 (3H, m), 6.76(1H, d, J=7.OHz), 3.68(3H, s), 3.43(4H, brs), 2.99(4H, t, J=4.9Hz), 2.24(3H, s), 1.42(9H, s).
MS(ES$^+$) m/z:441 (M + H)$^+$.

(375d) 1-(2-methoxy-3-methyl-phenyl)-3-(4-piperazin-1-yl-phenyl)-urea

354 mg (quantitative yield) of the title compound was obtained as a yellow solid from 4-{4-[3-(2-methoxy-3-methyl-phenyl)-ureido]-phenyl}-piperazine-1-carboxylic acid tert-butyl ester (397 mg) obtained in Example (375c) in the same manner as in Example (47a).
MS(ES$^+$) m/z:341 (M + H)$^+$.

(375e) 4-{4-[3-(2-methoxy-3-methyl-phenyl)-ureido]-phenyl}-piperazine-1-carboxylic acid (3-methyl-pyridin-2-yl)-amide

85 mg (56%) of the title compound was obtained as a yellow solid from 1-(2-methoxy-3-methyl-phenyl)-3-(4-piperazin-1-yl-phenyl)-urea (109 mg) obtained in Example (375d) and 2-tert-butoxycarbonylamino-3-methylpyridine (95 mg) obtained in Example (294c) in the same manner as in Example (294d).
$^1$H NMR(400MHz,DMSO-d6):δ(ppm)=9.08(1H, s), 8.84(1H, s), 8.19(1H, s), 8.16(1H, d, J=4.7Hz), 8.00(1H, d, J=8.3Hz), 7.57(1H, d, J=7.1Hz), 7.32(2H, d, J=8.6Hz), 7.08(1H, dd, J=7.3 and 4.9Hz), 6.93(2H, d, J=9.0Hz), 6.91(1H, d, J=8.3Hz), 6.77(1H, d, J=7.5Hz), 3.69(3H, s), 3.60(4H, t, J=4.3Hz), 3.06(4H, t, J=4.7Hz), 2.24(3H, s), 2.12(3H, s).
MS(ES$^+$) m/z:474 (M + H)$^+$.
Melting point: 178-180˚C.

(Example 376) 4- {4-[3-(2-methoxy-pyridin-3-yl)-ureido]-phenyl}-piperazine-1-carboxylic acid (Compound No. 1-878)

(376a) 4-{4-[3-(2-methoxy-pyridin-3-yl)-ureido]-phenyl}-piperazine-1-carboxylic acid tert-butyl ester

1.41 g (87%) of the title compound was obtained as a white solid from 4-[4-(4-nitro-phenoxycarbonylamino)-phenyl]-piperazine-1-carboxylic acid tert-butyl ester (1.74 g) obtained in Example (224a) and 2-methoxypyridine-3-amine (471 mg) in the same manner as in Example (224b).
$^1$H NMR(400MHz,DMSO-d6):δ(ppm)=9.13(1H, s), 8.40(1H, d, J=7.8Hz), 8.26(1H, s), 7.75-7.74(1H, m), 7.32(2H, d, J=7.0Hz), 6.97-6.91(3H, m), 3.98(3H, s), 3.46(4H, brs), 3.01(4H, brs), 1.42(9H, s).
MS(ES$^+$) m/z:428 (M + H)$^+$.

(376b) 1-(2-methoxy-pyridin-3-yl)-3-(4-piperazin-1-yl-phenyl)-urea

948 mg (89%) of the title compound was obtained as a yellow solid from 4-{4-[3-(2-methoxy-pyridin-3-yl)-ureido]-phenyl}-piperazine-1-carboxylic acid tert-butyl ester (1.40 g) obtained in Example (376a) in the same manner as in Example (47a).
MS(ES$^+$) m/z:328 (M + H)$^+$.

(376c) 4-{4-[3-(2-methoxy-pyridin-3-yl)-ureido]-phenyl}-piperazine-1-carboxylic acid (3-methyl-pyridin-2-yl)-amide

84 mg (53%) of the title compound was obtained as a white solid from 1-(2-methoxy-pyridin-3-yl)-3-(4-piperazin-1-yl-phenyl)-urea (112 mg) obtained in Example (376b) and 2-tert-butoxycarbonylamino-3-methylpyridine (90 mg) obtained in Example (294c) in the same manner as in Example (294d).
$^1$H NMR(400MHz,DMSO-d6):δ(ppm)=9.14(1H, s), 8.88(1H, s), 8.40(1H, dd, J=7.8 and 1.9Hz), 8.26(1H, s), 8.19(1H, dd, J=4.7 and 1.6Hz), 7.74(1H, dd, J=4.8 and 1.8Hz), 7.60(1H, d, J=7.5Hz), 7.33(2H, d, J=9.0Hz), 7.11(1H, dd, J=7.2 and 4.9Hz), 6.97-6.93(3H, m), 3.97(3H, s), 3.60(4H, t, J=4.9Hz), 3.07(4H, t, J=4.9Hz), 2.13(3H, s).
MS(ES$^+$) m/z:462 (M + H)$^+$.
Melting point: 139-141˚C.

(Example 377) 1-(6-{4-[2-(2-chloro-phenyl)-2-hydroxy-acetyl]-piperazin-1-yl}-pyridin-3-yl)-3-(2-fluoro-phenyl)-urea (Compound No. 1-800)

Triethylamine (84 μl) and benzotriazol-1-yloxytris(dimethylamino)phosphonium hexafluorophosphate (BOP reagent) (199 mg) were added to a solution of 1-(2-fluoro-phenyl)-3-(6-piperazin-1-yl-pyridin-3-yl)-urea (95 mg) obtained in Example (222b) and 2-chloromandelic acid (56 mg) in dimethylformamide (3 mL) at room temperature. The reaction mixture was stirred at room temperature for three hours and diluted with an ammonium chloride aqueous solution, followed by extraction with ethyl acetate. The organic layer was washed with a saturated sodium hydrogen carbonate aqueous solution and saturated brine and dried over sodium sulfate and concentrated. The residue was purified by column chromatography (dichloromethane:ethyl acetate 2:1). The obtained solid was vigorously stirred in diisopropyl ether and was collected by filtration and dried under reduced pressure, and 46 mg (32%) of the title compound was obtained as a white solid.
$^1$H NMR(400MHz,DMSO-d6):δ(ppm)=8.83(1H, s), 8.51(1H, s), 8.13(1H, d, J=2.7Hz), 8.09(1H, ddd, J=8.4, 8.2 and 1.7Hz), 7.70(1H, dd, J=9.0 and 2.7Hz), 7.45(1H, dd, J=7.5 and 2.0Hz), 7.42(1H, dd, J=7.5 and 1.7Hz), 7.35(1H, ddd, J=7.5, 7.5 and 1.7Hz), 7.31(1H, ddd, J=7.5, 7.5 and 2.0Hz), 7.24-7.17(1H, m), 7.10(1H, dd, J=8.4 and 8.4Hz), 7.01-6.94 (1H, m), 6.83(1H, d, J=9.0Hz), 5.93(1H, d, J=6.8Hz), 5.66(1H, d, J=6.8Hz), 3.70-3.55(3H, m), 3.51-3.43(4H, m), 3.19-3.06 (1H, m).
MS(ES$^+$) m/z:484 (M + H)$^+$.
Melting point: 127-128˚C.

(Example 378) 1-(4-{4-[2-(2-chloro-phenyl)-2-hydroxy-acetyl]-piperazin-1-yl}-phenyl)-3-(2-methoxy-5-methyl-phenyl)-urea (Compound No. 1-805)

200 mg (25%) of the title compound was obtained as a white solid from 1-(2-methoxy-5-methyl-phenyl)-3-(4-piperazin-1-yl-phenyl)-urea (532 mg) obtained in Example (47a) and 2-chloromandelic acid (292 mg) in the same manner as in Example 377.
$^1$H NMR(400MHz,DMSO-d6):δ(ppm)=9.04(1H, s), 8.03(1H, s), 7.95(1H, d, J=2.0Hz), 7.45(1H, dd, J=7.7 and 1.8Hz), 7.42(1H, dd, J=7.6 and 1.4Hz), 7.36(1H, ddd, J=7.8, 7.7 and 1.4Hz), 7.33(1H, ddd, J=7.8, 7.6 and 1.8Hz), 7.28(2H, d, J=9.0Hz), 6.86(2H, d, J=9.0Hz), 6.86(1H, d, J=8.2Hz), 6.70(1H, dd, J=8.2 and 2.0Hz), 5.91(1H, d, J=7.0Hz), 5.66(1H, d, J=7.0Hz), 3.81(3H, s), 3.75-3.44(4H, m), 3.13-3.02(2H, m), 2.98-2.88(1H, m), 2.79-2.67(1H, m), 2.21(3H, s).
MS(ES$^+$) m/z:509 (M + H)$^+$.
Melting point: 118-120˚C.

(Example 379) 1-(6- {4-[2-(2-chloro-4-hydroxy-phenyl)-2-hydroxy-acetyl]-piperazin-1-yl}-pyridin-3-yl)-3-(2-fluoro-phenyl)-urea (Compound No. 1-810)

(379a) (2-chloro-4-hydroxy-phenyl)-hydroxyacetic acid (Mol. Cryst. Liq. Cryst. Sci. Tecnol. Sect. A 1993, 237, 399 - 406.)

A mixture of 3-chlorophenol (6.40 g) and oxoacetic acid (4.60 g) in 2N aqueous sodium hydroxide solution (50 mL) was stirred at room temperature for 17 hours. 6N hydrochloric acid (17 mL) was added to the reaction mixture, followed by extraction with ethyl acetate. The organic layer was washed with saturated brine and dried over sodium sulfate and concentrated. The residue was vigorously stirred in hexane/diisopropyl ether (1:1) and was collected by filtration and dried under reduced pressure, and 1.20 g (12%) of the title compound was obtained as a white solid.
$^1$H NMR(400MHz,DMSO-d6):δ(ppm)=9.89(1H, s), 7.26(1H, d, J=8.4Hz), 6.78(1H, d, J=2.3Hz), 6.73(1H, dd, J=8.4 and 2.3Hz), 5.82(1H, brs), 5.20(1H, s).

(379b) 5-(2-chloro-4-hydroxy-phenyl)-2,2-dimethyl-1,3-dioxolan-4-one

A mixture of (2-chloro-4-hydroxy-phenyl)-hydroxyacetic acid (1.16 g) obtained in Example (379a) in 2,2-dimethoxypropane (30 mL) and toluene (30 mL) was stirred at 100˚C for six hours and concentrated. The residue was vigorously stirred in hexane/diisopropyl ether (1:1) and was collected by filtration and dried under reduced pressure, and 887 mg (64%) of the title compound was obtained as a white solid.
$^1$H NMR(400MHz,DMSO-d6):δ(ppm)=10.2(1H, brs), 7.28(1H, d, J=8.4Hz), 6.86(1H, d, J=2.3Hz), 6.78(1H, dd, J=8.4 and 2.3Hz), 5.77(1H, s), 1.66(3H, s), 1.63(3H, s).

(379c) 5-(2-chloro-4-methoxy-phenyl)-2,2-dimethyl-1,3-dioxolan-4-one

Diisopropylethylamine (0.40 mL) and chloromethyl-methyl ether (0.14 mL) were added to a solution of 5-(2-chloro-4-hydroxy-phenyl)-2,2-dimethyl-1,3-dioxolan-4-one (364 mg) obtained in Example (379b) in dichloromethane (5 mL) at room temperature. The reaction mixture was stirred at room temperature for 17 hours and concentrated and diluted with an ammonium chloride aqueous solution, followed by extraction with ethyl acetate. The organic layer was washed with saturated brine and dried over sodium sulfate and concentrated. The residue was purified by column chromatography (hexane:ethyl acetate 5:2 → 2:1) and 349 mg (81%) of the title compound was obtained as a colourless transparent syrup.
$^1$H NMR(400MHz,$CDCl_3$):δ(ppm)=7.31(1H, d, J=8.6Hz), 7.16(1H, d, J=2.8Hz), 6.99(1H, dd, J=8.6 and 2.8Hz), 5.70(1H, s), 5.18(2H, s), 3.47(3H, s), 1.75(3H, s), 1.68(3H, s).

(379d) (2-chloro-4-methoxy-phenyl)-hydroxyacetic acid

2N sodium hydroxide (1.8 mL) was added to a solution of 5-(2-chloro-4-methoxy-phenyl)-2,2-dimethyl-1,3-dioxolan-4-one (349 mg) obtained in Example (379c) in methanol (5 mL) at room temperature. The reaction mixture was stirred for 1.5 hours and concentrated and was neutralized with 1N hydrochloric acid (3.6 mL), followed by extraction with ethyl acetate. The organic layer was washed with saturated brine and dried over sodium sulfate and concentrated. The residue was vigorously stirred in hexane/diisopropyl ether (1:1) and was collected by filtration and dried under reduced pressure, and 193 mg (64%) of the title compound was obtained as a white solid.
$^1$H NMR(400MHz,DMSO-d6):δ(ppm)=7.39(1H, d, J=8.6Hz), 7.08(1H, d, J=2.7Hz), 7.00(1H, dd, J=8.6 and 2.7Hz), 5.25 (1H, s), 5.20(2H, s), 3.36(3H, s).

(379e) 1-(6-{4-[2-(2-chloro-4-hydroxy-phenyl)-2-hydroxy-acetyl]-piperazin-1-yl}-pyridin-3-yl)-3-(2-fluoro-phenyl)-urea

99 mg (61 %) of amide compound was obtained as a white solid from (2-chloro-4-methoxy-phenyl)-hydroxyacetic acid (74 mg) obtained in Example (379d) and 1-(2-fluoro-phenyl)-3-(6-piperazin-1-yl-pyridin-3-yl)-urea (97 mg) obtained in Example (222b) in the same manner as in Example 377. 4N hydrochloric acid was added to a solution of this amide compound in anhydrous tetrahydrofuran (2 mL) at room temperature. The reaction mixture was stirred for 24 hours and concentrated and diluted with a saturated sodium hydrogen carbonate aqueous solution, followed by extraction with ethyl acetate. The organic layer was washed with saturated brine and dried over sodium sulfate and concentrated. The residue was vigorously stirred in dichloromethane/hexane (1:1) and collected by filtration. The obtained solid was purified by preparative TLC (dichloromethane:methanol 10:1) and 21 mg (23%) of the title compound was obtained as a white solid.
$^1$H NMR(500MHz,DMSO-d6):δ(ppm)=9.93(1H, s), 8.82(1H, s), 8.50(1H, s), 8.14(1H, d, J=2.7Hz), 8.11(1H, ddd, J=8.4, 8.1 and 1.4Hz), 7.71(1H, dd, J=9.0 and 2.7Hz), 7.22(1H, dd, J=11.4 and 8.0Hz), 7.18(1H, d, J=8.8Hz), 7.12(1H, dd, J=8.1 and 8.0Hz), 7.02-6.96(1H, m), 6.83(1H, d, J=9.0Hz), 6.82(1H, d, J=2.2Hz), 6.74(1H, dd, J=8.8 and 2.2Hz), 5.54(1H, d, J=6.8Hz), 5.52(1H, d, J=6.8Hz), 3.75-3.67(1H, m), 3.58-3.39(5H, m), 3.29-3.23(1H, m), 2.98-2.90(1H, m).
MS(ES$^+$) m/z:500 (M + H)$^+$.
Melting point: 133-135˚C.

(Example 380) 1-(6-{4-[2-(2-chloro-phenyl)-2-hydroxy-acetyl]-piperazin-1-yl}-pyridin-3-yl)-3-(2-methoxy-5-methyl-phenyl)-urea (Compound No. 1-806)

66 mg (45%) of the title compound was obtained as a white solid from 1-(2-methoxy-5-methyl-phenyl)-3-(6-piperazin-1-yl-pyridin-3-yl)-urea (102 mg) obtained in Example (137a) and 2-chloromandelic acid (56 mg) in the same manner as in Example 377.
$^1$H NMR(500MHz,DMSO-d6):δ(ppm)=9.08(1H, s), 8.14(1H, d, J=2.8Hz), 8.10(1H, s), 7.94(1H, d, J=1.7Hz), 7.72(1H, dd, J=9.0 and 2.7Hz), 7.47(1H, dd, J=7.6 and 1.9Hz), 7.44(1H, dd, J=7.8 and 1.6Hz), 7.37(1H, ddd, J=7.6, 7.5 and 1.6Hz), 7.33(1H, ddd, J=7.8, 7.5 and 1.9Hz), 6.88(1H, d, J=8.3Hz), 6.83(1H, d, J=9.0Hz), 6.72(1H, dd, J=8.3 and 1.7Hz), 5.92(1H, d, J=6.8Hz), 5.68(1H, d, J=6.8Hz), 3.83(3H, s), 3.71-3.54(3H, m), 3.52-3.41(3H, m), 3.37-3.26(1H, m), 3.15-3.07 (1H, m), 2.22(3H, s).
MS(ES$^+$) m/z:510 (M + H)$^+$.
Melting point: 118-120˚C.

(Example 381) 4-{4-[3-(2-methoxy-5-methyl-phenyl)-ureido]-phenyl}-piperazine-1-carboxylic acid (4-hydroxymethyl-2-methyl-phenyl)-amide (Compound No. 1-759)

(381a) tert-butyl-dimethyl-(3-methyl-4-nitro-benzyloxy)-silane

Imidazole (0.881 g) and tert-butyldimethylsilyl chloride (1.17 g) were added to a solution of 3-methyl-4-nitrobenzyl alcohol (1.08 g) in dimethylformamide (30 mL) at room temperature. The reaction mixture was stirred for one hour and filtered and concentrated. The residue was purified by column chromatography (dichloromethane) and 1.82 g (quantitative yield) of the title compound was obtained as a pale yellow oil.
$^1$H NMR(400MHz,$CDCl_3$):δ(ppm)=7.84(1H, d, J=9.0Hz), 7.15(1H, d, J=7.1Hz), 7.14(1H, s), 4.64(2H, s), 2.49(3H, s), 0.84(9H, s), 0.00(6H, s).

(381b) 4-(tert-butyl-dimethyl-silanyloxymethyl)-2-methyl-phenylamine

A suspension of tert-butyl-dimethyl-(3-methyl-4-nitro-benzyloxy)-silane (1.82 g) obtained in Example (381a) and palladium-carbon (10%, 0.18 g) in anhydrous tetrahydrofuran (30 mL) was stirred under a hydrogen atmosphere at room temperature for 17 hours. The reaction mixture was filtered and concentrated. The residue was purified by column chromatography (hexane:ethyl acetate 10:1 → 4:1) and 1.21 g (74%) of the title compound was obtained as a colourless transparent oil.
$^1$H NMR(400MHz,$CDCl_3$):δ(ppm)=6.93(1H, s), 6.92(1H, d, J=9.3Hz), 6.57(1H, d, J=7.9Hz), 4.53(2H, s), 3.47(2H, brs), 2.09(3H, s), 0.85(9H, s), 0.01(6H, s).

(381c) 4-{4-[3-(2-methoxy-5-methyl-phenyl)-ureido]-phenyl}-piperazine-1-carboxylic acid (4-hydroxymethyl-2-methyl-phenyl)-amide

773 mg (77%) of silyl ether was obtained as a white solid from 1-(2-methoxy-5-methyl-phenyl)-3-(4-piperazin-1-yl-phenyl)-urea (550 mg) obtained in Example (47a) and 4-(tert-butyl-dimethyl-silanyloxymethyl)-2-methyl-phenylamine (407 mg) obtained in Example (381b) in the same manner as in Example 170. This silyl ether was deprotected with tetrabutylammonium fluoride in the same manner as in Example (209d) and 601 mg (95%) of the title compound was obtained as a white solid.

$^1$H NMR(400MHz,DMSO-d6):δ(ppm)=9.09(1H, s), 8.12(1H, s), 8.07(1H, s), 7.99(1H, s), 7.33(2H, d, J=9.0Hz), 7.13(1H, d, J=7.8Hz), 7.12(1H, s), 7.07(1H, d, J=8.6Hz), 6.95(2H, d, J=9.0Hz), 6.88(1H, d, J=8.2Hz), 6.73(1H, d, J=8.6Hz), 5.10 (1H, t, J=5.7Hz), 4.43(2H, d, J=5.4Hz), 3.84(3H, s), 3.58(4H, brs), 3.07(4H, t, J=4.5Hz), 2.23(3H, s), 2.16(3H, s).

MS(ES$^+$) m/z:504 (M + H)$^+$.

Melting point: 138-141˚C.

(Example 382) 4-{4-[3-(2-methoxy-5-methyl-phenyl)-ureido]-phenyl}-piperazine-1-carboxylic acid (2-chloro-4-hydroxymethyl-phenyl)-amide (Compound No. 1-763)

(382a) (4-amino-3-chloro-phenyl)-methanol

To a suspension of lithium aluminum hydride (0.43 g) in toluene (10 mL), a solution of 4-amino-3-chloro-benzoic acid methyl ester (1.06 g) in toluene/anhydrous tetrahydrofuran (1:1, 20 mL) was added dropwise slowly at room temperature over a period of 30 minutes. After one hour, 1N aqueous sodium hydroxide solution (3 mL) was added dropwise slowly to the reaction mixture. The mixture was filtered and concentrated. The residue was purified by column chromatography (dichloromethane: ethyl acetate 2:1) to obtain 909 mg (quantitative yield) as a pale yellow amorphous material.

MS(ES$^+$) m/z:158 (M + H)$^+$.

(382b) 4-(tert-butyl-dimethyl-silanyloxymethyl)-2-chloro-phenylamine

893 mg (quantitative yield) of the title compound was obtained as a yellow oil from (4-amino-3-chloro-phenyl)-methanol (493 mg) obtained in Example (382a) and tert-butyldimethylsilyl chloride (567 mg) in the same manner as in Example (381a). $^1$H NMR(400MHz,$CDCl_3$):δ(ppm)=7.11(1H, d, J=1.9Hz), 6.91(1H, dd, J=8.2 and 2.0Hz), 6.63(1H, d, J=7.9Hz), 4.50(2H, s), 3.89(2H, brs), 0.84(9H, s), 0.00(6H, s). (382c) 4-{4-[3-(2-methoxy-5-methyl-phenyl)-ureido]-phenyl}-piperazine-1-carboxylic acid (2-chloro-4-hydroxymethyl-phenyl)-amide

1.60 g (80%) of silyl ether was obtained as a white solid from 1-(2-methoxy-5-methyl-phenyl)-3-(4-piperazin-1-yl-phenyl)-urea (1.07 g) obtained in Example (47a) and 4-(tert-butyl-dimethyl-silanyloxymethyl)-2-chloro-phenylamine (851 mg) obtained in Example (382b) in the same manner as in Example 170. This silyl ether was deprotected with tetrabutylammonium fluoride in the same manner as in Example (209d) and 1.19 g (91 %) of the title compound was obtained as a white solid.

$^1$H NMR(400MHz,DMSO-d6):δ(ppm)=9.09(1H, s), 8.28(1H, s), 8.06(1H, s), 7.99(1H, s), 7.43(IH, d, J=8.3Hz), 7.39(IH, s), 7.33(2H, d, J=7.8Hz), 7.22(IH, d, J=8.6Hz), 6.94(2H, d, J=8.2Hz), 6.88(1H, d, J=7.8Hz), 6.73(1H, d, J=7.9Hz), 5.28 (1H, t, J=5.7Hz), 4.47(2H, d, J=5.9Hz), 3.83(3H, s), 3.60(4H, brs), 3.08(4H, brs), 2.22(3H, s).

MS(ES$^+$) m/z:525 (M + H)$^+$.

Melting point: 152-155˚C.

(Example 383) 4-{5-[3-(2-ethoxy-5-fluoro-phenyl)-ureido]-pyridin-2-yl}-piperazine-1-carboxylic acid (2,6-dimethyl-phenyl)-amide (Compound No. 1-65)

64 mg (63%) of the title compound was obtained as a pale pink powder from 1-(2-ethoxy-5-fluoro-phenyl)-3-(6-piperazin-1-yl-pyridin-3-yl)-urea (72 mg) obtained in Example (300a) and 2,6-dimethylphenyl isocyanate (33 μl) in the same manner as in Example 49.

$^1$H NMR(400MHz,DMSO-d6):δ(ppm)=9.32(1H, s), 8.23(1H, s), 8.19(1H, d, J=2.0Hz), 8.02(1H, dd, J=11.4 and 3.1Hz), 7.98(1H, s), 7.78(1H, dd, J=9.0 and 2.0Hz), 7.08-7.03(3H, m), 7.00(1H, dd, J=9.0 and 5.0Hz), 6.91(1H, d, J=9.0Hz), 6.72(1H, ddd, J=9.0, 8.6 and 3.1Hz), 4.12(2H, q, J=6.8Hz), 3.61-3.55(4H, m), 3.49-3.43(4H, m), 2.16(6H, s), 1.41(3H, t, J=6.8Hz).

MS(ES$^+$) m/z:507 (M + H)$^+$.

Melting point: 214-215˚C.

(Example 384) 4-{5-[3-(2-fluoro-phenyl)-ureido]-pyridin-2-yl}-piperazine-1-carboxylic acid (2-chloro-6-methyl-phenyl)-amide (Compound No. 1-920)

112 mg (77%) of the title compound was obtained as a white powder from 1-(2-fluoro-phenyl)-3-(6-piperazin-1-yl-pyridin-3-yl)-urea (95 mg) obtained in Example (222b) and 2-chloro-6-methylphenyl isocyanate (49 μl) in the same manner as in Example 49.

$^1H$ NMR(400MHz,DMSO-d6):δ(ppm)=8.94(1H, s), 8.57(1H, s), 8.24(1H, s), 8.20(1H, d, J=2.8Hz), 8.07(1H, ddd, J=8.2, 8.2 and 1.5Hz), 7.78(1H, dd, J=9.4 and 2.8Hz), 7.31(1H, dd, J=7.8 and 1.2Hz), 7.25-7.09(4H, m), 7.06-6.96(2H, m), 3.62-3.56(4H, m), 3.54-3.48(4H, m), 2.20(3H, s).
MS($ES^+$) m/z:483 $(M + H)^+$.
Melting point: 213-214˚C.

(Example 385) 4-{5-[3-(2-fluoro-phenyl)-ureido]-pyridin-2-yl}-piperazine-1-carboxylic acid (2-fluoro-6-trifluoromethyl-phenyl)-amide (Compound No. 1-795)

116 mg (74%) of the title compound was obtained as a white powder from 1-(2-fluoro-phenyl)-3-(6-piperazin-1-yl-pyridin-3-yl)-urea (95 mg) obtained in Example (222b) and 2-fluoro-6-trifluoromethylphenyl isocyanate (51 μl) in the same manner as in Example 49.
$^1H$ NMR(400MHz,DMSO-d6):δ(ppm)=8.97(1H, d, J=15.6Hz), 8.59(1H, d, J=7.5Hz), 8.36(1H, s), 8.22(1H, d, J=2.7Hz), 8.07(1H, ddd, J=8.4, 8.4 and 1.6Hz), 7.79(1H, dd, J=9.0 and 2.7Hz), 7.66-7.48(3H, m), 7.27-7.19(1H, m), 7.16-6.97(3H, m), 3.64-3.55(4H, m), 3.55-3.47(4H, m).
MS($ES^+$) m/z:521 $(M + H)^+$.
Melting point: 147-148˚C.

(Example 386) 4-{5-[3-(2-fluoro-phenyl)-ureido]-pyridin-2-yl}-piperazine-1-carboxylic acid (2,6-dichloro-phenyl)-amide (Compound No. 1-796)

117 mg (78%) of the title compound was obtained as a white powder from 1-(2-fluoro-phenyl)-3-(6-piperazin-1-yl-pyridin-3-yl)-urea (95 mg) obtained in Example (222b) and 2,6-dichlorophenyl isocyanate (68 mg) in the same manner as in Example 49.
$^1H$ NMR(400MHz,DMSO-d6):δ(ppm)=8.93(1H, s), 8.57(1H, s), 8.53(1H, s), 8.21(1H, s), 8.08(1H, dd, J=8.0 and 8.0Hz), 7.78(1H, d, J=9.0Hz), 7.50(2H, d, J=8.0Hz), 7.28(1H, t, J=8.0Hz), 7.24-7.18(1H, m), 7.12(1H, dd, J=8.2 and 8.0Hz), 7.07-6.96(2H, m), 3.63-3.57(4H, m), 3.54-3.48(4H, m).
MS($ES^+$) m/z:503 $(M + H)^+$.
Melting point: 151-152˚C.

(Example 387) 4-{5-[3-(2-fluoro-phenyl)-ureido]-pyridin-2-yl}-piperazine-1-carboxylic acid (2,6-difluoro-phenyl)-amide (Compound No. 1-799)

84 mg (89%) of the title compound was obtained as a white powder from 1-(2-fluoro-phenyl)-3-(6-piperazin-1-yl-pyridin-3-yl)-urea (63 mg) obtained in Example (222b) and 2,6-difluoro-phenyl isocyanate (31 μl) in the same manner as in Example 49.
$^1H$ NMR(400MHz,DMSO-d6):δ(ppm)=8.93(1H, brs), 8.57(1H, brs), 8.37(1H, s), 8.21(1H, s), 8.08(1H, dd, J=8.0 and 8.0Hz), 7.79(1H, d, J=10.1Hz), 7.34-7.18(2H, m), 7.16-6.96(5H, m), 3.62-3.56(4H, m), 3.55-3.48(4H, m).
MS($ES^+$) m/z:471 $(M + H)^+$.
Melting point: 137-138˚C.

(Example 388) 3-chloro-4-[(4-{4-[3-(2-fluoro-phenyl)-ureido]-phenyl}-piperazine-1-carbonyl)-amino]-benzoic acid (Compound No. 1-791)

(388a) 3-chloro-4-[(4-{4-[3-(2-fluoro-phenyl)-ureido]-phenyl}-piperazine-1-carbonyl)-amino]-benzoic acid methyl ester

136 mg (26%) of the title compound was obtained as a white powder from 1-(2-fluoro-phenyl)-3-(4-piperazin-1-yl-phenyl)-urea (314 mg) obtained in Example (125a) and 4-amino-3-chlorobenzoic acid methyl ester (185 mg) in the same manner as in Example 170.
$^1H$ NMR(400MHz,DMSO-d6):8(ppm)=8.85(1H, s), 8.46(1H, s), 8.45(1H, d, J=6.7Hz), 8.16(1H, dd, J=8.6 and 8.6Hz), 7.96(1H, s), 7.89-7.82(2H, m), 7.34(2H, d, J=9.0Hz), 7.23(1H, dd, J=10.3 and 10.3Hz), 7.13(1H, dd, J=7.5 and 7.5Hz), 7.01-6.95(3H, m), 3.85(3H, s), 3.63(4H, t, J=4.9Hz), 3.11(4H, t, J=3.1Hz).

(388b) 3-chloro-4-[(4-{4-[3-(2-fluoro-phenyl)-ureido]-phenyl}-piperazine-1-carbonyl)-amino]-benzoic acid

1N aqueous sodium hydroxide solution (2 mL) was added to a solution of 3-chloro-4-[(4-{4-[3-(2-fluoro-phenyl)-ureido]-phenyl}-piperazine-1-carbonyl)-amino]-benzoic acid methyl ester (135 mg) obtained in Example (388a) in anhydrous tetrahydrofuran/methanol (1:1, 10 mL). The reaction mixture was stirred at room temperature for 19.5 hours,

concentrated, and neutralized with 1N hydrochloric acid aqueous solution (2 mL) and was stirred in water/isopropyl ether (1:1). The deposited precipitate was collected by filtration and dried under reduced pressure, and 118 mg (90%) of the title compound was obtained as a white solid.
$^1$H NMR(400MHz,DMSO-d6):δ(ppm)=13.1(1H, s), 8.84(1H, s), 8.43(1H, d, J=2.3Hz), 8.41(1H, s), 8.15-8.11(1H, ddd, J=8.2, 8.2 and 1.6Hz), 7.91(1H, d, J=1.9Hz), 7.82(1H, dd, J=8.6 and 2.0Hz), 7.76(1H, d, J=8.6Hz), 7.31(2H, d, J=9.0Hz), 7.23-7.17(1H, ddd, J=11.6, 8.2 and 1.4Hz), 7.11(1H, t, J=7.8Hz), 6.99-6.96(1H, m), 6.94(2H, d, J=9.0Hz), 3.62(4H, t, J=4.9Hz), 3.10(4H, t, J=5.1Hz).
MS(ES$^+$) m/z:512 (M + H)$^+$.
Melting point: 250-253°C.

(Example 389) 4-{4-[3-(2-methoxy-5-methyl-phenyl)-ureido]-phenyl}-piperazine-1-carboxylic acid [2-chloro-5-(2,3-dihydroxy-propoxy)-phenyl]-amide (Compound No. 1-675)

(389a) 3-(4-chloro-3-nitro-phenoxy)-propane-1,2-diol

4.07 g (82%) of the title compound was obtained as a yellow oil from 4-chloro-3-nitrophenol (3.47 g) and 3-bromo-1,2-propanediol (4.0 mL) in the same manner as in Example (275a).
$^1$H NMR(400MHz,$CDCl_3$):δ(ppm)=7.43(1H, d, J=9.0Hz), 7.42(1H, d, J=2.0Hz), 7.08(1H, dd, J=8.8 and 2.9Hz), 4.16-4.07 (3H, m), 3.88-3.83(1H, m), 3.78-3.73(1H, m), 2.49(1H, d, J=4.7Hz), 1.89(1H, t, J=5.7Hz).

(389b) 4-(4-chloro-3-nitro-phenoxymethyl)-2,2-dimethyl-1,3-dioxolane

4.61 g (98%) of the title compound was obtained as a yellow oil from 3-(4-chloro-3-nitro-phenoxy)-propane-1,2-diol (4.07 g) obtained in Example (389a) in the same manner as in Example (275b).
$^1$H NMR(400MHz,$CDCl_3$):δ(ppm)=7.42(1H, d, J=9.0Hz), 7.42(1H, d, J=2.7Hz), 7.08(1H, dd, J=8.8 and 2.9Hz), 4.51-4.45 (1H, m), 4.17(1H, dd, J=8.4 and 5.4Hz), 4.06(1H, dd, J=9.8 and 5.5Hz), 4.00(1H, dd, J=9.6 and 5.3Hz), 3.89(1H, dd, J=8.6 and 5.5Hz), 1.46(3H, s), 1.40(3H, s).

(389c) 2-chloro-5-(2,2-dimethyl-1,3-dioxolan-4-ylmethoxy)-phenylamine

3.69 g (89%) of the title compound was obtained as a dark brown oil from 4-(4-chloro-3-nitro-phenoxymethyl)-2,2-dimethyl-1,3-dioxolane (4.61 g) obtained in Example (389b) in the same manner as in Example (275c).
$^1$H NMR(400MHz,$CDCl_3$):δ(ppm)=7.13(1H, d, J=8.6Hz), 6.38(1H, d, J=2.7Hz), 6.30(1H, dd, J=8.6 and 2.4Hz), 4.48-4.42 (1H, m), 4.17-4.10(3H, m), 4.00(1H, dd, J=9.3 and 5.5Hz), 3.88(2H, dd, J=9.0 and 5.8Hz), 1.46(3H, s), 1.40(3H, s).

(389d) 4-{4-[3-(2-methoxy-5-methyl-phenyl)-ureido]-phenyl}-piperazine-1-carboxylic acid [2-chloro-5-(2,3-dihydroxy-propoxy)-phenyl]-amide

1.79 g of urea compound was obtained as a brown solid from 1-(2-methoxy-5-methyl-phenyl)-3-(4-piperazin-1-yl-phenyl)-urea (1.40 g) obtained in Example (47a) and 2-chloro-5-(2,2-dimethyl-1,3-dioxolan-4-ylmethoxy)-phenylamine (1.06 g) obtained in Example (389c) in the same manner as in Example 170. This urea compound was deprotected in the same manner as in Example (275d) and 433 mg (two steps, 18%) of the title compound was obtained as a reddish brown solid.
$^1$H NMR(400MHz,DMSO-d6):δ(ppm)=9.08(1H, s), 8.19(1H, s), 8.06(1H, s), 7.99(1H, s), 7.34(2H, d, J=8.6Hz), 7.34(1H, d, J=8.6Hz), 7.22(1H, d, J=2.4Hz), 6.95(2H, d, J=8.2Hz), 6.89(1H, d, J=7.8Hz), 6.73(2H, d, J=7.8Hz), 4.96(1H, dd, J=4.7 and 0.8Hz), 4.68(1H, t, J=5.5Hz), 3.98(1H, dd, J=9.2 and 3.4Hz), 3.84(3H, s), 3.81-3.76(2H, m), 3.60(4H, brs), 3.44(2H, t, J=5.5Hz), 3.09(4H, t, J=4.3Hz), 2.23(3H, s).
MS(ES$^+$) m/z:585 (M + H)$^+$.
Melting point: 114-118°C.

(Example 390) {4-[(4-{4-[3-(2-methoxy-5-methyl-phenyl)-ureido]-phenyl}-piperazine-1-carbonyl)-amino]-3-methyl-benzyl}-phosphonic acid diethyl ester (Compound No. 1-769)

(390a) (3-methyl-4-nitrobenzyl)-phosphonic acid diethyl ester

A solution of 3-methyl-4-nitrobenzyl bromide (9.63 g) and triethyl phosphite (20 mL) in acetonitrile (40 mL) was heated under reflux for 24 hours and concentrated. The residue was stirred in hexane/diisopropyl ether (5:1) and the supernatant was removed. The oily residue was dried under reduced pressure, and 7.31 g (61 %) of the title compound

was obtained as a brown oil.
MS($ES^+$) m/z:288 $(M + H)^+$.
(390b) (4-amino-3-methyl-benzyl)-phosphonic acid diethyl ester
Zinc powder (33.3 g) was added in small portions to 1N hydrochloric acid/isopropanol solution (1: 2, 750 mL) of (3-methyl-4-nitrobenzyl)-phosphonic acid diethyl ester (7.31 g) obtained in Example (390a) at room temperature. After two hours, the reaction mixture was neutralized with 1N aqueous sodium hydroxide solution (250 mL) and filtered through Celite, followed by extraction with ethyl acetate. The organic layer was washed with saturated brine and dried over sodium sulfate and concentrated and dried under reduced pressure, and 5.2 g (80%) of the title compound was obtained as a brown oil.
MS($ES^+$) m/z:258 $(M + H)^+$.

(390c) 4-[(4-{4-[3-(2-methoxy-5-methyl-phenyl)-ureido]-phenyl}-piperazine-1- carbonyl)-amino]-3-methyl-benzyl}-phosphonic acid diethyl ester

1.39 g (quantitative yield) of the title compound was obtained as a light brown amorphous material from 1-(2-methoxy-5-methyl-phenyl)-3-(4-piperazin-1-yl-phenyl)-urea (763 mg) obtained in Example (47a) and (4-amino-3-methyl-benzyl)-phosphonic acid diethyl ester (577 mg) obtained in Example (390b) in the same manner as in Example 170.
$^1$H NMR(400MHz,DMSO-d6):δ(ppm)=9.07(1H, s), 8.09(1H, s), 8.04(1H, s), 7.96(1H, d, J=2.0Hz), 7.31(2H, d, J=9.0Hz), 7.11(1H, d, J=8.2Hz), 7.06(1H, s), 7.01(1H, d, J=7.8Hz), 6.92(2H, d, J=9.0Hz), 6.86(1H, d, J=8.2Hz), 6.70(1H, dd, J=7.8 and 2.0Hz), 3.98-3.90(4H, m), 3.82(3H, s), 3.57(4H, t, J=5.1Hz), 3.13(2H, d, J=21.5Hz), 3.06(4H, t, J=5.1Hz), 2.22(3H, s), 2.14(3H, s), 1.18(6H, t, J=7.1Hz).
MS($ES^+$) m/z:624 $(M + H)^+$.

(Example 391) {4-[(4-{4-[3-(2-methoxy-5-methyl-phenyl)-ureido]-phenyl} - piperazine-1-carbonyl)-amino]-3-methyl-benzyl}-phosphonic acid monoethyl ester (Compound No. 1-785)

A mixture of 4-[(4-{4-[3-(2-methoxy-5-methyl-phenyl)-ureido]-phenyl}-piperazine-1-carbonyl)-amino]-3-methyl-benzyl}-phosphonic acid diethyl ester (1.48 g) obtained in Example 390 and lithium bromide (1.03 g) in acetonitrile (15 mL) was heated under reflux for 20 hours. The reaction mixture was diluted with ethyl acetate, followed by extraction with water. The aqueous layer was neutralized with 1N hydrochloric acid (10 mL) and the supernatant was removed. The residue was dissolved in methanol. Water was added till white precipitate deposited. The deposited precipitate was collected by filtration and dried under reduced pressure, and 342 mg (24%) of the title compound was obtained as a pale grey solid.
$^1$H NMR(400MHz,DMSO-d6):δ(ppm)=9.06(1H, s), 8.08(1H, s), 8.04(1H, s), 7.96(1H, s), 7.31(2H, d, J=9.0Hz), 7.09-6.99 (3H, m), 6.94(2H, d, J=2.0Hz), 6.86(1H, d, J=8.2Hz), 6.70(1H, d, J=7.0Hz), 3.89-3.82(2H, m), 3.82(3H, s), 3.57(4H, brs), 3.39(1H, brs), 3.07(4H, brs), 2.99(2H, d, J=21.9Hz), 2.22(3H, s), 2.14(3H, s), 1.17(3H, t, J=6.8Hz).
MS($ES^+$) m/z:596 $(M + H)^+$.
Melting point: 165-173˚C.

(Example 392) 4- {4-[3-(2-methoxy-5-methyl-phenyl)-ureido]-phenyl}-piperazine-1-carboxylic acid [4-(2-hydroxy-ethyl)-2-methyl-phenyl]-amide (Compound No. 1-773)

(392a) (3-methyl-4-nitro-phenyl)-acetonitrile

To a mixture of ethyl cyanoacetate (2.8 mL) and potassium hydroxide (1.70 g) in dimethylsulfoxide (40 mL), a dimethylsulfoxide solution (10 mL) of 5-chloro-2-nitrotoluene (3.43 g) was added dropwise slowly (during dropwise addition, care should be taken that the temperature in the flask does not exceed 40˚C). Additional ethyl cyanoacetate (1.5 mL, in this case) was added until 5-chloro-2-nitrotoluene had disappeared, and the reaction mixture was stirred at room temperature for 19 hours. Concentrated hydrochloric acid (4 mL), acetic acid (5 mL) and water (4 mL) were added and the reaction mixture was heated under reflux for four hours and diluted with ethyl acetate and washed with a saturated sodium hydrogen carbonate aqueous solution and saturated brine and dried over sodium sulfate and concentrated. The residue was purified by column chromatography (hexane:ethyl acetate 10:1 → 3:1) and 1.26 g (36%) was obtained as a yellow oil.
$^1$H NMR(400MHz,$CDCl_3$):δ(ppm)=8.02(1H, d, J=8.6Hz), 7.36-7.33(2H, m), 3.83(2H, s), 2.64(3H, s).

(392b) (3-methyl-4-nitro-phenyl)-acetic acid methyl ester

Concentrated sulfuric acid (0.72 mL) was added to a solution of (3-methyl-4-nitro-phenyl)-acetonitrile (1.26 g)

obtained in Example (392a) in methanol (30 mL) at room temperature. The reaction mixture was heated under reflux for four days and concentrated and neutralized with a saturated sodium hydrogen carbonate aqueous solution, followed by extraction with ethyl acetate. The organic layer was washed with saturated brine and dried over sodium sulfate and concentrated. The residue was purified by column chromatography (hexane:ethyl acetate 10:1 → 4:1) and 964 mg (65%) of the title compound was obtained as a yellow oil.
$^1$H NMR(400MHz,$CDCl_3$):δ(ppm)=7.94(1H, d, J=8.6Hz), 7.25-7.23(2H, m), 3.71(3H, s), 3.67(2H, s), 2.60(3H, s).

(392c) 4-[2-(tert-butyl-dimethyl-silanyloxy)-ethyl]-2-methyl-phenylamine

To a suspension of lithium aluminum hydride (555 mg) in toluene (10 mL), a solution of (3-methyl-4-nitro-phenyl)-acetic acid methyl ester (601 mg) obtained in Example (392b) in toluene (10 mL) was added dropwise slowly at room temperature. After stirring at room temperature for 24 hours, the reaction mixture was heated under reflux for 24 hours. 1N aqueous sodium hydroxide solution (2 mL) was added dropwise slowly, and the mixture was filtered and dried over sodium sulfate and concentrated, and 172 mg of colourless transparent oil was obtained. This oil was subjected to silylation with imidazole (0.20 g) and tert-butyldimethylsilyl chloride (0.30 g) in dimethylformamide (2 mL) to obtain colourless transparent oil (128 mg). This colourless transparent oil was reduced with zinc powder (583 mg) in isopropanol (8 mL) and 1N hydrochloric acid (4 mL) to obtain 63 mg of a colourless transparent oil. Imidazole (113 mg) and tert-butyldimethylsilyl chloride (135 mg) were added to a solution of dimethylformamide (2 mL) of this oil and stirred at room temperature for 11 days and diluted with ethyl acetate and washed with water (twice) and saturated brine and dried over sodium sulfate and concentrated. The residue was purified by column chromatography (dichloromethane:ethyl acetate 10:1) and 39 mg (5%) of the title compound was obtained as a colourless transparent oil.
MS(FAB) m/z:266 $(M + H)^+$.

(392d) 4-{4-[3-(2-methoxy-5-methyl-phenyl)-ureido]-phenyl}-piperazine-1-carboxylic acid [4-(2-hydroxy-ethyl)-2-methyl-phenyl]-amide

58 mg (76%) of silyl ether was obtained as a white solid from 1-(2-methoxy-5-methyl-phenyl)-3-(4-piperazin-1-yl-phenyl)-urea (41 mg) obtained in Example (47a) and 4-[2-(tert-butyl-dimethyl-silanyloxy)-ethyl]-2-methyl-phenylamine (32 mg) obtained in Example (392c) in the same manner as in Example 170. This silyl ether was deprotected in the same manner as in Example (209d) and 36 mg (76%) of the title compound was obtained as a white solid.
$^1$H NMR(400MHz,DMSO-d6):δ(ppm)=9.08(1H, s), 8.08(1H, s), 8.06(1H, s), 7.98(1H, s), 7.33(2H, d, J=8.6Hz), 7.07(1H, d, J=8.2Hz), 7.02(1H, s), 6.97(1H, d, J=9.8Hz), 6.95(2H, d, J=8.6Hz), 6.89(1H, d, J=8.6Hz), 6.73(1H, d, J=7.8Hz), 4.62 (1H, t, J=5.2Hz), 3.84(3H, s), 3.59(2H, t, J=5.6Hz), 3.58(4H, brs), 3.07(4H, t, J=4.1Hz), 2.66(2H, t, J=7.2Hz), 2.23(3H, s), 2.14(3H, s).
MS($ES^+$) m/z:518 $(M + H)^+$.
Melting point: 119-121˚C.

(Example 393) 4-{4-[3-(2-methoxy-5-methyl-phenyl)-ureido]-phenyl}-piperazine-1-carboxylic acid 2-chloro-4-methoxycarbonylmethyl-phenyl ester (Compound No. 1-751)

(393a) (3-chloro-4-hydroxy-phenyl)-acetic acid methyl ester

Concentrated sulfuric acid (two drops) was added to a solution of 3-chloro-4-hydroxyphenyl acetic acid (1.86 g) in methanol (50 mL) at room temperature. The reaction mixture was stirred for 17 hours and concentrated and diluted with ethyl acetate and washed with a saturated sodium hydrogen carbonate aqueous solution and saturated brine and dried over sodium sulfate and concentrated and 1.72 g (86%) of the title compound was obtained as a deep yellow syrup
$^1$H NMR(400MHz,$CDCl_3$):δ(ppm)=7.24(1H, d, J=2.0Hz), 7.07(1H, dd, J=8.2 and 2.0Hz), 6.95(1H, d, J=8.2Hz), 5.50(1H, s), 3.69(3H, s), 3.53(2H, s). (393b) 4-{4-[3-(2-methoxy-5-methyl-phenyl)-ureido]-phenyl}-piperazine-1-carboxylic acid 2-chloro-4-methoxycarbonylmethyl-phenyl ester

215 mg (38%) of the title compound was obtained as a white solid from 1-(2-methoxy-5-methyl-phenyl)-3-(4-piperazin-1-yl-phenyl)-urea (340 mg) obtained in Example (47a) and (3-chloro-4-hydroxy-phenyl)-acetic acid methyl ester (201 mg) obtained in Example (393a) in the same manner as in Example 170.
$^1$H NMR(400MHz,DMSO-d6):δ(ppm)=9.07(1H, s), 8.04(1H, s), 7.96(1H, d, J=2.0Hz), 7.46(1H, d, J=1.6Hz), 7.32(2H, d, J=9.0Hz), 7.27-7.24(2H, m), 6.93(2H, d, J=9.0Hz), 6.86(1H, d, J=8.2Hz), 6.70(1H, dd, J=8.2 and 2.0Hz), 3.82(3H, s), 3.80-3.75(2H, m), 3.73(2H, s), 3.63(3H, s), 3.61-3.55(2H, m), 3.20-3.07(4H, m), 2.22(3H, s).
MS($ES^+$) m/z:567 $(M + H)^+$.
Melting point: 209-210˚C.

(Example 394) 4-{4-[3-(2-methoxy-5-methyl-phenyl)-ureido]-phenyl}-piperazine-1-carboxylic acid 4-carboxymethyl-2-chloro-phenyl ester (Compound No. 1-753)

1N aqueous sodium hydroxide solution (0.6 mL) was added to a solution of 4-{4-[3-(2-methoxy-5-methyl-phenyl)-ureido]-phenyl}-piperazine-1-carboxylic acid 2-chloro-4-methoxycarbonylmethyl-phenyl ester (113 mg) obtained in Example 393 in anhydrous tetrahydrofuran/ethanol (1:1, 4 mL) at room temperature. After 17 hours, the reaction mixture was neutralized with 1N hydrochloric acid (0.6 mL) and concentrated and diluted with ethyl acetate and washed with water and saturated brine and dried over sodium sulfate and concentrated. The residue was purified by column chromatography (dichloromethane:methanol 8:1) and 31 mg (28%) of the title compound was obtained as a white solid.
$^1$H NMR(400MHz,DMSO-d6):δ(ppm)=9.07(1H, s), 8.04(1H, s), 7.96(1H, d, J=2.0Hz), 7.44(1H, d, J=1.5Hz), 7.32(2H, d, J=9.0Hz), 7.25-7.21(2H, m), 6.93(2H, d, J=9.0Hz), 6.86(1H, d, J=8.2Hz), 6.70(1H, dd, J=8.3 and 2.4Hz), 3.82(3H, s), 3.79-3.74(2H, m), 3.62-3.55(2H, m), 3.59(2H, s), 3.62-3.59(4H, m), 2.22(3H, s).
MS(ES$^+$) m/z:553 (M + H)$^+$.
Melting point: 199-200˚C.

(Example 395) 4-{4-[3-(2-methoxy-S-methyl-phenyl)-ureido]-phenyl}-piperazine-1-carboxylic acid 2-chloro-4-(2-hydroxy-ethyl)-phenyl ester (Compound No. 1-802)

(395a) 2-chloro-4-(2-hydroxy-ethyl)-phenol

Borane/dimethyl sulfide complex (2 mol/L toluene solution, 12 mL) was added dropwise slowly to a solution of 3-chloro-4-hydroxyphenylacetic acid (3.73 g) in anhydrous tetrahydrofuran (40 mL) at 10˚C. The reaction mixture was stirred at room temperature for two hours and the reaction was quenched with methanol. The reaction mixture was concentrated, diluted with ethyl acetate and washed with cold 1N hydrochloric acid, a saturated sodium hydrogen carbonate aqueous solution and saturated brine and dried over sodium sulfate and concentrated. The residue was purified by column chromatography (dichloromethane:ethyl acetate 2:1) to obtain a yellow syrup. This yellow syrup was allowed to stand to yield a solid. This solid was vigorously stirred in hexane and was collected by filtration and dried under reduced pressure, and 1.12 g (32%) of the title compound was obtained as a white solid.
$^1$H NMR(500MHz,$CDCl_3$):δ(ppm)=7.20(1H, s), 7.04(1H, d, J=8.3Hz), 6.95(1H, d, J=8.3Hz), 5.66(1H, s), 3.83(2H, t, J=6.3Hz), 2.78(2H, t, J=6.3Hz), 1.49(1H, s).

(395b) 2-chloro-4-[2-(tetrahydro-pyran-2-yloxy)-ethyl]-phenol

Lithium tetrafluoroborate (19 mg) was added to a solution of 2-chloro-4-(2-hydroxy-ethyl)-phenol (173 mg) obtained in Example (395a) and 3,4-dihydro-2H-pyran (168 mg) in acetonitrile (5 mL) at room temperature. The reaction mixture was stirred for 24 hours and diluted with a saturated sodium hydrogen carbonate aqueous solution, followed by extraction with ethyl acetate. The organic layer was washed with saturated brine and dried over sodium sulfate and concentrated. The residue was purified by column chromatography (hexane:ethyl acetate 7:3) and 180 mg (70%) of the title compound was obtained as a colourless transparent syrup. MS(ES$^+$) m/z:257 (M + H)$^+$.

(395c) 4-{4-[3-(2-methoxy-5-methyl-phenyl)-ureido]-phenyl}-piperazine-1-carboxylic acid 2-chloro-4-(2-hydroxy-ethyl)-phenyl ester

66 mg (15%) of urethane compound was obtained as a white solid from 1-(2-methoxy-5-methyl-phenyl)-3-(4-piperazin-1-yl-phenyl)-urea (239 mg) obtained in Example (47a) and 2-chloro-4-[2-(tetrahydropyran-2-yloxy)-ethyl]-phenol (180 mg) obtained in Example (395b) in the same manner as in Example 170. p-Toluenesulfonic acid (3 mg) was added to a solution of this urethane compound (62 mg) in ethanol/tetrahydrofuran (1:1, 4 mL) at room temperature. The reaction mixture was stirred at room temperature for 24 hours and heated at 50˚C for four days and concentrated. The residue was purified by column chromatography (dichloromethane:ethyl acetate 1:1). The obtained solid was vigorously stirred in diisopropyl ether and was collected by filtration and dried under reduced pressure, and 29 mg (54%) of the title compound was obtained as a white solid.
$^1$H NMR(400MHz,DMSO-d6):δ(ppm)=9.07(1H, s), 8.04(1H, s), 7.96(1H, d, J=2.0Hz), 7.39(1H, d, J=0.8Hz), 7.32(2H, d, J=9.0Hz), 7.22-7.19(2H, m), 6.93(2H, d, J=9.0Hz), 6.86(1H, d, J=8.2Hz), 6.71(1H, dd, J=8.2 and 2.0Hz), 4.68(1H, t, J=5.1Hz), 3.82(3H, s), 3.80-3.72(2H, m), 3.64-3.55(2H, m), 3.61(2H, dt, J=6.5 and 5.1Hz), 3.19-3.07(4H, m), 2.72(2H, t, J=6.5Hz), 2.22(3H, s).
MS(ES$^+$) m/z:539 (M + H)$^+$.
Melting point: 189-190˚C.

(Example 396) 6-[3-(2-methoxy-5-methyl-phenyl)-ureido]-3,4-dihydro-1H-isoquinoline-2-carboxylic acid 2-chloro-4-(2-hydroxy-ethyl)-phenyl ester

(Compound No. 3-79)

A urethane compound was obtained from 1-(2-methoxy-5-methyl-phenyl)-3-(1,2,3,4-tetrahydro-isoquinolin-6-yl)-urea (87 mg) obtained in Example (202b) and 2-chloro-4-[2-(tetrahydropyran-2-yloxy)-ethyl]-phenol (68 mg) obtained in Example (395b) in the same manner as in Example 170. This urethane compound was deprotected with p-toluenesulfonic acid in the same manner as in Example (395c) and 12 mg (10%) of the title compound was obtained as a white solid.
$^1$H NMR(400MHz,DMSO-d6):δ(ppm)=2.30(3H, s), 2.82(2H, t, J=6.0Hz), 3.00-2.88(2H, m), 3.81(3H, s), 3.95-3.75(4H, m), 4.68(1H, s), 4.84(1H, s), 6.82(1H, s), 6.82-6.72(2H, m), 7.19-7.03(4H, m), 7.39-7.26(3H, m), 7.92(1H, s).
MS(APCI) m/z:510, 512 (M +H)$^+$.
Melting point: 95-98˚C.

(Example 397)

6-[3-(2-methoxy-5-methyl-phenyl)-ureido]-3,4-dihydro-1H-isoquinoline-2-carboxylic acid 4-carboxy-2-chloro-phenyl ester (Compound No. 3-149)

80 mg (48%) of methyl ester compound was obtained as a white solid from 1-(2-methoxy-5-methyl-phenyl)-3-(1,2,3,4-tetrahydro-isoquinolin-6-yl)-urea (100 mg) obtained in Example (202b) and 3-chloro-4-hydroxybenzonate methyl ester (123 mg) in the same manner as in Example 170. This methyl ester compound was deprotected with an aqueous sodium hydroxide solution in the same manner as in Example 394 and 16 mg (21 %) of the title compound was obtained as a white solid. $^1$H NMR(400MHz,DMSO-d6):δ(ppm)=9.25(1H, s), 8.13(1H, s), 7.99(1H, s), 7.96(1H, s), 7.90(1H, d, J=7.8Hz), 7.48(1H, d, J=9.4Hz), 7.39(1H, d, J=6.3Hz), 7.24-7.18(1H, m), 7.13(1H, dd, J=7.8 and 7.8Hz), 6.87(1H, d, J=8.2Hz), 6.72(1H, d, J=7.8Hz), 4.77(1H, s), 4.56(1H, s), 3.83(3H, s), 3.69-3.63(2H, m), 2.95-2.90(1H, m), 2.89-2.83(1H, m), 2.22(3H, s).
MS(ES$^+$) m/z:510 (M + H)$^+$.
Melting point: 196-198˚C.

(Example 398) 4-{4-[3-(2-fluoro-phenyl)-ureido]-phenyl}-piperazine-1-carboxylic acid (4-hydroxymethyl-2-methyl-phenyl)-amide (Compound No. 1-797)

272 mg (46%) of silyl ether was obtained as a white solid from 1-(2-fluorophenyl)-3-(4-piperazin-1-yl-phenyl)-urea (314 mg) obtained in Example (125a) and 4-(tert-butyl-dimethyl-silanyloxymethyl)-2-methyl-phenylamine (251 mg) obtained in Example (381b) in the same manner as in Example 170. This silyl ether was deprotected with tetrabutylammonium fluoride in the same manner as in Example (209d) and 184 mg (84%) of the title compound was obtained as a white solid.
$^1$H NMR(400MHz,DMSO-d6):δ(ppm)=8.83(1H, s), 8.43(1H, d, J=2.7Hz), 8.14(1H, dt, J=8.2 and 1.9Hz), 8.10(1H, s), 7.31(2H, d, J=9.0Hz), 7.22(1H, ddd, J=8.1, 8.1 and 1.5Hz), 7.13-7.09(2H, m), 7.10(1H, s), 7.04(1H, dd, J=7.8 and 1.5Hz), 6.99-6.94(1H, m), 6.94(2H, d, J=9.0Hz), 5.08(1H, t, J=5.9Hz), 4.42(2H, d, J=5.9Hz), 3.57(4H, t, J=4.7Hz), 3.07(4H, t, J=5.1Hz), 2.16(3H, s).
MS(ES$^+$) m/z:478 (M + H)$^+$.
Melting point: 229-230˚C.

(Example 399) 4- {4-[3-(2-methoxy-5-methyl-phenyl)-ureido]-phenyl}-piperazine-1-carboxylic acid [4-((S)-2,2-dimethyl-1,3-dioxolan-4-yl)-2-methyl-phenyl]-amide (Compound No. 1-775)

(399a) 3-methyl-4-nitrobenzaldehyde

A suspension of 3-methyl-4-nitrobenzyl alcohol (3.34 g) and manganese dioxide (3.48 g) in dichloromethane (40 mL) was stirred at room temperature for two days and filtered and concentrated. The residue was purified by column chromatography (dichloromethane) and 1.14 g (35%) of the title compound was obtained as a yellow oil.
$^1$H NMR(400MHz,$CDCl_3$):δ(ppm)=10.1(1H, s), 8.07(1H, d, J=8.6Hz), 7.87(1H, d, J=7.8Hz), 7.88(1H, s), 2.67(3H, s).

(399b) 2-methyl-1-nitro-4-vinyl-benzene

Sodium hydride (397 mg) was added to a suspension of methyltriphenylphosphonium iodide (3.34 g) in anhydrous tetrahydrofuran (30 mL) at 0˚C in small portions. The reaction mixture was warmed to room temperature.

A solution of 3-methyl-4-nitrobenzaldehyde (1.14 g) obtained in Example (399a) in anhydrous tetrahydrofuran (20 mL) was added. The reaction mixture was stirred for one and a half hours at room temperature, concentrated, diluted with ethyl acetate and washed with a potassium bisulfate aqueous solution and saturated brine and dried over sodium sulfate and concentrated. The residue was purified by column chromatography (hexane:ethyl acetate 10:1→ 5:1) and 604 mg (54%) of the title compound was obtained as a brown solid.
$^1$H NMR(400MHz,$CDCl_3$):δ(ppm)=7.97(1H, d, J=8.2Hz), 7.35(1H, dd, J=8.2 and 1.9Hz), 7.31(1H, s), 6.70(1H, dd, J=17.6 and 11.0Hz), 5.87(1H, d, J=17.6Hz), 5.45(1H, d, J=11.0Hz), 2.62(3H, s).

(399c) (S)-1-(3-methyl-4-nitro-phenyl)-ethane-1,2-diol

To a suspension of AD-mix α (5.2 g) in tert-butanol/water (1:1, 40 mL), 2-methyl-1-nitro-4-vinyl-benzene (603 mg) obtained in Example (399b) in tert-butanol/water (1:1, 10 mL) was added at 0˚C. The reaction mixture was warmed to room temperature slowly and stirred for 24 hours. Sodium sulfite (5.55 g) was added and the mixture was stirred for two hours, extracted with ethyl acetate, and washed with water and saturated brine and dried over sodium sulfate and concentrated. The residue was purified by column chromatography (hexane:ethyl acetate 3:1 → 1:1 → 0:1) and 478 mg (66%) of the title compound was obtained as a pale yellow solid.
$^1$H NMR(400MHz,$CDCl_3$):δ(ppm)=8.00(1H, d, J=8.6Hz), 7.38(1H, d, J=2.0Hz), 7.35(1H, s), 4.91-4.88(1H, m), 3.86-3.81 (1H, m), 3.67-3.61(1H, m), 2.63(3H, s).

(399d) (S)-2,2-dimethyl-4-(3-methyl-4-nitro-phenyl)-1,3-dioxolane

p-Toluenesulfonic acid (61 mg) was added to a solution of (S)-1-(3-methyl-4-nitro-phenyl)-ethane-1,2-diol (478 mg) obtained in Example (399c) and 2,2-dimethoxypropane (5.9 mL) in anhydrous tetrahydrofuran (10 mL) at room temperature. After 24 hours, the reaction mixture was diluted with ethyl acetate and washed with a saturated sodium hydrogen carbonate aqueous solution and saturated brine and dried over sodium sulfate and concentrated, and 575 mg (quantitative yield) of the title compound was obtained as a yellow oil.
$^1$H NMR(400MHz,$CDCl_3$):δ(ppm)=7.97(1H, d, J=8.6Hz), 7.32-7.30(2H, m), 5.10(1H, t, J=7.1Hz), 4.35(1H, dd, J=8.2 and 6.7Hz), 3.67(1H, t, J=8.0Hz), 2.62(3H, s), 1.56(3H, s), 1.50(3H, s).

(399e) 4-((S)-2,2-dimethyl-1,3-dioxolan-4-yl)-2-methyl-phenylamine

A suspension of (S)-2,2-dimethyl-4-(3-methyl-4-nitro-phenyl)-1,3-dioxolane (575 mg) obtained in Example (399d) in anhydrous tetrahydrofuran (10 mL) and a suspension of 10% palladium-carbon (0.058 g) in anhydrous tetrahydrofuran (10 mL) were stirred under a hydrogen atmosphere at room temperature for three days and filtered and concentrated. The residue was purified by column chromatography (hexane:ethyl acetate 3:1 → 1:1 → 0:1) and 465 mg (93%) of the title compound was obtained as a pale yellow solid.
$^1$H NMR(400MHz,$CDCl_3$):δ(ppm)=7.07(1H, s), 7.05(1H, d, J=7.8Hz), 6.66(1H, d, J=8.2Hz), 4.95(1H, dd, J=8.2 and 5.8Hz), 4.22(1H, dd, J=8.1 and 6.0Hz), 3.69(1H, t, J=8.2Hz), 3.63(2H, brs), 2.17(3H, s), 1.54(3H, s), 1.46(3H, s).

(399f) 4-{4-[3-(2-methoxy-5-methyl-phenyl)-ureido]-phenyl}-piperazine-1-carboxylic acid [4-((S)-2,2-dimethyl-1,3-dioxolan-4-yl)-2-methyl-phenyl]-amide

989 mg (76%) of the title compound was obtained as a white solid from 1-(2-methoxy-5-methyl-phenyl)-3-(4-piperazin-1-yl-phenyl)-urea (769 mg) obtained in Example (47a) and 4-((S)-2,2-dimethyl-1,3-dioxolan-4-yl)-2-methyl-phenylamine (468 mg) obtained in Example (399e) in the same manner as in Example 170.
$^1$H NMR(400MHz,DMSO-d6):δ(ppm)=9.09(1H, s), 8.16(1H, s), 8.07(1H, s), 7.99(1H, s), 7.34(2H, d, J=7.5Hz), 7.19(1H, s), 7.20(1H, d, J=8.6Hz), 7.14(1H, d, J=8.3Hz), 6.95(2H, d, J=7.4Hz), 6.89(1H, d, J=8.3Hz), 6.73(1H, d, J=7.4Hz), 5.00 (1H, t, J=7.4Hz), 4.27(1H, t, J=7.0Hz), 3.84(3H, s), 3.59(4H, brs), 3.59-3.55(1H, m), 3.08(4H, brs), 2.23(3H, s), 2.18(3H, s), 1.46(3H, s), 1.39(3H, s).
MS($ES^+$) m/z:574 $(M + H)^+$.
Melting point: 184-185˚C.

(Example 400) 4-{4-[3-(2-methoxy-5-methyl-phenyl)-ureido]-phenyl}-piperazine-1-carboxylic acid [4-((S)-1,2-dihydroxy-ethyl)-2-methyl-phenyl]-amide (Compound No. 1-781)

p-Toluenesulfonic acid (10 mg) was added to a solution of 4-{4-[3-(2-methoxy-5-methyl-phenyl)-ureido]-phenyl}-piperazine-1-carboxylic acid [4-((S)-2,2-dimethyl-1,3-dioxolan-4-yl)-2-methyl-phenyl]-amide (720 mg) obtained in

Example 399 in methanol (10 mL) at room temperature. The reaction mixture was stirred for eight days and concentrated. The residue was purified by column chromatography (dichloromethane:methanol 50:1 → 10:1). The obtained solid was vigorously stirred in hexane/diisopropyl ether (5:1) and collected by filtration and dried under reduced pressure, and 720 mg (89%) of the title compound was obtained as a pale yellow solid.
$^1$H NMR(400MHz,DMSO-d6):δ(ppm)=9.06(1H, s), 8.08(1H, s), 8.04(1H, s), 7.96(1H, s), 7.31(2H, d, J=8.6Hz), 7.10(2H, d, J=11.3Hz), 7.07(1H, dd, J=7.6 and 7.6Hz), 6.92(2H, d, J=8.6Hz), 6.86(1H, d, J=8.2Hz), 6.70(1H, d, J=8.2Hz), 5.11 (1H, d, J=4.3Hz), 4.66(1H, t, J=5.7Hz), 4.45(1H, dd, J=10.1 and 5.5Hz), 3.81(3H, s), 3.56(4H, brs), 3.39(2H, t, J=5.6Hz), 3.05(4H, brs), 2.20(3H, s), 2.13(3H, s).
MS(ES$^+$) m/z:534 (M + H)$^+$.
Melting point: 112-113˚C.

(Example 401) 4-{4-[3-(2-methoxy-5-methyl-phenyl)-ureido]-phenyl}-piperazine-1-carboxylic acid (2-chloro-4-methanesulphonylamino-phenyl)-amide (Compound No. 1-743)

Triethylamine (33 μl) and methanesulfonyl chloride (19 μl) were added to a solution of 4-{4-[3-(2-methoxy-5-methyl-phenyl)-ureido]-phenyl}-piperazine-1-carboxylic acid (4-amino-2-chloro-phenyl)-amide (102 mg) obtained in Example 168 in dimethylacetamide (5 mL) at room temperature. The reaction mixture was stirred for 18 days and diluted with ethyl acetate and washed with water and saturated brine and dried over sodium sulfate and concentrated. The residue was purified by preparative TLC (dichloromethane:methanol 10:1) and 14 mg (24%) of the title compound was obtained as a yellow solid.
MS(ES$^+$) m/z:588 (M + H)$^+$.
Melting point: 154-156˚C.

(Example 402) 4-{4-[3-(2-methoxy-5-methyl-phenyl)-ureido]-phenyl}-piperazine-1-carboxylic acid (4-dimethylamino-2-trifluoromethyl-phenyl)-amide (Compound No. 1-745)

(402a) $N^4$,$N^4$-dimethyl-2-trifluoromethyl-benzene-1,4-diamine

A suspension of 3-trifluoromethyl-N, N-dimethyl-4-nitroaniline (2.34 g) and 10% palladium-carbon (0.23 g) in anhydrous tetrahydrofuran (40 mL) was stirred under a hydrogen atmosphere at room temperature for 21 hours and filtered and concentrated. The residue was purified by column chromatography (hexane:ethyl acetate 5:1 → 1:1) and 1.97 g (96%) of the title compound was obtained as a brown oil.
$^1$H NMR(400MHz,$CDCl_3$):δ(ppm)=6.86-6.83(2H, m), 6.71(1H, d, J=8.2Hz), 3.76(2H, brs), 2.86(6H, s).

(402b) 4-{4-[3-(2-methoxy-5-methyl-phenyl)-ureido]-phenyl}-piperazine-1-carboxylic acid (4-dimethylamino-2-trifluoromethyl-phenyl)-amide

281 mg (49%) of the title compound was obtained as a white solid from 1-(2-methoxy-5-methyl-phenyl)-3-(4-piperazin-1-yl-phenyl)-urea (340 mg) obtained in Example (47a) and $N^4$,$N^4$-dimethyl-2-trifluoromethyl-benzene-1,4-diamine (204 mg) obtained in Example (402a) in the same manner as in Example 170.
$^1$H NMR(400MHz,DMSO-d6):δ(ppm)=9.08(1H, s), 8.07(2H, s), 7.99(1H, s), 7.33(2H, d, J=7.8Hz), 7.17(1H, d, J=9.0Hz), 6.94(2H, d, J=8.6Hz), 6.94(1H, d, J=8.6Hz), 6.89(1H, d, J=8.6Hz), 6.86(1H, d, J=1.6Hz), 6.73(1H, d, J=7.4Hz), 3.84(3H, s), 3.55(4H, t, J=4.1Hz), 3.05(4H, t, J=4.3Hz), 2.95(6H, s), 2.23(3H, s).
MS(ES$^+$) m/z:571 (M + H)$^+$.
Melting point: 236-239˚C.

(Example 403) 4- {4-[3-(2- fluoro-phenyl)-ureido]-phenyl} -piperazine-1 -carboxylic acid (2-hydroxymethyl-6-methyl-phenyl)-amide (Compound No. 1-793)

886 mg (75%) of silyl ether was obtained as a white solid from 1-(2-fluorophenyl)-3-(4-piperazin-1-yl-phenyl)-urea (629 mg) obtained in Example (125a) and 2-(tert-butyl-dimethyl-silanyloxymethyl)-6-methyl-phenylamine (503 mg) obtained in Example (185a) in the same manner as in Example 170. This silyl ether was deprotected in the same manner as in Example (209d) and 670 mg (quantitative yield) of the title compound was obtained as a white solid.
$^1$H NMR(400MHz,DMSO-d6):δ(ppm)=8.83(1H, s), 8.43(1H, s), 8.14(1H, t, J=7.8Hz), 7.93(1H, s), 7.31(2H, d, J=7.0Hz), 7.27(1H, d, J=7.0Hz), 7.21(1H, dd, J=9.6 and 9.6Hz), 7.11(2H, d, J=9.0Hz), 7.11(1H, d, J=9.0Hz), 6.94(2H, d, J=9.0Hz), 6.94(1H, d, J=9.0Hz), 5.02(1H, t, J=5.4Hz), 4.43(2H, d, J=5.0Hz), 3.57(4H, brs), 3.06(4H, brs), 2.13(3H, s).
MS(ES$^+$) m/z:478 (M + H)$^+$.
Melting point: 184-185˚C.

(Example 404) 4- {4-[3-(2-methoxy-S-methyl-phenyl)-ureido]-phenyl} -piperazine-1 - carboxylic acid (2-chloro-6-hydroxymethyl-phenyl)-amide (Compound No. 1-777)

(404a) (2-amino-3-chloro-phenyl)-methanol

Borane-tetrahydrofuran complex (1.0 mol/L, tetrahydrofuran solution, 17.5 mL) was added to a solution of 2-amino-3-chlorobenzoic acid (1.00 g) in anhydrous tetrahydrofuran (10 mL) at room temperature. The reaction mixture was heated under reflux for 23 hours and cooled to room temperature. 1N aqueous sodium hydroxide solution (17.5 mL) was added and the mixture was stirred at room temperature for seven hours, followed by extraction with ethyl acetate. The organic layer was washed with water, a saturated sodium hydrogen carbonate aqueous solution and saturated brine and dried over sodium sulfate and concentrated and dried under reduced pressure, and 1.00 g (quantitative yield) of the title compound was obtained as a brown oil.
MS(EI) m/z:157 ($M^+$).

(404b) 2-(tert-butyl-dimethyl-silanyloxymethyl)-6-chloro-phenylamine

Imidazole (0.70 g) and tert-butyldimethylsilyl chloride (0.964 g) were added to a solution of (2-amino-3-chlorophenyl)-methanol (1.00 g) obtained in Example (404a) in dimethylformamide (5 mL) at room temperature. The reaction mixture was stirred for 17 hours and diluted with ethyl acetate and washed with water (twice) and saturated brine and dried over sodium sulfate and concentrated. The residue was purified by column chromatography (hexane:ethyl acetate 10:1 → dichloromethane: ethyl acetate 10:1) and 582 mg (37%) of the title compound was obtained as a light brown oil.
MS(FAB) m/z: 272$(M + H)^+$.

(404c)(Example 404) 4-{4-[3-(2-methoxy-5-methyl-phenyl)-ureido]-phenyl}-piperazine-1-carboxylic acid (2-chloro-6-hydroxymethyl-phenyl)-amide

407 mg (64%) of silyl ether was obtained as a white solid from 1-(2-methoxy-5-methyl-phenyl)-3-(4-piperazin-1-yl-phenyl)-urea (340 mg) obtained in Example (47a) and 2-(tert-butyl-dimethyl-silanyloxymethyl)-6-chloro-phenylamine (271 mg) obtained in Example (404b) in the same manner as in Example 170. This silyl ether was deprotected in the same manner as in Example (209d) and 318 mg (95%) of the title compound was obtained as a white solid.
$^1$H NMR(400MHz,DMSO-d6):δ(ppm)=9.09(1H, s), 8.19(1H, s), 8.07(1H, s), 7.99(1H, s), 7.46(1H, d, J=7.0Hz), 7.39(1H, d, J=8.6Hz), 7.34(2H, d, J=9.0Hz), 7.29(1H, dd, J=8.0 and 8.0Hz), 6.95(2H, d, J=9.0Hz), 6.89(1H, d, J=7.8Hz), 6.73 (1H, d, J=8.2Hz), 5.22(1H, t, J=5.5Hz), 4.47(2H, d, J=5.5Hz), 3.84(3H, s), 3.61(4H, brs), 3.08(4H, brs), 2.23(3H, s).
MS($ES^+$) m/z:525 $(M + H)^+$.
Melting point: 170-171˚C.

(Example 405) 4- {4-[3-(2-methoxy-5-methyl-phenyl)-ureido]-phenyl}-piperazine-1-carboxylic acid (2-hydroxymethyl-6-trifluoromethyl-phenyl)-amide (Compound No. 1-779)

(405a) 2-(tert-butyl-dimethyl-silanyloxymethyl)-6-trifluoromethyl-phenylamine

A benzyl alcohol compound was obtained from 3-(trifluoromethyl) anthranilic acid (1 g) as a colourless transparent oil in the same manner as in Example (404a). This oil was reacted in the same manner as in Example (404b) and 868 mg (58%) of the title compound was obtained as a colourless transparent oil.
MS(EI) m/z:157 ($M^+$).

(405b) 4-{4-[3-(2-methoxy-5-methyl-phenyl)-ureido]-phenyl}-piperazine-1-carboxylic acid (2-hydroxymethyl-6-trifluoromethyl-phenyl)-amide

144 mg (36%) of silyl ether was obtained as a white solid from 1-(2-methoxy-5-methyl-phenyl)-3-(4-piperazin-1-yl-phenyl)-urea (204 mg) obtained in Example (47a) and 2-(tert-butyl-dimethyl-silanyloxymethyl)-6-trifluoromethyl-phenylamine (183 mg) obtained in Example (405a) in the same manner as in Example 170. This silyl ether was deprotected in the same manner as in Example (209d) and 27 mg (23%) of the title compound was obtained as a white solid.
$^1$H NMR(400MHz,DMSO-d6):δ(ppm)=9.06(1H, s), 8.16(1H, s), 8.04(1H, s), 7.96(1H, d, J=2.0Hz), 7.79(1H, d, J=6.7Hz), 7.59(1H, d, J=6.6Hz), 7.49(1H, dd, J=7.9 and 7.9Hz), 7.31(2H, d, J=9.0Hz), 6.93(2H, d, J=9.0Hz), 6.86(1H, d, J=8.2Hz), 6.71(1H, dd, J=8.2 and 1.6Hz), 5.25(1H, t, J=5.5Hz), 4.51(2H, brs), 3.82(3H, s), 3.57(4H, t, J=4.3Hz), 3.05(4H, t, J=4.7Hz), 2.22(3H, s).
MS($ES^+$) m/z:558 $(M + H)^+$.

Melting point: 139-140˚C.

(Example 406) 4- {4-[3-(2-methoxy-5-methyl-phenyl)-ureido]-phenyl}-piperazine-1-carboxylic acid {4-[(2-hydroxy-ethyl)-methyl-amino]-2-methyl-phenyl}-amide (Compound No. 1-757)

(406a) 2-[methyl-(3-methyl-4-nitro-phenyl)-amino]-ethanol

Potassium carbonate (3.32 g) and 2-(methylamino)ethanol (1.8 mL) were added to a solution of 5-fluoro-2-nitrotoluene (3.95 mL) in acetonitrile (40 mL) at room temperature. The reaction mixture was heated under reflux for 25 hours and concentrated and diluted with water, followed by extraction with ethyl acetate. The organic layer was washed with saturated brine and dried over sodium sulfate and concentrated. The residue was purified by column chromatography (dichloromethane:ethyl acetate 1:0 → 3:1) and 3.51 g (84%) of the title compound was obtained as a yellow solid.
$^1$H NMR(400MHz,$CDCl_3$):δ(ppm)=8.05(1H, d, J=9.0Hz), 6.54(1H, dd, J=9.2 and 2.9Hz), 6.46(1H, d, J=2.7Hz), 3.87(2H, q, J=5.5Hz), 3.60(2H, t, J=5.7Hz), 3.11(3H, s), 2.62(3H, s), 1.76(1H, t, J=5.5Hz).

(406b) [2-(tert-butyl-dimethyl-silanoxy)-ethyl]-methyl-(3-methyl-4-nitrophenyl)-amine

Imidazole (2.27 g) and tert-butyldimethylsilyl chloride (3.02 g) were added to a solution of 2-[methyl-(3-methyl-4-nitro-phenyl)-amino]-ethanol (3.51 g) obtained in Example (406a) in dimethylformamide (50 mL) at room temperature. The reaction mixture was stirred for 16 hours and diluted with ethyl acetate and washed with a saturated sodium hydrogen carbonate aqueous solution and saturated brine and dried over sodium sulfate and concentrated and dried under reduced pressure, and 5.95 g (quantitative yield) of the title compound was obtained as a yellow oil.
$^1$H NMR(400MHz,$CDCl_3$):δ(ppm)=8.07(1H, d, J=9.4Hz), 6.50(1H, dd, J=9.4 and 2.7Hz), 6.43(1H, d, J=2.7Hz), 3.79(2H, t, J=5.6Hz), 3.55(2H, t, J=5.5Hz), 3.08(3H, s), 2.63(3H, s), 0.85(9H, s), 0.00(6H, s).

(406c) $N^4$-[2-(tert-butyl-dimethyl-silanoxy)-ethyl]-2,$N^4$-dimethyl-benzene-1,4-diamine

A suspension of [2-(tert-butyl-dimethyl-silanoxy)-ethyl]-methyl-(3-methyl-4-nitrophenyl)-amine (5.42 g) obtained in Example (406b) and 10% palladium-carbon (0.54 g) in anhydrous tetrahydrofuran (60 mL) was stirred under a hydrogen atmosphere at room temperature for four days and filtered and concentrated. The residue was purified by column chromatography (dichloromethane:ethyl acetate 1:0 → 3:1) and 4.75 g (97%) of the title compound was obtained as a dark brown oil.
$^1$H NMR(400MHz,$CDCl_3$):δ(ppm)=6.59(1H, d, J=8.6Hz), 6.51(1H, d, J=2.5Hz), 6.47(1H, dd, J=8.4 and 2.5Hz), 3.71(2H, t, J=6.5Hz), 3.30(2H, t, J=6.5Hz), 3.21(2H, brs), 2.84(3H, s), 2.13(3H, s), 0.86(9H, s), 0.00(6H, s).

(406d) 4-{4-[3-(2-methoxy-5-methyl-phenyl)-ureido]-phenyl}-piperazine-1-carboxylic acid {4-[(2-hydroxy-ethyl)-methyl-amino]-2-methyl-phenyl}-amide

679 mg (86%) of silyl ether was obtained as a white solid from 1-(2-methoxy-5-methyl-phenyl)-3-(4-piperazin-1-yl-phenyl)-urea (408 mg) obtained in Example (47a) and $N^4$-[2-(tert-butyl-dimethyl-silanoxy)-ethyl]-2,$N^4$-dimethyl-benzene-1,4-diamine (353 mg) obtained in Example (406c) in the same manner as in Example 170. This silyl ether was deprotected in the same manner as in Example (209d) and 30 mg (5%) of the title compound was obtained as a white solid.
$^1$H NMR(400MHz,DMSO-d6):δ(ppm)=9.05(1H, s), 8.03(1H, s), 7.96(1H, d, J=2.4Hz), 7.88(1H, s), 7.30(2H, d, J=9.0Hz), 6.92(2H, d, J=9.0Hz), 6.87(2H, dd, J=8.2 and 8.2Hz), 6.70(1H, dd, J=8.2 and 1.6Hz), 6.50(1H, d, J=2.8Hz), 6.45(1H, dd, J=9.0 and 2.3Hz), 4.61(1H, t, J=5.4Hz), 3.82(3H, s), 3.54(4H, t, J=5.3Hz), 3.51(2H, t, J=5.7Hz), 3.15(2H, t, J=8.6Hz), 3.05(4H, t, J=4.9Hz), 2.88(3H, s), 2.22(3H, s), 2.09(3H, s).
MS($ES^+$) m/z:547 $(M + H)^+$.
Melting point: 208-210˚C.

(Example 407) 4-{4-[3-(5-fluoro-2-methoxy-phenyl)-ureido]-phenyl}-piperazine-1-carboxylic acid (2,6-difluoro-phenyl)-amide (Compound No. 1-1)

197 mg (99%) of the title compound was obtained as a pale pink powder from 1-(5-fluoro-2-methoxy-phenyl)-3-(4-piperazin-1-yl-phenyl)-urea(138 mg) obtained in Example (96a) and 2,6-difluorophenyl isocyanate (93 mg) in the same manner as in Example 49.
$^1$H NMR(400MHz,DMSO-d6):δ(ppm)=9.20(1H, s), 8.37(1H, s), 8.33(1H, s), 8.03(1H, dd, J=11.9 and 2.6Hz), 7.34(2H, d, J=9.0Hz), 7.29(1H, dd, J=8.4 and 8.4Hz), 7.13(1H, d, J=7.8Hz), 7.11(1H, d, J=7.8Hz), 7.00(1H, dd, J=9.2 and 6.0Hz), 6.96(2H, d, J=8.9Hz), 6.75(1H, ddd, J=8.2 ,8.2 and 3.1Hz), 3.88(3H, s), 3.60(4H, t, J=4.9Hz), 3.09(4H, t, J=4.9Hz).

MS($ES^+$) m/z:500 $(M + H)^+$.
Melting point: 154-155˚C.

(Example 408) 4- {4-[3-(2-methoxy-5-methyl-phenyl)-ureido]-phenyl}-piperazine-1-carboxylic acid [5-(2-hydroxy-ethoxy)-2-methyl-phenyl]-amide (Compound No. 1-771)

(408a) 2-(4-methyl-3-nitro-phenoxy)-ethanol

6.48 g (66%) of the title compound was obtained as a yellow solid from 4-methyl-3-nitrophenol (7.66 g) and 2-bromoethanol (3.9 mL) in the same manner as in Example (208a).
$^1$H NMR(400MHz,$CDCl_3$):δ(ppm)=7.52(1H, d, J=2.7Hz), 7.23(1H, d, J=8.2Hz), 7.08(1H, dd, J=8.2 and 2.7Hz), 4.12(2H, t, J=4.5Hz), 3.99(2H, t, J=4.1Hz), 2.32(1H, brs), 2.08(3H, s).

(408b) tert-butyl-[2-(4-methyl-3-nitro-phenoxy)-ethoxy]-dimethylsilane

8.97 g (88%) of the title compound was obtained as a yellow oil from 2-(4-methyl-3-nitro-phenoxy)-ethanol (6.48 g) obtained in Example (408a) and tert-butyldimethylsilyl chloride (5.94 g) in the same manner as in Example (208b).
$^1$H NMR(400MHz,$CDCl_3$):δ(ppm)=7.44(1H, d, J=2.4Hz), 7.13(1H, d, J=8.6Hz), 6.99(1H, dd, J=8.6 and 2.4Hz), 3.99(2H, t, J=4.9Hz), 3.89(2H, t, J=5.1Hz), 2.43(3H, s), 0.81(9H, s), 0.01(6H, s).

(408c) 5-[2-(tert-butyl-dimethyl-silanyloxy)-ethoxy]-2-methyl-phenylamine

A suspension of tert-butyl-[2-(4-methyl-3-nitro-phenoxy)-ethoxy]-dimethylsilane (8.97 g) obtained in Example (408b) and 10% palladium-carbon (0.89 g) in anhydrous tetrahydrofuran (100 mL) was stirred under a hydrogen atmosphere at room temperature for 24 hours. The reaction mixture was filtered and concentrated. The residue was purified by column chromatography (hexane:ethyl acetate 1:1) and 7.53 g (93%) of the title compound was obtained as a white solid.
$^1$H NMR(400MHz,$CDCl_3$):δ(ppm)=6.93(1H, d, J=9.0Hz), 6.29-6.28(2H, m), 3.99-3.93(4H, m), 3.61(2H, brs), 2.10(3H, s), 0.91(9H, s), 0.10(6H, s).

(408d) 4-{4-[3-(2-methoxy-5-methyl-phenyl)-ureido]-phenyl}-piperazine-1-carboxylic acid [5-(2-hydroxy-ethoxy)-2-methyl-phenyl]-amide

686 mg (53%) of silyl ether was obtained as a pale yellow solid from 1-(2-methoxy-5-methyl-phenyl)-3-(4-piperazin-1-yl-phenyl)-urea (681 mg) obtained in Example (47a) and 5-[2-(tert-butyl-dimethyl-silanyloxy)-ethoxy]-2-methylphenylamine (563 mg) obtained in Example (408c) in the same manner as in Example 170. This silyl ether was deprotected with tetrabutylammonium fluoride in an anhydrous tetrahydrofuran solution and 444 mg (79%) of the title compound was obtained as a white solid.
$^1$H NMR(400MHz,DMSO-d6):δ(ppm)=9.09(1H, s), 8.08(1H, s), 8.07(1H, s), 7.99(1H, s), 7.33(2H, d, J=7.8Hz), 7.06(IH, d, J=8.6Hz), 6.94(2H, d, J=8.6Hz), 6.88(2H, d, J=9.0Hz), 6.72(1H, d, J=7.8Hz), 6.64(1H, d, J=7.4Hz), 4.84(1H, t, J=5.7Hz), 3.92(2H, t, J=4.3Hz), 3.83(3H, s), 3.69(2H, dd, J=8.6 and 4.7Hz), 3.58(4H, brs), 3.08(4H, brs), 2.22(3H, s), 2.10(3H, s).
MS($ES^+$) m/z:534 $(M + H)^+$.
Melting point: 121-124˚C.

(Example 409) 6-[3-(2-methoxy-5-methyl-phenyl)-ureido]-3,4-dihydro-1H-isoquinoline-2-carboxylic acid [2-chloro-4-(2-hydroxy-ethoxy)-phenyl]-amide (Compound No. 3-74)

Silyl ether was obtained from 1-(2-methoxy-5-methyl-phenyl)-3-(1,2,3,4-tetrahydro-isoquinolin-6-yl)-urea (87 mg) obtained in Example (202b) and 4-[2-(tert-butyl-dimethyl-silanyloxy)-ethoxy]-2-chloro-benzoic acid (83 mg) obtained in Example (248d) in the same manner as in Example (248e). This silyl ether was deprotected with tetrabutylammonium fluoride in the same manner as in Example (248e) and 93 mg (71 %) of the title compound was obtained as a pale yellow solid.
$^1$H NMR(400MHz,DMSO-d6):δ(ppm)=2.22(3H, s), 2.82(2H, t, J=5.2Hz), 3.71-3.63(4H, m), 3.83(3H, s), 3.98(2H, t, J=4.5Hz), 4.54(2H, s), 4.86(1H, t, J=5.5Hz), 6.72(1H, brd, J=8.3Hz), 6.84-6.90(2H, m), 7.07-7.01(2H, m), 7.18(1H, brd, J=7.9Hz), 7.30(1H, d, J=9.4Hz), 7.36(1H, s), 7.97(1H, s), 8.11(1H, s), 8.13(1H, s), 9.23(1H, s).
MS(APCI) m/z:525, 527 $(M + H)^+$.
Melting point: 110-113˚C.

(Example 410) 6-[3-(3-ethoxy-phenyl)-ureido]-3,4-dihydro-1H-isoquinoline-2-carboxylic acid [2-chloro-4-(2-hydroxy-ethoxy)-phenyl]-amide (Compound No. 3-73)

Silyl ether was obtained from 1-(3-ethoxy-phenyl)-3-(1,2,3,4-tetrahydro-isoquinolin-6-yl)-urea (62 mg) obtained in Example (291b) and 4-[2-(tert-butyldimethyl-silanyloxy)-ethoxy]-2-chloro-benzoic acid (66 mg) obtained in Example (248d) in the same manner as in Example (248e). This silyl ether was deprotected with tetrabutylammonium fluoride in the same manner as in Example (248e) and 37 mg (35%) of the title compound was obtained as a white solid.
$^1$H NMR(400MHz,DMSO-d6):δ(ppm)=1.32(3H, t, J=6.7Hz), 2.82(2H, brs), 3.73-3.63(4H, m), 4.03-3.94(4H, m), 4.54(2H, s), 4.87(1H, t, J=4.9Hz), 6.51(1H, d, J=8.6Hz), 6.92-6.84(2H, m), 7.09-7.01(2H, m), 7.23-7.10(3H, m), 7.36-7.26(2H, m), 8.12(1H, s), 8.56(1H, s), 8.61(1H, s).
MS(APCI) m/z:525, 527 $(M + H)^+$.
Melting point: 148-150˚C.

(Example 411) 4- {4-[3-(2-fluoro-phenyl)-ureido]-phenyl}-3,6-dihydro-2H-pyridine-1-carboxylic acid [2-chloro-4-(2,3-dihydroxy-propoxy)-phenyl]-amide (Compound No. 5-31)

A urea compound was obtained from 1-(2-fluoro-phenyl)-3-[4-(1,2,3,6-tetrahydro-pyridin-4-yl)-phenyl]-urea (62 mg) obtained in Example (227b) and 2-chloro-4-(2,2-dimethyl-1,3-dioxolan-4-ylmethoxy)-benzoic acid (57 mg) obtained in Example (277c) in the same manner as in Example (248e). This urea compound was deprotected in the same manner as in Example (275d) and 16 mg (14%) of the title compound was obtained as a brown solid.
$^1$H NMR(400MHz,DMSO-d6):δ(ppm)=2.55-2.44(2H, m), 3.43(2H, brs), 3.65(2H, brs), 3.77(1H, brs), 3.86(1H, dd, J=9.5 and 6.5Hz), 4.00(1H, dd, J=9.8 and 3.1Hz), 4.11(2H, s), 4.67(1H, brs), 4.96(1H, d, J=3.9Hz), 6.14(1H, s), 6.87(1H, dd, J=8.8 and 2.6Hz), 7.06-6.96(2H, m), 7.13(1H, dd, J=7.2 and 7.2Hz), 7.27-7.19(1H, m), 7.31(1H, d, J=8.6Hz), 7.40(2H, d, J=8.6Hz), 7.43(2H, d, J=8.6Hz), 8.18-8.07(2H, m), 8.53(1H, s), 9.10(1H, s).
MS(APCI) m/z:555, 557 $(M + H)^+$.
Melting point: 161-165˚C.

(Example 412) 4- (5-[3-(2-ethoxy-phenyl)-ureido]-pyridin-2-yl) -piperazine- I - carboxylic acid [4-(2-hydroxy-ethoxy)-2-trifluoromethyl-phenyl]-amide (Compound No. 1-610)

92.9 mg (26%) of silyl ether was obtained as a white solid from 1-(2-ethoxyphenyl)-3-(6-piperazin-1-yl-pyridin-3-yl)-urea (171 mg) obtained in Example (141a) and 4-[2-(tert-butyl-dimethyl-silanyloxy)-ethoxy]-2-trifluoromethyl-phenylamine (168 mg) obtained in Example (209c) in the same manner as in Example 170. This silyl ether was deprotected with tetrabutylammonium fluoride in an anhydrous tetrahydrofuran solution and 37.9 mg (51%) of the title compound was obtained as a white solid.
$^1$H NMR(400MHz,DMSO-d6):δ(ppm)=9.21(1H, s), 8.23(1H, s), 8.19(1H, s), 8.12(1H, d, J=7.8Hz), 8.04(1H, d, J=2.8Hz), 7.78(1H, dd, J=9.0 and 2.8Hz), 7.32(1H, d, J=9.0Hz), 7.23(1H, dd, J=9.0 and 2.7Hz), 7.19(1H, d, J=2.7Hz), 7.01(1H, d, J=7.9Hz), 6.96-6.84(3H, m), 4.95-4.88(1H, m), 4.13(2H, q, J=6.9Hz), 4.10-4.05(2H, m), 3.77-3.69(2H, m), 3.57-3.51 (4H, m), 3.47-3.41(4H, m), 1.42(3H, t, J=6.9Hz).
MS$(ES^+)$ m/z:589 $(M + H)^+$.
Melting point: 181-182˚C.

(Example 413) 4-{4-[3-(2-fluoro-phenyl)-ureido]-phenyl}-piperazine-1-carboxylic acid (3,5-dichloro-pyridin-2-yl)-amide (Compound No. 1-718)

(413a) (3,5-dichloro-pyridin-2-yl)-thiocarbamic acid methyl ester

4.66 (49%) of the title compound was obtained as a brown solid from 2-amino-3,5-dichloropyridine (6.52 g) in the same manner as in Example (294a).
MS(FAB) m/z:237 $(M + H)^+$.

(413b) 4-{4-[3-(2-fluoro-phenyl)-ureido]-phenyl}-piperazine-1-carboxylic acid (3,5-dichloro-pyridin-2-yl)-amide

104 mg (44%) of the title compound was obtained as a beige solid from 1-(2-fluoro-phenyl)-3-(4-piperazin-1-yl-phenyl)-urea (148 mg) obtained in Example (125a) and (3,5-dichloro-pyridin-2-yl)-thiocarbamic acid methyl ester (0.39 g) obtained in Example (413a) in the same manner as in Example (294b).
$^1$H NMR(400MHz,DMSO-d6):δ(ppm)=9.26(1H, s), 8.82(1H, s), 8.44-8.41(1H, m), 8.38(1H, d, J=2.3Hz), 8.29(1H, d, J=2.4Hz), 8.28-8.25(1H, m), 8.19-8.11(1H, m), 7.31 (2H, d, J=8.6Hz), 7.20(1H, dd, J=10.3 and 10.3Hz), 7.11(1H, dd,

J=9.2 and 9.2Hz), 7.00-6.91(1H, m), 6.93(1H, d, J=9.0Hz), 3.59(4H, brs), 3.08(4H, brs).
MS($ES^+$) m/z:503 $(M + H)^+$.
Melting point: 199-201˚C.

(Example 414) 4-{4-[3-(2-ethoxy-5-fluoro-phenyl)-ureido]-phenyl}-piperazine-1-carboxylic acid (3,5-dichloro-pyridin-2-yl)-amide (Compound No. 1-722)

158 mg (76%) of the title compound was obtained as a beige solid from 1-(2-ethoxy-5-fluoro-phenyl)-3-(6-piperazin-1-yl-pyridin-3-yl)-urea (136 mg) obtained in Example (300a) and (3,5-dichloro-pyridin-2-yl)-thiocarbamic acid methyl ester (0.39 g) obtained in Example (413a) in the same manner as in Example (294b).
$^1$H NMR(400MHz,DMSO-d6):δ(ppm)=9.26(2H, s), 8.39-8.38(1H, m), 8.18-8.11(2H, m), 8.02-7.99(1H, m), 7.34-7.30(2H, m), 7.00-6.91(3H, m), 6.71-6.66(1H, m), 4.14-4.07(2H, m), 3.62-3.57(4H, m), 3.11-3.06(4H, m), 1.43-1.37(3H, m).
MS($ES^+$) m/z:548 $(M + H)^+$.
Melting point: 224-226˚C.

(Example 415) 4-{4-[3-(5-chloro-2-ethoxy-phenyl)-ureido]-phenyl}-piperazine-1-carboxylic acid (3,5-dichloro-pyridin-2-yl)-amide (Compound No. 1-724)

80 mg (41%) of the title compound was obtained as a beige solid from 1-(5-chloro-2-ethoxy-phenyl)-3-(4-piperazin-1-yl-phenyl)-urea (131 mg) obtained in Example (102a) and (3,5-dichloro-pyridin-2-yl)-thiocarbamic acid methyl ester (0.39 g) obtained in Example (413a) in the same manner as in Example (294b).
$^1$H NMR(400MHz,DMSO-d6):δ(ppm)=9.26(1H, s), 9.25(1H, s), 8.38(1H, d, J=2.4Hz), 8.22(1H, d, J=2.8Hz), 8.16(1H, d, J=2.3Hz), 8.11(1H, s), 7.32(2H, d, J=9.0Hz), 7.00-6.91(4H, m), 4.13(2H, q, J=7.1Hz), 3.59(4H, brs), 3.09(4H, brs), 1.40 (3H, t, J=7.1Hz).
MS($ES^+$) m/z:564 $(M + H)^+$.
Melting point: 225-227˚C.

(Example 416) 4- {5-[3-(2-methoxy-5-methyl-phenyl)-ureido]-pyridin-2-yl}-piperazine-1-carboxylic acid (3,5-dichloro-pyridin-2-yl)-amide (Compound No. 1-737)

15 mg (8%) of the title compound was obtained as a light brown solid from 1-(2-methoxy-5-methyl-phenyl)-3-(4-piperazin-1-yl-phenyl)-urea (124 mg) obtained in Example (47a) and (3,5-dichloro-pyridin-2-yl)-thiocarbamic acid methyl ester (0.39 g) obtained in Example (413a) in the same manner as in Example (294b).
$^1$H NMR(400MHz,DMSO-d6):δ(ppm)=9.26(1H, s), 9.07(1H, s), 8.38(1H, d, J=2.4Hz), 8.17-8.03 (m, 1H), 8.16(1H, dd, J=8.2 and 2.3Hz), 8.09(1H, s), 7.94(1H, d, J=1.6Hz), 7.75-7.71(1H, m), 6.87(2H, d, J=8.3Hz), 6.71(1H, d, J=11.7Hz), 3.82(3H, s), 3.59-3.54(4H, m), 3.48-3.42(4H, m), 2.22(3H, s).
MS(FAB) m/z:531 $(M + H)^+$.
Melting point: 130-132˚C.

(Example 417) 4- {4-[3-(2-ethoxy-phenyl)-ureido]-phenyl}-piperazine-1-carboxylic acid (3,5-dichloro-pyridin-2-yl)-amide (Compound No. 1-720)

16 mg (8%) of the title compound was obtained as a pale yellow solid from 1-(2-ethoxy-phenyl)-3-(4-piperazin-1-yl-phenyl)-urea (131 mg) obtained in Example (80a) and (3,5-dichloro-pyridin-2-yl)-thiocarbamic acid methyl ester (0.39 g) obtained in Example (413a) in the same manner as in Example (294b).
$^1$H NMR(400MHz,DMSO-d6):δ(ppm)=9.26(1H, s), 9.15(1H, s), 8.40-8.38(1H, m), 8.17(1H, s), 8.10(1H, d, J=7.4Hz), 7.95(1H, s), 7.32(2H, d, J=9.4Hz), 7.01-6.84(5H, m), 4.12(2H, q, J=6.9Hz), 3.65-3.57(4H, m), 3.13-3.05(4H, m), 1.41 (3H, t, J=6.9Hz).
MS($ES^+$) m/z:530 $(M + H)^+$.
Melting point: 201-203˚C.

(Example 418) 4-{4-[3-(5-chloro-2-ethoxy-phenyl)-ureido]-phenyl}-piperazine-1-carboxylic acid (3-methyl-pyridin-4-yl)-amide (Compound No. 1-700)

(418a) (3-methyl-pyridin-4-yl)-thiocarbamic acid methyl ester

6.22 (85%) of the title compound was obtained as a pale yellow solid from 4-amino-3-methylpyridine (4.36 g) in the same manner as in Example (294a).

$^1$H NMR(400MHz,$CDCl_3$):δ(ppm)=8.41(1H, d, J=5.5Hz), 8.37(1H, s), 8.00(1H, d, J=5.4Hz), 7.18(1H, brs), 2.47(3H, s), 2.25(3H, s).

(418b) 4- {4-[3-(5-chloro-2-ethoxy-phenyl)-ureido]-phenyl}-piperazine-1-carboxylic acid (3-methyl-pyridin-4-yl)-amide

109 mg (70%) of the title compound was obtained as a white solid from 1-(5-chloro-2-ethoxy-phenyl)-3-(4-piperazin-1-yl-phenyl)-urea (114 mg) obtained in Example (102a) and (3-methyl-pyridin-4-yl)-thiocarbamic acid methyl ester (0.52 g) obtained in Example (418a) in the same manner as in Example (294b).
$^1$H NMR(400MHz,DMSO-d6):δ(ppm)=9.28(1H, s), 8.33-8.20(4H, m), 8.14(1H, s), 7.52-7.46(1H, m), 7.41-7.32(2H, m), 7.06-6.91(4H, m), 4.14(2H, q, J=6.9Hz), 3.69-3.60(4H, m), 3.18-3.08(4H, m), 2.20(3H, s), 1.41(3H, t, J=6.9Hz).
MS(ES$^+$) m/z:509 (M + H)$^+$.
Melting point: 196-198˚C.

(Example 419) 4- {4-[3-(2-ethoxy-5-methyl-phenyl)-ureido]-phenyl} -piperazine-1 - carboxylic acid (3-methyl-pyridin-4-yl)-amide (Compound No. 1-702)

57 mg (35%) of the title compound was obtained as a white solid from 1-(2-ethoxy-5-methylphenyl)-3-(4-piperazin-1-yl-phenyl)-urea (114 mg) obtained in Example (83a) and (3-methyl-pyridin-4-yl)-thiocarbamic acid methyl ester (0.52 g) obtained in Example (418a) in the same manner as in Example (294b).
$^1$H NMR(400MHz,DMSO-d6):δ(ppm)=9.13(1H, s), 8.26(1H, s), 8.22(1H, d, J=5.5Hz), 8.19(1H, s), 7.96(1H, d, J=1.6Hz), 7.89(1H, s), 7.45(1H, d, J=5.5Hz), 7.32(2H, d, J=9.0Hz), 6.93(2H, d, J=9.0Hz), 6.85(1H, d, J=8.2Hz), 6.68(1H, d, J=8.2Hz), 4.07(2H, q, J=7.3Hz), 3.61(4H, t, J=4.9Hz), 3.10(4H, t, J=4.7Hz), 2.21(3H, s), 2.19(3H, s), 1.38(3H, t, J=6.8Hz).
MS(ES$^+$) m/z:489 (M + H)$^+$.
Melting point: 130-132˚C.

(Example 420) 4-{4-[3-(2-ethoxy-phenyl)-ureido]-phenyl}-piperazine-1-carboxylic acid (3-chloro-pyridin-2-yl)-amide (Compound No. 1-913)

112 mg (76%) of the title compound was obtained as a white solid from 1-(2-ethoxy-phenyl)-3-(4-piperazin-1-yl-phenyl)-urea (102 mg) obtained in Example (80a) and (3-chloro-pyridin-2-yl)-carbamic acid tert-butyl ester (82 mg) obtained in Example (336b) in the same manner as in Example (294d).
$^1$H NMR(400MHz,DMSO-d6):δ(ppm)=9.14(1H, s), 9.11(1H, s), 8.29(1H, dd, J=4.6 and 1.5Hz), 8.10(1H, dd, J=7.4 and 2.0Hz), 7.95(1H, s), 7.88(1H, dd, J=7.8 and 1.6Hz), 7.32(2H, d, J=9.0Hz), 7.20(1H, dd, J=8.1 and 4.9Hz), 6.99-6.83(3H, m), 6.93(2H, d, J=9.0Hz), 4.12(2H, q, J=7.1Hz), 3.60(4H, t, J=4.9Hz), 3.07(4H, t, J=4.5Hz), 1.41(3H, t, J=7.1Hz).
MS(ES$^+$) m/z:495 (M + H)$^+$.
Melting point: 104-105˚C.

(Example 421) 4-{4-[3-(3,5-dimethoxy-phenyl)-ureido]-phenyl}-piperazine-1-carboxylic acid (3-chloro-pyridin-2-yl)-amide (Compound No. 1-911)

161 mg (63%) of the title compound was obtained as a white solid from 1-(3,5-dimethoxy-phenyl)-3-(4-piperazin-1-yl-phenyl)-urea (178 mg) obtained in Example (216b) and (3-chloro-pyridin-2-yl)-carbamic acid tert-butyl ester (137 mg) obtained in Example (336b) in the same manner as in Example (294d).
$^1$H NMR(400MHz,DMSO-d6):δ(ppm)=9.11(1H, s), 8.54(1H, s), 8.36(1H, s), 8.29(1H, dd, J=4.6 and 1.5Hz), 7.88(1H, dd, J=8.2 and 1.6Hz), 7.29(2H, d, J=8.9Hz), 7.20(1H, dd, J=7.9 and 4.7Hz), 6.92(2H, d, J=9.4Hz), 6.65(2H, d, J=2.4Hz), 6.10(1H, t, J=2.2Hz), 3.70(6H, s), 3.59(4H, t, J=4.7Hz), 3.07(4H, t, J=4.7Hz).
MS(ES$^+$) m/z:511 (M + H)$^+$.
Melting point: 101-102˚C.

(Example 422) 4-{4-[3-(3-ethoxy-phenyl)-ureido]-phenyl}-piperazine-1-carboxylic acid (3-chloro-pyridin-2-yl)-amide (Compound No. 1-910)

55 mg (22%) of the title compound was obtained as a white solid from 1-(3-ethoxy-phenyl)-3-(4-piperazin-1-yl-phenyl)-urea (170 mg) obtained in Example (215b) and (3-chloro-pyridin-2-yl)-carbamic acid tert-butyl ester (137 mg) obtained in Example (336b) in the same manner as in Example (294d).
$^1$H NMR(400MHz,DMSO-d6) :δ(ppm)=9.11 (1H, s), 8.53 (1H, s), 8.39(1H, s), 8.29(1H, dd, J=4.6 and 1.5Hz), 7.88(1H, dd, J=7.8 and 1.6Hz), 7.30(2H, d, J=9.0Hz), 7.20(1H, dd, J=7.9 and 4.7Hz), 7.16-7.14(2H, m), 6.92(2H, d, J=9.0Hz), 6.87(1H, dd, J=8.2 and 1.2Hz), 6.49(1H, dd, J=8.1 and 2.2Hz), 3.98(2H, q, J=6.8Hz), 3.60(4H, t, J=4.7Hz), 3.07(4H, t,

J=4.7Hz), 1.32(3H, t, J=6.9Hz).
MS($ES^+$) m/z:495 $(M + H)^+$.
Melting point: 102-103˚C.

(Example 423) 4-{4-[3-(2-methoxy-3-methyl-phenyl)-ureido]-phenyl}-piperazine-1-carboxylic acid (3-chloro-pyridin-2-yl)-amide (Compound No. 1-912)

40 mg (41 %) of the title compound was obtained as a light brown solid from 1-(2-methoxy-3-methyl-phenyl)-3-(4-piperazin-1-yl-phenyl)-urea (68 mg) obtained in Example (375d) and (3-chloro-pyridin-2-yl)-carbamic acid tert-butyl ester (150 mg) obtained in Example (336b) in the same manner as in Example (294d).
$^1$H NMR(400MHz,DMSO-d6):δ(ppm)=9.11(1H, s), 9.11(1H, s), 8.30(1H, dd, J=4.7 and 1.5Hz), 8.19(1H, s), 8.00(1H, dd, J=8.2 and 1.2Hz), 7.88(1H, dd, J=8.2 and 1.6Hz), 7.32(2H, d, J=9.0Hz), 7.20(1H, dd, J=7.9 and 4.7Hz), 6.93(2H, d, J=9.0Hz), 6.77(1H, dd, J=7.8 and 0.8Hz), 3.69(3H, s), 3.60(4H, t, J=4.9Hz), 3.07(4H, t, J=5.1Hz), 2.24(3H, s).
MS($ES^+$) m/z:495 $(M + H)^+$.
Melting point: 80-82˚C.

(Example 424) 4-{4-[3-(2-methoxy-5-methyl-phenyl)-ureido]-phenyl}-piperidine-1-carboxylic acid (3-chloro-pyridin-2-yl)-amide (Compound No. 4-104)

47 mg (48%) of the title compound was obtained as a brownish white solid from 1-(2-methoxy-5-methyl-phenyl)-3-(4-piperazin-4-yl-phenyl)-urea (68 mg) obtained in Example (228e) and (3-chloro-pyridin-2-yl)-carbamic acid tert-butyl ester (60 mg) obtained in Example (336b) in the same manner as in Example (294d). $^1$H NMR(400MHz,DMSO-d6):δ (ppm)=1.64-1.49(2H, m), 1.83-1.73(2H, m), 2.23(3H, s), 2.76-2.65(1H, m), 2.91(2H, t, J=12.7Hz), 3.84(3H, s), 4.22(2H, d, J=13.3Hz), 6.74(1H, d, J=7.4Hz), 6.89(1H, d, J=8.6Hz), 7.16(2H, d, J=7.5Hz), 7.24-7.12(1H, m), 7.40(2H, d, J=7.4Hz), 7.90(1H, d, J=7.9Hz), 7.99(1H, s), 8.14(1H, s), 8.31(1H, d, J=4.3Hz), 9.03(1H, s), 9.24(1H, s).
MS(APCI) m/z:494, 496 $(M + H)^+$.
Melting point: 197-201˚C.

(Example 425) 4-{4-[3-(2-methoxy-3-methyl-phenyl)-ureido]-phenyl}-piperazine-1-carboxylic acid (3,5-difluoro-pyridin-2-yl)-amide (Compound No. 1-921)

33 mg (33%) of the title compound was obtained as a white solid from 1-(2-methoxy-3-methyl-phenyl)-3-(4-piperazin-1-yl-phenyl)-urea (68 mg) obtained in Example (375d) and (3,5-difluoro-pyridin-2-yl)-carbamic acid tert-butyl ester (110 mg) obtained in Example (338a) in the same manner as in Example (294d).
$^1$H NMR(400MHz,DMSO-d6):δ(ppm)=9.20(1H, s), 9.08(1H, s), 8.25(1H, d, J=2.8Hz), 8.19(1H, s), 8.00(1H, dd, J=8.0 and 1.0Hz), 7.91(1H, ddd, J=11.0, 8.6 and 2.8Hz), 7.32(2H, d, J=9.0Hz), 6.93(2H, d, J=9.0Hz), 6.92(1H, dd, J=8.0 and 8.0Hz), 6.77(1H, dd, J=8.0 and 1.0Hz), 3.69(3H, s), 3.59(4H, t, J=5.0Hz), 3.07(4H, t, J=5.0Hz), 2.24(3H, s).
MS($ES^+$) m/z:497 $(M + H)^+$.
Melting point: 187-188˚C.

(Example 426) 4-{4-[3-(2-methoxy-3-methyl-phenyl)-ureido]-phenyl}-piperazine-1-carboxylic acid (3-cyano-pyridin-2-yl)-amide (Compound No. 1-923)

35 mg (36%) of the title compound was obtained as a white solid from 1-(2-methoxy-3-methyl-phenyl)-3-(4-piperazin-1-yl-phenyl)-urea (68 mg) obtained in Example (375d) and (3-cyano-pyridin-2-yl)-carbamic acid tert-butyl ester (53 mg) obtained in Example (356a) in the same manner as in Example (294d).
$^1$H NMR(400MHz,DMSO-d6):δ(ppm)=9.81(1H, s), 9.08(1H, s), 8.57(1H, dd, J=4.9 and 1.8Hz), 8.21(1H, dd, J=7.6 and 1.8Hz), 8.19(1H, s), 8.00(1H, dd, J=8.0 and 1.0Hz), 7.32(2H, d, J=9.0Hz), 7.27(1H, dd, J=7.6 and 4.9Hz), 6.93(2H, d, J=9.0Hz), 6.92(1H, dd, J=8.0 and 8.0Hz), 6.76(1H, dd, J=8.0 and 1.0Hz), 3.69(3H, s), 3.64(4H, t, J=4.6Hz), 3.09(4H, t, J=4.6Hz), 2.24(3H, s).
MS($ES^+$) m/z:486 $(M + H)^+$.
Melting point: 201-202˚C.

(Example 427) 1-(4-{4-[2-hydroxy-2-(3-methyl-pyridin-2-yl)-acetyl]-piperazin-1-yl}-phenyl)-3-(2-methoxy-5-methyl-phenyl)-urea (Compound No. 1-816)

(427a) 3-methyl-pyridine-2-carbaldehyde

n-Butyl lithium (2.7 mol/L hexane solution, 11 mL) was added dropwise to a solution of 2-bromo-3-methylpyridine (5.10 g) in anhydrous tetrahydrofuran (60 mL) at -75˚C. After one hour, dimethylformamide (2.3 mL) was added at -75˚C. The reaction mixture was stirred at -75˚C for one hour and concentrated. The residue was purified by column chromatography (dichloromethane: ethyl acetate 10:1 → 1:1) and 2.00 g (55%) of the title compound was obtained as a yellow syrup. MS($ES^+$) m/z:122 $(M + H)^+$.

(427b) hydroxy-(3-methyl-pyridin-2-yl)-acetic acid ethyl ester

Triethylamine (0.23 mL) and trimethylsilyl cyanide (2.3 mL) were added to a solution of 3-methyl-pyridine-2-carbaldehyde (2.00 g) obtained in Example (427a) in dichloromethane (50 mL) at room temperature. The reaction mixture was stirred for 23 hours and concentrated to obtain a yellow syrup. Trimethylsilyl chloride (25 mL) was added to a solution of this yellow syrup in ethanol (25 mL) at room temperature. After 17 hours, the reaction mixture was concentrated and diluted with dichloromethane (50 mL), ethanol (25 mL) and a saturated sodium hydrogen carbonate aqueous solution (50 mL) and stirred for one hour. The mixture was concentrated to about 1/3 volume, followed by extraction with dichloromethane. The organic layer was dried over sodium sulfate and concentrated. The residue was purified by column chromatography (hexane:ethyl acetate 2:1 → 1:1) and 1.33 g (41 %) of the title compound was obtained as a yellow syrup. MS($ES^+$) m/z:196 $(M + H)^+$.

(427c) hydroxy-(3-methyl-pyridin-2-yl)-acetic acid

1N sodium hydroxide (1.4 mL) was added to a solution of hydroxy-(3-methyl-pyridin-2-yl)-acetic acid ethyl ester (180 mg) obtained in Example (427b) in ethanol (2 mL). The reaction mixture was stirred at room temperature for 2.5 hours and concentrated and neutralized with 1N hydrochloric acid (1.4 mL) and washed with ethyl acetate and dichloromethane (twice) and concentrated. After drying under reduced pressure, 195 mg of the title compound was obtained as a pale yellow solid.
MS($ES^+$) m/z:177 $(M + H)^+$.

(427d) 1-(4-{4-[2-hydroxy-2-(3-methyl-pyridin-2-yl)-acetyl]-piperazin-1-yl}-phenyl)-3-(2-methoxy-5-methyl-phenyl)-urea

45 mg (two steps, 20%) of the title compound was obtained as a white solid from 1-(2-methoxy-5-methylphenyl)-3-(4-piperazin-1-yl-phenyl)-urea (157 mg) obtained in Example (47a) and hydroxy-(3-methyl-pyridin-2-yl)-acetic acid (195 mg) obtained in Example (427c) in the same manner as in Example 377.
$^1$H NMR(400MHz,DMSO-d6):δ(ppm)=9.03(1H, s), 8.31(1H, d, J=4.7Hz), 8.02(1H, s), 7.95(1H, s), 7.62(1H, d, J=7.8Hz), 7.27(2H, d, J=9.0Hz), 7.24(1H, dd, J=7.8 and 4.7Hz), 6.85(1H, d, J=8.2Hz), 6.82(2H, d, J=9.0Hz), 6.70(1H, d, J=8.2Hz), 5.59(1H, d, J=5.3Hz), 5.33(1H, d, J=5.3Hz), 3.81(3H, s), 3.74-3.59(2H, m), 3.48-3.40(1H, m), 3.26-3.17(1H, m), 3.05-2.97 (2H, m), 2.96-2.87(1H, m), 2.70-2.61(1H, m), 2.37(3H, s), 2.21(3H, s).
MS($ES^+$) m/z:490 $(M + H)^+$.
Melting point: 199-200˚C.

(Example 428) 4-{4-[3-(4-cyclopropyl-thiazol-2-yl)-ureido]-phenyl}-piperazine-1-carboxylic acid [4-(2-hydroxy-ethoxy)-2-trifluoromethyl-phenyl]-amide (Compound No. 1-906)

(428a) 4-cyclopropyl-thiazol-2-ylamine

Benzyltrimethylammonium tribromide (25 g) was added to a solution of cyclopropyl methyl ketone (6.0 mL) in methanol (100 mL) at room temperature in small portions. The reaction mixture was stirred for 4.5 hours and diluted with water, followed by extraction with ethyl acetate. (3x100 mL) The combined organic layers were washed with a saturated sodium hydrogen carbonate aqueous solution and saturated brine and dried over sodium sulfate and concentrated. A solution of the residue and thiourea (4.86 g) in methanol (100 mL) was heated under reflux for 2.5 hours and concentrated. The residue was diluted with a saturated sodium hydrogen carbonate aqueous solution, followed by extraction with ethyl acetate. The organic layer was washed with saturated brine and dried over sodium sulfate and concentrated. The residue was heated under reflux in ethyl acetate and filtered and concentrated. The residue was purified by column chromatography (dichloromethane:ethyl acetate 1:1). The obtained solid was vigorously stirred in

hexane/diisopropyl ether (5:1), collected by filtration and washed with hexane and dried under reduced pressure, and 3.16 g (35%, two steps) of the title compound was obtained as a white solid.
MS(ES+) m/z: 141 (M + H)+.

(428b) 4-{4-[3-(4-cyclopropyl-thiazol-2-yl)-ureido)-phenyl}-piperazine-1-carboxylic acid tert-butyl ester

931 mg (52%) of the title compound was obtained as a white solid from 4-[4-(4-nitro-phenoxycarbonylamino)-phenyl]-piperazine-1-carboxylic acid tert-butyl ester (1.79 g) obtained in Example (224a) and 4-cyclopropyl-thiazol-2-ylamine (565 mg) obtained in Example (428a) and triethylamine (0.56 mL) in the same manner as in Example (224b).
$^1$H NMR(400MHz,DMSO-d6):δ(ppm)=10.4(1H, brs), 8.59(1H, s), 7.29(2H, d, J=9.0Hz), 6.90(2H, d, J=9.0Hz), 6.63(1H, s), 3.44(4H, t, J=4.7Hz), 3.01(4H, t, J=4.8Hz), 1.96-1.88(1H, m), 1.42(9H, s), 0.85-0.81(2H, m), 0.74-0.71(2H, m).
MS(ES+) m/z:444 (M + H)+.
Melting point: 196-198˚C.

(428c) 1-(4-cyclopropyl-thiazol-2-yl)-3-(4-piperazin-1-yl-phenyl)-urea

651 mg (95%) of the title compound was obtained as a beige solid from 4-{4-[3-(4-cyclopropyl-thiazol-2-yl)-ureido]-phenyl}-piperazine-1-carboxylic acid tert-butyl ester (887 mg) obtained in Example (428b) in the same manner as in Example (47a).
MS(ES+) m/z:343 (M + H)+.

(428d) 4-{4-[3-(4-cyclopropyl-thiazol-2-yl)-ureido]-phenyl}-piperazine-1-carboxylic acid [4-(2-hydroxy-ethoxy)-2-trifluoromethyl-phenyl]-amide

404 mg (57%) of silyl ether was obtained as a light brown solid from 1-(4-cyclopropyl-thiazol-2-yl)-3-(4-piperazin-1-yl-phenyl)-urea (343 mg) obtained in Example (428c) and 4-[2-(tert-butyl-dimethyl-silanyloxy)-ethoxy]-2-trifluoromethyl-phenylamine (335 mg) obtained in Example (209c) in the same manner as in Example 170. This silyl ether was deprotected with tetrabutylammonium fluoride in the same manner as in Example (209d) and 265 mg (79%) of the title compound was obtained as a white solid.
$^1$H NMR(400MHz,DMSO-d6):δ(ppm)=10.4(1H, s), 8.58(1H, s), 8.19(1H, s), 7.29(2H, d, J=9.0Hz), 7.29(1H, d, J=9.4Hz), 7.20(1H, dd, J=8.6 and 2.8Hz), 7.15(1H, d, J=2.8Hz), 6.94(2H, d, J=9.0Hz), 6.63(1H, s), 4.89(1H, t, J=5.5Hz), 4.05(2H, t, J=4.9Hz), 3.71(2H, q, J=5.5Hz), 3.55(4H, t, J=4.9Hz), 3.07(4H, t, J=4.9Hz), 1.96-1.89(1H, m), 1.96-1.89(2H, m), 0.75-0.71(2H, m).
MS(ES+) m/z:591 (M + H)+.
Melting point: 120-123˚C.

(Example 429) 4-{4-[3-(2-methoxy-pyridin-3-yl)-ureido]-phenyl}-piperazine-1-carboxylic acid [4-(2-hydroxy-ethoxy)-2-trifluoromethyl-phenyl]-amide (Compound No. 1-925)

538 mg (78%) of silyl ether was obtained as a white solid from 1-(2-methoxy-pyridin-3-yl)-3-(4-piperazin-1-yl-phenyl)-urea (327 mg) obtained in Example (376b) and 4-[2-(tert-butyl-dimethyl-silanyloxy)-ethoxy]-2-trifluoromethyl-phenylamine (335 mg) obtained in Example (209c) in the same manner as in Example 170. This silyl ether was deprotected with tetrabutylammonium fluoride in the same manner as in Example (209d) and 422 mg (94%) of the title compound was obtained as a white solid.
$^1$H NMR(400MHz,DMSO-d6):δ(ppm)=9.11(1H, s), 8.37(1H, dd, J=7.8 and 2.0Hz), 8.23(1H, s), 8.19(1H, s), 7.72(1H, dd, J=4.9 and 1.8Hz), 7.31(2H, d, J=9.0Hz), 7.29(1H, d, J=8.2Hz), 7.20(1H, dd, J=8.6 and 2.8Hz), 7.15(1H, d, J=2.7Hz), 6.93(2H, d, J=8.6Hz), 6.92(1H, d, J=7.8Hz), 4.89(1H, t, J=5.5Hz), 4.05(2H, t, J=4.9Hz), 3.96(3H, s), 3.72(2H, q, J=5.3Hz), 3.55(4H, t, J=4.6Hz), 3.05(4H, t, J=4.7Hz).
MS(ES+) m/z:575 (M + H)+.
Melting point: 120-123˚C.

(Example 430) 4- {4-[3-(2-methoxy-5-methyl-phenyl)-ureido]-phenyl}-3,6-dihydro-2H-pyridine-1-carboxylic acid (3-chloro-pyridin-2-yl)-amide (Compound No. 5-104)

63 mg (64%) of the title compound was obtained as a pale yellow solid from 1-(2-methoxy-5-methyl-phenyl)-3-[4-(1,2,3,6-tetrahydro-pyridin-4-yl)-phenyl]-urea (67 mg) obtained in Example (230b) and (3-chloro-pyridin-2-yl)-carbamic acid tert-butyl ester (60 mg) obtained in Example (336b) in the same manner as in Example (294d).
$^1$H NMR(400MHz,DMSO-d6):δ(ppm)=2.24(3H, s), 2.45-2.58(2H, s), 3.67(2H, brs), 3.85(3H, s), 4.14(2H, brs), 6.15(1H,

s), 6.75(1H, d, J=7.4Hz), 6.90(1H, d, J=7.5Hz), 7.22(1H, brs), 7.41(2H, d, J=7.8Hz), 7.45(2H, d, J=7.5Hz), 7.91(1H, d, J=7.0Hz), 7.99(1H, s), 8.18(1H, s), 8.33(1H, brs), 9.06(1H, s), 9.36(1H, s).
MS(APCI) m/z:492, 494 $(M + H)^+$.
Melting point: 201-204˚C.

(Example 431) 4-{4-[3-(2,3-dihydro-benzofuran-7-yl)-ureido]-phenyl}-piperazine-1-carboxylic acid (3-methyl-pyridin-2-yl)-amide (Compound No. 1-904)

(431a) 4-{4-[3-(2,3-dihydro-benzofuran-7-yl)-ureido]-phenyl}-piperazine-1-carboxylic acid tert-butyl ester

1.91 g (71 %) of the title compound was obtained as a pale beige solid from N-tert-butyloxycarbonyl-N'-(4-aminophenyl)-piperazine (1.78 g) and 2,3-dihydrobenzofuran-7-carboxylic acid (1.0 g) in the same manner as in Example (42b).
$^1$H NMR(400MHz,DMSO-d6):δ(ppm)=8.86(1H, s), 8.05(1H, s), 7.84(1H, d, J=7.4Hz), 7.30(2H, d, J=9.0Hz), 6.90(2H, d, J=9.0Hz), 6.85(1H, d, J=7.8Hz), 6.76(1H, dd, J=7.8 and 7.8Hz), 4.61(2H, t, J=8.6Hz), 3.45(4H, t, J=4.9Hz), 3.22(2H, t, J=8.6Hz), 3.00(4H, t, J=5.1Hz), 1.42(9H, s).
MS($ES^+$) m/z:439 $(M + H)^+$.

(431b) 1-(2,3-dihydro-benzofuran-7-yl)-3-(4-piperazin-1-yl-phenyl)-urea

1.47 g (quantitative yield) of the title compound was obtained as a light grey solid from 4-{4-[3-(2,3-dihydro-benzofuran-7-yl)-ureido]-phenyl}-piperazine-1-carboxylic acid tert-butyl ester (1.91 g) obtained in Example (431a) in the same manner as in Example (47a).
$^1$H NMR(400MHz,DMSO-d6):δ(ppm)=9.02(1H, s), 8.17(1H, s), 7.80(1H, d, J=7.8Hz), 7.28(2H, d, J=9.0Hz), 6.84(2H, d, J=7.5Hz), 6.83(1H, d, J=6.2Hz), 6.73(1H, dd, J=7.7 and 7.7Hz), 4.58(2H, t, J=8.8Hz), 3.53(4H, brs), 3.21(2H, t, J=8.6Hz), 2.94(4H, t, J=4.7Hz), 2.82(1H, brs).

(431c) 4-{4-[3-(2,3-dihydro-benzofuran-7-yl)-ureido]-phenyl}-piperazine-1-carboxylic acid (3-methyl-pyridin-2-yl)-amide

123 mg (52%) of the title compound was obtained as a pale yellow solid from 1-(2,3-dihydro-benzofuran-7-yl)-3-(4-piperazin-1-yl-phenyl)-urea (169 mg) obtained in Example (431b) and 2-tert-butoxycarbonylamino-3-methylpyridine (55 mg) obtained in Example (294c) in the same manner as in Example (294d).
$^1$H NMR(400MHz,DMSO-d6):δ(ppm)=8.83(2H, s), 8.16(1H, dd, J=4.7 and 1.5Hz), 8.03(1H, s), 7.82(1H, d, J=8.2Hz), 7.57(1H, d, J=6.7Hz), 7.29(2H, d, J=9.0Hz), 7.08(1H, dd, J=7.5 and 5.1Hz), 6.92(2H, d, J=9.0Hz), 6.83(1H, d, J=6.3Hz), 6.74(1H, dd, J=7.6 and 7.6Hz), 4.59(2H, t, J=8.6Hz), 3.59(4H, t, J=4.7Hz), 3.21(2H, t, J=8.8Hz), 3.05(4H, t, J=4.9Hz), 2.12(3H, s).
MS($ES^+$) m/z:473 $(M + H)^+$.
Melting point: 126-128˚C.

(Example 432) 1-{4-[3-(2-methoxy-5-methyl-phenyl)-ureido]-phenyl}-piperidine-4-carboxylic acid (3-methyl-pyridin-2-yl)-amide (Compound No. 2-147)

A solution of 1-{4-[3-(2-methoxy-5-methylphenyl)-ureido]-phenyl}-piperidine-4-carboxylic acid (767 mg) obtained in Example (196d), 2-amino-3-picoline (257 mg), HATU (O-(7-azabenzotriazol-1-yl)-N,N,N',N'-tetramethyluronium hexafluoride, 1.14 g) and triethylamine (0.42 mL) in dimethylacetamide (10 mL) was stirred at room temperature for eight days. The reaction mixture was diluted with ethyl acetate and washed with a saturated sodium hydrogen carbonate aqueous solution and concentrated. The residue was purified by column chromatography (dichloromethane: methanol 50:1 →10:1). The obtained solid was recrystallized (ethanol) and 211 mg (22%) of the title compound was obtained as a light brown solid.
$^1$H NMR(400MHz,DMSO-d6):δ(ppm)=9.97(1H, s), 9.02(1H, s), 8.23(1H, d, J=3.2Hz), 8.03(1H, s), 7.96(1H, d, J=1.5Hz), 7.64(1H, d, J=6.6Hz), 7.28(2H, d, J=9.0Hz), 7.18(1H, dd, J=7.6 and 4.9Hz), 6.89(2H, d, J=9.4Hz), 6.86(1H, d, J=8.2Hz), 6.70(1H, dd, J=8.0 and 1.4Hz), 3.82(3H, s), 3.63(2H, d, J=12.5Hz), 2.65(2H, dt, J=12.0 and 1.8Hz), 2.58-2.53(1H, m), 2.22(3H, s), 2.13(3H, s), 1.89(2H, d, J=11.0Hz), 1.82-1.72(2H, m).
MS($ES^+$) m/z:474 $(M + H)^+$.
Melting point: 200-203˚C.

(Example 433) 4- {4-[3-(2,3-dihydro-benzofuran-7-yl)-ureido]-phenyl}-piperazine-1-carboxylic acid [4-(2-hydroxy-ethoxy)-2-trifluoromethyl-phenyl]-amide (Compound No. 1-930)

489 mg (70%) of silyl ether was obtained as a pale yellow solid from 1-(2,3-dihydro-benzofuran-7-yl)-3-(4-piperazin-1-yl-phenyl)-urea (338 mg) obtained in Example (431b) and 4-[2-(tert-butyl-dimethyl-silanyloxy)-ethoxy]-2-trifluoromethyl-phenylamine (335 mg) obtained in Example (209c) in the same manner as in Example 170. This silyl ether was deprotected with tetrabutylammonium fluoride in the same manner as in Example (209d) and 408 mg (99%) of the title compound was obtained as a white solid.
$^1$H NMR(400MHz,DMSO-d6):δ(ppm)=8.84(1H, s), 8.19(1H, s), 8.03(1H, s), 7.82(1H, d, J=8.2Hz), 7.29(2H, d, J=9.0Hz), 7.29(1H, d, J=9.0Hz), 7.20(1H, dd, J=8.6 and 2.8Hz), 7.15(1H, d, J=2.7Hz), 6.91(2H, d, J=9.0Hz), 6.83(1H, d, J=6.7Hz), 6.74(1H, dd, J=7.8 and 7.8Hz), 4.89(1H, t, J=5.5Hz), 4.59(2H, t, J=8.8Hz), 4.05(2H, t, J=4.9Hz), 3.71(2H, q, J=5.3Hz), 3.55(4H, t, J=4.7Hz), 3.21(2H, t, J=8.6Hz), 3.04(4H, t, J=4.6Hz).
MS(ES$^+$) m/z:586 (M + H)$^+$.
Melting point: 218-220˚C.

(Example 434) 4- {4-[3-(3-methoxy-pyridin-4-yl)-ureido]-phenyl} -piperazine-1 - carboxylic acid (3-methyl-pyridin-2-yl)-amide (Compound No. 1-941)

(434a) 4-amino-3-methoxypyridine

1.10 g of the title compound was obtained as a yellow syrup from 3-methoxy-4-nitropyridine (1.07 g) in the same manner as in Example (373a).
MS(ES$^+$) m/z:125 (M + H)$^+$.

(434b) 4-{4-[3-(3-methoxy-pyridin-4-yl)-ureido]-phenyl}-piperazine-1-carboxylic acid tert-butyl ester

869 mg (two steps, 29%) of the title compound was obtained as a brown solid from 4-[4-(4-nitro-phenoxycarbonylamino)-phenyl]-piperazine-1-carboxylic acid tert-butyl ester (2.56 g) obtained in Example (224a) and 4-amino-3-methoxypyridine (1.10 g) obtained in Example (434a) in the same manner as in Example (224b).
$^1$H NMR(400MHz,DMSO-d6):δ(ppm)=9.15(1H, s), 9.06(1H, s), 8.13(1H, s), 8.11(1H, d, J=5.5Hz), 7.31(2H, d, J=9.0Hz), 7.06(1H, d, J=5.5Hz), 6.91(2H, d, J=9.0Hz), 3.93(3H, s), 3.44(4H, t, J=5.0Hz), 2.99(4H, t, J=5.0Hz), 1.41(9H, s)
MS(ES$^+$) m/z:428 (M + H)$^+$.

(434c) 1-(3-methoxy-pyridin-4-yl)-3-(4-piperazin-1-yl-phenyl)-urea

524 mg (81 %) of the title compound was obtained as a brown solid from 4-{4-[3-(3-methoxy-pyridin-4-yl)-ureido]-phenyl} -piperazine- I -carboxylic acid tert-butyl ester (846 mg) obtained in Example (434b) in the same manner as in Example (47a).
MS(ES$^+$) m/z:328 (M + H)$^+$.

(434d) 4-{4-[3-(3-methoxy-pyridin-4-yl)-ureido]-phenyl}-piperazine-1-carboxylic acid (3-methyl-pyridin-2-yl)-amide

20 mg (11%) of the title compound was obtained as a pale red solid from 1-(3-methoxy-pyridin-4-yl)-3-(4-piperazin-1-yl-phenyl)-urea (131 mg) obtained in Example (434c) and 2-tert-butoxycarbonylamino-3-methylpyridine (100 mg) obtained in Example (294c) in the same manner as in Example (294d).
$^1$H NMR(400MHz,DMSO-d6):δ(ppm)=9.13(1H, s), 9.08(1H, s), 8.84(1H, s), 8.16(1H, dd, J=4.8 and 1.3Hz), 8.13(1H, s), 8.10(1H, d, J=5.1Hz), 7.57(1H, dd, J=7.5, 1.3Hz), 7.31(2H, d, J=8.6Hz), 7.08(1H, dd, J=7.5 and 4.8Hz), 7.06(1H, d, J=5.1Hz), 6.93(2H, d, J=8.6Hz), 3.93(3H, s), 3.63-3.55(4H, m), 3.10-3.02(4H, m), 2.12(3H, s).
MS(ES$^+$) m/z:462 (M + H)$^+$.
Melting point: 128-130˚C.

(Example 435) 4-{2-fluoro-4-[3-(2-methoxy-phenyl)-ureido]-phenyl}-piperazine-1-carboxylic acid (3,5-difluoro-pyridin-2-yl)-amide (Compound No. 2-148)

72 mg (36%) of the title compound was obtained as a white solid from 1-(3-fluoro-4-piperazin-1-yl-phenyl)-3-(2-methoxy-phenyl)-urea (138 mg) obtained in Example (374b) and (3,5-difluoro-pyridin-2-yl)-carbamic acid tert-butyl ester (111 mg) obtained in Example (338a) in the same manner as in Example (294d).
$^1$H NMR(400MHz,$CDCl_3$):δ(ppm)=9.36(1H, s), 9.22(1H, s), 8.28(1H, d, J=2.7Hz), 8.20(1H, s), 8.12(1H, dd, J=7.8 and

1.5Hz), 7.94(1H, ddd, J=10.9, 8.6 and 2.7Hz), 7.49(1H, dd, J=14.6 and 1.9Hz), 7.05-7.00(3H, m), 6.96(1H, ddd, J=7.8, 7.6 and 1.5Hz), 6.90(1H, ddd, J=7.8, 7.6 and 1.5Hz), 3.88(3H, s), 3.61(4H, t, J=4.7Hz), 2.95(4H, t, J=4.7Hz).
MS($ES^+$) m/z:501 $(M + H)^+$.
Melting point: 179-180˚C.

(Example 436) 4-{2-fluoro-4-[3-(2-methoxy-phenyl)-ureido]-phenyl}-piperazine-1-carboxylic acid (3-cyano-pyridin-2-yl)-amide (Compound No. 2-150)

155 mg (79%) of the title compound was obtained as a white solid from 1-(3-fluoro-4-piperazin-1-yl-phenyl)-3-(2-methoxy-phenyl)-urea (138 mg) obtained in Example (374b) and (3-cyano-pyridin-2-yl)-carbamic acid tert-butyl ester (105 mg) obtained in Example (356a) in the same manner as in Example (294d).
$^1$H NMR(400MHz,DMSO-d6):δ(ppm)=9.80(1H, s), 9.33(1H, s), 8.57(1H, dd, J=4.9 and 1.7Hz), 8.22(1H, dd, J=7.7 and 1.7Hz), 8.17(1H, s), 8.09(1H, dd, J=7.8 and 1.6Hz), 7.47(1H, dd, J=14.5 and 1.6Hz), 7.27(1H, dd, J=7.7 and 4.9Hz), 7.03-6.97(3H, m), 6.93(1H, ddd, J=7.8, 7.6 and 1.6Hz), 6.88(1H, ddd, J=7.8, 7.6 and 1.6Hz) 3.87(3H, s), 3.68-3.61(4H, m), 3.00-2.93(4H, m).
MS($ES^+$) m/z:490 $(M + H)^+$.
Melting point: 210-211 ˚C.

(Example 437) 4- {4-[3-(2-methoxy-4-methyl-phenyl)-ureido]-phenyl} -piperazine-1-carboxylic acid (3,5-difluoro-pyridin-2-yl)-amide (Compound No. 1-938)

64 mg (32%) of the title compound was obtained as a white solid from 1-(2-methoxy-4-methyl-phenyl)-3-(4-piperazin-1-yl-phenyl)-urea (136 mg) obtained in Example (373c) and (3,5-difluoro-pyridin-2-yl)-carbamic acid tert-butyl ester (111 mg) obtained in Example (338a) in the same manner as in Example (294d).
$^1$H NMR(400MHz, DMSO-d6):δ(ppm)=9.23(1H, s), 9.03(1H, s), 8.28(1H, d, J=2.4Hz), 8.28(1H, s), 7.98(1H, d, J=8.2Hz), 7.94(1H, ddd, J=10.9, 8.6 and 2.4Hz), 7.32(2H, d, J=9.0Hz), 6.94(2H, d, J=9.0Hz), 6.84(1H, d, J=1.3Hz), 6.69(1H, dd, J=8.2 and 1.3Hz), 3.86(3H, s), 3.60(4H, t, J=4.8Hz), 3.07(4H, t, J=4.8Hz), 2.26(3H, s).
MS($ES^+$) m/z:497 $(M + H)^+$.
Melting point: 175-177˚C.

(Example 438) 4-{4-[3-(2-methoxy-4-methyl-phenyl)-ureido]-phenyl}-piperazine-1-carboxylic acid (3-cyano-pyridin-2-yl)-amide (Compound No. 1-936)

150 mg (77%) of the title compound was obtained as a white solid from 1-(2-methoxy-4-methyl-phenyl)-3-(4-piperazin-1-yl-phenyl)-urea (136 mg) obtained in Example (373c) and (3-cyano-pyridin-2-yl)-carbamic acid tert-butyl ester (105 mg) obtained in Example (356a) in the same manner as in Example (294d).
$^1$H NMR(400MHz, DMSO-d6):δ(ppm)=9.80(1H, s), 9.00(1H, s), 8.57(1H, d, J=4.7Hz), 8.21(1H, d, J=7.4Hz), 7.99(1H, s), 7.95(1H, d, J=8.0Hz), 7.30(2H, d, J=9.0Hz), 7.27(1H, dd, J=7.4 and 4.7Hz), 6.91(2H, d, J=9.0Hz), 6.81(1H, s), 6.67 (1H, d, J=8.0Hz), 3.84(3H, s), 3.68-3.60(4H, m), 3.12-3.03(4H, m), 2.25(3H, s).
MS($ES^+$) m/z:486 $(M + H)^+$.
Melting point: 202-203˚C.

(Example 439) 4-{4-[3-(2-methoxy-5-methyl-phenyl)-ureido]-phenyl}-piperazine-1-carboxylic acid (3-hydroxymethyl-pyridin-2-yl)-amide (Compound No. 1-934)

(439a) 2-aminonicotinic acid methyl ester

19.3 g (82%) of the title compound was obtained as a white solid from 2-amino nicotinic acid (21.4 g) in the same manner as in Example (179a).
$^1$H NMR(400MHz,$CDCl_3$):δ(ppm)= 8.20(1H, dd, J=4.7 and 1.6Hz), 8.11(1H, dd, J=7.8 and 1.5Hz), 6.62(1H, d, J=5.5Hz), 3.88(3H, s).

(439b) (2-amino-pyridin-3-yl)-methanol

2.72 g (32%) of the title compound was obtained as a white solid from 2-amino nicotinic acid methyl ester (10.3 g) obtained in Example (439a) in the same manner as in Example (382a).
$^1$H NMR(400MHz,$CDCl_3$):δ(ppm)=7.92(1H, d, J=5.1Hz), 7.28(1H, d, J=7.9Hz), 6.58(1H, dd, J=7.5 and 5.0Hz), 4.99(2H, brs), 4.60(2H, s), 3.04(1H, brs).

(439c) 3-(tert-butyl-dimethyl-silanyloxymethyl)-pyridin-2-ylamine

954 mg (80%) of the title compound was obtained as a colourless transparent syrup from (2-amino-pyridin-3-yl)-methanol (623 mg) obtained in Example (439b) in the same manner as in Example (208c).
MS($ES^+$) m/z:239 $(M + H)^+$.

(439d) [3-(tert-butyl-dimethyl-silanyloxymethyl)-pyridin-2-yl]-carbamic acid tert-butyl ester

1.12 g (83%) of the title compound was obtained as a colourless transparent syrup from 3-(tert-butyl-dimethyl-silanyloxymethyl)-pyridin-2-ylamine (954 mg) obtained in Example (439c) and di-tert-butyl dicarbonate (1.40 g) in the same manner as in Example (335b).
$^1$H NMR(400MHz,$CDCl_3$):δ(ppm)=8.36(1H, d, J=5.1Hz), 8.13(1H, brs), 7.46(1H, d, J=7.1Hz), 6.94(1H, dd, J=7.1 and 5.1Hz), 4.66(2H, s), 1.52(9H, s), 0.93(9H, s), 0.11(6H, s).

(439e) 4- {4-[3-(2-methoxy-5-methyl-phenyl)-ureido]-phenyl}-piperazine-1-carboxylic acid (3-hydroxymethyl-pyridin-2-yl)-amide

246 mg (81%) of silyl ether was obtained as a white solid from 1-(2-methoxy-5-methyl-phenyl)-3-(4-piperazin-1-yl-phenyl)-urea (170 mg) obtained in Example (47a) and [3-(tert-butyl-dimethyl-silanyloxymethyl)-pyridin-2-yl]-carbamic acid tert-butyl ester (203 mg) obtained in Example (439d) in the same manner as in Example (294d). This silyl ether was deprotected with tetrabutylammonium fluoride in the same manner as in Example (209d) and 91 mg (61 %) of the title compound was obtained as a white solid.
$^1$H NMR(400MHz,DMSO-d6):δ(ppm)=9.09(1H, s), 8.93(1H, s), 8.24(1H, dd, J=4.7 and 1.2Hz), 8.07(1H, s), 7.99(1H, d, J=1.5Hz), 7.86(1H, dd, J=7.4 and 1.2Hz), 7.34(2H, d, J=9.0Hz), 7.21(1H, dd, J=7.4 and 4.7Hz), 6.95(2H, d, J=9.0Hz), 6.88(1H, d, J=8.2Hz), 6.73(1H, dd, J=8.2 and 1.5Hz), 5.23(1H, t, J=5.7Hz), 4.36(2H, d, J=5.7Hz), 3.84(3H, s), 3.60(4H, t, J=4.7Hz), 3.07(4H, t, J=4.7Hz), 2.23(3H, s).
MS($ES^+$) m/z:491 $(M + H)^+$.
Melting point: 163-165˚C.

(Example 440) 4- {4-[3-(3-methoxy-pyridin-4-yl)-ureido]-phenyl}-piperazine-1-carboxylic acid [4-(2-hydroxy-ethoxy)-2-trifluoromethyl-phenyl]-amide (Compound No. 1-940)

200 mg (58%) of silyl ether was obtained as a grey white solid from 1-(3-methoxy-pyridin-4-yl)-3-(4-piperazin-1-yl-phenyl)-urea (164 mg) obtained in Example (434c) and 4-[2-(tert-butyl-dimethyl-silanyloxy)-ethoxy]-2-trifluoromethyl-phenylamine (167 mg) obtained in Example (209c) in the same manner as in Example 170. This silyl ether was deprotected with tetrabutylammonium fluoride in the same manner as in Example (209d) and 149 mg (89%) of the title compound was obtained as a white solid.
$^1$H NMR(400MHz,DMSO-d6):δ(ppm)=9.13(1H, s), 9.08(1H, s), 8.20(1H, s), 8.13(1H, s), 8.10(1H, d, J=5.1Hz), 7.31(2H, d, J=9.0Hz), 7.29(1H, d, J=8.2Hz), 7.20(1H, dd, J=8.9 and 2.6Hz), 7.15(1H, d, J=2.7Hz), 7.06(1H, d, J=5.9Hz), 6.93 (2H, d, J=9.0Hz), 4.90(1H, t, J=5.7Hz), 4.05(2H, t, J=4.9Hz), 3.93(3H, s), 3.72(2H, q, J=5.1Hz), 3.55(4H, t, J=4.5Hz), 3.05(4H, t, J=4.5Hz).
MS($ES^+$) m/z:575 $(M + H)^+$.
Melting point: 142-143˚C.

(Example 441) 2-[(4-{4-[3-(2-methoxy-5-methyl-phenyl)-ureido]-phenyl}-piperazine-1-carbonylamino)-nicotinic acid (Compound No. 1-927)

(441a) 2-tert-butoxycarbonylamino-nicotinic acid methyl ester

1.20 g (19%) of the title compound was obtained as a pale yellow solid from 2-aminonicotinic acid methyl ester (4.22 g) obtained in Example (439a) and di-tert-butyl dicarbonate (5.52 g) in the same manner as in Example (335b).
$^1$H NMR(400MHz,$CDCl_3$):δ(ppm)=10.2(1H, s), 8.61(1H, dd, J=5.1 and 1.9Hz), 8.27(1H, dd, J=7.8 and 1.9Hz), 6.98(1H, dd, J=7.8 and 4.7Hz), 3.94(3H, s), 1.54(9H, s).

(441b) 2-[(4-{4-[3-(2-methoxy-5-methyl-phenyl)-ureido]-phenyl}-piperazine-1-carbonylamino)-nicotinic acid

170 mg of solid was obtained from 1-(2-methoxy-5-methyl-phenyl)-3-(4-piperazin-1-yl-phenyl)-urea (205 mg) obtained in Example (47a) and 2-tert-butoxycarbonylamino-nicotinic acid methyl ester (183 mg) obtained in Example

(441a) in the same manner as in Example (294d). A mixture of this solid and potassium trimethylsilylsilanolate (195 mg) in anhydrous tetrahydrofuran (20 mL) was stirred at room temperature for 24 hours and diluted with water and filtered. The filtrate was made acidic with 1N hydrochloric acid. The deposited solid was collected by filtration and washed with acetonitrile and dried under reduced pressure, and 37 mg (12%) of the title compound was obtained as a green solid.
$^1$H NMR(400MHz,DMSO-d6):δ(ppm)=9.13(1H, s), 8.39(1H, d, J=7.1Hz), 8.32(1H, s), 8.08(1H, s), 7.98(1H, s), 7.35(2H, d, J=9.0Hz), 7.17(1H, dd, J=7.6 and 5.3Hz), 6.98(2H, d, J=8.6Hz), 6.89(2H, d, J=8.6Hz), 6.73(1H, dd, J=8.2 and 1.6Hz), 3.84(3H, s), 3.65(4H, brs), 3.14(4H, brs), 2.23(3H, s).
MS($ES^+$) m/z:505 $(M + H)^+$.
Melting point: 165-168˚C.

(Example 442) 4-{4-[3-(2-methoxy-5-methyl-phenyl)-ureido]-phenyl}-3,6-dihydro-2H-pyridine-1-carboxylic acid (3-hydroxymethyl-pyridin-2-yl)-amide (Compound No. 5-110)

Silyl ether was obtained from 1-(2-methoxy-5-methyl-phenyl)-3-[4-(1,2,3,6-tetrahydro-pyridin-4-yl)-phenyl]-urea (67 mg) obtained in Example (230b) and [3-(tert-butyl-dimethyl-silanyloxymethyl)-pyridin-2-yl]-carbamic acid tert-butyl ester (88 mg) obtained in Example (439d) in the same manner as in Example (294d). This silyl ether was deprotected with tetrabutylammonium fluoride in the same manner as in Example (209d) and 9.2 mg (10%) of the title compound was obtained as a white solid.
$^1$H NMR(400MHz,DMSO-d6):δ(ppm)=2.23(3H, s), 2.55-2.46(2H, m), 3.66(2H, t, J=5.3Hz), 3.84(3H, s), 4.13(2H, brs), 4.35(2H, brs), 5.24(1H, brs), 6.15(1H, s), 6.75(1H, d, J=8.6Hz), 6.90(1H, d, J=8.3Hz), 7.20(1H, dd, J=7.2 and 4.5Hz), 7.41(2H, d, J=8.2Hz), 7.46(2H, d, J=8.6Hz), 7.86(1H, d, J=7.5Hz), 7.99(1H, s), 8.19(1H, s), 8.24(1H, d, J=5.1Hz), 8.84 (1H, s), 9.39(1H, s).
MS(APCI) m/z:488 $(M + H)^+$.
Melting point: 103-108˚C.

(Example 443) 4-{4-[3-(2-methoxy-5-methyl-phenyl)-ureido]-phenyl}-piperidine-1-carboxylic acid (3-hydroxymethyl-pyridin-2-yl)-amide (Compound No. 4-110)

Silyl ether was obtained from 1-(2-methoxy-5-methyl-phenyl)-3-(4-piperidin-4-yl-phenyl)-urea (68 mg) obtained in Example (228e) and [3-(tert-butyl-dimethyl-silanyloxymethyl)-pyridin-2-yl]-carbamic acid tert-butyl ester (88 mg) obtained in Example (439d) in the same manner as in Example (294d). This silyl ether was deprotected with tetrabutylammonium fluoride in the same manner as in Example (209d) and 5 mg (5%) of the title compound was obtained as a white solid.
$^1$H NMR(400MHz,DMSO-d6):δ(ppm)=1.61-1.47(2H, m), 1.82-1.74(2H, m), 2.23(3H, s), 2.74-2.66(1H, m), 2.90(2H, t, J=12.7Hz), 3.84(3H, s), 4.22(2H, brd, J=12.9Hz), 4.38(2H, d, J=5.1Hz), 5.25(1H, t, J=5.5Hz), 6.74(1H, dd, J=8.4 and 1.4Hz), 6.89(1H, d, J=8.2Hz), 7.22-7.15(3H, m), 7.40(2H, d, J=8.6Hz), 7.86(1H, brd, J=7.5Hz), 7.99(1H, d, J=1.6Hz), 8.15(1H, s), 8.23(1H, dd, J=4.8 and 1.8Hz), 8.81(1H, s), 9.26(1H, s).
MS(APCI) m/z:490 $(M + H)^+$.
Melting point: 105-110˚C.

(Example 444) 4-{4-[3-(2-methoxy-5-methyl-phenyl)-ureido]-phenyl}-3,6-dihydro-2H-pyridine-1-carboxylic acid (3-fluoro-pyridin-2-yl)-amide (Compound No. 5-113)

48 mg (51%) of the title compound was obtained as a yellow solid from 1-(2-methoxy-5-methyl-phenyl)-3-[4-(1,2,3,6-tetrahydro-pyridin-4-yl)-phenyl]-urea (67 mg) obtained in Example (230b) and (3-fluoro-pyridin-2-yl)-carbamic acid tert-butyl ester (55 mg) obtained in Example (337b) in the same manner as in Example (294d). $^1$H NMR (400MHz,DMSO-d6):δ(ppm)=2.24(3H, s), 2.56-2.48(2H, m), 3.68(2H, t, J=5.7Hz), 3.85(3H, s), 4.15(2H, brs), 6.15(1H, brs), 6.75(1H, dd, J=8.2 and 1.6Hz), 6.90(1H, d, J=8.6Hz), 7.24(1H, ddd, J=8.2, 4.3 and 3.9Hz), 7.41(2H, d, J=8.6Hz), 7.46(2H, d, J=9.0Hz), 7.68(1H, dd, J=10.2 and 8.6Hz), 8.00(1H, d, J=2.0Hz), 8.20-8.16(2H, m), 9.14(1H, s), 9.36(1H, s).
MS(APCI) m/z:476 $(M + H)^+$.
Melting point: 193-196˚C.

(Example 445) 4-{4-[3-(2-methoxy-5-methyl-phenyl)-ureido]-phenyl}-piperidine-1-carboxylic acid (3-fluoro-pyridin-2-yl)-amide (Compound No. 4-113)

39 mg (41%) of the title compound was obtained as a light brown solid from 1-(2-methoxy-5-methyl-phenyl)-3-(4-piperidin-4-yl-phenyl)-urea (68 mg) obtained in Example (228e) and (3-fluoro-pyridin-2-yl)-carbamic acid tert-butyl ester (55 mg) obtained in Example (337b) in the same manner as in Example (294d).

$^1H$ NMR(400MHz,DMSO-d6):δ(ppm)=1.62-1.48(2H, m), 1.82-1.74(2H, m), 2.23(3H, s), 2.76-2.64(1H, m), 2.91(2H, t, J=12.3Hz), 3.84(3H, s), 4.23(2H, brd, J=12.9Hz), 6.74(1H, dd, J=8.0 and 1.4Hz), 6.89(1H, d, J=8.2Hz), 7.17(2H, d, J=8.6Hz), 7.22(1H, ddd, J=8.2, 4.7 and 3.5Hz), 7.40(2H, d, J=8.6Hz), 7.67(1H, ddd, J=10.1, 8.2 and 1.2Hz), 7.99(1H, d, J=2.0Hz), 8.14(1H, s), 8.17(1H, d, J=4.7Hz), 9.09(1H, s), 9.25(1H, s).
MS(APCI) m/z:478 $(M + H)^+$.
Melting point: > 250˚C.

(Example 446) 4-{4-[3-(2,3-dihydro-benzofuran-7-yl)-ureido]-phenyl}-piperazine-1-carboxylic acid (3-chloro-pyridin-2-yl)-amide (Compound No. 1-928)

123 mg (47%) of the title compound was obtained as a pale yellow solid from 1-(2,3-dihydro-benzofuran-7-yl)-3-(4-piperazin-1-yl-phenyl)-urea (203 mg) obtained in Example (431b) and (3-chloro-pyridin-2-yl)-carbamic acid tert-butyl ester (167 mg) obtained in Example (336b) in the same manner as in Example (294d).
$^1H$ NMR(400MHz,DMSO-d6):δ(ppm)=9.14(1H, s), 8.87(1H, s), 8.32(1H, d, J=4.3Hz), 8.06(1H, s), 7.91(1H, dd, J=8.2 and 1.2Hz), 7.85(1H, d, J=7.9Hz), 7.31(2H, d, J=9.0Hz), 7.22(1H, dd, J=8.1 and 4.9Hz), 6.94(2H, d, J=9.0Hz), 6.85(1H, d, J=7.0Hz), 6.76(1H, dd, J=7.6 and 7.7Hz), 4.61(2H, t, J=8. 8Hz), 3.60(4H, t, J=4.1Hz), 3.22(2H, t, J=8.6Hz), 3.07(4H, t, J=4.9Hz).
MS($ES^+$) m/z:493 $(M + H)^+$.
Melting point: 109-111˚C.

(Example 447) 4-{2-fluoro-4-[3-(2-fluoro-phenyl)-ureido]-phenyl}-piperazine-1-carboxylic acid [4-(2-hydroxy-ethoxy)-2-trifluoromethyl-phenyl]-amide (Compound No. 2-123)

(447a) 4-{2-fluoro-4-[3-(2-fluoro-phenyl)-ureido]-phenyl}-piperazine-1-carboxylic acid tert-butyl ester

1.17 g (90%) of the title compound was obtained as a pale grey solid from 4-(4-amino-2-fluoro-phenyl)-piperazine-1-carboxylic acid tert-butyl ester (866 mg) obtained in Example (253b) and 2-fluorophenyl isocyanate (0.41 mL) in the same manner as in Example 1.
$^1H$ NMR(400MHz,DMSO-d6):δ(ppm)=9.07(1H, s), 8.51(1H, s), 8.10(1H, ddd, J=8.2, 7.8 and 1.5Hz), 7.44(1H, dd, J=15 and 2Hz), 7.21(1H, ddd, J=11, 8, and 2Hz), 7.12(1H, dd, J=7.8 and 7.8Hz), 7.03-6.96(2H, m), 3.45(4H, t, J=4.5Hz), 2.88 (4H, t, J=4.9Hz), 1.42(9H, s).

(447b) 1-(3-fluoro-4-piperazin-1-yl-phenyl)-3-(2-fluoro-phenyl)-urea

845 mg (quantitative yield) of the title compound was obtained as a white solid from 4-{2-fluoro-4-[3-(2-fluoro-phenyl)-ureido]-phenyl}-piperazine-1-carboxylic acid tert-butyl ester (1.10 g) obtained in Example (447a) in the same manner as in Example (47a).
$^1H$ NMR(400MHz,DMSO-d6):δ(ppm)=9.24(1H, brs), 8.84(1H, brs), 8.58(1H, s), 8.09(1H, ddd, J=8, 8 and 2Hz), 7.47 (1H, d, J=13.3Hz), 7.24-7.19(1H, m), 7.12(1H, dd, J=7.8 and 7.8Hz), 7.08-6.97(3H, m), 3.25(4H, brs), 3.15-3.13(4H, m).

(447c) 4-{2-fluoro-4-[3-(2-fluoro-phenyl)-ureido]-phenyl}-piperazine-1-carboxylic acid [4-(2-hydroxy-ethoxy)-2-trifluoromethyl-phenyl]-amide

148 mg (31%) of silyl ether was obtained from 1-(3-fluoro-4-piperazin-1-yl-phenyl)-3-(2-fluoro-phenyl)-urea (280 mg) obtained in Example (447b) and 4-[2-(tert-butyl-dimethyl-silanyloxy)-ethoxy]-2-trifluoromethyl-phenylamine (168 mg) obtained in Example (209c) in the same manner as in Example 170. This silyl ether (136 mg) was deprotected with tetrabutylammonium fluoride in the same manner as in Example (209d) and 66 mg (58%) of the title compound was obtained as a white solid.
$^1H$ NMR(500MHz,DMSO-d6):δ(ppm)=9.09(1H, s), 8.53(1H, d, J=2 Hz), 8.21(1H, s), 8.12(1H, ddd, J=8, 8, and 2 Hz), 7.47(1H, d, J=13Hz), 7.30(1H, d, J=9Hz), 7.26-7.21(2H, m), 7.19-7.16(1H, m), 7.14(1H, dd, J=8 and 8Hz), 7.06-6.99 (3H, m), 4.93(1H, t, J=5Hz), 4.07(2H, t, J=5 Hz), 3.73(2H, dt, J=5 and 5Hz), 3.56(4H, t, J=5Hz), 2.94(4H, t, J=5Hz).
MS($ES^+$) m/z:580 $(M + H)^+$.
Melting point: 200-202˚C.

(Test Example 1)

(1) Preparation of DGAT1

According to a reported method, a microsome fraction which highly exhibited mouse DGAT1 was prepared as follows (Cases, S. et al., Proc. Natl. Acad. Sci. USA in 1998 Vol. 95, p.13018-13023). The cDNA which encodes mouse DGAT1 was cloned from a mouse cDNA library by polymerase chain reaction (hereinafter referred to as "PCR") and introduced into an insect cell virus vector (baculovirus expression system, Invitrogen). After insect cells (High Five, Sf9, Sf21, etc., Invitrogen) were infected with the virus, the cells were collected in buffer A [0.1M sucrose, 50 mM KCl, 40mM potassium phosphate (pH 7.4), 30mM EDTA, 1% Protease inhibitor cocktail (SIGMA, P-8340)] and crushed with a Polytron homogenizer. The supernatant obtained by subjecting the homogenate to low speed centrifugal processing (10,000xg, 30 minutes) was further subjected to higher-speed centrifugal processing (100,000xg, 60 minutes) and the precipitate was resuspended in buffer A as the microsome fraction and used as DGAT1 enzyme. The DGAT1 enzyme was divided into small volumes and stored at -80˚C.

(2) DGAT1 inhibitory effect test

The reaction liquid of the following composition [175 mM Tris-HCl (pH 8.0), 8 mM $MgCl_2$, 1 mg/ml BSA, 0.5 mM 1,2-dioleoyl-sn-glycerol (EtOH solution of 10-fold concentration, 10% added), 30 μM[$^{14}$C]-oleoyl-CoA (about 50 mCi/mmol), 0.5% triton X-100, DGAT1 enzyme (10 μg) obtained in Test Example 1(1), test compound or vehicle (DMSO/MeOH, 7:3 solution, 5% added), total volume 50 μl] was incubated at room temperature (23˚C) for 30 minutes. A reaction terminating solution (70 μl) consisting of isopropanol/1-heptane/water (80:20:2, v/v/v) was added to the reaction solution and stirred, and subsequently stirred with water (30 μl) and 1-heptane (100 μl). The 1-heptane layer (50 μl) was spotted onto a TLC plate and developed with a developing solvent consisting of 1-hexane/diethyl ether/acetic acid (85:15:1, v/v/v). Radioactivity of triglyceride fraction was quantified by BAS2000 bio image analyzer (Fuji Film) and the inhibitory effect of the test compounds was calculated by the following formula through comparison with control. Here, radioactivity at no reaction (0 minutes incubation) was assumed as background.

$$\text{Inhibitory ratio} = 100 - [(\text{Radioactivity when test compound was added})-(\text{Background})]/[(\text{Radioactivity by control})-(\text{Background})]\times 100$$

Compounds of Examples 22, 33, 35, 37, 39, 40, 46, 53, 54, 57, 58, 59, 60, 61, 62, 64, 65, 66, 73, 75, 76, 77, 78, 79, 80, 81, 82, 83, 84, 85, 86, 87, 88, 89, 90, 91, 92, 93, 94, 96, 97, 98, 99, 100, 101, 102, 103, 104, 105, 106, 107, 109, 110, 111, 112, 113, 114, 115, 116, 117, 118, 119, 120, 121, 122, 123, 124, 125, 126, 127, 128, 129, 130, 131, 132, 133, 134, 135, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 177, 178, 179, 180, 181, 183, 184, 185, 186, 187, 188, 192, 197, 198, 199, 200, 201, 202, 203, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 428, 429, 430, 431, 432, 433, 435, 436, 437, 438, 444, 445, 446, and 447 showed an inhibitory ratio of 50% or more when the concentration of the test compound was 0.1 μg/ml.

Tests for DGAT inhibitory effect are not limited to the method mentioned above and, for example, microsomes prepared from small intestine, adipose tissue or liver of animals such as rat, mice may be used as DGAT enzyme. In addition, microsomes prepared from cultured cells (3T3-L1 fat cell, primary culture fat cell, Caco2 cell, HepG2 cell) or cultured cells in which DGAT is highly exhibited can be used as DGAT enzyme. Furthermore, flush plates (PerkinElmer), which omit an extraction operation, can be used to evaluate a large number of test compounds efficiently in a short time.

(Test Example 2)

A mixture of compound (10 or 30 mg/kg) and emulsion containing 20% neutral fat (Intralipid 20%: Terumo Corporation) was orally administered to a male C57BL/6N mouse (7-15 weeks old, 17-35 g in weight, Charles River Laboratories Japan, Inc.) (0.3 mL/mouse). One hour after the administration, the abdomen was opened under inhalation anesthesia by isoflurane (Foren: Abbott Japan Co., LTD.) and 1-10 μl of small intestine lymph fluid was collected from small intestine lymphatic vessel. Triglyceride concentration in the lymph fluid was measured with a commercial kit (triglyceride E test Wako, Wako Pure Chemical Industries, Ltd.) and the decrease in triglyceride concentration was calculated against a 20% emulsion administered group (control group) and taken as neutral fat absorption inhibitory effect. Compounds of Examples 59, 62, 64, 73, 77, 79, 82, 85, 87, 89, 90, 91, 93, 94, 100, 103, 106, 109, 110, 111, 112, 113, 115, 117, 120, 121, 122, 125, 132, 133, 134, 135, 138, 139, 140, 141, 142, 144, 162, 166, 168, 169, 173, 177, 179, 180, 181, 184, 185, 187, 198, 202, 208, 209, 210, 211, 212, 213, 214, 215, 216, 217, 218, 220, 221, 222, 223, 224, 225, 226, 227, 229, 231, 232, 233, 234, 235, 236, 238, 248, 249, 250, 251, 253, 254, 256, 257, 268, 269, 271, 272, 275, 276, 277, 279, 280, 281, 282, 283, 293, 294, 298, 299, 300, 301, 302, 304, 305, 306, 308, 310, 324, 356, 377, 379, 381, 382, 384, 385, 386, 387, 388, 389, 392, 394, 396, 397, 398, 401, 402, 403, 404, 405, 407, 408, 409, 412, 413, 415, 416, 419, and 447 showed a neutral fat absorption inhibitory effect of 60% or more.

As is clear from the results mentioned above, the compound of the present invention has an excellent DGAT inhibitory effect and is useful as a therapeutic or prophylactic agent for adiposity, obesity, hyperlipidemia, hypertriglyceridemia, lipidosis, diabetes, arteriosclerosis, or hyperlipidemia, hypertriglyceridemia, lipidosis, diabetes, arteriosclerosis or hypertension caused by adiposity.

Preparation Example 1: Capsule

50 mg of compound of Example 132 or 135
Lactose 128 mg
Corn starch 70 mg
Magnesium stearate 2 mg
250 mg

Powder of the above formulation was mixed and passed through a sieve of 60 mesh, and then this powder was encapsulated in a No. 3 gelatin capsule of 250 mg to form a capsule.

Preparation Example 2: Tablet

50 mg of compound of Example 132 or 135
Lactose 126 mg
Corn starch 23 mg
Magnesium stearate 1 mg
200 mg

Powder of the above formulation was mixed and wet granulated using a corn starch sizing agent and dried, and then a 200 mg tablet was made by means of a tabletting machine. This tablet can be sugarcoated if necessary.

Industrial Applicability

The compound represented by the general formula (I) or a pharmacologically acceptable salt thereof according to the present invention has an excellent DGAT inhibitory effect and is useful as a medicine for prevention and/or treatment of the following diseases: a disease selected from the group consisting of adiposity, obesity, hyperlipidemia, hypertriglyceridemia, lipidosis, insulin resistance syndrome, impaired glucose tolerance, diabetes, diabetic complications (including diabetic peripheral neuropathy, diabetic nephropathy, diabetic retinopathy and diabetic macroangiopathy), cataract, gestational diabetes mellitus, polycystic ovary syndrome, arteriosclerosis (including arteriosclerosis caused by the diseases described above and below), atherosclerosis and diabetic atherosclerosis, or the following diseases caused by adiposity: a disease selected from the group consisting of hyperlipidemia, hypertriglyceridemia, lipidosis, insulin resistance syndrome, impaired glucose tolerance, diabetes, diabetic complications (including diabetic peripheral neuropathy, diabetic nephropathy, diabetic retinopathy and diabetic macroangiopathy), cataract, gestational diabetes mellitus, polycystic ovary syndrome, arteriosclerosis (including arteriosclerosis caused by the diseases described above and below), atherosclerosis, diabetic atherosclerosis, hypertension, cerebrovascular disorders, coronary artery diseases, fatty liver, respiratory abnormality, lower back pain, knee osteoarthritis, gout and cholecystitis in a warm-blooded animal (preferably a mammal including a human adult). Further, the novel compound represented by the general formula (I) or a pharmacologically acceptable salt thereof provided by the present invention has an excellent DGAT inhibitory effect

and is useful as an active ingredient of a medicine for prevention and/or treatment of the above diseases in a warm-blooded animal (preferably a mammal including a human adult). A preferable disease is a disease selected from adiposity, obesity, hyperlipemia, hypertriglyceridemia, lipidosis, diabetes and arteriosclerosis, or the following diseases caused by adiposity: a disease selected from the group consisting of hyperlipemia, hypertriglyceridemia, lipidosis, diabetes, arteriosclerosis, hypertension, cerebrovascular disorders and coronary artery diseases, more preferably adiposity, obesity or the following diseases caused by adiposity: hyperlipemia, hypertriglyceridemia, diabetes or arteriosclerosis, further preferably adiposity or obesity. Preferably, the compound represented by the general formula (I) or a pharmacologically acceptable salt thereof according to the present invention can be used as a medicine for treating the above diseases.

## Claims

**1.** A urea derivative having the general formula (I):

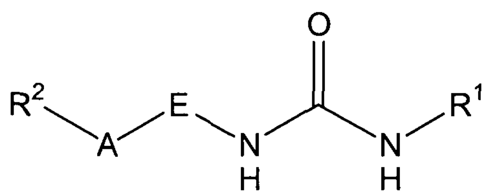

(I)

[wherein $R^1$ represents a $C_1$-$C_{10}$ alkyl group, a $C_3$-$C_6$ cycloalkyl group, a $C_6$-$C_{10}$ aryl group which may be independently mono- to pentasubstituted by a group selected from Substituent Group a or a heterocyclic group which may be independently mono- to trisubstituted by a group selected from Substituent Group a,

$R^2$ represents a hydrogen atom, a $C_1$-$C_6$ alkyl group, a $C_6$-$C_{10}$ aryl group which may be independently mono- to pentasubstituted by a group selected from Substituent Group a, a heterocyclic group which may be independently mono- to trisubstituted by a group selected from Substituent Group a, a $C_7$-$C_{16}$ aralkyl group which may be independently mono- to trisubstituted by a group selected from Substituent Group a, $C_3$-$C_6$ cycloalkyl group which may be monosubstituted by a $C_1$-$C_6$ alkyl group, a $C_7$-$C_{10}$ bicycloalkyl group or a tetralyl group,

E is a group having the formula (II), the formula (III), the formula (XXXIX) or the formula (XL):

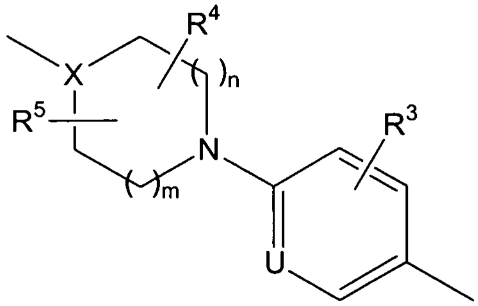

(II)

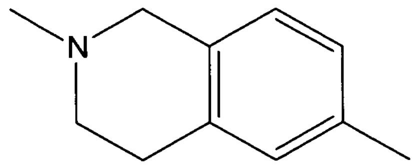

(III)

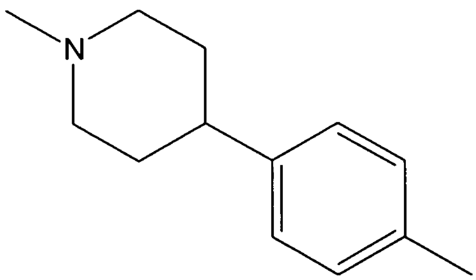

(XXXIX)

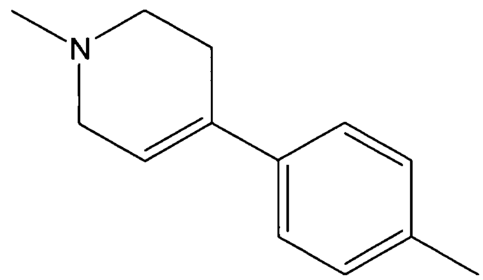

(XL)

(wherein $R^3$ represents a hydrogen atom, a $C_1$-$C_6$ alkyl group, a halogen atom or a cyano group, $R^4$ represents a hydrogen atom or a $C_1$-$C_6$ alkyl group, $R^5$ represents a hydrogen atom or a $C_1$-$C_6$ alkyl group, X and U, which are the same or different, represent a group represented by the formula CH or a nitrogen atom, m and n, which are the same or different, represent an integer of 1 or 2; provided that the X side of the group having the formula (II) and the nitrogen atom side of the groups having the formula (III), the formula (XXXIX) and the formula (XL) are each bonded to A in the compound represented by the general formula (I), and the carbon atom side of the aromatic ring of the groups having the formula (II), the formula (III), the formula (XXXIX) and the formula (XL) is bonded to the nitrogen atom in the compound represented by the general formula (I); and $R^4$ and $R^5$ may be bonded to different carbon atoms or the same carbon atom),

A represents a single bond, a group represented by the formula -O-C(=O)-, a group represented by the formula -O-C(=S)-, a group represented by the formula - NH-C(=O)-, a group represented by the formula -NH-C(=S)-, a carbonyl group, a thiocarbonyl group or a group represented by the formula -CH(OH)-C(=O)-(provided that the oxygen atom side, the nitrogen atom side and the side of the group represented by the formula CH(OH) are each bonded to $R^2$ in the compound represented by the general formula (I), and the carbonyl group side and the thiocarbonyl group side are each bonded to E in the compound represented by the general formula (I)),

excluding the case where $R^2$ represents a hydrogen atom and A represents a single bond,

Substituent Group a means the group consisting of a halogen atom, a $C_1$-$C_{10}$ alkyl group, a $C_1$-$C_6$ halogenated alkyl group, a $C_1$-$C_6$ hydroxyalkyl group, a $C_1$-$C_6$ alkyl group monosubstituted by -P(=O)(O-$C_1$-$C_6$ alkyl)$_2$, a $C_3$-$C_6$ cycloalkyl group, a $C_1$-$C_{10}$ alkoxy group, a $C_1$-$C_6$ halogenated alkoxy group, a $C_1$-$C_6$ alkoxy group mono- or disubstituted by a hydroxyl group, a $C_1$-$C_6$ alkoxy group monosubstituted by a $C_3$-$C_6$ cycloalkyl group, a $C_2$-$C_6$ alkenyloxy group, a $C_2$-$C_6$ alkynyloxy group, a ($C_1$-$C_6$ alkoxy)-($C_1$-$C_6$ alkyl) group, a $C_1$-$C_6$ alkylthio group, a carboxyl group, a $C_2$-$C_7$ alkoxycarbonyl group, an amino group, a mono-$C_2$-$C_7$ alkylcarbonylamino group, a mono-$C_1$-$C_6$ alkylsulfonylamino group, a mono-$C_1$-$C_6$ alkylamino group, a di-($C_1$-$C_6$ alkyl)amino group, a N-($C_1$-$C_6$ alkyl)-N-($C_1$-$C_6$ hydroxyalkyl)amino group, a di-($C_1$-$C_6$ hydroxyalkyl)amino group, a cyano group, a nitro group, a nitrogen-containing heterocyclic group which may be monosubstituted by a hydroxyl group, a $C_6$-$C_{10}$ aryl group which may be independently mono- to trisubstituted by a group selected from Substituent Group b, a $C_6$-$C_{10}$ aryloxy group which may be independently mono- to trisubstituted by a group selected from Substituent Group b, a hydroxyl group, a $C_1$-$C_6$ alkyl group disubstituted by hydroxyl groups, a $C_1$-$C_6$ alkyl group monosubstituted by -P(=O)(OH)(O-$C_1$-$C_6$ alkyl), a $C_1$-$C_6$ alkyl group monosubstituted by a carboxyl group, a $C_1$-$C_6$ alkyl group monosubstituted by a $C_2$-$C_7$ alkoxycarbonyl group and a 2,2-dimethyl-1,3-dioxa-4-cyclopentyl group, and

Substituent Group b means the group consisting of a halogen atom, a $C_1$-$C_6$ alkyl group and a $C_1$-$C_6$ halogenated alkyl group], or a pharmacologically acceptable salt thereof.

**2.** The urea derivative or the pharmacologically acceptable salt thereof according to claim 1, wherein $R^1$ represents a phenyl group independently mono- to trisubstituted by a group selected from the group consisting of a halogen atom, a $C_1$-$C_6$ alkyl group, a $C_1$-$C_6$ halogenated alkyl group, a $C_1$-$C_6$ alkoxy group, a carboxyl group, an amino group, a mono-$C_2$-$C_7$ alkylcarbonylamino group, a di-($C_1$-$C_6$ alkyl)amino group, a cyano group and a nitro group or a thiazolyl group which may be independently mono- or disubstituted by a $C_1$-$C_6$ alkyl group.

**3.** The urea derivative or the pharmacologically acceptable salt thereof according to claim 1, wherein $R^1$ is a phenyl group independently mono- to trisubstituted by a group selected from the group consisting of a halogen atom, a $C_1$-$C_6$ alkyl group and a $C_1$-$C_6$ alkoxy group or a thiazolyl group independently mono- or disubstituted by a $C_1$-$C_6$ alkyl group.

**4.** The urea derivative or the pharmacologically acceptable salt thereof according to claim 1, wherein $R^1$ is a phenyl group substituted by a group selected from the group consisting of a fluorine atom, a chlorine atom, a methyl group, a methoxy group and an ethoxy group at the 2- or 3-position; a phenyl group independently disubstituted by a group selected from the group consisting of a fluorine atom, a chlorine atom, a methyl group, a methoxy group and an ethoxy group at the 2, 3 or 5-position; or a 5-methyl-2-thiazolyl group.

**5.** The urea derivative or the pharmacologically acceptable salt thereof according to claim 1, wherein $R^1$ is a 2-fluorophenyl group, a 2-ethoxyphenyl group, a 3-ethoxyphenyl group, a 5-fluoro-2-methoxyphenyl group, a 2-ethoxy-5-fluorophenyl group, a 5-chloro-2-methoxyphenyl group, a 5-chloro-2-ethoxyphenyl group, a 2-methoxy-5-methylphenyl group or a 3,5-dimethoxymethylphenyl group.

**6.** The urea derivative or the pharmacologically acceptable salt thereof according to claim 1, wherein $R^1$ is a $C_1$-$C_6$ alkyl group; or a phenyl group which may be independently mono- to trisubstituted by a group selected from the group consisting of a halogen atom, a $C_1$-$C_6$ alkyl group, a $C_1$-$C_2$ halogenated alkyl group, a $C_1$-$C_3$ hydroxyalkyl group, a $C_1$-$C_6$ alkoxy group, a $C_1$-$C_2$ halogenated alkoxy group, a carboxyl group, an amino group, a mono-$C_2$-$C_3$ alkylcarbonylamino group, a di-$C_1$-$C_2$ alkylamino group, a cyano group, a nitro group and a phenyloxy group.

**7.** The urea derivative or the pharmacologically acceptable salt thereof according to claim 1, wherein $R^1$ is a $C_1$-$C_6$ alkyl group; or a phenyl group which may be independently mono- to trisubstituted by a group selected from the group consisting of a fluorine atom, a chlorine atom, a bromine atom, a methyl group, an ethyl group, a propyl group, an isopropyl group, a butyl group, an isobutyl group, a s-butyl group, a t-butyl group, a trifluoromethyl group, a methoxy group, an ethoxy group, a propoxy group, an isopropoxy group, a butoxy group, a carboxyl group, an amino group, a methylcarbonylamino group, a dimethylamino group, a cyano group and a nitro group.

**8.** The urea derivative or the pharmacologically acceptable salt thereof according to claim 1, wherein $R^1$ is a hexyl group; a phenyl group substituted by a fluorine atom, a chlorine atom, a methyl group, an ethyl group, a trifluoromethyl group, a methoxy group or an ethoxy group at the 2- and 5-positions; a phenyl group substituted by a butoxy group or a dimethylamino group at the 4-position; or a phenyl group substituted by a fluorine atom, a chlorine atom, a methyl group, an ethyl group, a methoxy group or an ethoxy group at the 2-position.

**9.** The urea derivative or the pharmacologically acceptable salt thereof according to claim 1, wherein $R^1$ is a 5-fluoro-2-methoxyphenyl, 2-ethoxy-5-fluorophenyl group, a 5-chloro-2-methoxyphenyl group, a 5-chloro-2-ethoxyphenyl group, a 2-methoxy-5-methylphenyl group, a 2-ethoxy-5-methylphenyl group, a 4-butoxyphenyl group, a 4-dimethylaminophenyl group, a 2-fluorophenyl group or a 2-ethoxyphenyl group.

**10.** The urea derivative or the pharmacologically acceptable salt thereof according to any one of claims 1 to 9, wherein $R^2$ is a phenyl or pyridyl group which may be independently mono- to trisubstituted by a group selected from the group consisting of a halogen atom, a $C_1$-$C_6$ alkyl group, a $C_1$-$C_6$ halogenated alkyl group, a $C_1$-$C_6$ hydroxyalkyl group, a $C_1$-$C_6$ alkoxy group, a $C_1$-$C_6$ alkoxy group mono- or disubstituted by a hydroxyl group, a carboxyl group, an amino group, a mono-$C_2$-$C_7$ alkylcarbonylamino group, a cyano group, a nitro group, a hydroxyl group, a $C_1$-$C_6$ alkyl group monosubstituted by a carboxyl group and a $C_1$-$C_6$ alkyl group monosubstituted by a $C_2$-$C_7$ alkoxycarbonyl group.

**11.** The urea derivative or the pharmacologically acceptable salt thereof according to any one of claims 1 to 9, wherein $R^2$ is a phenyl or pyridyl group which may be independently mono- to trisubstituted by a group selected from the group consisting of a halogen atom, a $C_1$-$C_6$ alkyl group, a $C_1$-$C_6$ halogenated alkyl group, a $C_1$-$C_6$ alkoxy group mono- or disubstituted by a hydroxyl group and a cyano group.

**12.** The urea derivative or the pharmacologically acceptable salt thereof according to any one of claims 1 to 9, wherein $R^2$ is a phenyl group substituted by a group selected from the group consisting of a fluorine atom, a chlorine atom, a methyl group and a trifluoromethyl group at the 2-position and substituted by a group selected from the group consisting of a 2-hydroxyethoxy group and a 2,3-dihydroxypropoxy group at the 4- or 5-position; a 3-methyl-2-pyridyl group; a 3-cyano-2-pyridyl group; or a 3,5-difluoro-2-pyridyl group.

**13.** The urea derivative or the pharmacologically acceptable salt thereof according to any one of claims 1 to 9, wherein $R^2$ is a 2-chloro-4-(2-hydroxyethoxy)phenyl group, a 2-chloro-5-(2-hydroxyethoxy)phenyl group, a 4-(2-hydroxyethoxy)-2-trifluoromethylphenyl group, a 2-chloro-4-(2,3-dihydroxypropoxy)phenyl group, a 4-(2,3-dihydroxypropoxy)-2-trifluoromethylphenyl group, a 2-chloro-5-(2,3-dihydroxypropoxy)phenyl group or a 3-methyl-2-pyridyl group.

**14.** The urea derivative or the pharmacologically acceptable salt thereof according to any one of claims 1 to 9, wherein $R^2$ is a $C_1$-$C_4$ alkyl group; or a phenyl or benzyl group which may be independently mono- to trisubstituted by a group selected from the group consisting of a halogen atom, a $C_1$-$C_6$ alkyl group, a $C_1$-$C_2$ halogenated alkyl group, a $C_1$-$C_3$ hydroxyalkyl group, a $C_1$-$C_6$ alkoxy group, a $C_1$-$C_2$ halogenated alkoxy group, a carboxyl group, an amino group, a mono-$C_2$-$C_3$ alkylcarbonylamino group, a di-$C_1$-$C_2$ alkylamino group, a cyano group, a nitro group and a phenyloxy group.

**15.** The urea derivative or the pharmacologically acceptable salt thereof according to any one of claims 1 to 9, wherein $R^2$ is a $C_1$-$C_4$ alkyl group; or a phenyl group which may be independently mono- to trisubstituted by a group selected from the group consisting of a fluorine atom, a chlorine atom, a bromine atom, a methyl group, an ethyl group, a propyl group, an isopropyl group, a butyl group, an isobutyl group, a s-butyl group, a t-butyl group, a trifluoromethyl group, a hydroxymethyl group, a methoxy group, an ethoxy group, a propoxy group, an isopropoxy group, a butoxy group, a carboxyl group, an amino group, an acetamido group, a dimethylamino group, a cyano group and a nitro group.

**16.** The urea derivative or the pharmacologically acceptable salt thereof according to any one of claims 1 to 9, wherein $R^2$ is a t-butyl group; a phenyl group substituted by a fluorine atom, a chlorine atom, a bromine atom, a methyl group, a trifluoromethyl group, a hydroxymethyl group, a carboxyl group, an amino group or a nitro group at the 2- and 6-positions; a phenyl group substituted by a fluorine atom, a chlorine atom, a bromine atom, a methyl group, an ethyl group, a propyl group, an isopropyl group, a butyl group, a trifluoromethyl group, a methoxy group, a carboxyl group, an amino group, an acetamido group or a cyano group at the 2- and 4-positions; or a phenyl group substituted by a chlorine atom, a bromine atom, a methyl group or a trifluoromethyl group at the 2-position.

**17.** The urea derivative or the pharmacologically acceptable salt thereof according to any one of claims 1 to 9, wherein $R^2$ is a 2,6-difluorophenyl group, a 2-fluoro-6-trifluoromethylphenyl group, a 2,6-dichlorophenyl group, a 2-chloro-6-methylphenyl group, a 2,6-dimethylphenyl group, a 2-hydroxymethyl-6-methylphenyl group, a 2-methoxy-6-methylphenyl group, a 2-carboxy-6-methylphenyl group, a 4-fluoro-2-trifluoromethylphenyl group, a 2-chloro-4-methylphenyl group, 4-carboxy-2-chlorophenyl group, a 4-amino-2-chlorophenyl group, a 4-acetamido-2-chlorophenyl group, a 4-methoxy-2-methylphenyl group, a 4-carboxy-2-methylphenyl group, a 4-amino-2-trifluoromethylphenyl group, a 4-acetamido-2-trifluoromethylphenyl group, a 2-methylphenyl group or a 2-trifluoromethylphenyl group.

**18.** The urea derivative or the pharmacologically acceptable salt thereof according to any one of claims 1 to 17, wherein E is a group having the above formula (II).

**19.** The urea derivative or the pharmacologically acceptable salt thereof according to claim 18, wherein $R^3$ is a hydrogen atom, a fluorine atom, a chlorine atom or a methyl group.

**20.** The urea derivative or the pharmacologically acceptable salt thereof according to claim 18, wherein $R^3$ is a hydrogen atom, a fluorine atom or a chlorine atom.

**21.** The urea derivative or the pharmacologically acceptable salt thereof according to claim 18, wherein $R^3$ is a hydrogen atom.

**22.** The urea derivative or the pharmacologically acceptable salt thereof according to any one of claims 18 to 21, wherein $R^4$ is a hydrogen atom or a methyl group.

**23.** The urea derivative or the pharmacologically acceptable salt thereof according to any one of claims 18 to 21, wherein $R^4$ is a hydrogen atom.

**24.** The urea derivative or the pharmacologically acceptable salt thereof according to any one of claims 18 to 23, wherein $R^5$ is a hydrogen atom or a methyl group.

**25.** The urea derivative or the pharmacologically acceptable salt thereof according to any one of claims 18 to 23, wherein $R^5$is a hydrogen atom.

**26.** The urea derivative or the pharmacologically acceptable salt thereof according to any one of claims 18 to 25, wherein X is a nitrogen atom.

**27.** The urea derivative or the pharmacologically acceptable salt thereof according to any one of claims 18 to 26, wherein U is a group represented by the formula CH.

**28.** The urea derivative or the pharmacologically acceptable salt thereof according to any one of claims 18 to 27, wherein m and n are 1.

**29.** The urea derivative or the pharmacologically acceptable salt thereof according to any one of claims 1 to 17, wherein E is a group having the above formula (XL).

**30.** The urea derivative or the pharmacologically acceptable salt thereof according to any one of claims 1 to 29, wherein A is a single bond, a group represented by the formula -O-C(=O)-, a group represented by the formula -NH-C(=O)- or a carbonyl group.

**31.** The urea derivative or the pharmacologically acceptable salt thereof according to any one of claims 1 to 29, wherein A is a group represented by the formula -O-C(=O)- or a group represented by the formula -NH-C(=O)-.

**32.** The urea derivative or the pharmacologically acceptable salt thereof according to any one of claims 1 to 29, wherein A is a group represented by the formula -NH-C(=O)-.

**33.** The urea derivative or the pharmacologically acceptable salt thereof according to any one of claims 1 to 29, wherein A is a group represented by the formula - CH(OH)-C(=O)-.

**34.** The urea derivative or the pharmacologically acceptable salt thereof according to claim 1, which is represented by
4-{4-[3-(2-ethoxy-phenyl)-ureido]-phenyl}-piperazine-1-carboxylic acid (3-methyl-pyridin-2-yl)-amide,
4-{4-[3-(2-methoxy-5-methyl-phenyl)-ureido]-phenyl}-piperazine-1-carboxylic acid (3-methyl-pyridin-2-yl)-amide,
4-{4-[3-(3,5-dimethoxy-phenyl)-ureido]-phenyl}-piperazine-1-carboxylic acid (3-methyl-pyridin-2-yl)-amide,
4-{5-[3-(2-ethoxy-5-fluoro-phenyl)-ureido]-pyridin-2-yl}-piperazine-1-carboxylic acid (3-methyl-pyridin-2-yl)-amide,
4-{5-[3-(5-chloro-2-methoxy-phenyl)-ureido]-pyridin-2-yl}-piperazine-1-carboxylic acid (3-methyl-pyridin-2-yl)-amide,
4-{4-[3-(2-methoxy-5-methyl-phenyl)-ureido]-phenyl}-piperazine-1-carboxylic acid [2-chloro-4-(2-hydroxy-ethoxy)-phenyl]-amide,
4-{4-[3-(2-methoxy-5-methyl-phenyl)-ureido]-phenyl}-piperazine-1-carboxylic acid [2-chloro-4-(2,3-dihydroxy-propoxy)-phenyl]-amide,
4- {4-[3-(2-methoxy-5-methyl-phenyl)-ureido]-phenyl} -piperazine- l -carboxylic acid [4-(2-hydroxy-ethoxy)-2-trifluoromethyl-phenyl]-amide,
4-{4-[3-(2-methoxy-5-methyl-phenyl)-ureido]-phenyl}-piperazine-1-carboxylic acid [2-chloro-5-(2-hydroxy-ethoxy)-phenyl]-amide,
4-{4-[3-(3-ethoxy-phenyl)-ureido]-phenyl}-piperazine-1-carboxylic acid (3-methyl-pyridin-2-yl)-amide,
4-{2-chloro-4-[3-(2-fluoro-phenyl)-ureido]-phenyl}-piperazine-1-carboxylic acid [4-(2-hydroxy-ethoxy)-2-trifluoromethyl-phenyl]-amide, or
4-{4-[3-(2-methoxy-5-methyl-phenyl)-ureido]-phenyl}-3,6-dihydro-2H-pyridine-1-carboxylic acid (3-methyl-pyridin-2-yl)-amide.

**35.** The urea derivative or the pharmacologically acceptable salt thereof according to claim 1, which is represented by
4-{4-[3-(S-chloro-2-methoxy-phenyl)-ureido]-phenyl}-piperazine-1-carboxylic acid (2,6-dichloro-phenyl)-amide,
4-{4-[3-(5-chloro-2-ethoxy-phenyl)-ureido]-phenyl}-piperazine-1-carboxylic acid (2,6-dichloro-phenyl)-amide,

4-{4-[3-(5-chloro-2-ethoxy-phenyl)-ureido]-phenyl}-piperazine-l-carboxylic acid (2-chloro-6-methyl-phenyl)-amide,
4-{4-[3-(2-ethoxy-5-methyl-phenyl)-ureido]-phenyl}-piperazine-1-carboxylic acid (2-methoxy-6-methyl-phenyl)-amide,
3-chloro-4-[(4-{4-[3-(2-methoxy-5-methyl-phenyl)-ureido]-phenyl}-piperazine-1 - carbonyl)-amino]-benzoic acid,
4-{4-[3-(5-fluoro-2-methoxy-phenyl)-ureido]-phenyl}-piperazine-1-carboxylic acid (4-methoxy-2-methyl-phenyl)-amide,
4-[(4-{4-[3-(2-methoxy-5-methyl-phenyl)-ureido]-phenyl}-piperazine-1-carbonyl)-amino]-3-methyl-benzoic acid,
4-{4-[3-(2-fluoro-phenyl)-ureido]-phenyl}-piperazine-1-carboxylic acid (2-chloro-6-methyl-phenyl)-amide,
4-{4-[3-(2-methoxy-5-methyl-phenyl)-ureido]-phenyl}-piperazine-1-carboxylic acid (4-amino-2-trifluoromethyl-phenyl)-amide, or
4-{4-[3-(2-methoxy-5-methyl-phenyl)-ureido]-phenyl}-piperazine-1-carboxylic acid (4-amino-2-chloro-phenyl)-amide.

**36.** An acyl-coenzyme A: diacylglycerol acyltransferase inhibitor comprising the urea derivative or the pharmacologically acceptable salt thereof according to any one of claims 1 to 35 as an active ingredient.

**37.** A pharmaceutical composition comprising the urea derivative or the pharmacologically acceptable salt thereof according to any one of claims 1 to 35 as an active ingredient.

**38.** The pharmaceutical composition according to claim 37, which has an acyl-coenzyme A: diacylglycerol acyltransferase inhibitory effect.

**39.** The pharmaceutical composition according to claim 38, which is for treatment and/or prevention of adiposity, obesity, hyperlipidemia, hypertriglyceridemia, lipidosis, insulin resistance syndrome, impaired glucose tolerance, diabetes, diabetic complications (including diabetic peripheral neuropathy, diabetic nephropathy, diabetic retinopathy and diabetic macroangiopathy), cataract, gestational diabetes mellitus, polycystic ovary syndrome, arteriosclerosis (including arteriosclerosis caused by the diseases described above and below), atherosclerosis or diabetic atherosclerosis.

**40.** The pharmaceutical composition according to claim 38, which is for treatment and/or prevention of the following diseases caused by adiposity: hyperlipidemia, hypertriglyceridemia, lipidosis, insulin resistance syndrome, impaired glucose tolerance, diabetes, diabetic complications (including diabetic peripheral neuropathy, diabetic nephropathy, diabetic retinopathy and diabetic macroangiopathy), cataract, gestational diabetes mellitus, polycystic ovary syndrome, arteriosclerosis (including arteriosclerosis caused by the diseases described above and below), atherosclerosis, diabetic atherosclerosis, hypertension, cerebrovascular disorders, coronary artery diseases, fatty liver, respiratory abnormality, lower back pain, knee osteoarthritis, gout or cholecystitis.

**41.** The pharmaceutical composition according to claim 38, which is for treatment and/or prevention of adiposity, obesity or hyperlipidemia, hypertriglyceridemia, diabetes, hypertension or arteriosclerosis caused by adiposity.

**42.** The pharmaceutical composition according to claim 38, which is for treatment and/or prevention of adiposity or obesity.

**43.** The pharmaceutical composition according to claim 38, which is for inhibiting absorption of fat from the small intestine.

**44.** Use of the urea derivative or the pharmacologically acceptable salt thereof according to any one of claims 1 to 35 for producing a pharmaceutical composition.

**45.** The use according to claim 44, wherein the pharmaceutical composition inhibits acyl-coenzyme A: diacylglycerol acyltransferase.

**46.** The use according to claim 44, wherein the pharmaceutical composition is a composition for treatment and/or prevention of adiposity, obesity, hyperlipidemia, hypertriglyceridemia, lipidosis, insulin resistance syndrome, impaired glucose tolerance, diabetes, diabetic complications (including diabetic peripheral neuropathy, diabetic nephropathy, diabetic retinopathy and diabetic macroangiopathy), cataract, gestational diabetes mellitus, polycystic ovary syndrome, arteriosclerosis (including arteriosclerosis caused by the diseases described above and below), atherosclerosis or diabetic atherosclerosis.

**47.** The use according to claim 44, wherein the pharmaceutical composition is a composition for treatment and/or prevention of the following diseases caused by adiposity: hyperlipidemia, hypertriglyceridemia, lipidosis, insulin resistance syndrome, impaired glucose tolerance, diabetes, diabetic complications (including diabetic peripheral neuropathy, diabetic nephropathy, diabetic retinopathy, and diabetic macroangiopathy), cataract, gestational diabetes mellitus, polycystic ovary syndrome, arteriosclerosis (including arteriosclerosis caused by the diseases described above and below), atherosclerosis, diabetic atherosclerosis, hypertension, cerebrovascular disorders, coronary artery diseases, fatty liver, respiratory abnormality, lower back pain, knee osteoarthritis, gout or cholecystitis.

**48.** The use according to claim 44, wherein the pharmaceutical composition is a composition for treatment and/or prevention of adiposity, obesity or hyperlipidemia, hypertriglyceridemia, diabetes, hypertension or arteriosclerosis caused by adiposity.

**49.** The use according to claim 44, wherein the pharmaceutical composition is a composition for treatment and/or prevention of adiposity or obesity.

**50.** The use according to claim 44, wherein the pharmaceutical composition is a composition for inhibiting absorption of fat from the small intestine.

**51.** A method for inhibiting acyl-coenzyme A, which comprises administering a pharmacologically effective amount of the urea derivative or the pharmacologically acceptable salt thereof according to any one of claims 1 to 35 to a warm-blooded animal.

**52.** A method for treating and/or preventing a disease, which comprises administering a pharmacologically effective amount of the urea derivative or the pharmacologically acceptable salt thereof according to any one of claims 1 to 35 to a warm-blooded animal.

**53.** The method according to claim 52, wherein the disease includes adiposity, obesity, hyperlipidemia, hypertriglyceridemia, lipidosis, insulin resistance syndrome, impaired glucose tolerance, diabetes, diabetic complications (including diabetic peripheral neuropathy, diabetic nephropathy, diabetic retinopathy and diabetic macroangiopathy), cataract, gestational diabetes mellitus, polycystic ovary syndrome, arteriosclerosis (including arteriosclerosis caused by the diseases described above and below), atherosclerosis or diabetic atherosclerosis.

**54.** The method according to claim 52, wherein the disease includes the following diseases caused by adiposity: hyperlipidemia, hypertriglyceridemia, lipidosis, insulin resistance syndrome, impaired glucose tolerance, diabetes, diabetic complications (including diabetic peripheral neuropathy, diabetic nephropathy, diabetic retinopathy and diabetic macroangiopathy), cataract, gestational diabetes mellitus, polycystic ovary syndrome, arteriosclerosis (including arteriosclerosis caused by the diseases described above and below), atherosclerosis, diabetic atherosclerosis, hypertension, cerebrovascular disorders, coronary artery diseases, fatty liver, respiratory abnormality, lower back pain, knee osteoarthritis, gout or cholecystitis.

**55.** The method according to claim 52, wherein the disease includes adiposity, obesity or hyperlipidemia, hypertriglyceridemia, diabetes, hypertension or arteriosclerosis caused by adiposity.

**56.** The method according to claim 52, wherein the disease includes adiposity or obesity.

**57.** A method for inhibiting absorption of fat from the small intestine, which comprises administering a pharmacologically effective amount of the urea derivative or the pharmacologically acceptable salt thereof according to any one of claims 1 to 35 to a warm-blooded animal.

**58.** The method according to any one of claims 51 to 57, wherein the warm-blooded animal is a human being.

**INTERNATIONAL SEARCH REPORT**

International application No.
PCT/JP2005/012635

A. CLASSIFICATION OF SUBJECT MATTER
Int.Cl[7] C07D211/62, A61K31/4439, 31/444, 31/451, 31/472, 31/495, 31/496, 31/551, A61P1/16, 3/04, 3/06, 3/10, 9/00, 9/10, 9/12, 11/00, 13/12, 15/08, 19/02, 19/06, 21/00, 25/02, 27/02, 27/12,
According to International Patent Classification (IPC) or to both national classification and IPC

B. FIELDS SEARCHED
Minimum documentation searched (classification system followed by classification symbols)
Int.Cl[7] C07D211/62, A61K31/4439, 31/444, 31/451, 31/472, 31/495, 31/496, 31/551, A61P1/16, 3/04, 3/06, 3/10, 9/00, 9/10, 9/12, 11/00, 13/12, 15/08, 19/02, 19/06, 21/00, 25/02, 27/02, 27/12,

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)
CAPLUS(STN), REGISTRY(STN), MEDLINE(STN), BIOSIS(STN), EMBASE(STN)

C. DOCUMENTS CONSIDERED TO BE RELEVANT

| Category* | Citation of document, with indication, where appropriate, of the relevant passages | Relevant to claim No. |
|---|---|---|
| X | HUSAIN, M. et al., Search for potent anthelmintics. Part XI. N1-p-[4-(Phenyl/ p-tolyl)-1-piperazino]phenyl-N3-alkyl/ aryl ureas and thioureas, Journal of the Indian Chemical Society, 1979, Vol.56, No.9, pages 919 to 920 | 1,6,7,10,11, 18-26,28,37, 44 |
| X | JP 62-255480 A (Pfizer Ltd.), 07 November, 1987 (07.11.87), Example 4 & EP 244115 A2 & US 4788196 A | 1,6,7,10,11, 18-26,28,37, 44 |
| A | WO 03/007955 A2 (CANCER RESEARCH TECHNOLOGY LTD.) 30 January, 2003 (30.01.03), Full text (Family: none) | 1-50 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 05 October, 2005 (05.10.05) | 25 October, 2005 (25.10.05) |
| Name and mailing address of the ISA/ Japanese Patent Office | Authorized officer |
| Facsimile No. | Telephone No. |

Form PCT/ISA/210 (second sheet) (January 2004)

**INTERNATIONAL SEARCH REPORT**

International application No.
PCT/JP2005/012635

C (Continuation). DOCUMENTS CONSIDERED TO BE RELEVANT

| Category* | Citation of document, with indication, where appropriate, of the relevant passages | Relevant to claim No. |
|---|---|---|
| A | JP 2000-26294 A (Sankyo Co., Ltd.), 25 January, 2000 (25.01.00), Full text (Family: none) | 1-50 |
| A | JP 53-124237 A (AZIENDE COLORI NAZIONALI AFFINI ACNA S.P.A.), 30 October, 1978 (30.10.78), Full text & FR 2376841 A1 & GB 1554543 A | 1-50 |
| A | GB 1077173 A (American Cyanamid Co.), 26 July, 1967 (26.07.67), Full text (Family: none) | 1-50 |

Form PCT/ISA/210 (continuation of second sheet) (January 2004)

**INTERNATIONAL SEARCH REPORT**

International application No.
PCT/JP2005/012635

**Box No. II Observations where certain claims were found unsearchable (Continuation of item 2 of first sheet)**

This international search report has not been established in respect of certain claims under Article 17(2)(a) for the following reasons:

1. [X] Claims Nos.: 51-58
because they relate to subject matter not required to be searched by this Authority, namely:
Claims 51 to 58 pertain to methods for treatment of the human body by therapy and thus relate to a subject matter which this International Searching Authority is not required, under the provisions of Article 17(2)(a)(i) of the PCT and Rule 39.1(iv) of the Regulations under the PCT, to search.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
This application involves the following four inventions.
1. The compound of claims 1-35, the inhibitor of claim 36, the medicinal composition of claims 37-43, and the use for producing a medicinal composition of claims 44-50 in each of which the part (E) in the general formula (I) is (II)
2. The compound of claims 1-35, the inhibitor of claim 36, the medicinal composition of claims 37-43, and the use for producing a medicinal composition of claims 44-50 in each of which the part (E) in the general formula (I) is (III)
(continued to extra sheet)

1. [ ] As all required additional search fees were timely paid by the applicant, this international search report covers all searchable claims.
2. [X] As all searchable claims could be searched without effort justifying an additional fee, this Authority did not invite payment of any additional fee.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**
[ ] The additional search fees were accompanied by the applicant's protest.
[ ] No protest accompanied the payment of additional search fees.

**INTERNATIONAL SEARCH REPORT**

International application No.
PCT/JP2005/012635

Continuation of A. CLASSIFICATION OF SUBJECT MATTER
(International Patent Classification (IPC))

Int.Cl$^7$ 43/00, C07D211/60, 213/75, 213/76, 217/06, 241/04, 243/08, 295/12, 295/18, 295/20, 401/12, 401/14, 405/12, 417/12

(According to International Patent Classification (IPC) or to both national classification and IPC)

Continuation of B. FIELDS SEARCHED
Minimum documentation searched (International Patent Classification (IPC))

Int.Cl$^7$ 43/00, C07D211/60, 213/75, 213/76, 217/06, 241/04, 243/08, 295/12, 295/18, 295/20, 401/12, 401/14, 405/12, 417/12

Minimum documentation searched (classification system followed by classification symbols)

Continuation of Box No.III of continuation of first sheet(2)

3. The compound of claims 1-35, the inhibitor of claim 36, the medicinal composition of claims 37-43, and the use for producing a medicinal composition of claims 44-50 in each of which the part (E) in the general formula (I) is (XXXIX)

4. The compound of claims 1-35, the inhibitor of claim 36, the medicinal composition of claims 37-43, and the use for producing a medicinal composition of claims 44-50 in each of which the part (E) in the general formula (I) is (XL)

A matter common among these four inventions resides in that they have the structure (nitrogenous heterocycle)-NHCONH-. However, this structure was known before the filing of this application as apparent from the fact that it is disclosed in JP 62-255480 A, WO 03/007955 A2, and FR 1516741 A. Consequently, there is no relationship among those four inventions which has a common special technical feature exceeding the prior art. They are hence not considered to be so linked as to form a single general inventive concept.

Form PCT/ISA/210 (extra sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2002306199 A
- WO 03045926 A

### Non-patent literature cited in the description


- **EIJI ITAGAKI.** STEP series, Metabolism, Endocrinology. KAIBASHOBO, LTD, 1998, 105
- **ZIMMET, P. et al.** *Nature,* 2001, vol. 414, 782-787
- **ENGSTROM, R. G. et al.** *Arch. Intern. Pharmacodyn.,* 1975, vol. 214, 308-321
- **BRAY, G. A. et al.** *Obes. Res.,* 1996, vol. 4, 263-270
- **DAVIDSON, M. H. et al.** *The Journal of the American Medical Association,* 1999, vol. 281, 235-242
- **COLEMAN, R. ; BELL, R.** *J. Biol. Chem.,* 1976, vol. 251, 4537-4543
- **COLEMAN, R.** *Methods in Enzymology,* 1992, vol. 209, 98-104
- **LEHNER, R. ; KUKSIS, A.** *Prog. Lipid Res.,* 1996, vol. 35, 169-201
- **R. BELL.** *Ann. Rev. Biochem.,* 1980, vol. 49, 459-487
- **CASES, S. et al.** *Proc. Natl. Acad. Sci. USA.,* 1998, vol. 95, 13018-13023
- **CASES, S. et al.** *J. Biol. Chem.,* 2001, vol. 276, 38870-38876
- **COLEMAN, R.A. ; LEE, D.P.** *Progress in Lipid Research,* 2004, vol. 43, 134-176
- **SMITH, S.J. et al.** *Nat. Genet.,* 2000, vol. 25, 87-90
- **CHEN, H. C.** *J. Clin. Invest.,* 2002, vol. 109, 1049-1055
- **BUHMAN, K.K.** *J. Biol. Chem.,* 2002, vol. 277, 25474-25479
- **GAZIANO, J. et al.** *Circulation,* 1997, vol. 96, 2520-2525
- **TOMODA, H. et al.** *J. Antibiot. (Tokyo,* 1995, vol. 48, 937-941
- **YANG, D.J. et al.** *J. Antibiot. (Tokyo,* 1996, vol. 49, 223-229
- **TOMODA, H. et al.** *J. Antibiot. (Tokyo,* 1999, vol. 52, 689-694
- **TABATA, N. et al.** *Phytochemistry,* 1997, vol. 46, 683-687
- *J. Org. Chem.,* 1996, vol. 61, 4175
- *J. Org. Chem.,* 2004, vol. 69, 1866
- *SYNTHESIS,* 1996, vol. 877
- *Tetrahedron Lett.,* 1994, vol. 35, 9003
- *Tetrahedron Lett.,* 2000, vol. 41, 385
- *J. Org. Chem.,* 1961, vol. 26, 2223
- *J. Med. Chem.,* 2003, vol. 46, 1690
- *J. Med. Chem.,* 2000, vol. 43, 2703
- *Org. Lett.,* 2001, vol. 3, 2317
- *J. Med. Chem.,* 1999, vol. 42, 5277
- *Pharmazie,* 1992, vol. 47, 295
- **T. W. GREENE et al.** Protective Groups in Organic Synthesis. John Wiley & Sons, Inc, 1999
- **THEODORA W. GREENE ; PETER G. M. WUTS.** Protective Groups in Organic Synthesis. A Wiley-Interscience Publication, 1999
- **COTTS, LAN G.C. et al.** *J. Chem. Soc. Perkin Trans.,* 1985, vol. 1, 1829-1836
- *Chem. Ber.,* vol. 12, 818-821
- *Mol. Cryst. Liq. Cryst. Sci. Tecnol. Sect. A,* 1993, vol. 237, 399-406
- **CASES, S. et al.** *Proc. Natl. Acad. Sci. USA,* 1998, vol. 95, 13018-13023